# EUROPEAN PATENT APPLICATION

(11) **EP 2 298 359 A1**
(43) Date of publication of application: **23.03.2011**
(21) Application number: 09758404.9
(22) Date of filing: 04.06.2009
(51) Int. Cl.: A61K 48/00, A61K 31/713, A61K 35/76, A61K 45/00, A61P 11/00, A61P 11/06, A61P 17/00, A61P 37/08

(54) **NUCLEIC ACID CAPABLE OF CONTROLLING DEGRANULATION OF MAST CELL**

(30) Priority: 04.06.2008 JP 2008146431
(71) Applicant: Kyowa Hakko Kirin Co., Ltd., Tokyo 100-8185 (JP)
(72) Inventor: KOSAKA, Kyoko, Hyogo 679-2122 (JP); YAMADA, Yoji, Sunto-gun,Shizuoka 411-8731 (JP); MIURA, Kasumi, Sunto-gun,Shizuoka 411-8731 (JP); MIYAZAWA, Tatsuya, Tokyo 194-8533 (JP); YOSHIDA, Tetsuo, Tokyo 194-8533 (JP)
(74) Representative: Vossius & Partner
(86) International application number: PCT/JP2009/060288
(87) International publication number: WO 2009/148137

(57) **Abstract**

Provided are a mast cell degranulation control agent, a diagnostic agent or therapeutic agent for a disease resulting from mast cell degranulation control, a method of controlling mast cell degranulation, and a screening method for a mast cell degranulation control agent, all of which involve the use of a nucleic acid and the like. These are useful in the diagnosis or treatment of a disease resulting from mast cell degranulation control.

## Description

### Technical Field

The present invention relates to a mast cell degranulation control agent, a diagnostic agent or therapeutic agent for a disease resulting from an abnormality of mast cell degranulation, control, a method of controlling mast cell degranulation, and a screening method for a mast cell degranulation control agent.

### Background Art

Micro-RNA (miRNA), which is one kind of nucleic acids, is a small non-coding single-stranded RNA of about 22 nucleotides that is not translated into a protein, and has known as being present in many types in organisms, including humans (non-patent documents 1 and 2). A micro-RNA is produced from a gene transcribed to a single or clustered micro-RNA precursor. Specifically, first, a primary-miRNA (pri-miRNA), which is a primary transcript, is transcribed from the gene, then, in stepwise processing from the pri-miRNA to a mature type micro-RNA, a precursor-miRNA (pre-miRNA) of about 70 nucleotides having a characteristic hairpin structure is produced from the pri-miRNA. Furthermore, the mature type micro-RNA is produced from the pre-miRNA by Dicer-mediated processing (non-patent document 3).

A mature type micro-RNA is thought to be involved in the post-transcriptional control of gene expression by complementarily binding to an mRNA for a target to suppress the translation of the mRNA, or to degrade the mRNA.
Although the mechanism in which micro-RNAs suppress the expression of target mRNAs has not been clarified in full, its outline is being elucidated through recent years' research. A micro-RNA suppresses the expression of the mRNA for a target by binding to a partially complementary sequence in the 3'-untranslational region (3'-UTR) of the target mRNA to suppress the translation thereof, or to degrade the target mRNA. Although the complementarity may not be complete, it has been shown that the complementarity of the 2nd to 8th nucleotides from the 5'-end of the micro-RNA is particularly important; this region is sometimes called the "seed sequence" of the micro-RNA (non-patent document 4). It has been shown that micro-RNAs having a same seed sequence suppress the expression of a same target mRNA even if their other sequences differ. Therefore, by making a sequence complementary to a sequence present at the 3'-end of an optionally chosen mRNA as the seed sequence, an RNA having micro-RNA-like activity can be designed. Usually, the term micro-RNA, unlike siRNA, often refers exclusively to an RNA that occurs naturally in cells, so a micro-RNA-like sequence designed as described above is sometimes particularly referred to as an artificial micro-RNA.

As of August 2007, in the micro-RNA database miRBase (http://microrna.sanger.ac.uk/), 533 species of micro-RNAs were registered for humans, and 5,071 species for all organisms. Of the micro-RNAs expressed in mammals, including humans, only some have their physiological functions elucidated to date, including miR-181, which is involved in hematopoietic lineage differentiation (non-patent document 5), miR-375, which is involved in insulin secretion (non-patent document 6), and the like; many have their bioactivities unclarified. However, studies using nematodes or Drosophila have shown that micro-RNAs play various important roles in the development and differentiation in organisms, and a report of the relation to human diseases has been reported suggesting a profound relation to cancers (non-patent document 7).

Mast cells are known to become activated by various stimuli to undergo degranulation and release or produce many inflammatory mediators (Non-patent Documents 8 to 10). These mediators are diverse and include amines, arachidonic acid metabolites, proteases, cytokines, chemokines and the like. For example, it is known that when an antigen is recognized by a mast cell, histamine and tryptase are quickly released upon degranulation, and chemical mediators such as prostaglandin D2 (PGD2), leukotriene (LT), and platelet activation factor (PAF), various chemokines such as macrophage inflammatory protein (MIP)-1α, and various cytokines such as granulocyte macrophage colony stimulation factor (GM-CSF) are newly synthesized and released. Regarding major basic proteins, which are cytotoxic proteins that have been thought to be produced by eosinophils, it has recently been shown that in the case of humans, they are produced in large amounts by mast cells (non-patent document 11).

Hence, mast cells are thought to play major roles in the pathogenesis of various allergic diseases; therefore, it is thought that by controlling a function of mast cells, treatment of allergic diseases is possible.
Although a wide variety of existing therapeutic agents for allergies are known to possess the action of suppressing the release of inflammatory mediators from mast cells, their studies have traditionally been conducted mainly using rodent mast cells, and there have been no adequate investigations of whether the existing therapeutic agents are actually effective on human mast cells. However, recently, a method of preparing human mast cells has been established, enabling analyses of the actions of existing drugs on human mast cells, and enabling comparisons with their actions on rodent mast cells. As a result, it is known that rodent mast cells and human mast cells have different reactivities to drugs (non-patent document 9). For example, sodium cromoglicate, which was used as a suppressant of inflammatory mediator release, remarkably suppressed the IgE-dependent release of inflammatory mediators in rat abdominal mast cells, but the action thereof on human mast cells was not potent (non-patent document 12). Azelastine hydrochloride, at high concentrations, suppressed the release of histamine, PGD2, and LT and production of GM-CSF and MIP-1α, from human mast cells in culture, but none of these activities were potent. Suplatast tosilate, which was used as an anti-cytokine drug, exhibited inflammatory mediator release suppressive action on rat mast cells, but lacked action on human mast cells (non-patent document 12).

Genes expressed in mast cells are important in that they are potential targets of therapeutic agents for a wide variety of allergic diseases; for example, drugs that act on GPCR expressed in mast cells are known to remarkably suppress the production of inflammatory mediators from cultured human mast cells. Specifically, the β2 adrenaline receptor stimulant isoproterenol suppresses the release of histamine, LT, PGD2, GM-CSF and MIP-1α from cultured human mast cells by 80% or more at a low concentration of 10 nmol/l (Non-patent Document 13).
As a result of a comparison of genes whose expression is induced in human and mouse mast cells activated by various stimuli, it was shown that the genes expressed in humans and mice do not always agree with each other (Non-patent Document 14). Additionally, as stated above, there are also interspecific differences in drug susceptibility between humans and mice.
In view of the association of micro-RNAs in gene expression control, it is thought that micro-RNAs expressed in mast cells can also be candidates for therapeutic agents for a wide variety of allergic diseases. At present, an analysis of micro-RNAs expressed in mouse marrow-derived mast cells (Non-patent Document 15) is the only available relevant report, with no reports on micro-RNAs expressed in human mast cells. Taking into account the interspecific differences between humans and mice, it is difficult to predict information on the expression of micro-RNAs in human mast cells on the basis of information on the expression of micro-RNAs in mouse mast cells. Additionally, there is no knowledge about the involvement of micro-RNAs in the diverse functions of mast cells.

### [Conventional Art Documents]

### [Non-patent Documents]

non-patent document 1 : Science, 294, 853-858, (2001)
non-patent document 2 : Cell, 113, 673-676, (2003)
non-patent document 3: Nature Reviews Genetics, 5, 522-531, (2004)
non-patent document 4: Current Biology, 15, R458-R460 (2005)
non-patent document 5: Science, 303, 83-86, (2004)
non-patent document 6: Nature, 432, 226-230, (2004)
non-patent document 7: Nature Reviews Cancer, 6, 259-269, (2006)
non-patent document 8: Himan Saibo no Rinsho, ed. Motohito Kurosawa, Sentan Igaku-sha Ltd., p 142 (2001)
non-patent document 9: Himan Saibo no Rinsho, ed. Motohito Kurosawa, Sentan Igaku-sha Ltd., p 559 (2001)
non-patent document 10: Crit. Rev. Immunol., 22, 115-140 (2002)
non-patent document 11: Blood, 98, 1127-1134 (2001)
non-patent document 12: Clin. Exp. Allergy, 28, 1228-1236 (1998)
non-patent document 13: J. Allergy Clin. Immunol., 103, 421-426 (1999)
non-patent document 14: Blood, 100, 3861-3868 (2002)
non-patent document 15: Genome Biology, 6, R71 (2005)

### [Summary of the Invention]

### Problems to Be Solved by the Invention

It is expected that by identifying nucleic acids such as micro-RNAs and precursors thereof expressed in various human organs, and analyzing the functions thereof to elucidate their relations to diseases, new therapeutic agents and diagnostic agents will be developed.
In particular, finding nucleic acids, such as micro-RNAs and precursors thereof, that act in mast cells is expected to lead to the elucidation of functions such as mast cell differentiation and degranulation, cytokine-producing mast cell differentiation and degranulation, inflammatory mediator production, cytokine production, and chemokine production, and hence lead to the development of methods of mast cell isolation, cultivation, differentiation control, degranulation control, inflammatory mediator production control, cytokine production control, and chemokine production control, and new therapies for allergic diseases and the like involving the utilization thereof.

It is an object of the present invention to provide a mast cell degranulation control agent, a diagnostic agent or therapeutic agent for a disease resulting from mast cell degranulation control, a method of controlling mast cell degranulation, and a screening method for a mast cell degranulation control agent, all of which involve the use of a nucleic acid and the like.

### Means for Solving the Problems

The present invention relates to the following (1) to (18).
(1) A mast cell degranulation control agent comprising as an active ingredient any one of the nucleic acids (a) to (h) below:
   (a) a nucleic acid consisting of a nucleotide sequence of any one of SEQ ID NOs:1 to 1543 and 3372 to 3741,
   (b) a nucleic acid of 17 to 28 nucleotides comprising a nucleic acid consisting of a nucleotide sequence of any one of SEQ ID NOs:1 to 1543 and 3372 to 3741,
   (c) a nucleic acid consisting of a nucleotide sequence having an identity of 90% or more to a nucleotide sequence of any one of SEQ ID NOs:1 to 1543 and 3372 to 3741,
   (d) a nucleic acid that hybridizes under stringent conditions with a complementary strand for a nucleic acid consisting of a nucleotide sequence of any one of SEQ ID NOs:1 to 1543 and 3372 to 3741,
   (e) a nucleic acid comprising the 2nd to 8th nucleotides of a nucleotide sequence of any one of SEQ ID NOs:1 to 1543 and 3372 to 3741,
   (f) a nucleic acid consisting of a nucleotide sequence of any one of SEQ ID NOs:1544 to 3371 and 3742 to 4147,
   (g) a nucleic acid consisting of a nucleotide sequence having an identity of 90% or more to a nucleotide sequence of any one of SEQ ID NOs:1544 to 3371 and 3742 to 4147, and
   (h) a nucleic acid that hybridizes under stringent conditions with a complementary strand for a nucleic acid consisting of a nucleotide sequence of any one of SEQ ID NOs:1544 to 3371 and 3742 to 4147.
(2) The mast cell degranulation control agent according to (1), wherein the nucleic acid is a micro-RNA or a micro-RNA precursor.
(3) A mast cell degranulation control agent comprising as an active ingredient a nucleic acid consisting of a nucleotide sequence complementary to the nucleotide sequence of the nucleic acid of (1).
(4) A mast cell degranulation control agent comprising as an active ingredient a double-stranded nucleic acid consisting of the nucleic acid of (1) and a nucleic acid consisting of a nucleotide sequence complementary to the nucleotide sequence of the former nucleic acid.
(5) A mast cell degranulation control agent comprising as an active ingredient a vector that expresses the nucleic acid of any one of (1) to (3) or the double-stranded nucleic acid of (4).
(6) A mast cell degranulation control agent comprising as an active ingredient a substance that controls the expression or a function of the nucleic acid of (1).
(7) The mast cell degranulation control agent according to (6), wherein the substance that controls the expression or a function of the nucleic acid is an siRNA or an antisense oligonucleotide.
(8) A mast cell degranulation control agent comprising as an active ingredient a substance that suppresses the expression of a target gene for the nucleic acid of (1).
(9) The mast cell degranulation control agent according to (8), wherein the substance that suppresses the expression of a target sequence for a nucleic acid is an siRNA or an antisense oligonucleotide.
(10) A diagnostic agent or therapeutic agent for a disease resulting from an abnormality of mast cells, comprising as an active ingredient the nucleic acid of any one of (1) to (3), the double-stranded nucleic acid of (4), the vector of (5), or the substance of any one of (6) to (9).
(11) A diagnostic agent for a disease resulting from an abnormality of mast cells, comprising as an active ingredient a reagent for detecting the amount expressed of the nucleic acid of (1), a mutation of the nucleic acid or a mutation of the genome that encodes the nucleic acid.
(12) The diagnostic agent or therapeutic agent according to (10) or (11), wherein the disease resulting from an abnormality of mast cells is a disease selected from the group consisting of atopic dermatitis, asthma, chronic obstructive pulmonary disease and allergic diseases.
(13) A method for treating a disease resulting from an abnormality of mast cells, comprising administering an effective amount of the degranulation suppressant of any one of (1) to (9) to a subject in need thereof.
(14) The method according to (13), wherein the disease resulting from an abnormality of mast cells is a disease selected from the group consisting of atopic dermatitis, asthma, chronic obstructive pulmonary disease and allergic diseases.
(15) A use of the degranulation suppressant of any one of (1) to (9) for producing a therapeutic agent for a disease resulting from an abnormality of mast cells.
(16) The use according to (15), wherein the disease resulting from an abnormality of mast cells is a disease selected from the group consisting of atopic dermatitis, asthma, chronic obstructive pulmonary disease and allergic diseases.
(17) A method of controlling mast cell degranulation, comprising using the nucleic acid of any one of (1) to (3), the double-stranded nucleic acid of (4), the vector of (5), or the substance of any one of (6) to (9).
(18) A screening method for a mast cell degranulation control agent, wherein the promotion or suppression of the expression or a function of the nucleic acid of (1) serves as an index.

### Effect of the Invention

According to the present invention, it is possible to provide a mast cell degranulation control agent, a diagnostic agent or therapeutic agent for a disease resulting from mast cell degranulation control, a method of controlling mast cell degranulation, and a screening method for a mast cell degranulation control agent, all of which involve the use of a nucleic acid and the like.

### [Mode for Carrying out the Invention]

The nucleic acid used in the present invention may be any molecule, as far as it is a molecule resulting from polymerization of a nucleotide or a molecule functionally equivalent to the nucleotide; for example, an RNA, which is a ribonucleotide polymer, a DNA, which is a deoxyribonucleotide polymer, a mixed polymer of RNA and DNA, and a nucleotide polymer, including a nucleotide analogue, can be mentioned; furthermore, the nucleic acid may be a nucleotide polymer, including a nucleic acid derivative. In addition, the nucleic acid in the present invention may be a single-stranded nucleic acid or a double-stranded nucleic acid. Double-stranded nucleic acids include double-stranded nucleic acids wherein one strand hybridizes with the other strand under stringent conditions.

The nucleotide analogue may be any molecule, as far as it is a molecule prepared by modifying a ribonucleotide, a deoxyribonucleotide, an RNA or a DNA in order to improve the nuclease resistance thereof, to stabilize the same, to increase the affinity thereof for a complementary chain nucleic acid, to increase the cell permeability thereof, or to visualize the same, compared with the RNA or DNA; for example, a nucleotide analogue modified at the sugar moiety thereof, a nucleotide analogue modified at phosphoric acid diester bond and the like can be mentioned.

The nucleotide analogue modified at the sugar moiety thereof may be any one, as far as an optionally chosen chemical structural substance has been added to, or substituted for, a portion or all of the chemical structure of the sugar of the nucleotide; for example, a nucleotide analogue substituted by 2'-O-methylribose, a nucleotide analogue substituted by 2'-O-propylribose, a nucleotide analogue substituted by 2'-methoxyethoxyribose, a nucleotide analogue substituted by 2'-O-methoxyethylribose, a nucleotide analogue substituted by 2'-O-[2-(guanidium)ethyl]ribose, a nucleotide analogue substituted by 2'-O-fluororibose, a bridged nucleic acid (BNA) having two cyclic structures as a result of introduction of a bridging structure into the sugar moiety, more specifically a locked nucleic acid (LNA) wherein the oxygen atom at the 2' position and the carbon atom at the 4' position have been bridged via methylene, and an ethylene bridged nucleic acid) (ENA) [Nucleic Acid Research, 32, e175 (2004)] can be mentioned, and a peptide nucleic acid (PNA) [Acc. Chem. Res., 32, 624 (1999)], an oxypeptide nucleic acid (OPNA) [J. Am. Chem. Soc., 123, 4653 (2001)], and a peptide ribonucleic acid (PRNA) [J. Am. Chem. Soc., 122, 6900 (2000)] and the like can also be mentioned.

The nucleotide analogue modified at phosphoric acid diester bond may be any one, as far as an optionally chosen chemical substance has been added to, or substituted for, a portion or all of the chemical structure of the phosphoric acid diester bond of the nucleotide; for example, a nucleotide analogue substituted by a phosphorothioate bond, a nucleotide analogue substituted by an N3'-P5' phosphoamidate bond, and the like can be mentioned [SAIBO KOGAKU, 16, 1463-1473 (1997)] [RNAi Method and Antisense Method, Kodansha (2005)].

The nucleic acid derivative may be any molecule, as long as it is a molecule prepared by adding another chemical substance to the nucleic acid in order to improve the nuclease resistance thereof, to stabilize the same, to increase the affinity thereof for a complementary chain nucleic acid, to increase the cell permeability thereof, or to visualize the same, compared with the nucleic acid; for example, a 5'-polyamine conjugated derivative, a cholesterol conjugated derivative, a steroid conjugated derivative, a bile acid conjugated derivative, a vitamin conjugated derivative, a Cy5 conjugated derivative, a Cy3 conjugated derivative, a 6-FAM conjugated derivative, a biotin conjugated derivative and the like can be mentioned.

As examples of nucleic acids used in the present invention, the following nucleic acids (a) to (k) can be mentioned.
(a) A nucleic acid consisting of a nucleotide sequence of any one of SEQ ID NOs:1 to 1543 and 3372 to 3741.
(b) A nucleic acid of 17 to 28 nucleotides comprising a nucleic acid consisting of a nucleotide sequence of any one of SEQ ID NOs:1 to 1543 and 3372 to 3741.
(c) A nucleic acid consisting of a nucleotide sequence having an identity of 90% or more to a nucleotide sequence of any one of SEQ ID NOs:1 to 1543 and 3372 to 3741.
(d) A nucleic acid that hybridizes under stringent conditions with a complementary strand for a nucleic acid consisting of a nucleotide sequence of any one of SEQ ID NOs:1 to 1543 and 3372 to 3741.
(e) A nucleic acid comprising the 2nd to 8th nucleotides of a nucleotide sequence of any one of SEQ ID NOs:1 to 1543 and 3372 to 3741.
(f) A double-stranded nucleic acid consisting of one of the nucleic acids (a) to (e) and a nucleic acid comprising a nucleotide sequence complementary to the nucleotide sequence of the nucleic acid, or a nucleic acid comprising the double-stranded nucleic acid.
(g) A double-stranded nucleic acid consisting of one of the nucleic acids (a) to (e) and a nucleic acid that hybridizes under stringent conditions with the nucleic acid, or a nucleic acid comprising the double-stranded nucleic acid.
(h) A single-stranded nucleic acid having a hairpin structure wherein the double-stranded nucleic acid (f) or (g) is joined via a spacer oligonucleotide consisting of 8 to 28 nucleotides, or a nucleic acid comprising the single-stranded nucleic acid.
(i) A nucleic acid consisting of a nucleotide sequence of any one of SEQ ID NOs:1544 to 3371 and 3742 to 4147.
(j) A nucleic acid consisting of a nucleotide sequence having an identity of 90% or more to a nucleotide sequence of any one of SEQ ID NOs:1544 to 3371 and 3742 to 4147.
(k) A nucleic acid that hybridizes under stringent conditions with a complementary strand for a nucleic acid consisting of a nucleotide sequence of any one of SEQ ID NOs:1544 to 3371 and 3742 to 4147.

As the above-described nucleic acids, micro-RNAs are preferably used. A micro-RNA refers to a single-stranded RNA 17 to 28 nucleotides long. The peripheral genome sequence, including the sequence, of a micro-RNA has a sequence capable of forming a hairpin structure, and the micro-RNA can be cut out from either one strand of the hairpin. A micro-RNA complementarily binds to an mRNA serving as a target therefor to suppress the translation of the mRNA or promote the decomposition of the mRNA, thereby mediating the post-translational control of gene expression.

As examples of micro-RNAs used in the present invention, human micro-RNAs consisting of a nucleotide sequence of any one of SEQ ID NOs: 1 to 150 and 3372 to 3406 can be mentioned. Furthermore, as micro-RNAs having the same function as the function of human micro-RNAs consisting of a nucleotide sequence of any one of SEQ ID NOs:1 to 150 and 3372 to 3406, nucleic acids consisting of a nucleotide sequence of any one of SEQ ID NOs: 151 to 1237 and 3407 to 3684, which are orthologues of the human micro-RNAs, can be mentioned. As specific examples of orthologues of the human micro-RNA of SEQ ID NO:1, those consisting of a nucleotide sequence of any one of SEQ ID NOs:151 to 173 and 3407 can be mentioned. Tables of the correspondence of micro-RNAs consisting of a nucleotide sequence of any one of SEQ ID NOs:1 to 150 and 3372 to 3406 and orthologues thereof are shown in Table 1-1 to Table 1-8. The biological species shown in the uppermost fields of Table 1-1 to Table 1-8 are as follows: hsa, Homo sapiens, human being; mmu, Mus musculus, mouse; rno, Rattus norvegicus, rat; xla, Xenopus laevis, African clawed toad; xtr, Xenopus tropicalis, tropical clawed toad; gga, Gallus gallus, chicken; cfa, Canis familiaris, dog; mdo, Monodelphis domestica, gray short-tailed opossum; age, Ateles geoffroyi, black-handed spider monkey; 11a, Lagothrix lagotricha, Humboldt's wooly monkey; sla, Saguinus labiatus, red-bellied tamarin; mml, Macaca mulatta, rhesus monkey; mne, Macaca nemestrina, pig-tailed macaque; ggo, Gorilla gorilla, gorilla; ppa, Pan paniscus, bonobo; ptr, Pan troglodytes, chimpanzee; ppy, Pongo pygmaeus, orangutan; lca, Lemur catta, ring-tailed lemur; cgr, Cricetulus griseus, Chinese hamster; bta, Bos taurus, cattle; oar, Ovis aries, sheep; ssc, Sus scrofa, swine; dre, Danio rerio, zebrafish; fru, Fugu rubripes, globefish; tni, Tetraodon nigroviridis, green spotted puffer. Because humanderived micro-RNAs and orthologues thereof have high sequence identity, they are considered to possess similar functions. Also because micro-RNAs in the same subgroup as classified by alphabetical figures added to the ends of their names also share high sequence identity, they are considered to possess similar functions.

**[Table 1-1]**

| **sequence number** | **human miRNA** | **orthologue sequence number** | | | | | | | | | | | | |
|---|---|---|---|---|---|---|---|---|---|---|---|---|---|---|
| | | mmu | rno | cgr | age | lla | sla | mml | mne | pbi | ggo | ppa | ptr | ppy |
| seqNo | miRNAname | mmu | rno | cgr | age | lla | sla | mml | mne | pbi | ggo | ppa | ptr | ppy |
| 1 | hsa-miR-16 | 151 | 152 | | 153 | 154 | 155 | 156 | 157 | | 158 | 159 | 160 | 161 |
| 2 | hsa-miR-195 | 174 | 175 | | | | | 176 | | | 177 | 178 | | |
| 3 | hsa-miR-17-3p | 3408 | 181 | | 182 | 183 | 184 | 185 | 186 | | 187 | 188 | 189 | 190 |
| | | 196 | | | | | | 201 | | | | | | |
| 4 | hsa-miR-18a | 217 | 197 | | 198 | 199 | 200 | 218 | 202 | | 203 | 204 | 205 | 206 |
| 5 | hsa-miR-18b | | | | | | | | | | | | | |
| | | 3412 | 224 | | | | | 228 | | | | | | |
| 6 | hsa-miR-20b | 223 | 3413 | | 225 | 226 | 227 | 229 | 230 | | 231 | 232 | 233 | 234 |
| 7 | hsa-miR-21 | 250 | 251 | 252 | 253 | | | 254 | 255 | | 256 | 257 | 258 | 259 |
| 8 | hsa-miR-24 | 268 | 269 | | | | | 270 | 271 | | 272 | 273 | 274 | 275 |
| 9 | hsa-miR-25 | 285 | 286 | | | 287 | | 288 | 289 | | 290 | 291 | 292 | 293 |
| 10 | hsa-miR-32 | 301 | 302 | | | | 303 | 304 | 305 | | 306 | 307 | 308 | 309 |
| | | 3415 | 315 | | | | | 318 | | | | | | |
| 11 | hsa-miR-26b | 314 | 316 | | | 317 | | 319 | 320 | | 321 | 322 | 323 | 324 |
| | | | 3418 | | | | | | | | | | | |
| | | 3416 | 336 | | | | | | | | | | | |
| 12 | hsa-miR-30a-3p | 3417 | 337 | | | | | 338 | | | 339 | 340 | 341 | 342 |
| | | 347 | | | | | | | | | | | | |
| | | 348 | 349 | | | | | 354 | | | | | | |
| 13 | hsa-miR-34a | 3419 | 350 | | 351 | 352 | 353 | 355 | 356 | | 357 | 358 | 359 | 360 |
| | | 372 | | | | | | | | | | | | |
| | | 380 | | | | | | 375 | | | | | | |
| 14 | hsa-miR-449 | 373 | 374 | | | | | 381 | | | | | | |
| 15 | hsa-miR-449b | | | | | | | | | | | | | |
| 16 | hsa-miR-107 | 382 | 383 | | | 384 | | 385 | 386 | | 387 | 388 | 389 | 390 |
| 17 | hsa-miR-107 | | | | | | | | | | | | 400 | |
| | | 3420 | | | | | | 405 | | | | | | |
| 18 | hsa-miR-148b | 404 | 3421 | | | | | 406 | | | | | | |
| | | 418 | 419 | | | | | 421 | | | | | | |
| 19 | hsa-miR-190 | 3422 | 420 | | | | | 422 | | | 423 | 424 | 425 | |
| | | 3423 | | | | | | 433 | | | | | | |
| 20 | hsa-miR-199a | 3424 | 3425 | | | 431 | 432 | 3426 | 434 | | 435 | 436 | 437 | 438 |
| 21 | hsa-miR-199b | | | | | | | | | | | | | |
| 22 | hsa-miR-202* | | | | | | | | | | | | | |
| | | 3429 | | | | | | 450 | | | | | | |
| 23 | hsa-miR-208 | 448 | 449 | | | | | 451 | | | | | | |
| 24 | hsa-miR-210 | 454 | 455 | | | | | 456 | | | | | | |
| 25 | hsa-miR-211 | 461 | 462 | | | | | 463 | 464 | | | | | 465 |
| 26 | hsa-miR-214 | 467 | 468 | | 469 | | 470 | 471 | 472 | | 473 | 474 | 475 | 476 |
| 27 | hsa-miR-218 | 484 | 485 | | 486 | 487 | 488 | 489 | 490 | | 491 | 492 | 493 | 494 |
| 28 | hsa-miR-299-5p | 3431 | 3432 | | | | | 506 | | | | | | |
| 29 | hsa-miR-325 | 3433 | 3434 | | | | | 507 | | | | | | |
| 30 | hsa-miR-328 | 509 | 510 | | | | | | | | | | | |
| 31 | hsa-miR-329 | 512 | 513 | | | | | 514 | | | | | | |
| 32 | hsa-miR-338 | 3435 | 515 | | | | | 3436 | | | | | | |
| 33 | hsa-miR-345 | | | | | | | | | | | | | |
| 34 | hsa-miR-425-5p | 3437 | 3438 | | | | | 3439 | | | | | | |
| 35 | hsa-miR-484 | 523 | 524 | | | | | 525 | | | | | | |
| 36 | hsa-miR-485-5p | 3441 | 3442 | | | | | 527 | | | | | | |
| 37 | hsa-miR-486 | 528 | | | | | | 3444 | | | | | | |
| 38 | hsa-miR-488 | 3445 | | | | | | | | | | | | |
| 39 | hsa-miR-510 | | | | | | | | | | | | | |
| 40 | hsa-miR-515-3p | | | | | | | | | | | | | |
| 41 | hsa-miR-515-5p | | | | | | | | | | | | | |
| 42 | hsa-miR-517* | | | | | | | | | | | | | |
| | | | | | | | | 3446 | | | | | | |
| | | | | | | | | 529 | | | | | | |
| | | | | | | | | 530 | | | | | | |
| | | | | | | | | 3447 | | | | | | |
| 43 | hsa-miR-520d | | | | | | | 531 | | | | | | |
| 44 | hsa-miR-520f | | | | | | | 532 | | | | | | |
| | | | | | | | | 3448 | | | | | | |
| 45 | hsa-miR-520h | | | | | | | 533 | | | | | | |
| 46 | hsa-miR-522 | | | | | | | 534 | | | | | | |
| 47 | hsa-miR-525* | | | | | | | | | | | | | |
| 48 | hsa-miR-573 | | | | | | | 535 | | | | | | |
| 49 | hsa-miR-587 | | | | | | | | | | | | | |
| 50 | hsa-miR-593 | | | | | | | | | | | | | |

**[Table 1-2]**

| **sequence number** | **human miRNA** | **orthologue sequence number** | | | | | | | | | | | | |
|---|---|---|---|---|---|---|---|---|---|---|---|---|---|---|
| | | mmu | rno | cgr | age | lla | sla | mml | mne | pbi | ggo | ppa | ptr | ppy |
| 51 | hsa-miR-595 | | | | | | | | | | | | | |
| 52 | hsa-miR-604 | | | | | | | | | | | | | |
| 53 | hsa-miR-612 | | | | | | | | | | | | | |
| 54 | hsa-miR-625 | | | | | | | | | | | | | |
| 55 | hsa-miR-634 | | | | | | | | | | | | | |
| 56 | hsa-miR-635 | | | | | | | | | | | | | |
| 57 | hsa-miR-637 | | | | | | | | | | | | | |
| 58 | hsa-miR-647 | | | | | | | | | | | | | |
| | | | | | | | | 3449 | | | | | | |
| | | | | | | | | 536 | | | | | | |
| 59 | hsa-miR-650 | | | | | | | 537 | | | | | | |
| 60 | hsa-miR-654 | | | | | | | 3450 | | | | | | |
| 61 | hsa-miR-658 | | | | | | | | | | | | | |
| 62 | hsa-miR-660 | | | | | | | 538 | | | | | | |
| 63 | hsa-miR-668 | 541 | | | | | | 542 | | | | | | |
| 64 | hsa-miR-675 | 3451 | | | | | | 543 | | | | | | |
| 65 | hsa-miR-765 | | | | | | | 544 | | | | | | |
| 66 | hsa-miR-766 | | | | | | | | | | | | | |
| 67 | hsa-miR-194 | 545 | 546 | | 547 | | | 548 | 549 | | 550 | | 551 | 552 |
| 68 | hsa-miR-500 | | | | | | | | | | | | | |
| 69 | hsa-miR-197 | 560 | | | 561 | | | 562 | 563 | | | 564 | 565 | 566 |
| 70 | hsa-miR-221 | 568 | 569 | | | | | 570 | | | 571 | 572 | | 573 |
| | | 582 | | | | | | 596 | | | | | | |
| | | 583 | 589 | | | | | 597 | | | | | | |
| | | 584 | 590 | | | | | 598 | | | | | | |
| | | 585 | 591 | | | | | 599 | | | | | | |
| | | 586 | 592 | | | | | 600 | | | | | | |
| | | 587 | 593 | | | | | 601 | | | | | | |
| | | 3453 | 594 | | | | | 602 | | | | | | |
| 71 | hsa-let-7e | 588 | 595 | | | | | 603 | | | | | | |
| 72 | hsa-miR-1 | 669 | 670 | | | | | 671 | | | | 672 | | |
| 73 | hsa-miR-206 | 681 | 682 | | | | | 683 | 684 | | | | | 685 |
| 74 | hsa-miR-613 | | | | | | | | | | | | | |
| 75 | hsa-miR-9 | 691 | 692 | | 693 | 694 | | 695 | 696 | | 697 | | 698 | |
| | | 3458 | 708 | | | | | 712 | | | | 716 | 718 | 720 |
| 76 | hsa-miR-23b | 707 | 709 | | 710 | | 711 | 713 | 714 | | 715 | 717 | 719 | 721 |
| | | 3459 | 740 | | | | | 744 | | | | | | |
| 77 | hsa-miR-27b | 739 | 741 | | 742 | | 743 | 745 | 746 | | 747 | 748 | 749 | 750 |
| 78 | hsa-miR-28 | 774 | 775 | | 776 | 777 | 778 | 779 | 780 | | 781 | 782 | 783 | 784 |
| 79 | hsa-miR-31 | | | | | | | 786 | 787 | | 788 | 789 | 790 | 791 |
| | | | | | | | | 794 | | | | | | |
| 80 | hsa-miR-33b | 3461 | 793 | | | | | 795 | 796 | | 797 | 798 | 799 | 800 |
| 81 | hsa-miR-96 | 805 | 806 | | | | 807 | 808 | 809 | | 810 | 811 | 812 | |
| 82 | hsa-miR-100 | 818 | 879 | | 820 | 821 | 822 | 823 | | | 824 | 825 | 826 | 827 |
| | | 834 | | | 836 | | 839 | 841 | 843 | | 845 | 847 | 849 | 851 |
| 83 | hsa-miR-106a | 3462 | 835 | | 837 | 838 | 840 | 842 | 844 | | 846 | 848 | 850 | 852 |
| 84 | hsa-miR-126* | 3463 | 858 | | | | | | | | | | | |
| 85 | hsa-miR-127 | 863 | 864 | | 865 | 866 | 867 | 868 | 869 | | | | 870 | 871 |
| | | | | | | | | 877 | | | | | | |
| 86 | hsa-miR-128b | 3465 | 874 | | 875 | | 876 | 878 | | | | 879 | 880 | 881 |
| 87 | hsa-miR-129 | 3466 | 891 | | | | | 3467 | | | | | | |
| 88 | hsa-miR-133a | | | | 898 | 899 | 900 | | 901 | | 902 | 903 | 904 | |
| 89 | hsa-miR-133b | | | | | | | | | | | | | |
| | | 3469 | 914 | | | | | 918 | | | | | | |
| 90 | hsa-miR-135b | 913 | 915 | | 916 | 917 | | 919 | | | 920 | 921 | 922 | 923 |
| 91 | hsa-miR-136 | 936 | 937 | | | | | 938 | | | 939 | 940 | 941 | 942 |
| 92 | hsa-miR-142-5p | 946 | 947 | | | | | 948 | | | | | | |
| | | 955 | 956 | | | | | 957 | | | | | | |
| 93 | hsa-miR-146a | 961 | 962 | | | | | 3472 | | | | | | |
| 94 | hsa-miR-146b | | | | | | | | | | | | | |
| | | 967 | 970 | | | | | 977 | | | | | | |
| | | 3474 | 971 | | | | | 978 | | | 983 | 986 | 989 | |
| | | 968 | 972 | | | 974 | | 979 | 981 | | 984 | 987 | 990 | 992 |
| 95 | hsa-miR-181a | 969 | 973 | | | 975 | 976 | 980 | 982 | | 985 | 988 | 991 | 993 |
| 96 | hsa-miR-182* | | | | | | | | | | | | | |
| 97 | hsa-miR-183 | 1019 | 1020 | | | | 1021 | 1022 | 1023 | | 1024 | 1025 | 1026 | |
| 98 | hsa-miR-187 | 1035 | 1036 | | | | | 1037 | 1038 | | 1039 | 1040 | | 1041 |
| 99 | hsa-miR-192 | 1048 | 1049 | | | | | 1050 | | | | | | |
| 100 | hsa-miR-215 | 1057 | 1058 | | | | | 1059 | 1060 | | 1061 | | 1062 | 1063 |

**[Table 1-3]**

| **number** | **sequence human miRNA** | **orthologue sequence number** | | | | | | | | | | | | |
|---|---|---|---|---|---|---|---|---|---|---|---|---|---|---|
| | | mmu | rno | cgr | age | lla | sla | mml | mne | pbi | ggo | ppa | ptr | ppy |
| | | 1067 | | | | | | | | | | | | |
| 101 | hsa-miR-200a* | 3477 | | | | | | | | | | | | |
| 102 | hsa-miR-216 | 3478 | 1069 | | | | | 1070 | | | 1071 | 1072 | | 1073 |
| 103 | hsa-miR-217 | 1082 | 1083 | | | | | | | | 1084 | 1085 | | |
| 104 | hsa-miR-220 | | | | | | | | | | 1093 | 1094 | 1095 | |
| 105 | hsa-miR-222 | 1096 | 1097 | | 1098 | | | 1099 | | | | | | |
| 106 | hsa-miR-223 | 1106 | 1107 | | | | 1108 | 1109 | | | 1110 | 1111 | 1112 | 1113 |
| 107 | hsa-miR-224 | 1120 | 1121 | | | | | 1122 | 1123 | | 1124 | 1125 | 1126 | 1127 |
| 108 | hsa-miR-296 | 3480 | 3481 | | | | | 3482 | | | | | | |
| | | 1130 | | | | | | | | | | | | |
| 109 | hsa-miR-302b* | 3483 | | | | | | | | | | | | |
| 110 | hsa-miR-302c* | | | | | | | | | | | | | |
| 111 | hsa-miR-362 | 3484 | | | | | | 3485 | | | | | | |
| 112 | hsa-miR-373* | | | | | | | | | | | | | |
| | | | | | | | | 1135 | | | | | | |
| 113 | hsa-miR-374 | 1133 | 1134 | | | | | 1136 | | | | | | |
| | | | | | | | | 1140 | | | | | | |
| 114 | hsa-miR-376a | 3487 | 1139 | | | | | 1141 | | | | | | |
| 115 | hsa-miR-378 | 3488 | 3489 | | | | | | | | | | | |
| 116 | hsa-miR-409-3p | | | | | | | | | | | | | |
| 117 | hsa-miR-409-5p | 1143 | 1144 | | | | | 1145 | | | | | | |
| 118 | hsa-miR-429 | 1146 | 1147 | | | | | 1148 | | | | | | |
| | | 3490 | 1157 | | | | | | | | | | | |
| 119 | hsa-miR-200c | 1156 | 1158 | | | | | | | | | | | |
| 120 | hsa-miR-432* | | | | | | | | | | | | | |
| 121 | hsa-miR-448 | 1170 | 1171 | | | | | 1172 | | | | | | |
| 122 | hsa-miR-450 | | 3491 | | | | | | | | | | | |
| 123 | hsa-miR-451 | 1175 | 1176 | | | | | 1177 | | | | | | |
| 124 | hsa-miR-452 | 1182 | | | | | | | | | | | | |
| 125 | hsa-miR-487b | 1183 | 1184 | | | | | 1185 | | | | | | |
| 126 | hsa-miR-489 | | | | | | | | | | | | | |
| 127 | hsa-miR-514 | | | | 1192 | | | 1193 | | 1194 | | | 1195 | |
| 128 | hsa-miR-517c | | | | | | | 3493 | | | | | | |
| | | | | | | | | 3494 | | | | | | |
| | | | | | | | | 1197 | | | | | | |
| 129 | hsa-miR-518c | | | | | | | 1198 | | | | | | |
| 130 | hsa-miR-524* | | | | | | | | | | | | | |
| 131 | hsa-miR-542-3p | 1199 | 1200 | | | | | 1201 | | | | | | |
| 132 | hsa-miR-544 | 1202 | | | | | | 1203 | | | | | | |
| 133 | hsa-miR-545 | | | | | | | | | | | | | |
| 134 | hsa-miR-550 | | | | | | | | | | | | | |
| 135 | hsa-miR-552 | | | | | | | | | | | | | |
| 136 | hsa-miR-596 | | | | | | | | | | | | | |
| 137 | hsa-miR-601 | | | | | | | 1204 | | | | | | |
| 138 | hsa-miR-608 | | | | | | | | | | | | | |
| 139 | hsa-miR-609 | | | | | | | 1205 | | | | | | |
| 140 | hsa-miR-617 | | | | | | | | | | | | | |
| 141 | hsa-miR-627 | | | | | | | | | | | | | |
| 142 | hsa-miR-632 | | | | | | | 1206 | | | | | | |
| 143 | hsa-miR-644 | | | | | | | 1207 | | | | | | |
| 144 | hsa-miR-659 | | | | | | | | | | | | | |
| 145 | hsa-miR-769-5p | | | | | | | | | | | | | |
| 146 | hsa-miR-801 | | | | | | | | | | | | | |
| 147 | hsa-miR-365 | 1208 | 1209 | | | | | 1210 | | | | | | |
| 148 | hsa-miR-142-3p | 1219 | 1220 | | | | | 1221 | | | | | | |
| 149 | hsa-miR-200a | 1224 | 1225 | | | | | 1226 | | | | | | |
| 150 | hsa-miR-381 | 1234 | 1235 | | | | | 3498 | | | | | | |

**[Table 1-4]**

| **sequence number** | human miRNA | orthologue sequence number | | | | | | | | | | | | |
|---|---|---|---|---|---|---|---|---|---|---|---|---|---|---|
| | | mmu | rno | cgr | age | lla | sla | mml | mne | pbi | ggo | ppa | ptr | ppy |
| | | 3499 | 3502 | | | | | | | | | | | |
| | | 3500 | 3503 | | | | | 3508 | | | | | | |
| 3372 | hsa-miR-29a | 3501 | 3504 | | 3505 | 3506 | 3507 | 3509 | 3510 | | 3511 | 3512 | 3513 | 3514 |
| 3373a | hsa-miR-29b | | | | | | | | | | | | | |
| | | 3537 | 3542 | | | | | 3550 | | | | | | |
| | | 3538 | 3543 | | | | | 3551 | | | | | 3564 | |
| | | 3539 | 3544 | | | | | 3552 | 3555 | | 3558 | 3561 | 3565 | |
| | | 3540 | 3545 | | | 3548 | | 3553 | 3556 | | 3559 | 3562 | 3566 | |
| 3374 | hsa-miR-30a-5p | 3541 | 3546 | | 3547 | 3549 | | 3554 | 3557 | | 3560 | 3563 | 3567 | 3568 |
| 3375 | hsa-miR-30b | | | | | | | | | | | | | |
| 3376 | hsa-miR-105 | | | | | 3601 | 3602 | 3603 | 3604 | | 3605 | 3606 | 3607 | 3608 |
| 3377 | hsa-miR-124a | | | | 3611 | 3612 | | 3613 | | | 3614 | 3615 | 3616 | 3617 |
| | | | | | | | | 877 | | | | | | |
| 3378 | hsa-miR-128a | 3465 | 874 | | 875 | | 876 | 878 | | | | 879 | 880 | 881 |
| 3379 | hsa-miR-150 | 3624 | 3625 | | | | | 3626 | | | | | | |
| 3380 | hsa-miR-154 | 3631 | 3632 | | | | | 3633 | 3634 | | 3635 | 3636 | 3637 | 3638 |
| 3381 | hsa-miR-299-3p | 3639 | | | | | | 3640 | | | | | | |
| 3382 | hsa-miR-380-5p | | | | | | | | | | | | | |
| 3383 | hsa-miR-383 | 3642 | 3643 | | | | | 3644 | | | | | | |
| 3384 | hsa-miR-411 | 3649 | 3650 | | | | | 3651 | | | | | | |
| 3385 | hsa-miR-423 | 3652 | 3653 | | | | | 3654 | | | | | | |
| 3386 | hsa-miR-433 | 3655 | 3656 | | | | | 3657 | | | | | | |
| 3387 | hsa-miR-454-3p, | | | | | | | 3659 | | | | | | |
| 3388 | hsa-miR-501 | 3662 | 3663 | | | | | 3664 | | | | | | |
| 3389 | hsa-miR-504 | 3665 | | | | | | 3666 | | | | | | |
| 3390 | hsa-miR-506 | | | | | | | 3667 | | 3668 | | | 3669 | |
| 3391 | hsa-miR-507 | | | | | | | 3671 | | 3672 | | | 3673 | |
| 3392 | hsa-miR-508 | | | | | | | | | | | | 3675 | |
| 3393 | hsa-miR-511 | | | | | | | | | | | | | |
| 3394 | hsa-miR-512-3p | | | | | | | 3677 | | | | | | |
| 3395 | hsa-miR-527 | | | | | | | | | | | | | |
| 3396 | hsa-miR-562 | | | | | | | | 3678 | | | | | |
| 3397 | hsa-miR-567 | | | | | | | | | | | | | |
| 3398 | hsa-miR-589 | | | | | | | | | | | | | |
| 3399 | hsa-miR-597 | | | | | | | 3679 | | | | | | |
| 3400 | hsa-miR-605 | | | | | | | 3680 | | | | | | |
| 3401 | hsa-miR-619 | | | | | | | | | | | | | |
| 3402 | hsa-miR-629 | | | | | | | | | | | | | |
| 3403 | hsa-miR-640 | | | | | | | 3681 | | | | | | |
| 3404 | hsa-miR-767-5p | | | | | | | 3682 | | | | | | |
| 3405 | hsa-miR-770-5p | | | | | | | 3683 | | | | | | |
| 3406 | hsa-miR-802 | | | | | | | 3684 | | | | | | |

**[Table 1-5]**

| sequence number | human miRNA | orthologue sequence number | | | | | | | | | | | | |
|---|---|---|---|---|---|---|---|---|---|---|---|---|---|---|
| | | ssy | lca | cfa | mdo | gga | xla | xtr | bta | oar | ssc | dre | fru | tni |
| | | | | | | | | 166 | | | | 169 | | |
| | | | | | | | | 167 | | | | 170 | | |
| 1 | hsa-miR-16 | | 162 | 163 | 164 | 165 | | 3407 | 168 | | | 171 | 172 | 173 |
| 2 | hsa-miR-195 | | | 179 | | | | | 180 | | | | | |
| 3 | hsa-miR-17-3p | | 191 | 3409 | 192 | 193 | | 194 | 195 | | | 3410 | | |
| | | | | | | | | | | | | 213 | | |
| | | | | | | 209 | | 210 | 211 | | | 222 | | |
| 4 | hsa-miR-18a | | 207 | | 208 | 219 | 3411 | 220 | 221 | | 212 | 214 | 215 | 216 |
| 5 | hsa-miR-18b | | | | | | | | | | | | | |
| | | | | | | 238 | | 241 | 243 | | | 246 | | |
| 6 | hsa-miR-20b | | 235 | 236 | 237 | 239 | 240 | 242 | 244 | | 245 | 247 | 248 | 249 |
| 7 | hsa-miR-21 | | | 260 | 261 | 262 | | | 263 | | 264 | 265 | 266 | 267 |
| | | | | | | | | 3414 | | | | | | |
| 8 | hsa-miR-24 | | | 276 | 277 | 278 | | 279 | 280 | | 281 | 282 | 283 | 284 |
| 9 | hsa-miR-25 | | | 294 | 295 | | | 296 | 297 | | | 298 | 299 | 300 |
| 10 | hsa-miR-32 | | | 310 | 311 | 312 | | | | | 313 | | | |
| | | | | 325 | | | | | 329 | | | 332 | | |
| 11 | hsa-miR-26b | | | 326 | | 327 | | 328 | 330 | | 331 | 333 | 334 | 335 |
| 12 | hsa-miR-30a-3p | | | 343 | | 344 | | 345 | | | | 346 | | |
| | | | | | | | | | 367 | | | | | |
| | | | | 361 | | 364 | | | 368 | | | 370 | | |
| 13 | hsa-miR-34a | | | 362 | 363 | 365 | | 366 | 369 | | | 371 | | |
| 14 | hsa-miR-449 | | | 376 | 377 | 378 | | 379 | | | | | | |
| 15 | hsa-miR-449b | | | | | | | | | | | | | |
| 16 | hsa-miR-107 | | | 391 | 392 | 393 | | 394 | 395 | | 396 | 397 | 398 | 399 |
| 17 | hsa-miR-140 | | | | | 401 | | 402 | | | 403 | | | |
| | | | | 407 | | | | 410 | 412 | | | | | |
| 18 | hsa-miR-148b | | | 408 | | 409 | | 411 | 413 | | 414 | 415 | 416 | 417 |
| | | | | | | | | | | | | 427 | | |
| 19 | hsa-miR-190 | | | | | 426 | | | | | | 428 | 429 | 430 |
| | | | | | | | | 439 | 3428 | | | | | |
| 20 | hsa-miR-199a | | | | 443 | 3427 | | 444 | 445 | | | 440 | 441 | 442 |
| 21 | hsa-miR-199b | | | | | | | | | | | | | |
| 22 | hsa-miR-202* | | | | | 446 | | | 447 | | | | | |
| 23 | hsa-miR-208 | | | | 452 | | | 453 | | | | | | |
| 24 | hsa-miR-210 | | | | | | | 457 | | | | 458 | 459 | 460 |
| 25 | hsa-miR-211 | | | | | 466 | | | | | | | | |
| 26 | hsa-miR-214 | | | | 477 | | | 478 | 479 | | 480 | 481 | 482 | 483 |
| | | | | | | | | | | | | 3430 | 502 | 504 |
| 27 | hsa-miR-218 | | 495 | 496 | 497 | 498 | | 499 | 500 | | | 501 | 503 | 505 |
| 28 | hsa-miR-299-5p | | | | | | | | | | | | | |
| 29 | hsa-miR-325 | | | | | | | | | | 508 | | | |
| | 30 hsa-miR-328 | | | 511 | | | | | | | | | | |
| 31 | hsa-miR-329 | | | | | | | | | | | | | |
| 32 | hsa-miR-338 | | | 516 | 517 | | | 518 | | | | 519 | 520 | 521 |
| 33 | hsa-miR-345 | | | | | | | | | | | | | |
| 34 | hsa-miR-425-5p | | | 3440 | | | | 522 | | | | | | |
| 35 | hsa-miR-484 | | | | | | | | | 526 | | | | |
| 36 | hsa-miR-485-5p | | | 3443 | | | | | | | | | | |
| 37 | hsa-miR-486 | | | | | | | | | | | | | |
| 38 | hsa-miR-488 | | | | | | | | | | | | | |
| 39 | hsa-miR-510 | | | | | | | | | | | | | |
| 40 | hsa-miR-515-3p | | | | | | | | | | | | | |
| 41 | hsa-miR-515-5p | | | | | | | | | | | | | |
| 42 | hsa-miR-517* | | | | | | | | | | | | | |
| 43 | hsa-miR-520d | | | | | | | | | | | | | |
| 44 | hsa-miR-520f | | | | | | | | | | | | | |
| 45 | hsa-miR-520h | | | | | | | | | | | | | |
| 46 | hsa-miR-522 | | | | | | | | | | | | | |
| 47 | hsa-miR-525* | | | | | | | | | | | | | |
| 48 | hsa-miR-573 | | | | | | | | | | | | | |
| 49 | hsa-miR-587 | | | | | | | | | | | | | |
| 50 | hsa-miR-593 | | | | | | | | | | | | | |

**[Table 1-6]**

| sequence number | human miRNA | orthologue sequence number | | | | | | | | | | | | |
|---|---|---|---|---|---|---|---|---|---|---|---|---|---|---|
| | | ssy | lca | cfa | mdo | gga | xla | xtr | bta | oar | ssc | dre | fru | tni |
| 51 | hsa-miR-595 | | | | | | | | | | | | | |
| 52 | hsa-miR-604 | | | | | | | | | | | | | |
| 53 | hsa-miR-612 | | | | | | | | | | | | | |
| 54 | hsa-miR-625 | | | | | | | | | | | | | |
| 55 | hsa-miR-634 | | | | | | | | | | | | | |
| 56 | hsa-miR-635 | | | | | | | | | | | | | |
| 57 | hsa-miR-637 | | | | | | | | | | | | | |
| 58 | hsa-miR-647 | | | | | | | | | | | | | |
| 59 | hsa-miR-650 | | | | | | | | | | | | | |
| 60 | hsa-miR-654 | | | | | | | | | | | | | |
| 61 | hsa-miR-658 | | | | | | | | | | | | | |
| 62 | hsa-miR-660 | | | 539 | | | | | 540 | | | | | |
| 63 | hsa-miR-668 | | | | | | | | | | | | | |
| 64 | hsa-miR-675 | | | | | | | | | | | | | |
| 65 | hsa-miR-765 | | | | | | | | | | | | | |
| 66 | hsa-miR-768 | | | | | | | | | | | | | |
| | | | | | | | | | | | | 3452 | | |
| 67 | hsa-miR-194 | | | 553 | | 554 | | 555 | | | | 556 | 557 | 558 |
| 68 | hsa-miR-500 | | | 559 | | | | | | | | | | |
| 69 | hsa-miR-197 | | | 567 | | | | | | | | | | |
| 70 | hsa-miR-221 | | | 574 | 575 | 576 | | 577 | 578 | | | 579 | 580 | 581 |
| | | | | | | | | | | | | 643 | | |
| | | | | | | 616 | | | | | | 644 | | |
| | | | | | | 617 | | | 632 | | | 645 | 653 | 661 |
| | | | | | | 618 | | 625 | 633 | | | 646 | 654 | 662 |
| | | | | 604 | 610 | 619 | | 626 | 634 | | | 647 | 655 | 663 |
| | | | | 605 | 611 | 620 | | 627 | 635 | | | 648 | 856 | 664 |
| | | | | 606 | 612 | 621 | | 628 | 636 | | | 649 | 657 | 665 |
| | | | | 607 | 613 | 622 | | 629 | 637 | | 640 | 650 | 658 | 666 |
| | | | | 608 | 614 | 623 | | 630 | 638 | | 641 | 651 | 659 | 667 |
| 71 | hsa-let-7e | | | 609 | 615 | 624 | | 631 | 639 | | 642 | 652 | 660 | 668 |
| | | | | | | 675 | | 3454 | | | | | | |
| 72 | hsa-miR-1 | | | 673 | 674 | 676 | | 677 | | | | 678 | 679 | 680 |
| 73 | hsa-miR-206 | | | 686 | 687 | 688 | | 689 | | | | 690 | | |
| 74 | hsa-miR-613 | | | | | | | | | | | | | |
| | | | | | | | | 702 | | | | | | |
| | | | | | | | | 3455 | | | 3456 | | | |
| 75 | hsa-miR-9 | | | 699 | 700 | 701 | | 703 | | | 3457 | 704 | 705 | 706 |
| | | | | 723 | 725 | | | 728 | 730 | | | 733 | 735 | 737 |
| 76 | hsa-miR-23b | | 722 | 724 | 726 | 727 | | 729 | 731 | | 732 | 734 | 736 | 738 |
| | | | | | | | | | | | | 763 | | |
| | | | | | | | | | | | | 764 | | |
| | | | | | | | | 757 | | | | 765 | 768 | 771 |
| | | | | 752 | 754 | | | 758 | 760 | | | 786 | 769 | 772 |
| 77 | hsa-miR-27b | | 751 | 753 | 755 | 756 | | 759 | 761 | | 762 | 767 | 770 | 773 |
| 78 | hsa-miR-28 | | | | | | | | | | 785 | | | |
| 79 | hsa-miR-31 | | | | | | | 3460 | | | | 792 | | |
| | | | | | | | | 803 | | | | | | |
| 80 | hsa-miR-33b | | | 801 | | 802 | | 804 | | | | | | |
| 81 | hsa-miR-96 | | | | 813 | | | 814 | | | | 815 | 816 | 817 |
| 82 | hsa-miR-100 | | | | 828 | 829 | | 830 | | | | 831 | 832 | 833 |
| 83 | hsa-miR-106a | | | 853 | | 854 | | 855 | 856 | | 857 | | | |
| 84 | hsa-miR-126≠ | | | 3464 | | 859 | | 860 | 861 | | | 862 | | |
| 85 | hsa-miR-127 | | | 872 | | | | | 873 | | | | | |
| 86 | hsa-miR-128b | | | 882 | 883 | 884 | | 885 | 886 | | 887 | 888 | 889 | 890 |
| 87 | hsa-miR-129 | | | 892 | 893 | | | 894 | | | | 895 | 896 | 897 |
| | | | | | | | | 909 | | | | | | |
| | | | | | | 906 | | 912 | | | | | | |
| | | | | | | 911 | | 3468 | | | | | | |
| 88 | hsa-miR-133a | | | | 905 | 907 | 908 | 910 | | | | | | |
| 89 | hsa-miR-133b | | | | | | | | | | | | | |
| | | | | | | | | | | | | 929 | | |
| | | | | | 924 | | | | | | | 930 | 932 | 934 |
| 90 | hsa-miR-135b | | | | 925 | 926 | | 927 | | | 928 | 931 | 933 | 935 |
| 91 | hsa-miR-136 | | | 943 | | | | | | 944 | 945 | | | |
| | | | | | | | | | | | | 3471 | 951 | 953 |
| 92 | hsa-miR-142-5p | | | | | | | 949 | 3470 | | | 950 | 952 | 954 |
| | | | | 958 | | 959 | | 3473 | | | | 960 | | |
| 93 | hsa-miR-146a | | | 963 | | 964 | | 965 | | | | 966 | | |
| 94 | hsa-miR-146b | | | | | | | | | | | | | |
| | | | | 994 | | | | | | | | | | |
| | | | | 995 | 998 | | | | 1005 | | | 1010 | | |
| | | | | 996 | 999 | 1001 | | 1003 | 1006 | | 1008 | 1011 | 1013 | 1015 |
| 95 | hsa-miR-181a | | | 997 | 1000 | 1002 | | 1004 | 1007 | | 1009 | 1012 | 1014 | 1016 |
| 96 | hsa-miR-182* | | | | | | | | 1017 | | | 1018 | 3475 | 3476 |
| 97 | hsa-miR-183 | | | 1027 | 1028 | 1029 | | 1030 | | | 1031 | 1032 | 1033 | 1034 |
| 98 | hsa-miR-187 | | | | 1042 | 1043 | | 1044 | | | | 1045 | 1046 | 1047 |
| 99 | hsa-miR-192 | | | 1051 | | | | 1052 | 1053 | | | 1054 | 1055 | 1056 |
| 100 | hsa-miR-215 | | | | | 1064 | | 1065 | 1066 | | | | | |

**[Table 1-7]**

| sequence number | human miRNA | orthologue sequence number | | | | | | | | | | | | |
|---|---|---|---|---|---|---|---|---|---|---|---|---|---|---|
| | | ssy | lca | cfa | mdo | gga | xla | xtr | bta | oar | ssc | dre | fru | tni |
| 101 | hsa-miR-200a* | | | | 1068 | | | | | | | | | |
| 102 | hsa-miR-216 | | 1074 | | 1075 | 1076 | | 1077 | | | 1078 | 1079 | 1080 | 1081 |
| 103 | hsa-miR-217 | | | | 1086 | 1087 | | 1088 | | | 1089 | 1090 | 1091 | 1092 |
| 104 | hsa-miR-220 | | | | | | | | | | | | | |
| 105 | hsa-miR-222 | | | | 3479 | 1100 | | 1101 | 1102 | | | 1103 | 1104 | 1105 |
| 106 | hsa-miR-223 | | | | 1114 | 1115 | | 1116 | | | | 1117 | 1118 | 1119 |
| 107 | hsa-miR-224 | | | 1128 | | | | | | | 1129 | | | |
| 108 | hsa-miR-296 | | | | | | | | | | | | | |
| 109 | hsa-miR-302b* | | | | | 1131 | | | | | | | | |
| 110 | hsa-miR-302c* | | | | | 1132 | | | | | | | | |
| 111 | hsa-miR-362 | | | | | | | | | | | | | |
| 112 | hsa-miR-373* | | | | | | | | | | | | | |
| | | | | 3486 | | | | | | | | | | |
| 113 | hsa-miR-374 | | | 1137 | | | | | 1138 | | | | | |
| 114 | hsa-miR-376a | | | 1142 | | | | | | | | | | |
| 115 | hsa-miR-378 | | | | | | | | | | | | | |
| 116 | hsa-miR-409-3p | | | | | | | | | | | | | |
| 117 | hsa-miR-409-5p | | | | | | | | | | | | | |
| 118 | hsa-miR-429 | | | 1149 | | 1150 | 1151 | 1152 | | | | 1153 | 1154 | 1355 |
| | | | | | 1160 | | | | 1164 | | | 1166 | | |
| 119 | hsa-miR-200c | | | 1159 | 1161 | 1162 | | 1163 | 1165 | | | 1167 | 1168 | 1169 |
| 120 | hsa-miR-432* | | | | | | | | | | | | | |
| 121 | hsa-miR-448 | | | 1173 | | | | | | | | | | |
| 122 | hsa-miR-450 | | | 1174 | | | | | | | | | | |
| 123 | hsa-miR-451 | | | | 1178 | 1179 | | 1180 | | | | 1181 | | |
| 124 | hsa-miR-452 | | | | | | | | | | | | | |
| 125 | hsa-miR-487b | | | 3492 | | | | | 1186 | | | | | |
| 126 | hsa-miR-489 | | | | | 1187 | | 1188 | | | | 1189 | 1190 | 1191 |
| 127 | hsa-miR-514 | 1196 | | | | | | | | | | | | |
| 128 | hsa-miR-517c | | | | | | | | | | | | | |
| 129 | hsa-miR-518c | | | | | | | | | | | | | |
| 130 | hsa-miR-524* | | | | | | | | | | | | | |
| 131 | hsa-miR-542-3p | | | 3495 | | | | | | | | | | |
| 132 | hsa-miR-544 | | | | | | | | | | | | | |
| 133 | hsa-miR-545 | | | | | | | | | | | | | |
| 134 | hsa-miR-550 | | | | | | | | | | | | | |
| 135 | hsa-miR-552 | | | | | | | | | | | | | |
| 136 | hsa-miR-596 | | | | | | | | | | | | | |
| 137 | hsa-miR-601 | | | | | | | | | | | | | |
| 138 | hsa-miR-608 | | | | | | | | | | | | | |
| 139 | hsa-miR-609 | | | | | | | | | | | | | |
| 140 | hsa-miR-617 | | | | | | | | | | | | | |
| 141 | hsa-miR-627 | | | | | | | | | | | | | |
| 142 | hsa-miR-632 | | | | | | | | | | | | | |
| 143 | hsa-miR-644 | | | | | | | | | | | | | |
| 144 | hsa-miR-659 | | | | | | | | | | | | | |
| 145 | hsa-miR-769-5p | | | | | | | | | | | | | |
| 146 | hsa-miR-801 | | | | | | | | | | | | | |
| 147 | hsa-miR-365 | | | 1211 | 1212 | 1213 | | 1214 | 1215 | | | 1216 | 1217 | 1218 |
| 148 | hsa-miR-142-3p | | | | 3496 | 1222 | | 1223 | | | | 3497 | | |
| 149 | hsa-miR-200a | | | | 1227 | 1228 | | 1229 | 1230 | | | 1231 | 1232 | 1233 |
| 150 | hsa-miR-361 | | | 1236 | | | | | 1237 | | | | | |

**[Table 1-8]**

| sequence number | human miRNA | orthologue sequence number | | | | | | | | | | | | |
|---|---|---|---|---|---|---|---|---|---|---|---|---|---|---|
| | | ssy | lca | cfa | mdo | gga | xla | xtr | bta | oar | ssc | dre | fru | tni |
| | | | | | | | | 3523 | | | | | | |
| | | | | 3515 | | 3520 | | 3524 | | | | | | |
| | | | | 3516 | 3518 | 3521 | | 3525 | 3527 | | 3529 | 3531 | 3533 | 3535 |
| 3372 | hsa-miR-29a | | | 3517 | 3519 | 3522 | | 3526 | 3528 | | 3530 | 3532 | 3534 | 3536 |
| 3373 | hsa-miR-29b | | | | | | | | | | | | | |
| | | | | | | 3574 | | 3579 | 3584 | | | 3590 | | |
| | | | | 3569 | | 3575 | | 3580 | 3585 | | | 3591 | | |
| | | | | 3570 | | 3576 | | 3581 | 3586 | | | 3592 | 3595 | 3598 |
| | | | | 3571 | | 3577 | | 3582 | 3587 | | | 3593 | 3596 | 3599 |
| 3374 | hsa-miR-30a-5p | | | 3572 | 3573 | 3578 | | 3583 | 3588 | | 3589 | 3594 | 3597 | 3600 |
| 3375 | hsa-miR-30b | | | | | | | | | | | | | |
| | | | | | | | | | | | 3609 | | | |
| 3376 | hsa-miR-105 | | | | | | | | | | 3610 | | | |
| | | | | | | 3619 | | | | | | | | |
| 3377 | hsa-miR-124a | | | | 3618 | 3620 | | 3621 | 3622 | | 3623 | | | |
| 3378 | hsa-miR-128a | | | 882 | 883 | 884 | | 885 | 886 | | 887 | 888 | 889 | 890 |
| 3379 | hsa-miR-150 | | | 3627 | | | | 3628 | 3629 | | | 3630 | | |
| 3380 | hsa-miR-154 | | | | | | | | | | | | | |
| 3381 | hsa-miR-299-3p | | | | | | | | | | | | | |
| 3382 | hsa-miR-380-5p | | | | | | | | 3641 | | | | | |
| 3383 | hsa-miR-383 | | | 3645 | 3646 | 3647 | | 3648 | | | | | | |
| 3384 | hsa-miR-411 | | | | | | | | | | | | | |
| 3385 | hsa-miR-423 | | | | | | | | | | | | | |
| 3386 | hsa-miR-433 | | | 3658 | | | | | | | | | | |
| | | | | | | | | | | | | 3660 | | |
| 3387 | hsa-miR-454-3p | | | | | | | | | | | 3661 | | |
| 3388 | hsa-miR-501 | | | | | | | | | | | | | |
| 3389 | hsa-miR-504 | | | | | | | | | | | | | |
| 3390 | hse-msR-506 | 3670 | | | | | | | | | | | | |
| 3391 | hsa-miR-507 | 3574 | | | | | | | | | | | | |
| 3392 | hsa-miR-508 | 3676 | | | | | | | | | | | | |
| 3393 | hsa-miR-511 | | | | | | | | | | | | | |
| 3394 | hsa-miR-512-3p | | | | | | | | | | | | | |
| 3395 | hsa-miR-527 | | | | | | | | | | | | | |
| 3396 | hsa-miR-562 | | | | | | | | | | | | | |
| 3367 | hsa-miR-567 | | | | | | | | | | | | | |
| 3398 | hsa-miR-589 | | | | | | | | | | | | | |
| 3399 | hsa-miR-597 | | | | | | | | | | | | | |
| 3400 | hsa-miR-605 | | | | | | | | | | | | | |
| 3401 | hsa-miR-619 | | | | | | | | | | | | | |
| 3402 | hsa-miR-629 | | | | | | | | | | | | | |
| 3403 | hsa-miR-640 | | | | | | | | | | | | | |
| 3404 | hra-miR-767-5p | | | | | | | | | | | | | |
| 3405 | hsa-miR-770-5p | | | | | | | | | | | | | |
| 3406 | hsa-miR-802 | | | | | | | | | | | | | |

Regarding the mechanism in which a micro-RNA suppresses the translation of the mRNA of a target gene therefor, it is known that an mRNA having a nucleotide sequence complementary to the 2nd to 8th nucleotides from the 5'-end of the micro-RNA is recognized as a micro-RNA target gene (Current Biology, 15, R458-R460 (2005)). By this mechanism, the expression of the mRNA is suppressed by the micro-RNA. Therefore, micro-RNAs having the same nucleotide sequence at the 2nd to 8th nucleotides from the 5'-end suppress the expression of the mRNA of the same target gene to exhibit the same function. As micro-RNAs having the same nucleotide sequence at the 2nd to 8th nucleotides from the 5'-end as the nucleotide sequence of a micro-RNA consisting of a nucleotide sequence of any one of SEQ ID NOs:1 to 150 and 3372 to 3406, nucleic acids consisting of a nucleotide sequence of any one of SEQ ID NOs:1238 to 1543 and 3685 to 3741 can be mentioned. As specific examples of the micro-RNAs, for the micro-RNA of SEQ ID ND:1, micro-RNAs consisting of a nucleotide sequence of any one of SEQ ID NOs:1238 to 1286 can be mentioned. Tables of the correspondence of micro-RNAs having the same nucleotide sequence at the 2nd to 8th nucleotides from the 5'-end as the nucleotide sequence of a micro-RNA consisting of a nucleotide sequence of any one of SEQ ID NOs:1 to 150 and 3372 to 3406 are shown in Table 2-1 to Table 2-8. Because micro-RNAs having a common seed sequence are considered to share the same target nucleotide sequence, they are considered to possess similar functions.

**[Table 2-1]**

| sequence number | human miRNA | sequence of 2nd to 8th on 6'terminal side | sequence number of mRNA having sequence with the same 2nd to 8th nucleotides on 5'terminnal side | | | | | | | | | | | | |
|---|---|---|---|---|---|---|---|---|---|---|---|---|---|---|---|
| | | | hsa | mmu | rno | cgr | age | lla | sla | mml | mne | pbi | ggo | ppa | ptr |
| | | | 1238 | 1242 | | | | | | 1254 | | | | | |
| | | | 1239 | 1243 | 1246 | | | | | 1255 | | | | | |
| 1 | hsa-miR-16 | | 1240 | 1244 | 1247 | | 1249 | 1251 | | 1256 | 1258 | | 1260 | 1262 | 1264 |
| 2 | hsa-miR-195 | AGCAGCA | 1241 | 1245 | 1248 | | 1250 | | 1252 1253 1257 | | 1259 | | 1261 | 1263 | 1265 |
| 3 | hsa-miR-17-3p | CUGCAGU | | 1287 | 1288 | | | | | | | | | | |
| 4 | hsa-miR-18a | | | | | | | | | | | | | | |
| 5 | hsa-miR-18b | AAGGUGC | | | | | | | | | | | | | |
| 6 | hsa-miR-20b | AAAGUGC | | | | | | | | | | | | | |
| 7 | hsa-miR-21 | AGCUUAU | 1290 | 1291 | | | | | | 1292 | | | | | |
| 8 | hsa-miR-24 | GGCUCAG | | | | | | | | | | | | | |
| | | | 1293 | 1297 | | | | | | 1307 | | | | | |
| | | | 1294 | 1288 | 1301 | | | | | 1308 | | | | | |
| 9 | hsa-miR-25 | | 1295 | 1299 | 1302 | | | | | 1309 | | | | | |
| 10 | hsa-miR-32 | AUUGCAC | 1296 | 1300 | 1303 | | 1304 | 1305 | 1306 | 1310 | 1311 | | 1312 | 1313 | 1314 |
| | | | 1333 | | | | | | | | | | | | |
| 11 | hsa-miR-26b | UCAAGUA | 1334 | | | | | | | | | | | | |
| | | | 1335 | | | | | | | | | | | | |
| 12 | hsa-miR-30a-3p | UUUCAGU | 1336 | | | | | | | | | | | | |
| 13 | hsa-miR-34a | | | | | | | | | | | | | | |
| 14 | hsa-miR-449 | | | | | | | | | | | | | | |
| 15 | hsa-miR-449b | GGCAGUG | 1337 | 1338 | | | | | | | | | | | |
| 16 | hsa-miR-107 | OCAGGAU | 1339 | 1340 | 1341 | | 1342 | 1343 | | 1344 | 1345 | | 1346 | 1347 | 1348 |
| 17 | hsa-miR-140 | GUGGUUU | | | | | | | | | | | | | |
| | | | 1359 | | | | | | | | | | | | |
| 18 | hsa-miR-148b | CAGUGCA | 1360 | 1361 | 1362 | | | | | 1363 | | | | | |
| 19 | hsa-miR-190 | GAUAUGU | 1369 | | | | | | | | | | | | |
| 20 | hsa-miR-199a | | | | | | | | | | | | | | |
| 21 | hsa-miR-199b | CCAGUGU | | | | | | | | | | | | | |
| 22 | hsa-miR-202* | UUCCUAU | | | | | | | | | | | | | |
| 23 | hsir-miR-208 | UAAGACG | 1370 | | | | | | | | | | | | |
| 24 | hsa-miR-210 | UGUGCGU | | | | | | | | | | | | | |
| 25 | hsa-miR-211 | UCCCUUU | 1372 | 1373 | 1374 | | | | 1375 | 1376 | 1377 | | 1378 | 1379 | 1380 |
| 26 | hsa-miR-214 | CAGCAGG | | 1392 | | | | | | | | | | | |
| 27 | hsa-miR-218 | UGUGCUU | | | | | | | | | | | | | |
| 28 | hsa-miR-299-5p | GGUUUAC | | | | | | | | | | | | | |
| 29 | hsa-miR-325 | CUAGUAG | | | | | | | | | | | | | |
| 30 | hsa-miR-328 | UGGCCCU | | | | | | | | | | | | | |
| 31 | hsa-miR-329 | AGACACC | 1393 | 1394 | | | | | | 1395 | | | | | |
| 32 | hsa-miR-338 | CCAGCAU | | | | | | | | | | | | | |
| 33 | hss-miR-395 | GCUGACU | | | | | | | | | | | | | |
| 34 | hsa-miR-425-5p | AUGACAC | | 1396 | | | | | | | | | | | |
| | 38 hsa-miR-484 | CAGGCUC | | | 1398 | | | | | | | | | | |
| 36 | hsc-miR-4855p | GAGGCUG | | | | | | | | | | | | | |
| 37 | hsa-miR-486 | CCUGUAC | | | | | | | | | | | | | |
| 38 | hsa-miR-488 | CCAGAUA | | | | | | | | | | | | | |
| 39 | hsa-miR-510 | ACUCAGG | | | | | | | | | | | | | |
| | | | 1399 | | | | | | | | | | | | |
| 40 | hse-miR-515-3p | AGUGCCU | 1400 | | | | | | | | | | | | |
| 41 | hsa-miR-615-5p | UCUCCAA | 1401 | | | | | | | | | | | | |
| 42 | hse-miR-517* | CUCUAGA | | | | | | | | | | | | | |
| | | | 1402 | | | | | | | | | | | | |
| | | | 1403 | | | | | | | | | | | | |
| | | | 1404 | | | | | | | | | | | | |
| | | | 1405 | | | | | | | 1423 | | | | | |
| | | | 1406 | | | | | | | 1424 | | | | | |
| | | | 1407 | 1413 | | | | | | 1425 | | | | | |
| | | | 1408 | 1414 | | | | | | 1426 | | | | | |
| | | | 1409 | 1415 | | | | | | 1427 | | | | | |
| | | | 1410 | 1416 | | | | | | 1428 | | | | | |
| | | | 1411 | 1417 | | | | | | 1429 | | | | | |
| 43 | hsa-miR-520d | AAGUGCU | 1412 | 1418 | 1419 | | 1420 | 1421 | 1422 | 1430 | 1431 | | 1432 | 1433 | 1434 |
| 44 | hsa-miR-520 | AGUGCUU | | 1455 | | | | | | | | | | | |
| 45 | hsa-miR-520h | CAAAGUG | 1457 | | | | | | | | | | | | |
| 46 | hsa-miR-522 | AAAUGGU | | | | | | | | 1458 | | | | | |
| 47 | hsa-miR-525* | AAGGCGC | 1459 | | | | | | | | | | | | |
| 48 | hsa-miR-573 | UGAAGUG | | | | | | | | | | | | | |
| 49 | hsa-miR-587 | UUCCAUA | | | | | | | | | | | | | |
| 50 | hsa-miR-593 | GGCACCA | | | | | | | | | | | | | |

**[Table 2-2]**

| sequence number | human miRNA | sequence of 2nd to miRNA 8th on 5'terminal side | sequence number of mRNA having sequence with the same 2nd to 8th nucleotides on 5' terminal side | | | | | | | | | | | |
|---|---|---|---|---|---|---|---|---|---|---|---|---|---|---|
| | | | hsa | mmu | mo | cgr | age | lia | sla mml | mne | pbi | ggo | ppa | ptr |
| 51 | hsa-miR-595 | AAGUGUG | | | | | | | | | | | | |
| 52 | hsa-miR-604 | GGOUGOG | | | | | | | | | | | | |
| 53 | hsa-miR-612 | CUGGGCA | 1460 | | | | | | | | | | | |
| 54 | hsa-miR-625 | GGGGGAA | | | | | | | | | | | | |
| 55 | hsa-miR-634 | ACCAGCA | | | | | | | | | | | | |
| 56 | hsa-miR-635 | CUUGGGC | | | | | | | | | | | | |
| 57 | hsa-miR-637 | CUOGGGG | | | | | | | | | | | | |
| 58 | hsa-miR-647 | UGGCUGC | | | | | | | | | | | | |
| 59 | hsa-miR-650 | GGAGGCA | | | | | | | | | | | | |
| 60 | hsa-miR-654 | GGUGGGC | 1461 | | | | | | | | | | | |
| 61 | hsa-miR-658 | GCGGAGG | | | | | | | | | | | | |
| 62 | hsa-miR-660 | ACCCAUU | | | | | | | | | | | | |
| 63 | hsa-miR-668 | GUCACUC | | | | | | | | | | | | |
| 64 | hsa-miR-675 | GGUGCGG | | | | | | | | | | | | |
| 65 | hsa-miR-765 | GGAGGAG | | | | | | | | | | | | |
| 66 | hsa-miR-766 | CUCGAGC | | | | | | | | | | | | |
| 67 | hsa-miR-194 | GUAACAG | | | | | | | | | | | | |
| 68 | hsa-miR-500 | UGCACCU | | | | | | | | | | | | |
| 69 | hsa-miR-197 | UCACCAC | | | | | | | | | | | | |
| 70 | hsa-miR-221 | GCUACAU | | | | | | | | | | | | |
| | | | 1462 | | | | | | | | | | | |
| | | | 1463 | | | | | | | | | | | |
| | | | 1464 | | | | | | | | | | | |
| | | | 1465 | | | | | | | | | | | |
| | | | 1466 | | | | | | | | | | | |
| | | | 1467 | | | | | | | | | | | |
| | | | 1468 | | | | | | | | | | | |
| 71 | hsa-et-7e | GAGGUAG | 1469 | 1470 | 1471 | | 1472 | | 1473 | | | 1474 | 1475 | 1476 |
| 72 | hsa-miR-1 | | | | | | | | | | | | | |
| 73 | hsa-miR-206 | | | | | | | | | | | | | |
| 74 | hsa-miR-613 | GGAAUGU | | | | | | | | | | | | |
| 75 | hsa-miR-9 | CUUUGGU | | | | | | | | | | | | |
| | | | 1481 | | | | | | | | | | | |
| 76 | hsa-miR-23b | UCACAUU | 1482 | | | | | | | | | | | |
| 77 | hsa-miR-27b | UCACAGU | 1483 | | | | | | | | | | | |
| 78 | hsa-miR-28 | AGGAGCU | 1484 | 1485 | 1486 | | | | | | | | | |
| 79 | hsa-miR-31 | GCAAGAU | | | | | | | | | | | | |
| 80 | hsa-miR-33b | UGCAUUG | 1488 | | | | | | | | | | | |
| 81 | hsa-miR-96 | UUGGGAC | 1489 | | | | 1490 | | | | | | | |
| | | | 1492 | 1494 | 1496 | | | | 1499 | | | | | |
| 82 | hsa-miR-100 | ACCGGUA | 1493 | 1495 | 1497 | | | 1498 | 1500 | 1501 | | 1502 | 1503 | 1504 |
| 83 | hsa-miR-106a | AAAGUGC | | | | | | | | | | | | |
| 84 | hsa-miR-126* | AUUAUUA | | | | | | | | | | | | |
| 85 | hsa-miR-127 | COGAUCC | | | | | | | | | | | | |
| 86 | hsa-miR-128b | CACAGUG | 1514 | | | | | | | | | | | |
| 87 | hsa-miR-129 | UUUUUGC | | | | | | | | | | | | |
| 88 | hsa-miR-133a | | | | | | | | | | | | | |
| 89 | hsa-miR-133b | UGGUCCC | | | | | | | | | | | | |
| 90 | hsa-miR-135b | AUGGCUU | 1516 | | | | | | | | | | | |
| 91 | hsa-miR-136 | CUCCAUU | | | | | | | | | | | | |
| 92 | hsa-miR-142-5p | AUAAACU | | | | | | | | | | | | |
| 93 | hsa-miR-146a | | | | | | | | | | | | | |
| 94 | hsa-miR-146b | GAGAACU | | | | | | | | | | | | |
| | | | 1517 | | | | | | | | | | | |
| | | | 1518 | | | | | | | | | | | |
| 95 | hsa-miR-181a | ACAUUGA | 1519 | | | | | | | | | | | |
| 96 | hsa-miR-182* | GGUUCUA | | | | | | | | | | | | |
| 97 | hsa-miR-183 | AUGGCAC | | | | | | | | | | | | |
| 98 | hsa-miR-187 | GGUGUGU | | | | | | | | | | | | |
| 99 | hsa-miR-192 | | | | | | | | | | | | | |
| 100 | hsa-miR-215 | UGACGUA | | | | | | | | | | | | |

**[Table 2-3]**

| sequence number | human miRNA | sequence of 2nd to 8th on 5'terminal side | sequence number of mRNA having sequence with the some 2nd to 8th nucleotides on 5'terminal side | | | | | | | | | | | | |
|---|---|---|---|---|---|---|---|---|---|---|---|---|---|---|---|
| | | | hsa | mmu | rno | cgr | age | lla | sla | mml | mne | pbl | ggo | ppa | ptr |
| 101 | hsa-miR-200a* | AUCUUAC | 1520 | | | | | | | | | | | | |
| 102 | hsa-miR-216 | AAUCUCA | | | | | | | | | | | | | |
| 103 | hsn-miR-217 | ACUGCAU | | | | | | | | | | | | | |
| 104 | hsa-miR-220 | CACACCG | | | | | | | | | | | | | |
| 105 | hsa-miR-222 | GCUACAU | | | | | | | | | | | | | |
| 106 | hsa-miR-223 | GUCAGUU | | | | | | | | | | | | | |
| 107 | hsa-miR-224 | AAGUCAC | | | | | | | | | | | | | |
| 108 | hsa-miR-296 | GGGCCCC | | | | | | | | | | | | | |
| 109 | hsa-miR-302b* | CUUUAAC | 1521 | | | | | | | | | | | | |
| 110 | hsa-miR-302c* | UUAAGAU | | | | | | | | | | | | | |
| 111 | hsa-miR-362 | AUCCUUG | | | | | | | | | | | | | |
| 112 | hsa-miR-373* | GUGAAAA | 1522 | 1523 | | | | | | | | | | | |
| 113 | hsa-miR-374 | UAUAAUA | 1524 | | | | | | | | | | | | |
| 114 | hsa-miR-376a | UCAUAGA | 1525 | | | | | | | | | | | | |
| 115 | hsa-miR-378 | UCCUGAC | | | | | | | | | | | | | |
| 116 | hsa-miR-409 3p | GAAUGUU | | | | | | | | | | | | | |
| 117 | hsa-miR-409-5p | GGUUACC | | | | | | | | | | | | | |
| 118 | hsa-miR-429 | | | | | | | | | | | | | | |
| 119 | hsa-miR-200c | AAUAGUG | 1526 | | | | | | | 1527 | | | | | |
| 120 | hsa-miR-432* | UGGAUGG | | | | | | | | | | | | | |
| 121 | hsa-miR-448 | UGCAUAU | | | | | | | | | | | | | |
| 122 | hsa-miR-450 | UUUUGCG | | | | | | | | | | | | | |
| 123 | hsa-miR-451 | AAGGGUU | | | | | | | | | | | | | |
| 124 | hsa-miR-152 | GUUUGCA | | | | | | | | | | | | | |
| 125 | hsa-miR-487b | AUCGUAC | | | | | | | | | | | | | |
| 126 | hsa-miR-489 | GUGACAU | | | | | | | | | | | | | |
| 127 | hsa-miR-514 | UUGAGAC | | | | | | | | | | | | | |
| 128 | hsa-miR-517c | UCGUGCA | 1528 | | | | | | | | | | | | |
| | | | 1529 | | | | | | | | | | | | |
| | | | 1530 | | | | | | | | | | | | |
| | | | 1531 | | | | | | | | | | | | |
| 129 | hsa-miR-518c | AAAGCGC | 1532 | | | | | | | | | | | | |
| | | | | | | | | | | 1534 | | | | | |
| 130 | hsa-miR-524* | UACAAAG | 1533 | | | | | | | 1535 | | | | | |
| 131 | hsa-miR-542-3p | GUGAGAG | | | | | | | | | | | | | |
| 132 | hsa-miR-544 | UUCUGCA | | | | | | | | | | | | | |
| 133 | hsa-miR-545 | UGAGGAA | | | | | | | | | | | | | |
| 134 | hsa-miR-550 | GUGUUAC | 1536 | 1537 | | | | | | | | | | | |
| 135 | hsa-miR-552 | AGAGGUG | | | | | | | | | | | | | |
| 136 | hsa-miR-596 | AGGOUGC | | | | | | | | | | | | | |
| 137 | hsa-miR-601 | GGUCUAG | | | | | | | | | | | | | |
| 138 | hsa-miR-608 | GGGGUGG | | | | | | | | | | | | | |
| 139 | hsa-miR-609 | GGGUGUU | | | | | | | | | | | | | |
| 140 | hsa-miR-617 | GACUUCC | | | | | | | | | | | | | |
| 141 | hsa-miR-627 | UGAGUCU | | | | | | | | 1538 | | | | | |
| 142 | hsa-miR-632 | UGUGUGC | | | | | | | | | | | | | |
| 143 | hsa-miR-644 | GUGUGGC | | | | | | | | | | | | | |
| 144 | hsa-miR-659 | UUGGUUC | | | | | | | | | | | | | |
| 145 | hsa-miR-769-5p | GAGACCU | | | | | | | | | | | | | |
| 146 | hsa-miR-801 | AUUGCUC | | | | | | | | | | | | | |
| 147 | hsa-miR-365 | AAUGCCC | | | | | | | | | | | | | |
| 148 | hsa-miR-142-3p | GUAGUGU | | | | | | | | | | | | | |
| 149 | hsa-miR-200a | AACACUG | 1539 | 1540 | 1541 | | | | | | | | | | |
| 150 | hsa-miR-361 | UAUCAGA | | | | | | | | | | | | | |

**[Table 2-4]**

| sequence number | human miRNA | sequence of 2nd to 8th on 5'terminal side | sequence number of mRNA having sequence with the same 2nd to 8th nucleotides on 5'terminal side | | | | | | | | | | | | |
|---|---|---|---|---|---|---|---|---|---|---|---|---|---|---|---|
| | | | hsa | mmu | rno | cgr | age | lla | sla | mml | mne | pbi | ggo | ppa | ptr |
| 3372 | hsa-miR-29a | | | | | | | | | | | | | | |
| 3373 | hsa-miR-29b | AGCACCA | 3685 | | | | | | | | | | | | |
| | | | 3686 | | | | | | | | | | | | |
| 3374 | hsa-miR-30a-5p | | 3687 | | | | | | | | | | | | |
| 3375 | hsa-miR-30b | GUAAACA | 3688 | 3689 | 3690 | | | | | | | | | | |
| 3376 | hsa-miR-105 | CAAAUGC | | | | | | | | | | | | | |
| 3377 | hsa-miR-124a | UAAGGCA | | | | | | | | | | | | | |
| 3378 | hsa-miR-128a | CACAGUG | | | | | | | | | | | | | |
| 3379 | hsa-miR-150 | CUCCCAA | | | | | | | | | | | | | |
| 3380 | hsa-miR-154 | AGGUUAU | | | | | | | | | | | | | |
| 3381 | hsa-miR-299-3p | AUGUGGG | | | | | | | | | | | | | |
| 3382 | hsa-miR-380-5p | GGUUGAC | 3691 | | | | | | | | | | | | |
| 3383 | hsa-miR-383 | GAUCAGA | | | | | | | | | | | | | |
| 3384 | hsa-miR-411 | AGUAGAC | | | | | | | | | | | | | |
| 3385 | hsa-miR-423 | GCUCGGU | | | | | | | | | | | | | |
| 3386 | hsa-miR-433 | UCAUGAU | | | | | | | | | | | | | |
| | | | | 3696 | | | | | | | | | | | |
| | | | 3692 | 3697 | 3701 | | | | | 3705 | | | | | |
| | | | 3693 | 3698 | 3702 | | | | | 3706 | | | | | |
| | | | 3894 | 3699 | 3703 | | | | | 3707 | | | | | |
| 3387 | hsa-miR-454-3p | AGUGCAA | 3695 | 3700 | 3704 | | | | | 3708 | 3709 | | 3710 | 3711 | |
| 3388 | hsa-miR-501 | AUCCUUU | | | | | | | | | | | | | |
| 3389 | hsa-miR-504 | GACCCUG | | | | | | | | | | | | | |
| 3390 | hsa-miR-506 | AAGGCAC | | 3732 | 3733 | | | | | | | | | | |
| 3391 | hsa-miR-507 | UUUGCAC | 3738 | | | | | | | | | | | | |
| 3392 | hsa-miR-508 | GAUUGUA | | | | | | | | | | | | | |
| 3393 | hsa-miR-511 | UGUCUUU | | | | | | | | | | | | | |
| 3394 | hsa-miR-512-3p | AGUGCUG | | 3739 | | | | | | | | | | | |
| 3395 | hsa-miR-527 | UGCAAAG | 3741 | | | | | | | | | | | | |
| 3396 | hsa-miR-562 | AAGUAGC | | | | | | | | | | | | | |
| 3397 | hsa-miR-567 | GUAUGUU | | | | | | | | | | | | | |
| 3398 | hsa-miR-589 | CAGAACA | | | | | | | | | | | | | |
| 3399 | hsa-miR-597 | GUGUCAC | | | | | | | | | | | | | |
| 3400 | hsa-miR-605 | AAAUCCC | | | | | | | | | | | | | |
| 3401 | hsa-miR-619 | ACCUGGA | | | | | | | | | | | | | |
| 3402 | hsa-miR-629 | UUCUCCC | | | | | | | | | | | | | |
| 3403 | hsa-miR-640 | UGAUCCA | | | | | | | | | | | | | |
| 3404 | hsa-miR-767-5p | GCACCAU | | | | | | | | | | | | | |
| 3405 | hsa-miR-770-5p | CCAGUAC | | | | | | | | | | | | | |
| 3406 | hsa-miR-802 | AGUAACA | | | | | | | | | | | | | |

**[Table 2-5]**

| sequence number | human miRNA | sequence of 2nd to 8th on 5'terminal side | sequence number mTNA having sequence with the same 2nd to 8th nucleotides on 5'terminal side | | | | | | | | | | | | | |
|---|---|---|---|---|---|---|---|---|---|---|---|---|---|---|---|---|
| | | | ppy | ssy | lca | cfa | mdo | gga | xla | xtr | bta | oar | ssc | dre | fru | tni |
| | | | | | | | | | | | | | | 1281 | | |
| | | | | | | 1269 | | | | 1275 | 1278 | | | 7282 | | |
| 1 | hsa-miR-16 | | 1266 | | | 1270 | | 1273 | | 1276 | 1279 | | | 1283 | | |
| 2 | hsa-miR-195 | AGCAGCA | 1267 | | 7268 | 1271 | 1272 | 1274 | | 1277 | 1280 | | | 1284 | 1285 | 1286 |
| 3 | hsa-miR-17-3p | CUGCAGU | | | | | | | | | | | | 1289 | | |
| 4 | hsa-miR-18a | | | | | | | | | | | | | | | |
| 5 | hsa-miR-18b | AAGGUGC | | | | | | | | | | | | | | |
| 6 | hsa-miR-20b | AAAGUGC | | | | | | | | | | | | | | |
| 7 | hss-miR-21 | AGCUUAU | | | | | | | | | | | | | | |
| 8 | hsa-miR-24 | GGCUCAG | | | | | | | | | | | | | | |
| | | | | | | | | | | 1323 | | | | | | |
| | | | | | | 1317 | | | | 1324 | | | | 1328 | | |
| 9 | hsa-miR-25 | | | | | 1318 | | 1321 | | 1325 | | | | 1329 | | |
| 10 | hsa-miR-32 | AUUGCAC | 1315 | | 1316 | 1319 | 1320 | 1322 | | 1326 | 1327 | | | 1330 | 1331 | 1332 |
| 11 | hsa-miR-26b | UGAAGUA | | | | | | | | | | | | | | |
| 12 | hsa-miR-30a-3p | UUUCAGU | | | | | | | | | | | | | | |
| 13 | hsa-miR-34a | | | | | | | | | | | | | | | |
| 14 | hsa-miR-449 | | | | | | | | | | | | | | | |
| 15 | hsa-miR-449b | GGCAGUG | | | | | | | | | | | | | | |
| 16 | hsa-miR-107 | GCAGCAU | 1349 | | | 1350 | 1351 | 1352 | | 1353 | 1354 | | 1355 | 1356 | 1357 | 1358 |
| 17 | hsa-miR-140 | GUGCUUU | | | | | | | | | | | | | | |
| 18 | hsa-miR-148b | CAGUGCA | | | | 1364 | 1365 | | | | | | | 1366 | 1367 | 1368 |
| 19 | hsa-miR-190 | GAUAUGU | | | | | | | | | | | | | | |
| 20 | hsa-miR-199a | | | | | | | | | | | | | | | |
| 21 | hsa-miR-199b | CCAGUGU | | | | | | | | | | | | | | |
| 22 | hsa-miR-202* | UUCCUAU | | | | | | | | | | | | | | |
| 23 | hsa-miR-208 | UAAGACG | | | | | | | | | | | | 1371 | | |
| 24 | hsa-miR-210 | UGUGGGU | | | | | | | | | | | | | | |
| | | | | | | | | | | | | | | | | 1390 |
| 25 | hsa-miR-211 | UCCCUUU | 1381 | | | 1382 | 1383 | 1384 | | 1385 | 1386 | | 1387 | 1388 | 1389 | 1391 |
| 26 | hss-miR-214 | CAGCAGG | | | | | | | | | | | | | | |
| 27 | hsa-miR-218 | UGUGCUU | | | | | | | | | | | | | | |
| 28 | hsa-miR-299-5p | GGUUUAC | | | | | | | | | | | | | | |
| 29 | hsa-miR-325 | CUAGUAG | | | | | | | | | | | | | | |
| 30 | hsa-miR-328 | UGGCCCU | | | | | | | | | | | | | | |
| 31 | hsa-miR-329 | AGAGACC | | | | | | | | | | | | | | |
| 32 | hsa-miR- 338 | CCAGGAU | | | | | | | | | | | | | | |
| 33 | hsa-miR-345 | GCUGACU | | | | | | | | | | | | | | |
| 34 | hsa-miR-426-5p | AUGACAC | | | | | | | | | | | | 1397 | | |
| 35 | hsa-miR-484 | CAGGCUC | | | | | | | | | | | | | | |
| 36 | hsa-miR-485-5p | GAGGCUG | | | | | | | | | | | | | | |
| 37 | hsa-miR-486 | CCUGUAC | | | | | | | | | | | | | | |
| 38 | hsa-miR-488 | CCAGAUA | | | | | | | | | | | | | | |
| 39 | hsa-miR-510 | ACUCAGG | | | | | | | | | | | | | | |
| 40 | hsa-miR-515-3p | AGUGCCO | | | | | | | | | | | | | | |
| 41 | hsa-miR-516-5p | UCUCCAA | | | | | | | | | | | | | | |
| 42 | hsa-miR-517* | GUCUAGA | | | | | | | | | | | | | | |
| | | | | | | | 1437 | | | | | | | 1450 | | |
| | | | | | | | 1438 | | | | | | | 1451 | | |
| | | | | | | | 1439 | 1442 | | 1447 | | | | 1452 | | |
| | | | | | | | 1440 | 1443 | 1445 | 1448 | | | | 1453 | | |
| 43 | hsa-miR-520d | AAGUGCU | 1435 | | | 1436 | 1441 | 1444 | 1446 | 1449 | | | | 1454 | | |
| 44 | hsa-miR-520f | AGUGCUU | | | | | | 1456 | | | | | | | | |
| 45 | hsa-miR-520h | CAAAGUG | | | | | | | | | | | | | | |
| 46 | hsa-miR-522 | AAAUGGU | | | | | | | | | | | | | | |
| 47 | hsa-miR-525* | AAGGCGC | | | | | | | | | | | | | | |
| 48 | hsa-miR-573 | UGAAGUG | | | | | | | | | | | | | | |
| 49 | hsa-miR-587 | UUCCAUA | | | | | | | | | | | | | | |
| 50 | hsa-miR-593 | GGCACCA | | | | | | | | | | | | | | |

**[Table 2-6]**

| sequence number | human miRNA | sequence of 2nd to 8th on 5'terminal side | sequence number of mRNA having sequence with the same 2nd to 8th nucleotides on 5'terminal side | | | | | | | | | | | | | |
|---|---|---|---|---|---|---|---|---|---|---|---|---|---|---|---|---|
| | | | ppy | ssy | lca | cfa | mdo | gga | xla | xtr | bta | oar | ssc | dre | fru | tni |
| 51 | hs a-miR-595 | AAGUGUG | | | | | | | | | | | | | | |
| 52 | hs a-miR-604 | GGCUGCG | | | | | | | | | | | | | | |
| 53 | hs a-miR-612 | GUGGGGA | | | | | | | | | | | | | | |
| 54 | hs a-miR 625 | GGGGGAA | | | | | | | | | | | | | | |
| 55 | hs a-miR-634 | ACCAOCA | | | | | | | | | | | | | | |
| 56 | hs a-miR-635 | CUUGGGC | | | | | | | | | | | | | | |
| 57 | hs a-miR-637 | CUGGGGG | | | | | | | | | | | | | | |
| 58 | hs a-miR-647 | UGGCUGC | | | | | | | | | | | | | | |
| 59 | hs a-miR-650 | GGAGGCA | | | | | | | | | | | | | | |
| 60 | hs a-miR-634 | GGUGGGC | | | | | | | | | | | | | | |
| 61 | hs a-miR-658 | GCGGAGG | | | | | | | | | | | | | | |
| 62 | hs a-miR-660 | ACCCAUU | | | | | | | | | | | | | | |
| 63 | hs a-miR-668 | GUCACUC | | | | | | | | | | | | | | |
| 64 | hs a-miR-675 | GGUGCGG | | | | | | | | | | | | | | |
| 65 | hs a-miR-765 | GGAGGAG | | | | | | | | | | | | | | |
| 66 | hs a-miR-766 | CUCCAGC | | | | | | | | | | | | | | |
| 67 | hs a-miR-194 | GUAACAG | | | | | | | | | | | | | | |
| 68 | hs a-miR-500 | UGCACCU | | | | | | | | | | | | | | |
| 69 | hs a-miR-197 | UCACCAC | | | | | | | | | | | | | | |
| 70 | hs a-miR-221 | GCUACAU | | | | | | | | | | | | | | |
| 71 | hs a-let-7e | GAGGUAG | 1477 | | | 1478 | | | | 1419 | 1480 | | | | | |
| 72 | hs a-miR-1 | | | | | | | | | | | | | | | |
| 73 | hs a-miR-206 | | | | | | | | | | | | | | | |
| 74 | hs a-miR-613 | GGAAUGU | | | | | | | | | | | | | | |
| 75 | hsa-miR-9 | CUUUGGU | | | | | | | | | | | | | | |
| 76 | hsa-miR-23b | UCACAUU | | | | | | | | | | | | | | |
| 77 | hsa-miR-27b | UCACAGU | | | | | | | | | | | | | | |
| 78 | hsa-miR-28 | AGGAGCU | | | | 1487 | | | | | | | | | | |
| 79 | hsa-miR-31 | GGAAGAU | | | | | | | | | | | | | | |
| 80 | hsa-miR-33b | UGCAUUG | | | | | | | | | | | | | | |
| 81 | hsa-miR-96 | UUGGCAC | | | | 1491 | | | | | | | | | | |
| | | | | | | 1506 | | | | | 1510 | | | | | |
| 82 | hs a-miR-100 | AGCCGUA | 1505 | | | 3507 | | 150H | | 1509 | 1511 | | 1512 | 1513 | | |
| 83 | hs a-miR-106a | AAAGUGC | | | | | | | | | | | | | | |
| 84 | hs a-miR-126* | AUUAUUA | | | | | | | | | | | | | | |
| 85 | hs a-miR-121 | CGGAUCC | | | | | | | | | | | | | | |
| 86 | hs a-miR-128b | GACAGUG | | | | | | | | | | | | | | |
| 87 | hs a-miR-129 | UUUUUGC | | | | | | | | | | | | 1515 | | |
| 88 | hs a-miR-133a | | | | | | | | | | | | | | | |
| 89 | hs a-miR-133 | UGGUGCC | | | | | | | | | | | | | | |
| 90 | hs a-miR-135b | AUGGCUU | | | | | | | | | | | | | | |
| 91 | hs a-miR-136 | CUCCAUU | | | | | | | | | | | | | | |
| 92 | hs a-miR-142-5p | AUAAAGU | | | | | | | | | | | | | | |
| 93 | hs a-miR-146a | | | | | | | | | | | | | | | |
| 94 | hs a-miR-146b | GAGAACU | | | | | | | | | | | | | | |
| 95 | hs a-miR-181a | ACAUUCA | | | | | | | | | | | | | | |
| 96 | hsa-miR-182* | GGUUCUA | | | | | | | | | | | | | | |
| 97 | hsa-miR-183 | AUGGCAC | | | | | | | | | | | | | | |
| 98 | hsa-miR-187 | OGUOUCU | | | | | | | | | | | | | | |
| 99 | hsa-miR-192 | | | | | | | | | | | | | | | |
| 100 | hsa-miR-215 | UGACCUA | | | | | | | | | | | | | | |

**[Table 2-7]**

| sequence number | human miRNA | sequence of 2nd to 8th on 5'terminal side | sequence number of mRNA having sequence with the same 2nd to 8^{th} nucleotides on 5' terminal side | | | | | | | | | | | | | |
|---|---|---|---|---|---|---|---|---|---|---|---|---|---|---|---|---|
| | | | ppy | ssy | lca | cfa | mdo | gga | xla | xtr | bta | oar | ssc | dre | fru | tni |
| 101 | hs a-miR-200a* | AUCUUAC | | | | | | | | | | | | | | |
| 102 | hs a-miR-216 | AAUCUCA | | | | | | | | | | | | | | |
| 103 | hs a-miR-217 | ACUGCAU | | | | | | | | | | | | | | |
| 104 | hs a-miR-220 | CACACCG | | | | | | | | | | | | | | |
| 105 | hs a-miR-222 | GCUACAU | | | | | | | | | | | | | | |
| 106 | hs a-miR-223 | GUCAGUU | | | | | | | | | | | | | | |
| 107 | hs a-miR-224 | AAGUCAC | | | | | | | | | | | | | | |
| 108 | hs a-miR-296 | GGGCCCC | | | | | | | | | | | | | | |
| 109 | hs a-miR-302b* | CUUUAAC | | | | | | | | | | | | | | |
| 110 | hs a-miR-302c* | UUAACAU | | | | | | | | | | | | | | |
| 111 | hs a-miR-362 | AUCCUUG | | | | | | | | | | | | | | |
| 112 | hs a-miR-373* | GUCAAAA | | | | | | | | | | | | | | |
| 113 | hs a-miR-374 | UAUAAUA | | | | | | | | | | | | | | |
| 114 | hsa-miR-376a | UCAUAGA | | | | | | | | | | | | | | |
| 115 | hsa-miR-378 | UCCUGAG | | | | | | | | | | | | | | |
| 116 | hsa-miR-409-3p | GAAUGUU | | | | | | | | | | | | | | |
| 117 | hsa-miR-409-5p | GGUUACC | | | | | | | | | | | | | | |
| 118 | hsa-miR-429 | | | | | | | | | | | | | | | |
| 119 | hsa-miR-200c | AAUACUG | | | | | | | | | | | | | | |
| 120 | hsa-miR-432* | UGGAUGG | | | | | | | | | | | | | | |
| 121 | hsa-miR-448 | UGGAUAU | | | | | | | | | | | | | | |
| 122 | hsa-miR-450 | UUUUGCG | | | | | | | | | | | | | | |
| 123 | hsa-miR-451 | AACCGUU | | | | | | | | | | | | | | |
| 124 | hsa-miR-452 | GUUUGCA | | | | | | | | | | | | | | |
| 125 | hsa-miR-487b | AUCGUAC | | | | | | | | | | | | | | |
| 126 | hsa-miR-489 | GUGACAU | | | | | | | | | | | | | | |
| 127 | hs a-miR-514 | UUGACAC | | | | | | | | | | | | | | |
| 128 | hs a-miR-517c | UCGUGCA | | | | | | | | | | | | | | |
| 129 | hs a-miR-518c | AAAGCGC | | | | | | | | | | | | | | |
| 130 | hs a-miR-524* | UACAAAG | | | | | | | | | | | | | | |
| 131 | hs a-miR-542-3p | GUGACAG | | | | | | | | | | | | | | |
| 132 | hs a-miR-544 | UUCUGCA | | | | | | | | | | | | | | |
| 133 | hs a-miR-545 | UCAGCAA | | | | | | | | | | | | | | |
| 134 | hs a-miR-550 | GUCUUAC | | | | | | | | | | | | | | |
| 135 | hs a-miR-552 | ACAGGUG | | | | | | | | | | | | | | |
| 136 | hs a-miR-596 | AGCCUGC | | | | | | | | | | | | | | |
| 137 | hs a-miR-601 | GGUCUAG | | | | | | | | | | | | | | |
| 138 | hs a-miR-608 | GGGGUGG | | | | | | | | | | | | | | |
| 139 | hs a-miR-609 | GGGUGUU | | | | | | | | | | | | | | |
| 140 | hs a-miR-617 | GACUUCC | | | | | | | | | | | | | | |
| 141 | hs a-miR-627 | UGAGUCU | | | | | | | | | | | | | | |
| 142 | hs a-miR-632 | UGUCUGC | | | | | | | | | | | | | | |
| 143 | hs a-miR-644 | GUGUGGC | | | | | | | | | | | | | | |
| 144 | hs a-miR-659 | UUGGUUC | | | | | | | | | | | | | | |
| 145 | hs a-miR-769-5p | GAGACCU | | | | | | | | | | | | | | |
| 146 | hs a-miR-801 | AUUGCUG | | | | | | | | | | | | | | |
| 147 | hs a-miR-365 | AAUGGGC | | | | | | | | | | | | | | |
| 148 | hs a-miR-142-3p | GUAGUGU | | | | | | | | | | | | | | |
| 149 | hs a-miR-200a | AACACUG | | | | | 1542 | | | | | | | 1543 | | |
| 150 | hs a-miR-361 | UAUCAGA | | | | | | | | | | | | | | |

**[Table 2-8]**

| sequence number | human miRNA | sequence of 2nd to 8th on 5' terminal side | sequence number of mRNA having sequence with the same 2nd to 8th nucleotides on 5' terminal side | | | | | | | | | | | | | |
|---|---|---|---|---|---|---|---|---|---|---|---|---|---|---|---|---|
| | | | ppy | ssy | lca | cfa | mdo | gga | xla | xtr | bta | oar | ssc | dre | fru | tni |
| 3372 | hsa-miR-29a | | | | | | | | | | | | | | | |
| 3373 | hsa-miR-29b | AGGACCA | | | | | | | | | | | | | | |
| 3374 | hsa-miR-30a-5p | | | | | | | | | | | | | | | |
| 3375 | hsa-miR-30b | GUAAACA | | | | | | | | | | | | | | |
| 3376 | hsa-miR-105 | CAAAUGC | | | | | | | | | | | | | | |
| 3377 | hsa-miR-124a | UAAGGCA | | | | | | | | | | | | | | |
| 3378 | hsa-miR-128a | CACAGUG | | | | | | | | | | | | | | |
| 3379 | hsa-miR-150 | CUCCCAA | | | | | | | | | | | | | | |
| 3380 | hsa-miR-154 | AGGUUAU | | | | | | | | | | | | | | |
| 3331 | hsa-miR-299-3p | AUGUGGG | | | | | | | | | | | | | | |
| 3382 | hsa-miR-360-5p | GGUUGAC | | | | | | | | | | | | | | |
| 3333 | hsa-miR-383 | GAUGAGA | | | | | | | | | | | | | | |
| 3384 | hsa-miR-411 | AGUAGAC | | | | | | | | | | | | | | |
| 3385 | hsa-miR-423 | GCUCGGU | | | | | | | | | | | | | | |
| 3386 | hsa-miR-933 | UCAUGAU | | | | | | | | | | | | | | |
| | | | | | | | | | | | | | | 3722 | | |
| | | | | | | | | | | | | | | 3723 | | |
| | | | | | | | | | | 3713 | | | | 3729 | | |
| | | | | | | | | 3715 | | 3719 | | | | 3725 | | |
| | | | | | | 3712 | | 3716 | | 3720 | | | | 3726 | 3728 | 3730 |
| 3387 | has-miR-454-3p | AGUGCAA | | | | 3713 | 3714 | 3717 | | 3721 | | | | 3727 | 3729 | 3731 |
| 3388 | hsa-miR-501 | AUCCUUU | | | | | | | | | | | | | | |
| 3389 | hsa-miR-504 | GACGGUG | | | | | | | | | | | | | | |
| 3390 | hsa-miR-506 | AAGGCAC | | | | 3734 | | | | | | | | 3735 | 3736 | 3737 |
| 3391 | hse-miR-507 | UUUGCAC | | | | | | | | | | | | | | |
| 3392 | hsa-miR-508 | GAUUGUA | | | | | | | | | | | | | | |
| 3393 | hsa-miR-511 | UGUCUUU | | | | | | | | | | | | | | |
| | | | | | | | | | | 3740 | | | | | | |
| 3395 | hsa-miR-527 | UGCAAAG | | | | | | | | | | | | | | |
| 3396 | hsa-miR-562 | AAGUAGC | | | | | | | | | | | | | | |
| 3397 | hsa-miR-567 | GUAUGUU | | | | | | | | | | | | | | |
| 3398 | hsa-miR-589 | CAGAACA | | | | | | | | | | | | | | |
| 3399 | hsa-miR-597 | GUGUCAC | | | | | | | | | | | | | | |
| 3400 | hsa-miR-605 | AAAUCCC | | | | | | | | | | | | | | |
| 3401 | hsa-miR-619 | ACCUGGA | | | | | | | | | | | | | | |
| 3402 | hsa-miR-629 | UUCUGCG | | | | | | | | | | | | | | |
| 3403 | hsa-miR-640 | UGAUCCA | | | | | | | | | | | | | | |
| 3404 | hsa-miR-767-5p | GGACCAU | | | | | | | | | | | | | | |
| 3405 | hsa-miR-770-5p | GCAGUAC | | | | | | | | | | | | | | |
| 3406 | hsa-miR-802 | AGUAACA | | | | | | | | | | | | | | |

As the above-described nucleic acids, micro-RNA precursors are also used preferably. A micro-RNA precursor is a nucleic acid about 50 to about 200 nucleotides long, more preferably about 70 to about 100 nucleotides long, including the above-described nucleic acids used in the present invention, and capable of forming a hairpin structure. A micro-RNA is produced from a micro-RNA precursor via processing by a protein called Dicer.

As examples of micro-RNA precursors used in the present invention for the human micro-RNAs of SEQ ID NO:1, nucleic acids consisting of a nucleotide sequence of any one of SEQ ID NOs:1544 to 1545 can be mentioned. For the micro-RNAs of SEQ ID NOs:2 to 1543 and 3372 to 3741, nucleic acids consisting of a nucleotide sequence of any one of SEQ ID NOs:1546 to 3371 and 3742 to 4147 can be mentioned. Tables of the correspondence of micro-RNAs and micro-RNA precursors used in the present invention are shown in Table 3-1 to Table 3-39.

**[Table 3-1]**

| Sequence number | miRNA name | sequence number | miRNA precisions name |
|---|---|---|---|
| 1 | hsa-miR-16 | 1544 | hsa-mir-16-1 |
| | | 1545 | hsa-mir-16-2 |
| 2 | hsa-miR-195 | 1546 | hsa-mir-195 |
| 3 | hsa-miR-17-3p | 1547 | hsa-mir-17 |
| 4 | hsa-miR-18a | 1548 | hsa-mir-18a |
| 5 | hsa-miR-18b | 1549 | hsa-mir-18b |
| 6 | hsa-miR-20b | 1550 | hsa-mir-20b |
| 7 | hsa-miR-21 | 1551 | hsa-mir-21 |
| 8 | hsa-miR-24 | 1552 | hsa-mir-24-1 |
| | | 1553 | hsa-mir-24-2 |
| 9 | sa-miR-25 | 1554 | hsa-mir-25 |
| 10 | hsa-miR-32 | 1555 | hsa-mir-32 |
| 11 | hsa-miR-26b | 1556 | hsa-mir-26b |
| 12 | hsa-miR-34a-3p | 1557 | hsa-mir-30a |
| 13 | hsa-miR-34a | 1558 | hsa-mir-34a |
| 14 | hsa-miR-449 | 1559 | hsa-mir-449 |
| 15 | 5 hsa-miR-449b | 1560 | hsa-mir-449b |
| 16 | hsa-miR-107 | 1561 | hsa-mir-107 |
| 17 | hsa-miR-140 | 1562 | hsa-mit-140 |
| 18 | hsa-miR-148b | 1563 | hsa-mir-148b |
| 19 | hsa-miR-190 | 1564 | hsa-mir-190 |
| 20 | hsa-miR-199a | 1565 | hsa-mir-199a-1 |
| | | 1566 | hsa-miR-199a-2 |
| 21 | hsa-miR-199b | 1567 | hsa-mir-199b |
| 22 | hsa-miR-202* | 1568 | hsa-mir-202 |
| 23 | hsa-miR-208 | 1559 | hsa-mir-208 |
| 24 | hsa-miR-210 | 1570 | hsa-mir-210 |
| 25 | hsa-miR-211 | 1571 | hsa-mir-211 |
| 26 | hsa-miR-214 | 1572 | hsa-mir-214 |
| 27 | hsa-miR-218 | 1573 | hsa-mir-218-1 |
| | | 1574 | hsa-mir-218-2 |
| 28 | hsa-miR-299-5p | 1575 | hsa-mir-299 |
| 29 | hsa-miR-325 | 1576 | hsa-mir-325 |
| 30 | hsa-miR-328 | 1577 | hsa-mir-328 |
| 31 | hsa-miR-328 | 1578 | hsa-mir-329-1 |
| | | 1579 | hsa-mir-329-2 |
| 32 | hsa-miR-338 | 1580 | hsa-mir-338 |
| 33 | hsa-miR-345 | 1581 | hsa-mir-345 |
| 34 | hsa-miR-425-5p | 1582 | hsa-mir-425 |
| 35 | hsa-miR-484 | 1583 | hsa-mir-484 |
| 36 | hsa-miR-485-5p | 1584 | hsa-mir-485 |
| 37 | hsa-miR-486 | 1585 | hsa-mir-486 |
| 38 | hsa-miR-488 | 1586 | hsa-mir-488 |
| 39 | hsa-miR-510 | 1587 | hsa-mir-510 |
| 40 | hsa-miR-515-3p | 1588 | hsa-mir-515-1 |
| | | 1589 | hsa-mir-515-2 |
| 41 | hsa-miR-515-5p | 1588 | hsa-mir-515-1 |
| | | 1589 | hsa-mir-515-2 |
| 42 | hsa-miR-517* | 1590 | hsa-mir-517a |
| | | 1591 | hsa-mir-517b |
| | | 1592 | hsa-mir-517c |
| 43 | hsa-miR-520d | 1583 | hsa-mir-520d |
| 44 | hsa-miR-520f | 1594 | hsa-mir-520f |
| 45 | hsa-miR-520h | 1595 | hsa-mir-520h |
| 46 | hsa-miR-522 | 1596 | hsa-mir-522 |
| 47 | hsa-miR-525* | 1597 | hsa-mir-525 |
| 48 | hsa-miR-573 | 1598 | hsa-mir-573 |
| 49 | hsa-miR-587 | 1599 | hsa-mir-587 |
| 50 | hsa-miR-593 | 1600 | hsa-mir-593 |

**[Table 3-2]**

| sequence number | miRNA name | sequence number | miRNA precursor name |
|---|---|---|---|
| 51 | hsa-miR-595 | 1601 | hsa-mir-595 |
| 52 | hsa-miR-604 | 1602 | hsa-mir-604 |
| 53 | hsa-miR-612 | 1603 | hsa-mir-612 |
| 54 | hsa-miR-625 | 1604 | hsa-mir-625 |
| 55 | hsa-miR-634 | 1605 | hsa-mir-634 |
| 56 | hsa-miR-635 | 1606 | hsa-mir-635 |
| 57 | hsa-miR-637 | 1607 | hsa-mir-637 |
| 58 | hsa-miR-647 | 1608 | hsa-mir-647 |
| 59 | hsa-miR-650 | 1609 | hsa-mir-650 |
| 60 | hsa-miR-654 | 1610 | hsa-mir-654 |
| 61 | hsa-miR-658 | 1611 | hsa-mir-658 |
| 62 | hsa-miR-660 | 1612 | hsa-mir-660 |
| 63 | hsa-miR-668 | 1613 | hsa-mir-668 |
| 64 | hsa-miR-675 | 1614 | hsa-mir-675 |
| 65 | hsa-miR-765 | 1615 | hsa-mir-765 |
| 66 | hsa-miR-766 | 1616 | hsa-mir-766 |
| 67 | hsa-miR-194 | 1617 | hsa-mir-194-1 |
| | | 1618 | hsa-mir-194-2 |
| 68 | hsa-miR-500 | 1619 | hsa-mir-500 |
| 69 | hsa-miR-197 | 1620 | hsa-mir-197 |
| 70 | hsa-miR-221 | 1621 | hsa-mir-221 |
| 71 | hsa-let-7e | 1622 | hsa-let-7e |
| 72 | hsa-mR-1 | 1623 | hsa-mir-1-1 |
| | | 1624 | hsa-mir-1-2 |
| 73 | hsa-miR-206 | 1625 | hsa-mir-206 |
| 74 | hsa-miR-613 | 1626 | hsa-mir-613 |
| 75 | hsa-miR-9 | 1627 | hsa-mir-9-1 |
| | | 1628 | hsa-mir-9-2 |
| | | 1629 | hsa-mir-9-3 |
| 76 | hsa-miR-23b | 1630 | hsa-mir-23b |
| 77 | hsa-miR-27b | 1631 | hsa-mir-27b |
| 78 | hsa-miR-28 | 1632 | hsa-mir-28 |
| 79 | hsa-miR-31 | 1633 | hsa-mir-31 |
| 80 | hsa-miR-33b | 1634 | hsa-mir-33b |
| 81 | hsa-miR-96 | 1635 | hsa-mir-96 |
| 82 | hsa-miR-100 | 1636 | hsa-mir-100 |
| 83 | hsa-miR-106a | 1637 | hsa-mir-106a |
| 84 | hsa-miR-126* | 1638 | hsa-mir-126 |
| 85 | hsa-miR-127 | 1639 | hsa-mir-127 |
| 86 | hsa-miR-128b | 1640 | hsa-mir-128b |
| 87 | hsa-miR-129 | 1641 | hsa-mir-129-1 |
| | | 1642 | hsa-mir-129-2 |
| 88 | hsa-miR-133a | 1643 | hsa-mir-133a-1 |
| | | 1644 | hsa-mir-133a-2 |
| 89 | hsa-miR-133b | 1645 | hsa-mir-133b |
| 90 | hsa-miR-135b | 1646 | hsa-mir-135b |
| 91 | hsa-miR-136 | 1647 | hsa-mir-136 |
| 92 | hsa-miR-142-5p | 1648 | hsa-mir-142 |
| 93 | hsa-miR-146a | 1649 | hsa-mir-146a |
| 94 | hsa-miR-146b | 1650 | hsa-mir-146b |
| 95 | hsa-miR-181a | 1651 | hsa-mir-181a |
| 96 | hsa-miR-182* | 1652 | hsa-mir-182 |
| 97 | hs8-miR-183 | 1653 | hsa-mir-183 |
| 98 | hsa-miR-187 | 1654 | hsa-mir-187 |
| 99 | hsa-miR-192 | 1655 | hsa-mir-192 |
| 100 | hsa-miR-215 | 1656 | hsa-mir-215 |

**[Table 3-3]**

| sequence number | miRNA name | sequence number | miRNA precursor name |
|---|---|---|---|
| 101 | hsa-miR-200a* | 1657 | hsa-mir-200a |
| 102 | hsa-miR-216 | 1658 | hsa-mir-216 |
| 103 | hsa-miR-217 | 1659 | hsa-mir-217 |
| 104 | hsa-miR-220 | 1660 | hsa-mir-200 |
| 105 | hsa-miR-222 | 1661 | hsa-mir-222 |
| 106 | hsa-miR-223 | 1662 | hsa-mir-223 |
| 107 | hsa-miR-224 | 1663 | hsa-mir-224 |
| 108 | hsa-miR-296 | 1664 | hsa-mir-296 |
| 109 | hsa-miR-302b* | 1685 | hsa-mir-302b |
| 110 | hsa-miR-302c* | 1666 | hsa-mir-302c |
| 111 | hsa-miR-362 | 1667 | hsa-mir-362 |
| 112 | hsa-miR-373* | 1668 | hsa-mir-373 |
| 113 | hsa-miR-374 | 1669 | hsa-mir-374 |
| 114 | hsa-miR-376a | 1670 | hsa-mir-376a-1 |
| | | 1671 | hsa-mir-376a-2 |
| 115 | hsa-miR-378 | 1672 | hsa-mir-378 |
| 116 | hsa-miR-409-3p | 1673 | hsa-mir-409 |
| 117 | hsa-miR-409-5p | 1673 | hsa-mir-409 |
| 118 | hsa-miR-429 | 1674 | hsa-mir-429 |
| 119 | hsa-miR-200c | 1675 | hsa-mir-200c |
| 120 | hsa-miR-432* | 1676 | hsa-mir-432 |
| 121 | hsa-miR-448 | 1677 | hsa-mir-448 |
| 122 | hsa-miR-450 | 1678 | hsa-mir-450-1 |
| | | 1679 | hsa-mir-450-2 |
| 123 | hsa-miR-451 | 1680 | hsa-mir-451 |
| 124 | hsa-miR-452 | 1681 | hsa-mir-452 |
| 125 | hsa-miR-487b | 1682 | hsa-mir-487b |
| 126 | hsa-miR-489 | 1683 | hsa-mir-489 |
| 127 | hsa-miR-514 | 1684 | hsa-mir-514-1 |
| | | 1685 | hsa-mir-514-2 |
| | | 1888 | hsa-mir-514-3 |
| 128 | hsa-miR-517c | 1592 | hsa-mir-517c |
| 129 | hsa-miR-518c | 1687 | hsa-mir-518c |
| 130 | hsa-miR-524* | 1688 | hsa-mir524 |
| 131 | hsa-miR-542-3p | 1689 | hsa-mir-542 |
| 132 | hsa-miR-544 | 1690 | hsa-mir-544 |
| 133 | hsa-miR-545 | 1691 | hsa-mir-545 |
| 134 | hsa-miR-550 | 1692 | hsa-mir-550-1 |
| | | 1693 | hsa-mir-550-2 |
| 135 | hsa-miR-552 | 1694 | hsa-mir-552 |
| 136 | hsa-miR-596 | 1695 | hsa-mir-596 |
| 137 | hsa-miR-601 | 1696 | hsa-mir-601 |
| 138 | hsa-miR-608 | 1697 | hsa-mir-608 |
| 139 | hsa-miR-609 | 1698 | hsa-mir-609 |
| 140 | hsa-miR-817 | 1699 | hsa-mir-617 |
| 141 | hsa-miR-627 | 1700 | hsa-mir-627 |
| 142 | hsa-miR-632 | 1701 | hsa-mir-632 |
| 143 | hsa-miR-644 | 1702 | hsa-mir-644 |
| 144 | hsa-miR-659 | 1703 | hsa-mir-659 |
| 145 | hsa-miR- 769-5p | 1704 | hsa-mir-789 |
| 146 | hsa-miR-801 | 1705 | hsa-mir-801 |
| 147 | hsa-miR-365 | 1706 | hsa-mir-365-1 |
| | | 1707 | hsa-mir-365-2 |
| 148 | hsa-miR-142-3p | 1648 | hsa-mir-142 |
| 149 | hsa-miR-200a | 1657 | hsa-mir-200a |
| 150 | hsa-miR-361 | 1708 | hsa-mir-361 |

**[Table 3-4]**

| sequence number | miRNA name | sequence number | miRNA precursor name |
|---|---|---|---|
| 151 | mmu-miR-16 | 1709 | mmu-mir-16-1 |
| | | 1710 | mmu-mir-16-2 |
| 152 | mo-miR-16 | 1711 | mo-mir-16 |
| 153 | age-miR-16 | 1712 | age-mir-16 |
| 154 | lla-miR-16 | 1713 | lla-mir-16 |
| 155 | sla-miR-16 | 1714 | sla-mir-16 |
| 156 | mml-miR-16 | 1715 | mml-mir-16-1 |
| | | 1716 | mml-mir-16-2 |
| 157 | mne-miR-16 | 1717 | mne-mir-16 |
| 158 | ggo-miR-16 | 1718 | ggo-mir-16 |
| 159 | ppa-miR-16 | 1719 | ppa-mir-16 |
| 360 | ptr-miR-16 | 1720 | ptr-mir-16 |
| 161 | ppy-miR-16 | 1721 | ppy-mir-16 |
| 162 | lca-miR-16 | 1722 | lca-mir-16 |
| 163 | cfa-miR-16 | 1723 | cfa-mir-16-1 |
| | | 1724 | cfa-mir-16-2 |
| 164 | mdo-miR-16 | 1725 | mdo-mir-16 |
| 165 | gga-miR-16 | 1726 | gga-mir-16-1 |
| | | 1727 | gga-mir-16-2 |
| 166 | xtr-miR-16a | 1728 | xtr-mir-16a |
| 167 | xtr-miR-16b | 1729 | xtr-mir-16b |
| 168 | bta-miR-16 | 1730 | bta-mir-16 |
| 169 | dre-rniR-1Ea | 1731 | dre-mir-16a |
| 170 | dre-miR-16b | 1732 | dre-mir-16b |
| 171 | dre-miR-16c | 1733 | dre-mir-16c |
| 172 | fru-miR-16 | 1734 | fru-mir-16 |
| 173 | tni-miR-16 | 1735 | tni-mir-16 |
| 174 | mmu-miR-195 | 1736 | mmu-mir-195 |
| 175 | mo-miR-195 | 1737 | mo-mir-195 |
| 176 | mml-miR-195 | 1738 | mml-mir-195 |
| 177 | ggo-miR-195 | 1739 | ggo-mir-195 |
| 178 | ppa-miR-195 | 1740 | ppa-mir-195 |
| 179 | cfa-miR-195 | 1741 | cfa-mir-195 |
| 180 | bta-miR-195 | 1742 | bta-mir-195 |
| 181 | rno-miR-17-3p | 1743 | rno-mir- 17-1 |
| | | 1744 | rno-mir-17-2 |
| 182 | age-miR-17-3p | 1745 | age-mir-17 |
| 183 | lla-miR-17-3p | 1746 | lla-mir-17 |
| 184 | sla-miR-17-3p | 1747 | sla-mir-17 |
| 185 | mml-miR-17-3p | 174.8 | mml-mir-17 |
| 186 | mne-miR-17-3p | 1749 | mne-mir-17 |
| 187 | ggo-miR-17-3p | 1750 | ggo-mir-17 |
| 188 | ppa-miR-17-3p | 1751 | ppa-mir-17 |
| 189 | ptr-miR-17-3p | 1752 | ptr-mir-17 |
| 190 | ppy-miR-17-3p | 1753 | ppv-mir-17 |
| 191 | Ica-miR-17-3p | 1754 | lca-mir-17 |
| 192 | mdo-miR-17-3p | 1755 | mdo-mir-17 |
| 193 | gga-miR-17-3p | 1756 | gga-mir-17 |
| 194 | xtr-miR-17-3p | 1757 | xtr-mir-17 |
| 195 | bta-miR-17-3p | 1758 | bta-mir-17 |
| 196 | mmu-miR-18a | 1759 | mmu-mir-18a |
| 197 | rno-miR-18a | 1760 | rno-mir-18a |
| 198 | age-miR-18 | 1761 | age-mir-18 |
| 199 | lla-miR-18 | 1762 | lla-mir-18 |
| 200 | sla-miR-18 | 1763 | sla-mir-18 |

**[Table 3-5]**

| sequence number | miRNA name | sequence number | miRNA precursor name |
|---|---|---|---|
| 201 | mml-miR-18 | 1764 | mml-mir-18 |
| 202 | mne-miR-18 | 1765 | mne-mir-18 |
| 203 | ggo-miR-18 | 1766 | ggo-mir-18 |
| 204 | ppa-miR-18 | 1767 | ppa-mir-18 |
| 205 | ptr-miR-18 | 1768 | ptr-mir-18 |
| 206 | ppy-miR-18 | 1769 | ppy-mir-18 |
| 207 | lca-miR-18 | 1770 | lca-mir-18 |
| 208 | mdo-miR-18 | 1771 | mdo-mir-18 |
| 209 | gga-miR-18a | 1772 | gga-mir-18a |
| 210 | xtr-miR-18a | 1773 | xtr-mir-18a |
| 211 | bta-miR-18a | 1774 | bta-mir-18a |
| 212 | ssc-miR-18 | 1775 | ssc-mir-18 |
| 213 | dre-miR-18a | 1776 | dre-mir-18a |
| 214 | dre-miR-18c | 1777 | dre-mir-18c |
| 215 | fru-miR-18 | 1778 | fru-mir-18 |
| 216 | tni-miR-18 | 1779 | tni-mir-18 |
| 217 | mmu-miR-18b | 1780 | mmu-mir-18b |
| 218 | mmi-miR-18b | 1781 | mml-mir-18b |
| 219 | gga-miR-18b | 1782 | gga-mir-18b |
| 220 | xtr-miR-18b | 1783 | xtr-mir-18b |
| 221 | bta-miR-18b | 1784 | bta-mir-18b |
| 222 | dre-miR-18b | 1785 | dre-mir-18b |
| 223 | mmu-miR-20b | 1786 | mmu-mir-20b |
| 224 | mo-miR-20a | 1787 | mo-mir-20a |
| 225 | age-miR-20 | 1788 | age-mir-20 |
| 226 | lla-miR-20 | 1789 | lla-mir-20 |
| 227 | sla-miR-20 | 1790 | sla-mir-20 |
| 228 | mml-miR-20a | 1791 | mml-mir-20a |
| 229 | mml-miR-20b | 1792 | mml-mir-20b |
| 230 | mne-miR-20 | 1793 | mne-mir-20 |
| 231 | ggo-miR-20 | 1794 | ggo-mir-20 |
| 232 | ppa-miR-20 | 1195 | ppa-mir-20 |
| 233 | ptr-miR-20 | 1796 | ptr-mir-20 |
| 234 | ppy-miR-20 | 1797 | ppy-mir-20 |
| 235 | lca-miR-20 | 1798 | lca-mir-20 |
| 236 | cfa-miR-20 | 1799 | cfa-mir-20 |
| 237 | mdo-miR-20 | 1800 | mdo-mir-20 |
| 238 | gga-miR-20a | 1801 | gga-mir-20a |
| 239 | gga-miR-20b | 1802 | gga-mir-20b |
| 240 | xla-miR-20 | 1803 | xla-mir-20 |
| 241 | xtr-miR-20a | 1804 | xtr-mir-20a |
| 242 | xtr-miR-20b | 1805 | xtr-mir-20b |
| 243 | bta-miR-20a | 1806 | bta-mir-20a |
| 244 | bta-miR-20b | 1807 | bta-mir-20b |
| 245 | ssc-miR-20 | 1808 | ssc-mir-20 |
| 246 | dre-miR-20a | 1809 | dre-mir-20a |
| 247 | dre-miR-20b | 1810 | dre-mir-20b |
| 248 | fru-miR-20 | 1811 | fru-mir-20 |
| 249 | tni-miR-20 | 1812 | tni-mir-20 |
| 250 | mmu-miR-21 | 1813 | mmu-mir-21 |

**[Table 3-6]**

| sequence number | miRNA name | sequence number | miRNA precursor name |
|---|---|---|---|
| 251 | mo-miR-21 | 1814 | mo-mir-21 |
| 252 | cgr-miR-21 | 1815 | cgr-mir-21 |
| 253 | age-miR-21 | 1816 | aga-mir-21 |
| 254 | mml-miR-21 | 1817 | mml-mir-21 |
| 255 | mne-miR-21 | 1818 | mne-mir-21 |
| 256 | Mo-miR-21 | 1819 | ggo-mir-21 |
| 257 | ppa-miR-21 | 1820 | ppa-mir-21 |
| 258 | ptr-miR-21 | 1821 | ptr-mir-21 |
| 259 | ppy-miR-21 | 1822 | ppy-mir-21 |
| 260 | cfa-miR-21 | 1823 | cfa-mir-21 |
| 261 | mdo-miR-21 | 1824 | mdo-mir-21 |
| 262 | gga-miR-21 | 1825 | gga-mir-21 |
| 263 | bta-miR-21 | 1826 | bta-mir-21 |
| 264 | ssc-miR-21 | 1827 | ssc-mir-21 |
| 265 | dre-miR-21 | 1828 | dre-mir-21-1 |
| | | 1829 | dre-mir-21-2 |
| 266 | fru-miR-2 | 1830 | fru-mir-21 |
| 267 | tni-miR-21 | 1831 | tni-mir-21 |
| 268 | mmu-miR-24 | 1832 | mmu-mir-24-1 |
| | | 1833 | mmu-mir-24-2 |
| 269 | mo-miR-24 | 1834 | mo-mir-24-1 |
| | | 1835 | mo-mir-24-2 |
| 270 | mml-miR-24 | 1836 | mml-mir-24-1 |
| | | 1837 | mml-mir-24-2 |
| 271 | mne-miR-24 | 1838 | mne-mir-24-1 |
| | | 1839 | mne-mir-24-2 |
| 272 | ggo-miR-24 | 1840 | ggo-mir-24 |
| 273 | ppa-miR-24 | 1841 | ppa-mir-24-1 |
| | | 1842 | ppa-mir-24-2 |
| 274 | ptr-miR-24 | 1843 | ptr-mir-24 |
| 275 | ppy-miR-24 | 1844 | ppy-mir-24-1 |
| | | 1845 | ppy-mir-24-2 |
| 276 | cfa-miR-24 | 1846 | cfa-mir-24-1 |
| | | 1847 | cfa-mir-24-2 |
| 277 | mdo-miR-24 | 1848 | mdo-mir-24-1 |
| | | 1849 | mdo-mir-24-2 |
| 278 | gga-miR-24 | 1850 | gga-mir-24 |
| 279 | xtr-miR-24b | 1851 | xtr-mir-24b |
| 280 | bta-miR-24 | 1852 | bta-mir-24 |
| 281 | ssc-miR-24 | 1853 | ssc-mir-24 |
| 282 | dre-miR-24 | 1854 | dre-mir-24-1 |
| | | 1855 | dre-mir-24-2 |
| | | 1856 | dre-mir-24-3 |
| | | 1857 | dre-mir-24-4 |
| 283 | fru-miR-24 | 1858 | fru-mir-24-1 |
| | | 1859 | fru-mir-24-2 |
| 284 | tni-miR-24 | 1860 | tni-mir-24-1 |
| | | 1861 | tni-mir-24-2 |
| 285 | mmu-miR-25 | 1862 | mmu-mir-25 |
| 286 | mo-miR-25 | 1863 | rno-mir-25 |
| 287 | lla-miR-25 | 1864 | lla-mir-25 |
| 288 | mml-miR-25 | 1865 | mml-mir-25 |
| 289 | mne-miR-25 | 1866 | mne-mir-25 |
| 290 | ggo-miR-25 | 1867 | ggo-mir-25 |
| 291 | ppa-miR-25 | 1868 | ppa-mir-25 |
| 292 | ppa-míR-25 | 1869 | ptr-mir-25 |
| 293 | ppy-miR-25 | 1870 | ppy-mir-25 |
| 294 | cfa-miR-25 | 1871 | cfa-mir-25 |
| 295 | mdo-miR-25 | 1872 | mdo-mir-25 |
| 296 | xtr-miR-25 | 1873 | xtr-mir-25-1 |
| | | 1814 | xtr-mir-25-2 |
| 297 | bta-miR-25 | 1875 | bta-mir-25 |
| 298 | dre-miR-25 | 1876 | dre-mir-25 |
| 299 | fru-miR-25 | 1877 | fru-mir-25 |
| 300 | tni-miR-25 | 1878 | tni-mir-25 |

**[Table 3-7]**

| sequence number | miRNA name | sequence number | miRNA precursor name |
|---|---|---|---|
| 301 | mmu-miR-32 | 1879 | mmu-mir-32 |
| 302 | rno-miR-32 | 1880 | rno-mir-32 |
| 303 | sla-miR-32 | 1881 | sla-mir-32 |
| 304 | mml-miR-32 | 1882 | mml-mir-32 |
| 305 | mne-miR-32 | 1883 | mne-mir-32 |
| 306 | ggo-miR-32 | 1884 | ggo-mir-32 |
| 307 | ppa-miR-32 | 1885 | ppa-mir-32 |
| 308 | ptr-miR-32 | 1886 | ptr-mir-32 |
| 309 | ppy-miR-32 | 1887 | ppy-mir-32 |
| 310 | cfa-miR-32 | 1888 | cfa-mir-32 |
| 311 | mdo-miR-32 | 1889 | mdo-mir-32 |
| 312 | gga-miR-32 | 1890 | gga-mir-32 |
| 313 | ssc-miR-32 | 1891 | ssc-mir-32 |
| 314 | mmu-miR-26b | 1892 | mmu-mir-26b |
| 315 | rno-miR-26a | 1893 | rno-mir-26a |
| 316 | rno-miR-26b | 1894 | rno-mir-26b |
| 317 | lla-miR-26a | 1895 | lla-mir-26a |
| 318 | mml-miR-26a | 1896 | mml-mir-26a-1 |
| | | 1897 | mml-mir-26a-2 |
| 319 | mml-miR-26b | 1898 | mml-mir-26b |
| 320 | mne-miR-26a | 1899 | mne-mir-26a |
| 321 | ggo-miR-26a | 1900 | ggo-mir-26a |
| 322 | ppa-miR-26a | 1901 | ppa-mir-26a |
| 323 | ptr-miR-26a | 1902 | ptr-mir-26a |
| 324 | ppy-miR-26a | 1903 | ppy-mir-26a |
| 325 | cfa-miR-26a | 1904 | cfa-mir-26a-1 |
| | | 1905 | cfa-mir-26a-2 |
| 326 | cfa-miR-26b | 1906 | cfa-mir-26b |
| 327 | gga-miR-26a | 1907 | gga-mir-26a |
| 328 | xtr-miR-26 | 1908 | xtr-mir-26-1 |
| | | 1909 | xtr-mir-26-2 |
| 329 | bta-miR-26a | 1910 | bta-mir-26a |
| 330 | bta-miR-26b | 1911 | bta-mir-26b |
| 331 | ssc-miR-26a | 1912 | ssc-mir-26a |
| 332 | dre-miR-26a | 1913 | dre-mir-26a-1 |
| | | 1914 | dre-mir-26a-2 |
| | | 1915 | dre-mir-26a-3 |
| 333 | dre-miR-26b | 1916 | dre-mir-26b |
| 334 | fru-miR-26 | 1917 | fru-mir-26 |
| 335 | tni-miR-26 | 1918 | tni-mir-26 |
| 336 | mo-miR-30d* | 1919 | mo-mir-30d |
| 337 | mo-miR-30e* | 1920 | mo-mir-30e |
| 338 | mml-miR-30a-3p | 1921 | mml-mir-30a |
| 339 | ggo-miR-30a-3p | 1922 | ggo-mir-30a |
| 340 | ppa-miR-30a-3p | 1923 | ppa-mir-30a |
| 341 | ptr-miR-30a-3p | 1924 | ptr-mir-30a |
| 342 | ppy-miR-30a-3p | 1925 | ppy-mir-30a |
| 343 | cfa-miR-30e | 1926 | cfa-mir-30e |
| 344 | gga-miR-30a-3p | 1927 | gga-mir-30a |
| 345 | xtr-miR-30a-3p | 1928 | xtr-mir-30a |
| 346 | dre-miR-30e* | 1929 | dre-mir-30e-2 |
| 347 | mmu-miR-34a | 1930 | mmu-mir-34a |
| 348 | mmu-miR-34b-5p | 1931 | mmu-mir-34b |
| 349 | mo-miR-34a | 1932 | mn-mir-34a |
| 350 | mo-miR-34c | 1933 | mo-mir-34c |

**[Table 3-8]**

| sequence number | miRNA name | sequence number | miRNA precursor name |
|---|---|---|---|
| 351 | age-miR-34a | 1934 | age-mir-34a |
| 352 | lla-miR-34a | 1935 | lla-mir-34a |
| 353 | sla-miR-34a | 1936 | sla-mir-34a |
| 354 | mml-miR-34a | 1937 | mml-mir-34a |
| 355 | mml-miR-34c-5p | 1938 | mml-mir-34c |
| 356 | mne-miR-34a | 1939 | mne-mir-34a |
| 357 | ggo-miR-34a | 1940 | ggo-mir-34a |
| 358 | ppa-miR-34a | 1941 | ppa-mir-34a |
| 359 | ptr-miR-34a | 1942 | pfr-mir-34a |
| 360 | ppy-miR-34a | 1943 | ppy-mir-34a |
| 361 | cfa-miR-34a | 1944 | cfa-mir-34a |
| 362 | cfa-miR-34c | 1945 | cfa-mir-34c |
| 363 | mdo-miR-34a | 1946 | mdo-mir-34a |
| 364 | gga-miR-34a | 1947 | gga-mir-34a |
| 365 | gga-miR-34c | 1948 | gga-mir-34c |
| 366 | xtr-miR-34a | 1949 | xtr-mir-34a |
| 367 | bta-miR-34a | 1950 | bta-mir-34a |
| 368 | bta-miR-34b | 1951 | bta-mir-34b |
| 369 | bta-miR-34c | 1952 | bta-mir-34c |
| 370 | dre-miR-34 | 1953 | dre-mir-34 |
| 371 | dre-miR-34c | 1954 | dre-mir-34c |
| 372 | mmu-miR-449a | 1955 | mmu-mir-449a |
| 373 | mmu-miR-449c | 1956 | mmu-mir-449c |
| 374 | mo-miR-449a | 1957 | ma-mir-449a |
| 375 | mml-miR-449a | 1958 | mml-mir-449a |
| 376 | cfa-miR-449 | 1959 | cfa-mir-449 |
| 377 | mdo-miR-449 | 1960 | mdo-mir-449 |
| 378 | gga-miR-449 | 1961 | gga-mir-449 |
| 379 | xtr-miR-449 | 1962 | xtr-mir-449 |
| 380 | mmu-miR-449b | 1963 | mmu-mir-449b |
| 381 | mml-miR-449b | 1964 | mml-mir-449b |
| 382 | mmu-miR-107 | 1965 | mmu-mir-107 |
| 383 | mo-miR-107 | 1966 | mo-mir-107 |
| 384 | lla-miR-107 | 1967 | lla-mir-107 |
| 385 | mml-miR-107 | 1968 | mml-mir-107 |
| 386 | mne-miR-107 | 1969 | mne-mir-107 |
| 387 | ggo-miR-107 | 1970 | ggo-mir-107 |
| 388 | ppa-miR-107 | 1971 | ppa-mir-107 |
| 389 | ptr-miR-107 | 1972 | ptr-mir-107 |
| 390 | ppy-miR-107 | 1973 | ppy-mir-107 |
| 391 | cfa-miR-107 | 1974 | cfa-mir-107 |
| 392 | mdo-miR-107 | 1975 | mdo-mir-107 |
| 393 | gga-miR-107 | 1976 | gga-mir-107 |
| 394 | xtr-miR-107 | 1977 | xtr-mir-107 |
| 395 | bta-miR-107 | 1978 | bta-mir-107 |
| 396 | ssc-miR-107 | 1979 | ssc-mir- 07 |
| 397 | dre-miR-107 | 1980 | dre-mir-107 |
| 398 | fru-miR-107 | 1981 | fru-mir-107 |
| 399 | tni-miR-107 | 1982 | tni-mir-107 |
| 400 | ptr-miR-140 | 1983 | pfr-mir-140 |

**[Table 3-9]**

| sequence number | miRNA name | sequence number | miRNA precursor name |
|---|---|---|---|
| 401 | gga-miR-140 | 1984 | gga-mir-140 |
| 402 | xtr-miR-140 | 1985 | xtr-mir-140 |
| 403 | ssc-miR-140 | 1986 | ssc-mir-140 |
| 404 | mmu-miR-148b | 1987 | mmu-mir-148b |
| 405 | mml-miR-148a | 1988 | mml-mir-148a |
| 406 | mml-miR-148b | 1989 | mml-mir-148b |
| 407 | cfa-miR-148a | 1990 | cfa-mir-148a |
| 408 | cfa-miR-148b | 1991 | cfa-mir-148b |
| 409 | gga-miR-148a | 1992 | gga-mir-148a |
| 410 | xtr-miR-148a | 1993 | xtr-mir-148a |
| 411 | xtr-miR-448b | 1994 | xtr-mir-148b |
| 412 | bta-miR-148a | 1995 | bta-mir-148a |
| 413 | bta-miR-148b | 1996 | bta-mir-148b |
| 414 | ssc-miR-148a | 1997 | ssc-mir-148a |
| 415 | dre-miR-148 | 1998 | dre-mir-148 |
| 416 | fru-miR-148 | 1999 | fru-mir-148 |
| 417 | tni-miR-148 | 2000 | tni-mir-148 |
| 418 | mmu-miR-190 | 2001 | mmu-mir-190 |
| 419 | mo-miR-190 | 2002 | ma-mir-190 |
| 420 | mo-miR-190b | 2003 | mo-mir-190b |
| 421 | mml-miR-190a | 2004 | mml-mir-190a |
| 422 | mml-miR-190b | 2005 | mml-mir-190b |
| 423 | ggo-miR-190 | 2006 | ggo-mir-190 |
| 424 | ppa-miR-190 | 2007 | ppa-mir-190 |
| 425 | ptr-miR-190 | 2008 | ptr-mir-190 |
| 426 | gga-miR-190 | 2009 | gga-mir-190 |
| 427 | dre-miR-190 | 2010 | dre-mir-190 |
| 428 | dre-miR-190b | 2011 | dre-mir-190b |
| 429 | fru-miR-190 | 2012 | fru-mir-190 |
| 430 | tni-miR-190 | 2013 | tni-mir-190 |
| 431 | lla-miR-199a | 2014 | lla-mir-199a |
| 432 | sla-miR-199a | 2015 | sla-tnir-199a |
| 433 | mml-miR-199a | 2016 | mml-mir-199a-1 |
| | | 2017 | mml-mir-199a-2 |
| 434 | mne-miR-189a | 2018 | mne-mir-199a |
| 435 | mo-miR-199a | 2019 | ggo-mir-199a |
| 436 | ppa-miR-199a | 2020 | ppa-mir-199a |
| 437 | ptr-mir-199a | 2021 | ptr-mir-199a |
| 438 | ppy-miR-199a | 2022 | ppy-mir-199a |
| 439 | xtr-miR-199a | 2023 | xtr-mir-199a |
| 440 | dre-miR-199 | 2024 | dre-mir-199-1 |
| | | 2025 | dre-mir-199-2 |
| | | 2026 | dre-mir-199-3 |
| 441 | fru-miR-199 | 2027 | fru-mir-199-1 |
| | | 2028 | fru-mir-199-2 |
| | | 2029 | fru-mir-199-3 |
| 442 | tni-miR-199 | 2030 | tni-mir-199-1 |
| | | 2031 | tni-mir-199-2 |
| | | 2032 | tni-mir-199-3 |
| 443 | mdo-miR-199b | 2033 | mdo-mir-199b |
| 444 | xtr-miR-199b | 2034 | xtr-mir-199b |
| 445 | bta-miR-199b | 2035 | bta-mir-199b |
| 446 | gga-miR-202* | 2036 | gna-mir-202 |
| 447 | xtr-miR-202* | 2037 | xtr-mir-202-1 |
| | | 2038 | xtr-mir-202-2 |
| 448 | mmu-miR-208b | 2039 | mmu-mir-208b |
| 449 | mo-miR-208 | 2040 | mo-mir-208 |
| 450 | mml-miR-208a | 2041 | mml-mir-208a |

**[Table 3-10]**

| sequence number | miRNA name | sequence number | miRNA precursor name |
|---|---|---|---|
| 451 | mml-miR-208b | 2042 | mml-mir-208b |
| 452 | mdo-miR-208 | 2043 | mdo-mir-208 |
| 453 | xtr-miR-208 | 2044 | xtr-mir-208 |
| 454 | mmu-miR-210 | 2045 | mmu-mir-210 |
| 455 | mmu-miR-210 | 2046 | mo-mir-210 |
| 456 | mml-miR-210 | 2047 | mml-mir-210 |
| 457 | xtr-miR-210 | 2048 | xtr-mir-210 |
| 458 | dre-miR-210 | 2049 | dre-mir-210 |
| 459 | fru-miR-210 | 2050 | fru-mir-210 |
| 460 | tni-miR-210 | 2051 | tni-mir-210 |
| 461 | mnu-miR-21 | 2052 | mmu-mir-211 |
| 462 | mo-niR-211 | 2053 | mo-mir-211 |
| 463 | mml-miR-211 | 2054 | mml-mir-211 |
| 464 | mne-miR-211 | 2055 | mne-mir-211 |
| 465 | ppy-miR-211 | 2056 | ppy-mir-211 |
| 466 | gga-miR-211 | 2057 | gga-mir-211 |
| 467 | mmu-miR-214 | 2058 | mmu-mir-214 |
| 468 | mo-miR-214 | 2059 | ma-mir-214 |
| 469 | age-miR-214 | 2060 | age-mir-214 |
| 470 | sla-miR-214 | 2061 | sla-mir-214 |
| 471 | mml-miR-214 | 2062 | mml-mir-214 |
| 472 | mnr-miR-214 | 2063 | mne-mir-214 |
| 473 | ggo-miR-214 | 2064 | ggo-mir-214 |
| 474 | ppa-miR-214 | 2085 | ppa-mir-214 |
| 475 | ptr-miR-214 | 2066 | ptr-mir-214 |
| 476 | ppy-miR-214 | 2067 | ppy-mir-214 |
| 477 | mdo-miR-214 | 2068 | mdo-mir-214 |
| 478 | xtr-miR-214 | 2069 | xtr-mir-214 |
| 479 | bta-miR-214 | 2070 | bta-mir-214 |
| 480 | ssc-miR-214 | 2071 | ssc-mir-214 |
| 481 | dre-miR-214 | 2072 | dre-mir-214 |
| 482 | fru-miR-214 | 2073 | fru-mir-214 |
| 483 | tni-miR-214 | 2074 | tni-mir-214 |
| 484 | mmu-miR-218 | 2075 | mmu-mir-218-1 |
| | | 2076 | mmu-mir-218-2 |
| 485 | mo-miR-218 | 2077 | mo-mir-218-1 |
| | | 2078 | mo-mir-218-2 |
| 486 | age-miR-218 | 2079 | age-mir-218 |
| 487 | lla-miR-218 | 2080 | lla-mir-218-1 |
| | | 2081 | lla-mir-218-2 |
| 488 | sla-miR-218 | 2082 | sla-mir-218-1 |
| | | 2083 | sla-mir-218-2 |
| 489 | mml-miR-218 | 2084 | mml-mir-218-1 |
| | | 2085 | mml-mir-218-2 |
| 490 | mne-miR-218 | 2086 | mne-mir-218-1 |
| | | 2087 | mne-mir-218-2 |
| 491 | ggo-miR-218 | 2088 | ggo-mir-218 |
| 492 | ppa-miR-218 | 2089 | ppa-mir-218-1 |
| | | 2090 | ppa-mir-218-2 |
| 493 | ptr-miR-218 | 2091 | ptr-mir-218-1 |
| | | 2092 | ptr-mir-218-2 |
| 494 | ppy-miR-218 | 2093 | ppy-mir-218-1 |
| | | 2094 | ppy-mir-218-2 |
| 495 | lca-miR-218 | 2095 | lca-mir-218 |
| 496 | cfa-miR-218 | 2096 | cfa-mir-218-1 |
| | | 2097 | cfa-mir-218-2 |
| 497 | mdo-miR-218 | 2098 | mdo-mir-218 |
| 498 | gga-miR-218 | 2099 | gga-mir-218-1 |
| | | 2100 | gga-mir-218-2 |
| | | 2101 | gga-mir-218-3 |
| 499 | xtr-miR-218 | 2102 | xtr-mir-218-1 |
| | | 2103 | xtr-mir-218-2 |
| 500 | bta-miR-218 | 2104 | bta-mir-218 |

**[Table 3-11]**

| sequence number | miRHA name | sequence number | miRNA precursor name |
|---|---|---|---|
| 501 | dre-miR-218b | 2105 | dre-mir-218b |
| 502 | fru-miR-218a | 2106 | fru-mir-218a-1 |
| | | 2107 | fru-mir-218a-2 |
| 503 | fru-miR-218b | 2108 | fru-mir-218b |
| 504 | tni-miR-218a | 2109 | tni-mir-218a-1 |
| | | 2100 | tni-mir-218a-1 |
| 505 | tni-miR-218b | 2111 | tni-mir-218b |
| 506 | mml-miR-299-5p | 2112 | mml-mir-299 |
| 507 | mml-miR-325 | 2113 | mml-mir-325 |
| 508 | ssc-miR-325 | 2114 | ssc-mir-325 |
| 509 | mmu-miR-328 | 2115 | mmu-mir-328 |
| 510 | mo-miR-328 | 2116 | mo-mir-328 |
| 511 | cfa-miR-328 | 2117 | cfa-mir-328 |
| | 512 mmu-miR-329 | 2118 | mmu-mir-329 |
| 513 | mo-miR-329 | 2119 | mo-mir-329 |
| 514 | mml-miR-329 | 2120 | mnl-mir-329-1 |
| | | 2121 | mml-mir-329-2 |
| 515 | mo-miR-338 | 2122 | mo-mir-338 |
| 516 | cfa-miR-338 | 2123 | cfa-mir-838 |
| 517 | mdo-miR-338 | 2124 | mdo-mir-338 |
| 518 | xtr-miR-338 | 2125 | xtr-mir-338-1 |
| | | 2126 | xtr-mir-338-2 |
| 519 | dre-miR-338 | 2127 | dre-mir-338-1 |
| | | 2128 | dre-mir-338-2 |
| | | 2129 | dre-mir-338-3 |
| | | 2130 | dre-mir-338-4 |
| 520 | fru-miR-338 | 2131 | fru-mir-338 |
| 521 | tni-miR-338 | 2132 | tni-mir-338 |
| 522 | xtr-miR-425-5p | 2133 | xtr-mir-425 |
| 523 | mmu-miR-484 | 2134 | mmu-mir--484 |
| 524 | mo-miR-484 | 2135 | mo-mir-484 |
| 525 | mml-miR-484 | 2136 | mml-mir-484 |
| 526 | bta-miR-484 | 2137 | bta-mir-484 |
| 527 | mml-miR-485-5p | 2138 | mml-mir-485 |
| 528 | mmu-miR-486 | 2139 | mmu-mir-486 |
| 529 | mml-miR-520b | 2140 | mml-mir-520b |
| 530 | mml-miR-520c | 2141 | mml-mir-520c |
| 531 | mml-miR-520e | 2142 | mml-mir-520e |
| 532 | mml-miR-520f | 2143 | mml-mir-520f |
| 533 | mml-miR-520h | 2144 | mml-mir-520h |
| 534 | mml-miR-522 | 2145 | mml-mir-522 |
| 535 | mml-miR-573 | 2146 | mml-mir-573 |
| 536 | mml-miR-650b | 2147 | mml-mir-650b |
| 537 | mml-miR-650c | 2148 | mml-mir-650c |
| 538 | mml-miR-660 | 2149 | mml-mir-660 |
| 539 | cfa-miR-660 | 2150 | cfa-mir-660 |
| 540 | bta-miR-660 | 2151 | bta-mir-660 |
| 541 | mmu-miR-668 | 2152 | mmu-mir-668 |
| 542 | mml-miR-668 | 2153 | mml-mir-668 |
| 543 | mml-miR-675 | 2154 | mm-mir-675 |
| 544 | mml-miR-765 | 2155 | mml-mir-765 |
| 545 | mmu-miR-194 | 2156 | mmu-mir-194-1 |
| | | 2157 | mmu-mir-194-2 |
| 546 | mo-miR-194 | 2158 | mo-mir-194-1 |
| | | 2159 | mo-mir-194-2 |
| 547 | age-miR-194 | 2160 | age-mir-194 |
| 548 | mml-miR-194 | 2161 | mml-mir-194-1 |
| | | 2162 | mml-mir-194-2 |
| 549 | mme-miR-194 | 2163 | mne-mir-194 |
| 550 | ggo-miR-194 | 2164 | ggo-mir-194 |

**[Table 3-12]**

| sequence | miRNA name | sequence | miRNA precusor name |
|---|---|---|---|
| 551 | ptr-miR-194 | 2165 | ptr-mir-194 |
| 552 | ppy-miR-194 | 2166 | ppy-mir-194 |
| 553 | cfa-miR-194 | 2167 | cfa-mir-194 |
| 554 | gga-miR-194 | 2168 | gga-mir-194 |
| 555 | xtr-miR-194 | 2169 | xtr-mir-194-1 |
| | | 2170 | xtr-mir-194-2 |
| 556 | dre-miR-194b | 2171 | dre-mir-194b |
| 557 | fru-miR-194 | 2172 | fru-mir-194 |
| 558 | tni-miR-194 | 2173 | tri-mir-194 |
| 559 | cfa-miR-500 | 2174 | cfa-mir-500 |
| 560 | mmu-miR-197 | 2175 | mmu--mir-197 |
| 561 | age-miR-197 | 2176 | age-mir-197 |
| 562 | mml-miR-197 | 2177 | mml-mir-197 |
| 563 | mme-miR-197 | 2178 | mne-mir-197 |
| 564 | ppa-miR-197 | 2179 | ppa-mir-197 |
| 565 | ptr-miR-197 | 2180 | ptr-mir-197 |
| 566 | ppy-miR-197 | 2181 | ppy-mir-197 |
| 567 | cfa-miR-197 | 2182 | cfa-mir-197 |
| 568 | mmu-miR-221 | 2183 | mmu-mir-221 |
| 569 | mo-miR-221 | 2184 | mo-mir-221 |
| 570 | mml-miR-221 | 2185 | mml-mir-221 |
| 571 | ggo-miR-221 | 2186 | ggo-mir-221 |
| 572 | ppa-miR-221 | 2187 | ppa-mir-221 |
| 573 | ppy-miR-221 | 2188 | ppy-mir-221 |
| 574 | cfa-miR-221 | 2189 | cfa-mir-221 |
| 575 | mdo-miR-221 | 2190 | mdo-mir-221 |
| 576 | gga-miR-221 | 2191 | gga-mir-221 |
| 577 | xtr-miR-221 | 2192 | xtr-mir-221 |
| 578 | bta-miR-221 | 2193 | bta-mir-221 |
| 579 | dre-mir-221 | 2194 | dre-mir-221 |
| 580 | fru-miR-221 | 2195 | fru-mir-221 |
| 581 | tni-miR-221 | 2196 | tni-mir-22 |
| 582 | mmu-let-7a | 2197 | mmu-let-7a-1 |
| | | 2198 | mmu-let-7a-2 |
| 583 | mmu-let-7b | 2199 | mmu-let-7b |
| 584 | mmu-let-7c | 2200 | mmu-let-7c-1 |
| | | 2201 | mmu-let-7c-2 |
| 585 | mmu-let-7d | 2202 | mmu-let-7d |
| 586 | mmu-let-7e | 2203 | mmu-let-7e |
| 587 | mmu-let-7f | 2204 | mmu-let-7f-1 |
| | | 2205 | mmu-let-7f-2 |
| 588 | mmu-let-7i | 2206 | mmu-let-7i |
| 589 | rno-let-7a | 2207 | rno-let-7a-1 |
| | | 2208 | rno-let-7a-2 |
| 590 | rno-let-7b | 2209 | rno-let-7b |
| 591 | rno-let-7c | 2210 | rno-let-7c-1 |
| | | 2211 | rno-let-7c-2 |
| 592 | rno-let-7d | 2212 | rno-let-7d |
| 593 | rno-let-7e | 2213 | rno-let-7e |
| 594 | rno-let-7f | 2214 | rno-let-7f-1 |
| | | 2215 | rno-let-7f-2 |
| 595 | rno-let-7i | 2216 | rno-let-7i |
| 586 | nml-let-7a | 2217 | mml-let-7a-1 |
| | | 2218 | mml-let-7a-2 |
| | | 2219 | mml-let-7a-3 |
| 597 | mml-let-7b | 2220 | mm-let-7b |
| 598 | mml-llet-7c | 2221 | mml-let-7c |
| 599 | mml-let-7d | 2222 | mml-let-7d |
| 600 | mml-let-7e | 2223 | mml-let-7e |

**[Table 3-13]**

| sequence number | miRMA name | sequence number | miRNA precursor name |
|---|---|---|---|
| 601 | mml-let-7f | 2224 | mm-let-7f-1 |
| | | 2225 | mml-let-7f-2 |
| 602 | mml-lest-7g | 2226 | mml-let-7g |
| 603 | mm-let-7i | 2227 | mml-let-7i |
| 604 | cfa-let-7a | 2228 | cfa-let-7a |
| 605 | cfa-let-7c | 2229 | cfa-let-7c |
| 606 | cfa-let-7e | 2230 | cfa-let-7e |
| 607 | cfa-let-7f | 2231 | cfa-let-7f |
| 608 | cfa-let-7g | 2232 | cfa-let-7g |
| 609 | cfa-let-7j | 2233 | cfa-let-7j |
| 610 | mdo-let-7a | 2234 | mdo-let-7a-1 |
| | | 2235 | mdo-let-7a-2 |
| | | 2236 | mdo-let-7a-3 |
| 611 | mdo-let-7b | 2237 | mdo-let-76 |
| 612 | mdo-let-7d | 2238 | mdo-let-7d |
| 613 | mdo-let-7f | 2239 | mdo-let-7f-1 |
| | | 2240 | mdo-let-7f-2 |
| 614 | mdo-let-7g | 2241 | mdo-let-7g |
| 615 | mdo-let-7i | 2242 | mdo-let-7i |
| 616 | gga-let-7a | 2243 | gga-let-7a-1 |
| | | 2244 | gga-let-7a-2 |
| | | 2245 | gga-let-7a-3 |
| 617 | gga-let-7b | 2246 | gga-let-7b |
| 618 | gga-let-7c | 2247 | gga-let-7c |
| 619 | gga-let-7d | 2248 | gga-let-7d |
| 620 | gga-let-7f | 2249 | gga-let-7f |
| 621 | gga-let-7g | 2250 | gga-let-7g |
| 622 | gga-let-7i | 2251 | gga-let-7i |
| 623 | gga-let-7i | 2252 | gga-let-7j |
| 624 | gga-let-7k | 2253 | gga-let-7k |
| 625 | xtr-let-7a | 2254 | xtr-let-7a |
| 626 | xtr-let-7b | 2255 | xtr-let-7b |
| 627 | xtr-let-7c | 2256 | xtr-let-7c |
| 628 | xtr-let-7e | 2257 | xtr-let-7e-1 |
| | | 2258 | xtr-let-7e-2 |
| 629 | xtr-let-7f | 2259 | xtr-let-7f |
| 630 | xtr-let-7g | 2260 | xtr-let-7g |
| 631 | xtr-let-7i | 2261 | xtr-let-7i |
| 632 | bta-let-7a | 2262 | bta-let-7a-1 |
| | | 2263 | bta-let-7a-2 |
| | | 2264 | bta-let-7a-3 |
| 633 | bta-let-7b | 2265 | bta-let-7b |
| 634 | bta-let-7c | 2266 | bta-let-7c |
| 635 | bta-let-7d | 2267 | bta-let-7d |
| 636 | bta-let-7e | 2268 | bta-let-7e |
| 637 | bta-let-7f | 2269 | bta-let-7f-1 |
| | | 2270 | bta-let-7f-2 |
| 638 | bta-let-7g | 2271 | bta-let-7g |
| 639 | bta-let-7i | 2272 | bta-let-7i |
| 640 | ssc-let-7c | 2273 | ssc-let-7c |
| 641 | ssc-let-7f | 2274 | ssc-let-7f |
| 642 | ssc-let-7i | 2275 | ssc-let-7i |
| 643 | dre-let-7a | 2276 | dre-let-7a-1 |
| | | 2277 | dre-let-7a-2 |
| | | 2278 | dre-let-7a-3 |
| | | 2279 | dre-let-7a-4 |
| | | 2280 | dre-let-7a-5 |
| | | 2281 | dre-let-7a-6 |
| 644 | dre-let-7b | 2282 | dre-let-7b |
| 645 | dre-let-7c | 2283 | dra-let-7c-1 |
| | | 2284 | dre-lat-7c-2 |
| 646 | dre-let-7d | 2285 | dre-let-7d-1 |
| | | 2286 | dre-let-7d-2 |
| 647 | dre-let-7e | 2287 | dre-let-7e |
| 648 | dre-let-7f | 2288 | dre-let-7f |
| 649 | dre-let-7g | 2289 | dre-let-7g-1 |
| | | 2290 | dre-let-7g-2 |
| 650 | dre-let-7h | 2291 | dra-let-7h |

**[Table 3-14]**

| sequence number | miRNA name | sequence number | miRNA precursor name |
|---|---|---|---|
| 651 | dre-let-7i | 2292 | dre-let-7i |
| 652 | dre-let-7j | 2293 | dre-let-7j |
| 653 | fru-let-7a | 2294 | fru-let-7a-1 |
| | | 2295 | fru-let-7a-2 |
| | | 2296 | fru-let-7a-3 |
| 654 | fru-let-7b | 2297 | fru-let-7b |
| 655 | fru-let-7d | 2298 | fru-let-7d |
| 656 | fru-let-7e | 2299 | fru-let-7e |
| 657 | fru-let-7g | 2300 | fru-let-7g |
| 658 | fru-let-7h | 2301 | fru-let-7h |
| 659 | fru-let-7i | 2302 | fru-lat-7i |
| 660 | fru-1et-7j | 2303 | fru-let-7j |
| 661 | tni-let-7a | 2304 | tni-let-7a-1 |
| | | 2305 | tni-let-7a-2 |
| | | 2306 | tni-let-7a-3 |
| 662 | tni-let-7b | 2307 | tni-let-7b |
| 663 | tni-let-7d | 2308 | tni-let-7d |
| 664 | tni-let-7e | 2309 | tni-let-7e |
| 665 | tni-let-7g | 2310 | tni-let-7g |
| 666 | tni-let-7h | 2311 | tni-let-7h |
| 667 | tni-let-7i | 2312 | tni-let-7i |
| 668 | tni-let-7j | 2313 | tni-let-7j |
| 669 | mmu-miR-1 | 2314 | mmu-mir-1-1 |
| | | 2315 | mmu-mir-1-2 |
| | | 2316 | mm-mir-1-2-as |
| 670 | mo-miR-1 | 2317 | mo-mir-1 |
| 671 | mml-miR-1 | 2318 | mml-mir-1-1 |
| | | 2319 | mml-mir-1-2 |
| 672 | ppa-miR-1 | 2320 | ppa-mir-1 |
| 673 | cfa-miR-1 | 2321 | cfa-mir-1-1 |
| | | 2322 | cfa-mir-1-2 |
| 674 | mdo-miR-1 | 2323 | mdo-mir-1 |
| 675 | gga-miR-1a | 2324 | gga-mir-1a-1 |
| | | 2325 | gga-mir-1a-2 |
| | 676 gga-miR-1b | 2326 | gga-mir-1b |
| 677 | xtr-miR-1b | 2327 | xtr-mir-1b |
| 678 | dre-miR-1 | 2328 | dre-mir-1-1 |
| | | 2329 | dre-mir-1-2 |
| 679 | fru-miR-1 | 2330 | fru-mir1 |
| 680 | tri-miR-1 | 2331 | tni-mir-1 |
| 681 | mmu-miR-206 | 2332 | mmu-mir-206 |
| 682 | mo-miR-206 | 2333 | mo-mir-206 |
| 683 | mml-miR-206 | 2334 | mml-mir-206 |
| 684 | mne-miR-206 | 2335 | mne-mir-206 |
| 685 | ppy-miR-206 | 2336 | ppy-mir-206 |
| 686 | cfa-miR-206 | 2337 | cfa-mir-206 |
| 687 | mdo-miR-206 | 2338 | mdo-mir-206 |
| 688 | gga-miR-206 | 2339 | gga-mir-206 |
| 689 | xtr-miR-206 | 2340 | xtr-mir-206 |
| 690 | dre-miR-206 | 2341 | dre-mir206-1 |
| | | 2342 | dre-mir-206-2 |
| 691 | mmu-miR-9 | 2343 | mmu-mir-9-1 |
| | | 2344 | mmu-mir-9-2 |
| | | 2345 | mmu-mir-9-3 |
| 692 | mo-miR-9 | 2346 | mo-mir-9-1 |
| | | 2347 | mo-mir-9-2 |
| | | 2348 | mo-mir-9-3 |
| 693 | age-miR-9 | 2349 | age-mir-9 |
| 694 | lla-miR-9 | 2350 | lla-mir-9 |
| 695 | mml-miR-9 | 2351 | mml-mir-9-1 |
| | | 2352 | mml-mir-9-2 |
| | | 2353 | mml-mir-9-3 |
| 696 | mne-miR-9 | 2354 | mne-mir-9 |
| 697 | ggo-miR-9 | 2355 | ggo-mir-9 |
| 698 | ptr-miR-9 | 2356 | ptr-mir-9 |
| 699 | cfa-miR-9 | 2357 | cfa-mir-9-1 |
| | | 2358 | cfa-mir-9-2 |
| | | 2359 | cfa-mir9-3 |
| 700 | mdo-miR-9 | 2360 | mdo-mir-9-1 |
| | | 2361 | mdo-mir-9-2 |

**[Table 3-15]**

| sequence number | miRNA name | sequence number | miRNA precursor name |
|---|---|---|---|
| 701 | gga-miR-9 | 2362 | gga-mir-9-1 |
| | | 2363 | gga-mir-9-2 |
| 702 | xtr-miR-9 | 2364 | xtr-mir-9-3 |
| 703 | xtr-miR-9b | 2365 | xtr-mir-3b |
| 704 | dre-miR-9 | 2366 | dre-mir-9-1 |
| | | 2367 | dre-mir-9-2 |
| | | 2368 | dre-mir-9-3 |
| | | 2369 | dre-mir-9-4 |
| | | 2370 | dre-mir-9-5 |
| | | 2371 | dre-mir-9-6 |
| | | 2372 | dre-mir-9-7 |
| 705 | fru-miR-9 | 2373 | fru-mir-9-1 |
| | | 2374 | fru-mir-9-2 |
| | | 2375 | fru-mir-9-3 |
| | | 2376 | fru-mir-9-4 |
| 706 | tni-miR-9 | 2377 | tni-mir-9-1 |
| | | 2378 | tn-mir-9-2 |
| | | 2379 | tni-mir-9-3 |
| | | 2380 | tni-mir-9-4 |
| 707 | mmu-miR-23b | 2381 | mmu-mir-23b |
| 708 | mo-miR-23a | 2382 | mo-mir-23a |
| 709 | mo-miR-23b | 2383 | mo-mir-23b |
| 710 | age-miR-23a | 2384 | age-mir-23a |
| 711 | sla-miR-23a | 2385 | sla-mir-23a |
| 712 | sla-miR-23a | 2386 | mml-mir-23a |
| 713 | mml-miR-23b | 2387 | mml-mir-23b |
| 714 | mme-miR-23a | 2388 | mme-mir-23a |
| 715 | ggo-miR-23a | 2389 | ggo-mir-23a |
| 716 | ppa-miR-23a | 2390 | ppa-mir-23a |
| 717 | ppa-miR-28b | 2391 | ppa-mir-23b |
| 718 | ptr-miR-23a | 2392 | ptr-mir-23a |
| 719 | ptr-miR-23b | 2393 | ptr-mir-23b |
| 720 | ppy-miR-23a | 2394 | ppy-mir-23a |
| 721 | ppy-miR-23b | 2395 | ppy-mir-23b |
| 722 | lca-miR-23a | 2396 | lca-mir-23a |
| 723 | cfa-miR-23a | 2397 | cfa-mir-23a |
| 724 | cfa-miR-23b | 2398 | cfa-mir-23b |
| 725 | mdo-miR-23a | 2399 | mdo-mir-23a |
| 726 | mdo-miR-23b | 2400 | mdo-mir-23b |
| 727 | gga-miR-23b | 2401 | gga-mir-23b |
| 728 | xtr-miR-23 | 2402 | xtr-mir-23a-1 |
| | | 2403 | xtr-mir-23a-2 |
| 729 | xtr-miR-23b | 2404 | xtr-mir-23b |
| 730 | bta-miR-23a | 2405 | bta-mir-23a |
| 731 | bta-miR-23b | 2406 | bta-mir-23b |
| 732 | ssc-miR-23a | 2407 | ssc-mir-23a |
| 733 | dre-miR-23a | 2408 | dre-mir-23a-1 |
| | | 2409 | dre-mir-23a-2 |
| | | 2410 | dre-mir-23a-3 |
| 734 | dre-miR-23b | 2411 | dre-mir-23b |
| 735 | fru-miR-23a | 2412 | fru-mir-23a-1 |
| | | 2413 | fru-mir-23a-2 |
| | | 2414 | fru-mir-23a-3 |
| 736 | fru-miR-23b | 2415 | fru-mir-23b |
| 737 | tni-miR-23a | 2416 | tri-mir-23a-1 |
| | | 2417 | tni-mir-23a-2 |
| | | 2418 | tni-mir-23a-3 |
| 738 | tni-miR-23b | 2419 | tri-mir-23b |
| 739 | mmu-miR-27b | 2420 | mmu-mir-27b |
| 740 | mo-miR-27e | 2421 | mo-mir-27a |
| 741 | mo-miR-27b | 2422 | mo-mir-27b |
| 742 | mo-miR-27a | 2423 | age-mir-27a |
| 743 | sla-miR-27a | 2424 | sla-mir-27a |
| 744 | mml-miR-27a | 2425 | mml-mir-27a |
| 745 | mml-miR-27b | 2426 | mml-mir-27b |
| 746 | mme-miR-27a | 2427 | mne-mir-27a |
| 747 | ggo-miR-27a | 2428 | ggo-mir-27a |
| 748 | ppa-miR-27a | 2429 | ppa-mir-27a |
| 749 | ptr-miR-27a | 2430 | ptr-mir-27a |
| 750 | ppy-miR-27a | 2431 | ppy-mir-27a |

**[Table 3-16]**

| sequence number | miRNA name | sequence number | miRNA precursor name |
|---|---|---|---|
| 751 | lca-miR-27a | 2432 | lca-mir-27a |
| 752 | cfa-miR-27a | 2433 | cfa-mir-27a |
| 753 | cfa-miR-27b | 2434 | cfa-mir-21b |
| 754 | mdo-miR-27a | 2435 | mdo-mir-27a |
| 755 | mdo-miR-27b | 2436 | mdo-mir-27b |
| 756 | gga-miR-27b | 2437 | gga-mir-27b |
| 757 | xtr-miR-27a | 2438 | xtr-mir-27a |
| 758 | xtr-miR-27b | 2439 | xtr-mir-27b |
| 759 | xtr-miR-27c | 2440 | xtr-mir-27c-1 |
| | | 2441 | xtr-mir-27c-2 |
| 760 | bta-miR-27a | 2442 | bta-mir-27a |
| 761 | bta-miR-27b | 2443 | bta-mir-27b |
| 762 | ssc-miR-27a | 2444 | ssc-mir-27a |
| 763 | dre-miR-27a | 2445 | dre-mir-27a |
| 764 | dre-miR-27b | 2446 | dre-mir-27b |
| 765 | dre-miR-27c | 2447 | dre-mir-27c |
| 766 | dre-miR-27d | 2448 | dre-mir-27d |
| 761 | dre-miR-27e | 2449 | dre-mir-27e |
| 768 | fru-miR-27b | 2450 | fru-mir-27b |
| 769 | fru-miR-27c | 2451 | fru-mir-27c |
| 770 | fru-miR-27e | 2452 | fru-mir-27e |
| 771 | tni-miR-27b | 2453 | tni-mir-27b |
| 772 | tni-miR-27c | 2454 | tni-mir-27c |
| 773 | tni-miR-27e | 2455 | tni-mir-27e |
| 774 | mmu-miR-28 | 2456 | mmu-mir-28 |
| 775 | mo-miR-28 | 2457 | mo-mir-28 |
| 776 | age-miR-28 | 2458 | age-mir-28 |
| 777 | lla-miR-28 | 2459 | lla-mir-28 |
| 778 | sla-miR-28 | 2460 | sla-mir-28 |
| 779 | mml-miR-28 | 2461 | mml-mir-28 |
| 780 | mne-miR-28 | 2462 | mne-mir-28 |
| 781 | ggo-miR-28 | 2463 | ggo-mir-28 |
| 782 | ppa-miR-28 | 2464 | ppa-mir-28 |
| 783 | ptr-miR-28 | 2465 | ptr-mir-28 |
| 784 | ppy-miR-28 | 2466 | ppy-mir-28 |
| 785 | ssc-miR-28 | 2467 | ssc-mir-28 |
| 786 | mml-miR-31 | 2468 | mml-mir-31 |
| 787 | mne-miR-31 | 2469 | mne-mir-31 |
| 788 | ggo-miR-31 | 2470 | ggo-mir-31 |
| 789 | ppa-miR-31 | 2471 | ppa-mir-31 |
| 790 | ptr-miR-31 | 2472 | ptr-mir-31 |
| 791 | ppy-miR-31 | 2473 | ppy-mir-31 |
| 792 | dre-miR-31 | 2474 | dre-mir-31 |
| 793 | mo-miR-33 | 2475 | mo-mir-33 |
| 794 | mml-miR-33a | 2476 | mml-mir-33a |
| 795 | mml-miR-33b | 2477 | mml-mir-33b |
| 796 | mne-miR-33 | 2478 | mne-mir-33 |
| 797 | ggo-miR-33 | 2479 | ggo-mir-33 |
| 798 | ppa-miR-33 | 2480 | ppa-mir-33 |
| 799 | ptr-miR-33 | 2481 | ptr-mir-33 |
| 800 | ppy-miR-33 | 2482 | ppy-mir-33 |

**[Table 3-17]**

| sequence number | miRNA name | sequence number | miRNA precursor name |
|---|---|---|---|
| 801 | cfa-miR-33 | 2483 | cfa-mir-33 |
| 802 | gga-miR-33 | 2484 | gga-mir-33 |
| 803 | xtr-miR-33a | 2485 | xtr-mir-33a |
| 804 | xtr-miR-33b | 2486 | xtr-mir-33b |
| 805 | mmu-miR-96 | 2487 | mmu-mir-96 |
| 806 | mo-miR-96 | 2488 | mo-mir-96 |
| 807 | sla-miR-96 | 2489 | sla-mir-96 |
| 808 | mml-miR-96 | 2490 | mml-mir-96 |
| 809 | mne-miR-96 | 2491 | mne-mir-86 |
| 810 | ggo-miR-96 | 2492 | ggo-mir-96 |
| 811 | ppa-miR-96 | 2493 | ppa-mir-96 |
| 812 | ptr-miR-96 | 2494 | ptr-mir-96 |
| 813 | mdo-miR-96 | 2495 | mdo-mir-96 |
| 814 | xtr-miR-96 | 2496 | xtr-mir-96 |
| 815 | dre-miR-96 | 2497 | dre-mir-96 |
| 816 | fru-miR-96 | 2498 | fru-mir-96 |
| 817 | tni-miR-96 | 2499 | tni-mir-96 |
| 818 | mmu-miR-100 | 2500 | mmu-mir-100 |
| 819 | mo-miR-100 | 2501 | mo-mir-100 |
| 820 | age-miR-100 | 2502 | age-mir-100 |
| 821 | lla-miR-100 | 2503 | lla-mir-100 |
| 822 | sla-miR-100 | 2504 | sla-mir-100 |
| 823 | mml-miR-100 | 2505 | mml-mir-100 |
| 824 | ggo-miR-100 | 2506 | ggo-mir-100 |
| 825 | ppa-miR-100 | 2507 | ppa-nir-100 |
| 826 | ptr-miR-10 | 2508 | ptr-mir-100 |
| 827 | ppy-miR-100 | 2509 | ppy-mir-100 |
| 828 | mdo-miR-100 | 2510 | mdo-mir-100 |
| 829 | gga-miR-100 | 2511 | gga-mir-100 |
| 830 | xtr-miR-100 | 2512 | xtr-mir-100 |
| 831 | dre-miR-100 | 2513 | dre-mir-100-1 |
| | | 2514 | dre-mir-100-2 |
| 832 | fru-miR-100 | 2515 | fru-mir-100 |
| 833 | tni-miR-100 | 2516 | tri-mir-100 |
| 834 | mmu-miR-106a | 2517 | mmu-mir-106a |
| 835 | rno-miR-106b | 2518 | rno-mir-106b |
| 836 | age-miR-106a | 2519 | age-mir-106a |
| 837 | age-miR-106b | 2520 | age-mir-106b |
| 838 | lla-miR-106b | 2521 | lla-mir-106b |
| 839 | sla-miR-106a | 2522 | sla-mir-106a |
| 840 | sla-miR-106b | 2523 | sla-mir-106b |
| 841 | mml-miR-106a | 2524 | mml-mir-106a |
| 842 | mml-miR-106b | 2525 | mml-mir-106b |
| 843 | mne-miR-106a | 2526 | mne-mir-106a |
| 844 | mne-miR-106b | 2527 | mne-mir-106b |
| 845 | ggo-miR-106a | 2528 | ggo-mir-106a |
| 846 | ggo-miR-106b | 2529 | ggo-mir-106b |
| 847 | ppa-miR-106a | 2530 | ppa-mir-106a |
| 848 | ppa-miR-106b | 2531 | ppa-mir-106b |
| 849 | ptr-miR-106a | 2532 | ptr-mir-106a |
| 850 | ptr-miR-106b | 2533 | ptr-mir-106b |

**[Table 3-18]**

| sequence number | miRNA name | sequence number | miRNA precursor name |
|---|---|---|---|
| 851 | ppy-miR-106a | 2534 | ppy-mir-106a |
| 852 | ppy-miR-106b | 2535 | ppy-mir-106b |
| 853 | cfa-miR-106b | 2536 | cfa-mir-106b |
| 854 | gga-miR-106 | 2537 | gga-mir-106 |
| 855 | xtr-miR-106 | 2538 | xtr-mir-106 |
| 856 | bta-miR-106 | 2539 | bta-mir-106 |
| 857 | ssc-miR-106a | 2540 | ssc-mir-106a |
| 858 | rno-miR-126* | 2541 | rno-mir-126 |
| 859 | gga-miR-126* | 2542 | gga-mir-126 |
| 860 | xtr-miR-126* | 2543 | xtr-mir-126 |
| 861 | bta-miR-126* | 2544 | bta-mir-126 |
| 862 | dre-miR-126* | 2545 | dre-mir-126 |
| 863 | mmu-miR-127 | 2546 | mmu-mir-127 |
| 864 | rno-miR-127 | 2547 | rno-mir-127 |
| 865 | age-miR-127 | 2548 | age-mir-127 |
| 866 | lla-miR-127 | 2549 | lla-mir-127 |
| 867 | sla-miR-127 | 2550 | sla-mir-127 |
| 868 | mml-miR-127 | 2551 | mml-mir-127 |
| 869 | mne-miR-127 | 2552 | mne-mir-127 |
| 870 | ptr-miR-127 | 2553 | ptr-mir-127 |
| 871 | ppy-miR-127 | 2554 | ppy-mir-127 |
| 872 | cfa-miR-127 | 2555 | cfa-mir-127 |
| 873 | bta-miR-127 | 2556 | bta-mir-127 |
| 874 | rno-miR-128 | 2557 | rno-mir-128-1 |
| | | 2558 | rno-mir-128-2 |
| 875 | age-miR-128 | 2559 | age-mir-128 |
| 876 | sla-miR-128 | 2560 | sla-mir-128 |
| 877 | mml-miR-128a | 2561 | mml-mir-128a |
| 878 | mml-miR-128b | 2562 | mml-mir-128b |
| 879 | ppa-miR-128 | 2563 | ppa-mir-128 |
| 880 | ptr-miR-128 | 2564 | ptr-mir-128 |
| 881 | ppy-miR-128 | 2565 | ppy-mir-128 |
| 882 | 882 cfa-miR-128 | 2566 | cfa-mir-128-1 |
| | | 2567 | cfa-mir-128-2 |
| 883 | mdo-miR-128 | 2568 | mdo-mir-128 |
| 884 | gga-miR-128 | 2569 | gga-mir-128-1 |
| | | 2570 | gga-mir-128-2 |
| 885 | xtr-miR-128 | 2571 | xtr-mir-128-1 |
| | | 2572 | xtr-mir-128-2 |
| 886 | bta-miR-128 | 2573 | bta-mir-128 |
| 887 | ssc-miR-128 | 2574 | ssc-mir-128 |
| 888 | dre-miR-128 | 2575 | dre-mir-128-1 |
| | | 2576 | dre-mir-128-2 |
| 889 | fru-miR-128 | 2577 | fru-mir-128-1 |
| | | 2578 | fru-mir-128-2 |
| 890 | tni-miR-128 | 2579 | tni-mir-128-1 |
| | | 2580 | tni-mir-128-2 |
| 891 | rno-miR-129 | 2581 | rno-mir-129-1 |
| | | 2582 | rno-mir-129-2 |
| 892 | cfa-miR-129 | 2583 | cfa-mir-129-1 |
| | | 2584 | cfa-mir-129-2 |
| 893 | mdo-mir-129 | 2585 | mdo-mir-129 |
| 894 | xtr-miR-129 | 2586 | xtr-mir-129-1 |
| | | 2587 | xtr-mir-128-2 |
| 895 | dre-miR-129 | 2588 | dre-mir-129-1 |
| | | 2589 | dre-mir-129-2 |
| 896 | fru-miR-129 | 2590 | fru-mir-129-1 |
| | | 2591 | fru-mir-129-2 |
| 897 | tni-miR-129 | 2592 | tni-mir-129-1 |
| | | 2593 | tni-mir-129-2 |
| 898 | age-miR-133a | 2594 | age-mir-133a |
| 899 | lla-miR-133a | 2595 | lla-mir-133a |
| 900 | sla-miR-133a | 2596 | sla-mir-133a |

**[Table 3-19]**

| sequence number | miRNA name | sequence number | miRNA precursor name |
|---|---|---|---|
| 901 | mne-miR-133a | 2597 | mne-mir-133a |
| 902 | ggo-miR-133a | 2598 | ggo-mir-133a |
| 903 | ppa-miR-133a | 2599 | ppa-mir-133a |
| 904 | ptr-miR-133a | 2600 | ptr-mir-133a |
| 905 | mdo-miR-133a | 2601 | mdo-mir-133a |
| 906 | gga-miR-133a | 2602 | gga-mir-133a-1 |
| | | 2603 | gga-mir-133a-2 |
| 907 | gga-miR-133c | 2604 | gga-mir-133c |
| 908 | xla-miR-133a | 2605 | xla-mir-133a |
| 909 | xtr-miR-133a | 2606 | xtr-mir-133a |
| 910 | xtr-miR-133d | 2607 | xtr-mir-133d |
| 911 | gga-miR-133b | 2608 | gga-mir-133b |
| 912 | xtr-miR-133b | 2609 | xtr-mir-133b |
| 913 | mmu-miR-135b | 2610 | mmu-mir-135b |
| 914 | rno-miR-135a | 2611 | rno-mir-135a |
| 915 | rno-miR-135b | 2612 | rno-mir-135b |
| 916 | age-miR-135 | 2613 | age-mir-135-1 |
| | | 2614 | age-mir-135-2 |
| 917 | lla-miR-135 | 2615 | lla-mir-135-1 |
| | | 2616 | lla-mir-135-2 |
| 918 | mml-miR-135a | 2617 | mml-mir-135a-1 |
| | | 2618 | mml-mir-135a-2 |
| 919 | mml-miR-135b | 2619 | mml-mir-135b |
| 920 | ggo-miR-135 | 2620 | ggo-mir-135 |
| 921 | ppa-miR-135 | 2621 | ppa-mir-135-1 |
| | | 2622 | ppa-mir-135-2 |
| 922 | ptr-miR-135 | 2623 | ptr-mir-135 |
| 923 | ppy-miR-135 | 2624 | ppy-mir-135 |
| 924 | mdo-miR-135a | 2625 | mdo-mir-135a |
| 925 | mdo-miR-135b | 2626 | mdo-mir-135b |
| 926 | gga-miR-135a | 2627 | gga-mir-135a-1 |
| | | 2628 | gga-mir-135a-2 |
| | | 2629 | gga-mir-135a-3 |
| 927 | xtr-miR-135 | 2630 | xtr-mir-135-1 |
| | | 2631 | xtr-mir-135-2 |
| 928 | ssc-miR-135 | 2632 | ssc-mir-135-1 |
| | | 2633 | ssc-mir-135-2 |
| 929 | dre-miR-135a | 2634 | dre-mir-135a |
| 930 | dre-miR-135b | 2635 | dre-mir-135b |
| 931 | dre-miR-135c | 2636 | dre-mir-135c-1 |
| | | 2637 | dre-mir-135c-2 |
| | | 2638 | dre-mir-135c-3 |
| 932 | fru-miR-135a | 2639 | fru-mir-135a |
| 933 | fru-miR-135b | 2640 | fru-mir-135b |
| 934 | tni-miR-135a | 2641 | tni-mir-135a |
| 935 | tni-miR-135b | 2642 | tni-mir-135b |
| 936 | mmu-miR-136 | 2643 | mmu-mir-136 |
| 937 | rno-miR-136 | 2644 | rno-mir-136 |
| 938 | mml-miR-136 | 2645 | mml-mir-136 |
| 939 | ggo-miR-136 | 2646 | ggo-mir-136 |
| 940 | ppa-miR-136 | 2647 | ppa-mir-136 |
| 941 | ptr-miR-136 | 2648 | ptr-mir-136 |
| 942 | ppy-miR-136 | 2649 | ppy-mir-136 |
| 943 | cfa-miR-136 | 2650 | cfa-mir-136 |
| 944 | oar-miR-136 | 2651 | oar-mir-136 |
| 945 | ssc-miR-136 | 2652 | ssc-mir-136 |
| 946 | mmu-miR-142-5p | 2653 | mmu-mir-142 |
| 947 | rno-miR-142-5p | 2654 | rno-mir-142 |
| 948 | mml-miR-142-5p | 2655 | mml-mir-142 |
| 949 | xtr-miR-142-5p | 2656 | xtr-mir-142-1 |
| | | 2657 | xtr-mir-142-2 |
| 950 | dre-miR-142b-5p | 2658 | dre-mir-142b |

**[Table 3-20]**

| sequence number | miRNA name | sequence number | miRNA precusor name |
|---|---|---|---|
| 951 | fru-miR-142a | 2659 | fru-mir-142a |
| 952 | fru-miR-142b | 2660 | fru-mir-142b |
| 953 | tni-miR-142a | 2661 | tni-mir-142a |
| 954 | tni-miR-142b | 2662 | tni-mir-142b |
| 955 | mmu-miR-146a | 2663 | mmu-mir-146a |
| 956 | rno-miR-146a | 2664 | rno-mir-146a |
| 957 | mml-miR-146a | 2665 | mml-mir-146a |
| 958 | cfa-miR-146a | 2666 | cfa-mir-146a |
| 959 | gga-miR-146a | 2667 | gga-mir-146a |
| 960 | dre-miR-146a | 2668 | dre-mir-146a |
| 961 | mmu-miR-146b | 2669 | mmu-mir-146b |
| 962 | rno-miR-146b | 2670 | rno-mir-146b |
| 963 | cfa-miR-146b | 2671 | cfa-mir-146b |
| 964 | gga-miR-146b | 2672 | gga-mir-146b |
| 965 | xtr-miR-146b | 2673 | xtr-mir-146b |
| 966 | dre-miR-146b | 2674 | dre-mir-146b-1 |
| | | 2675 | dre-mir-146b-2 |
| 967 | mmu-miR-181a | 2676 | mmu-mir-181a-1 |
| | | 2677 | mmu-mir-181a-2 |
| 968 | mmu-miR-181c | 2678 | mmu-mir-181c |
| 969 | mmu-miR-181d | 2679 | mmu-mir-181d |
| 970 | rno-miR-181a | 2680 | rno-mir-181a-1 |
| | | 2681 | rno-mir-181a-2 |
| 971 | rno-miR-181b | 2682 | rno-mir-181b-1 |
| | | 2683 | rno-mir-181b-2 |
| 972 | rno-miR-181c | 2684 | rno-mir-181c |
| 973 | rno-miR-181d | 2685 | rno-mir-181d |
| 974 | lla-miR-181 a | 2686 | lla-mir-181a-1 |
| 975 | lla-miR-181b | 2687 | lla-mir-181b |
| 976 | sla-miR-181a | 2688 | sla-mir-181a-2 |
| 977 | mml-miR-181a | 2689 | mml-mir-181a-1 |
| | | 2690 | mml-mir-181a-2 |
| 978 | mml-miR-181b | 2691 | mml-mir-181b-1 |
| | | 2692 | mml-mir-181b-2 |
| 979 | mml-miR-181c | 2693 | mml-mir-181c |
| 980 | mml-miR-181d | 2694 | mml-mir-181d |
| 981 | mne-miR-181a | 2695 | mne-mir-181a-1 |
| | | 2696 | mne-mir-181a-2 |
| 982 | mne-miR-181b | 2697 | mne-mir-181b |
| 983 | ggo-miR-181a | 2698 | ggo-mir-181a-1 |
| | | 2699 | ggo-mir-181a-2 |
| 984 | ggo-miR-181b | 2700 | ggo-mir-181b |
| 985 | ggo-miR-181c | 2701 | ggo-mir-181c |
| 986 | ppa-miR-181a | 2702 | ppa-mir-181a-1 |
| | | 2703 | ppa-mir-181a-2 |
| 987 | ppa-miR-181b | 2704 | ppa-mir-181b |
| 988 | ppa-miR-181c | 2705 | ppa-mir-181c |
| 989 | ptr-miR-181a | 2706 | ptr-mir-181a-1 |
| | | 2707 | ptr-mir-181a-2 |
| 990 | ptr-miR-181b | 2708 | ptr-mir-181b |
| 991 | ptr-miR-181c | 2709 | ptr-mir-181c |
| 992 | ppy-miR-181a | 2710 | ppy-mir-181a-1 |
| 993 | ppy-miR-181b | 2711 | ppy-mir-181b |
| 994 | cfa-miR-181a | 2712 | cfa-mir-181a-1 |
| | | 2713 | cfa-mir-181a-2 |
| 995 | cfa-miR-181b | 2714 | cfa-mir-181b-1 |
| | | 2715 | cfa-mir-181b-2 |
| 996 | cfa-miR-181c | 2716 | cfa-mir-181c |
| 997 | cfa-miR-181d | 2717 | cfa-mir-181d |
| 998 | mdo-miR-181a | 2718 | mdo-mir-181a |
| 999 | mdo-miR-181b | 2719 | mdo-mir-181b |
| 1000 | mdo-miR-181c | 2720 | mdo-mir-181c |

**[Table 3-21]**

| sequence number | miRNA name | sequence number | miRNA precusor name |
|---|---|---|---|
| 1001 | gga-miR-181a | 2721 | gga-mir-181a-1 |
| | | 2722 | gga-mir-181a-2 |
| 1002 | gga-miR-181b | 2723 | gga-mir-181b-1 |
| | | 2724 | gga-mir-181b-2 |
| 1003 | xtr-miR-181a | 2725 | xtr-mir-181a-1 |
| | | 2726 | xtr-mir-181a-2 |
| 1004 | xtr-miR-181b | 2727 | xtr-mir-181b-1 |
| | | 2728 | xtr-mir-181b-2 |
| 1005 | bta-miR-181a | 2729 | bta-mir-181a |
| 1006 | bta-miR-181 b | 2730 | bta-mir-181b |
| 1007 | bta-miR-181c | 2731 | bta-mir-181c |
| 1008 | ssc-miR-181b | 2732 | ssc-mir-181b |
| 1009 | ssc-miR-181c | 2733 | ssc-mir-181c |
| 1010 | dre-miR-181a | 2734 | dre-mir-181a-1 |
| | | 2735 | dre-mir-181a-2 |
| 1011 | dre-miR-181b | 2736 | dre-mir-181b-1 |
| | | 2737 | dre-mir-181b-2 |
| 1012 | dre-miR-181c | 2738 | dre-mir-181 c |
| 1013 | fru-miR-181a | 2739 | fru-mir-181a-1 |
| | | 2740 | fru-mir-181a-2 |
| 1014 | fru-miR-181b | 2741 | fru-mir-181b-1 |
| | | 2742 | fru-mir-181b-2 |
| 1015 | tni-miR-181a | 2743 | tni-mir-181a-1 |
| | | 2744 | tni-mir-181a-2 |
| 1016 | tni-miR-181b | 2745 | tni-mir-181b-1 |
| | | 2746 | tni-mir-181b-2 |
| 1017 | xtr-miR-182* | 2747 | xtr-mir-182 |
| 1018 | dre-miR-182* | 2748 | dre-mir-182 |
| 1019 | mmu-miR-183 | 2749 | mmu-mir-183 |
| 1020 | rno-miR-183 | 2750 | rno-mir-183 |
| 1021 | sla-miR-183 | 2751 | sla-mir-183 |
| 1022 | mml-miR-183 | 2752 | mml-mir-183 |
| 1023 | mne-miR-183 | 2753 | mne-mir-183 |
| 1024 | ggo-miR-183 | 2754 | ggo-mir-183 |
| 1025 | ppa-miR-183 | 2755 | ppa-mir-183 |
| 1026 | ptr-miR-183 | 2756 | ptr-mir-183 |
| 1027 | cfa-miR-183 | 2757 | cfa-mir-183 |
| 1028 | mdo-miR-183 | 2758 | mdo-mir-183 |
| 1029 | gga-miR-183 | 2759 | gga-mir-183 |
| 1030 | xtr-miR-183 | 2760 | xtr-mir-183 |
| 1031 | ssc-miR-183 | 2761 | ssc-mir-183 |
| 1032 | dre-miR-183 | 2762 | dre-mir-183 |
| 1033 | fru-miR-183 | 2763 | fru-mir-183 |
| 1034 | tni-miR-183 | 2764 | tni-mir-183 |
| 1035 | mmu-miR-187 | 2765 | mmu-mir-187 |
| 1036 | rno-miR-187 | 2766 | rno-mir-187 |
| 1037 | mml-miR-187 | 2767 | mml-mir-187 |
| 1038 | mne-miR-187 | 2768 | mne-mir-187 |
| 1039 | ggo-miR-187 | 2759 | ggo-mir-187 |
| 1040 | ppa-miR-187 | 2770 | ppa-mir-187 |
| 1041 | ppy-miR-187 | 2771 | ppy-mir-187 |
| 1042 | mdo-miR-187 | 2772 | mdo-mir-187 |
| 1043 | gga-miR-187 | 2773 | gga-mir-187 |
| 1044 | xtr-miR-187 | 2774 | xtr-mir-187 |
| 1045 | dre-miR-187 | 2775 | dre-mir-187 |
| 1046 | fru-miR-187 | 2776 | fru-mir-187 |
| 1047 | tni-miR-181 | 2777 | tni-mir-187 |
| 1048 | mmu-miR-192 | 2778 | mmu-mir-192 |
| 1049 | rno-miR-132 | 2779 | rno-mir-192 |
| 1050 | mmi-miR-192 | 2780 | mml-mir-192 |

**[Table 3-22]**

| sequence number | miRNA name | sequence number | miRNA precusor name |
|---|---|---|---|
| 1051 | cfa-miR-192 | 2781 | cfa-mir-192 |
| 1052 | xtr-miR-192 | 2782 | xtr-mir-192 |
| 1053 | bta-miR-192 | 2783 | bta-mir-192 |
| 1054 | dre-miR-192 | 2784 | dre-mir-192 |
| 1055 | fru-miR-192 | 2785 | fru-mir-192 |
| 1056 | tni-miR-192 | 2786 | tni-mir-192 |
| 1057 | mmu-miR-215 | 2387 | mmu-mir-215 |
| 1058 | rno-miR-215 | 2788 | rno-mir-215 |
| 1059 | mml-miR-215 | 2789 | mml-mir-215 |
| 1060 | mne-miR-215 | 2790 | mne-mir-215 |
| 1061 | ggo-miR-215 | 2791 | ggo-mir-215 |
| 1062 | ptr-miR-215 | 2792 | ptr-mir-215 |
| 1063 | ppy-miR-215 | 2793 | ppy-mir-215 |
| 1064 | gga-miR-215 | 2794 | gga-mir-215 |
| 1065 | xtr-miR-215 | 2795 | xtr-mir-215 |
| 1066 | bta-miR-215 | 2796 | bta-mir-215 |
| 1067 | mmu-miR-200a* | 2797 | mmu-mir-200a |
| 1068 | mdo-miR-200a* | 2798 | mdo-mir-200a |
| 1069 | rno-miR-216a | 2799 | rno-mir-216a |
| 1070 | mml-miR-216a | 2800 | mml-mir-216a |
| 1071 | ggo-miR-216 | 2801 | ggo-mir-216 |
| 1072 | ppa-miR-216 | 2802 | ppa-mir-216 |
| 1073 | ppy-miR-216 | 2803 | ppy-mir-216 |
| 1074 | lca-miR-216 | 2804 | lca-mir-216 |
| 1075 | mdo-miR-216 | 2805 | mdo-mir-216 |
| 1076 | gga-miR-216 | 2806 | gga-mir-216 |
| 1077 | xtr-miR-216 | 2807 | xtr-mir-216 |
| 1078 | ssc-miR-216 | 2808 | ssc-mir-216 |
| 1079 | dre-miR-216a | 2809 | dre-mir-216a-1 |
| | | 2810 | dre-mir-216a-2 |
| 1080 | fru-miR-216a | 2811 | fru-mir-216a |
| 1081 | tni-miR-216a | 2812 | tni-mir-216a |
| 1082 | mmu-miR-217 | 2813 | mmu-mir-217 |
| 1083 | rno-miR-217 | 2814 | rno-mir-217 |
| 1084 | ggo-miR-217 | 2815 | ggo-mir-217 |
| 1085 | ppa-miR-217 | 2816 | ppa-mir-217 |
| 1086 | mdo-miR-217 | 2817 | mdo-mir-217 |
| 1087 | gga-miR-217 | 2818 | gga-mir-217 |
| 1088 | xtr-miR-217 | 2819 | xtr-mir-217 |
| 1089 | ssc-miR-217 | 2820 | ssc-mir-217 |
| 1090 | dre-miR-217 | 2821 | dre-mir-217-1 |
| | | 2822 | dre-mir-217-2 |
| 1091 | fru-miR-217 | 2823 | fru-mir-217 |
| 1092 | tni-miR-217 | 2824 | tni-mir-217 |
| 1093 | ggo-miR-220 | 2825 | ggo-mir-220 |
| 1094 | ppa-miR-220 | 2826 | ppa-mir-220 |
| 1095 | ptr-miR-220 | 2827 | ptr-mir-220 |
| 1096 | mmu-miR-222 | 2828 | mmu-mir-222 |
| 1097 | rno-miR-222 | 2829 | rno-mir-222 |
| 1098 | age-miR-222 | 2830 | age-mir-222 |
| 1099 | mml-miR-222 | 2831 | mml-mir-222 |
| 1100 | gga-miR-222 | 2832 | gga-mir-222 |

**[Table 3-23]**

| sequence number | miRNA name | sequence number | miRNA precursor name |
|---|---|---|---|
| 1101 | xtr-miR-222 | 2833 | xtr-mir-222 |
| 1102 | bta-miR-222 | 2834 | bta-mir-222 |
| 1103 | dre-miR-222 | 2835 | dre-mir-222 |
| 1104 | fru-miR-222 | 2836 | fru-mir-222 |
| 1105 | tni-miR-222 | 2837 | tni-mir-222 |
| 1106 | mmu-miR-223 | 2838 | mmu-mir-223 |
| 1107 | rno-miR-223 | 2839 | rno-mir-223 |
| 1108 | sla-miR-223 | 2840 | sla-mir-223 |
| 1109 | mml-miR-223 | 2841 | mml-mir-223 |
| 1110 | ggo-miR-223 | 2842 | ggo-mir-223 |
| 1111 | ppa-miR-223 | 2843 | ppa-mir-223 |
| 1112 | ptr-miR-223 | 2844 | ptr-mir-223 |
| 1113 | ppy-miR-223 | 2845 | ppy-mir-223 |
| 1114 | mdo-miR-223 | 2846 | mdo-mir-223 |
| 1115 | gga-miR-223 | 2847 | gga-mir-223 |
| 1116 | xtr-miR-223 | 2848 | xtr-mir-223 |
| 1117 | dre-miR-223 | 2849 | dre-mir-223 |
| 1118 | fru-miR-223 | 2850 | fru-mir-223 |
| 1119 | tni-miR-223 | 2851 | tni-mir-223 |
| 1120 | mmu-miR-224 | 2852 | mmu-mir-224 |
| 1121 | rno-miR-224 | 2853 | rno-mir-224 |
| 1122 | mml-miR-224 | 2854 | mml-mir-224 |
| 1123 | mne-miR-224 | 2855 | mne-mir-224 |
| 1124 | ggo-miR-224 | 2856 | ggo-mir-224 |
| 1125 | ppa-miR-224 | 2857 | ppa-mir-224 |
| 1126 | ptr-miR-224 | 2858 | ptr-mir-224 |
| 1127 | ppy-miR-224 | 2859 | ppy-mir-224 |
| 1128 | cfa-miR-224 | 2860 | cfa-mir-224 |
| 1129 | ssc-miR-224 | 2861 | ssc-mir-224 |
| 1130 | mmu-miR-302b* | 2862 | mmu-mir-302b |
| 1131 | gga-miR-302b* | 2863 | gga-mir-302b |
| 1132 | gga-miR-302c* | 2864 | gga-mir-302c |
| 1133 | mmu-miR-374 | 2865 | mmu-mir-374 |
| 1134 | rno-miR-374 | 2866 | rno-mir-374 |
| 1135 | mml-miR-374a | 2867 | mml-mir-374a |
| 1136 | mml-miR-374b | 2868 | mml-mir-374b |
| 1137 | cfa-miR-374b | 2869 | cfa-mir-374b |
| 1138 | bta-miR-374 | 2870 | bta-mir-374 |
| 1139 | rno-miR-376b-3p | 2871 | rno-mir-376b |
| 1140 | mml-miR-376a | 2872 | mml-mir-376a-1 |
| | | 2873 | mml-mir-376a-2 |
| 1141 | mml-miR-376b | 2874 | mml-mir-376b |
| 1142 | cfa-miR-376 | 2875 | cfa-mir-376-1 |
| | | 2876 | cfa-mir-376-2 |
| | | 2877 | cfa-mir-376-3 |
| 1143 | mmu-miR-409-5p | 2878 | mmu-mir-409 |
| 1144 | rno-miR-409-5p | 2879 | rno-mir-409 |
| 1145 | mml-miR-409-5p | 2880 | mml-mir-409 |
| 1146 | mmu-miR-429 | 2881 | mmu-mir-429 |
| 1147 | rno-miR-429 | 2882 | rno-mir-429 |
| 1148 | mml-miR-429 | 2883 | mml-mir-429 |
| 1149 | cfa-miR-429 | 2884 | cfa-mir-429 |
| 1150 | gga-miR-429 | 2885 | gga-mir-429 |

**[Table 3-24]**

| sequence number | miRNA name | sequence number | miRNA precursor name |
|---|---|---|---|
| 1151 | xla-miR-429 | 2886 | xla-mir-429 |
| 1152 | xtr-miR-429 | 2887 | xtr-mir-429 |
| 1153 | dre-miR-429 | 2888 | dre-mir-429 |
| 1154 | fru-miR-429 | 2889 | fru-mir-429 |
| 1155 | tni-miR-429 | 2890 | tni-mir-429 |
| 1156 | mmu-miR-200c | 2891 | mmu-mir-200c |
| 1157 | mo-miR-200b | 2892 | mo-mir-200b |
| 1158 | mo-miR-200c | 2893 | mo-mir-200c |
| 1159 | cfa-miR-200c | 2894 | cfa-mir-200c |
| 1160 | mdo-miR-200b | 2895 | mdo-mir-200b |
| 1161 | mdo-miR-200c | 2896 | mdo-mir-200c |
| 1162 | gga-miR-200b | 2897 | gga-mir-200b |
| 1163 | xtr-miR-200b | 2898 | xtr-mir-200b |
| 1164 | bta-miR-200b | 2899 | bta-mir-200b |
| 1165 | bta-miR-200c | 2900 | bta-mir-200c |
| 1166 | dre-miR-200b | 2901 | dre-mir-200b |
| 1167 | dre-miR-200c | 2902 | dre-mir-200c |
| 1168 | fru-miR-200b | 2903 | fru-mir-200b |
| 1169 | tni-miR-200b | 2904 | tni-mir-200b |
| 1170 | mmu-miR-448 | 2905 | mmu-mir-448 |
| 1171 | mo-miR-448 | 2906 | mo-mir-448 |
| 1172 | mml-miR-448 | 2907 | mml-mir-448 |
| 1173 | cfa-miR-448 | 2908 | cfa-mir-448 |
| 1174 | cfa-miR-450a | 2909 | cfa-mir-450a |
| 1175 | mmu-miR-451 | 2910 | mmu-mir-451 |
| 1176 | mo-miR-451 | 2911 | mo-mir-451 |
| 1177 | mml-miR-451 | 2912 | mml-mir-451 |
| 1178 | mdo-miR-451 | 2913 | mdo-mir-451 |
| 1179 | gga-miR-451 | 2914 | gga-mir-451 |
| 1180 | xtr-miR-451 | 2915 | xtr-mir-451 |
| 1181 | dre-miR-451 | 2916 | dre-mir-451 |
| 1182 | mmu-miR-452 | 2917 | mmu-mir-452 |
| 1183 | mmu-miR-487b | 2918 | mmu-mir-487b |
| 1184 | mo-miR-487b | 2919 | mo-mir-487b |
| 1185 | mml-miR-487b | 2920 | mml-mir-487b |
| 1186 | bta-miR-487b | 2921 | bta-mir-487b |
| 1187 | gga-miR-489 | 2922 | gga-mir-489 |
| 1188 | xtr-miR-489 | 2923 | xtr-mir-489 |
| 1189 | dre-miR-489 | 2924 | dre-mir-489 |
| 1190 | fru-miR-489 | 2925 | fru-mir-489 |
| 1191 | tni-miR-489 | 2926 | tni-mir-489 |
| 1192 | age-miR-514 | 2927 | age-mir-514 |
| 1193 | mml-miR-514 | 2928 | mml-mir-514-1 |
| | | 2929 | mml-mir-514-2 |
| 1194 | pbi-miR-514 | 2930 | pbi-mir-514 |
| 1195 | tr-miR-514 | 2931 | ptr-mir--514-1 |
| | | 2932 | ptr-mir-514-2 |
| | | 2933 | ptr-mir-514-3 |
| | | 2934 | ptr-mir-514-4 |
| 1196 | ssy-miR-514 | 2935 | ssy-mir-514 |
| 1197 | mml-miR-518c | 2936 | mml-mir-518c |
| 1198 | mml-miR-518d | 2937 | mml-mir-518d |
| 1199 | mml-miR-542-3p | 2938 | mmu-mir-542 |
| 1200 | mo-miR-542-3p | 2939 | mo-mir-542 |

**[Table 3-25]**

| sequence number | miRNA name | sequence number | miRNA precursor name |
|---|---|---|---|
| 1201 | mml-miR-542-3p | 2940 | mml-mir-542 |
| 1202 | mmu-miR-544 | 2941 | mmu-mir-544 |
| 1203 | mml-miR-544 | 2942 | mml-mir-544 |
| 1204 | mml-miR-601 | 2943 | mml-mir-601 |
| 1205 | mml-miR-609 | 2944 | mml-mir-609 |
| 1206 | mml-miR-632 | 2945 | mml-mir-532 |
| 1207 | mml-miR-644 | 2946 | mml-mir-644 |
| 1208 | mmu-miR-365 | 2947 | mmu-mir-365-1 |
| | | 2948 | mmu-mir-365-2 |
| 1209 | mo-miR-365 | 2949 | mo-mir-365 |
| 1210 | mml-miR-365 | 2950 | mml-mir-365-1 |
| | | 2951 | mml-mir-365-2 |
| 1211 | cfa-miR-365 | 2952 | cfa-mir-365-1 |
| | | 2953 | cfa-mir-365-2 |
| 1212 | mdo-miR-365 | 2954 | mdo-mir-365 |
| 1213 | gga-miR-365 | 2955 | gga-mir-365-1 |
| | | 2956 | gga-mir-365-2 |
| 1214 | xtr-miR-365 | 2957 | xtr-mir-365-1 |
| 1215 | bta-miR-365 | 2958 | bta-mir-365 |
| 1216 | dre-miR-365 | 2959 | dre-mir-365-1 |
| | | 2960 | dre-mir-365-2 |
| | | 2961 | dre-mir-365-3 |
| | | 2962 | dre-mir-365-4 |
| 1217 | fru-miR-365 | 2963 | fru-mir-365 |
| 1218 | tni-miR-365 | 2964 | tni-mir-365 |
| 1219 | mmu-miR-142-3p | 2653 | mmu-mir-142 |
| 1220 | mo-miR-142-3p | 2654 | mo-mir-142 |
| 1221 | mml-miR-142-3p | 2655 | mml-mir-142 |
| 1222 | gga-miR-142-3p | 2965 | gga-mir-142 |
| 1223 | xtr-miR-142-3p | 2656 | xtr-mir-142-1 |
| | | 2657 | xtr-mir-142-2 |
| 1224 | mmu-miR-200a | 2797 | mmu-mir-200a |
| 1225 | mo-miR-200a | 2966 | mo-mir-200a |
| 1226 | mml-miR-200a | 2967 | mml-mir-200a |
| 1227 | mdo-miR-200a | 2798 | mdo-mir-200a |
| 1228 | gga-miR-200a | 2968 | gga-mir-200a |
| 1229 | xtr-miR-200a | 2969 | xtr-mir-200a |
| 1230 | bta-miR-200a | 2970 | bta-mir-200a |
| 1231 | dra-miR-200a | 2971 | dre-mir-200a |
| 1232 | fru-miR-200a | 2972 | fru-mir-200a |
| 1283 | tni-miR-200a | 2973 | tni-mir-200a |
| 1234 | mmu-miR-361 | 2974 | mmu-mir-361 |
| 1235 | mo-miR-361 | 2975 | mo-mir-361 |
| 1236 | cfa-miR-361 | 2976 | cfa-mir-361 |
| 1237 | bta-miR-361 | 2977 | bta-mir-361 |
| 1238 | hsa-miR-15a | 2978 | hsa-mir-15a |
| 1239 | hsa-miR-15b | 2979 | hsa-mir-15b |
| 1240 | hsa-miR-424 | 2980 | hsa-mir-424 |
| 1241 | hsa-miR-497 | 2981 | hsa-mir-497 |
| 1242 | mmu-miR-15a | 2982 | mmu-mir-15a |
| 1243 | mmu-miR-15b | 2983 | mmu-mir-15b |
| 1244 | mmu-miR-322 | 2984 | mmu-mir-322 |
| 1245 | mmu-miR-497 | 2985 | mmu-mir-497 |
| 1246 | mo-miR-15b | 2986 | mo-mir-15b |
| 1247 | mo-miR-322 | 2987 | mo-mir-322 |
| 1248 | mo-miR-497 | 2988 | mo-mir-497 |
| 1249 | age-miR-15a | 2989 | age-mir-15a |
| 1250 | age-miR-15b | 2990 | age-mir-15b |

**[Table 3-26]**

| sequence number | miRNA name | sequence number | miRNA precursor name |
|---|---|---|---|
| 1251 | IIa-miR-15a | 2991 | IIa-mir-15a |
| 1252 | IIa-miR-15b | 2992 | IIa-mir-15b |
| 1253 | sla-miR-15a | 2993 | sla-mir-15a |
| 1254 | mml-miR-15a | 2994 | mml-mir-15a |
| 1255 | mml--miR-15b | 2995 | mml-mir-15b |
| 1256 | mml-miR-424 | 2996 | mml-mir-424 |
| 1257 | mml-miR-497 | 2997 | mml-mir-497 |
| 1258 | mne-miR-15a | 2998 | mne-mir-15a |
| 1259 | mne-miR-15b | 2999 | mne-mir-15b |
| 1260 | ggo-miR-15a | 3000 | ggo-mir-15a |
| 1261 | ggo-miR-15b | 3001 | ggo-mir-15b |
| 1262 | ppa-miR-15a | 3002 | ppa-mir-15a |
| 1263 | ppa-miR-15b | 3003 | ppa-mir-15b |
| 1264 | ptr-miR-15a | 3004 | ptr-mir-15a |
| 1265 | ptr-miR-15b | 3005 | ptr-mir-15b |
| 1266 | ppy-miR-15a | 3006 | ppy-mir-15a |
| 1267 | ppy-miR-15b | 3007 | ppy-mir-15b |
| 1268 | lca-miR-15a | 3008 | lca-mir-15a |
| 1269 | cfa-miR-15a | 3009 | cfa-mir-15a |
| 1270 | cfa-miR-15b | 3010 | cfa-mir-15b |
| 1271 | cfa-miR-497 | 3011 | cfa-mir-497 |
| 1272 | mdo-miR-15a | 3012 | mdo-mir-15a |
| 1273 | gga-miR-15a | 3013 | gga-mir-15a |
| 1274 | gga-miR-15b | 3014 | gga-mir-15b |
| 1275 | xtr-miR-15a | 3015 | xtr-mir-15a |
| 1276 | xtr-miR-15b | 3016 | xtr-mir-15b |
| 1277 | xtr-miR-15c | 3017 | xtr-mir-15c |
| 1278 | bta-miR-15a | 3018 | bta-mir-15a |
| 1279 | bta-miR-15b | 3019 | bta-mir-15b |
| 1280 | bta-miR-497 | 3020 | bta-mir-497 |
| 1281 | dre-miR-15a | 3021 | dre-mir-15a-1 |
| | | 3022 | dre-mir-15a-2 |
| 1282 | dre-miR-15b | 3023 | dre-mir-15b |
| 1283 | dre-miR-457a | 3024 | dre-mir-457a |
| 1284 | dre-miR-457b | 3025 | dre-mir-457b |
| 1285 | fru-miR-15a | 3026 | fru-mir-15a |
| 1285 | tni-miR-15a | 3027 | tni-mir-15a |
| 1287 | mmu-miR-20b* | 1786 | mmu-mir-20b |
| 1288 | mo-miR-20b-3p | 3028 | mo-mir-20b |
| 1289 | dre-miR-20a* | 1809 | dre-mir-20a |
| 1290 | hsa-miR-590-5p | 3029 | hsa-mir-590 |
| 1291 | mmu-miR-590-5p | 3030 | mmu-mir-590 |
| 1292 | mml-miR-590-5p | 3031 | mml-mir-590 |
| 1293 | hsa-miR-92a | 3032 | hsa-mir-92a-1 |
| | | 3033 | hsa-mir-92a-2 |
| 1294 | hsa-miR-92b | 3034 | hsa-mir-92b |
| 1295 | hsa-miR-363 | 3035 | hsa-mir-363 |
| 1296 | hsa-miR-367 | 3036 | hsa-mir-367 |
| 1297 | mmu-miR-92a | 3037 | mml-mir-92a-1 |
| | | 3038 | mmu-mir-92a-2 |
| 1298 | mnu-miR-92b | 3039 | mmu-mir-92b |
| 1299 | mmu-miR-363 | 3040 | mmu-mir-363 |
| 1300 | mmu-miR-367 | 3041 | mmu-mir-367 |

**[Table 3-27]**

| sequence number | miRNA name | sequence number | miRNA precursor name |
|---|---|---|---|
| 1301 | mo-miR-92a | 3042 | mo-mir-92a-1 |
| | | 3043 | mo-mir-92a-2 |
| 1302 | mo-miR-92b | 3044 | mo-mir-92b |
| 1303 | mo-miR-363 | 3045 | mo-mir-363 |
| 1304 | age-miR-92 | 3046 | age-mir-92-1 |
| | | 3047 | age-mir-92-2 |
| 1305 | IIa-miR-92 | 3048 | IIa-mir-92-1 |
| | | 3049 | IIa-mir-92-2 |
| 1306 | sla-miR-92 | 3050 | sla-mir-92-1 |
| | | 3051 | sla-mir-92-2 |
| 1307 | mml-miR-92a | 3052 | mml-mir-92a-1 |
| | | 3053 | mml-mir-92a-2 |
| 1308 | mml-miR-92b | 3054 | mml-mir-92b |
| 1309 | mml-miR-363 | 3055 | mml-mir-363 |
| 1310 | mml-miR-367 | 3056 | mml-mir-367 |
| 1311 | mne-miR-92 | 3057 | mne-mir-92 |
| 1312 | ggo-miR-32 | 3058 | ggo-mir-92-1 |
| | | 3059 | ggo-mir-92-2 |
| 1313 | ppa-mir-92 | 3060 | ppa-mir-92-1 |
| | | 3061 | ppa-mir-92-2 |
| 1314 | ptr-miR-92 | 3062 | ptr-mir-92-1 |
| | | 3063 | ptr-mir-92-2 |
| 1315 | ppy-mir-92 | 3064 | ppy-mir-92-1 |
| | | 3065 | ppy-mir-92-2 |
| 1316 | lca-miR-92 | 3066 | lca-mir-92-1 |
| | | 3067 | lca-mir-92-2 |
| 1317 | cfa-miR-92a | 3068 | cfa-mir-92a-1 |
| | | 3069 | cfa-mir-92a-2 |
| 1318 | cfa-miR-92b | 3070 | cfa-mir-92b |
| 1319 | cfa-miR-363 | 3071 | cfa-mir-363 |
| 1320 | mdo-miR-92 | 3072 | mdo-mir-92 |
| 1321 | gga-miR-92 | 3073 | gga-mir-92 |
| 1322 | gga-miR-367 | 3074 | gga-mir-367 |
| 1323 | xtr-miR-92a | 3075 | xtr-mir-92a-1 |
| | | 3076 | xtr-mir-92a-2 |
| 1324 | xtr-miR-92b | 3077 | xtr-mir-92b |
| 1325 | xtr-miR-363-3p | 3078 | xtr-mir-363 |
| 1326 | xtr-miR-367 | 3079 | xtr-mir-367 |
| 1327 | bta-miR-32 | 3080 | bta-mir-92 |
| 1328 | dre-miR-92a | 3081 | dre-mir-92a-1 |
| | | 3082 | dre-mir-92a-2 |
| 1329 | dre-miR-92b | 3083 | dre-mir-92b |
| 1330 | dre-miR-363 | 3084 | dre-mir-363 |
| 1331 | fru-miR-92 | 3085 | fru-mir-92-1 |
| | | 3086 | fru-mir-92-2 |
| 1332 | tni-miR-92 | 3087 | tni-mir-92-1 |
| | | 3088 | tni-mir-92-2 |
| 1333 | hsa-miR-26a | 3089 | hsa-mir-26a-1 |
| | | 3090 | hsa-mir-26a-2 |
| 1334 | hsa-miR-1297 | 3091 | hsa-mir-1297 |
| 1335 | hsa-miR-30d* | 3092 | hsa-mir-30d |
| 1336 | hsa-miR-30e* | 3093 | hsa-mir-30e |
| 1337 | hsa-miR-34c-5p | 3094 | hsa-mir-34c |
| 1338 | mmu-miR-699 | 3095 | mmu-mir-699 |
| 1339 | hsa-miR-103 | 3096 | hsa-mir-103-1 |
| | | 3097 | hsa-mir-103-2 |
| 1340 | mmu-miR-103 | 3098 | mmu-mir-103-1 |
| | | 3099 | mmu-mir-103-2 |
| 1341 | mo-miR-103 | 3100 | mo-mir-103-1 |
| | | 3101 | mo-mir-103-2 |
| 1342 | age-miR-103 | 3102 | age-mir-103 |
| 1343 | lla-miR-103 | 3103 | lla-mir-103 |
| 1344 | mml-miR-103 | 3104 | mml-mir-103-1 |
| | | 3105 | mml-mir-103-2 |
| 1345 | mne-miR-103 | 3106 | mne-mir-103 |
| 1346 | ggo-miR-103 | 3107 | ggo-mir-103 |
| 1347 | ppa-miR-103 | 3108 | ppa-mir-103 |
| 1348 | ptr-miR-103 | 3109 | ptr-mir-103 |
| 1349 | ppy-miR-103 | 3110 | ppy-mir-103 |
| 1350 | cfa-miR-103 | 3111 | cfa-mir-103 |

**[Table 3-28]**

| sequence number | miRNA name | sequence number | miRNA precursor name |
|---|---|---|---|
| 1351 | mdo-miR-103 | 3112 | mdo-mir-103-1 |
| | | 3113 | mdo-mir-103-2 |
| 1352 | gga-miR-103 | 3114 | gga-mir-103-1 |
| | | 3115 | gga-mir-103-2 |
| 1353 | xtr-miR-103 | 3116 | xtr-mir-103-1 |
| | | 3117 | xtr-mir-103-2 |
| 1354 | bta-miR-103 | 3118 | bta-mir-103-1 |
| | | 3119 | bta-mir-103-2 |
| 1355 | ssc-miR-103 | 3120 | ssc-mir-103 |
| 1356 | dre-miR-103 | 3121 | dre-mir-103 |
| 1357 | fru-miR-103 | 3122 | fru-mir-103 |
| 1358 | tni-miR-103 | 3123 | tni-mir-103 |
| 1359 | hsa-miR-148a | 3124 | hsa-mir-148a |
| 1360 | hsa-miR-152 | 3125 | hsa-mir-152 |
| 1361 | mmu-miR-152 | 3126 | mmu-mir-152 |
| 1362 | mo-miR-152 | 3127 | mo-mir-152 |
| 1363 | mml-miR-152 | 3128 | mml-mir-152 |
| 1364 | cfa-miR-152 | 3129 | cfa-mir-152 |
| 1365 | mdo-miR-152 | 3130 | mdo-mir-152 |
| 1366 | dre-miR-152 | 3131 | dre-mir-152 |
| 1367 | fru-miR-152 | 3132 | fru-mir-152 |
| 1368 | tni-miR-152 | 3133 | tni-mir-1 52 |
| 1369 | hsa-miR-190b | 3134 | hsa-mir-190b |
| 1370 | hsa-miR-208b | 3135 | hsa-mir-208b |
| 1371 | dre-miR-736 | 3136 | dre-mir-736 |
| 1372 | hsa-miR-204 | 3137 | hsa-mir-204 |
| 1373 | mmu-miR-204 | 3138 | mmu-mir-204 |
| 1374 | mo-miR-204 | 3139 | mo-mir-204 |
| 1375 | sla-miR-204 | 3140 | sla-mir-204 |
| 1376 | mml-miR-204 | 3141 | mml-mir-204 |
| 1377 | mne-miR-204 | 3142 | mne-mir-204 |
| 1378 | ggo-miR-204 | 3143 | ggo-mir-204 |
| 1379 | ppa-miR-204 | 3144 | ppa-mir-204 |
| 1380 | ptr-miR-204 | 3145 | ptr-mir-204 |
| 1381 | ppy-miR-204 | 3146 | ppy-mir-204 |
| 1382 | cfa-miR-204 | 3147 | cfa-mir-204 |
| 1383 | mdo-miR-204 | 3148 | mdo-mir-204 |
| 1384 | gga-miR-204 | 3149 | gga-mir-204-1 |
| | | 3150 | gga-mir-204-2 |
| 1385 | xtr-miR-204 | 3151 | xtr-mir-204-1 |
| | | 3152 | xtr-mir-204-2 |
| 1386 | bta-miR-204 | 3153 | bta-mir-204 |
| 1387 | ssc-miR-204 | 3154 | ssc-mir-204 |
| 1388 | dre-miR-204 | 3155 | dre-mir-204-1 |
| | | 3156 | dre-mir-204-2 |
| | | 3157 | dre-mir-204-3 |
| 1389 | fru-miR-204 | 3158 | fru-mir-204 |
| 1390 | tni-miR-204a | 3159 | tni-mir-204a |
| 1391 | tni-miR-204b | 3160 | tni-mir-204b |
| 1392 | mmu-miR-761 | 3161 | mmu-mir-761 |
| 1393 | hsa-miR-362-3p | 1667 | hsa-mir-362 |
| 1394 | mmu-miR-362-3p | 3162 | mmu-mir-362 |
| 1395 | mml-miR-362-3p | 3163 | mml-mir-362 |
| 1396 | mmu-miR-489 | 3164 | mmu-mir-489 |
| 1397 | dre-miR-731 | 3165 | dre-mir-731 |
| 1398 | mo-miR-344-5p | 3166 | mo-mir-344-1 |
| 1399 | hsa-miR-33b* | 1634 | hsa-mir-33b |
| 1400 | hsa-miR-519e | 3167 | hsa-mir-519e |

**[Table 3-29]**

| sequence number | miRNA name | sequence number | miRNA precursor name |
|---|---|---|---|
| 1401 | hsa-miR-519e* | 3167 | hsa-mir-519e |
| 1402 | hsa-miR-302a | 3168 | hsa-mir-302a |
| 1403 | hsa-miR-302b | 1665 | hsa-mir-302b |
| 1404 | hsa-miR-302c | 1666 | hsa-mir-302c |
| 1405 | hsa-miR-302d | 3169 | hsa-mir-302d |
| 1406 | hsa-miR-302e | 3170 | hsa-mir-302e |
| 1407 | hsa-miR-372 | 3171 | hsa-mir-372 |
| 1408 | hsa-miR-373 | 1668 | hsa-mir-373 |
| 1409 | hsa-miR-520a-3p | 3172 | hsa-mir-520a |
| 1410 | hsa-miR-520b | 3173 | hsa-mir-520b |
| 1411 | hsa-miR-520c-3p | 3174 | hsa-mir-520c |
| 1412 | hsa-miR-520e | 3175 | hsa-mir-520e |
| 1413 | mmu-miR-291a-3p | 3176 | mmu-mir-291a |
| 1414 | mmu-miR-294 | 3177 | mmu-mir-294 |
| 1415 | mmu-miR-295 | 3178 | mmu-mir-295 |
| 1416 | mmu-miR-302a | 3179 | mmu-mir-302a |
| 1417 | mmu-miR-302b | 2862 | mmu-mir-302b |
| 1418 | mmu-miR-302d | 3180 | mmu-mir-302d |
| 1419 | mo-miR-291a-3p | 3181 | mo-mir-291a |
| 1420 | age-miR-93 | 3182 | age-mir-93 |
| 1421 | lla-miR-93 | 3183 | lla-mir-93 |
| 1422 | sla-miR-93 | 3184 | sla-mir-93 |
| 1423 | mml-miR-93 | 3185 | mml-mir-93 |
| 1424 | mml-miR-302a | 3186 | mml-mir-302a |
| 1425 | mml-miR-302b | 3187 | mml-mir-302b |
| 1426 | mml-miR-302c | 3188 | mml-mir-302c |
| 1427 | mml-miR-302d | 3189 | mml-mir-302d |
| 1428 | mml-miR-372 | 3190 | mml-mir-372 |
| 1429 | mml-miR-373 | 3191 | mml-mir-373 |
| 1430 | mml-miR-519a | 3192 | mml-mir-519a |
| 1431 | mne-miR-93 | 3193 | mne-mir-93 |
| 1432 | ggo-miR-93 | 3194 | ggo-mir-93 |
| 1433 | ppa-miR-93 | 3195 | ppa-mir-93 |
| 1434 | ptr-miR-93 | 3196 | ptr-mir-93 |
| 1435 | ppy-miR-93 | 3197 | ppy-mir-93 |
| 1436 | cfa-miR-106a | 3198 | cfa-mir-106a |
| 1437 | mdo-miR-93 | 3199 | mdo-mir-93 |
| 1438 | mdo-miR-302a | 3200 | mdo-mir-302a |
| 1439 | mdo-miR-302b | 3201 | mdo-mir-302b |
| 1440 | mdo-miR-302c | 3202 | mdo-mir302c |
| 1441 | mdo-miR-302d | 3203 | mdo-mir-302d |
| 1442 | gga-miR-302b | 2863 | gga-mir-302b |
| 1443 | gga-miR-302c | 2864 | gga-mir-302c |
| 1444 | gga-miR-302d | 3204 | gga-mir-302d |
| 1445 | xla-miR-427 | 3205 | xla-mir-427 |
| 1446 | xla-miR-428 | 3206 | xla-mir-428 |
| 1447 | xtr-miR-93a | 3207 | xtr-mir-93a |
| 1448 | xtr-miR-302 | 3208 | xtr-mir-302 |
| 1449 | xtr-miR-428 | 3209 | xtr-mir-428 |
| 1450 | dre-miR-430a | 3210 | dre-mir-430a-1 |
| | | 3211 | dre-mir-430a-2 |
| | | 3212 | dre-mir-431a-3 |
| | | 3213 | dre-mir-430a-4 |
| | | 3214 | dre-mir-430a-5 |
| | | 3215 | dre-mir-430a-6 |
| | | 3216 | dre-mir-430a-7 |
| | | 3217 | dre-mir-430a-8 |
| | | 3218 | dre-mir-430a-9 |
| | | 3219 | dre-mir-430a-10 |
| | | 3220 | dre-mir-430a-11 |
| | | 3221 | dre-mir-430a-12 |
| | | 3222 | dre-mir-430a-13 |
| | | 3223 | dre-mir-430a-14 |
| | | 3224 | dre-mir-430a-15 |
| | | 3225 | dre-mir-430a-16 |
| | | 3226 | dre-mir-430a-17 |
| | | 3227 | dre-mir-430a-18 |
| | | 3228 | dre-mir-430a-19 |
| | | 3229 | dre-mir-430a-20 |
| | | 3230 | dre-mir-430a-21 |
| | | 3231 | dre-mir-430a-22 |
| | | 3232 | dre-mir-430a-23 |

**[Table 3-30]**

| sequence number | miRNA name | sequence number | miRNA precursor name |
|---|---|---|---|
| 1451 | dre-miR-430b | 3233 | dre-mir-430b-1 |
| | | 3234 | dre-mir-430b-2 |
| | | 3235 | dre-mir-430b-3 |
| | | 3236 | dre-mir-430b-4 |
| | | 3237 | dra-mir-430b-5 |
| | | 3238 | dre-mir-430b-6 |
| | | 3239 | dre-mir-430b-7 |
| | | 3240 | dre-mir-430b-8 |
| | | 3241 | dre-mir-431-9 |
| | | 3242 | dre-mir-430b-10 |
| | | 3243 | dre-mir-430b-11 |
| | | 3244 | dre-mir-430b-12 |
| | | 3245 | dre-mir-430b-13 |
| | | 3246 | dre-mir-430b-14 |
| | | 3247 | dre-mir-430b-15 |
| | | 3248 | dre-mir-430b-16 |
| | | 3249 | dre-mir-430b-17 |
| | | 3250 | dre-mir-430b-18 |
| | | 3251 | dre-mir-430b-18 |
| | | 3252 | dre-mir-430b-20 |
| | | 3253 | dre-mir-430b-21 |
| | | 3254 | dre-mir-430b-22 |
| | | 3255 | dre-mir-430b-23 |
| 1452 | dre-miR-430c | 3256 | dre-mir-430c-1 |
| | | 3257 | dre-mir-430c-2 |
| | | 3258 | dra-mir-430c-3 |
| | | 3259 | dre-mir-430c-4 |
| | | 3260 | dre-mir-430c-5 |
| | | 3261 | dre-mir-430c-6 |
| | | 3262 | dre-mir-430c-7 |
| | | 3263 | dre-mir-430c-8 |
| | | 3264 | dre-mir-430c-9 |
| | | 3265 | dre-mir-430c-10 |
| | | 3266 | dre-mir-430c-11 |
| | | 3267 | dre-mir-430c-12 |
| | | 3268 | dre-mir-430c-13 |
| | | 3269 | dre-mir-430c-14 |
| | | 3270 | dre-mir-430c-15 |
| | | 3271 | dre-mir-430c-16 |
| | | 3272 | dre-mir-430c-17 |
| | | 3273 | dre-mir-430c-18 |
| | | 3274 | dre-mir-430c-19 |
| | | 3275 | dre-mir-430c-20 |
| | | 3276 | dre-mir-430c-21 |
| 1453 | dre-miR-430i | 3277 | dre-mir-430i-1 |
| | | 3278 | dre-mir-430i-2 |
| | | 3279 | dre-mir-430i-3 |
| 1454 | dre-miR-430j | 3280 | dre-mir-430j |
| 1455 | mmu-miR-302c | 3281 | mmu-mir-302c |
| 1456 | gga-miR-302a | 3282 | gga-mir-302a |
| 1457 | hsa-miR-520g | 3283 | hsa-mir-520g |
| 1458 | mml-miR-518e | 3284 | mr-mir-518e |
| 1459 | hsa-miR-524-3p | 1688 | hsa-mir-524 |
| 1460 | hsa-miR-1285 | 3285 | hsa-mir-1285-3 |
| | | 3286 | hsa-mir-1285-2 |
| 1461 | hsa-miR-541 | 3287 | hsa-mir-541 |
| 1462 | hsa-let-7a | 3288 | hsa-let-7a-1 |
| | | 3289 | hsa-let-7a-2 |
| | | 3290 | hsa-let-7a-3 |
| 1463 | hsa-let-7b | 3291 | hsa-let-7b |
| 1464 | hsa-let-7c | 3292 | hsa-let-7c |
| 1465 | hsa-let-7d | 3293 | hsa-let-7d |
| 1466 | hsa-let-7d | 3294 | hsa-let-7f-1 |
| | | 3295 | hsa-let-7f-2 |
| 1467 | hsa-let-7g | 3296 | hse-let-7g |
| 1468 | hsa-let-7i | 3297 | hse-let-7i |
| 1469 | hsa-miR-98 | 3298 | hsa-mir-98 |
| 1470 | mmu-miR-98 | 3299 | mmu-mir-98 |
| 1471 | mo-miR-98 | 3300 | mo-mir-98 |

**[Table 3-31]**

| sequence number | miRNA name | sequence number | miRNA precursor name |
|---|---|---|---|
| 1472 | age-miR-98 | 3301 | age-mir-98 |
| 1473 | mml-miR-98 | 3302 | mml-mir-98 |
| 1474 | ggo-miR-98 | 3303 | ggo-mir-98 |
| 1475 | ppa-miR-98 | 3304 | ppa-mir-98 |
| 1476 | ptr-miR-98 | 3305 | ptr-mir-98 |
| 1477 | ppy-miR-98 | 3306 | ppy-mir-98 |
| 1478 | cfa-miR-98 | 3307 | cfa-mir-98 |
| 1479 | xtr-miR-98 | 3308 | xtr-mir-98 |
| 1480 | bta-miR-98 | 3309 | bta-mir-98 |
| 1481 | hsa-miR-23a | 3310 | hsa-mir-23a |
| 1482 | hsa-miR-130a* | 3311 | hsa-mir-130a |
| 1483 | hsa-miR-27a | 3312 | hsa-mir-27a |
| 1484 | hsa-miR-708 | 3313 | hsa-mir-708 |
| 1485 | mmu-miR-708 | 3314 | mmu-mir-708 |
| 1486 | mo-miR-708 | 3315 | mo-mir-708 |
| 1481 | cfa-miR-708 | 3316 | cfa-mir-708 |
| 1488 | hsa-miR-33a | 3317 | hsa-mir-33a |
| 1489 | hsa-miR-1271 | 3318 | hsa-mir-1271 |
| 1490 | age-miR-507 | 3319 | age-mir-507 |
| 1491 | cfa-miR-1271 | 3320 | cfa-mir-1271 |
| 1492 | hsa-miR-99a | 3321 | hsa-mir-99a |
| 1493 | hsa-miR-99b | 3322 | hsa-mir-99b |
| 1494 | mmu-miR-99a | 3323 | mmu-mir-99a |
| 1495 | mm-miR-99b | 3324 | mmu--mir-99b |
| 1496 | mo-miR-99a | 3325 | mo-mir-99a |
| 1497 | mo-miR-99b | 3326 | mo-mir-99b |
| 1498 | Ila-miR-99a | 3327 | Ila-mir-99a |
| 1499 | mmi-miR-99a | 3328 | mml-mir-99a |
| 1500 | mml-miR-99b | 3329 | mml-mir-99b |
| 1501 | mne-miR-99a | 3330 | mne-mir-99a |
| 1502 | ggo-miR-99a | 3331 | ggo-mir-99a |
| 1503 | ppa-miR-99a | 3332 | ppa-mir-99a |
| 1504 | ptr-miR-99a | 3333 | ptr-mir-89a |
| 1505 | ppy-miR-99a | 3334 | ppy-mir-99a |
| 1506 | cfa-miR-99a | 3335 | cfa-mir-99a-1 |
| | | 3336 | cfa-mir-99a-2 |
| 1507 | cfa-miR-99b | 3337 | cfa-mir-99b |
| 1508 | gga-miR-99a | 3338 | gga-mir-99a |
| 1509 | xtr-miR-99 | 3339 | xtr-mir-99 |
| 1510 | bta-miR-99a | 3340 | bta-mir-99a |
| 1511 | bta-miR-99b | 3341 | bta-mir-99b |
| 1512 | ssc-miR-99b | 3342 | ssc-mir-99b |
| 1513 | dre-miR-99 | 3343 | dre-mir-99-1 |
| | | 3344 | dre-mir-99-2 |
| 1514 | hsa-miR-128 | 3345 | hsa-mir-128-1 |
| | | 3346 | hsa-mir-128-2 |
| 1515 | dre-miR-722 | 3347 | dre-mir-722 |
| 1516 | hsa-miR-135a | 3348 | hsa-mir-135a-1 |
| | | 3349 | hsa-mir-135a-2 |
| 1517 | hsa-miR-181b | 3350 | hsa-mir-181b-1 |
| | | 3351 | hsa-mir-181b-2 |
| 1518 | hsa-miR-181c | 3352 | hsa-mir-181c |
| 1519 | hsa-miR-181d | 3353 | hsa-mir-181d |
| 1520 | hsa-miR-200b* | 3354 | hsa-mir-200b |
| 1521 | hsa-miR-302d* | 3169 | hsa-mir-302d |
| 1522 | hsa-miR-616* | 3355 | hsa-mir-616 |
| 1523 | mmu-miR-294* | 3177 | mmu-mir-294 |
| 1524 | hsa-miR-374b | 3356 | hsa-mir-374b |
| 1525 | hsa-miR-376b | 3357 | hsa-mir-376b |
| 1526 | hsa-miR-200b | 3354 | hsa-mir-200b |
| 1527 | mml-miR-548a | 3358 | mml-mir-548a |
| 1528 | hsa-miR-517a | 1590 | hsa-mir-517a |

**[Table 3-32]**

| sequence number | miRHA name | sequence number | miRNA precursor name |
|---|---|---|---|
| 1529 | hsa-miR-518a-3p | 3359 | hsa-mir-518a-I |
| | | 3360 | hsa-mir-518a-2 |
| 1530 | hsa-miR-518b | 3361 | hsa-mir-518b |
| 1531 | hsa-miR-518d-3p | 3362 | hsa-mir-518d |
| 1532 | hsa-miR-518f | 3363 | hsa-mir-518f |
| 1533 | hsa-miR-520d-5p | 1593 | hsa-mir-520d |
| 1534 | mml-miR-518a-5p | 3364 | mml-mir-518a |
| 1535 | mml-miR-520d-5p | 3365 | mml-mir-520d |
| 1536 | hsa-miR-200c* | 1675 | hsa-mir-200c |
| 1537 | nmu-miR-200c* | 2891 | mmu-mir-200c |
| 1538 | mml-miR-580 | 3366 | mml-mir-580 |
| 1539 | hsa-miR-141 | 3367 | hsa-mir-141 |
| 1540 | mmu-miR-141 | 3368 | mmu-mir-141 |
| 1541 | mo-miR-141 | 3369 | mo-mir-141 |
| 1542 | mdo-miR-141 | 3370 | mdo-mir-141 |
| 1543 | dre-miR-141 | 3371 | dre-mir-141 |
| 3372 | hsa-miR-29a | 3742 | hsa-mir-29a |
| 3373 | hsa-miR-29b | 3743 | hsa-mir-29b-1 |
| | | 3744 | hsa-mir-29b-2 |
| 3374 | hsa-miR-30a-5p | 1557 | hsa-mir-30a |
| 3375 | hsa-miR-30b | 3745 | hsa-mir-30b |
| 3376 | hsa-miR-105 | 3746 | hsa-mir-105-1 |
| | | 3747 | hsa-mir-105-2 |
| 3377 | hsa-miR-124a | 3748 | hsa-mir-124-1 |
| | | 3749 | hsa-mir-124-2 |
| | | 3750 | hsa-mir-124-3 |
| 3378 | hsa-miR-128a | 3345 | hsa-mir-128-1 |
| 3379 | hsa-miR-150 | 3751 | hsa-mir-150 |
| 3380 | hsa-miR-154 | 3752 | hsa-mir-154 |
| 3381 | hsa-miR-299-3p | 1575 | hsa-mir-299 |
| 3382 | hsa-miR-380-5p | 3753 | hsa-mir-380 |
| 3383 | hsa-miR-383 | 3754 | hsa-mir-383 |
| 3384 | hsa-miR-411 | 3755 | hsa-mir-411 |
| 3385 | hsa-miR-423 | 3756 | hsa-mir-423 |
| 3386 | hsa-miR-433 | 3757 | hsa-mir-433 |
| 3387 | hsa-miR-454-3p | 3758 | hsa-mir-454 |
| 3388 | hsa-miR-501 | 3759 | hsa-mir-501 |
| 3389 | hsa-miR-504 | 3760 | hsa-mir-504 |
| 3390 | hsa-miR-506 | 3761 | hsa-mir-506 |
| 3391 | hsa-miR-507 | 3762 | hsa-mir-507 |
| 3392 | hsa-miR-508 | 3763 | hsa-mir-508 |
| 3393 | hsa-miR-511 | 3764 | hsa-mir-511-1 |
| | | 3765 | hsa-mir-511-2 |
| 3394 | hsa-miR-512-3p | 3766 | hsa-mir-512-1 |
| | | 3767 | hsa-mir-512-2 |
| 3395 | hsa-miR-527 | 3768 | hsa-mir-527 |
| 3396 | hsa-miR-562 | 3769 | hsa-mir-562 |
| 3397 | hsa-miR-567 | 3770 | hsa-mir-567 |
| 3398 | hsa-miR-589 | 3771 | hsa-mir-589 |
| 3399 | hsa-miR-597 | 3772 | hsa-mir-597 |
| 3400 | hsa-miR-605 | 3773 | hsa-mir-605 |
| 3401 | hsa-miR-619 | 3774 | hsa-mir-619 |
| 3402 | hsa-miR-629 | 3775 | hsa-mir-629 |
| 3403 | hsa-miR-640 | 3776 | hsa-mir-640 |

**[Table 3-33]**

| sequence number | miRNA name | sequence number | miRNA precursor name |
|---|---|---|---|
| 3404 | hsa-miR-767-5p | 3777 | hsa-mir-767 |
| 3405 | hsa-miR-370-5p | 3778 | hsa-mir-770 |
| 3406 | hsa-miR-802 | 3779 | hsa-mir-802 |
| 3407 | xtr-miR-16c | 3780 | xtr-mir-16c |
| 3408 | mmu-miR-17* | 3781 | mmu-mir-17 |
| 3409 | cfa-miR-17 | 3782 | cfa-mir-17 |
| 3410 | dre-miR-17a* | 3783 | dre-mir-17a-1 |
| | | 3784 | dre-mir-17a-2 |
| 3411 | xla-miR-18 | 3785 | xla-mir-18 |
| 3412 | mmu-miR-20a | 3786 | mmu-mir-20a |
| 3413 | mo-miR-20b-5p | 3028 | mo-mir-20b |
| 3414 | xtr-miR-24a | 3787 | xtr-mir-24a |
| 3415 | mmu-miR-26a | 3788 | mmu-mir-26a-1 |
| | | 3789 | mmu-mir-26a-2 |
| 3416 | mmu-miR-30a* | 3790 | mmu-mir-30a |
| 3417 | mmu-miR-30e* | 3791 | mmu-mir-30e |
| 3418 | mo-miR-30a* | 3792 | mo-mir-30a |
| 3419 | mmu-miR-34c | 3793 | mmu-mir-34c |
| 3420 | mmu-miR-148a | 3794 | mmu-mir-148a |
| 3421 | mo-miR-148b-3p | 3795 | mo-mir-148b |
| 3422 | mmu-miR-190b | 3796 | mmu-mir-190b |
| 3423 | mmu-miR-199a-5p | 3797 | mmu-mir-199a-1 |
| | | 3798 | mmu-mir-199a-2 |
| 3424 | mmu-miR-199b* | 3799 | mmu-mir-199b |
| 3425 | mo-miR-199a-5p | 3800 | mo-mir-199a |
| 3426 | mml-miR-199a-5p | 2016 | mml-mir-199a-1 |
| | | 2017 | mml-mir-199a-2 |
| 3427 | gga-miR-199 | 3801 | gga-mir-199-1 |
| | | 3802 | gga-mir-199-2 |
| 3428 | bta-miR-199a-5p | 3803 | bta-mir-199a |
| 3429 | mmu-miR-208a | 3804 | mmu-mir-208a |
| 3430 | dre-miR-218a | 3805 | dre-mir-218a-1 |
| | | 3806 | dre-mir-218a-2 |
| 3431 | mmu-miR-299* | 3807 | mmu-mir-299 |
| 3432 | mo-miR-299 | 3808 | mo-mir-299 |
| 3433 | mmu-miR-325* | 3809 | mmu-mir-325 |
| 3434 | mo-miR-325-5p | 3810 | mo-mir-325 |
| 3435 | mmu-miR-338-3p | 3811 | mmu-mir-338 |
| 3436 | mml-miR-338-3p | 3812 | mml-mir-338 |
| 3437 | mmu-miR-425 | 3813 | mmu-mir-425 |
| 3438 | mo-miR-425 | 3814 | mo-mir-425 |
| 3439 | mml-miR-425 | 3815 | mml-mir-425 |
| 3440 | cfa-miR-425 | 3816 | cfa-mir-425 |
| 3441 | mmu-miR-485 | 3817 | mmu-mir-485 |
| 3442 | mo-miR-4B5 | 3818 | mo-mir-485 |
| 3443 | cfa-miR-485 | 3819 | cfa-mir-485 |
| 3444 | mml-miR-486-5p | 3820 | mml-mir-486 |
| 3445 | mmu-miR-488* | 3821 | mmu-mir-488 |
| 3446 | mml-miR-520a | 3822 | mml-mir-520a |
| 3447 | mml-miR-520d-3p | 3365 | mml-mir-520d |
| 3448 | mml-miR-520g | 3823 | mml-mir-520g |
| 3449 | mml-miR-650a | 3824 | mml-mir-650a-1 |
| | | 3825 | mml-mir-650a-2 |
| 3450 | mml-miR-654-5p | 3826 | mml-mir-654 |
| 3451 | mmu-miR-675-5p | 3827 | mmu-mir-675 |
| 3452 | dre-miR-194a | 3828 | dre-mir-194a |
| 3453 | mmu-let-7g | 3829 | mmu-let-7g |
| 3454 | xtr-miR-1a | 3830 | xtr-mir-1a-1 |
| | | 3831 | xtr-mir-1a-2 |

**[Table 3-34]**

| sequence number | miRNA name | sequence number | miRNA precursor name |
|---|---|---|---|
| 3455 | xtr-miR-9a | 3832 | xtr-mir-9a-1 |
| | | 3833 | xtr-mir-9a-2 |
| 3456 | ssc-miR-9-1 | 3834 | ssc-mir-9-1 |
| | | 3835 | ssc-mir-9-2 |
| 3457 | ssc-miR-9-2 | 3834 | ssc-mir-9-1 |
| | | 3835 | ssc-mir-9-2 |
| 3458 | mmu-miR-23a | 3836 | mmu-mir-23a |
| 3459 | mmu-miR-27a | 3837 | mmu-mir-27a |
| 3460 | xtr-miR-31b | 3838 | xtr-mir-31b |
| 3461 | mmu-miR-33 | 3839 | mmu-mir-33 |
| 3462 | mmu-miR-106b | 3840 | mmu-mir-106b |
| 3463 | mmu-miR-126-5p | 3841 | mmu-mir-126 |
| 3464 | cfa-miR-126 | 3842 | cfa-mir-126 |
| 3465 | mmu-miR-128 | 3843 | mmu-mir-128-1 |
| | | 3844 | mmu-mir-128-2 |
| 3466 | mmu-miR-129-5p | 3845 | mmu-mir-129-1 |
| | | 3846 | mmu-mir-129-2 |
| 3467 | mml-miR-129-5p | 3847 | mml-mir-129 |
| 3468 | xtr-miR-133c | 3848 | xtr-mir-133c |
| 3469 | mmu-miR-135a | 3849 | mmu-mir-135a-1 |
| | | 3850 | mmu-mir-135a-2 |
| 3470 | bta-miR-142 | 3851 | bta-mir-142 |
| 3471 | dre-miR-142a-5p | 3852 | dre-mir-142a |
| 3472 | mml--miR-146b-5p | 3853 | mml-mir-146b |
| 3473 | xtr-miR-146 | 3854 | xtr-mir-146 |
| 3474 | mmu-miR-181b | 3855 | mmu-mir-181b-1 |
| | | 3856 | mmu-mir-181 b-2 |
| 3475 | fru-miR-182 | 3857 | fru-mir-182 |
| 3476 | tni-miR-182 | 3858 | tni-mir-182 |
| 3477 | mmu-miR-200b* | 3859 | mmu-mir-200b |
| 3478 | mmu-miR-216a | 3860 | mmu-mir-216a |
| 3479 | mdo-miR-222a | 3861 | mdo-mir-222a |
| 3480 | mmu-miR-296-5p | 3862 | mmu-mir-296 |
| 3481 | mo-miR-296* | 3863 | mo-mir-296 |
| 3482 | rnml-miR-296-5p | 3864 | mml-mir-296 |
| 3483 | mmu-miR-302c* | 3281 | mmu-mir-302c |
| 3484 | mmu-miR-362-5p | 3162 | mmu-mir-362 |
| 3485 | mml-miR-362-5p | 3163 | mml-mir-362 |
| 3486 | cfa-miR-374a | 3865 | cfa-mir-374a |
| 3487 | mmu-mR-376b | 3866 | mmu-mir-376b |
| 3488 | mmu-miR-378* | 3867 | mmu-mir-378 |
| 3489 | mo-miR-378* | 3868 | mo-mir-378 |
| 3490 | mmu-miR-200b | 3859 | mmu-mir-200b |
| 3491 | mo-miR-450a | 3869 | mo-mir-450a |
| 3492 | cfa-miR-487 | 3870 | cfa-mir-487 |
| 3493 | mml-miR-517a | 3871 | mml-mir-517a |
| 3494 | mml-miR-518b | 3872 | mml-mir-518b |
| 3495 | cfa-miR-542 | 3873 | cfa-mir-542 |
| 3496 | mdo-miR-142 | 3874 | mdo-mir-142 |
| 3497 | cfa-miR-142a-3p | 3852 | dre-mir-142a |
| 3498 | mml-miR-361-5p | 3875 | mml-mir-361 |
| 3499 | mmu-miR-29a | 3876 | mmu-mir-29a |
| 3500 | mmu-miR-29b | 3877 | mmu-mir-29b-1 |
| | | 3878 | mmu-mir-29b-2 |
| 3501 | mmu-miR-29c | 3879 | mmu-mir-29c |
| 3502 | mo-miR-29a | 3880 | mo-mir-29a |

**[Table 3-35]**

| sequence number | miRHA name | sequence number | miRNA precursor name |
|---|---|---|---|
| 3503 | mo-miR-29b | 3881 | mo-mir-29b-1 |
| | | 3882 | mo-mir-29b-2 |
| 3504 | mo-miR-29c | 3883 | mo-mir-29c |
| 3505 | age-miR-29b | 3884 | age-mir-29b |
| 3506 | Ila-miR-29b | 3885 | Ila-mir-29b |
| 3507 | sla-miR-29b | 3886 | sla-mir-29b |
| 3508 | mml-miR-29b | 3887 | mml-mir-29b-1 |
| | | 3888 | mml-mir-29b-2 |
| 3509 | mml-miR-29c | 3889 | mml-mir-29c |
| 3510 | mne-miR-29b | 3890 | mne-mir-29b |
| 3511 | ggo-miR-29b | 3891 | ggo-mir-29b-1 |
| | | 3892 | ggo-mir-29b-2 |
| 3512 | ppa-miR-29b | 3893 | ppa-mir-29b-1 |
| | | 3894 | ppa-mir-29b-2 |
| 3513 | ptr-miR-29b | 3895 | ptr-mir-29b-1 |
| | | 3896 | ptr-mir-29b-2 |
| 3514 | ppy-miR-29b | 3897 | ppy-mir-29b-1 |
| | | 3898 | ppy-mir-29b-2 |
| 3515 | cfa-miR-29a | 3899 | cfa-mir-29a |
| 3516 | cfa-miR-29b | 3900 | cfa-mir-29b-1 |
| | | 3901 | cfa-mir-29b-2 |
| 3517 | cfa-miR-29c | 3902 | cfa-mir-29c |
| 3518 | mdo-miR-29a | 3903 | mdo-mir-29a |
| 3519 | mdo-miR-29b | 3904 | mdo-mir-29b |
| 3520 | gga-miR-29a | 3905 | gga-mir-29a |
| 3521 | gga-miR-29b | 3906 | gga-mir-29b-1 |
| | | 3907 | gga-mir-29b-2 |
| 3522 | gga-miR-29c | 3908 | gga-mir-29c |
| 3523 | xtr-miR-29a | 3909 | xtr-mir-29a |
| 3524 | xtr-miR-29b | 3910 | xtr-mir-29b |
| 3525 | xtr-miR-29c | 3911 | xtr-mir-29c |
| 3526 | xtr-miR-29d | 3912 | xtr-mir-29d |
| 3527 | bta-miR-29b | 3913 | bta-mir-29b |
| 3528 | bta-miR-29c | 3914 | bta-mir-29c |
| 3529 | ssc-miR-29b | 3915 | ssc-mir-29b |
| 3530 | ssc-miR-29c | 3916 | ssc-mir-29c |
| 3531 | dre-miR-29a | 3917 | dre-mir-29a-1 |
| | | 3918 | dre-mir-29a-2 |
| 3532 | dre-miR-29b | 3919 | dre-mir-29b-1 |
| | | 3920 | dre-mir-29b-2 |
| | | 3921 | dre-mir-29b-3 |
| 3533 | fru-miR-29a | 3922 | fru-mir-29a-1 |
| | | 3923 | fru-mir-29a-2 |
| 3534 | fru-miR-29b | 3924 | fru-mir-29b-1 |
| | | 3925 | fru-mir-29b-2 |
| 3535 | tni-miR-29a | 3926 | tni-mir-29a-1 |
| | | 3927 | tni-mir-29a-2 |
| 3536 | tni-miR-29b | 3928 | tni-mir-29b-1 |
| | | 3929 | tni-mir-29b-2 |
| 3537 | mmu-miR-30a | 3790 | mmu-mir-30a |
| 3538 | mmu-miR-30b | 3930 | mmu-mir-30b |
| 3539 | mmu-miR-30c | 3931 | mmu-mir-30c-1 |
| | | 3932 | mmu--mir-30c-2 |
| 3540 | mmu-miR-30d | 3933 | mmu-mir-30d |
| 3541 | mmu-miR-30e | 3791 | mmu-mir-30e |

**[Table 3-36]**

| sequence number | miRNA name | sequence number | miRNA precursor name |
|---|---|---|---|
| 3542 | mo-miR-30a | 3792 | mo-mir-30a |
| 3543 | mo-miR-30b-5p | 3934 | mo-mir-30b |
| 3544 | mo-miR-30c | 3935 | mo-mir-30c-1 |
| | | 3936 | mo-mir-30c-2 |
| 3545 | mo-miR-30d | 1919 | mo-mir-30d |
| 3546 | mo-miR-30e | 1920 | mo-mir-30e |
| 3547 | age-miR-30b | 3937 | age-mir-30b |
| 3548 | lla-miR-30b | 3938 | lla-mir-30b |
| 3549 | lla-miR-30c | 3939 | lla-mir-30c |
| 3550 | mml-miR-30a-5p | 1921 | mml-mir-30a |
| 3551 | mml-miR-30b | 3940 | mml-mir-30b |
| 3552 | mml-miR-30c | 3941 | mml-mir-30c-1 |
| | | 3942 | mml-mir-30c-2 |
| 3553 | mml-miR-30d | 3943 | mml-mir-30d |
| 3554 | mml-miR-30e | 3944 | mml-mir-30e |
| 3555 | mne-miR-30b | 3945 | mne-mir-30b |
| 3556 | mne-miR-30c | 3946 | mne-mir-30c |
| 3557 | mne-miR-30d | 3947 | mne-mir-30d |
| 3558 | ggo-miR-30a-5p | 1922 | ggo-mir-30a |
| 3559 | ggo-miR-30b | 3948 | go-mir-30b |
| 3560 | ggo-miR-30d | 3949 | mo-mir-30d |
| 3561 | ppa-miR-30a-5p | 1923 | ppa-mir-30a |
| 3562 | ppa-miR-30b | 3950 | ppe-mir-30b |
| 3563 | ppa-miR-30d | 3951 | ppa-mir-30d |
| 3564 | ptr-miR-30a-5p | 1924 | ptr-mir-30a |
| 3565 | ptr-miR-30b | 3952 | ptr-mir-30b |
| 3566 | ptr-miR-30c | 3953 | ptr-mir-30c |
| 3567 | ptr-miR-30d | 3954 | ptr-mir-30d |
| 3568 | ppy-miR-30a-5p | 1925 | ppy-mir-30a |
| 3569 | cfa-miR-30a | 3955 | cfa-mir-30a |
| 3570 | cfa-miR-30b | 3956 | cfa-mir-30b |
| 3571 | cfa-miR-30c | 3957 | cfa-mir-30c-1 |
| | | 3958 | cfa-mir-30c-2 |
| 3572 | cfa-miR-30d | 3959 | cfa-mir-30d |
| 3573 | mdo-miR-30a | 3960 | mdo-mir-30a |
| 3574 | gga-miR-30a-5p | 1927 | gga-mir-30a |
| 3575 | gga-miR-30b | 3961 | gga-mir-30b |
| 3576 | gga-miR-30c | 3962 | gga-mir-30c-1 |
| | | 3963 | gga-mir-30c-2 |
| 3577 | gga-miR-30d | 3964 | gga-mir-30d |
| 3578 | gga-miR-30e | 3965 | gga-mir-30e |
| 3579 | xtr-miR-30a-5p | 1928 | xtr-mir-30a |
| 3580 | xtr-miR-30b | 3966 | xtr-mir-30b |
| 3581 | xtr-miR-30c | 3987 | xtr-mir-30c-1 |
| | | 3968 | xtr-mir-30c-2 |
| 3582 | xtr-miR-30d | 3969 | xtr-mir-30d |
| 3583 | xtr-miR-30e | 3970 | xtr-mir-30e |
| 3584 | bta-miR-30a-5p | 3971 | bta-mir-30a |
| 3585 | bta-miR-30b | 3972 | bta-mir-30b |
| 3586 | bta-miR-30c | 3973 | bta-mir-30c |
| 3587 | bta-miR-30d | 3974 | bta-mir-30d |
| 3588 | bta-miR-30e-5p | 3975 | bta-mir-30e |
| 3589 | ssc-miR-30c | 3976 | ssc-mir-30c |
| 3590 | dre-miR-30a | 3977 | dre-mir-30a |
| 3591 | dre-miR-30b | 3978 | dre-mir-30b |
| 3592 | de-miR-30c | 3979 | dre-mir-30c |

**[Table 3-37]**

| sequence number | miRRA name | sequence number | miRNA precursor name |
|---|---|---|---|
| 3593 | dre-miR-30d | 3980 | dre-mir-30d |
| 3594 | dre-miR-30e | 1929 | dre-mir-30e-2 |
| 3595 | fru-miR-30b | 3981 | fru-mir-30b |
| 3596 | fru-miR-30c | 3982 | fru-mir-30c |
| 3597 | fru-miR-30d | 3983 | fru-mir-30d |
| 3598 | tni-miR-30b | 3984 | tni-mir-30b |
| 3599 | tni-miR-30c | 3985 | tni-mir-30c |
| 3600 | tni-miR-30d | 3986 | tni-mir-30d |
| 3601 | lla-miR-105 | 3987 | lla-mir-105 |
| 3602 | sla-miR-105 | 3988 | sla-mir-105 |
| 3603 | mml-miR-105 | 3989 | mml-mir-105-1 |
| | | 3990 | mml-mir-105-2 |
| 3604 | mne-miR-105 | 3991 | mne-mir-105 |
| | 3605 ggo-miR-105 | 3992 | ggo-mir-105 |
| 3606 | ppa-miR-105 | 3993 | ppa-mir-105 |
| 3607 | ptr-miR-105 | 3994 | ptr-mir-105 |
| 3608 | ppy-miR-105 | 3995 | ppy-mir-105 |
| 3609 | ssc-miR-105-1 | 3996 | ssc-mir-105-1 |
| | | 3997 | ssc-mir-105-2 |
| 3610 | ssc-miR-105-2 | 3996 | ssc-mir-105-1 |
| | | 3997 | ssc-mir-105-2 |
| 3611 | age-miR-124a | 3998 | age-mir-124a |
| 3612 | lla-miR-124a | 3999 | lla-mir-124a |
| 3613 | mml-miR-124a | 4000 | mml-mir-124a-2 |
| | | 4001 | mml-mir-124a-2 |
| 3614 | ggo-miR-124a | 4002 | ggo-mir-124a |
| 3615 | ppa-miR-124a | 4003 | ppa-mir-124a |
| 3616 | ptr-miR-124a | 4004 | ptr-mir-124a |
| 3617 | ppy-miR-124a | 4005 | ppy-mir-124a |
| 3618 | mdo-miR-124a | 4006 | mdo-mir-124a-1 |
| | | 4007 | mdo-mir-124a-2 |
| | | 4008 | mdo-mir-124a-3 |
| 3619 | gga-miR-124a | 4009 | gga-mir-124a |
| 3620 | gga-miR-124b | 4010 | gga-mir-124b-1 |
| | | 4011 | gga-mir-124b-2 |
| 3621 | xtr-miR-124 | 4012 | xtr-mir-124 |
| 3622 | bta-miR-124a | 4013 | bta-mir-124a |
| 3623 | ssc-miR-124a | 4014 | ssc-mir-124a |
| 3624 | mmu-miR-150 | 4015 | mmu-mir-150 |
| 3625 | mo-miR-150 | 4016 | mo-mir-150 |
| 3626 | mml-miR-150 | 4017 | mml-mir-150 |
| 3627 | cfa-miR-150 | 4018 | cfa-mir-150 |
| 3628 | xtr-miR-150 | 4019 | xtr-mir-150 |
| 3629 | bta-miR-150 | 4020 | bta-mir-150 |
| 3630 | dre-miR-150 | 4021 | dre-mir-150 |
| 3631 | mmu-miR-154 | 4022 | mmu-mir-154 |
| 3632 | mo-miR-154 | 4023 | mo-mir-154 |
| 3633 | mml-miR-154 | 4024 | mml-mir-154 |
| 3634 | mne-miR-154 | 4025 | mne-mir-154 |
| 3635 | ggo-miR-154 | 4026 | ggo-mir-154 |
| 3636 | ppa-miR-154 | 4027 | ppa-mir-154 |
| 3637 | ptr-miR-154 | 4028 | ptr-mir-154 |
| 3638 | ppy-miR-154 | 4029 | ppy-mir-154 |
| 3639 | mmu-miR-299 | 3807 | mmu-mir-299 |
| 3640 | mmi-miR-299-3p | 2112 | mml-mir-299 |
| 3641 | bta-miR-380-5p | 4030 | bta-mir-380 |
| 3642 | mmu-miR-383 | 4031 | mmu-mit-383 |
| 3643 | mo-miR-383 | 4032 | mo-mir-383 |

**[Table 3-38]**

| sequence number | miRNA name | sequence number | miRNA precursor name |
|---|---|---|---|
| 3644 | mml-miR-383 | 4033 | mml-mir-383 |
| 3645 | cfa-miR-383 | 4034 | cfa-mir-383 |
| 3646 | mdo-miR-383 | 4035 | mdo-mir-383 |
| 3647 | gga-miR-383 | 4036 | gga-mir-383 |
| 3648 | xb-miR-383 | 4037 | xtr-mir-383 |
| 3649 | mmu-miR-411 | 4038 | mmu-mir-411 |
| 3650 | mo-miR-411 | 4039 | mo-mir-411 |
| 3651 | mml-miR-411 | 4040 | mml-mir-411 |
| 3652 | mmu-miR-423-3p | 4041 | mmu-mir-423 |
| 3653 | mo-miR-423 | 4042 | mo-mir-423 |
| 3654 | mml-miR-423-3p | 4043 | mml-mir-423 |
| 3655 | mmu-miR-433 | 4044 | mmu-mir-433 |
| 3656 | mo-miR-433 | 4045 | mo-mir-433 |
| 3657 | mml-miR-433 | 4046 | mml-mir-433 |
| 3658 | cfa-miR-433 | 4047 | cfa-mir-433 |
| 3659 | mml-miR-454 | 4048 | mml-mir-454 |
| 3660 | dre-miR-454a | 4049 | dre-mir-454a |
| 3661 | dre-miR-454b | 4050 | dre-mir-454b |
| 3662 | mmu-miR-501-5p | 4051 | mmu-mir-501 |
| 3663 | mo-miR-501 | 4052 | mo-mir-501 |
| 3664 | mml-miR-501 | 4053 | mml-mir-501 |
| 3665 | mmu-miR-504 | 4054 | mmu-mir-504 |
| 3666 | mml-miR-504 | 4055 | mml-mir-504 |
| 3667 | mml-miR-506 | 4056 | mml-mir-506 |
| 3668 | pbi-miR-506 | 4057 | pbi-mir-506 |
| 3669 | ptr-miR-506 | 4058 | ptr-mir-506 |
| 3670 | ssy-miR-506 | 4059 | ssy-mir-506 |
| 3671 | mml-miR-507 | 4060 | mml-mir-507 |
| 3672 | pbi-miR-507 | 4061 | pbi-mir-507 |
| 3673 | ptr-miR-507 | 4062 | ptr-mir-507 |
| 3674 | ssy-miR-507 | 4063 | ssy-mir-507 |
| 3675 | ptr-miR-508 | 4064 | ptr-mir-508 |
| 3676 | ssy-miR-508 | 4065 | ssy-mir-508 |
| 3677 | mml-miR-512-3p | 4066 | mml-mir-512-1 |
| | | 4067 | mml-mir-512-2 |
| 3678 | mml-miR-562 | 4068 | mml-mir-562 |
| 3679 | mml-miR-597 | 4069 | mml-mir-597 |
| 3680 | mml-miR-605 | 4070 | mml-mir-605 |
| 3681 | mml-miR-640 | 4071 | mml-mir-640 |
| 3682 | mml-miR-767-5p | 4072 | mml-mir-767 |
| 3683 | mml-miR-770-5p | 4073 | mml-mir-770 |
| 3684 | mml-miR-802 | 4074 | mml-mir-802 |
| 3685 | hsa-miR-29c | 4075 | hsa-mir-29c |
| 3686 | hsa-miR-30c | 4076 | hsa-mir-30c-1 |
| | | 4077 | hsa-mir-30c-2 |
| 3687 | hsa-miR-30d | 3092 | hsa-mir-30d |
| 3688 | hsa-miR-30e | 3093 | hsa-mir-30e |
| 3689 | mmu-miR-384-5p | 4078 | mmu-mir-384 |
| 3690 | mo-miR-384-5p | 4079 | mo-mir-384 |
| 3691 | hsa-miR-563 | 4080 | hsa-mir-563 |
| 3692 | hsa-miR-130a | 3311 | hsa-mir-130a |
| 3693 | hsa-miR-130b | 4081 | hsa-mir-130b |
| 3694 | hsa-miR-301a | 4082 | hsa-mir-301a |
| 3695 | hsa-miR-301 b | 4083 | hsa-mir-301b |
| 3696 | mmu-miR-130a | 4084 | mmu-mir-130a |
| 3697 | mmu-miR-130b | 4085 | mmu-mir-130b |
| 3698 | mmu-miR-301a | 4086 | mmu-mir-301a |
| 3699 | mmu-miR-301b | 4087 | mmu-mir-301b |
| 3700 | mmu-miR-721 | 4088 | mmu-mir-721 |

**[Table 3-39]**

| sequence number | mRNAname | sequence number | miRNA precursor name |
|---|---|---|---|
| 3701 | mo-miR-130a | 4089 | mo-mir-130a |
| 3702 | mo-miR-130b | 4090 | mo-mir-130b |
| 3703 | mo-miR-301 a | 4091 | mo-mir-301a |
| 3704 | mo-miR-301b | 4092 | mo-mir-301b |
| 3705 | mml-miR-130a | 4093 | mml-mir-130a |
| 3706 | mml-miR-130b | 4094 | mml-mir-130b |
| 3707 | mml-miR-301a | 4095 | mml-mir-301a |
| 3708 | mml-miR-301b | 4096 | mml-mir-301b |
| 3709 | mme-miR-130a | 4097 | mne-mir-130a |
| 3710 | ggo-miR-130a | 4098 | ggo-mir-130a |
| 3711 | ppa-miR-130a | 4099 | ppa-mir-130a |
| 3712 | cfa-miR-130a | 4100 | cfa-mir-130a |
| 3713 | cfa-miR-130b | 4101 | cfa-mir-130b |
| 3714 | mdo-miR-130a | 4102 | mdo-mir-130a |
| 3715 | gga-miR-130a | 4103 | gga-mir-130a |
| 3716 | gga-miR-130b | 4104 | gga-mir-130b |
| 3717 | gga-miR-301 | 4105 | gga-mir-301 |
| 3718 | xtr-miR-130a | 4106 | xtr-mir-130a |
| 3719 | xtr-miR-130b | 4107 | xtr-mir-130b |
| 3720 | xtr-miR-130c | 4108 | xtr-mir-130c |
| 3721 | xtr-miR-301 | 4109 | xtr-mir-301-1 |
| | | 4110 | xtr-mir-301-2 |
| 3722 | dre-miR-130a | 4111 | dre-mir-130a-1 |
| | | 4112 | dre-mir-130a-2 |
| 3723 | dre-miR-130b | 4113 | dre-mir-130b |
| 3724 | dre-miR-130c | 4114 | dre-mir-130c-1 |
| | | 4115 | dre-mir-130c-2 |
| 3725 | dre-miR-301a | 4116 | dre-mir-301a-1 |
| | | 4117 | dre-mir-301a-2 |
| 3726 | dre-miR-301b | 4118 | dre-mir-301b |
| 3727 | dre-miR-301c | 4119 | dre-mir-301c |
| 3728 | fru-miR-130 | 4120 | fru-mir-130 |
| 3729 | fru-miR-301 | 4121 | fru-mir-301 |
| 3730 | tni-miR-130 | 4122 | tni-mir-130 |
| 3731 | tni-miR-301 | 4123 | tni-mir-301 |
| 3732 | mmu-miR-124 | 4124 | mmu-mir-124-1 |
| | | 4125 | mmu-mir-124-2 |
| | | 4126 | mmu-mir-124-3 |
| 3733 | mo-miR-124 | 4127 | mo-mir-124-1 |
| | | 4128 | mo-mir-124-2 |
| | | 4129 | mo-mir-124-3 |
| 3734 | cfa-miR-124 | 4130 | cfa-mir-124-1 |
| | | 4131 | cfa-mir-124-2 |
| | | 4132 | cfa-mir-124-5 |
| 3735 | dre-miR-124 | 4133 | dre-mir-124-1 |
| | | 4134 | dre-mir-124-2 |
| | | 4135 | dre-mir-124-3 |
| | | 4136 | dre-mir-124-4 |
| | | 4137 | dre-mir-124-5 |
| | | 4138 | dre-mir-124-6 |
| 3736 | fru-miR-124 | 4139 | fru-mir-124-1 |
| | | 4140 | fru-mir-124-2 |
| | | 4141 | fru-mir-124-3 |
| 3737 | tni-miR-124 | 4142 | tri-mir-124-1 |
| | | 4143 | tni-mir-124-2 |
| | | 4144 | tni-mir-124-3 |
| 3738 | hsa-miR-557 | 4145 | hsa-mir-557 |
| 3739 | mmu-miR-1186 | 4146 | mmu-mir-1186 |
| 3740 | xtr-miR-93b | 4147 | xtr-mir-93b |
| 3741 | hsa-miR-518a-5p | 3359 | hsa-mir-518a-1 |
| | | 3360 | hsa-mir-518a-2 |

In the present invention, a nucleic acid having an identity of 90% or more to a nucleotide sequence of any one of SEQ ID NOs:1 to 4147 means a nucleic acid having an identity of at least 90% or more, preferably 93% or more, more preferably 95% or more, still more preferably 96% or more, particularly preferably 97% or more, most preferably 98% or more, to a nucleic acid consisting of a nucleotide sequence of any one of SEQ ID NOs:1 to 3371, as calculated using an analytical software program such as BLAST (J. Mol. Biol., 215, 403 (1990)) or FASTA (Methods in Enzymology, 183, 63 (1990)).

As mentioned above, stringent conditions are conditions that allow signals to be detected by adding to a membrane blotted with one strand the other strand labeled with ³²P-ATP in a hybridization buffer consisting of 7.5 mL, 0.6 mL of 1M Na₂HPO₄ (pH 7.2), 21 mL of 10% SDS, 0.6 mL of 50xDenhardt's solution, and 0.3 mL of 10 mg/mL sonicated salmon sperm DNA, carrying out a reaction at 50°C overnight, thereafter washing the membrane with 5xSSC/5% SDS liquid at 50°C for 10 minutes, and further washing the same with 1xSSC/1% SDS liquid at 50°C for 10 minutes, thereafter taking out the membrane, and applying it to an X-ray film.

The method of detecting the expression of nucleic acid such as a micro-RNA using a nucleic acid may be any method, as far as the presence of nucleic acid such as a micro-RNA or micro-RNA precursor in a sample can be detected; for example, (1) Northern hybridization, (2) dot blot hybridization, (3) in situ hybridization, (4) quantitative PCR, (5) differential hybridization, (6) microarray, (7) ribonuclease protection assay and the like can be mentioned.

The method of detecting the amount expressed of a nucleic acid such as a micro-RNA, a mutation of the nucleic acid, or a mutation of the genome that encodes the nucleic acid using a nucleic acid used in the present invention may be any method, as far as it enables detection of a mutation of the nucleotide sequence of a nucleic acid such as a micro-RNA or micro-RNA precursor in a sample; for example, a method comprising detecting a hetero-double-strand formed by hybridization of a nucleic acid having a non-mutated nucleotide sequence and a nucleic acid having a mutated nucleotide sequence, a method comprising directly sequencing a sample-derived nucleotide sequence to detect the presence or absence of a mutation, and the like can be mentioned.

The vector that expresses a nucleic acid used in the present invention may be any vector, as far as it has been designed to biosynthesize a nucleic acid, such as a micro-RNA, used in the present invention, when introduced to, and transcribed in, a cell. As vectors capable of expressing a nucleic acid, such as a micro-RNA, used in the present invention in cells, specifically, pcDNA6.2-GW/miR (manufactured by Invitrogen Company), pSilencer 4.1-CMV (manufactured by Ambion Company), pSINsi-hH1 DNA (manufactured by Takara Bio Company), pSINsi-hU6 DNA (manufactured by Takara Bio Company), pENTR/U6 (manufactured by Invitrogen Company) and the like can be mentioned.

The method of suppressing the expression of a gene having a target nucleotide sequence for a nucleic acid, such as a micro-RNA, used in the present invention (hereinafter also referred to as a target gene) may be any method, as far as it suppresses the expression of a gene having the target nucleotide sequence. Here, to suppress the expression encompasses a case where the translation of an mRNA is suppressed, and a case where cleavage or degradation of an mRNA results in a decreased amount of protein translated from the mRNA. As substances that suppress the expression of an mRNA having the target nucleotide sequence, specifically nucleic acids such as siRNAs and antisense oligonucleotides can be mentioned. The siRNAs can be prepared on the basis of information on the continuous sequence of the mRNA (Genes Dev., 13, 3191 (1999)). The number of nucleotide residues constituting one strand of the siRNA is preferably 17 to 30 residues, more preferably 18 to 25 residues, still more preferably 19 to 23 residues.

Micro-RNAs used in the present invention also include an artificial micro-RNA that is a single-stranded RNA 17 to 28 nucleotides long, comprising a sequence complementary to a 7-nucleotide continuous sequence present in a gene (target gene) having a target nucleotide sequence for a micro-RNA of any one of SEQ ID NOs:1 to 1543 and 3372 to 3741 as the 2nd to 8th nucleotides. In case of an RNA comprising an artificial micro-RNA sequence extended anteriorly and posteriorly to form a hairpin structure, wherein the micro-RNA sequence can be cut out from either one strand of the hairpin structure by the biosynthetic pathway of the micro-RNA in the cell, the extended sequence is called an artificial micro-RNA precursor. With the gene whose expression is to be suppressed as a target gene, it is possible to design artificial micro-RNAs and artificial micro-RNA precursors using the method described above.

A target nucleotide sequence for a micro-RNA refers to the nucleotide sequence of a nucleic acid consisting of several nucleotides recognized by a micro-RNA used in the present invention, wherein the expression of the mRNA having the nucleotide sequence is suppressed by the micro-RNA. Because an mRNA having a nucleotide sequence complementary to the 2nd to 8th nucleotides from the 5'-end of a micro-RNA undergoes suppression of the translation thereof by the micro-RNA (Current Biology, 15, R458-R460 (2005)), a nucleotide sequence complementary to the 2nd to 8th nucleotides from the 5-end of a micro-RNA used in the present invention can be mentioned as a target nucleotide sequence of the micro-RNA. For example, by providing a target sequence complementary to the 2nd to 8th nucleotides on the 5' terminal side of a micro-RNA, and selecting an mRNA comprising a sequence completely identical to a set of 3' UTR nucleotide sequences of human mRNAs by a method such as character string search, the target nucleotide sequence can be determined. A set of 3' UTR nucleotide sequences of human mRNAs can be prepared using information on genomic sequences and gene positions that can be acquired from "UCSC Human Genome Browser Gateway (http://genome.ucsc.edu/cgi-bin/hgGateway)".

As specific examples of genes having a target nucleotide sequence of a micro-RNA of any one of SEQ ID NOs:1 to 1543 and 3372 to 3741, the genes shown in Table 4-1 to Tables 4 to 148, represented by names (Official Symbols and Gene IDs) used in the EntreGene database (http://www.ncbi.nlm.nih.gov/Entrez/) of the US National Center for Biotechnology Information (NCBI), can be mentioned.

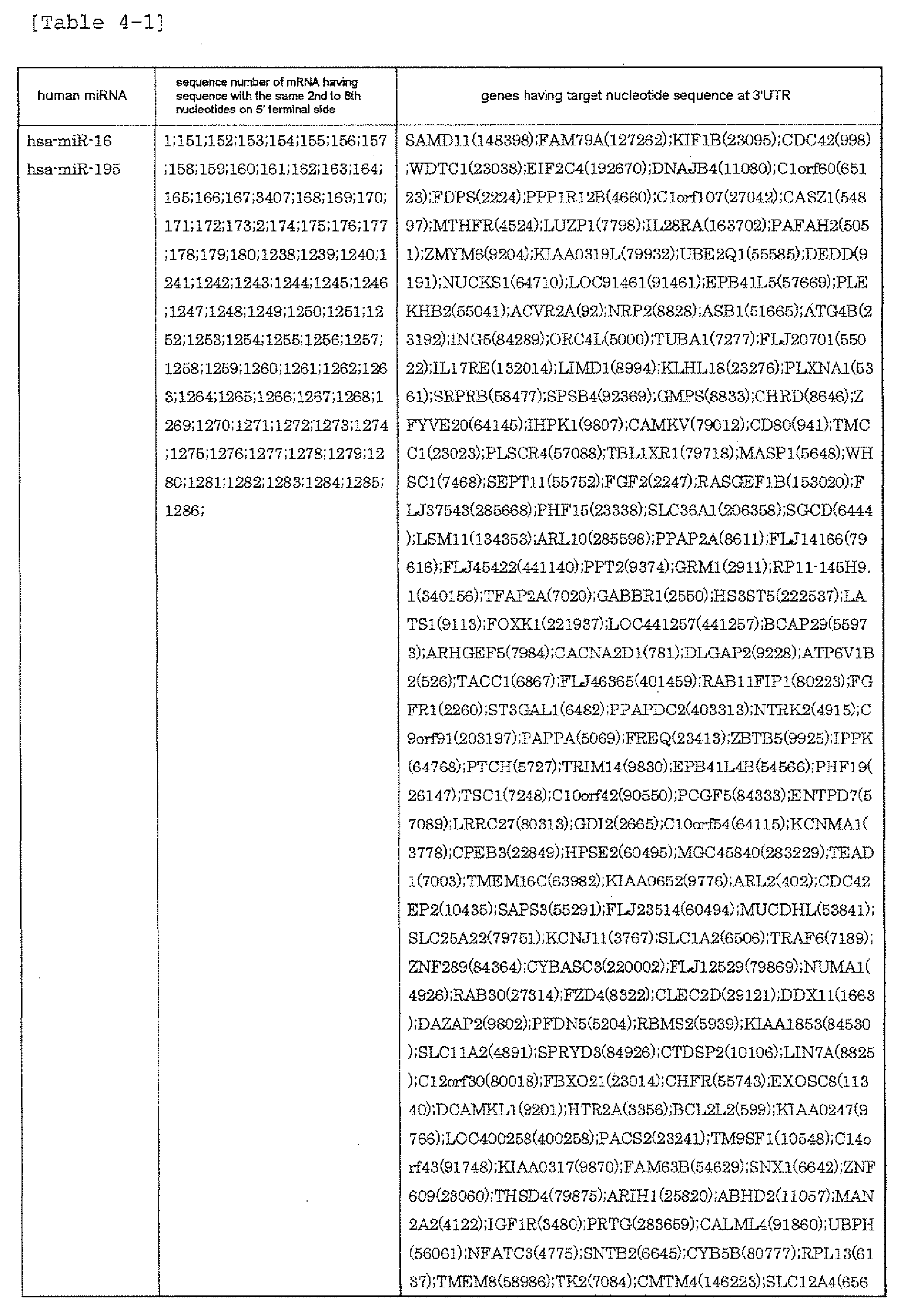

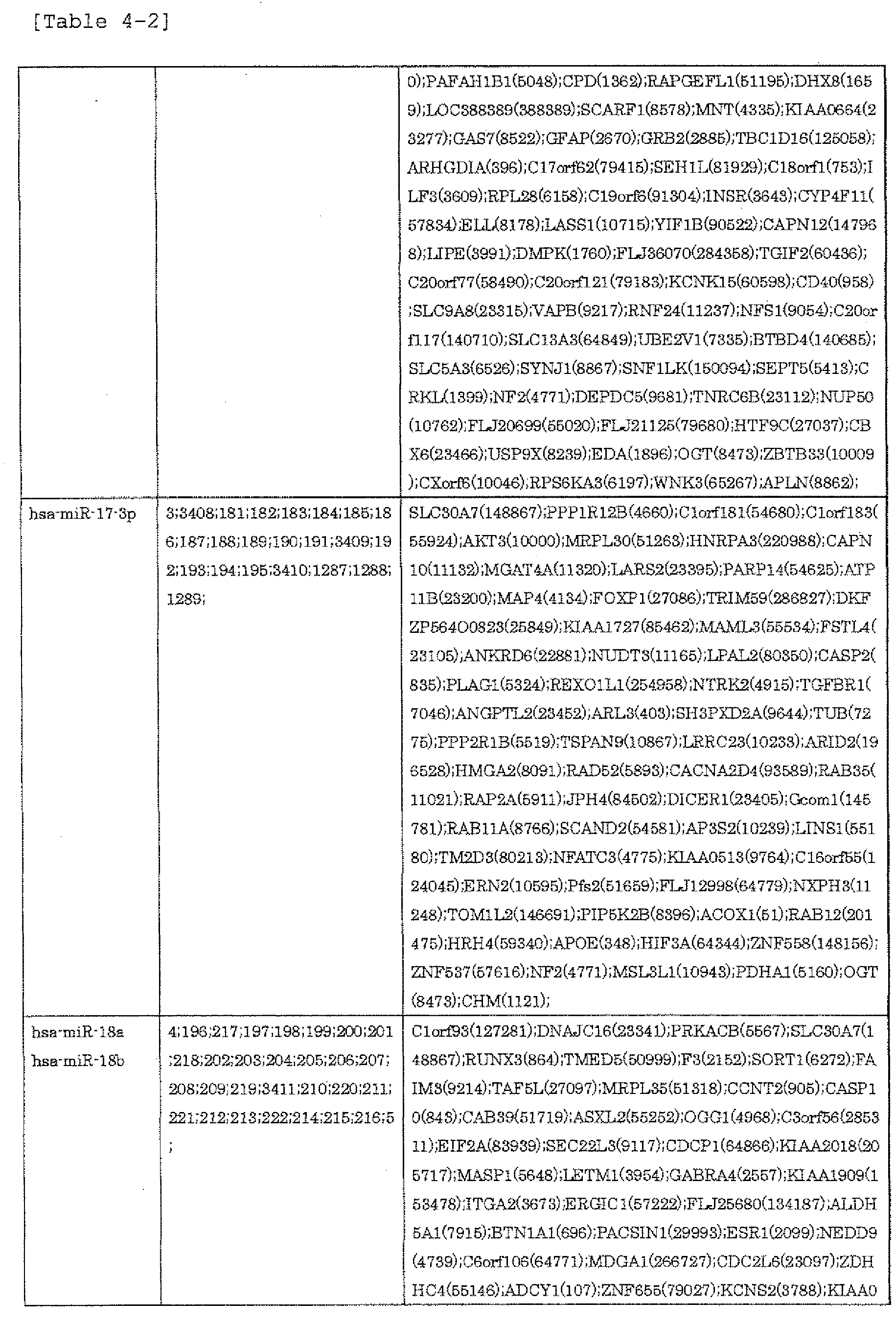

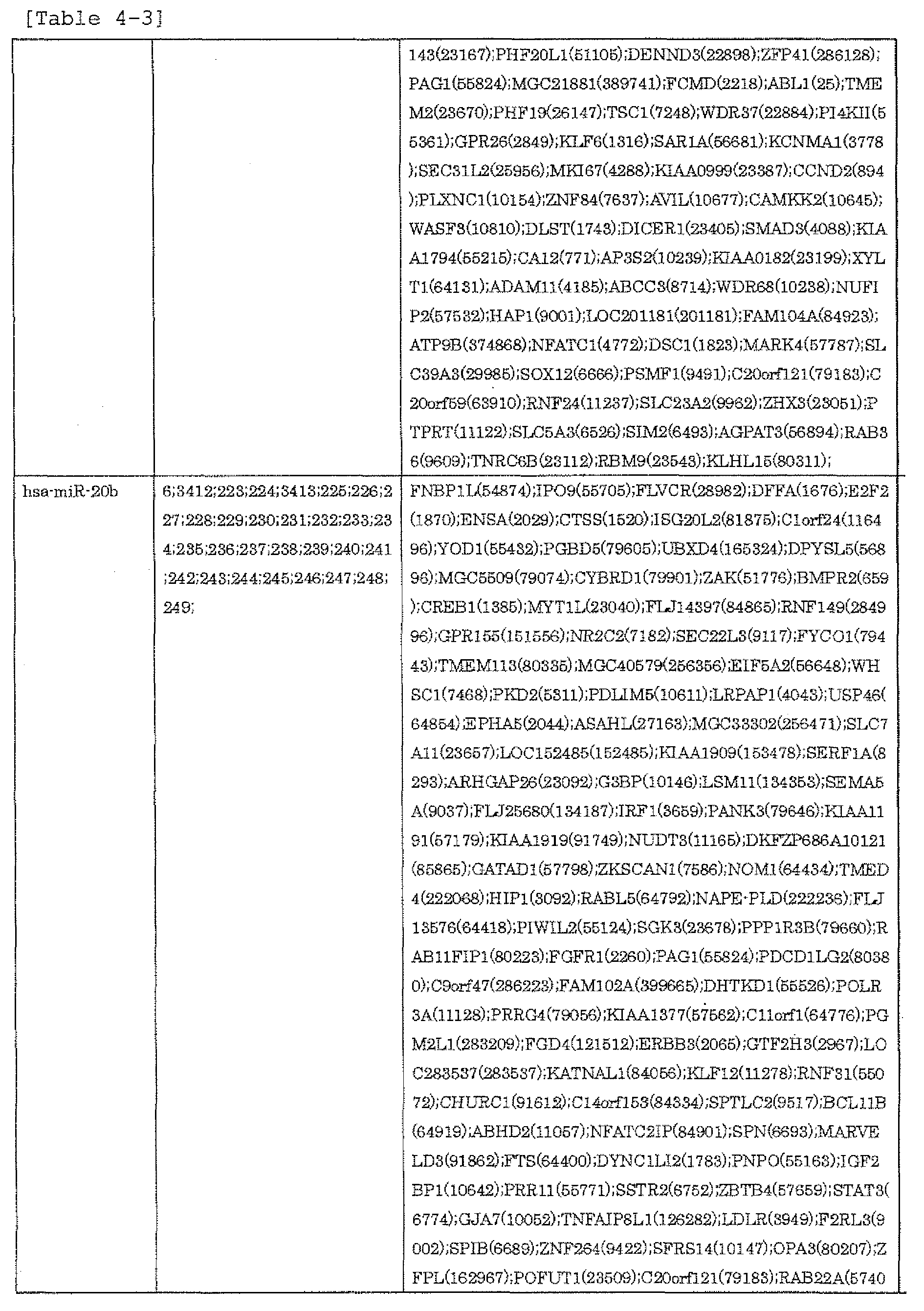

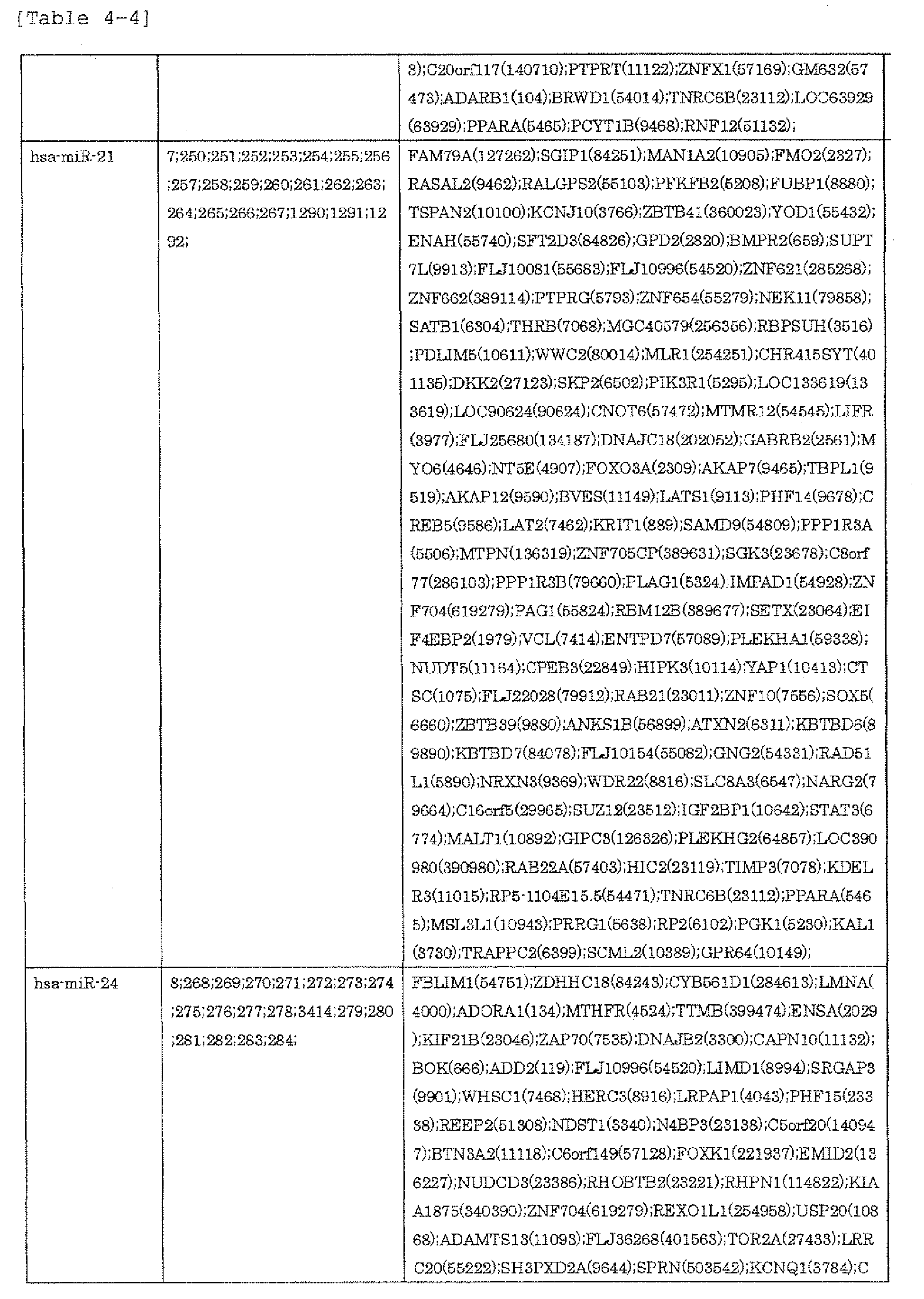

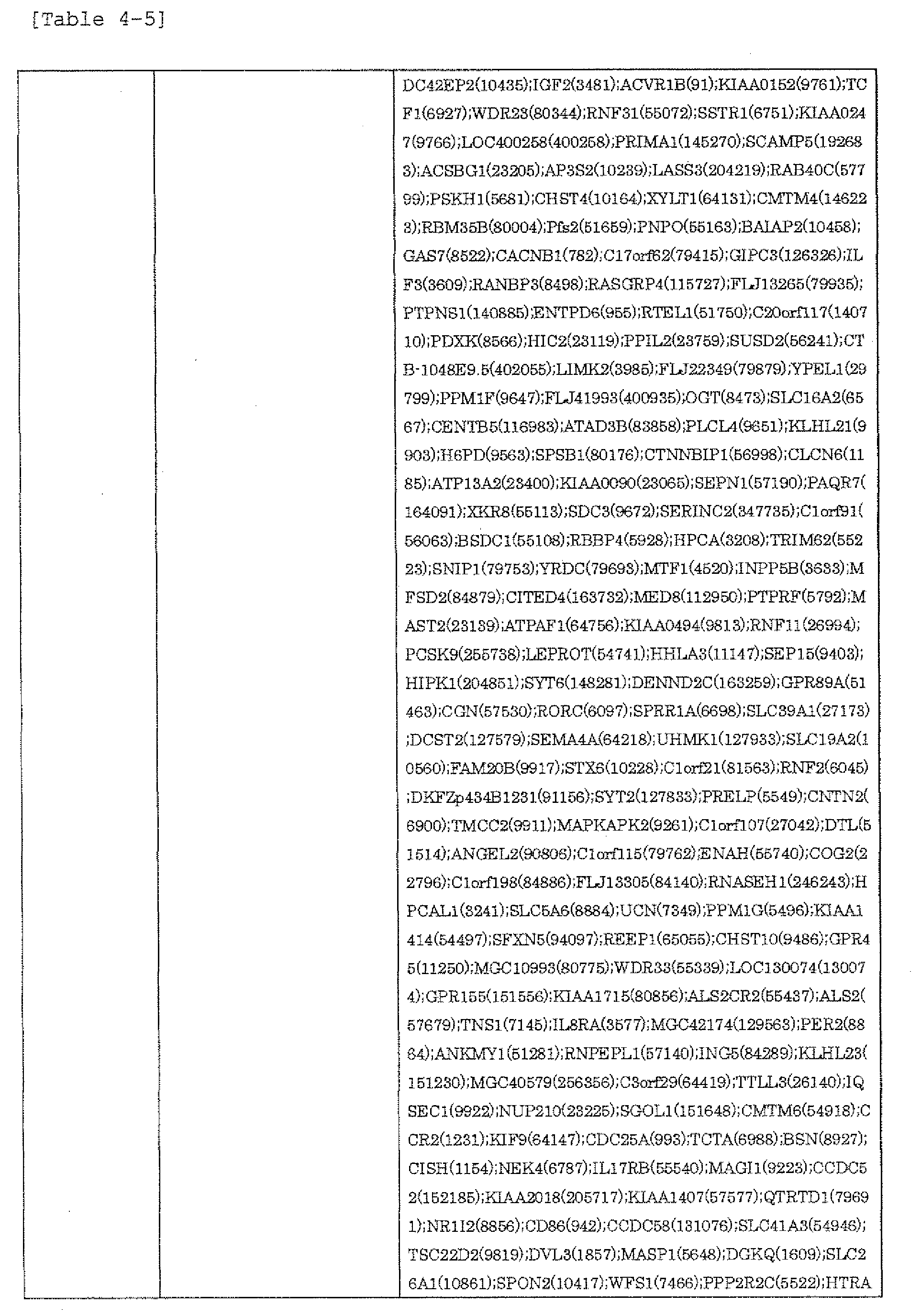

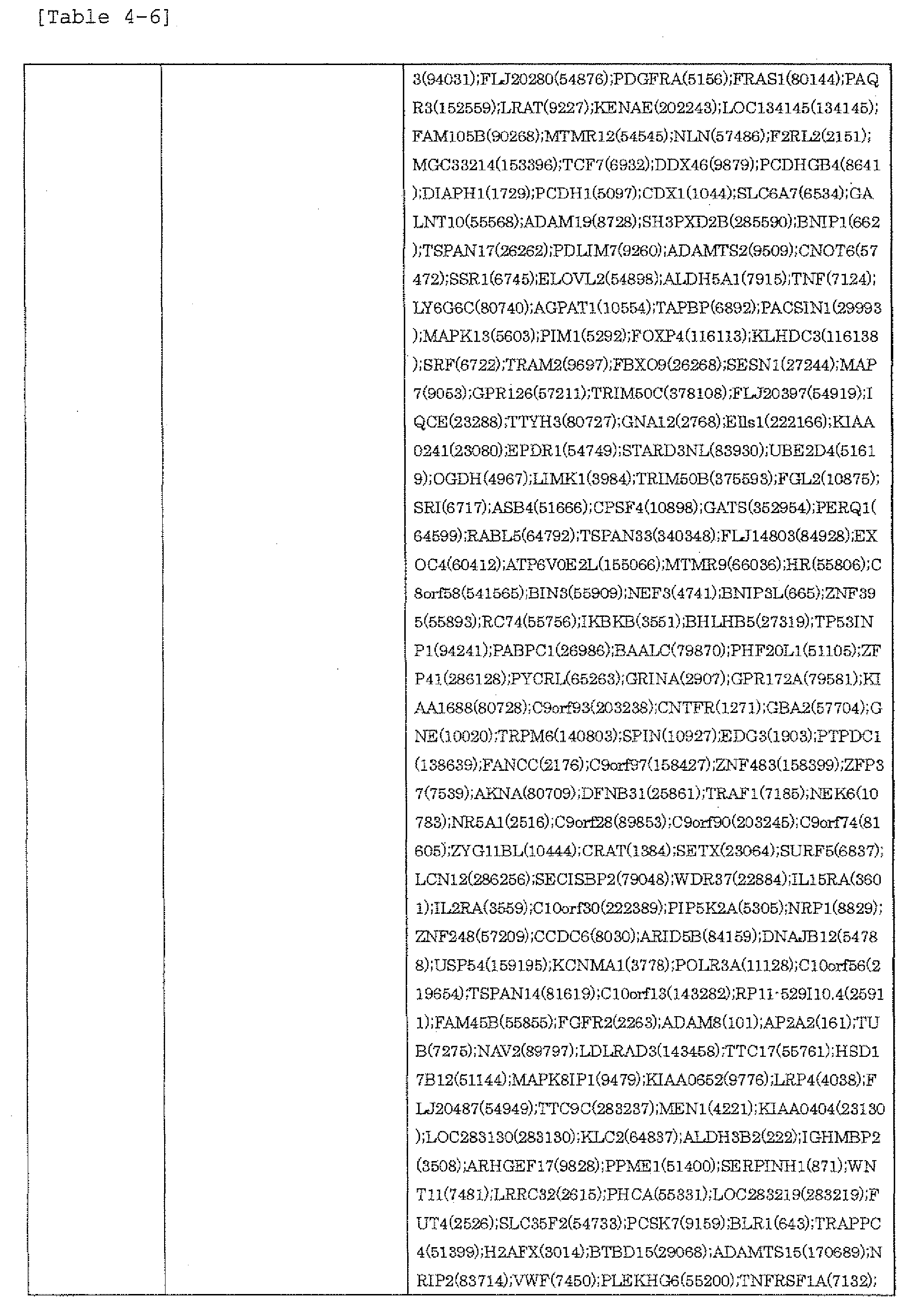

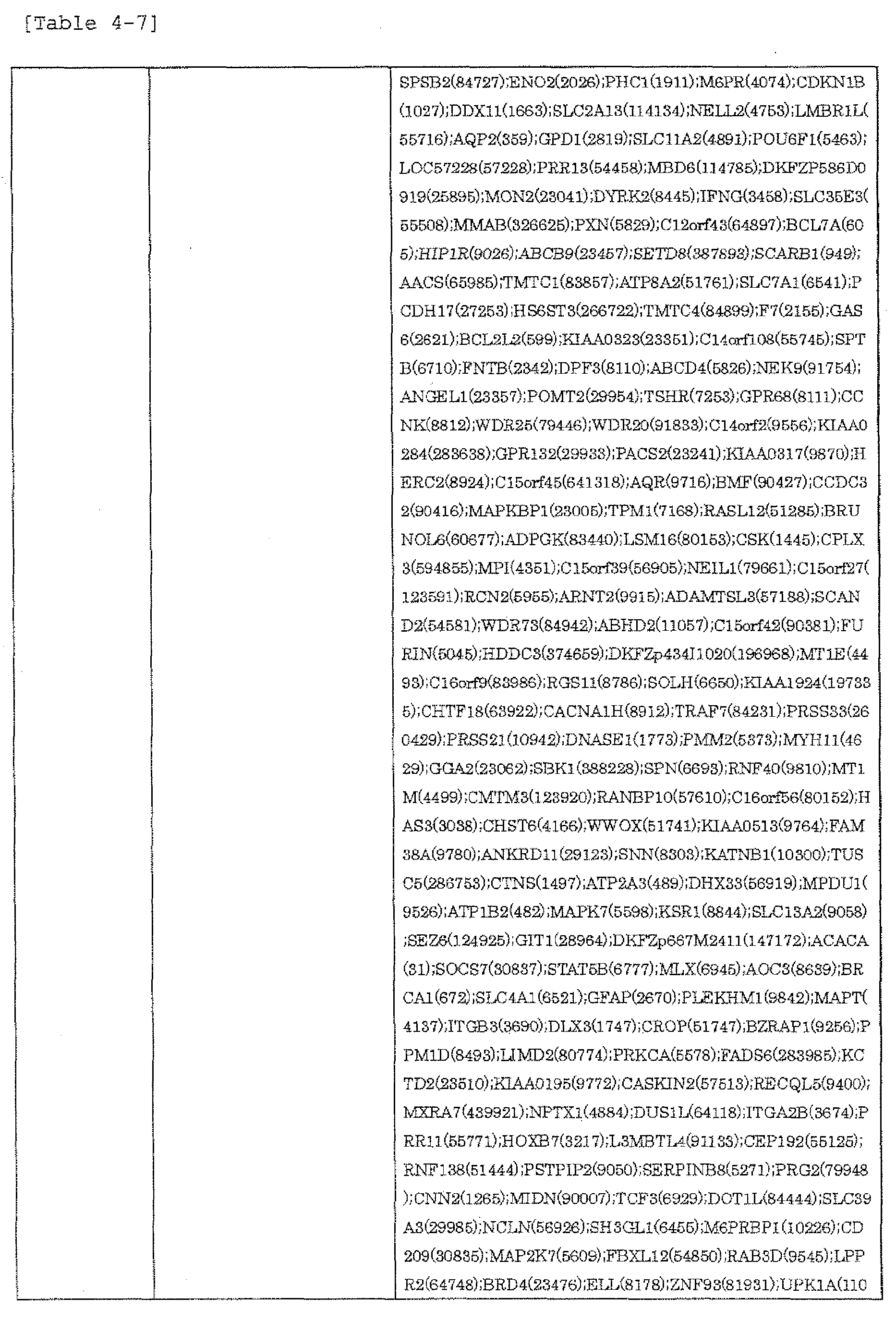

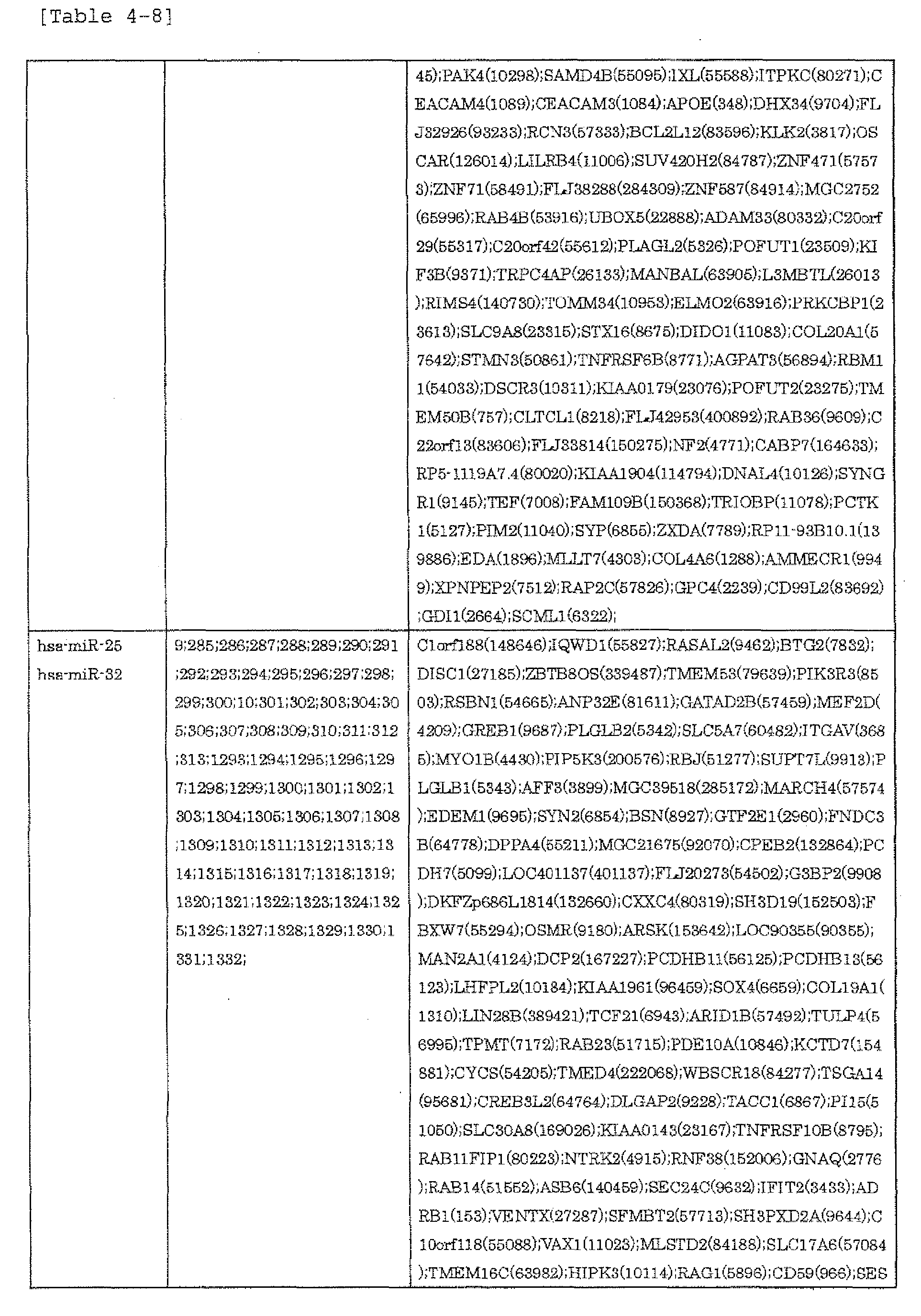

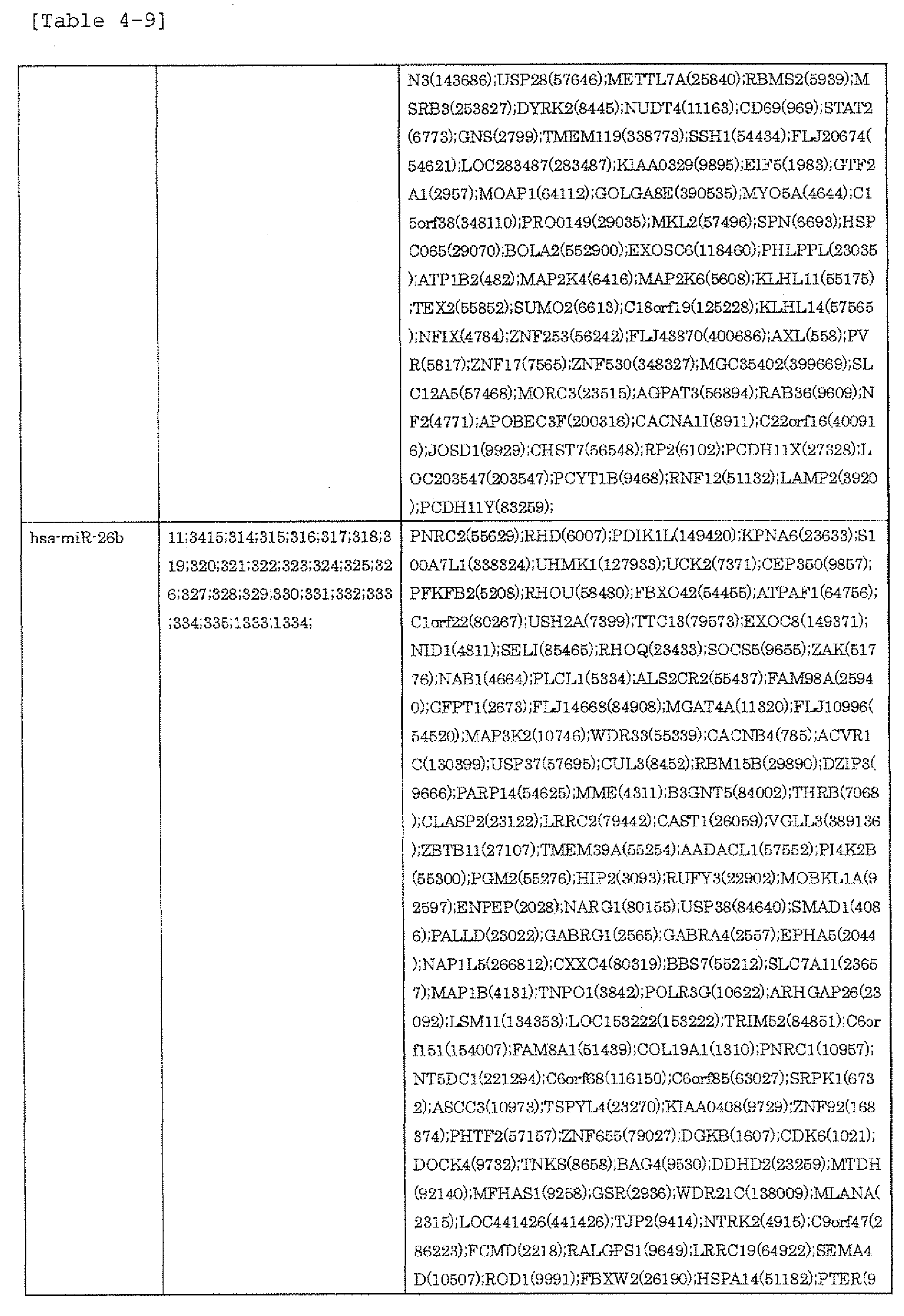

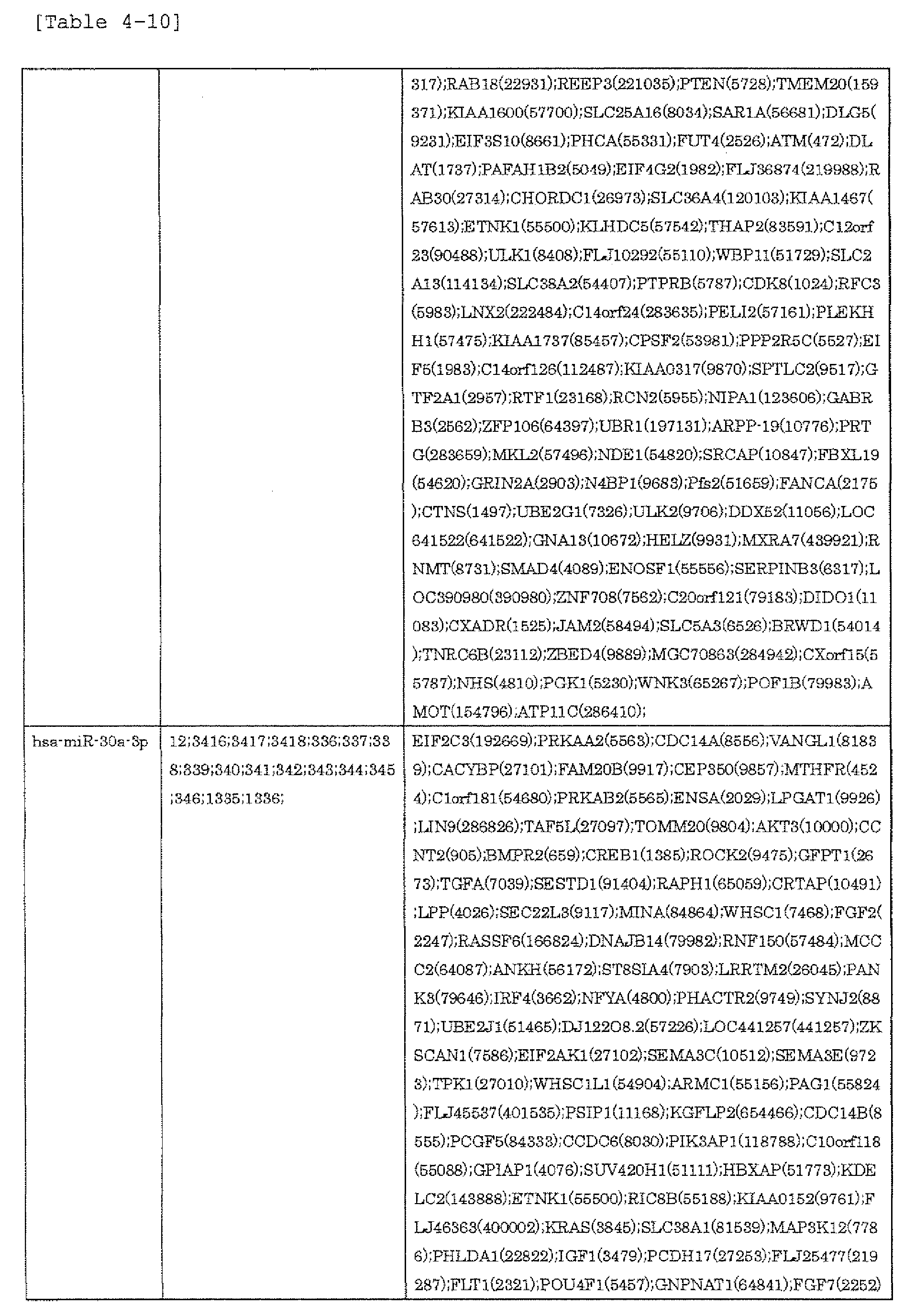

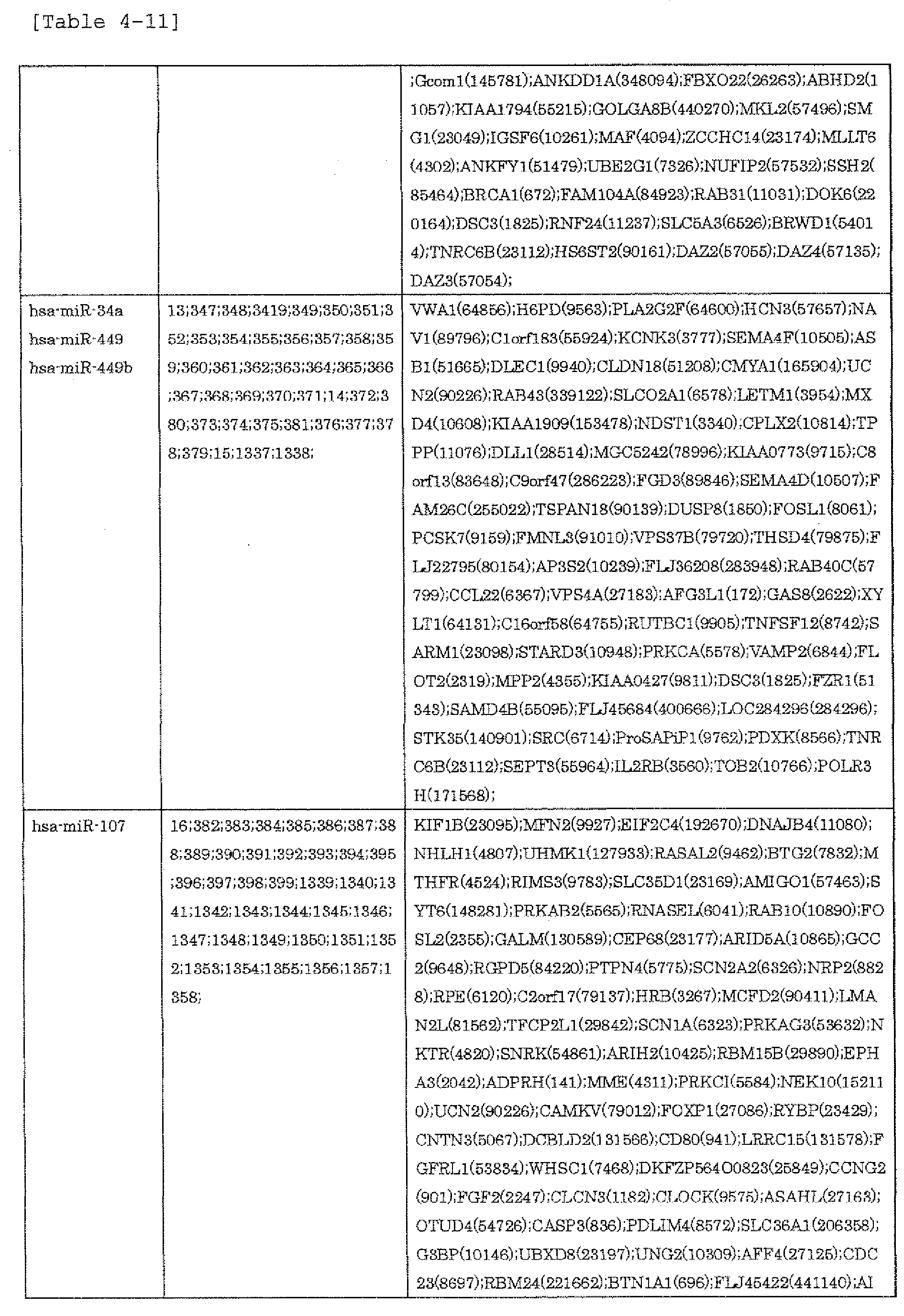

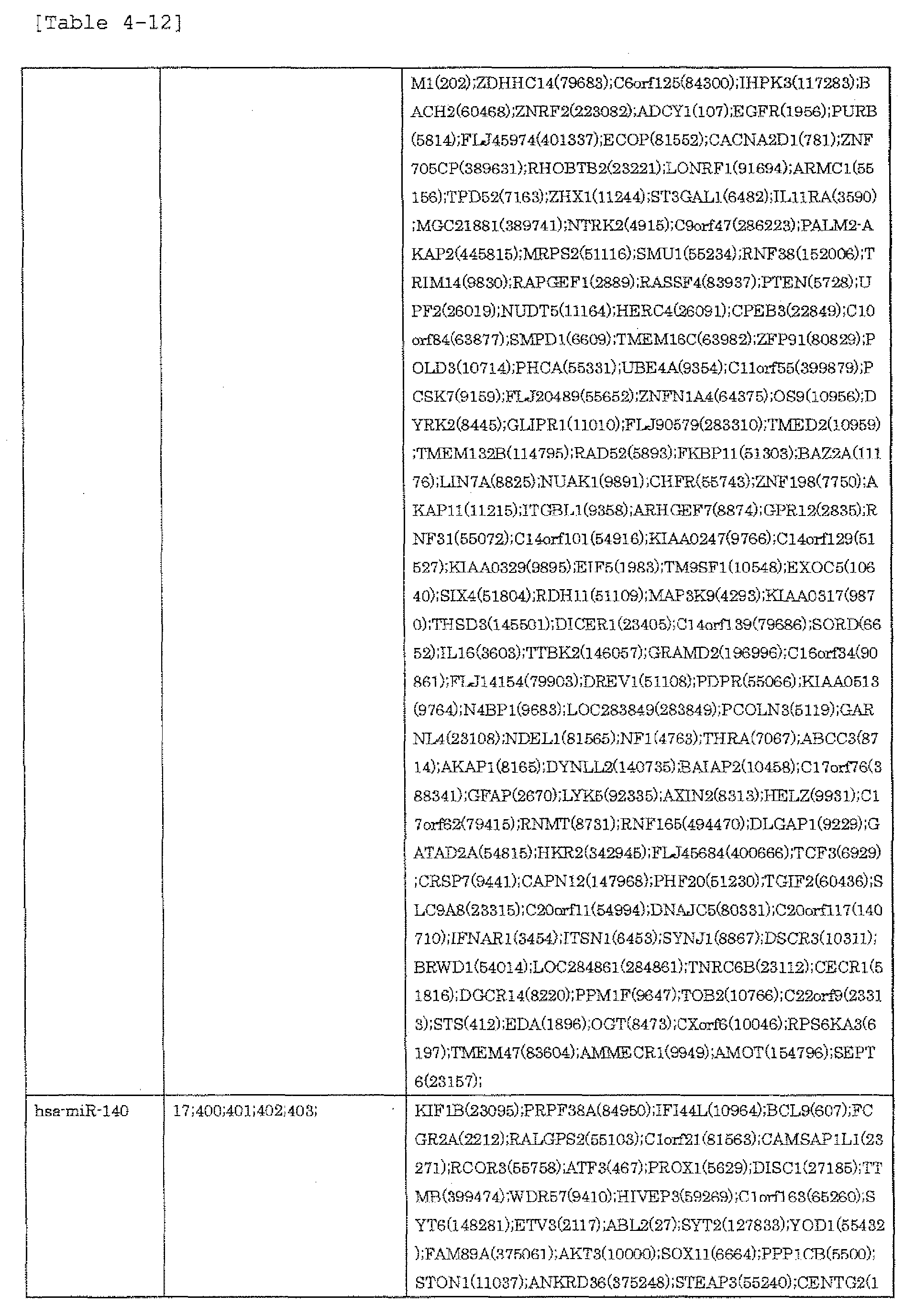

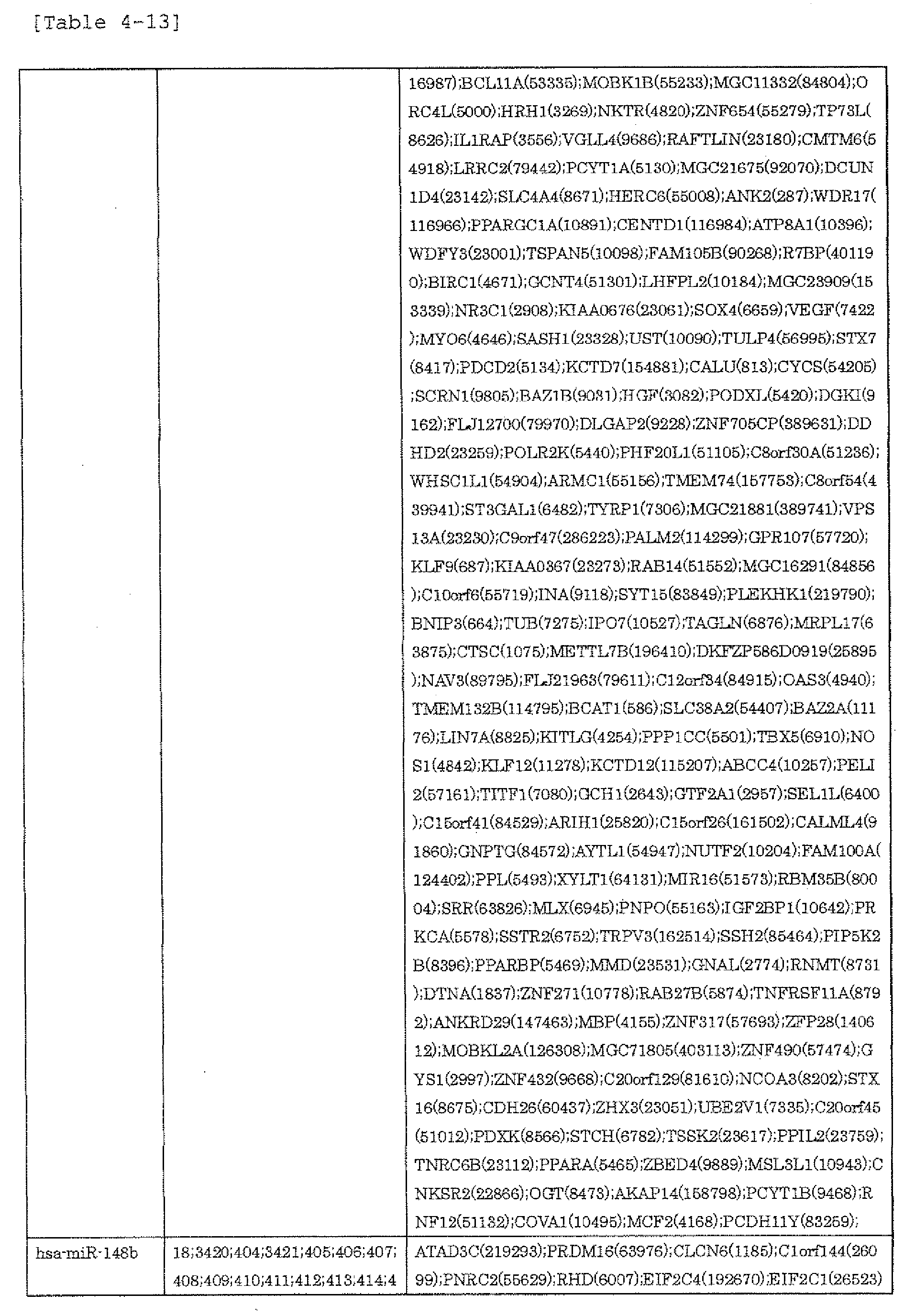

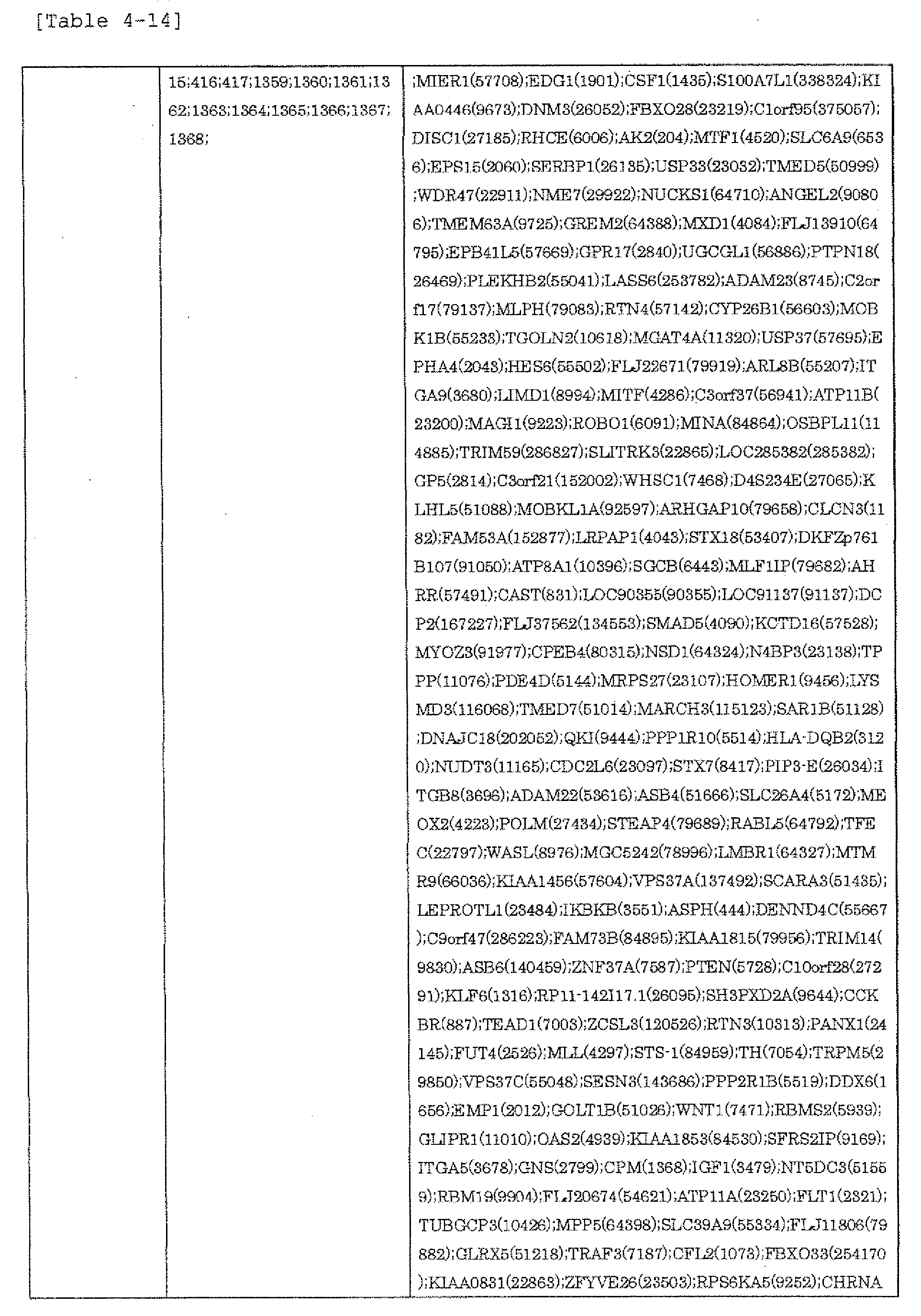

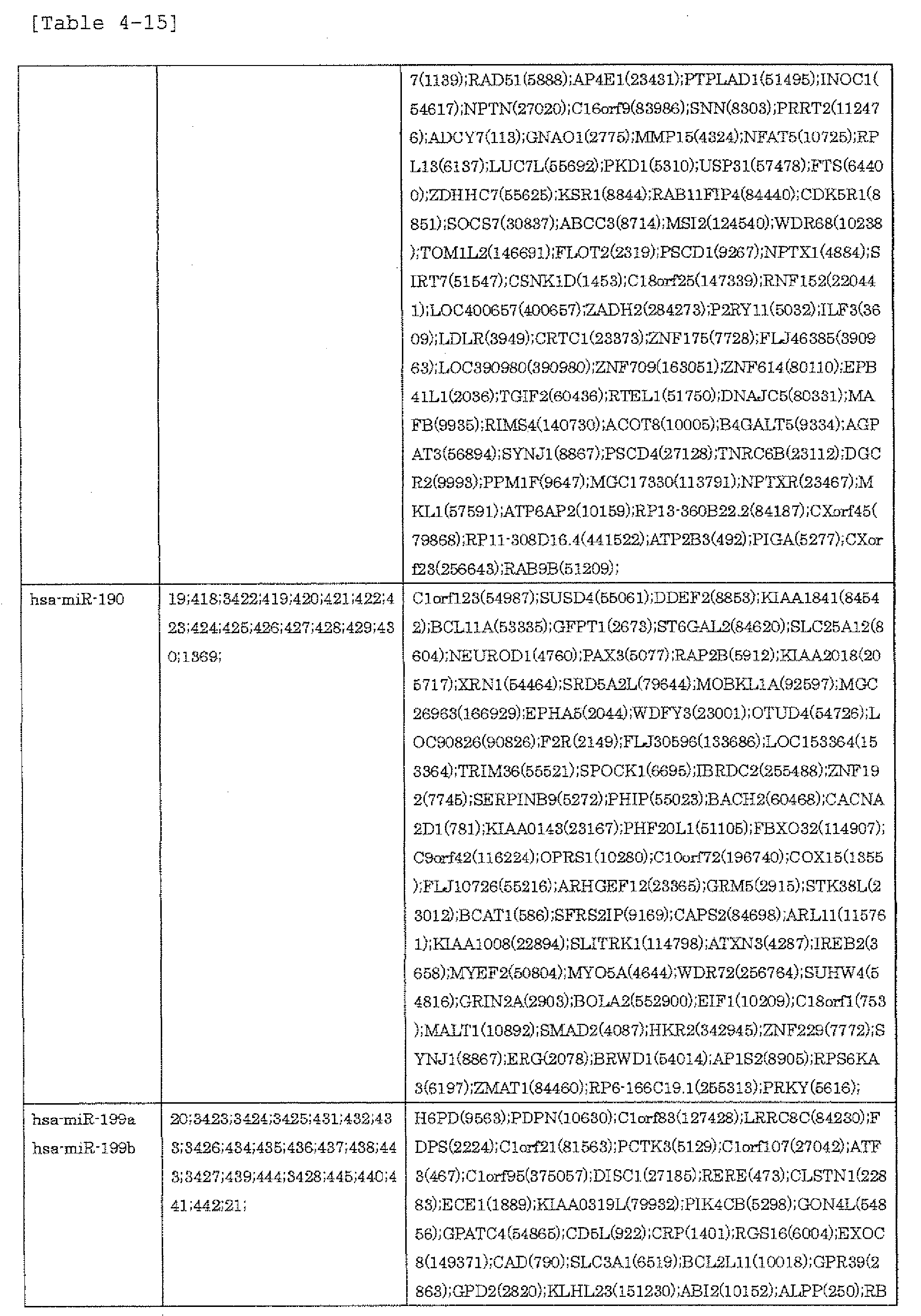

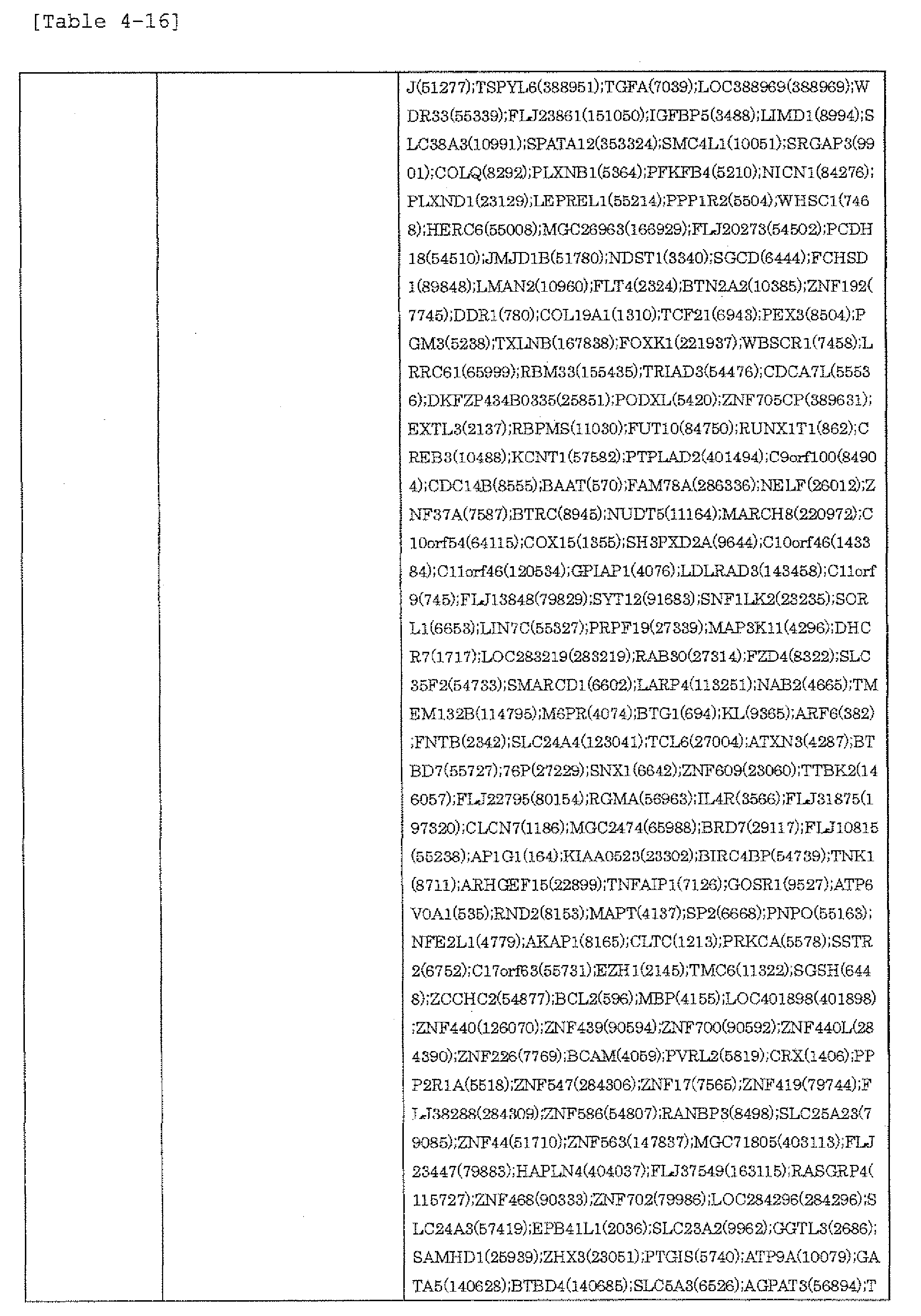

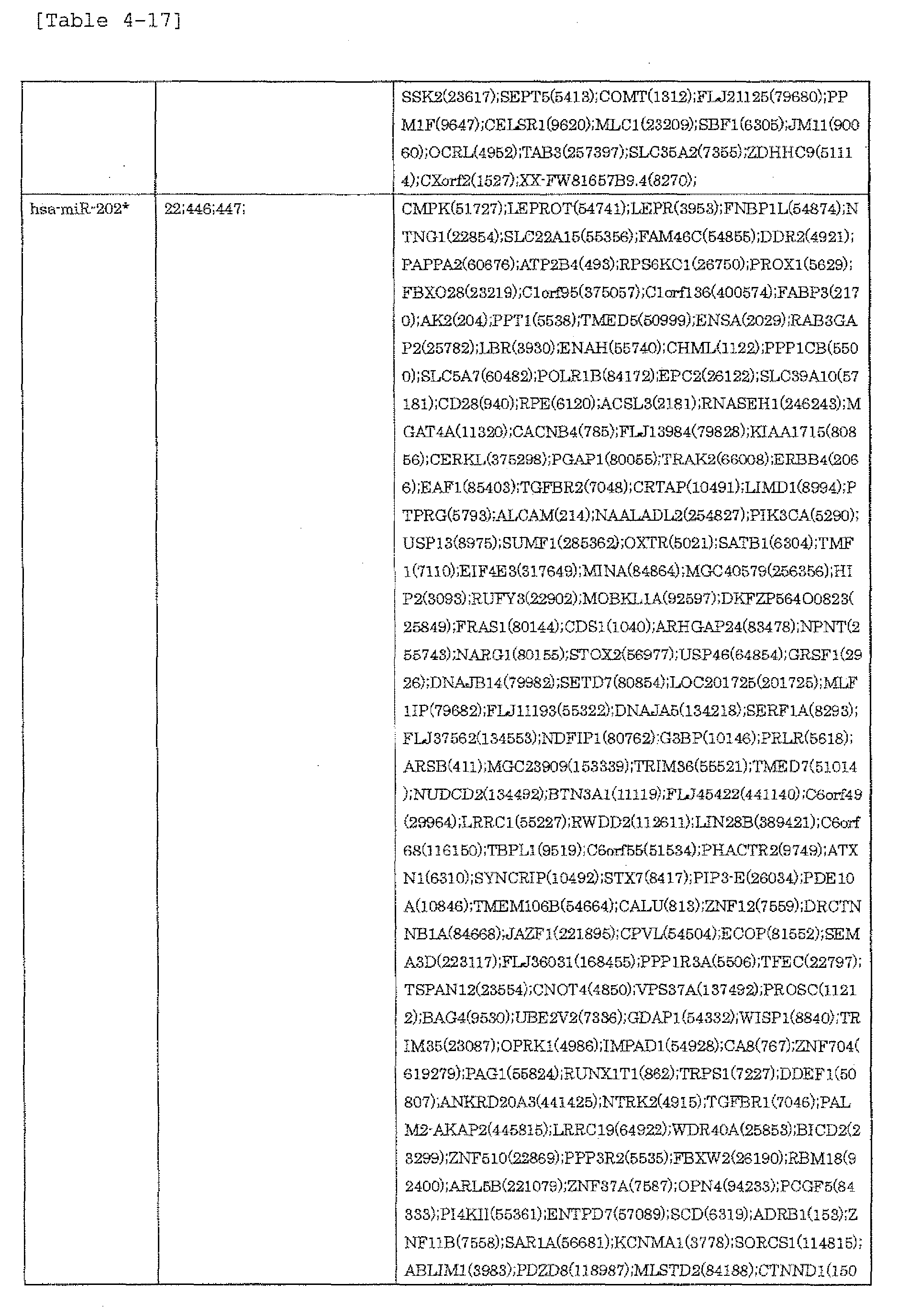

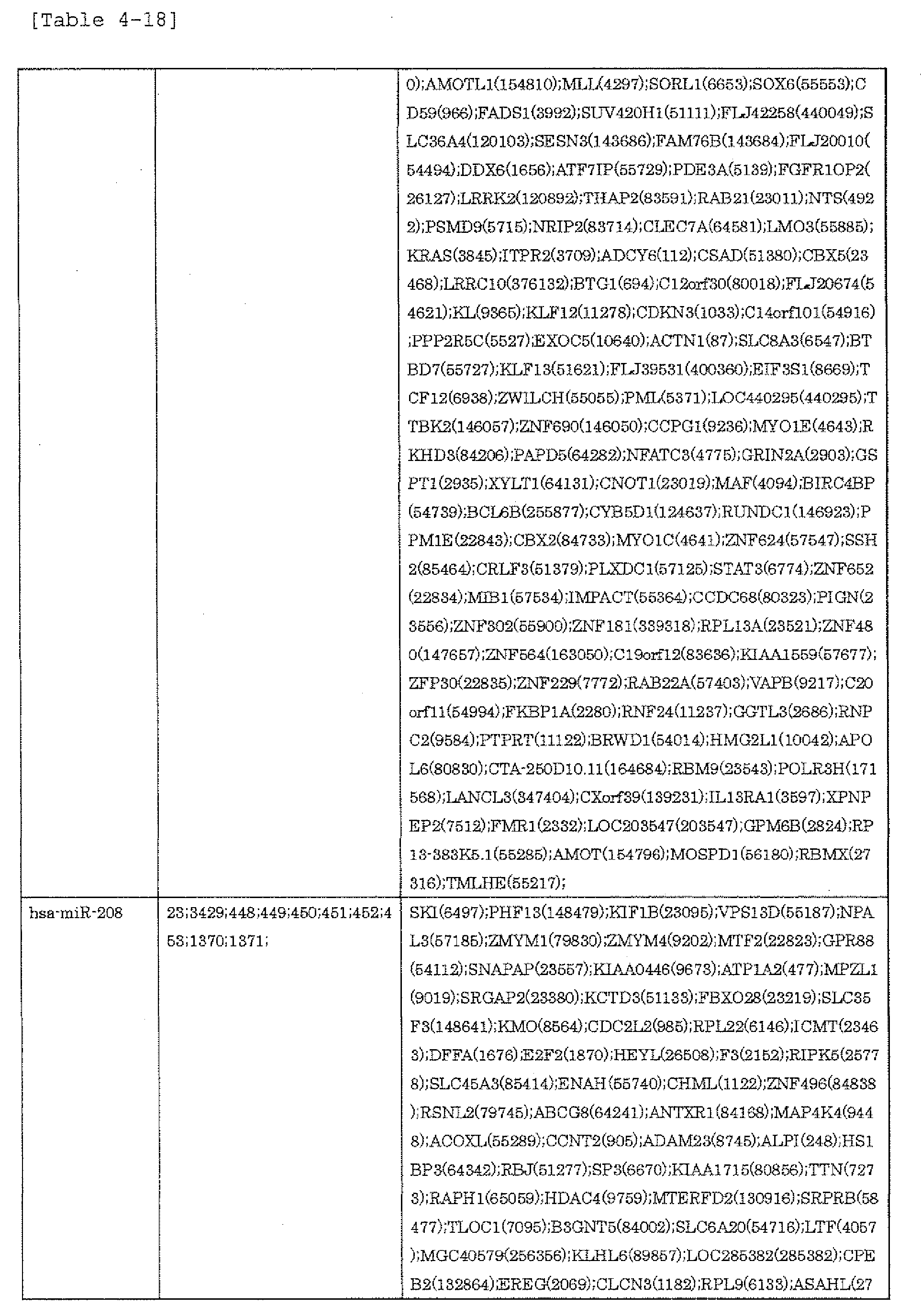

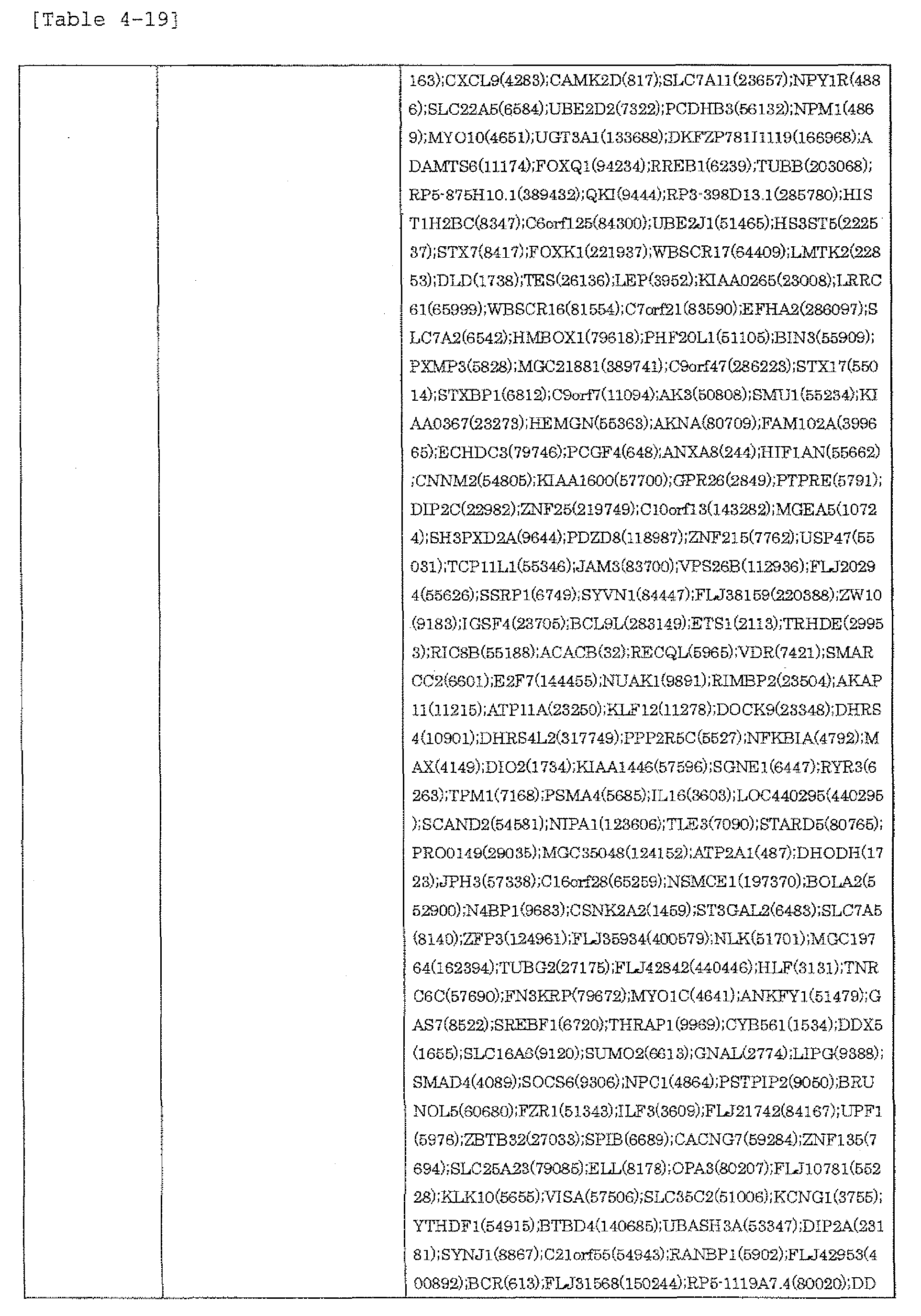

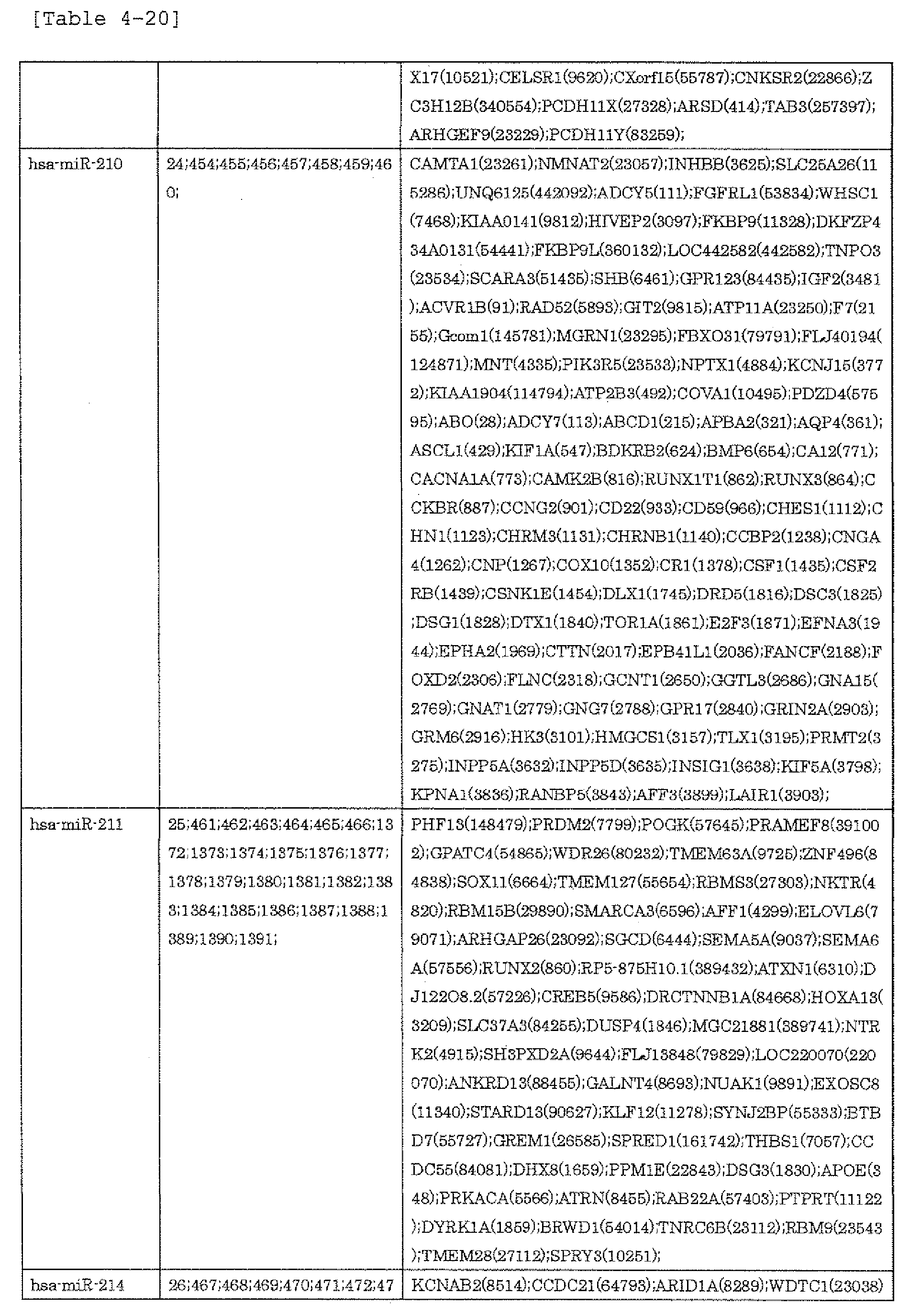

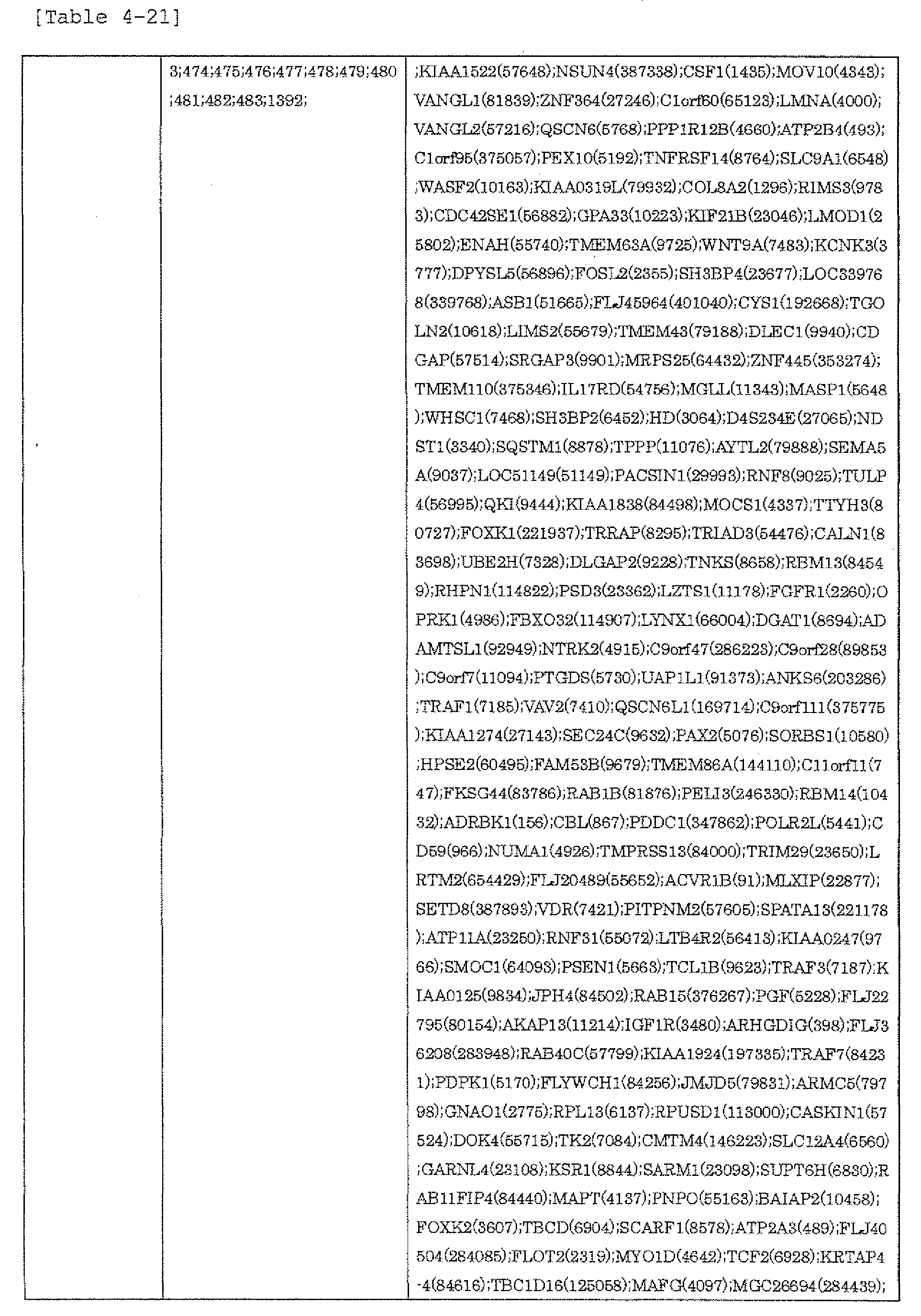

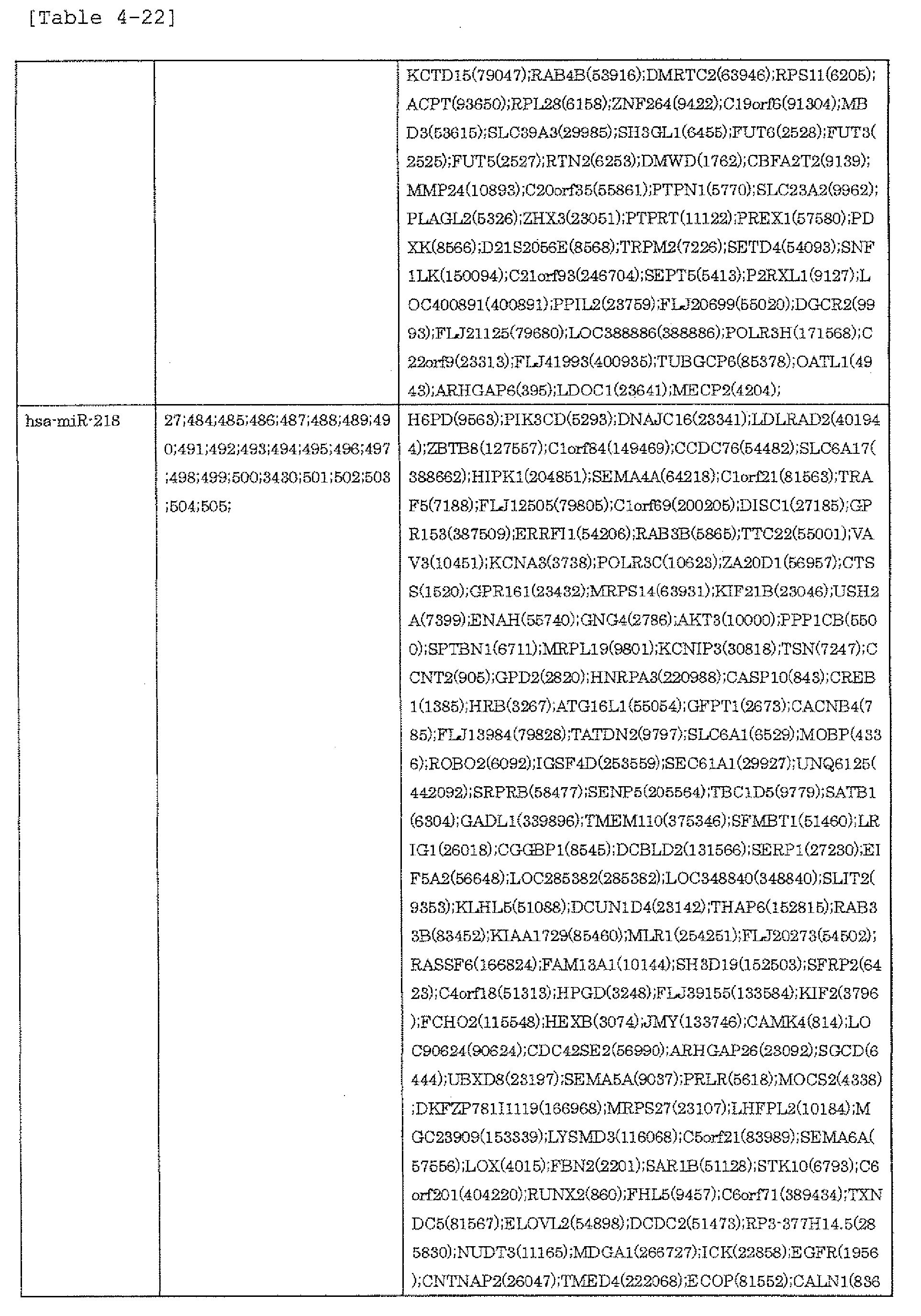

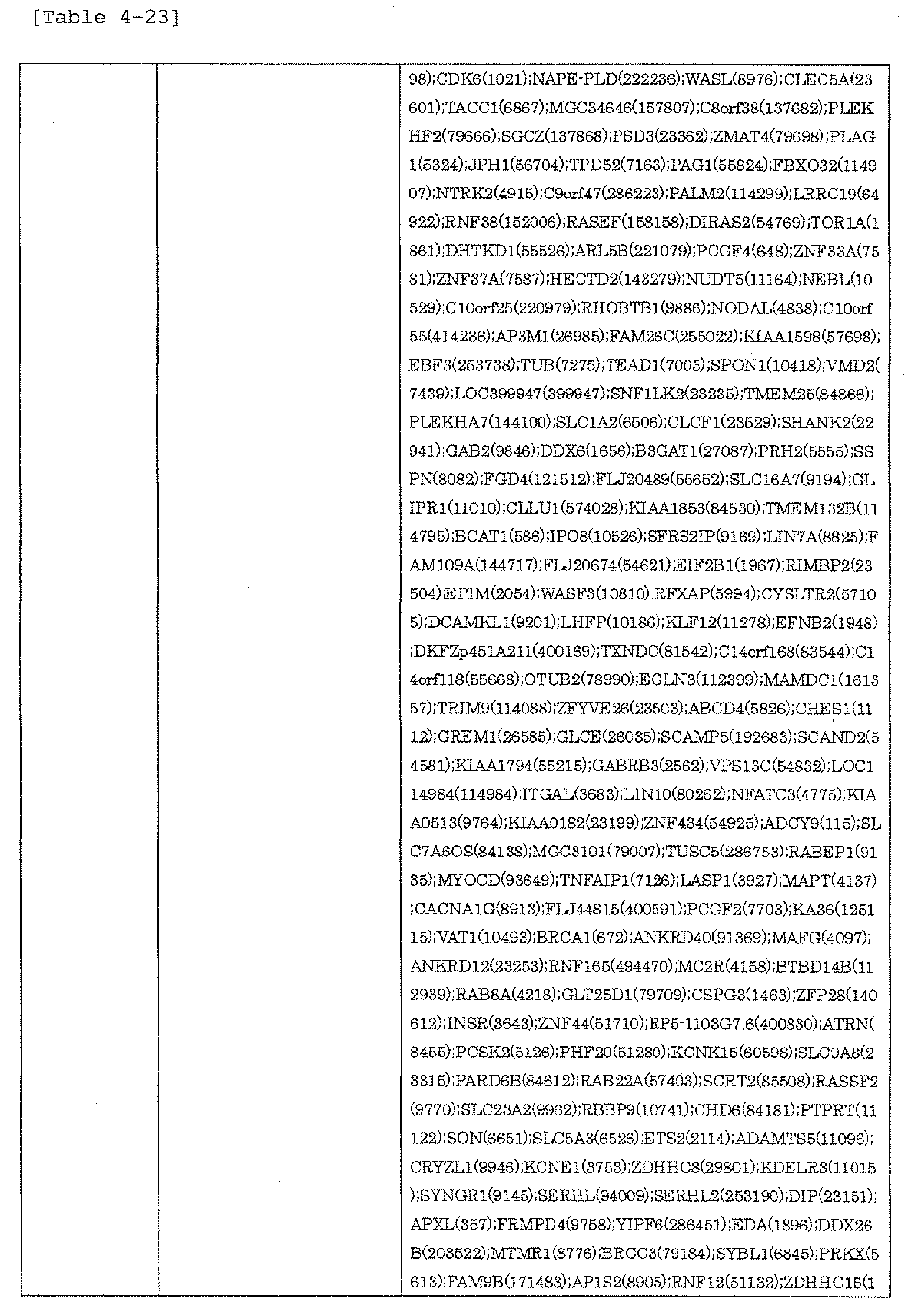

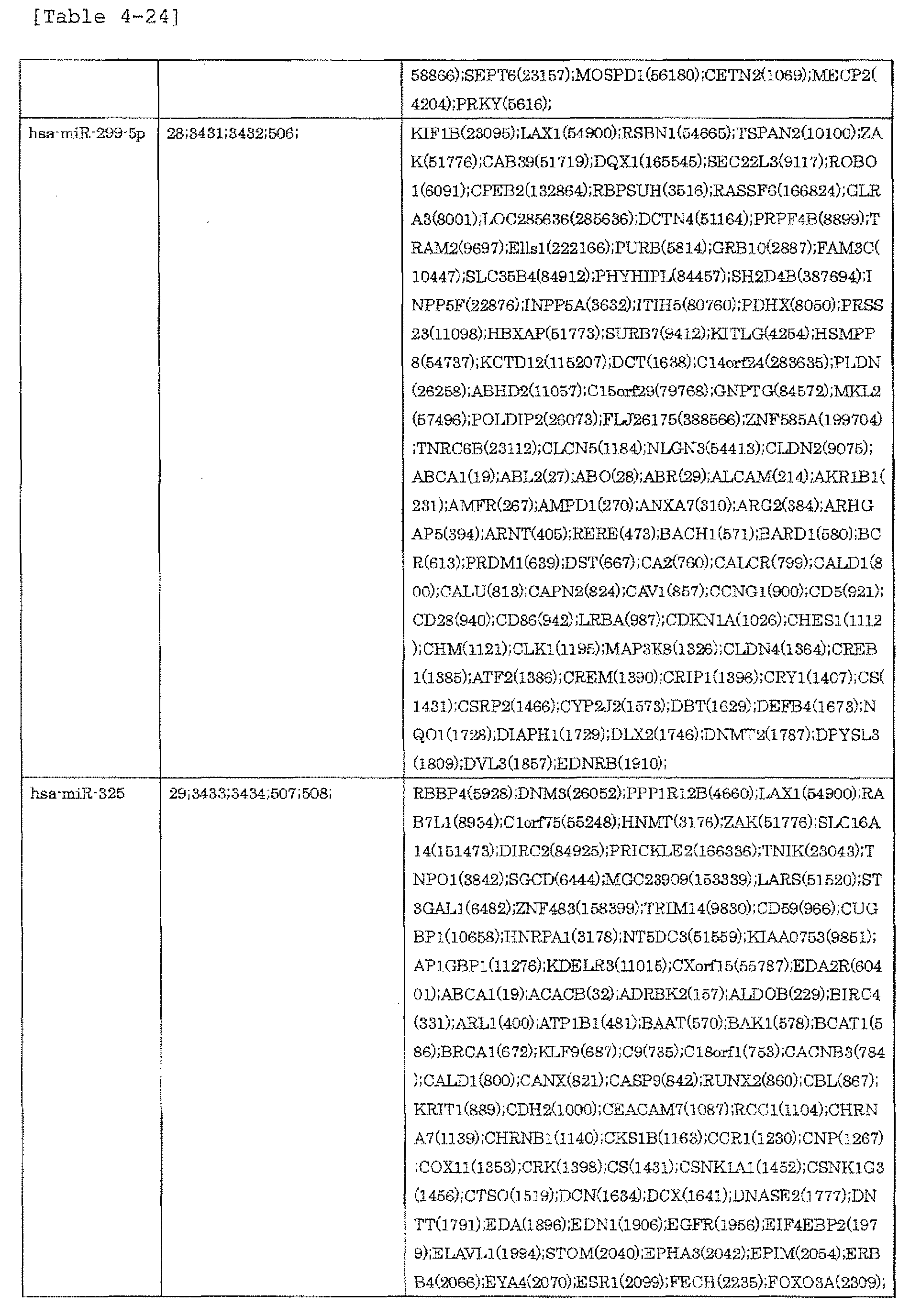

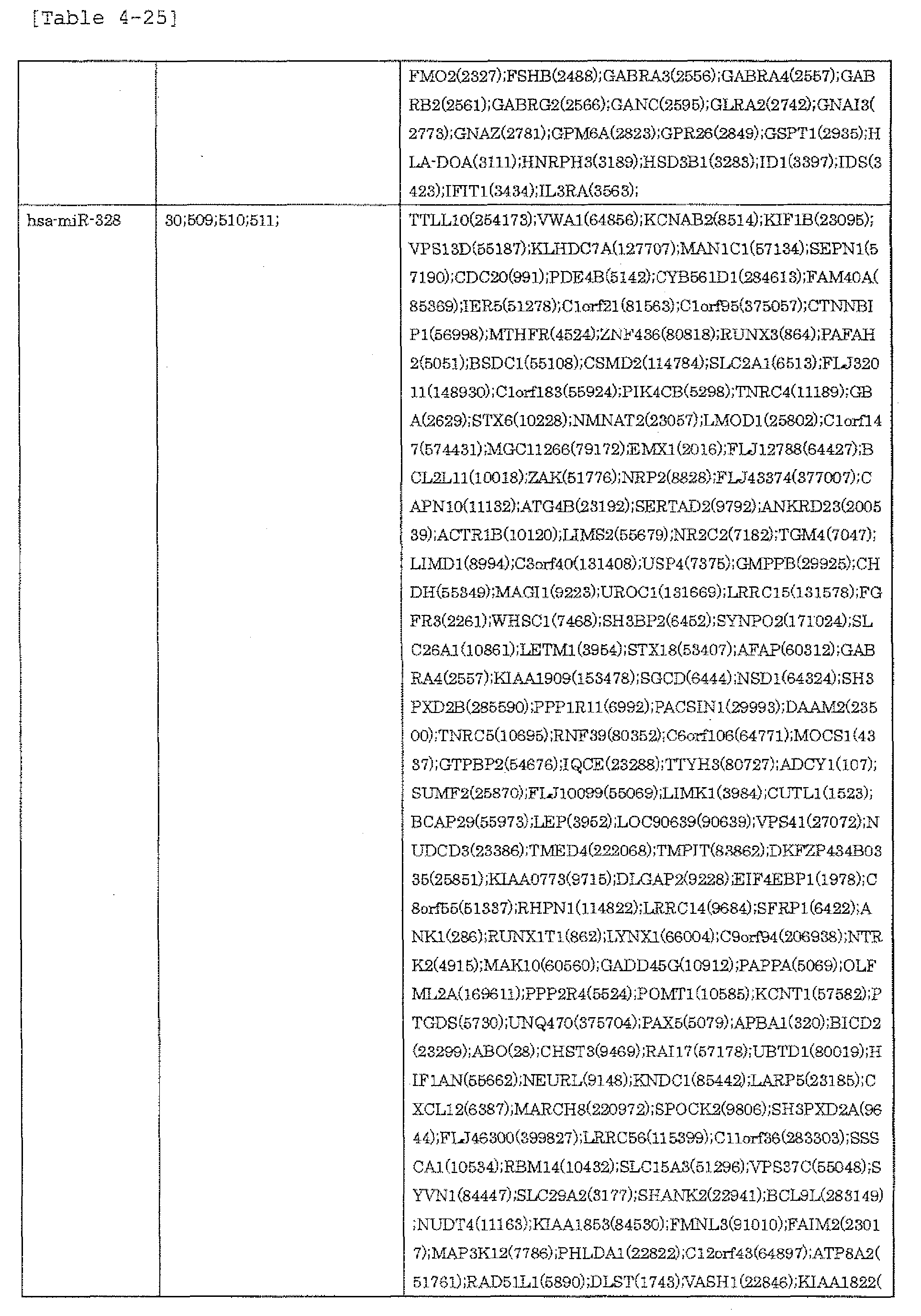

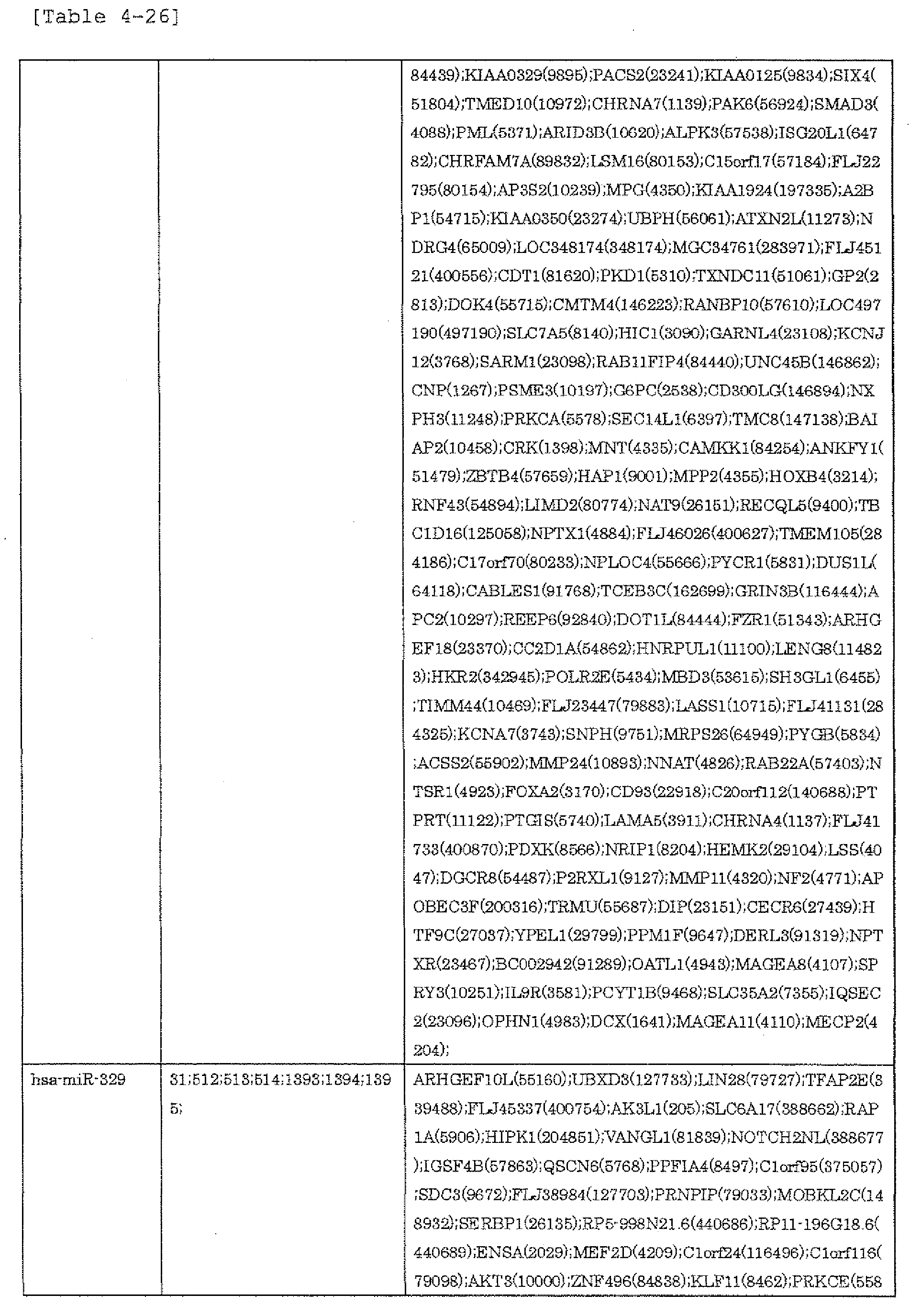

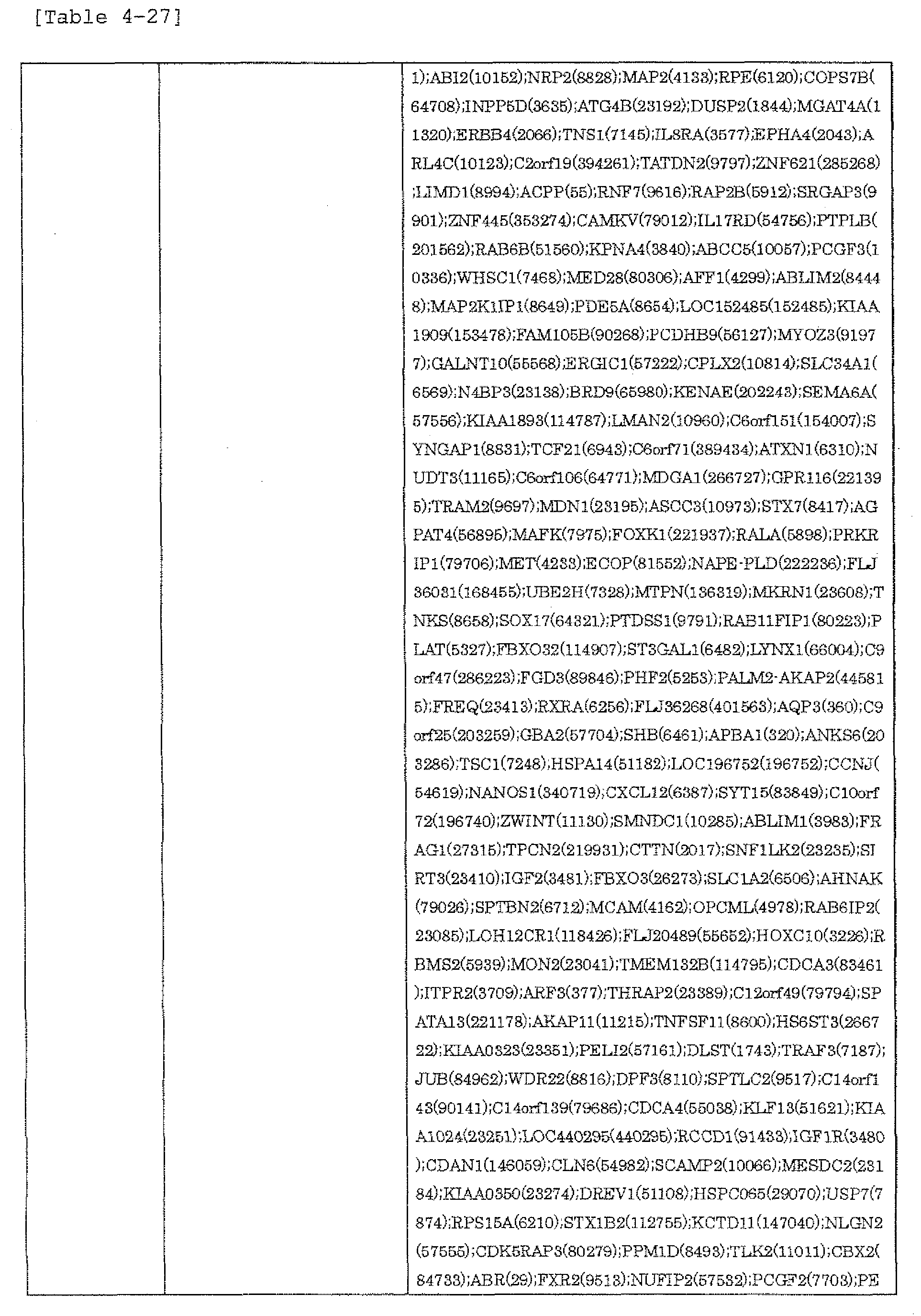

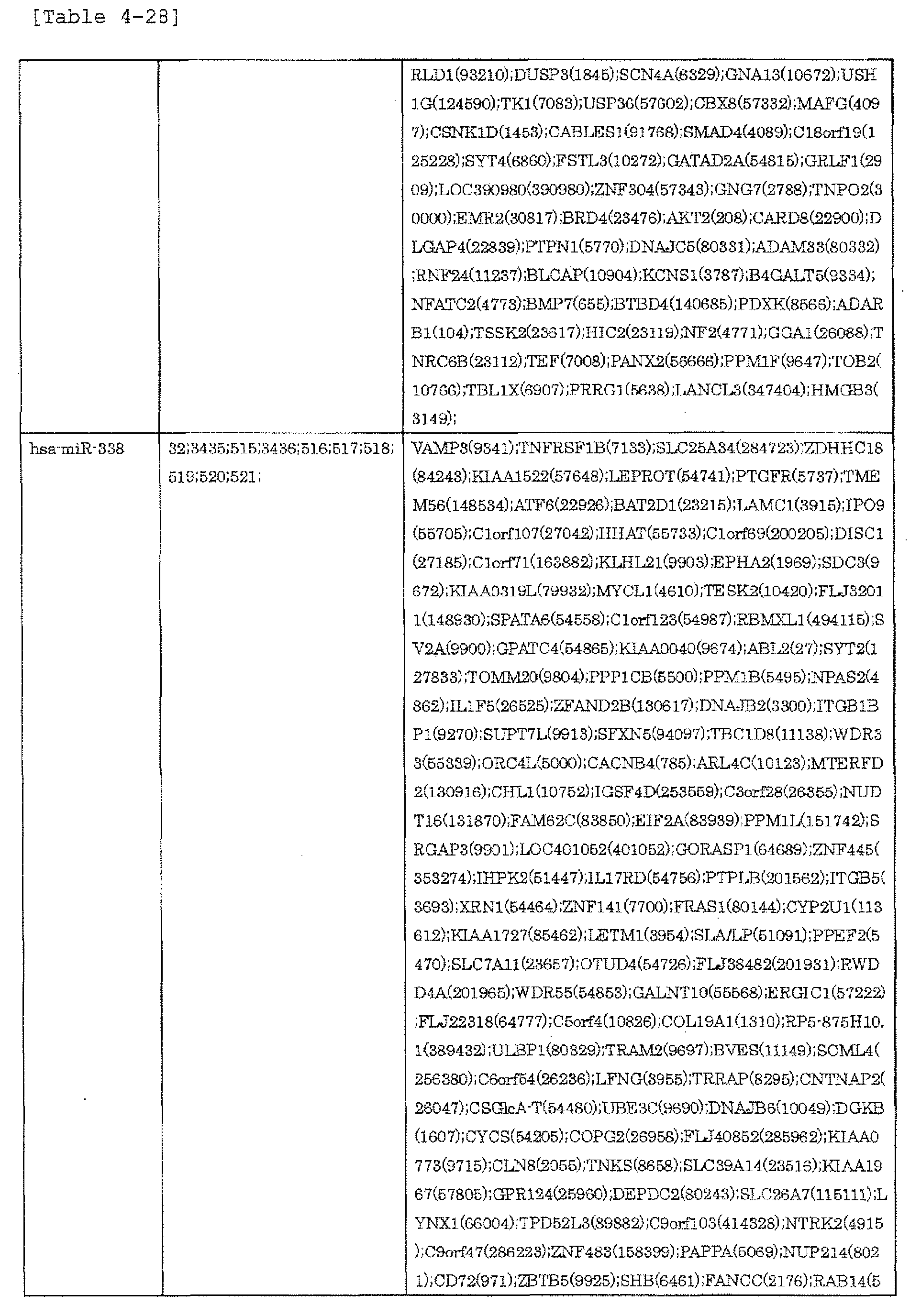

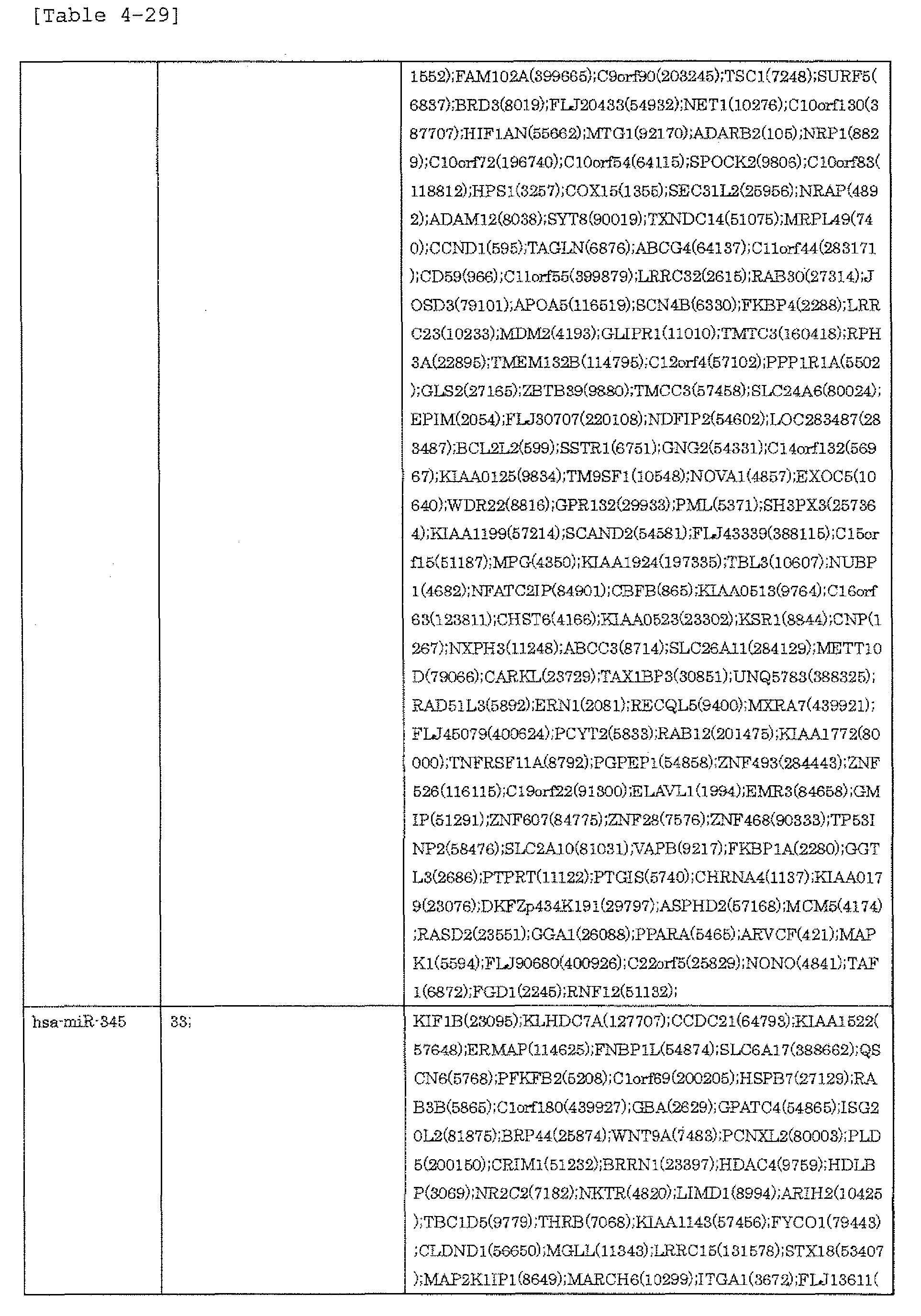

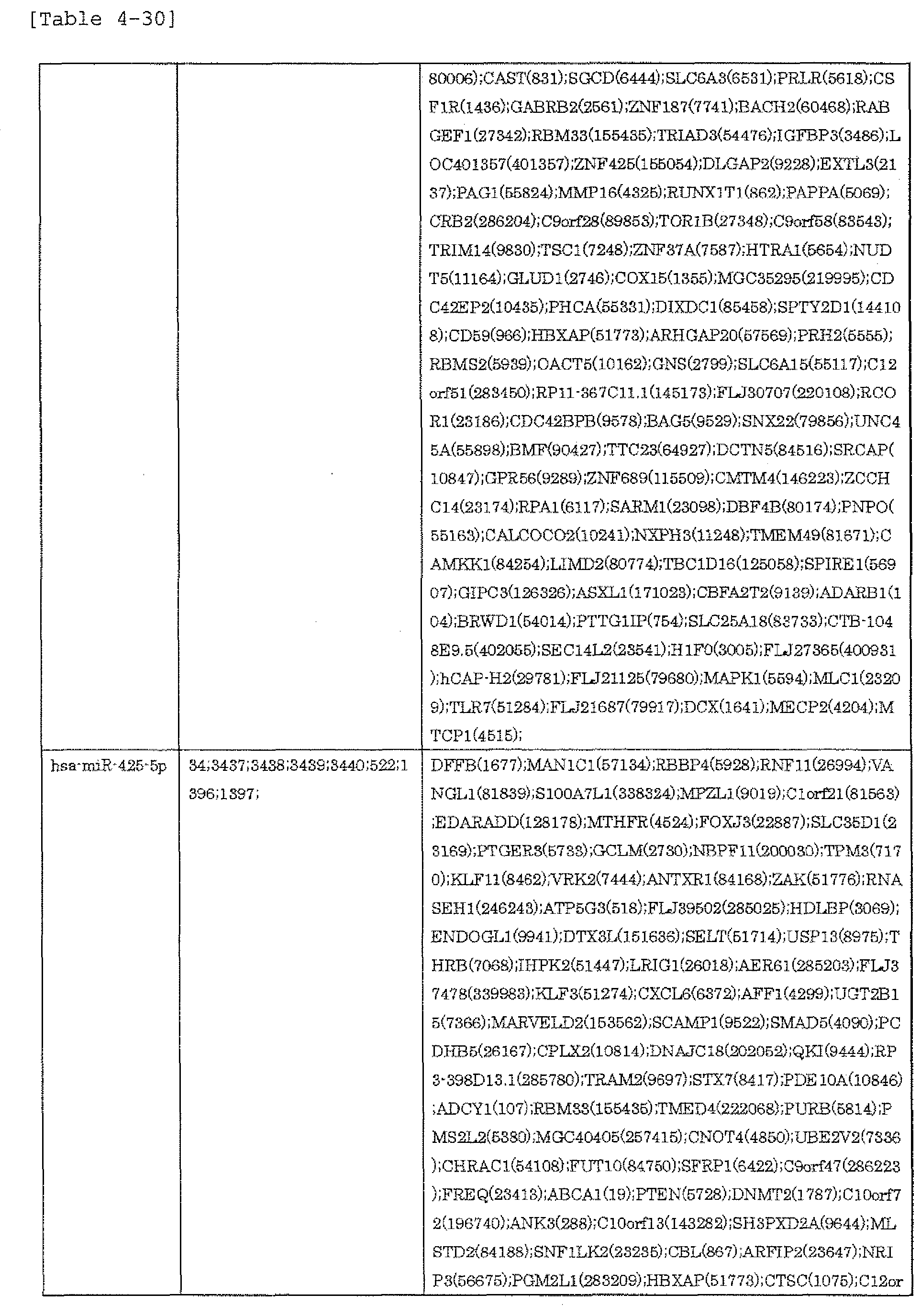

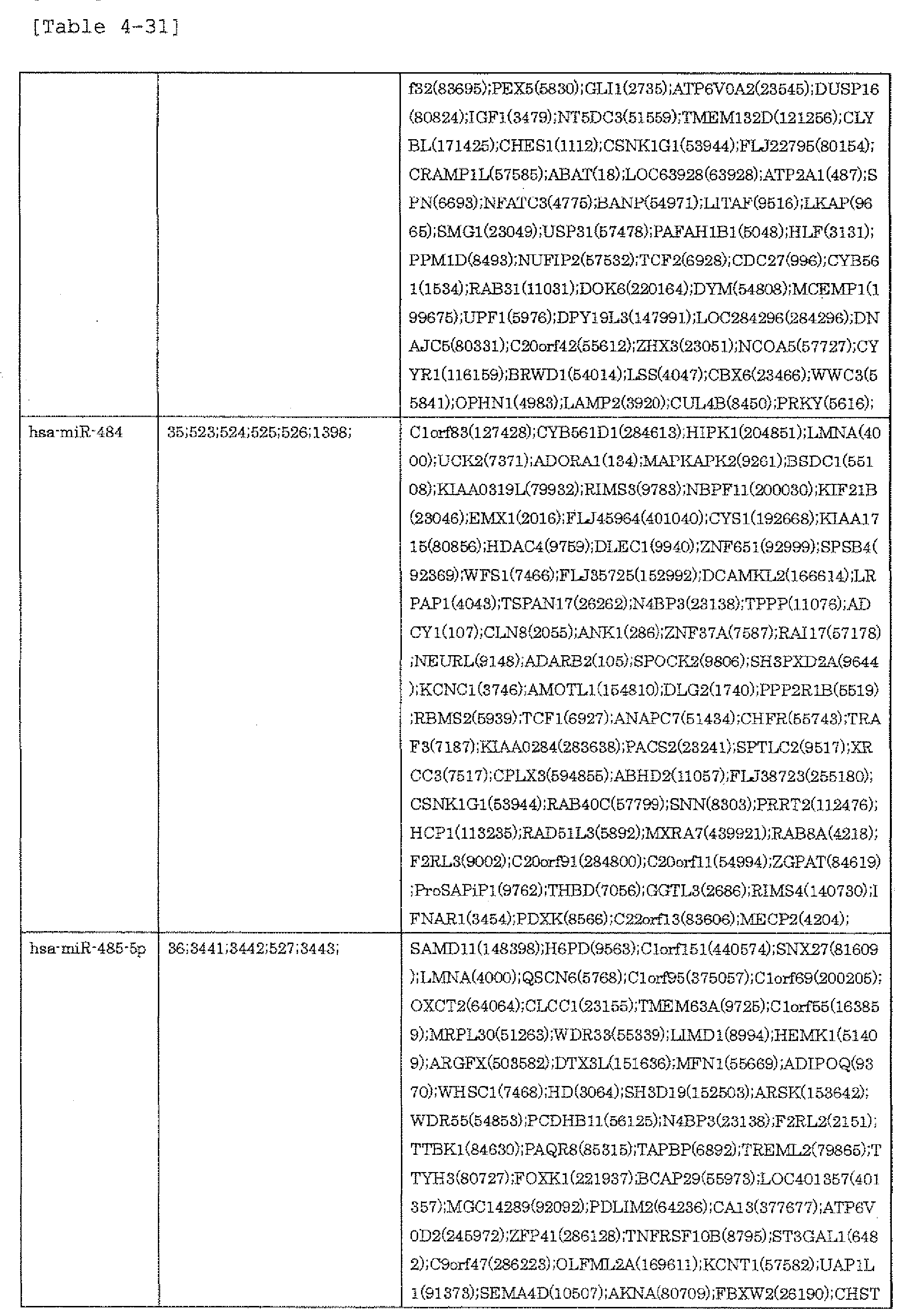

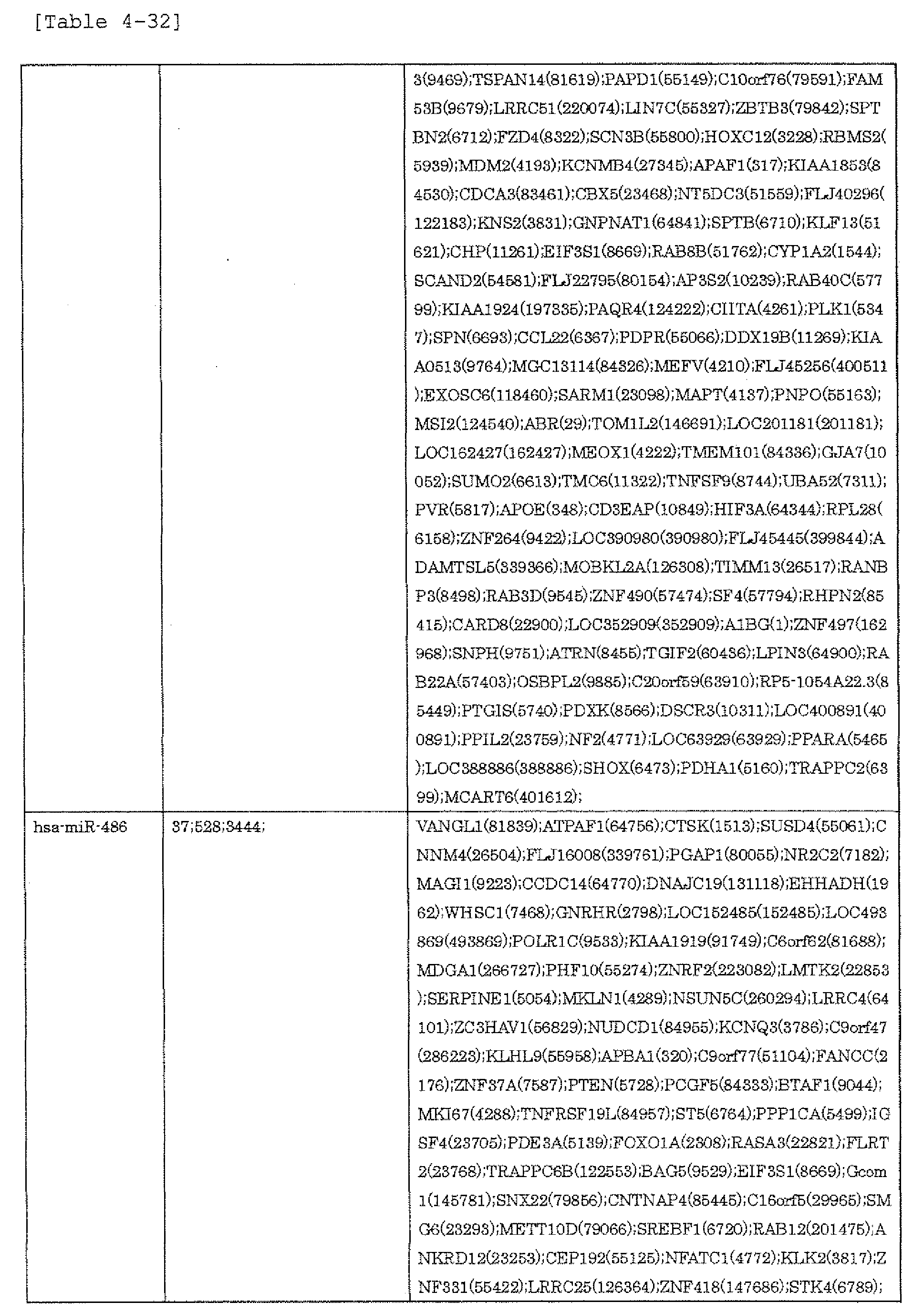

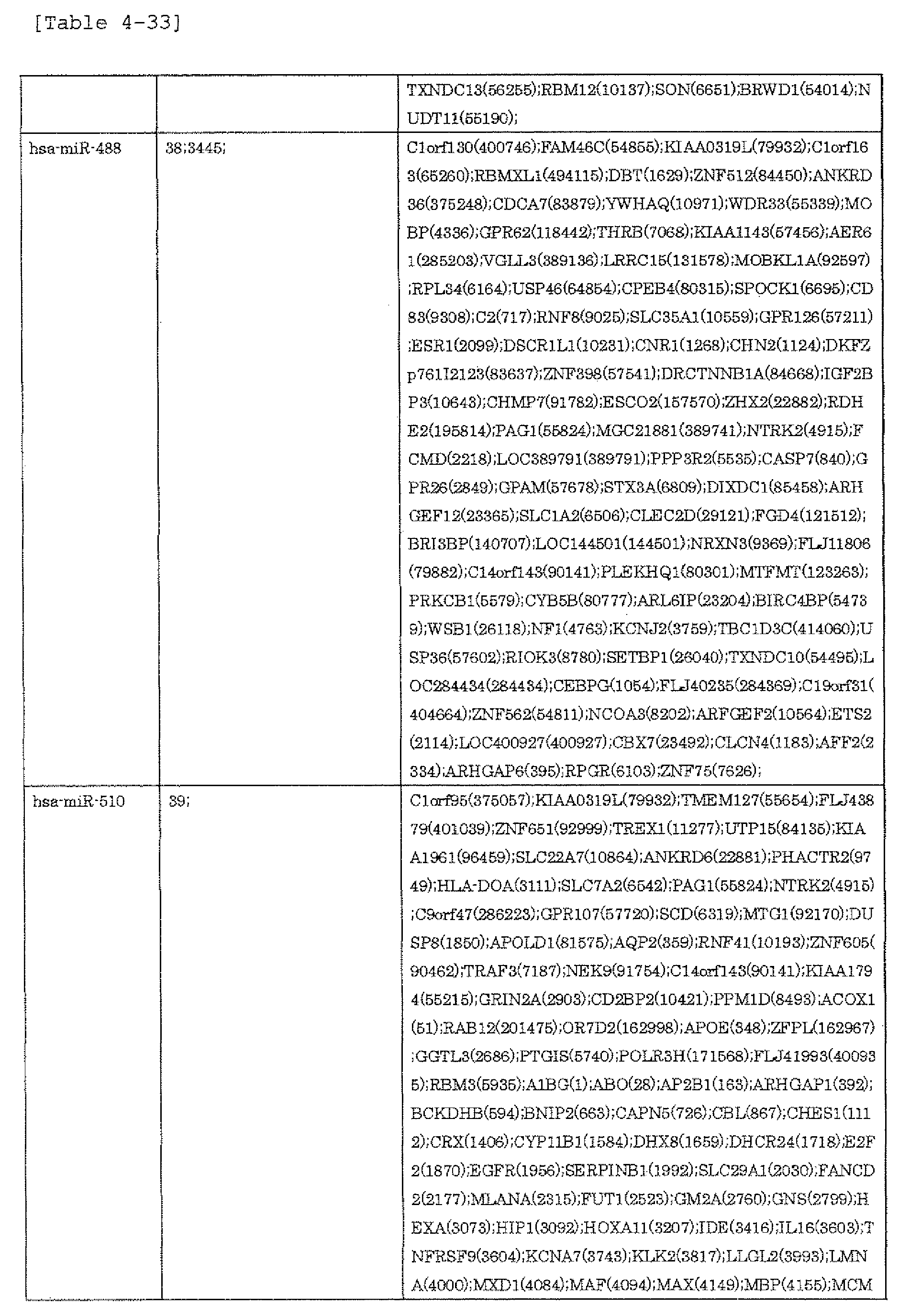

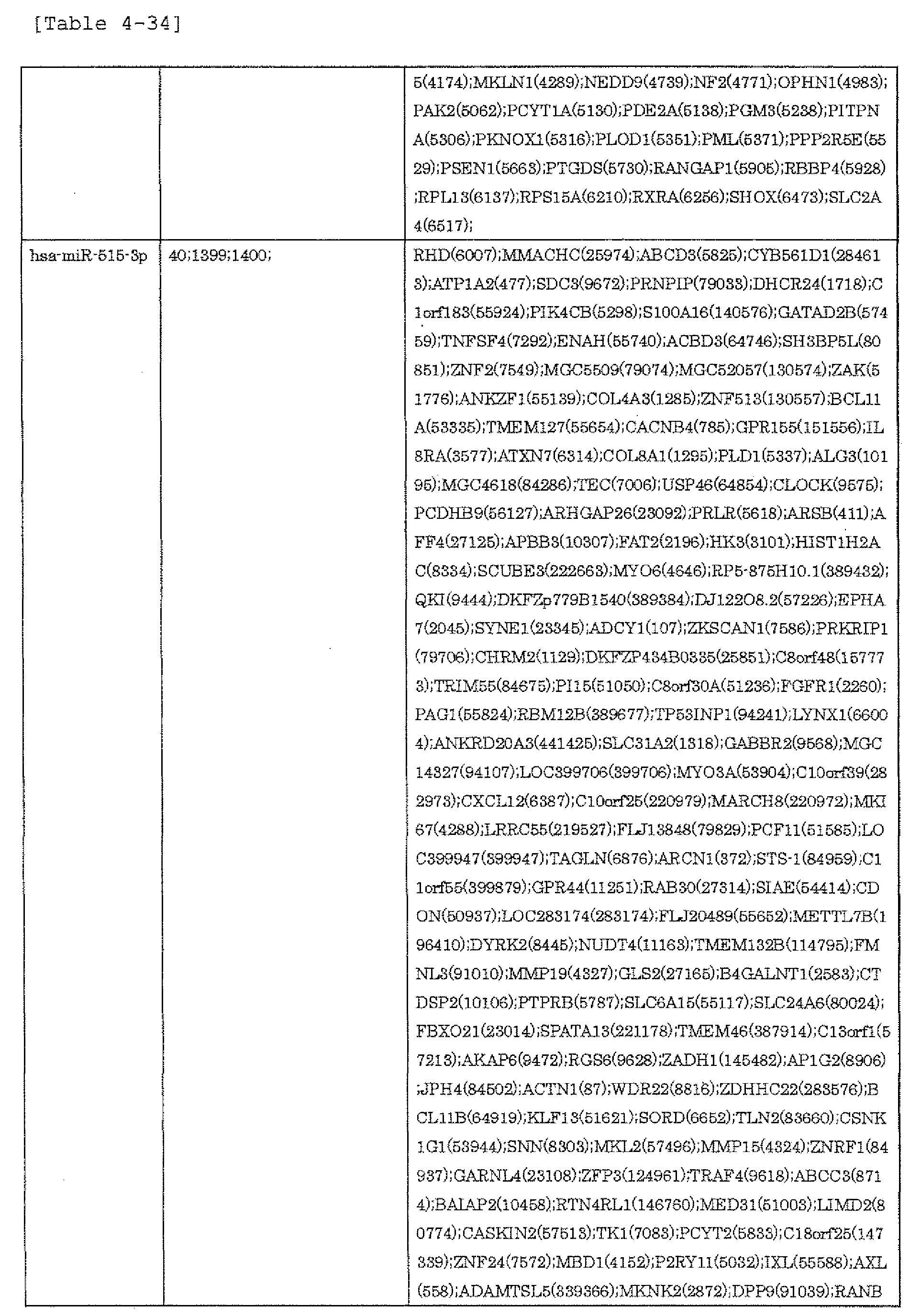

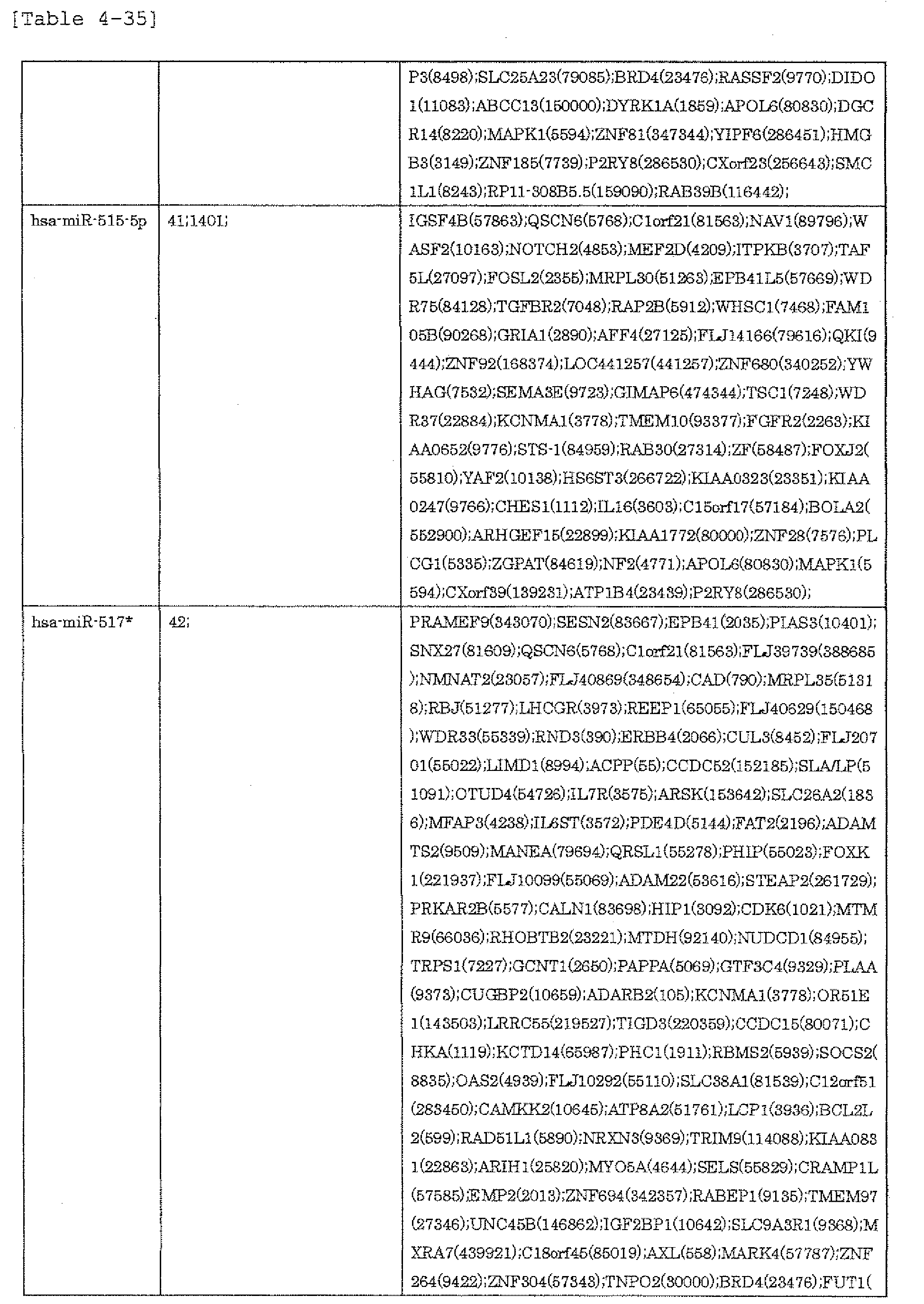

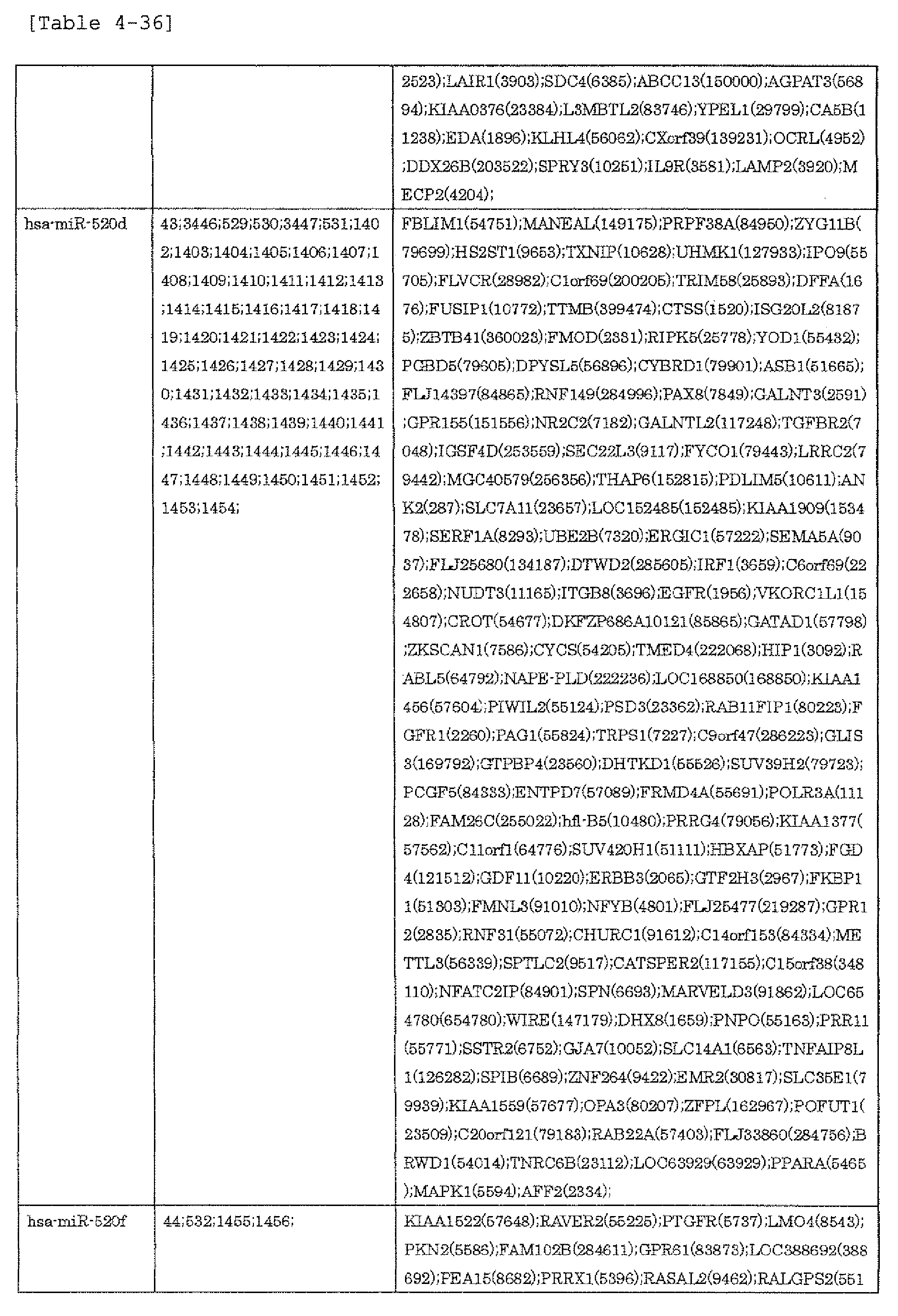

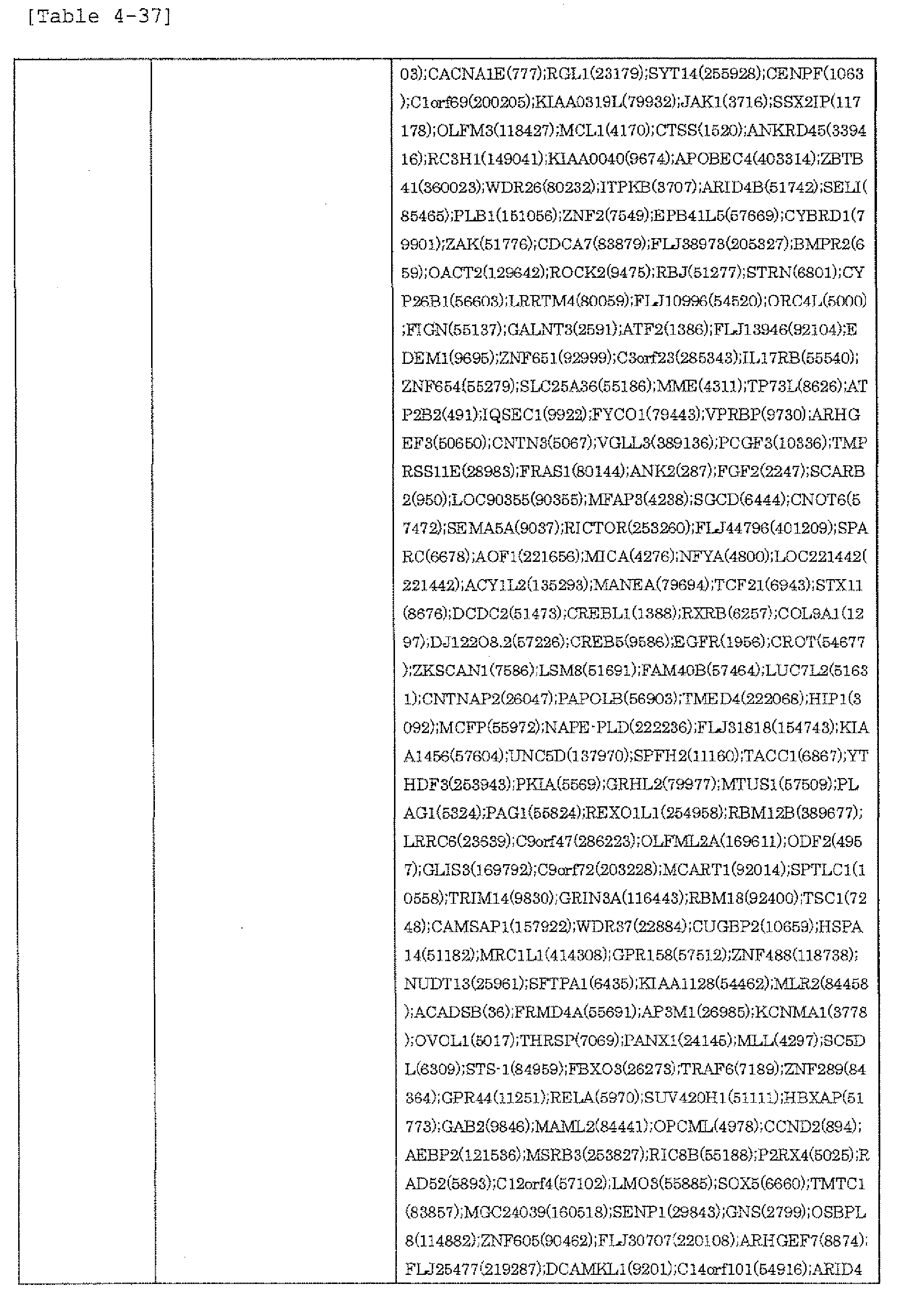

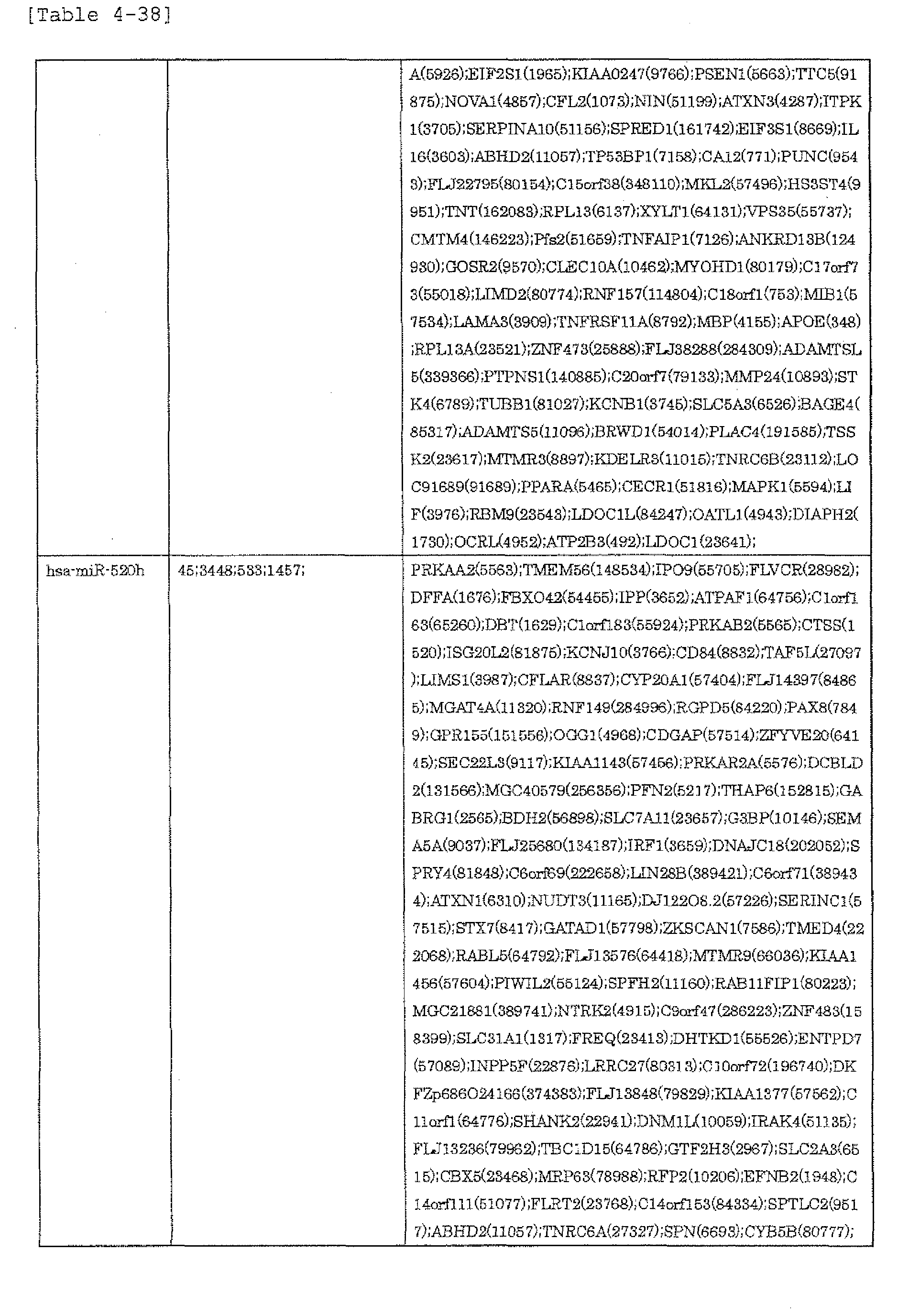

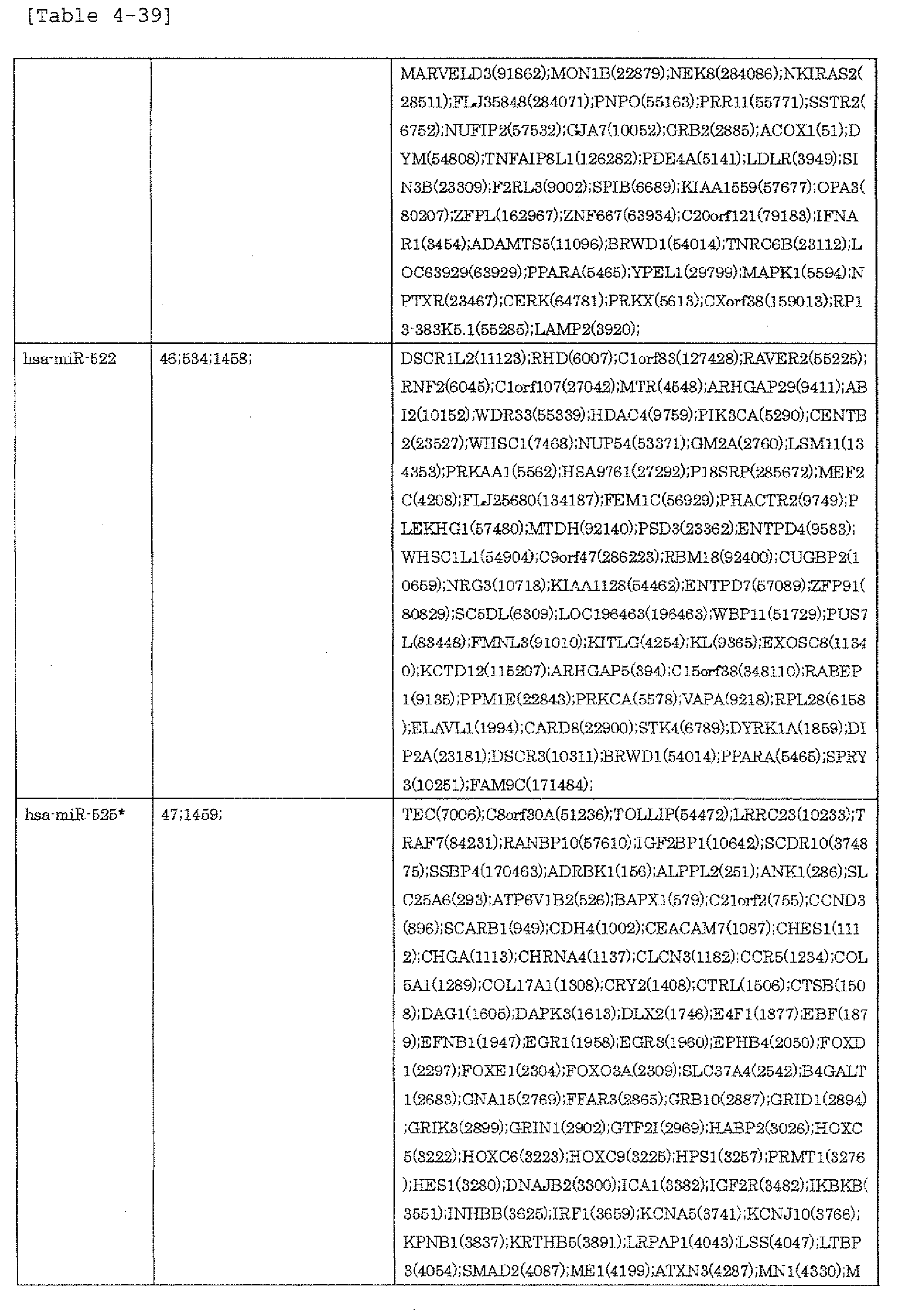

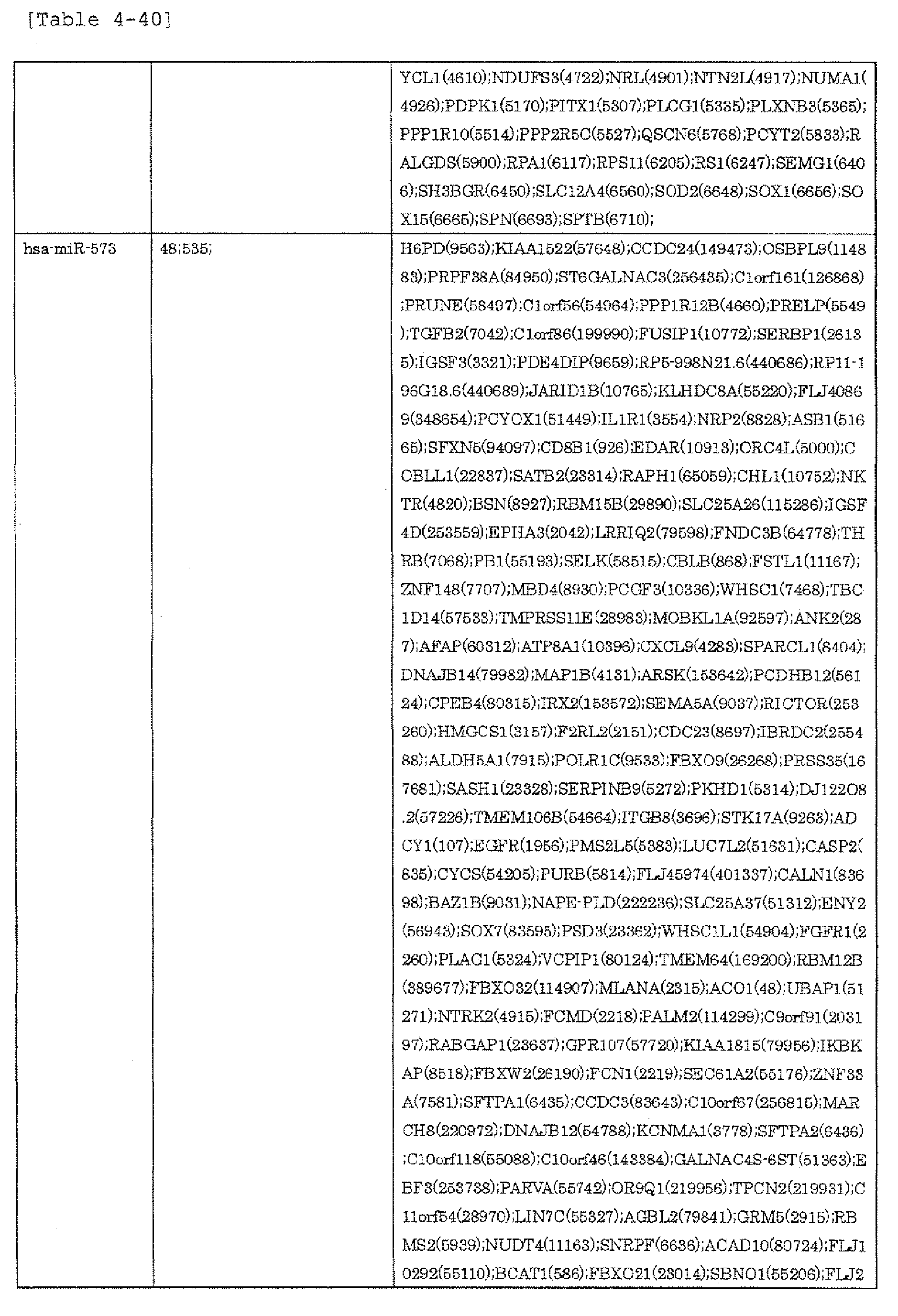

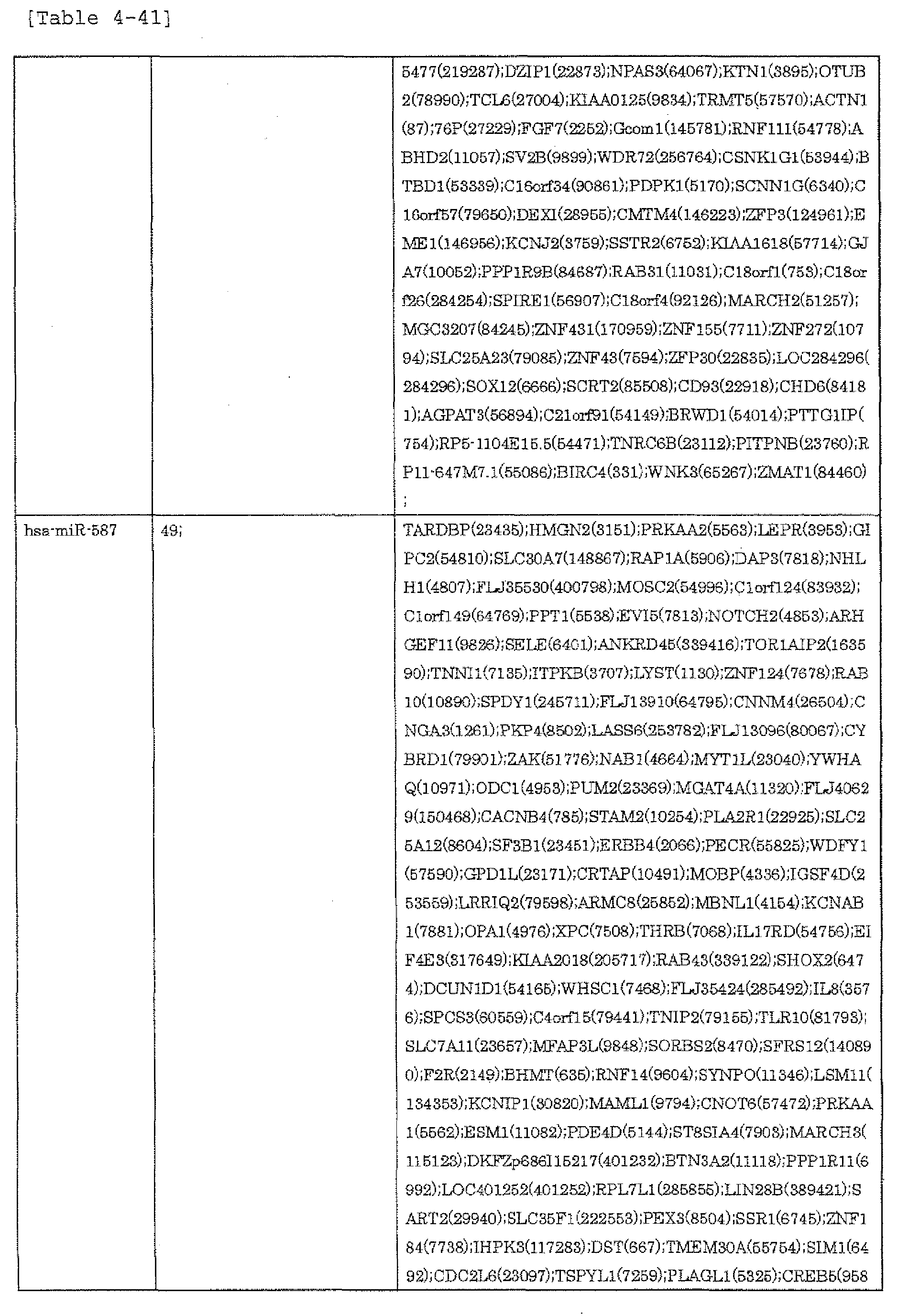

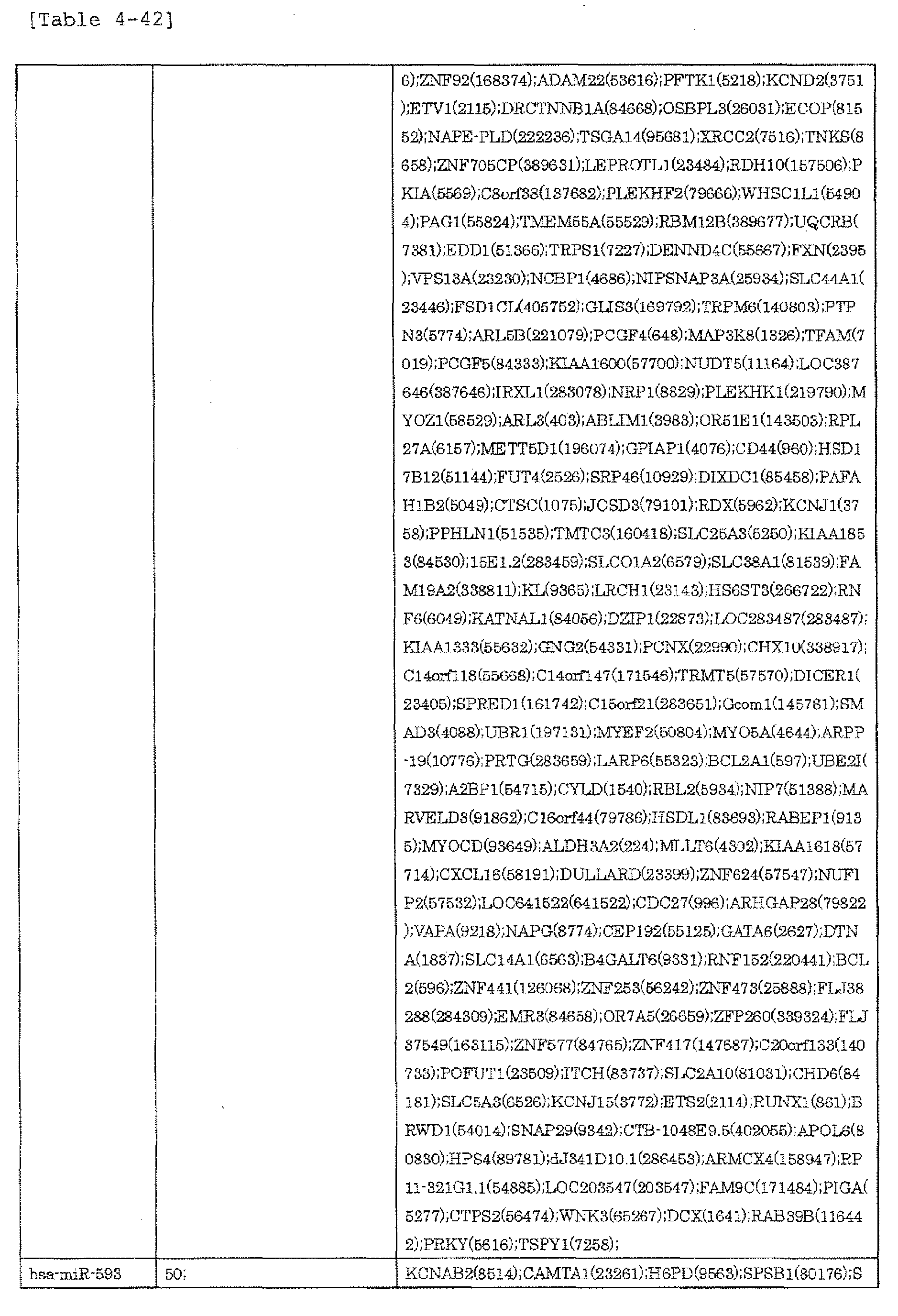

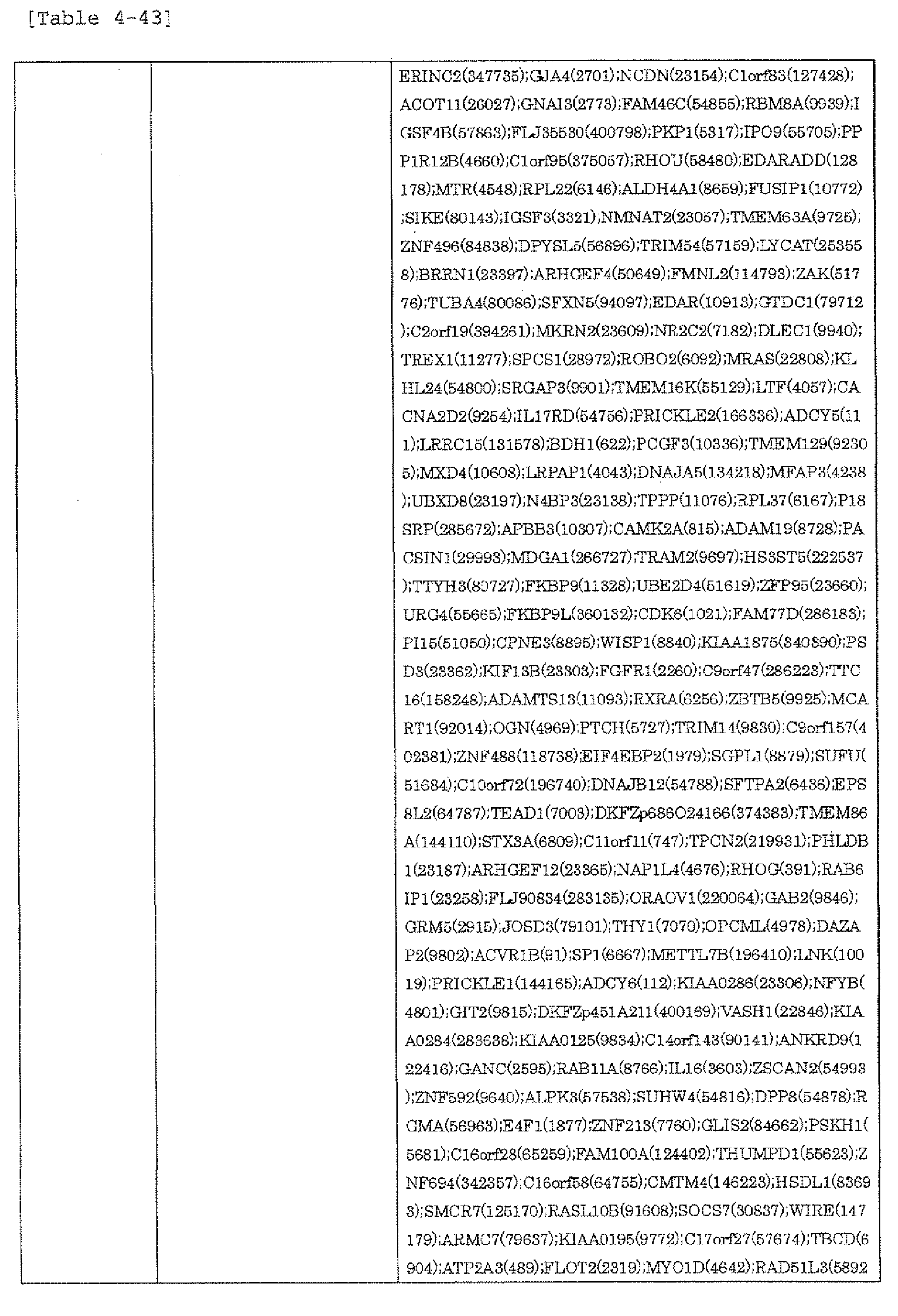

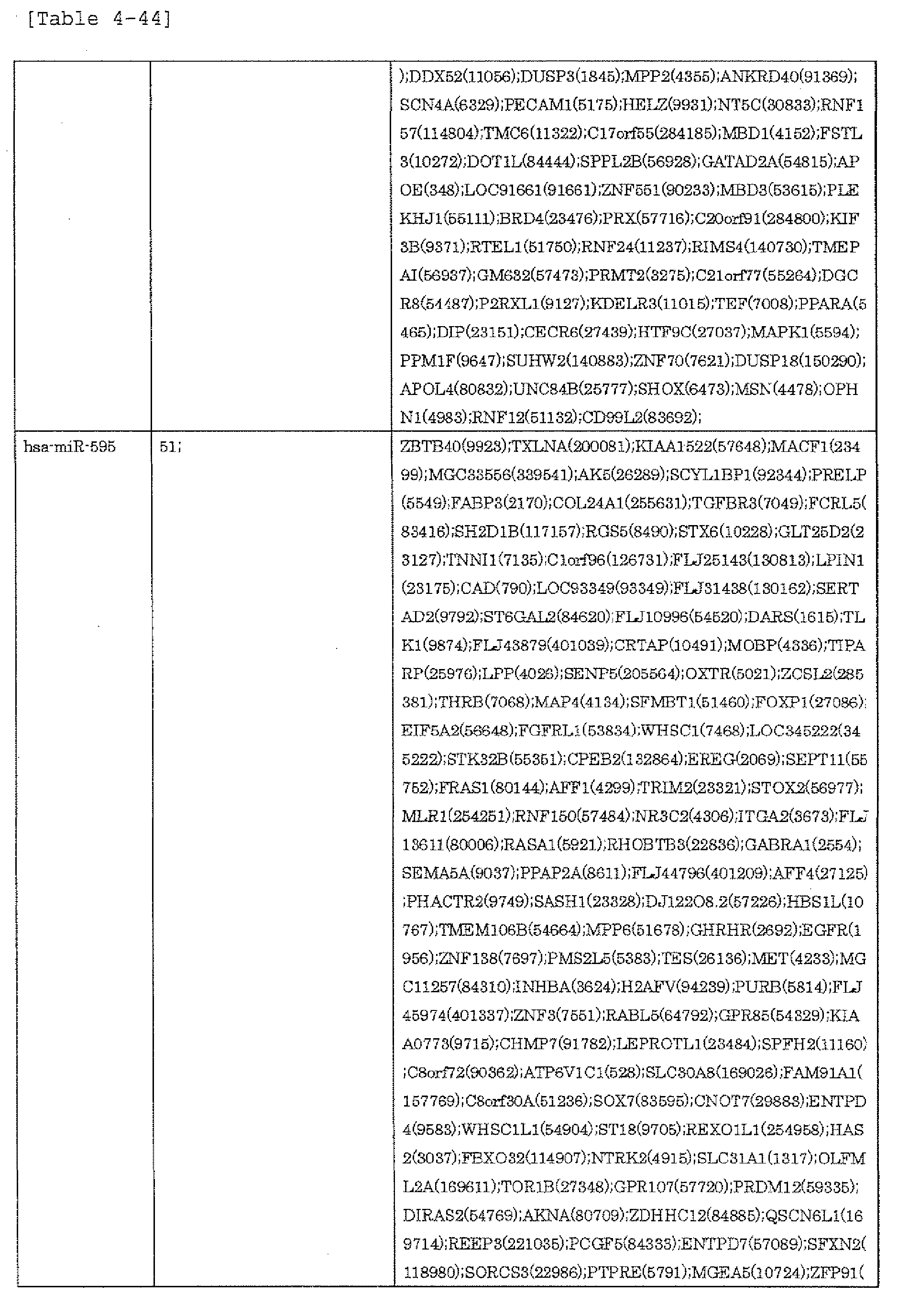

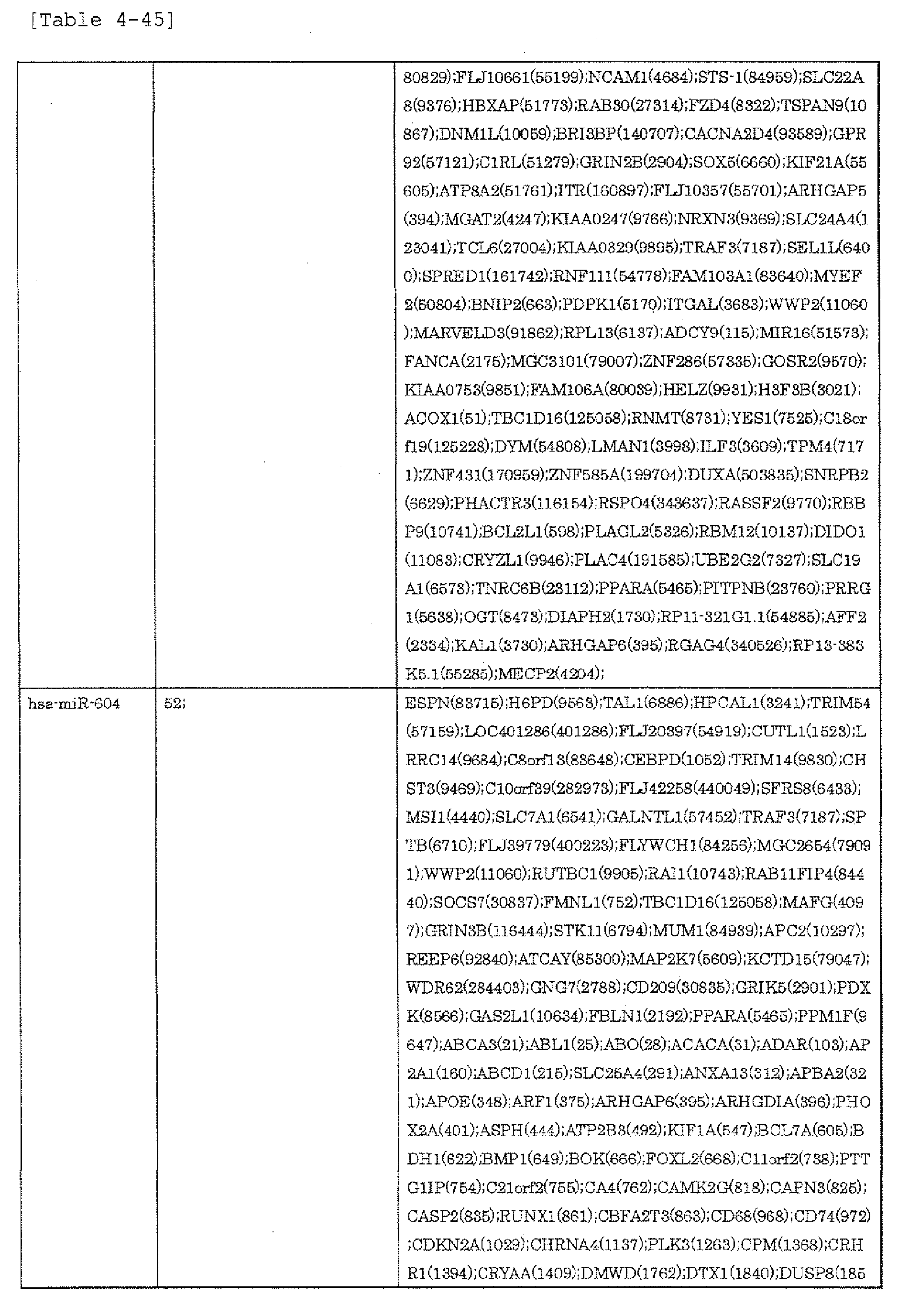

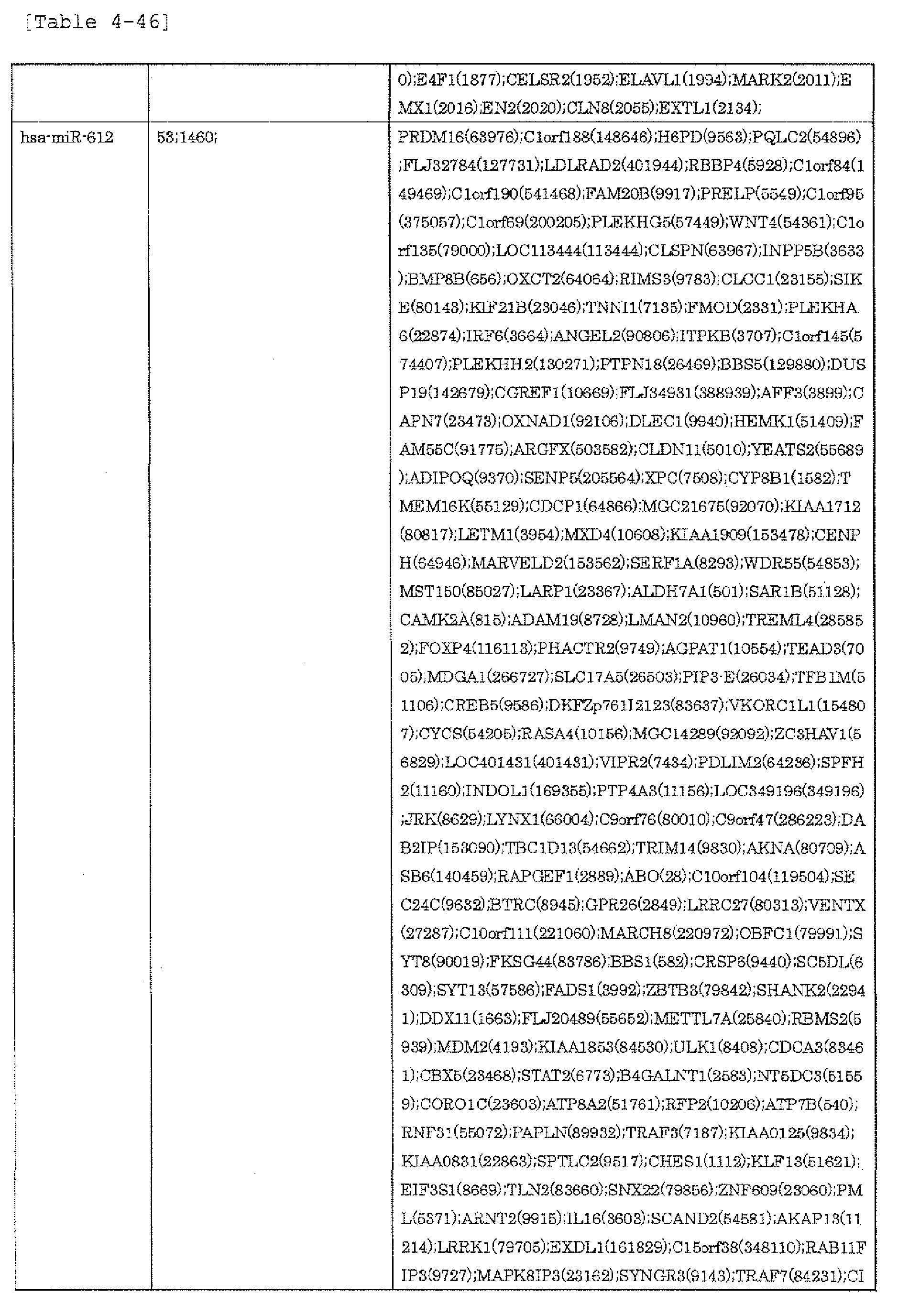

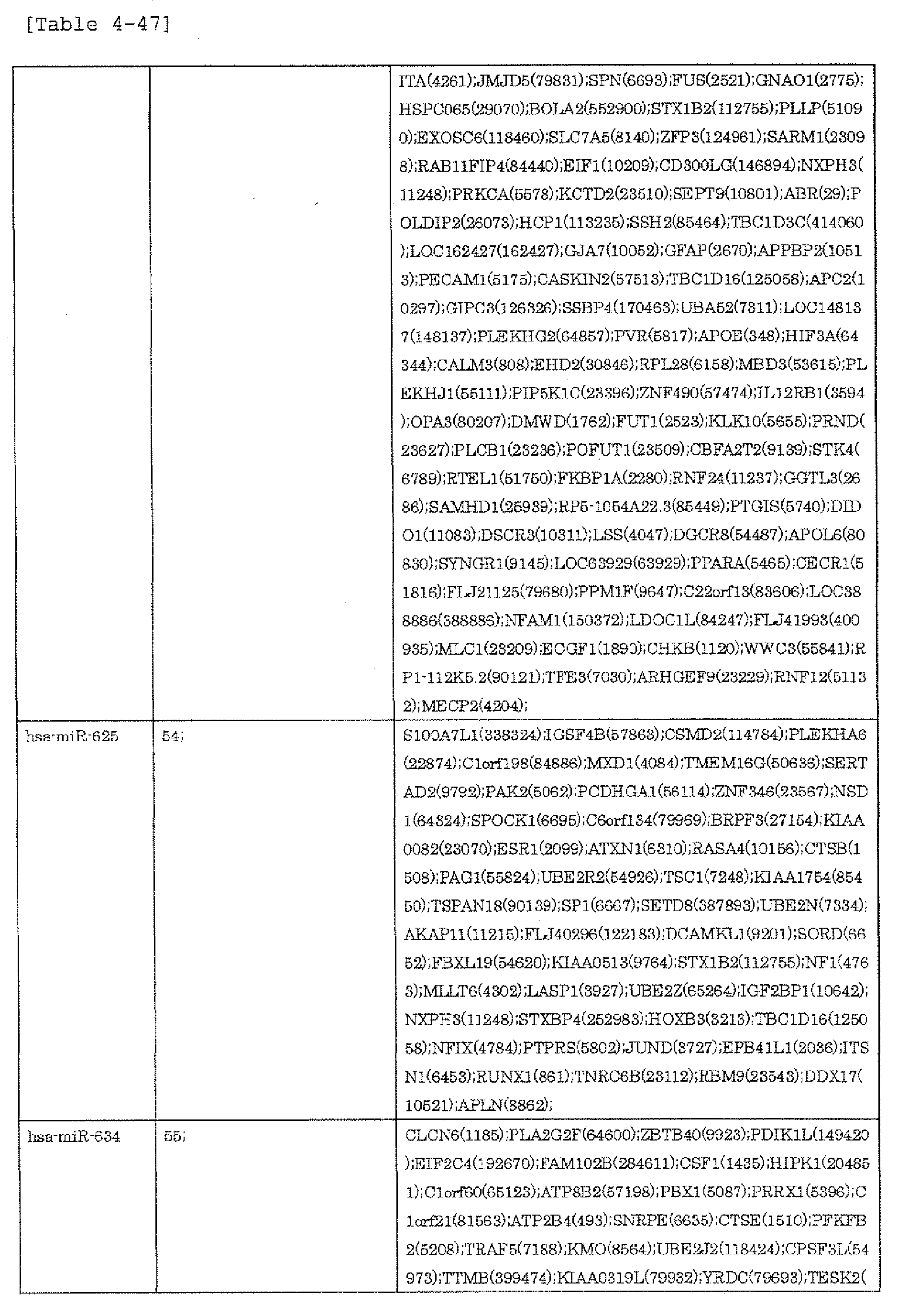

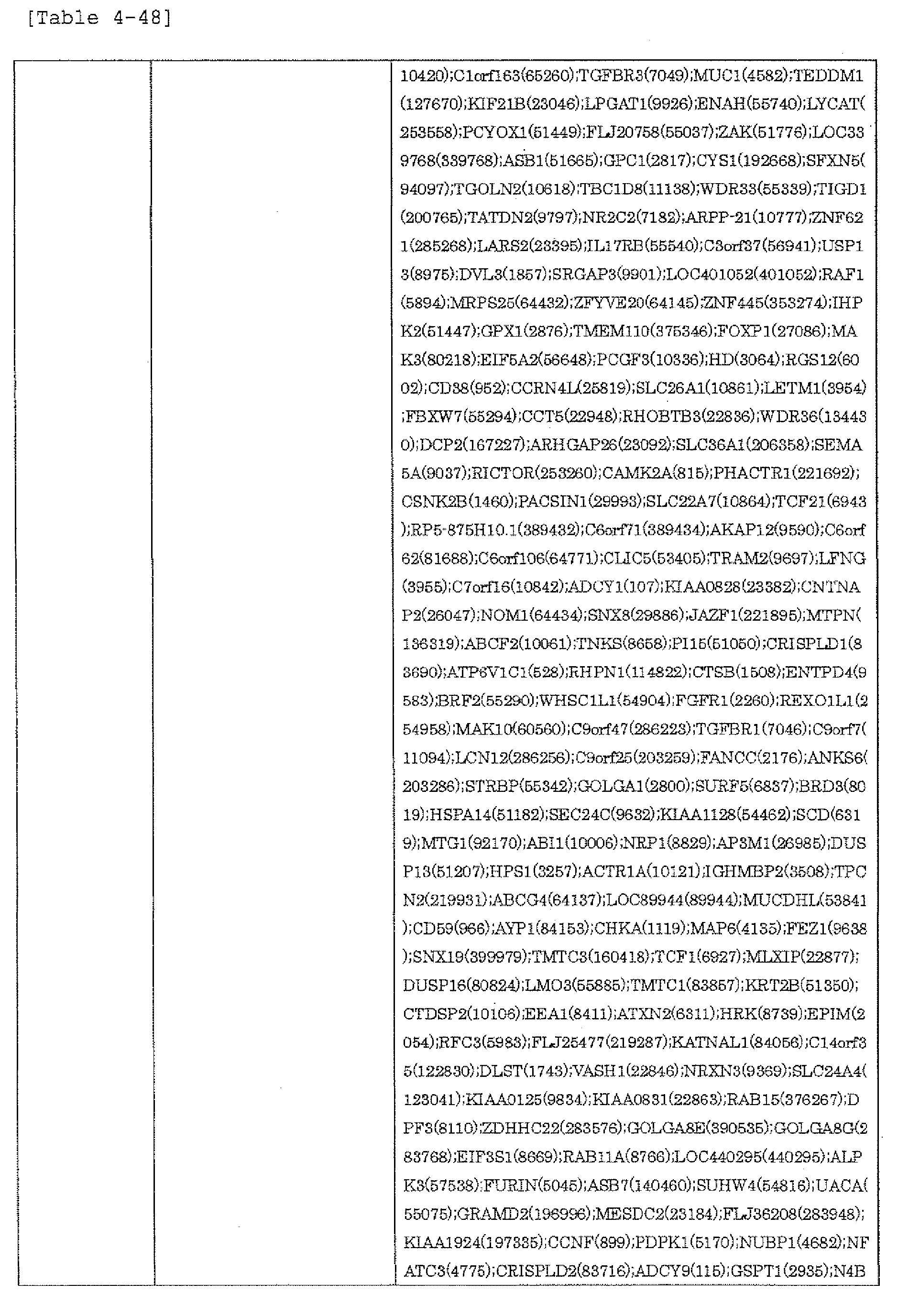

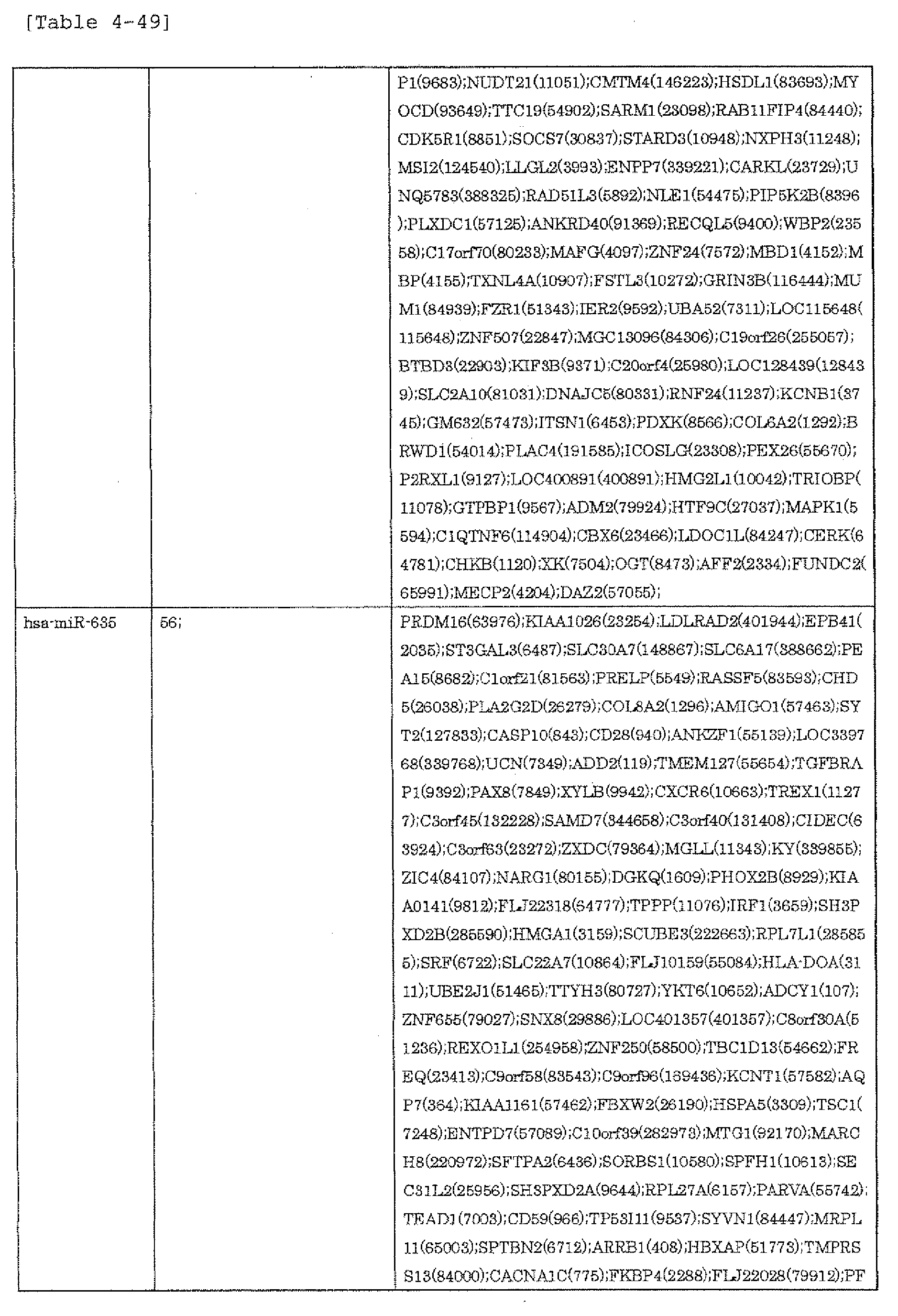

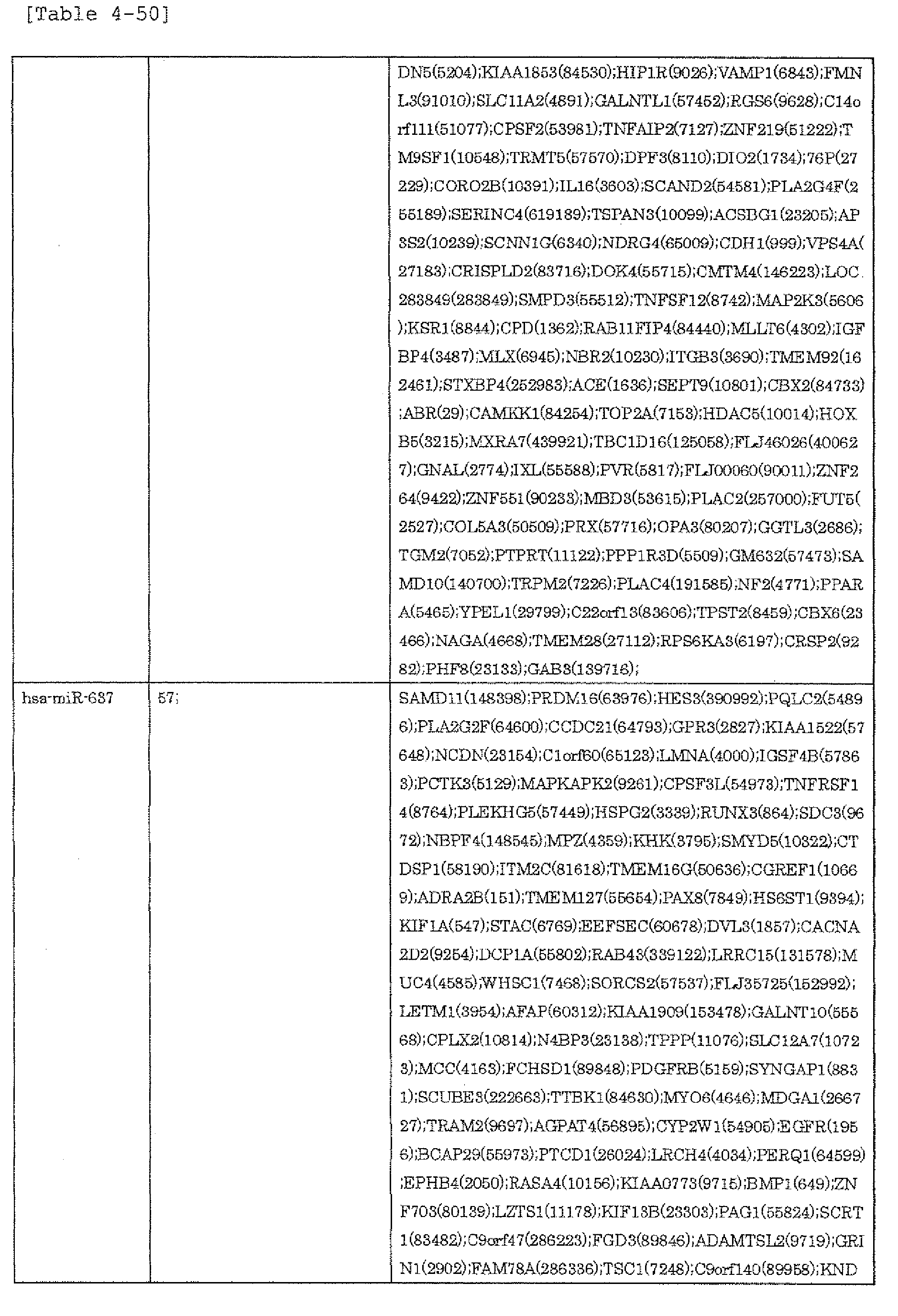

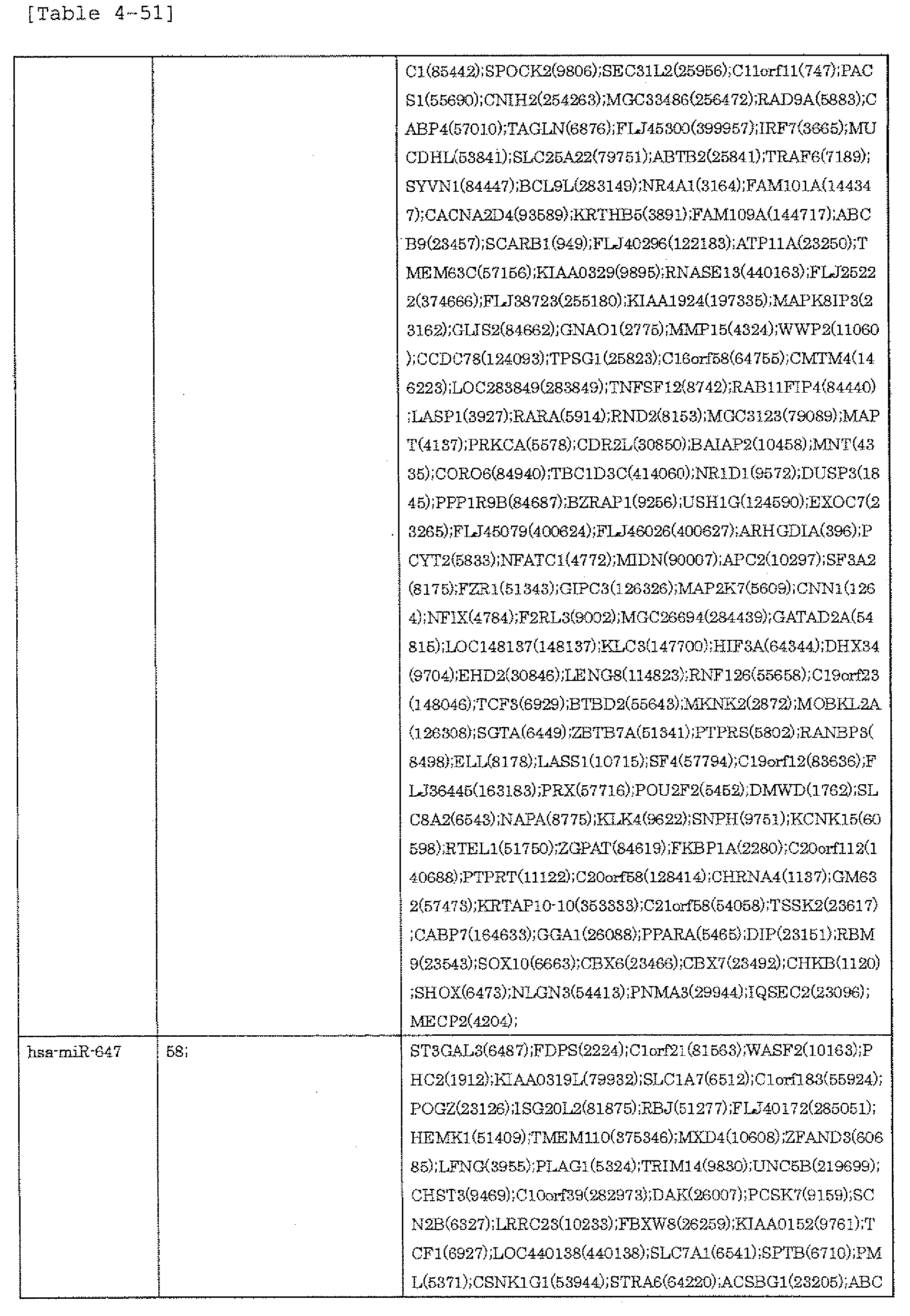

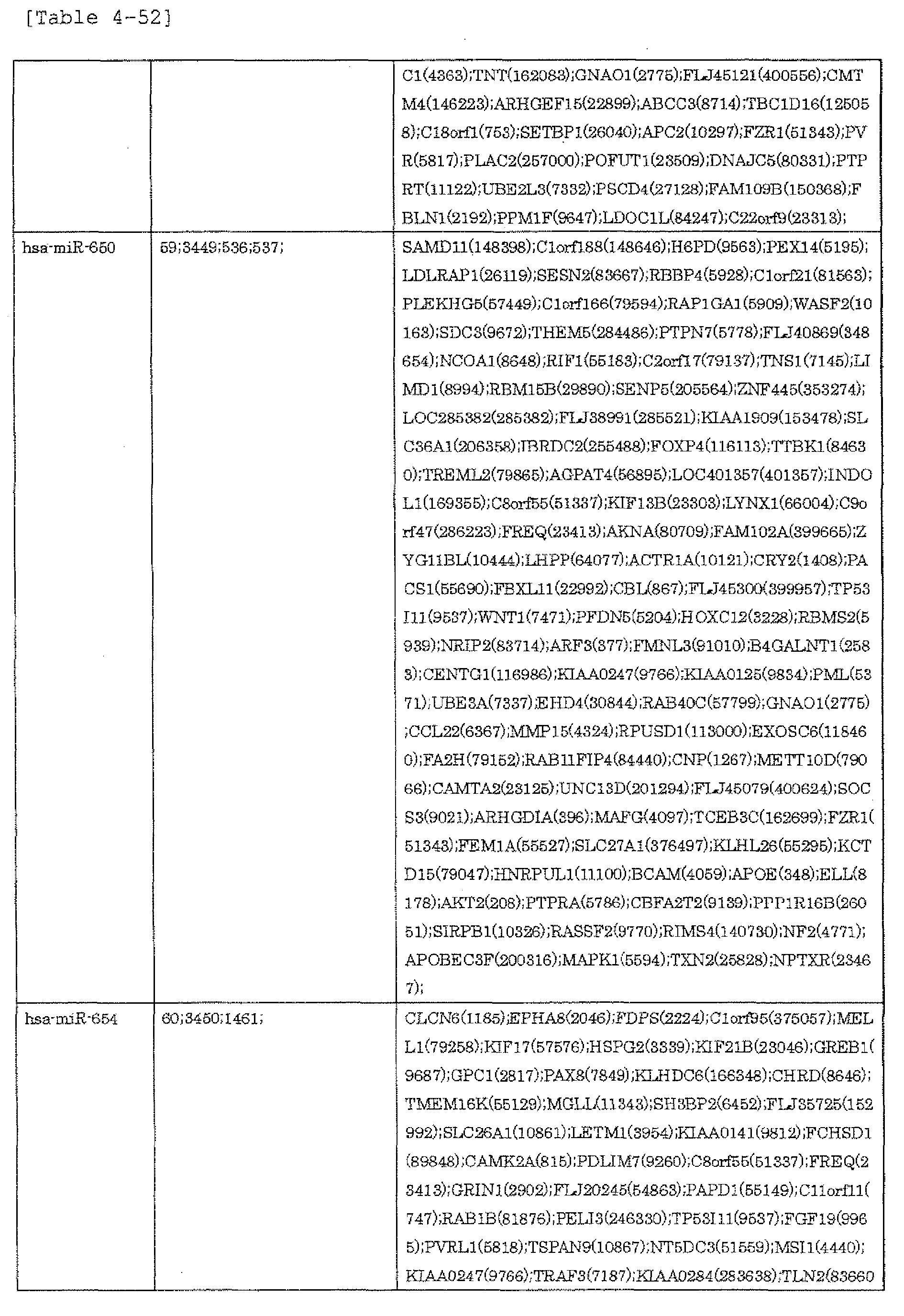

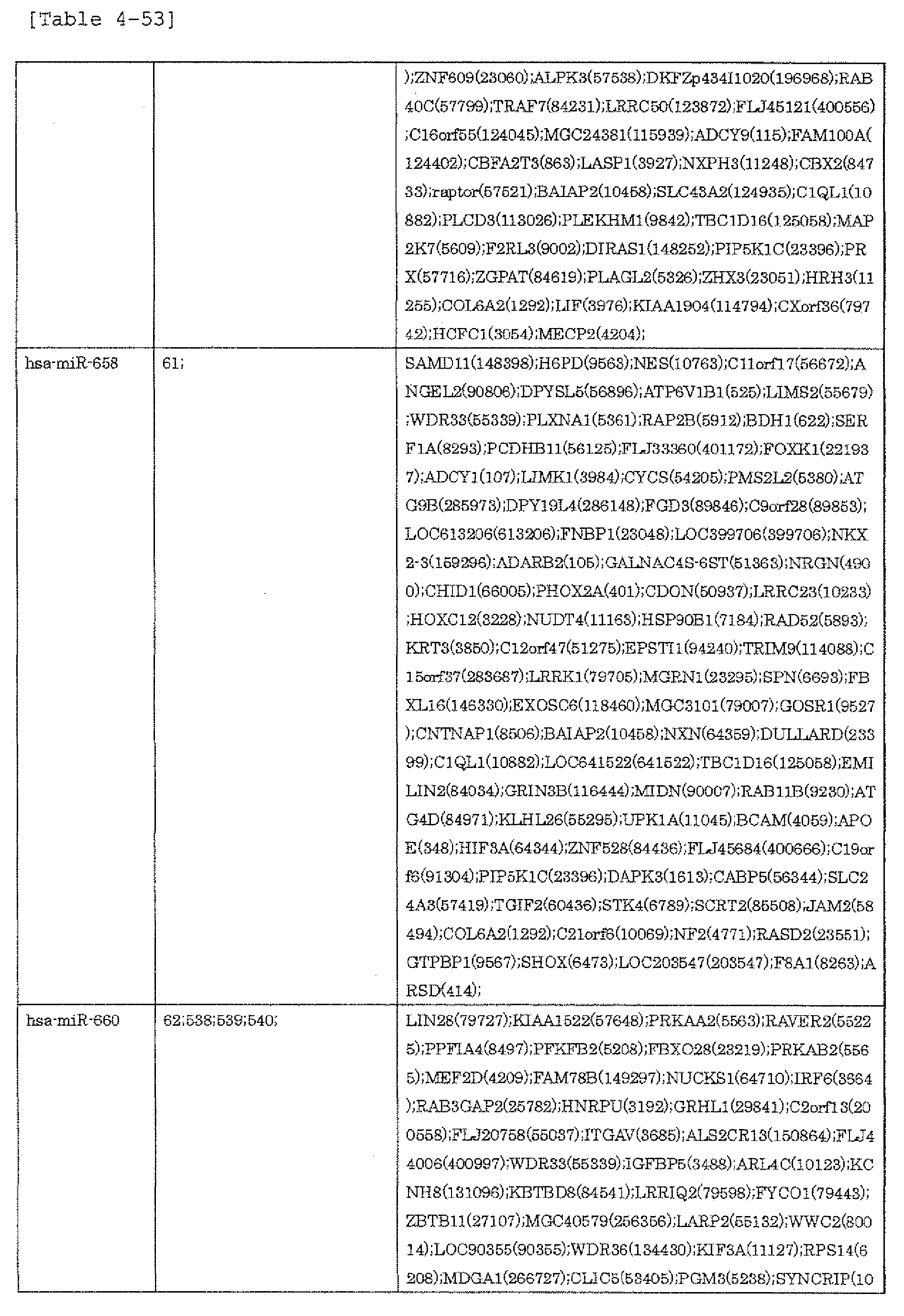

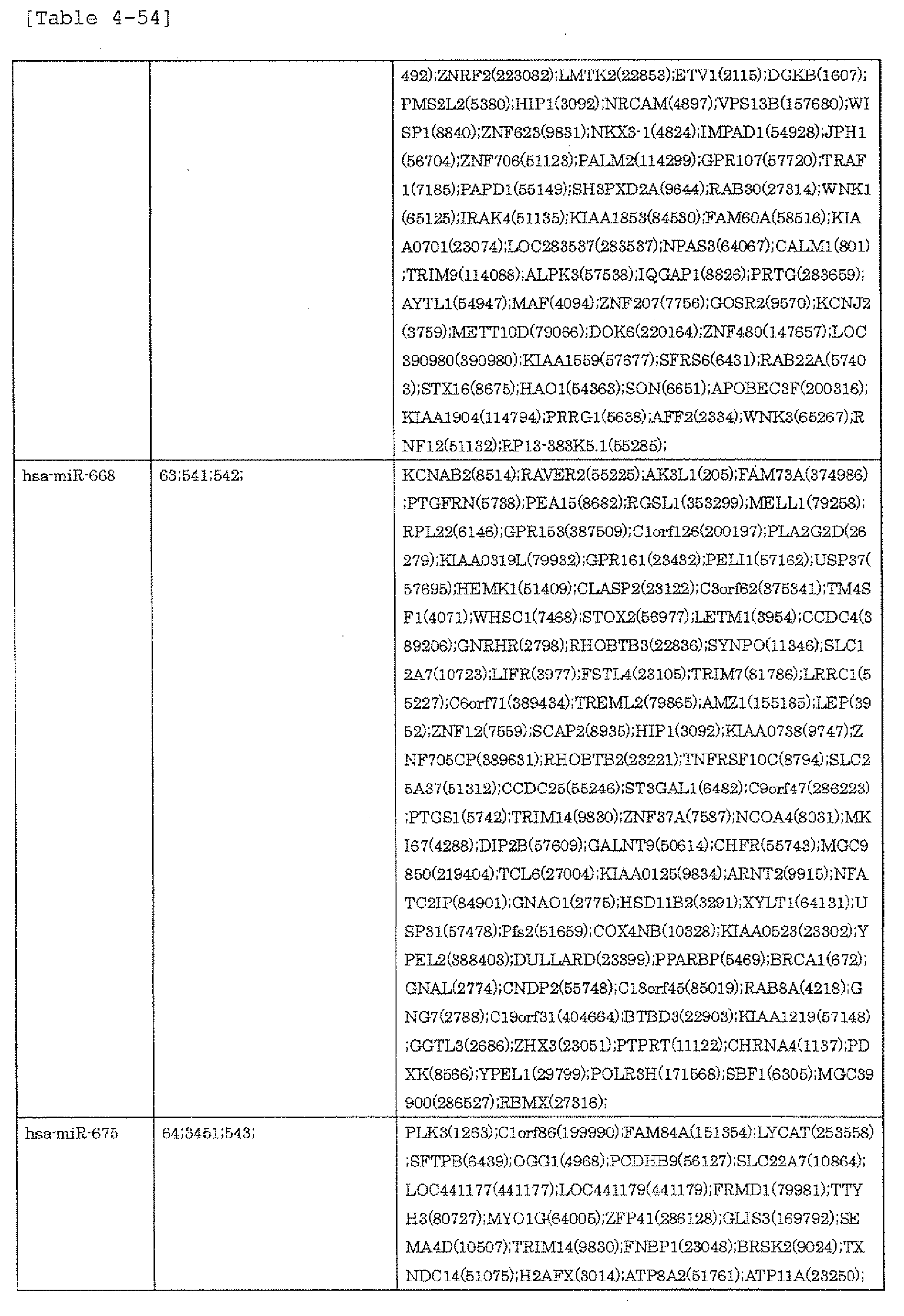

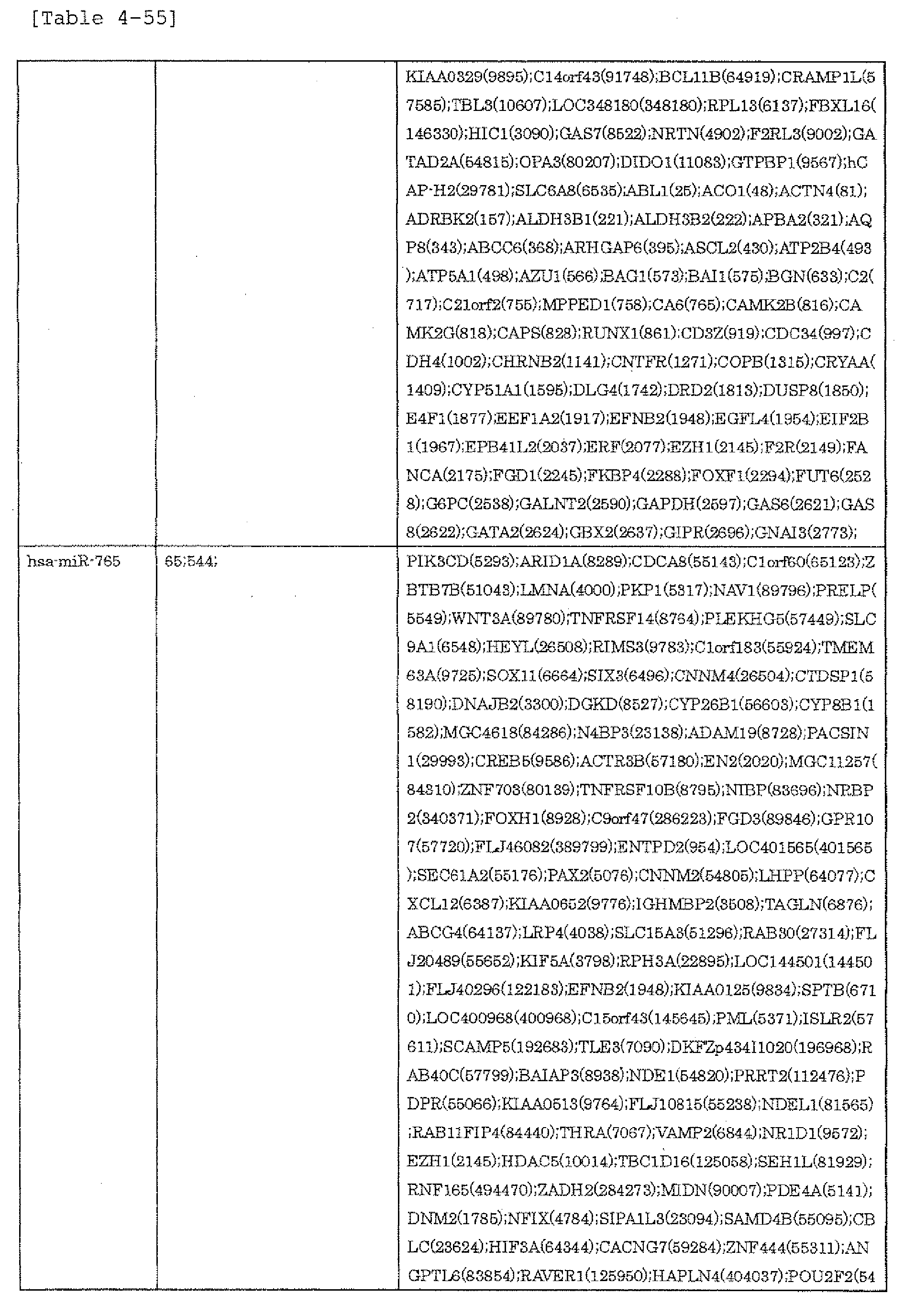

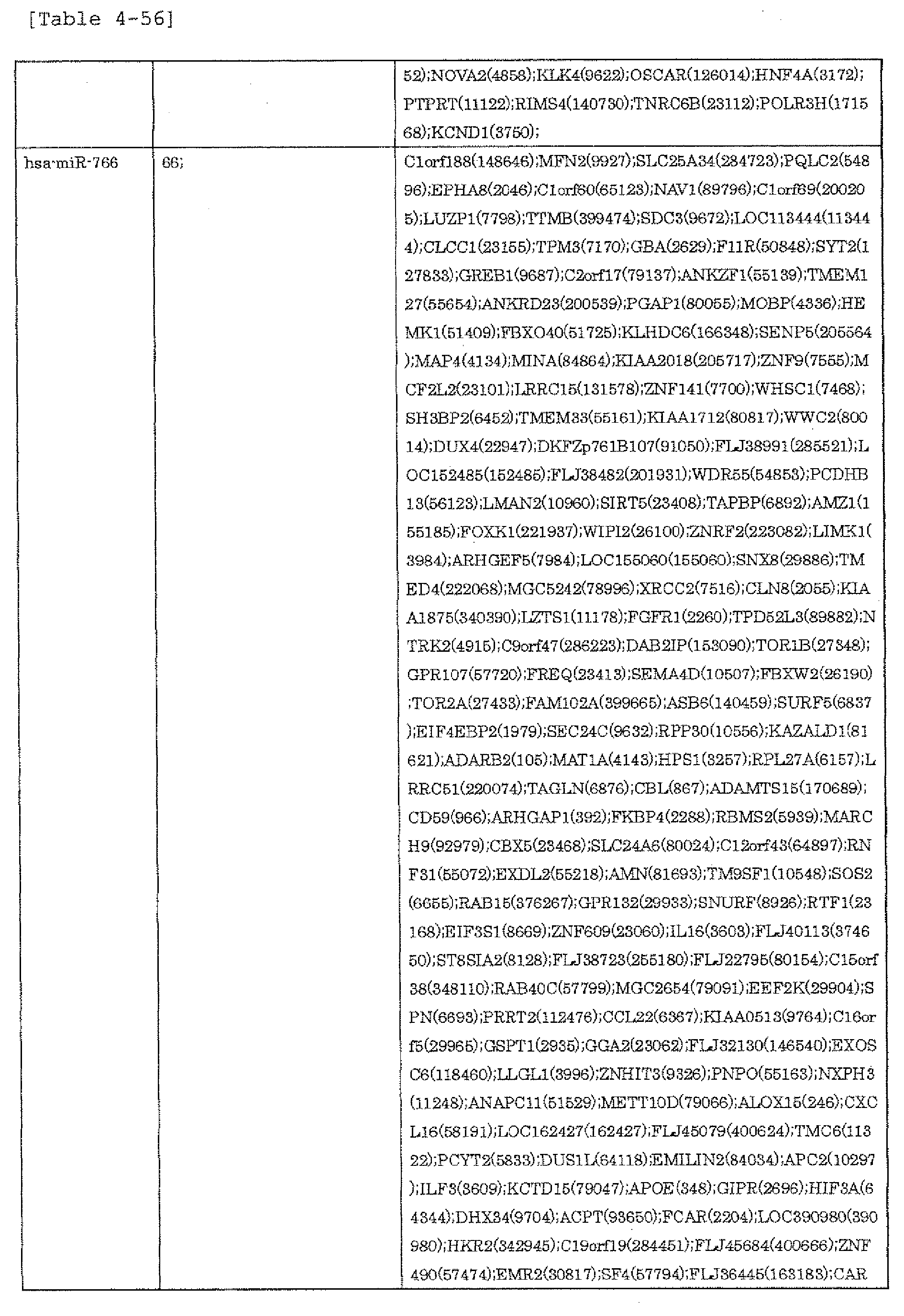

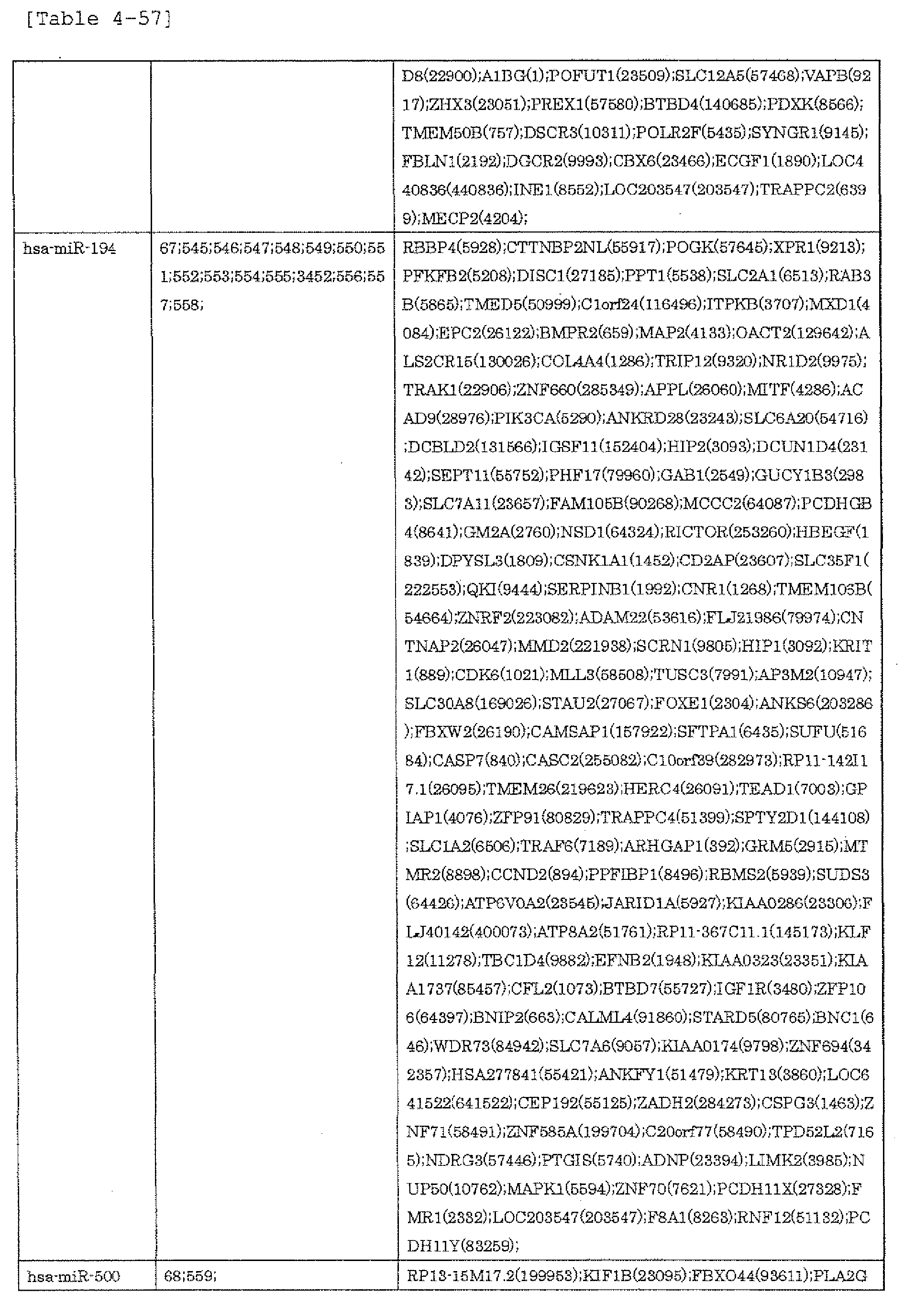

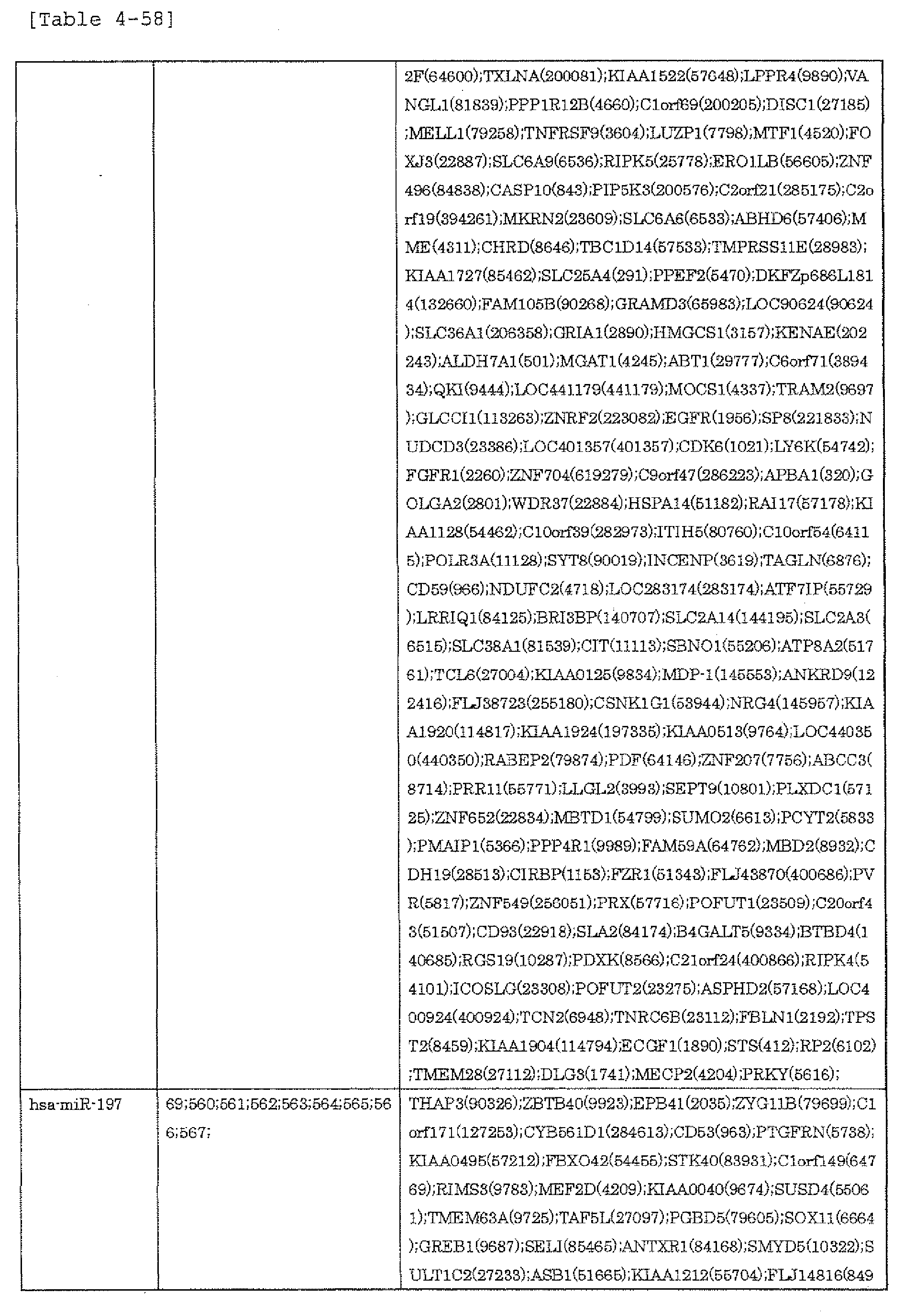

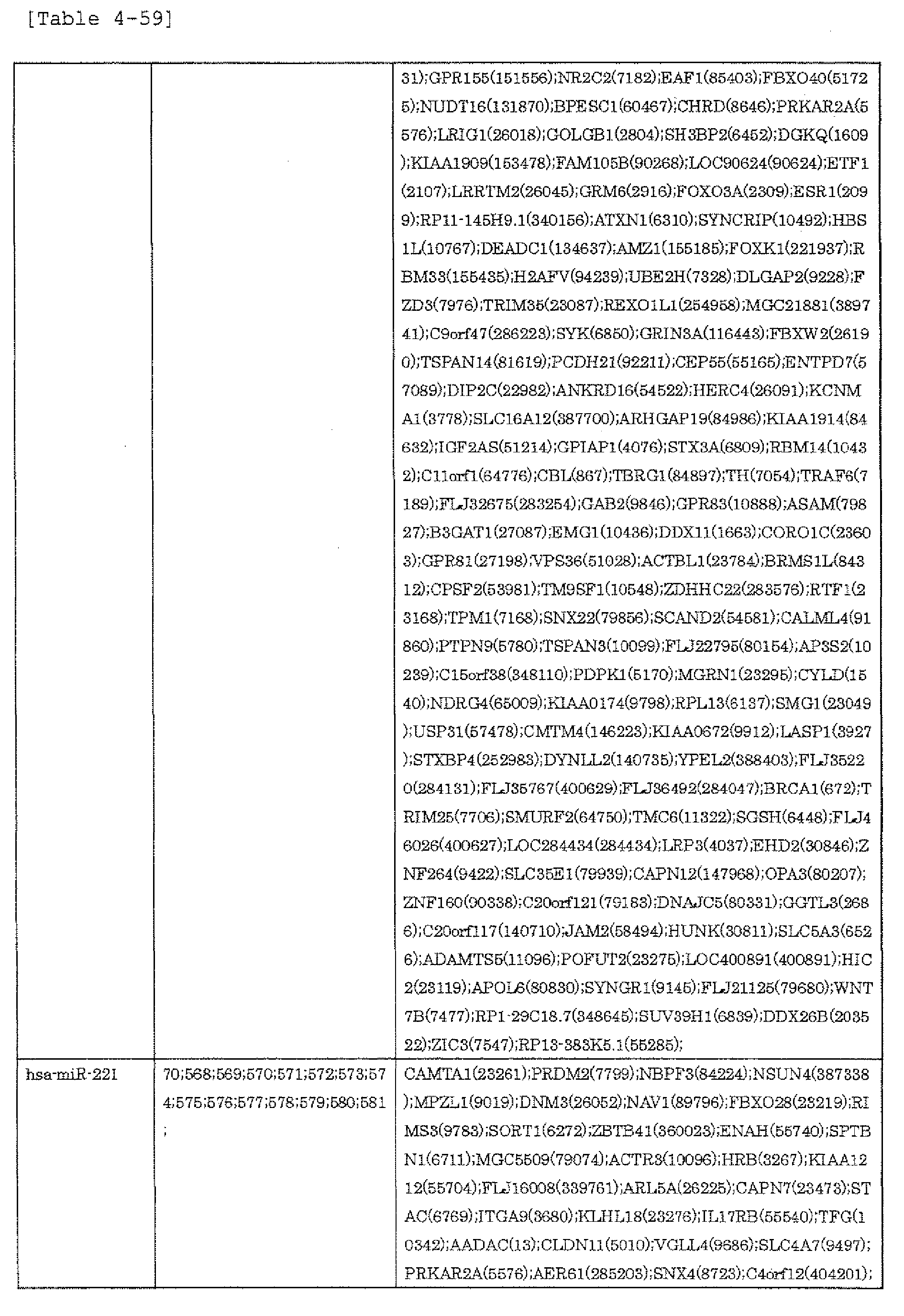

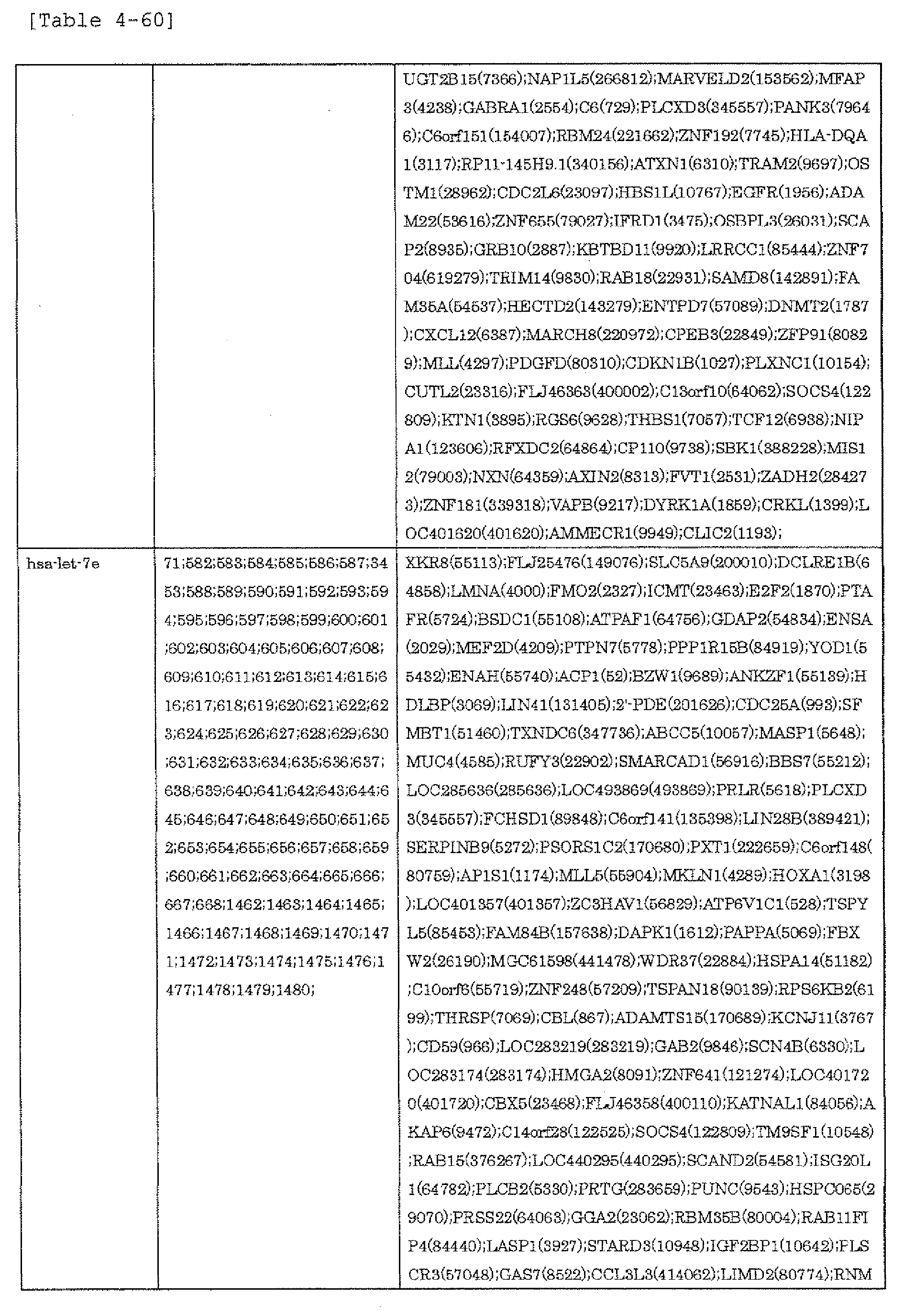

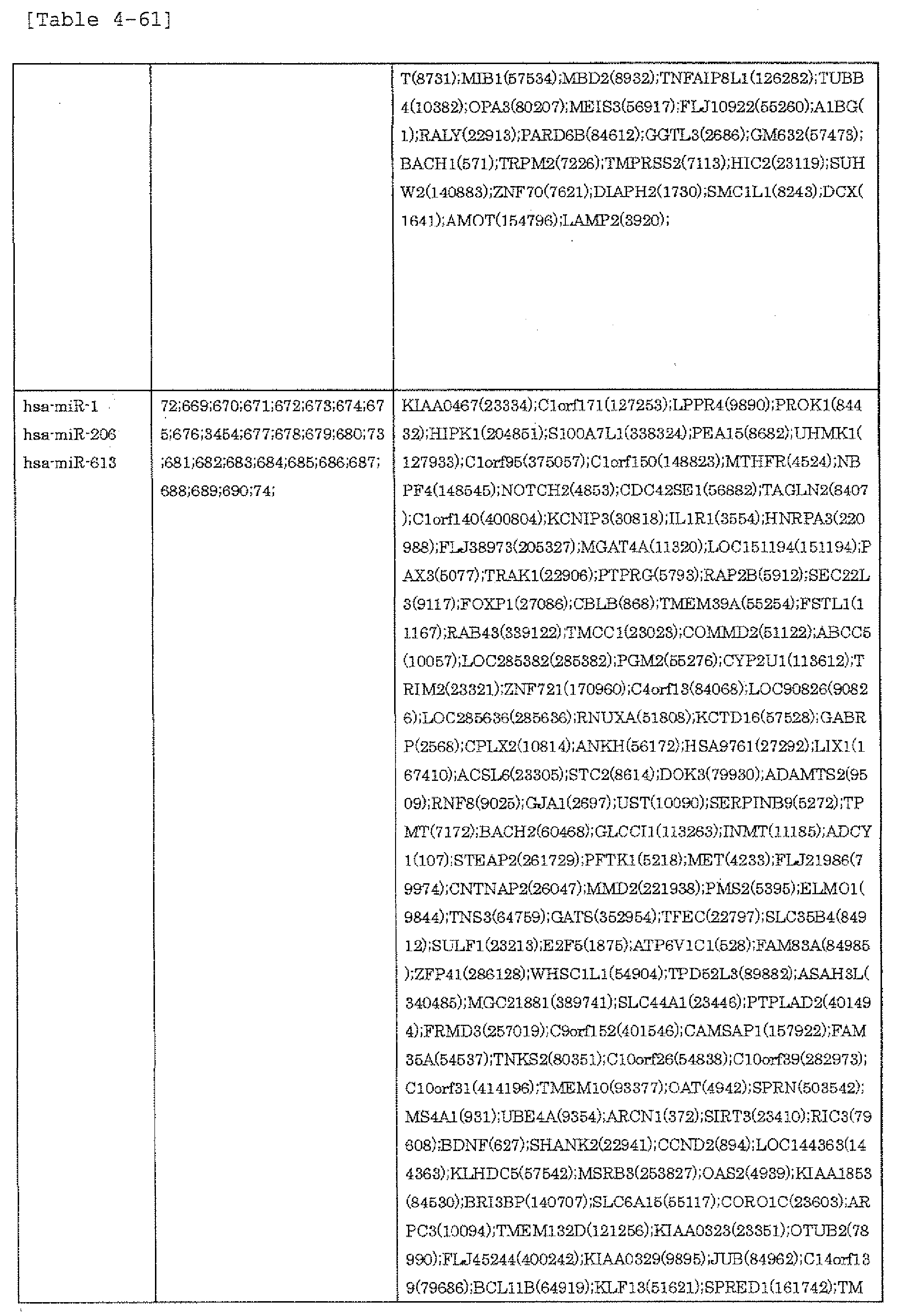

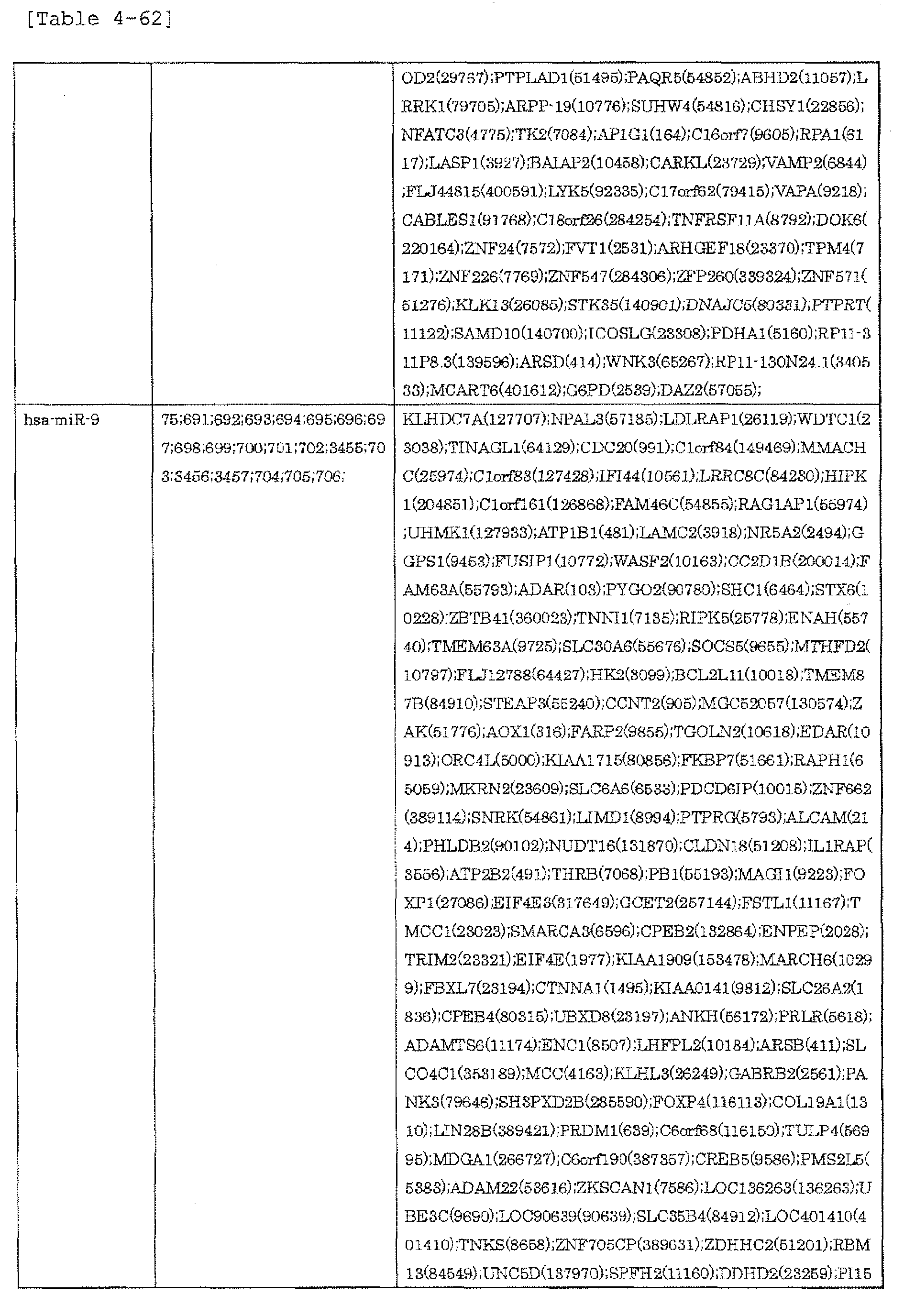

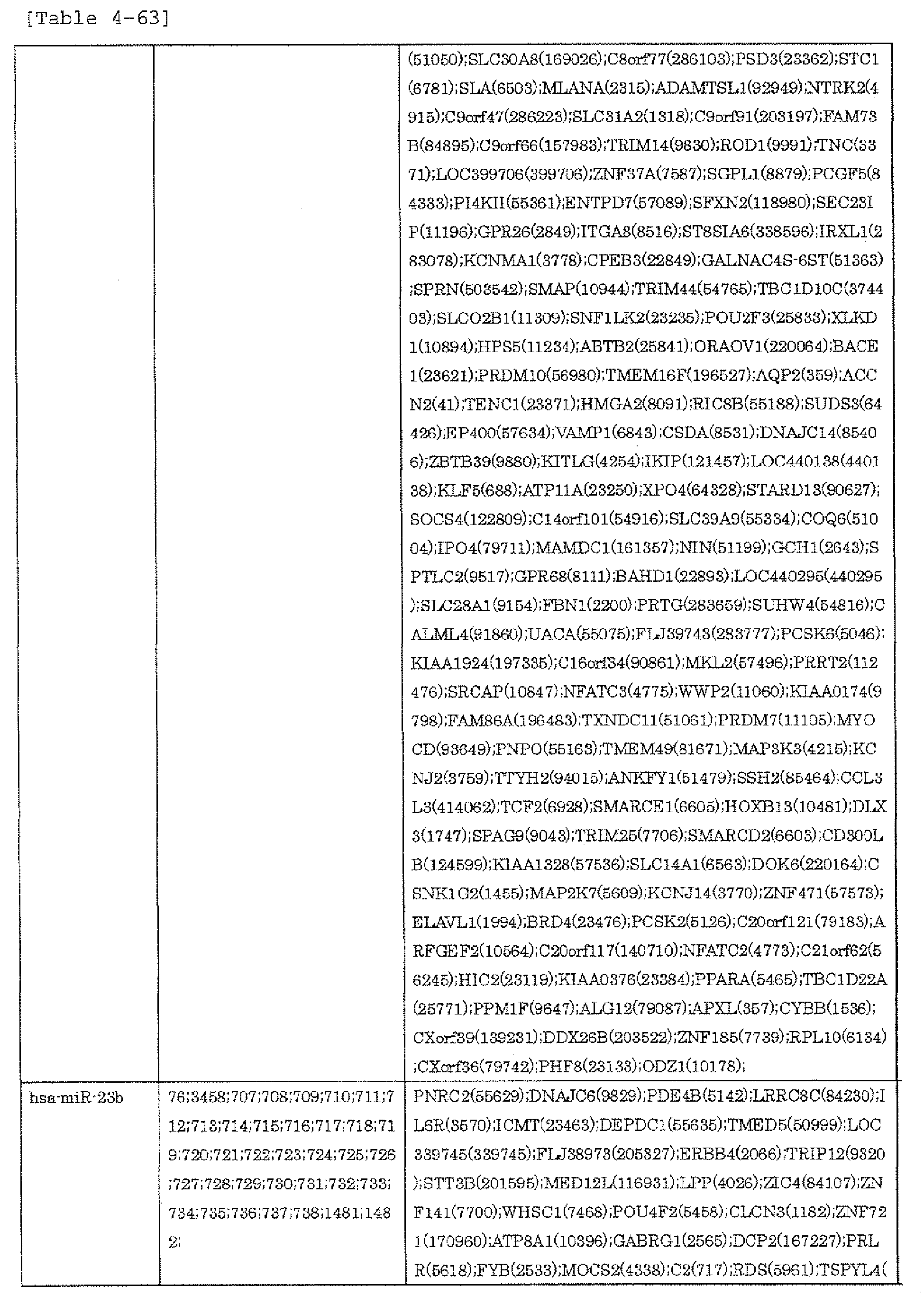

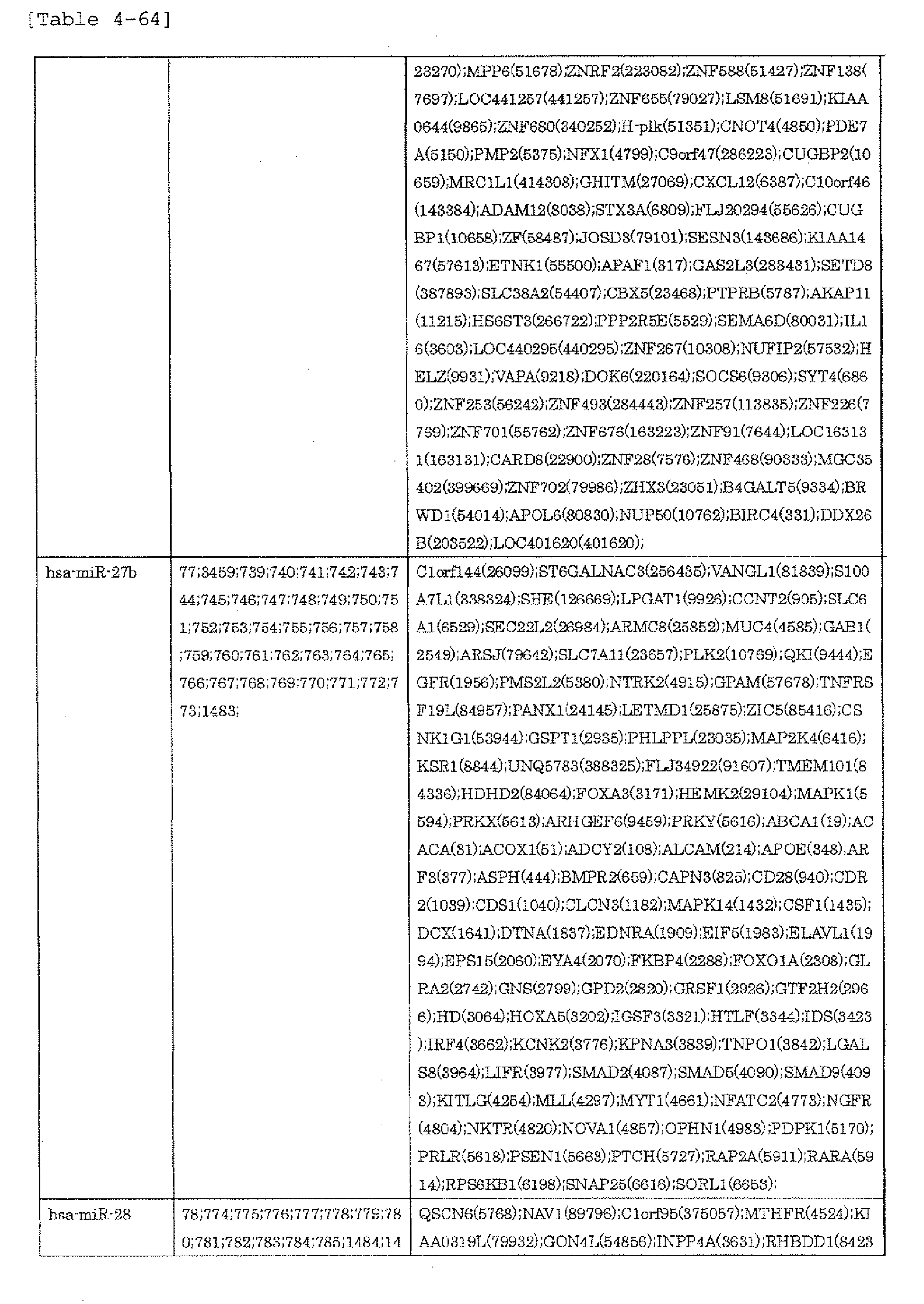

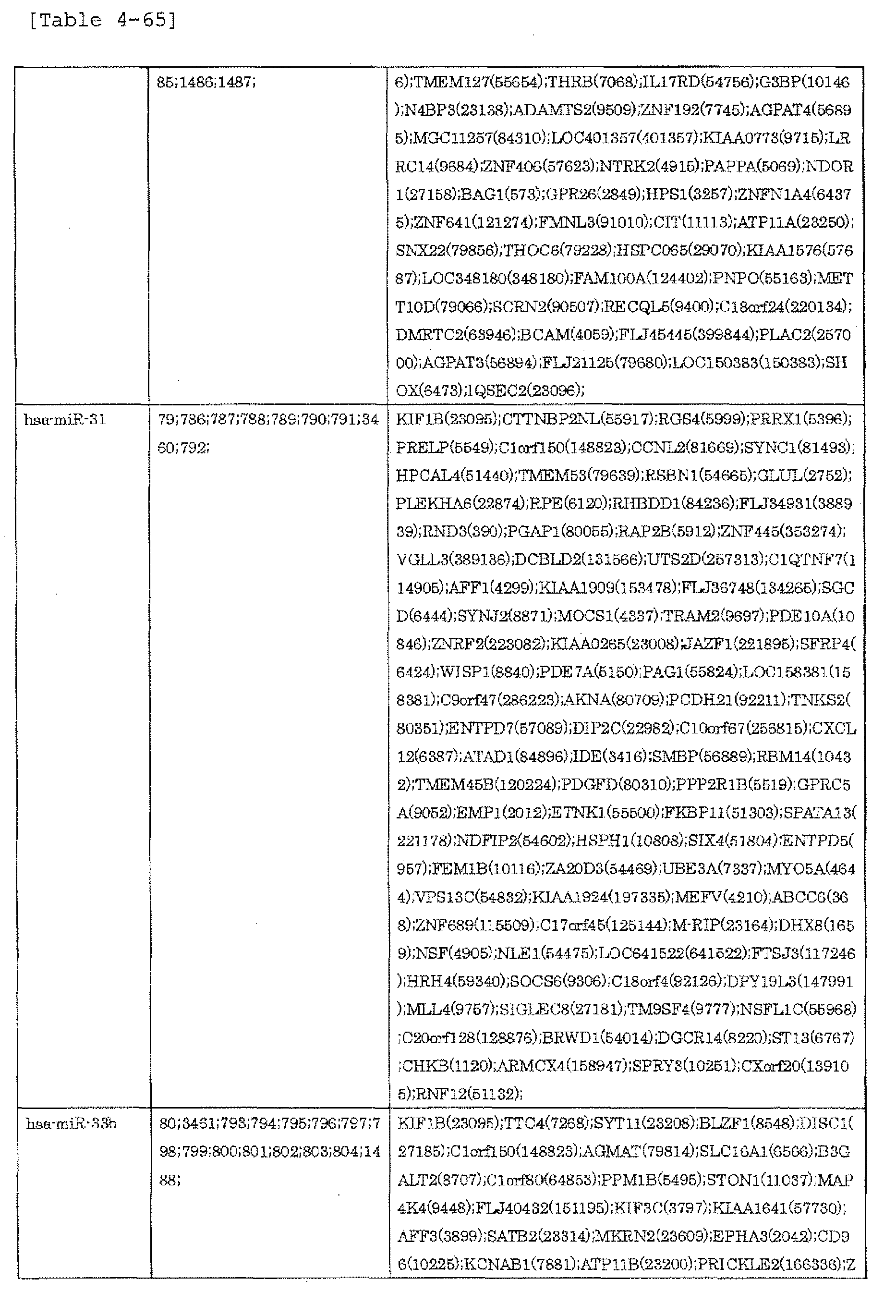

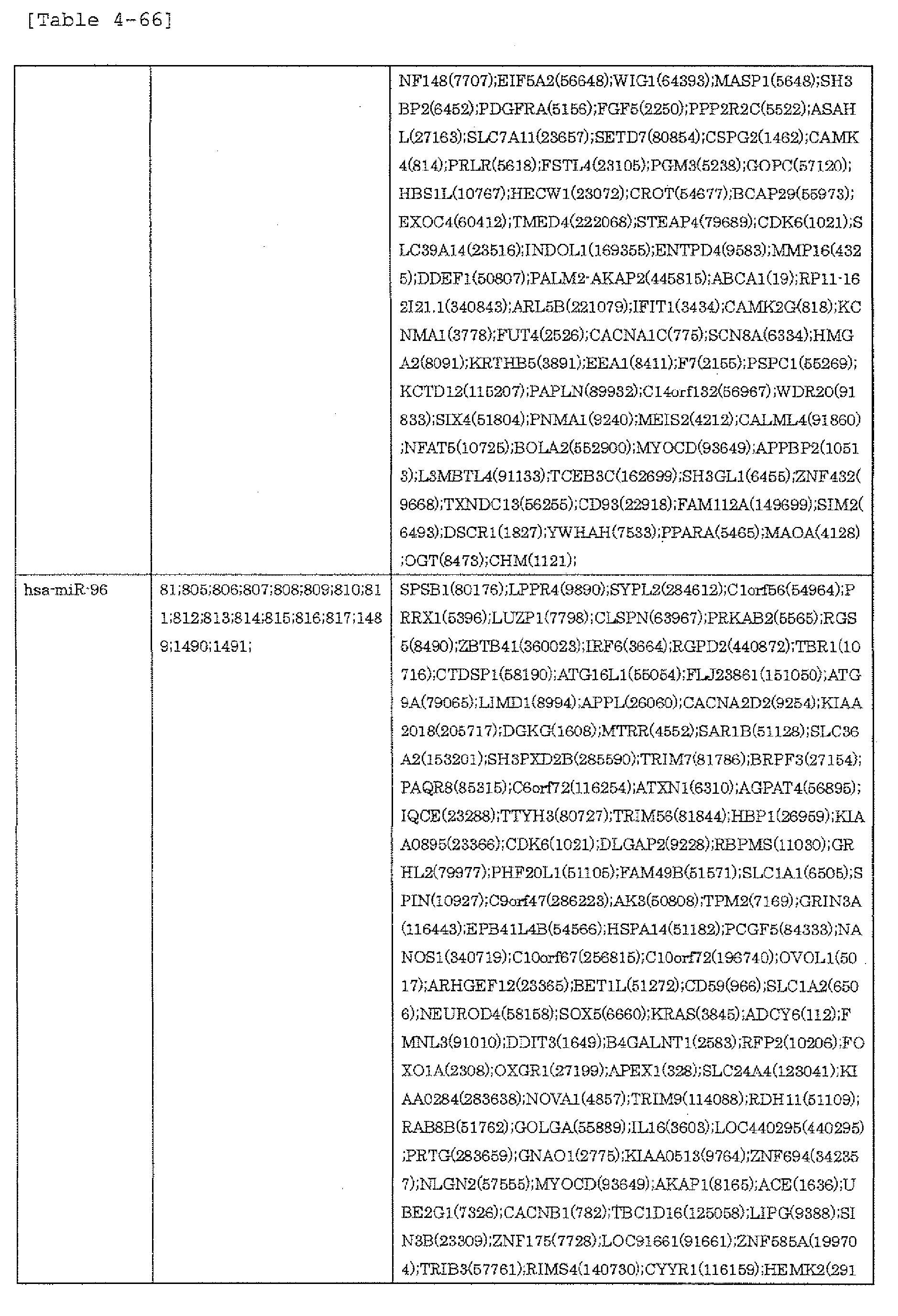

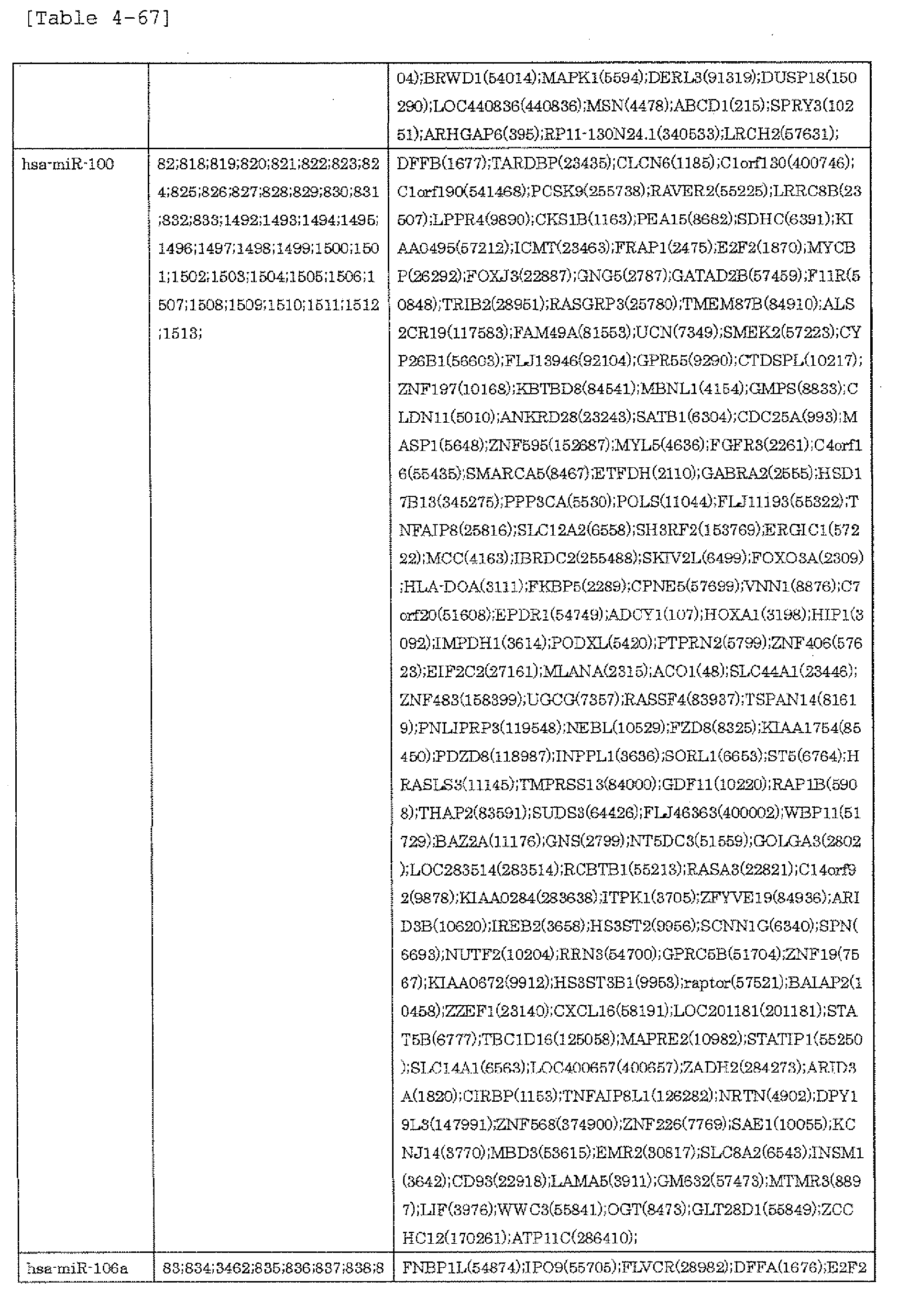

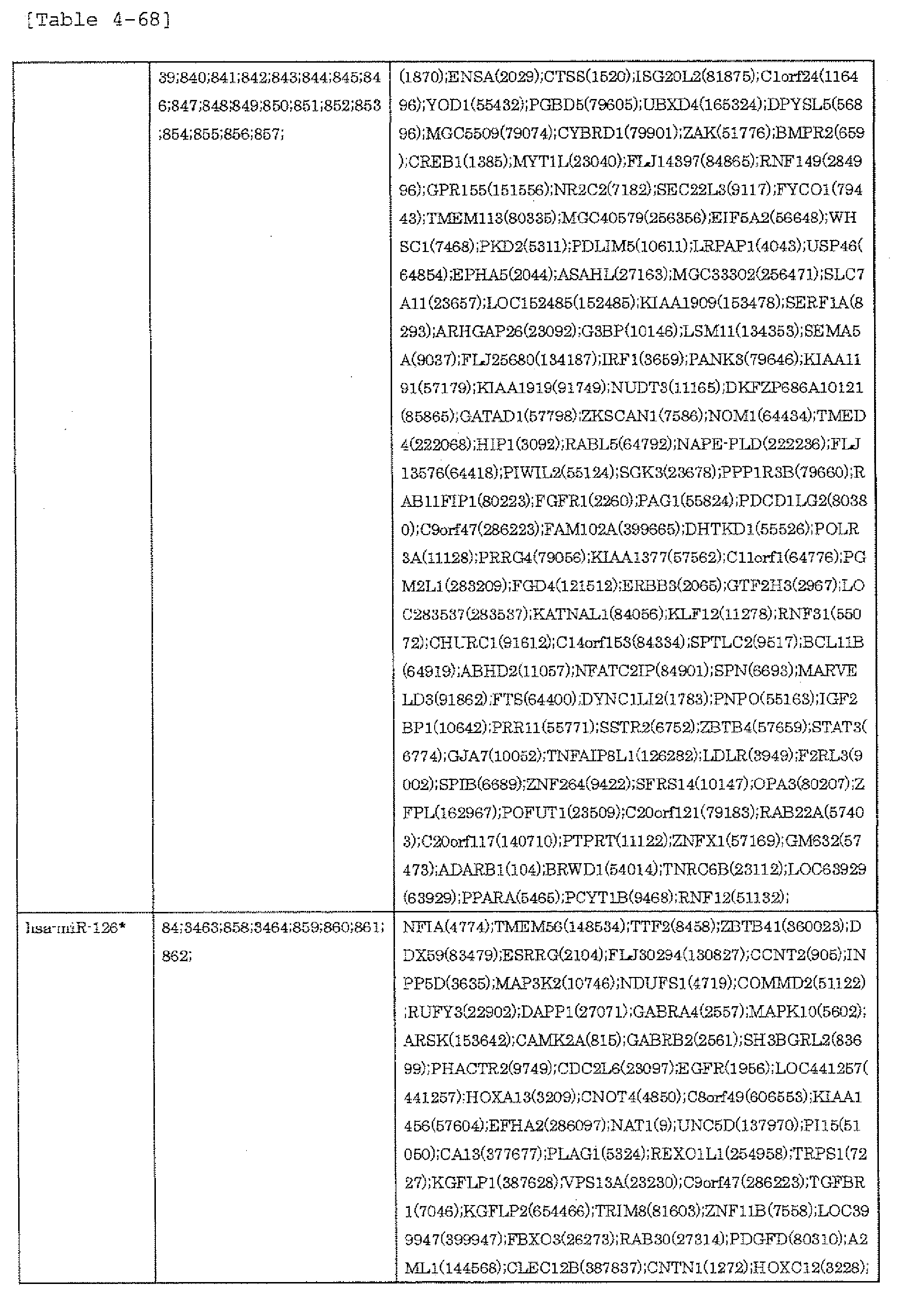

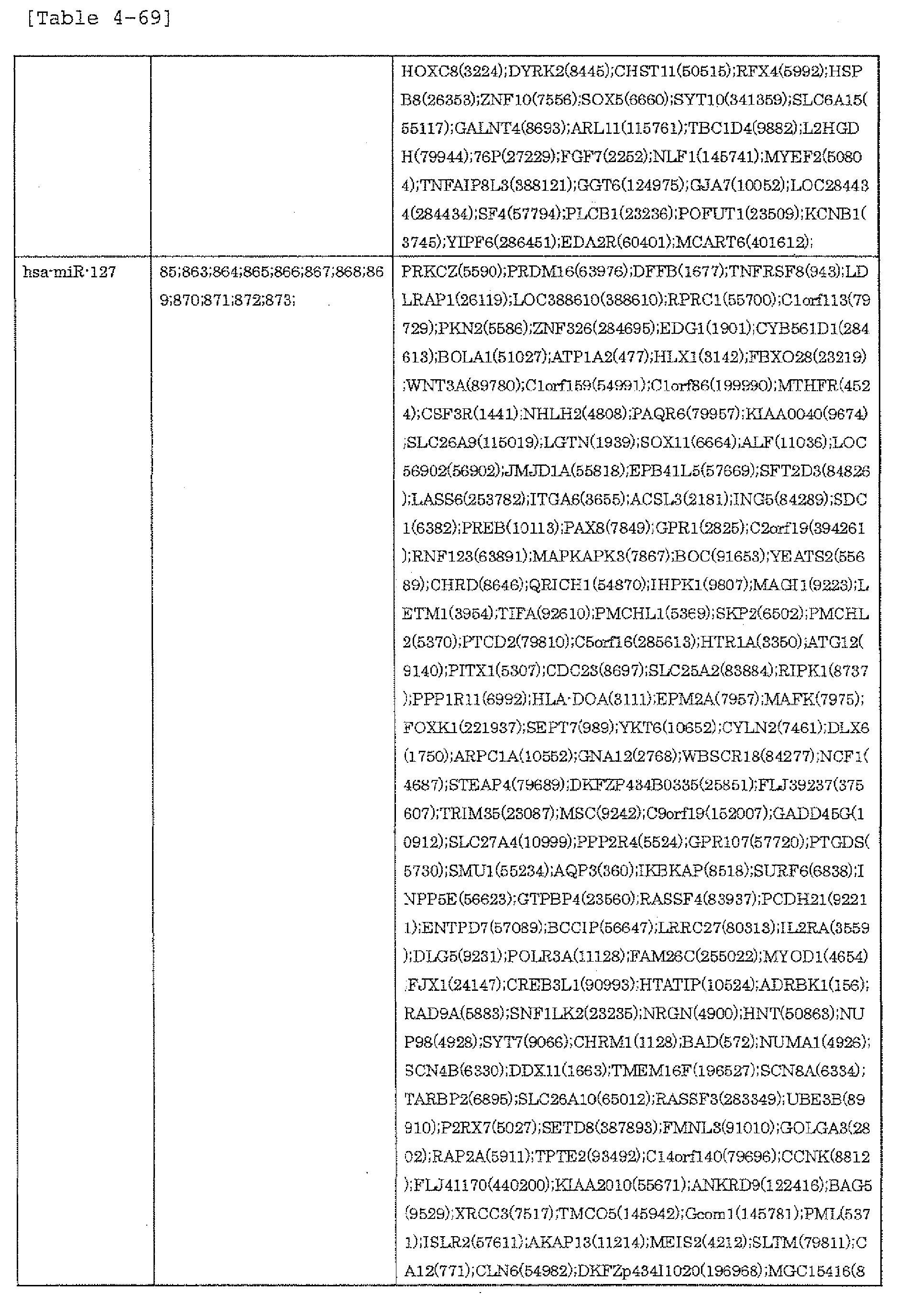

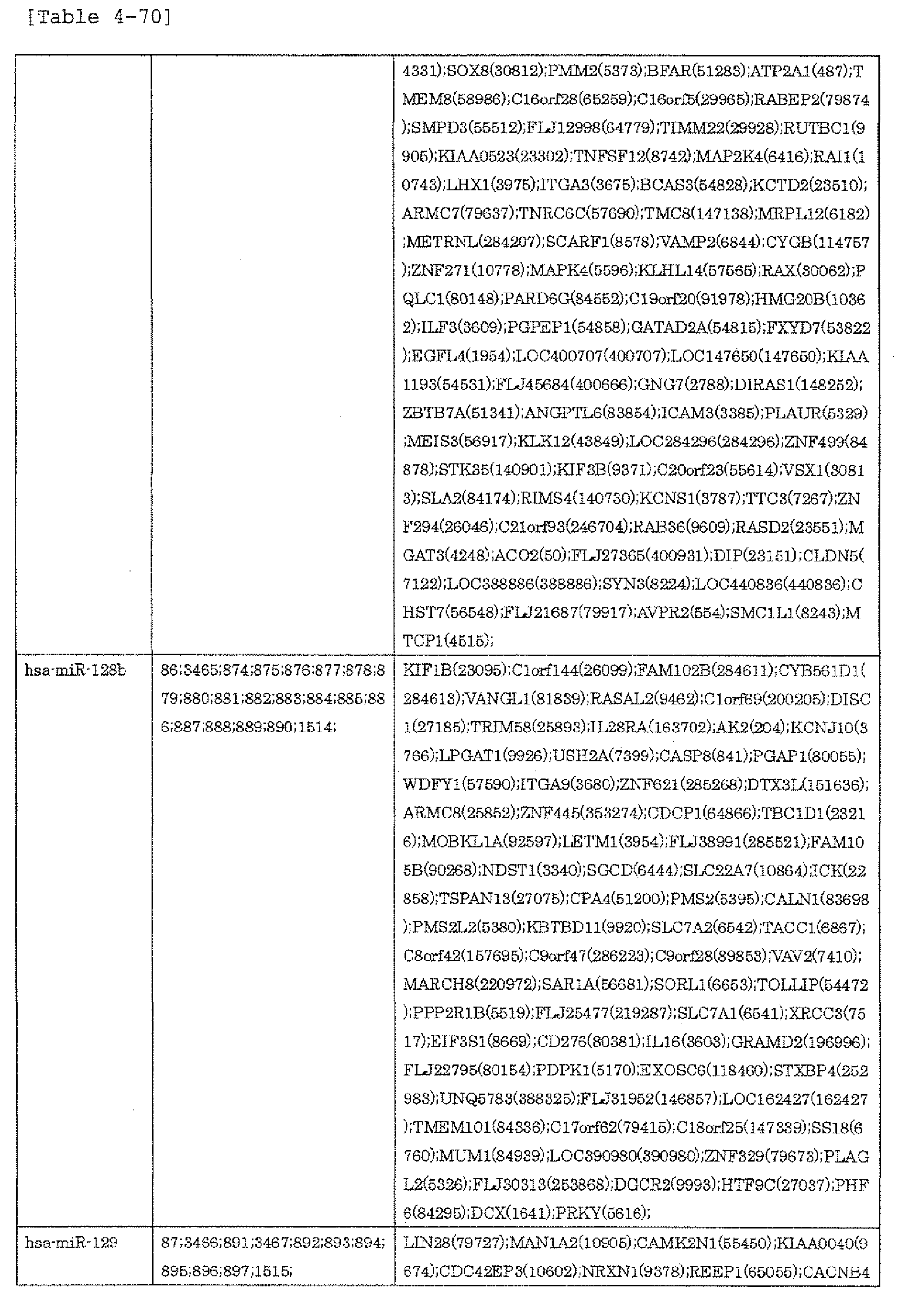

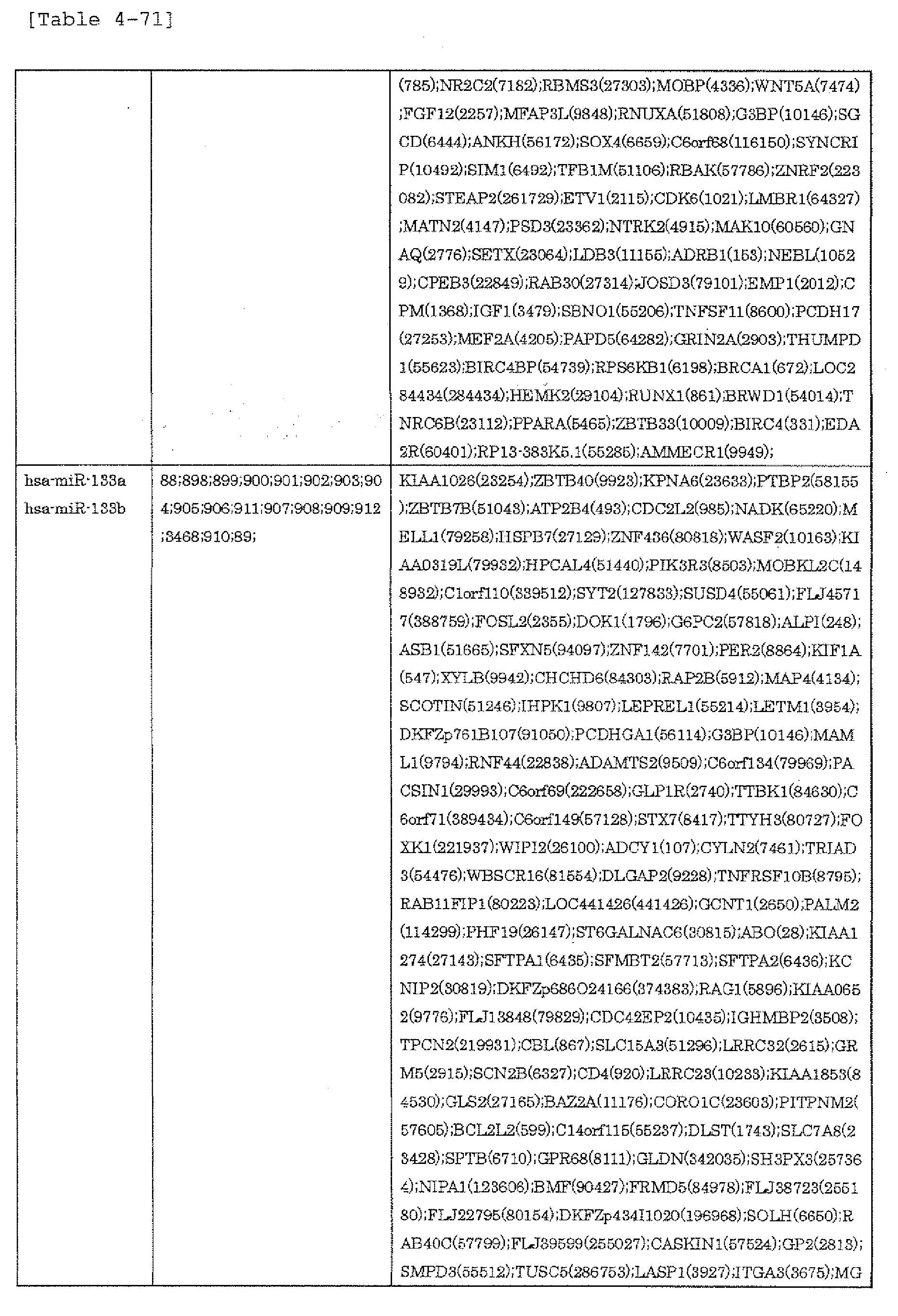

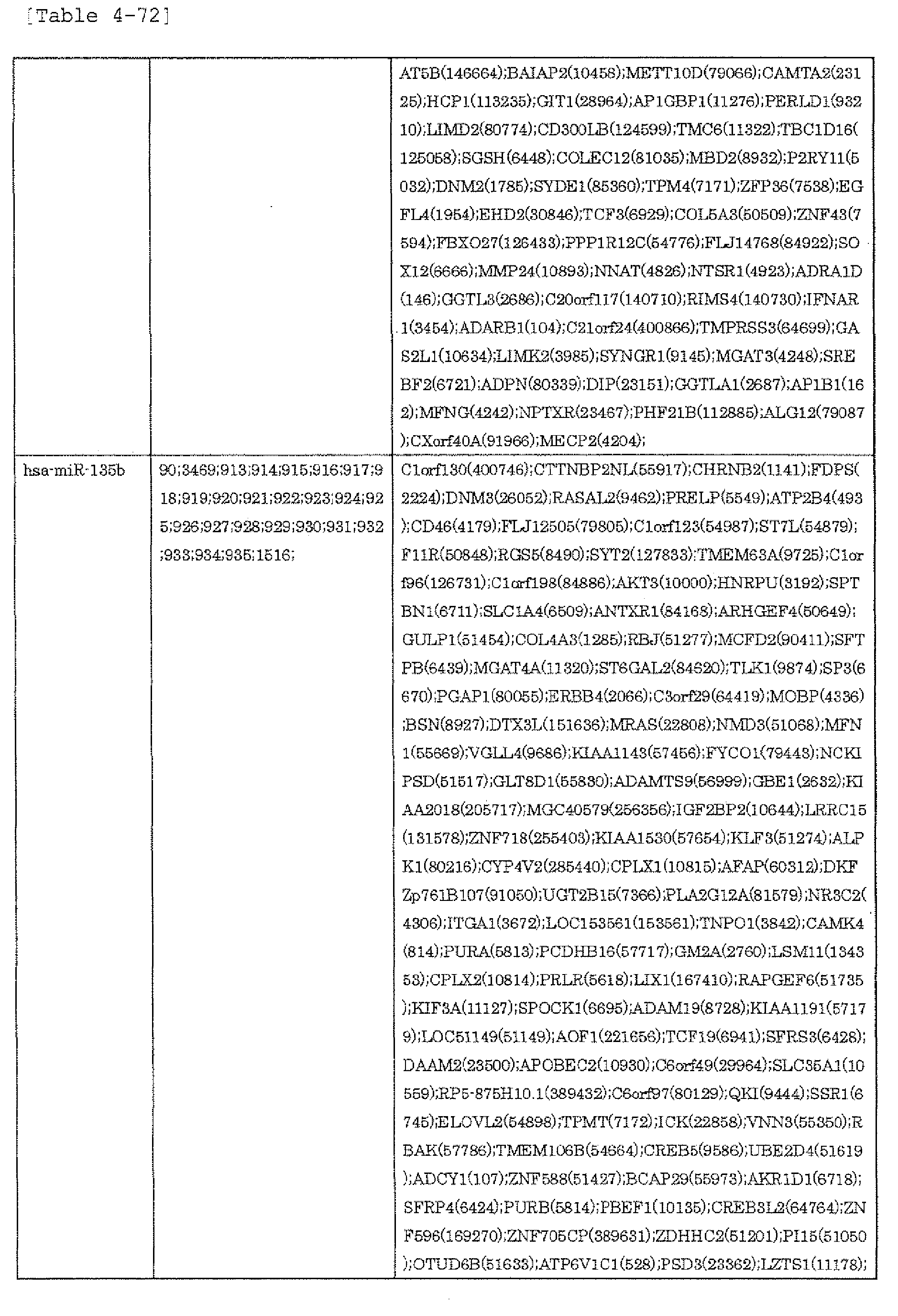

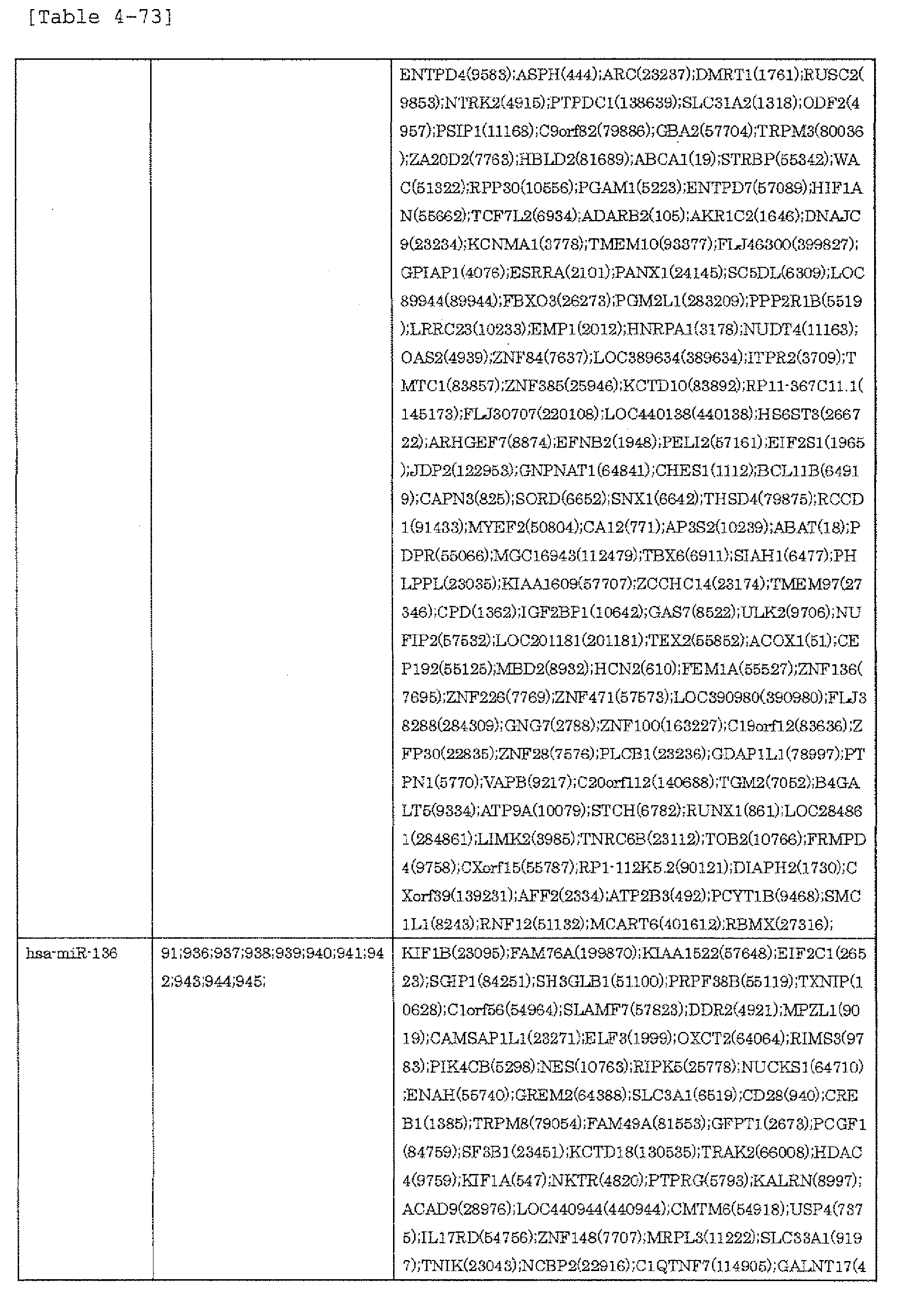

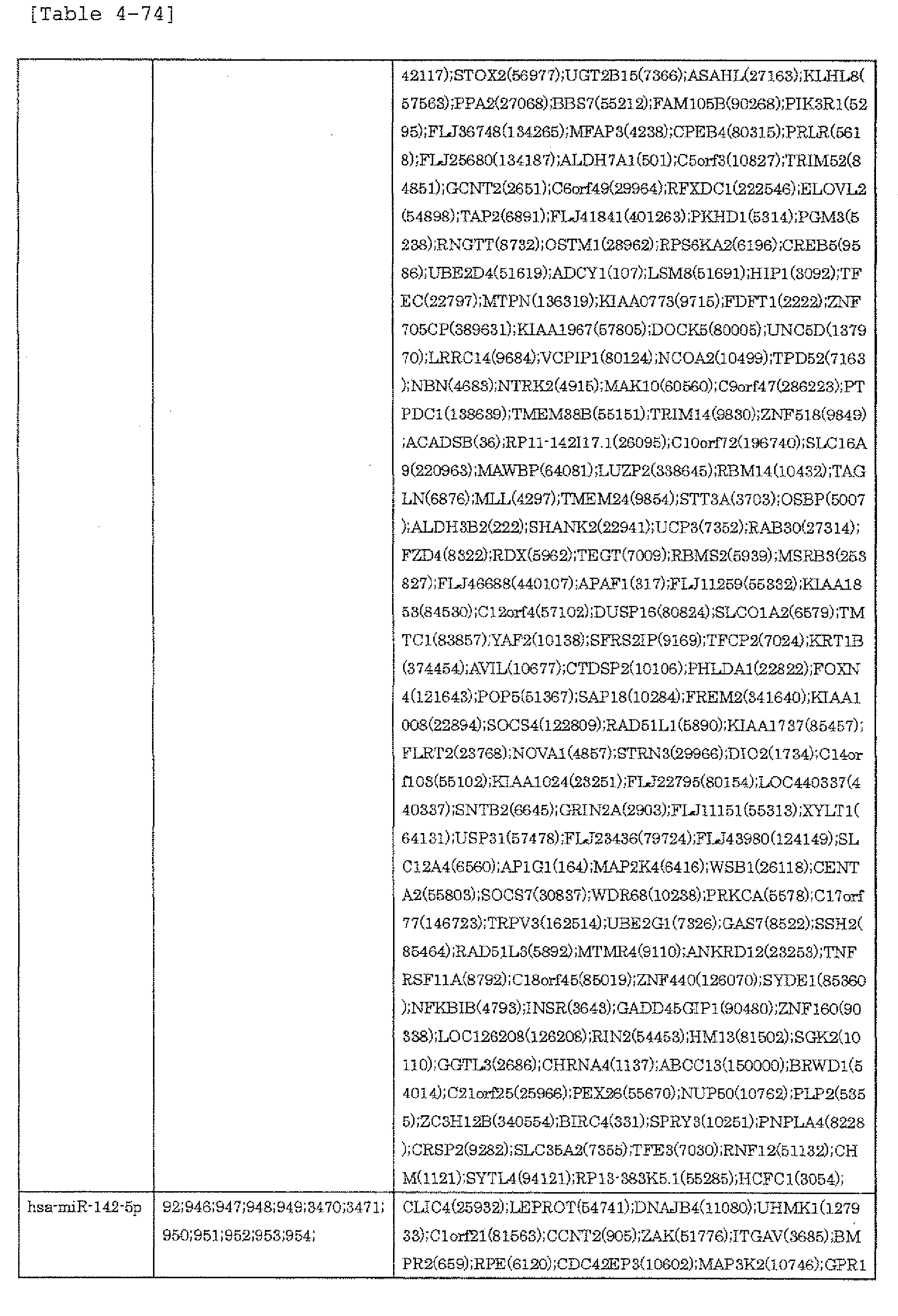

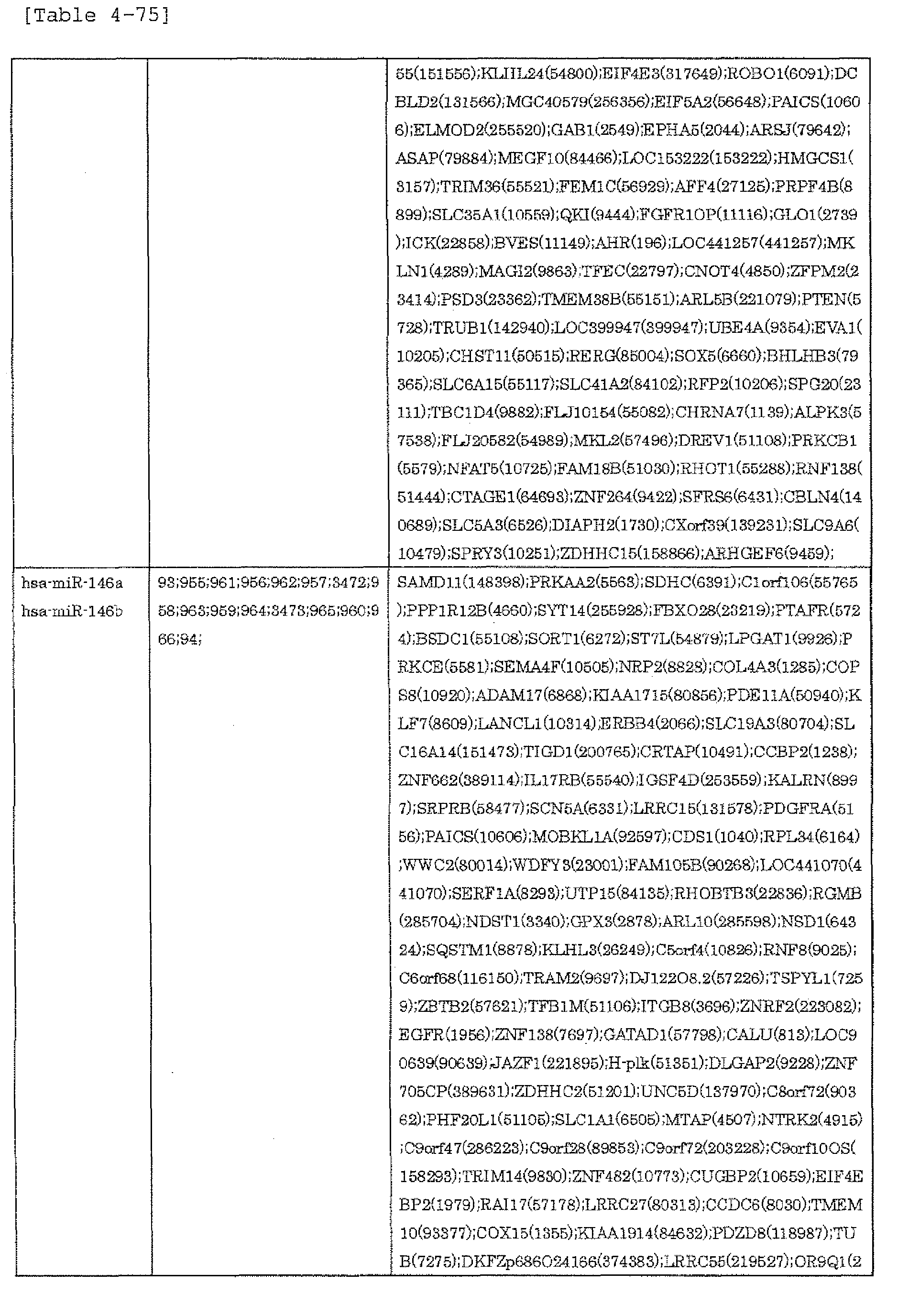

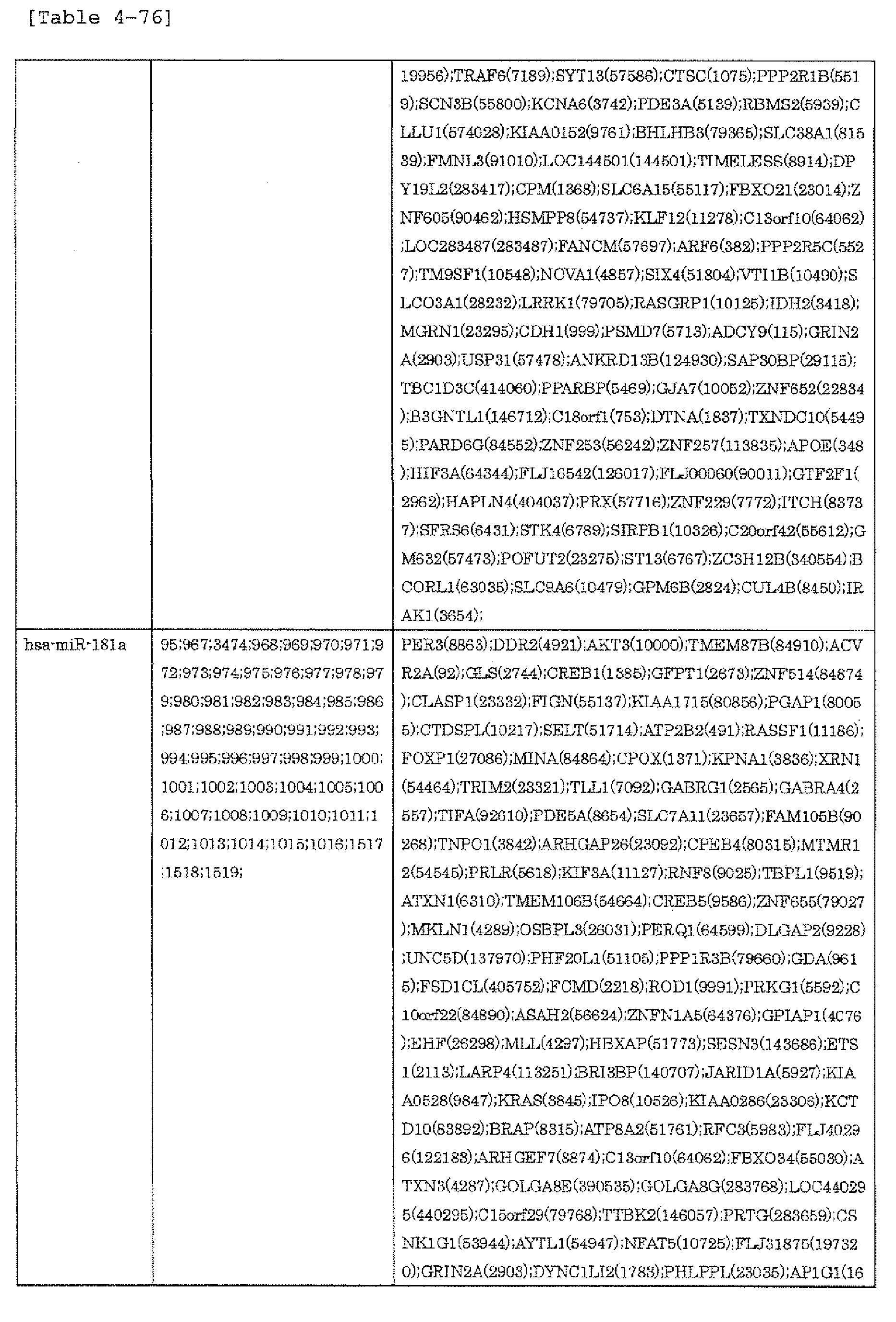

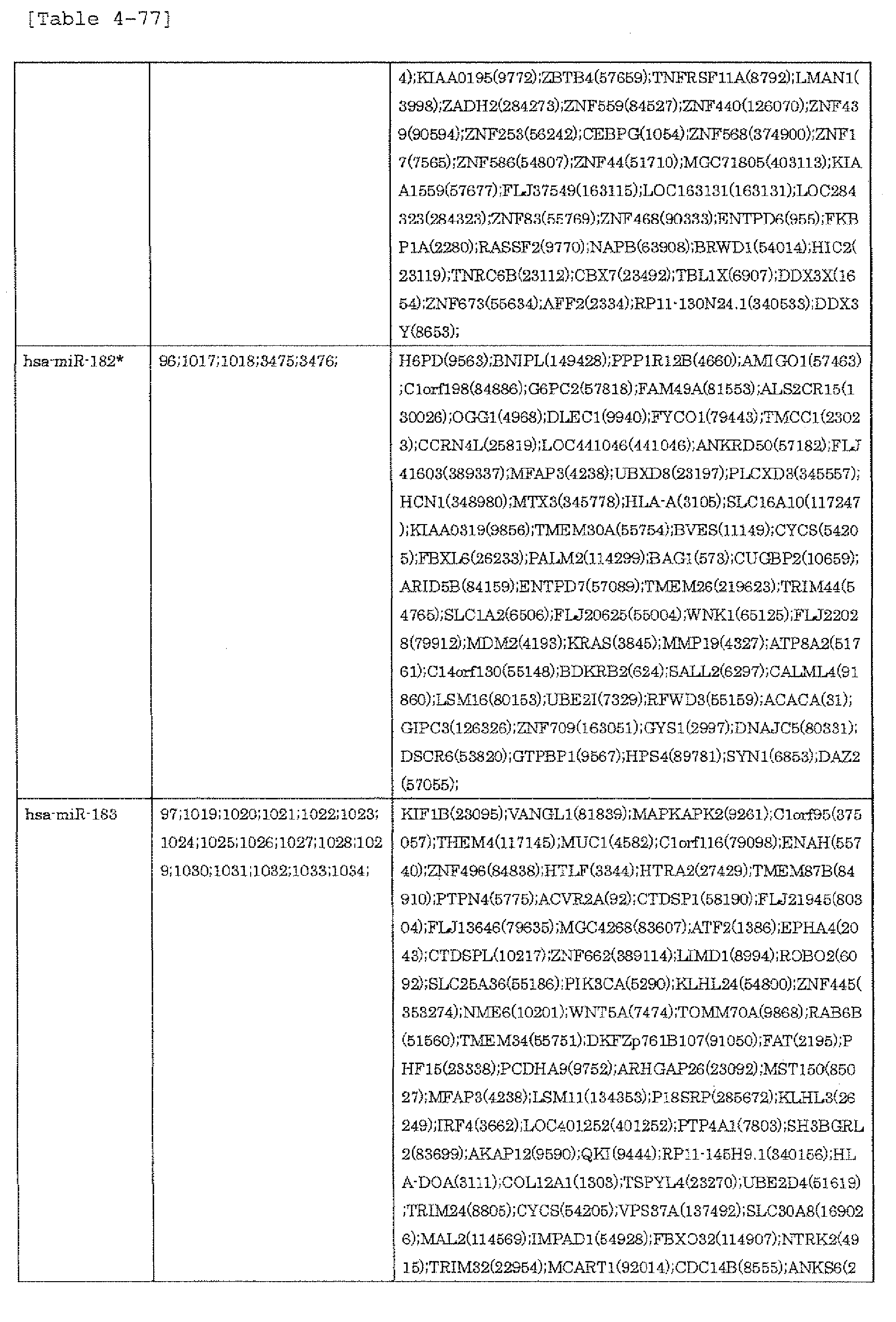

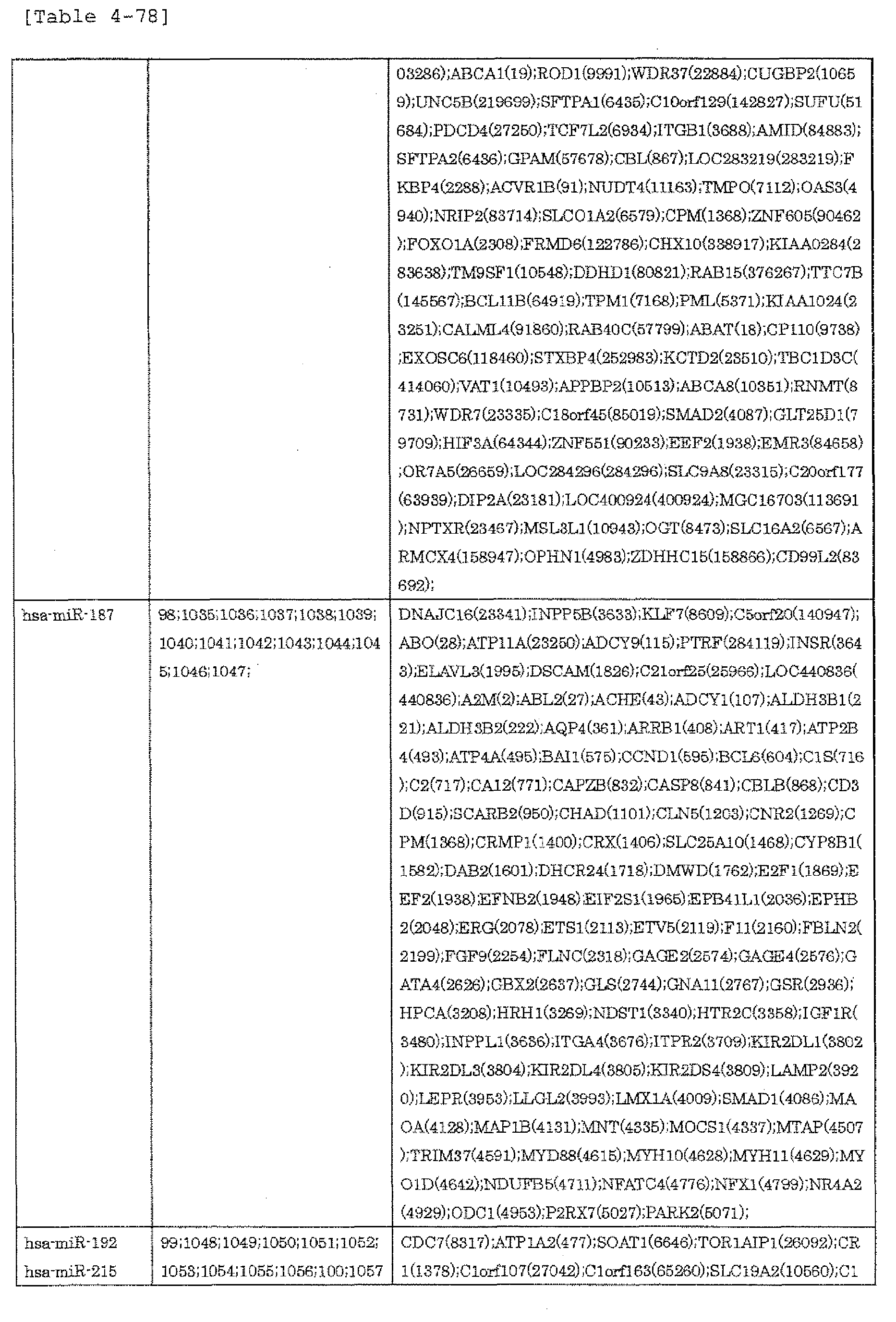

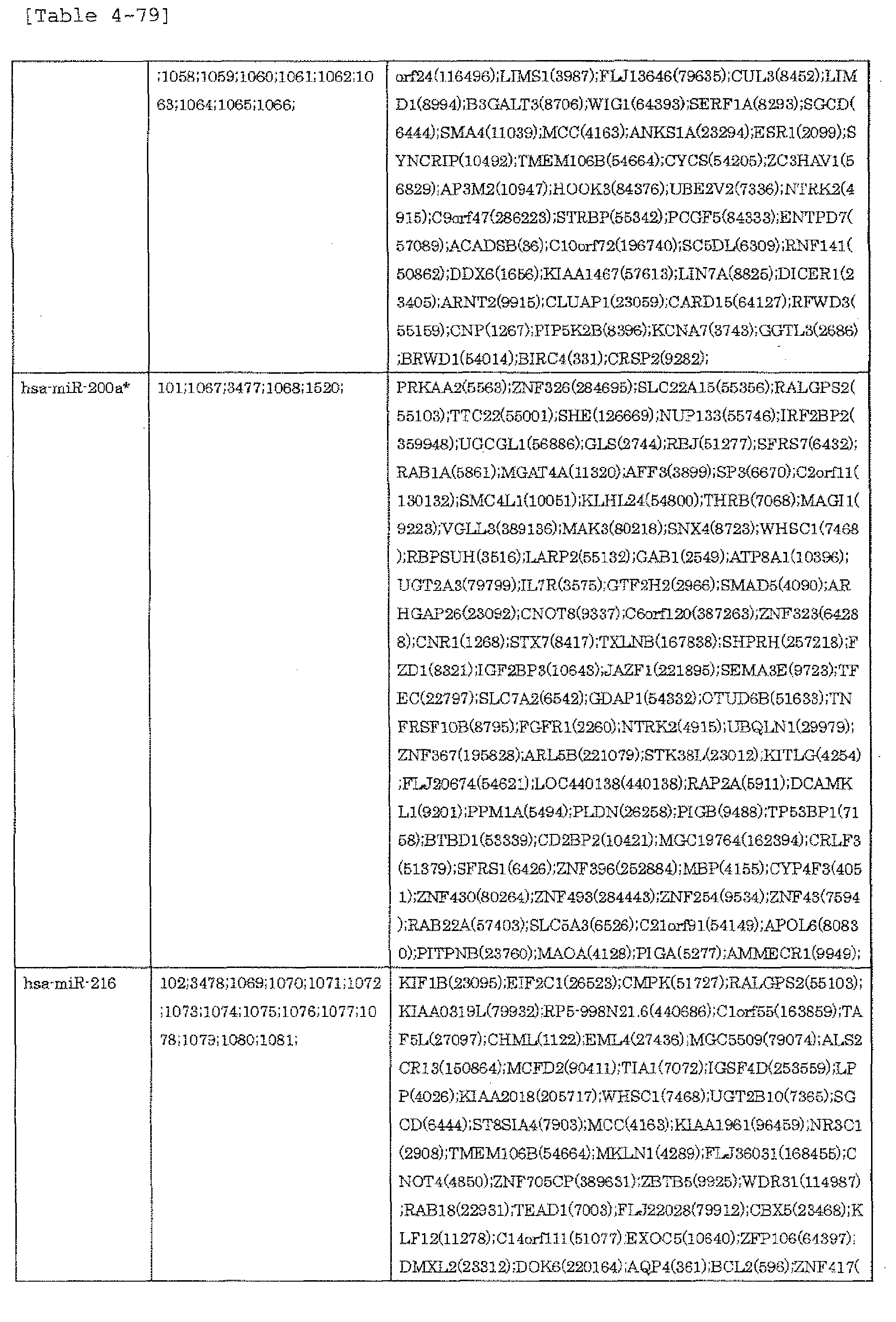

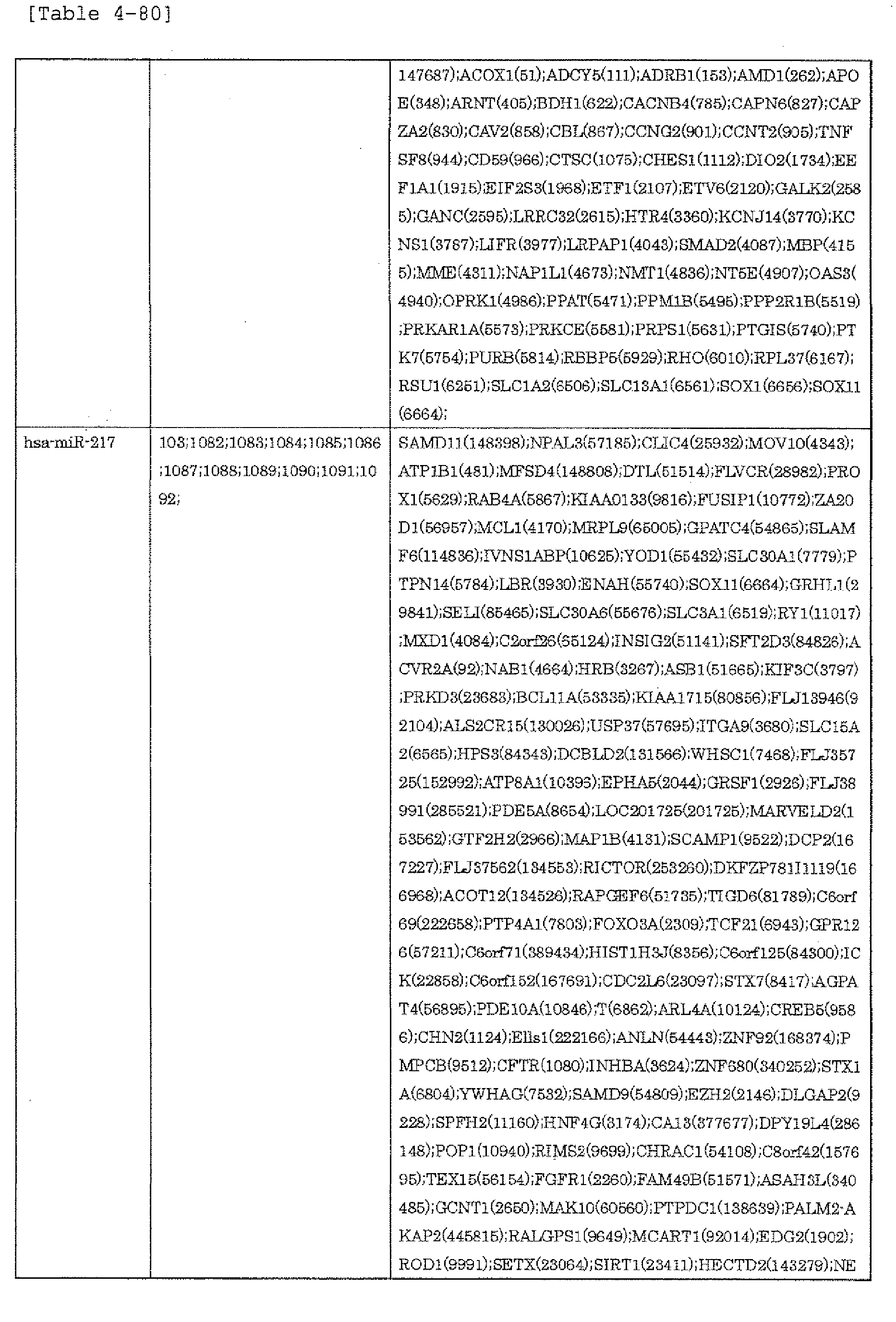

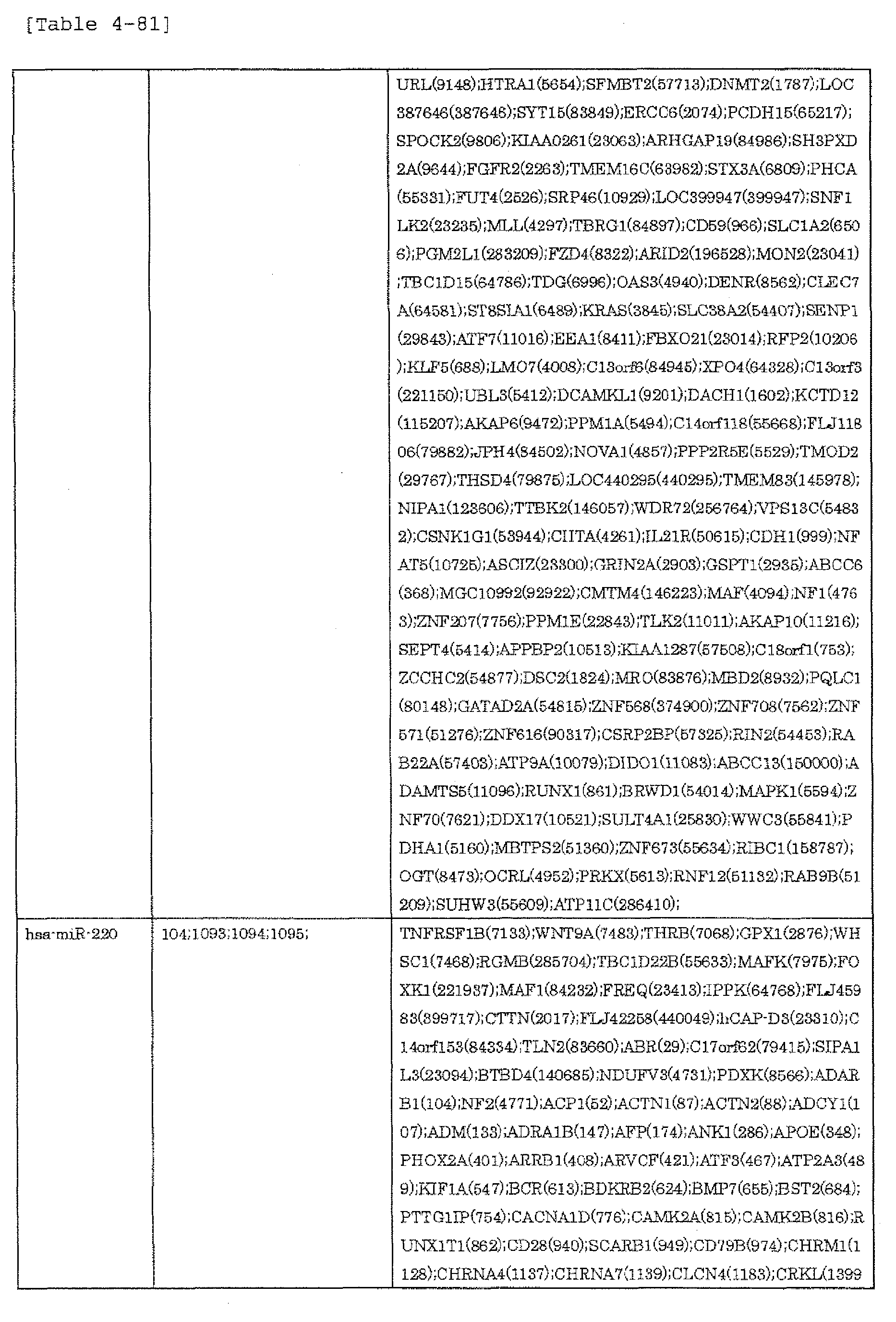

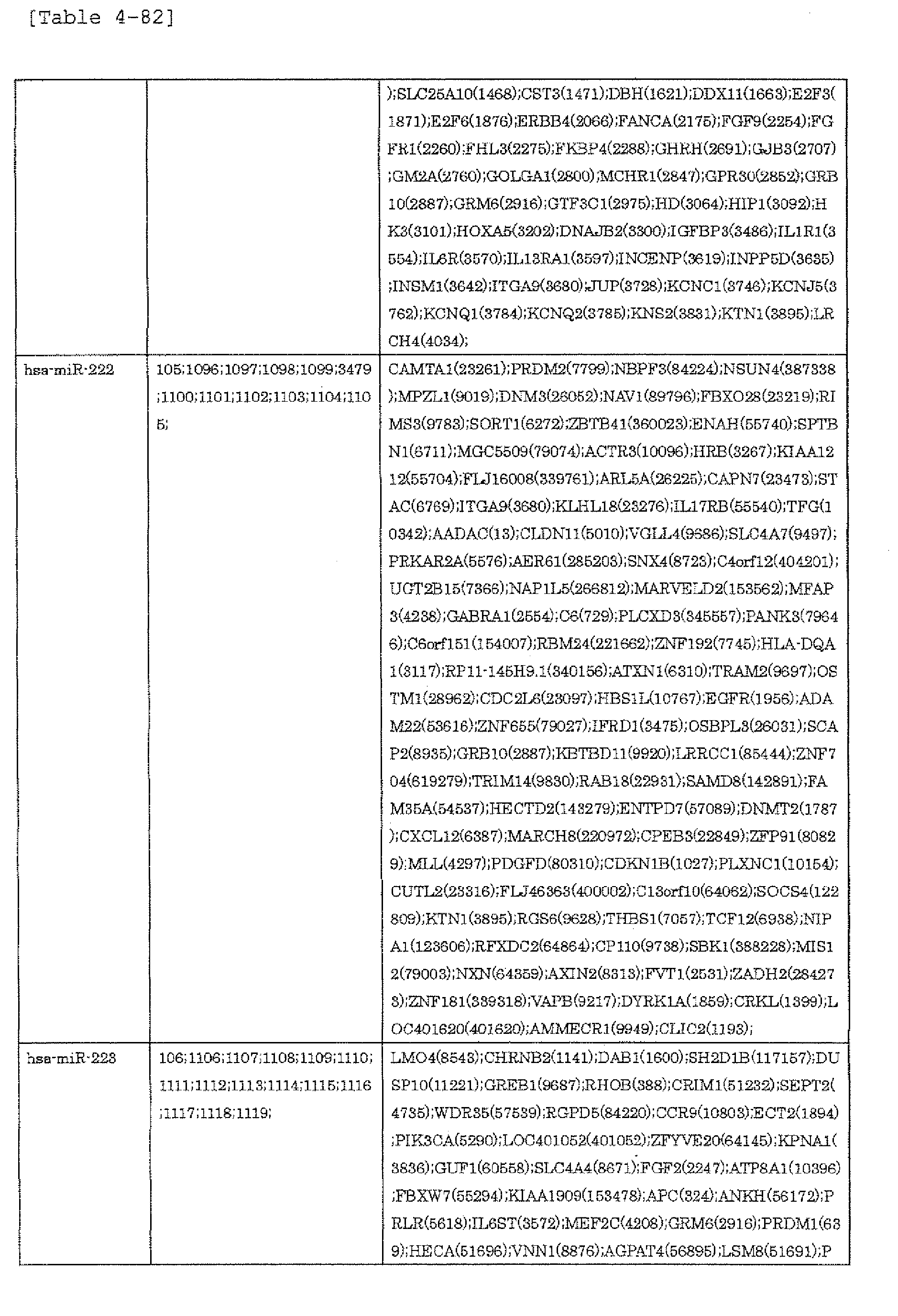

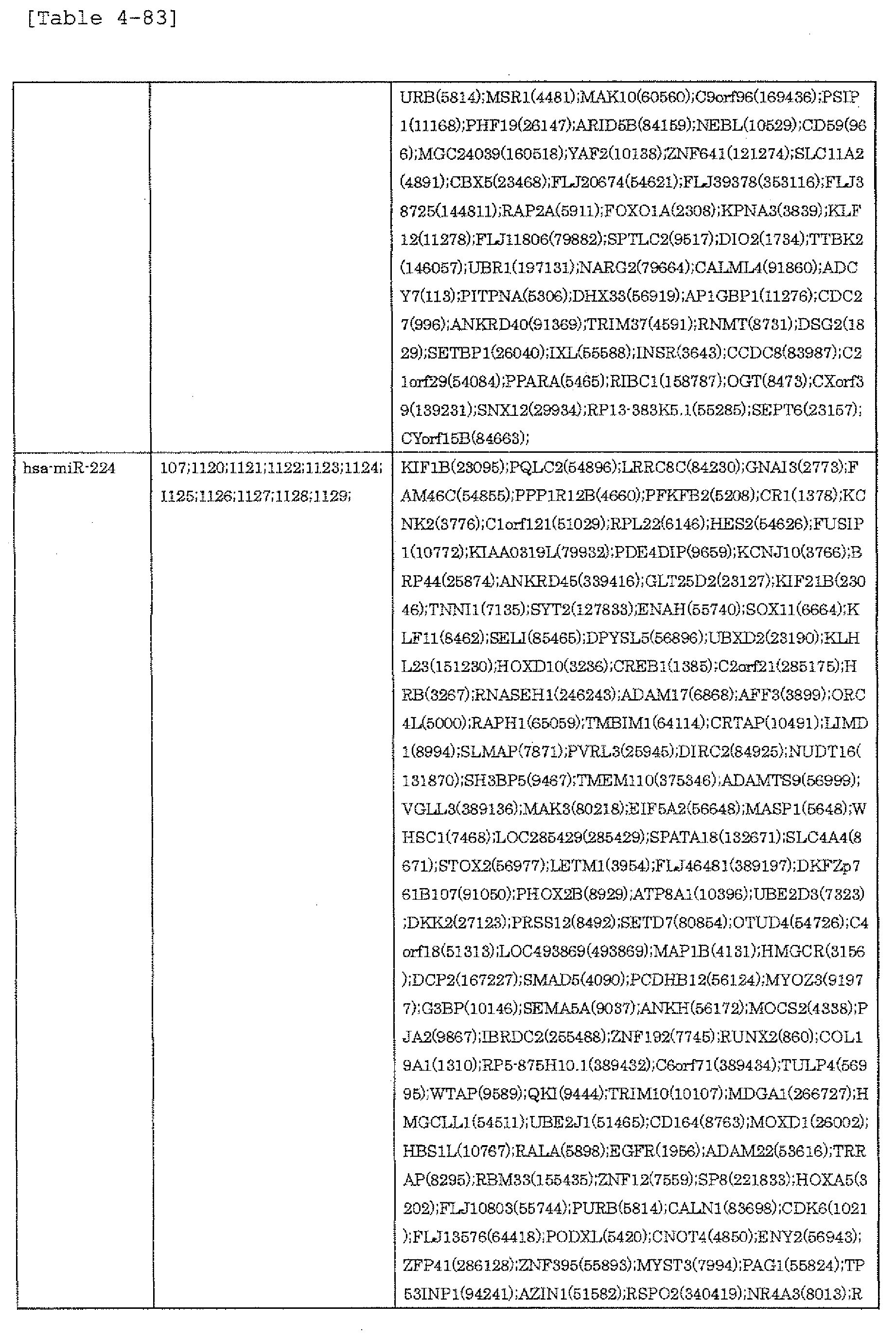

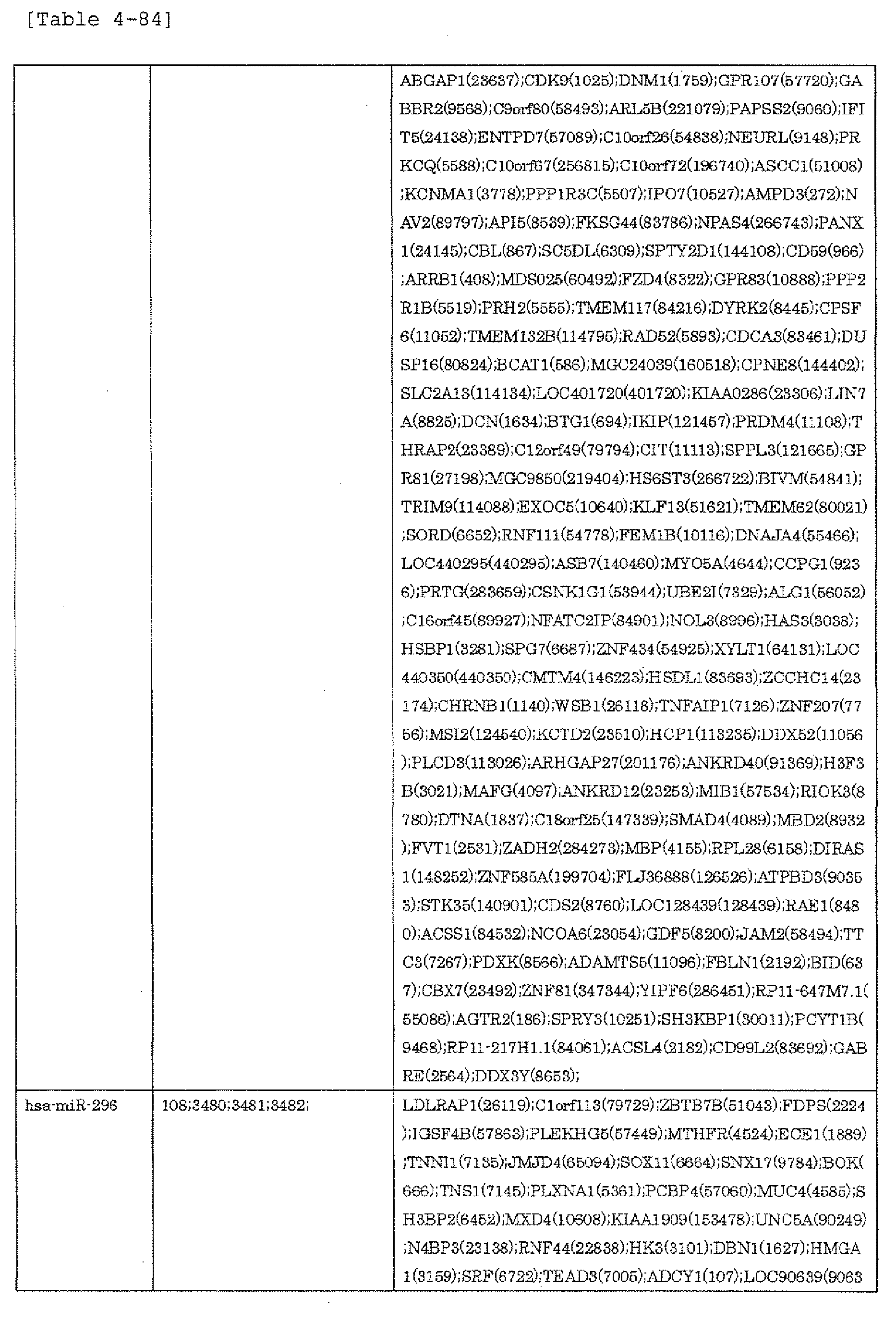

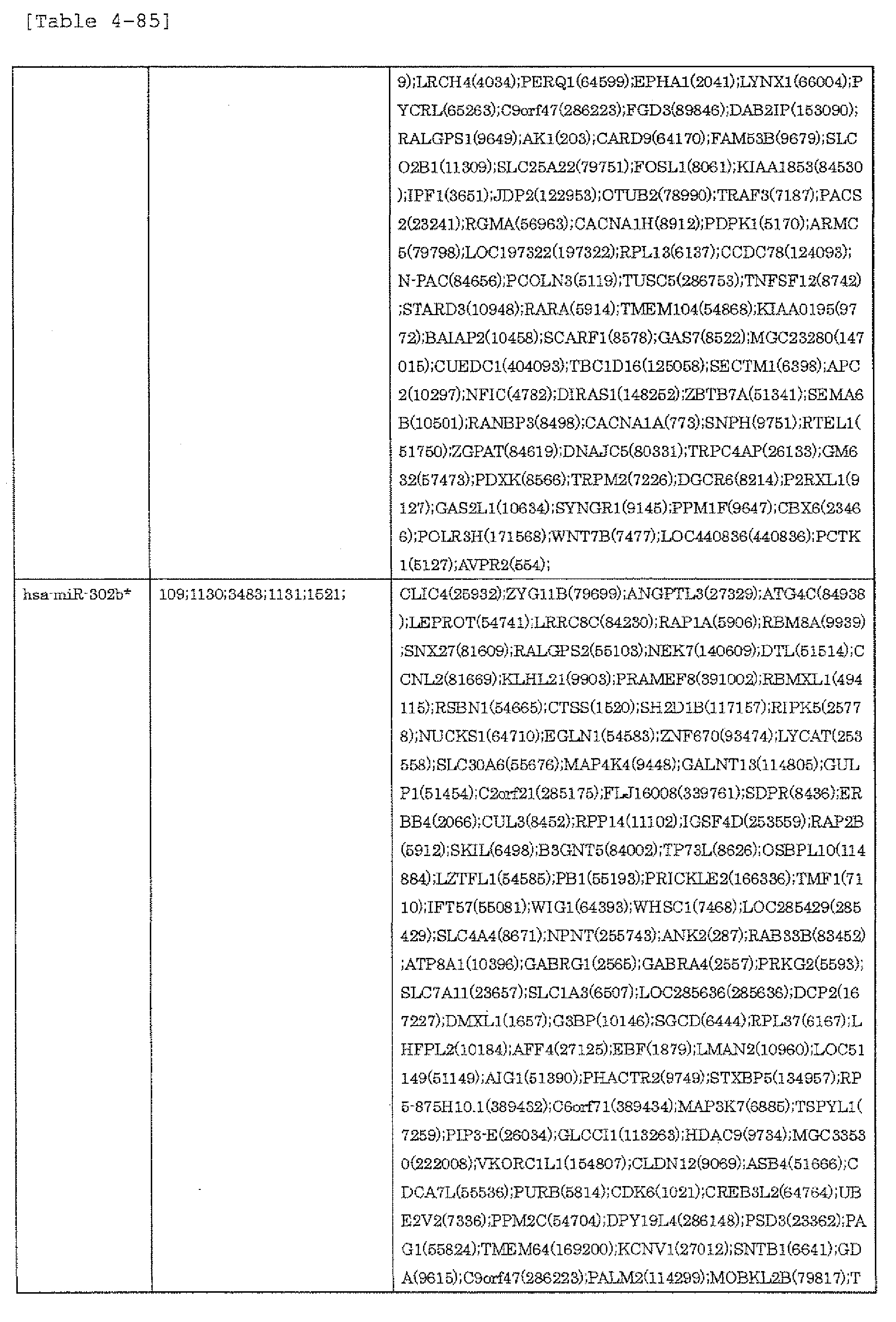

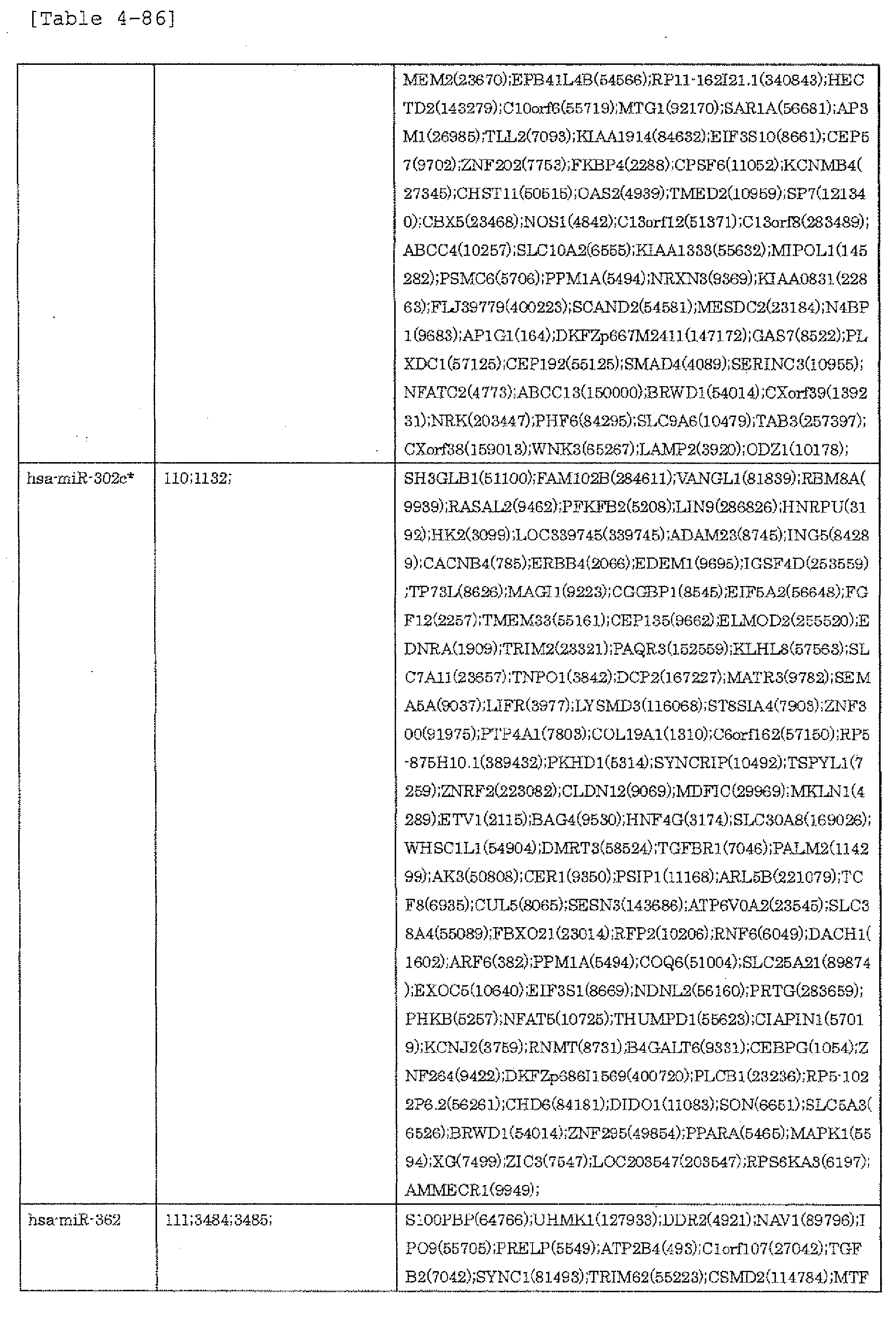

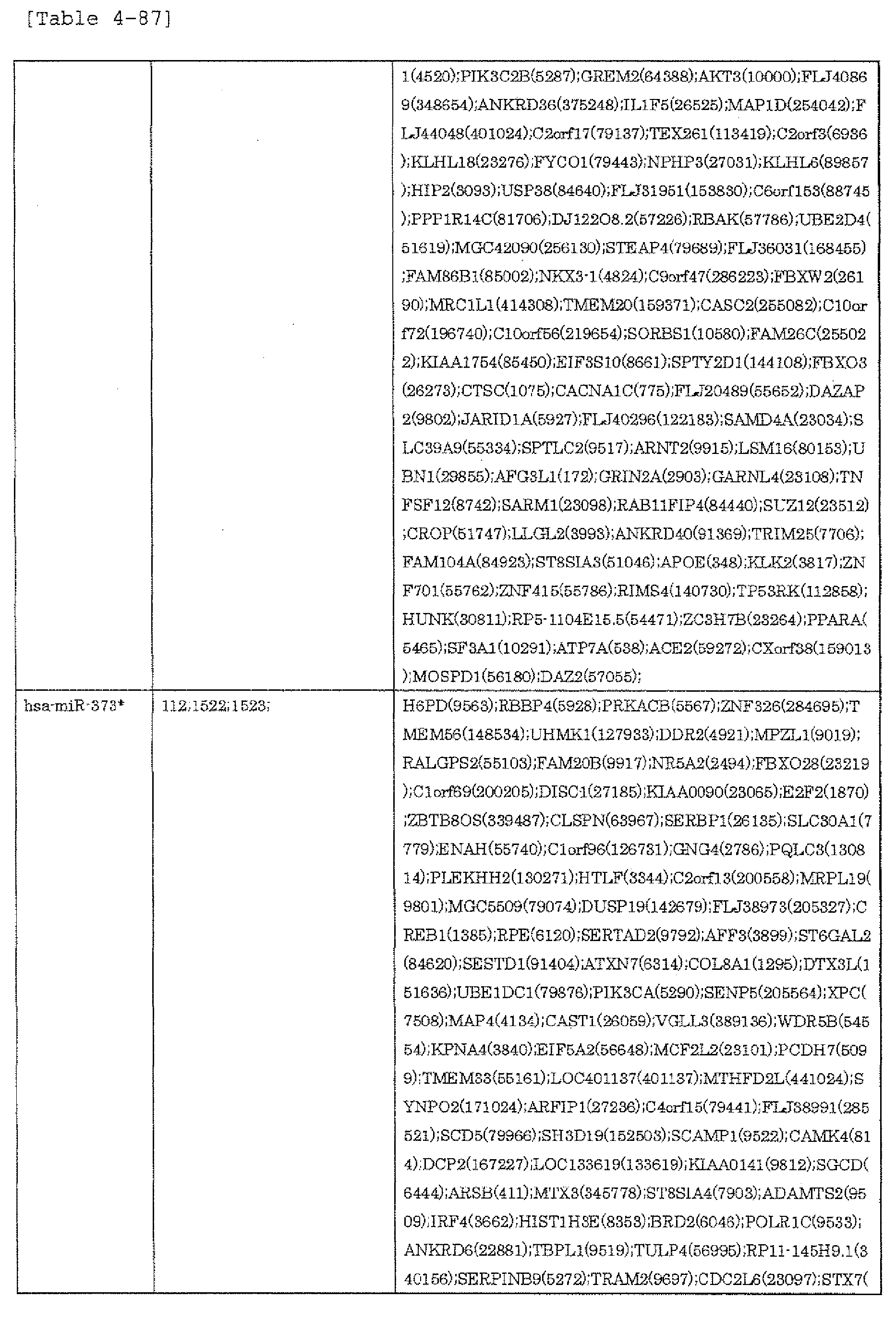

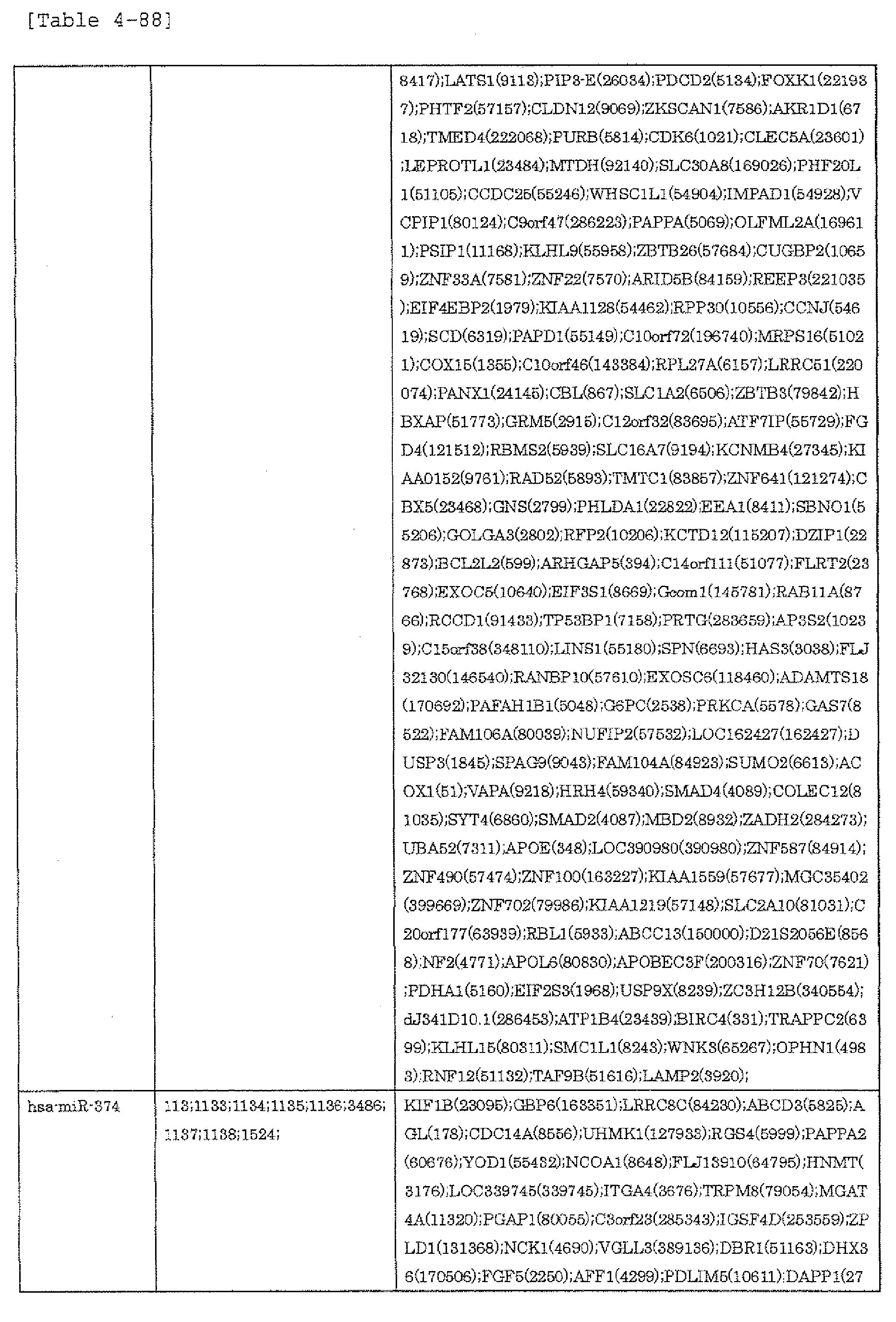

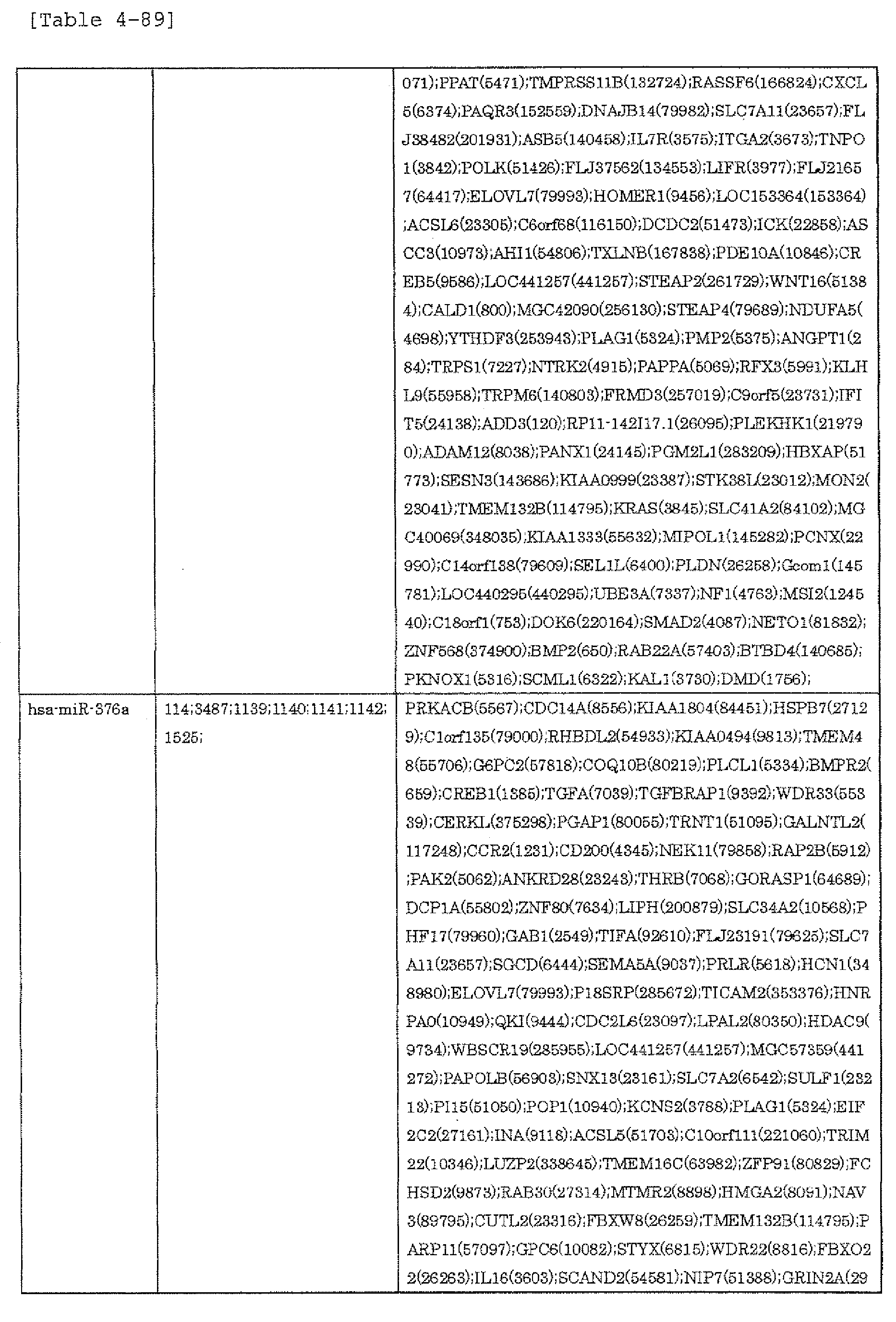

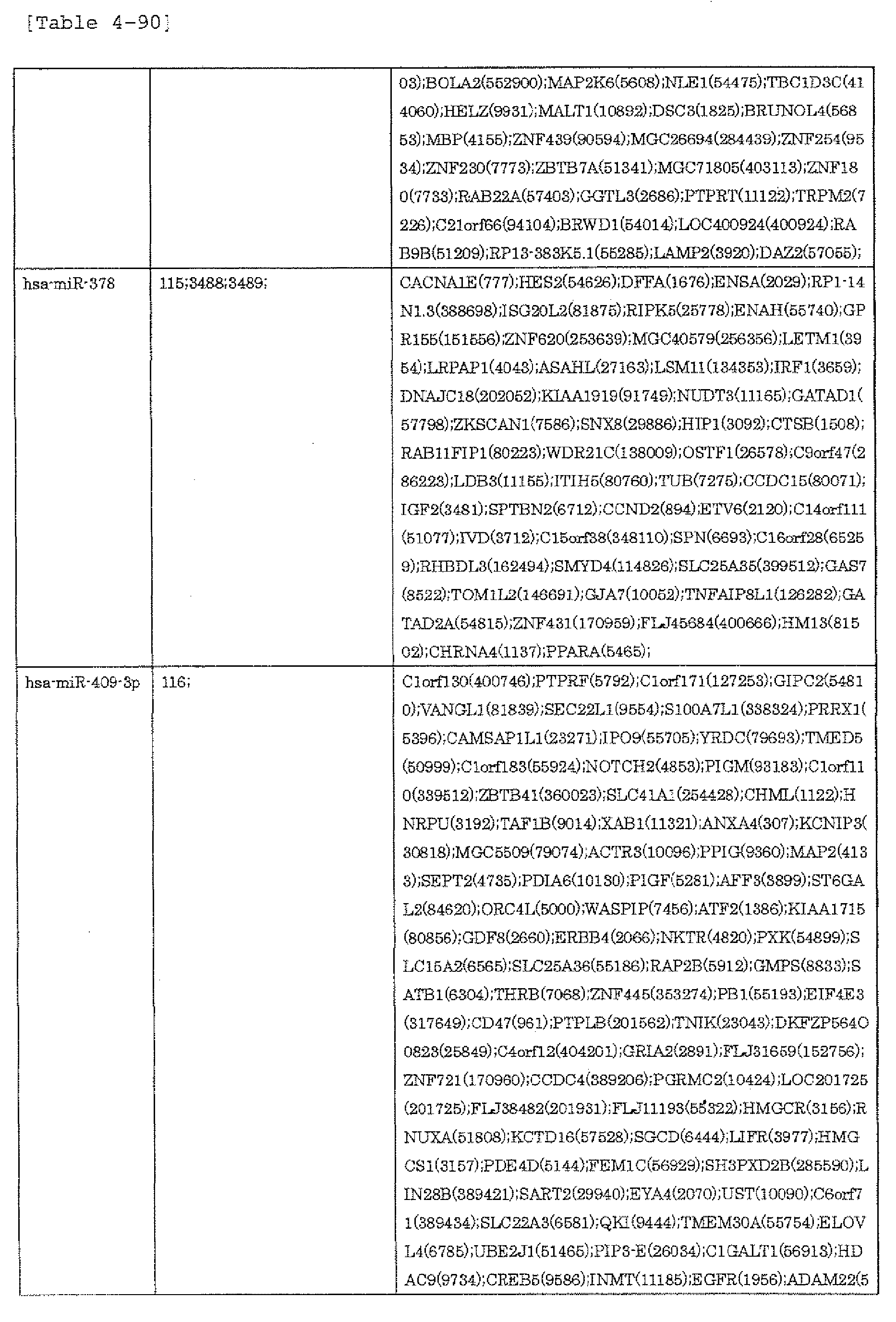

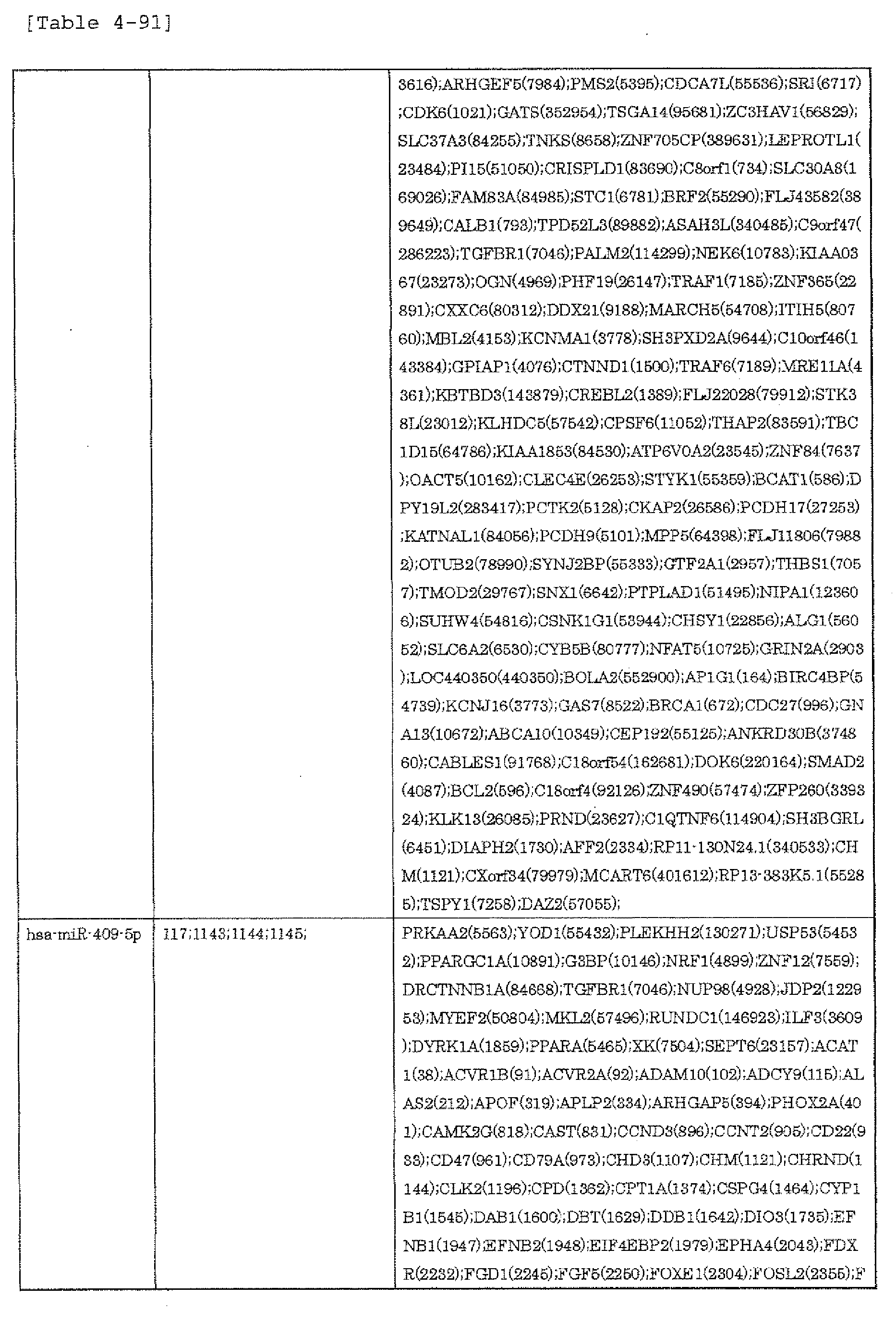

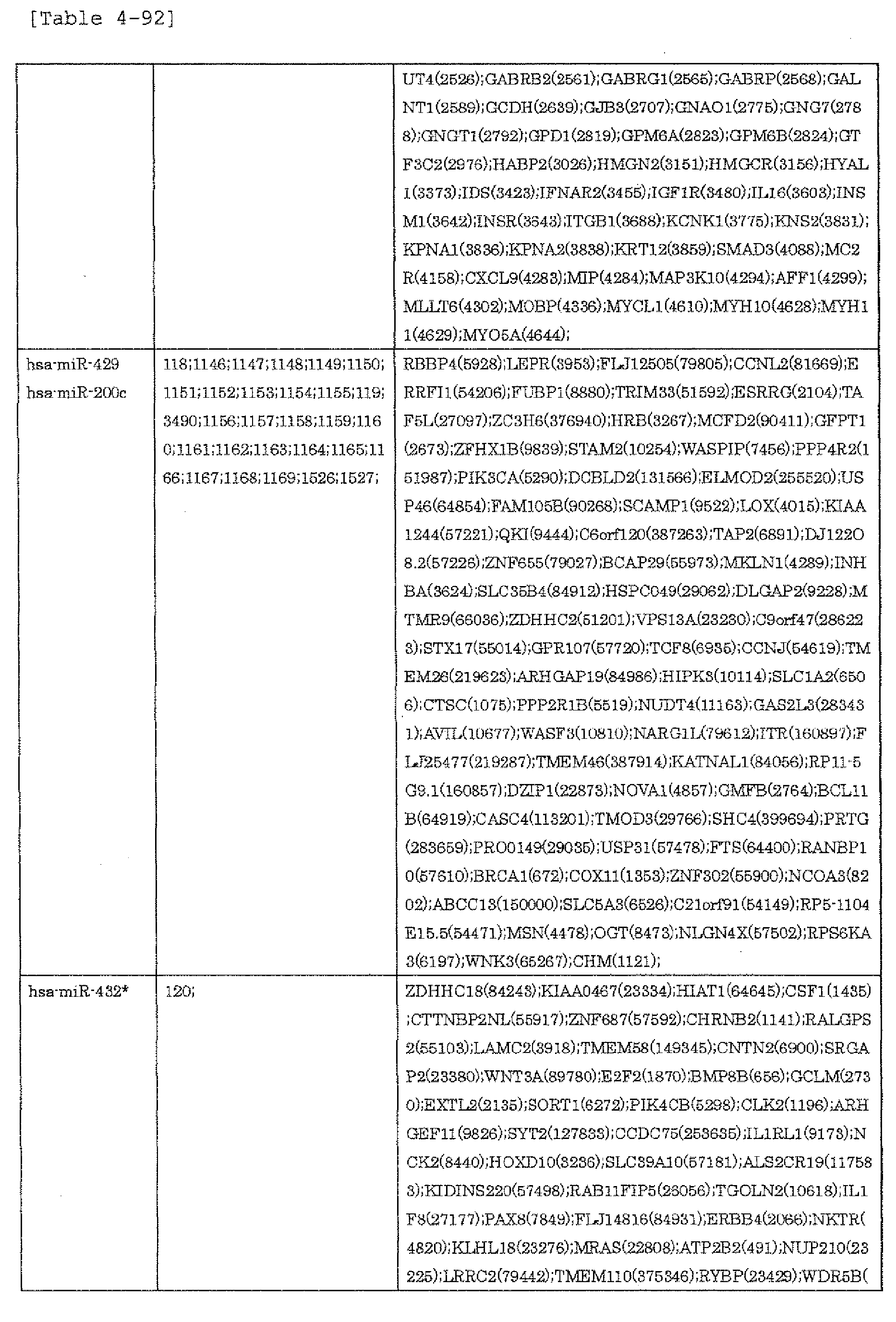

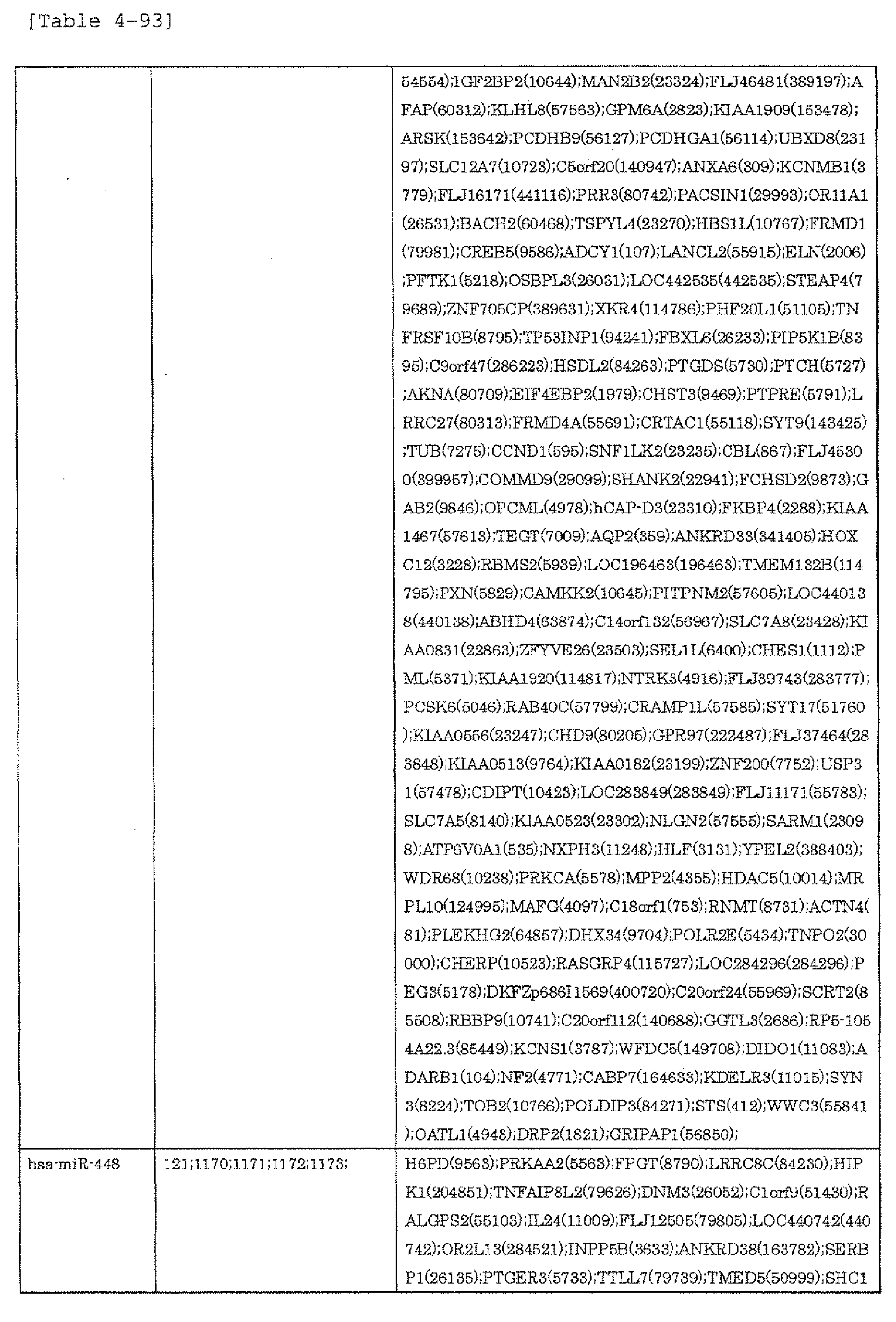

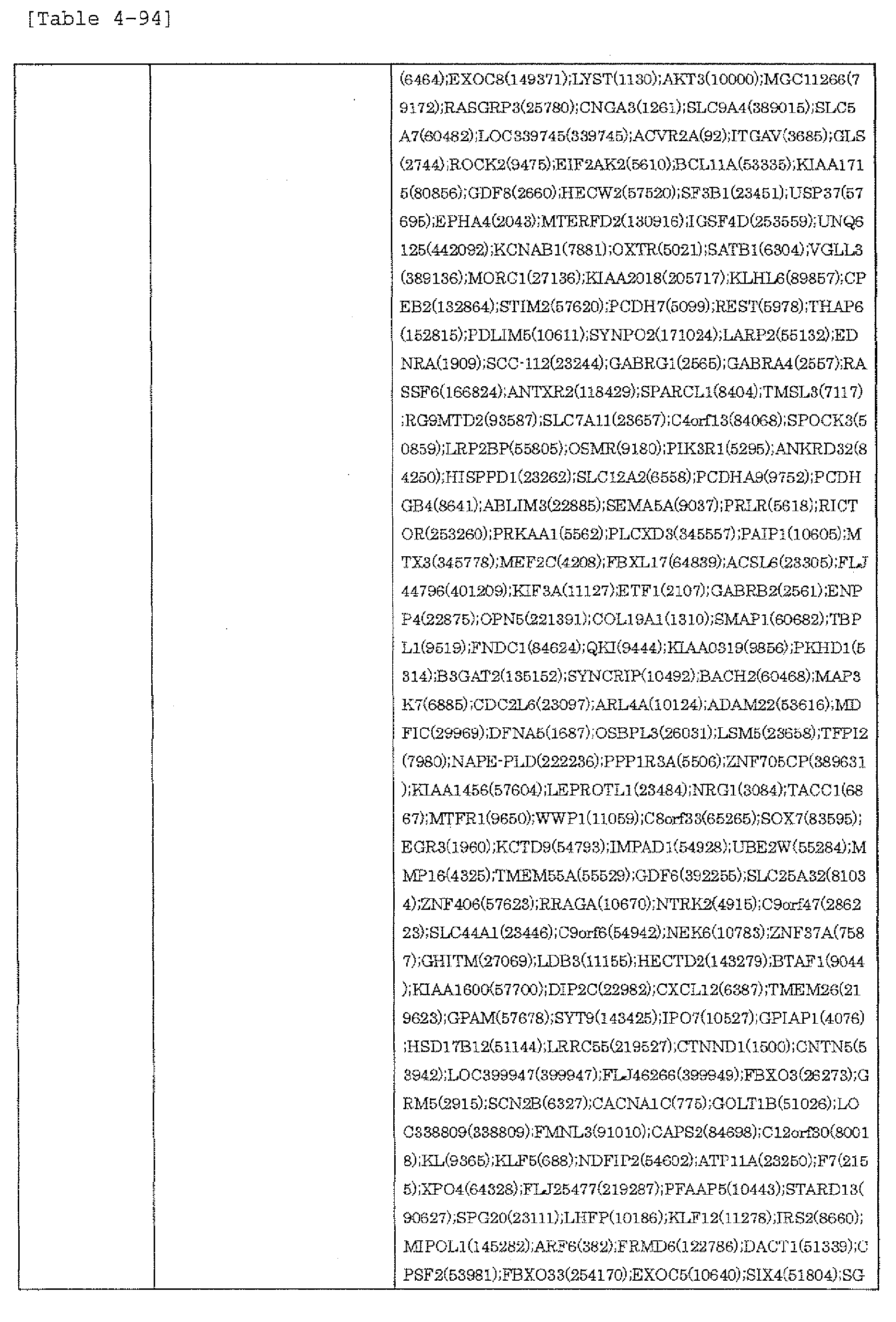

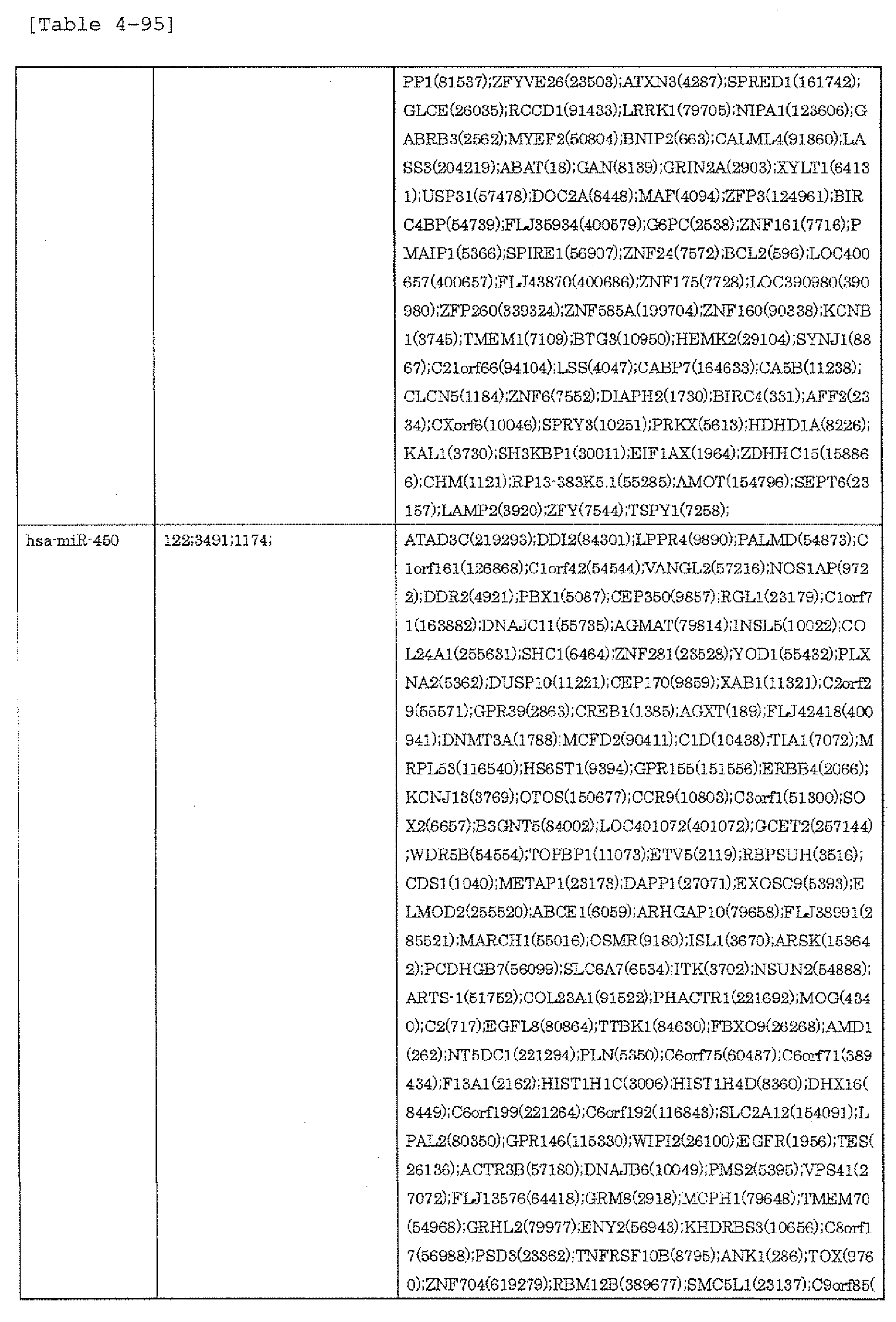

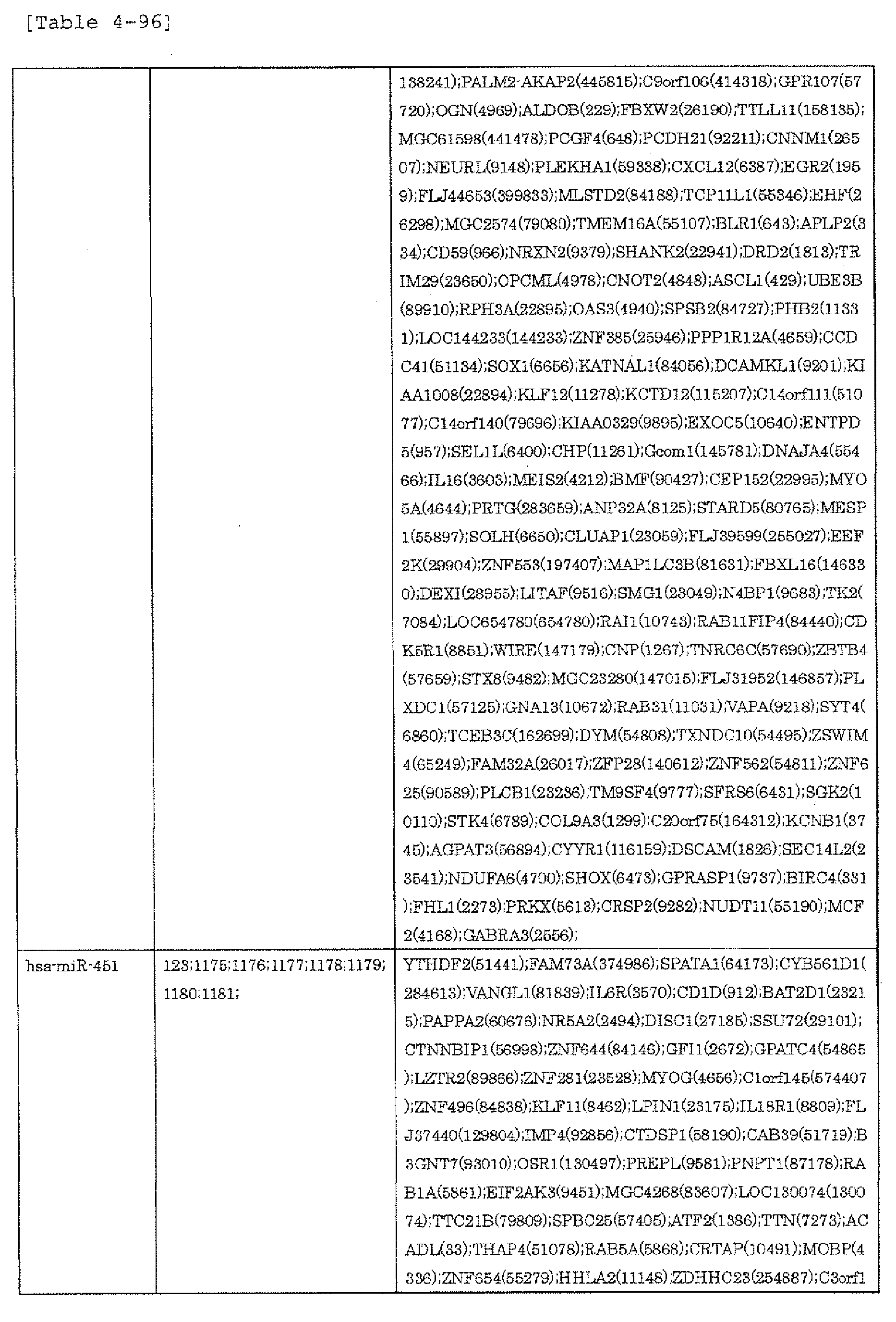

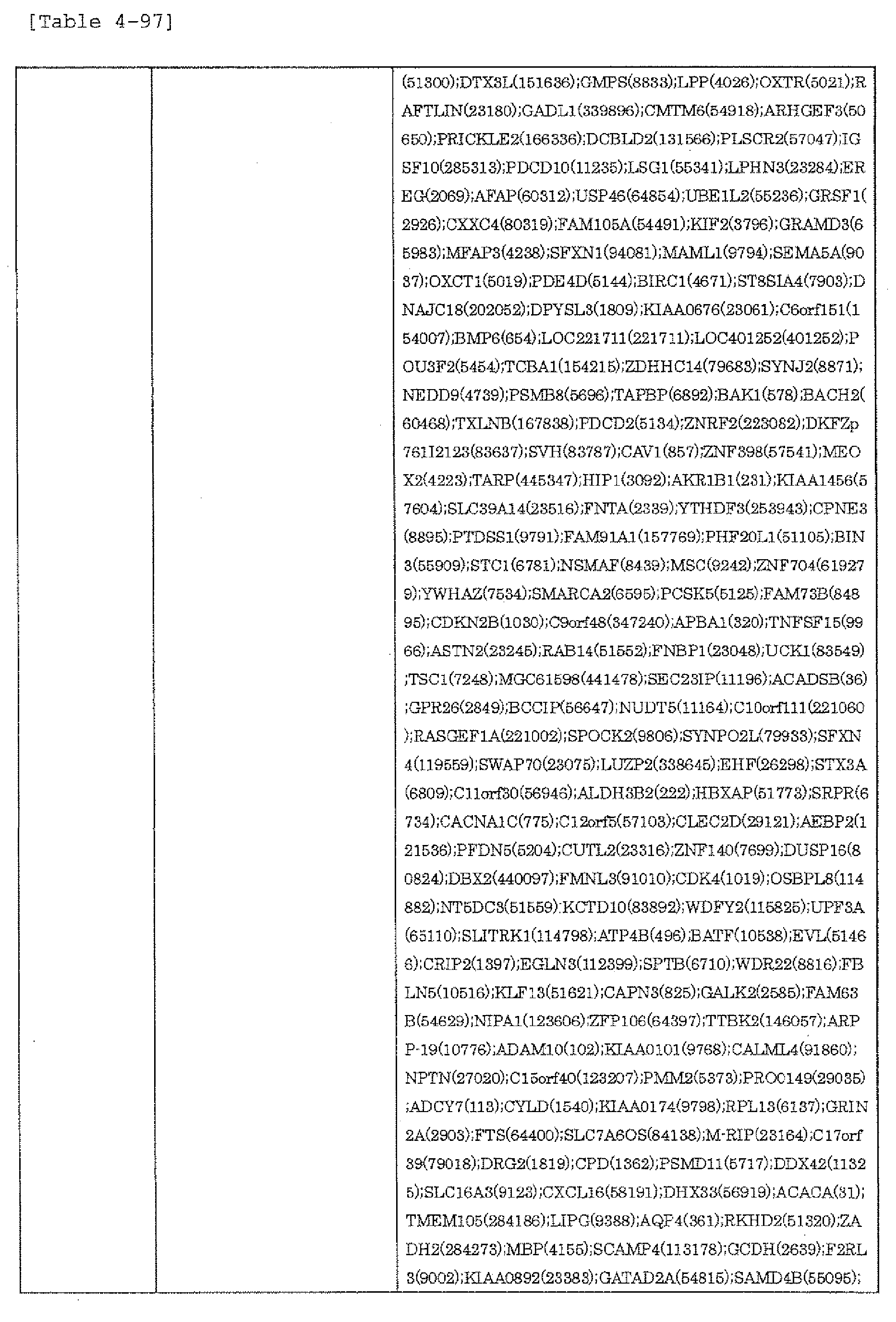

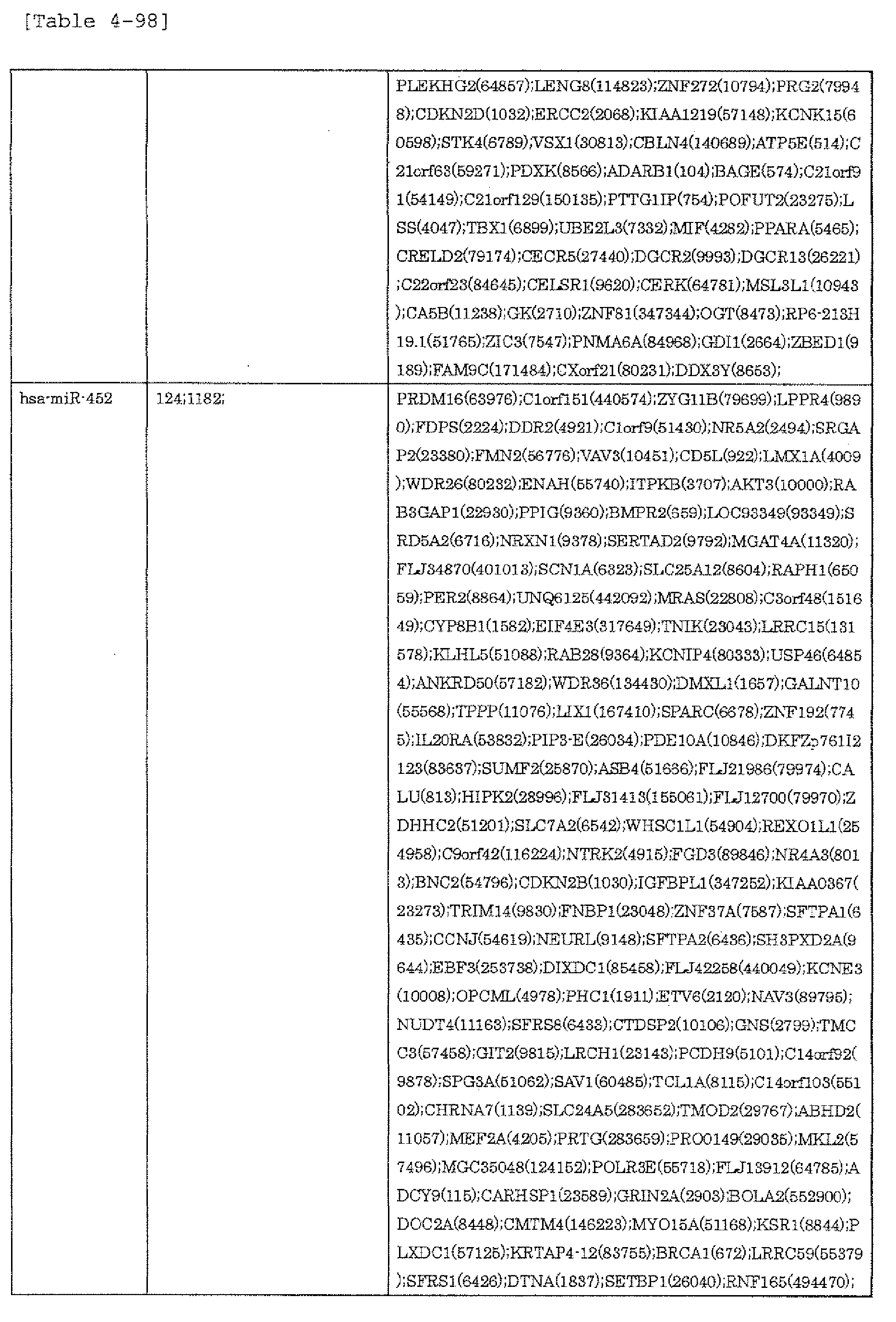

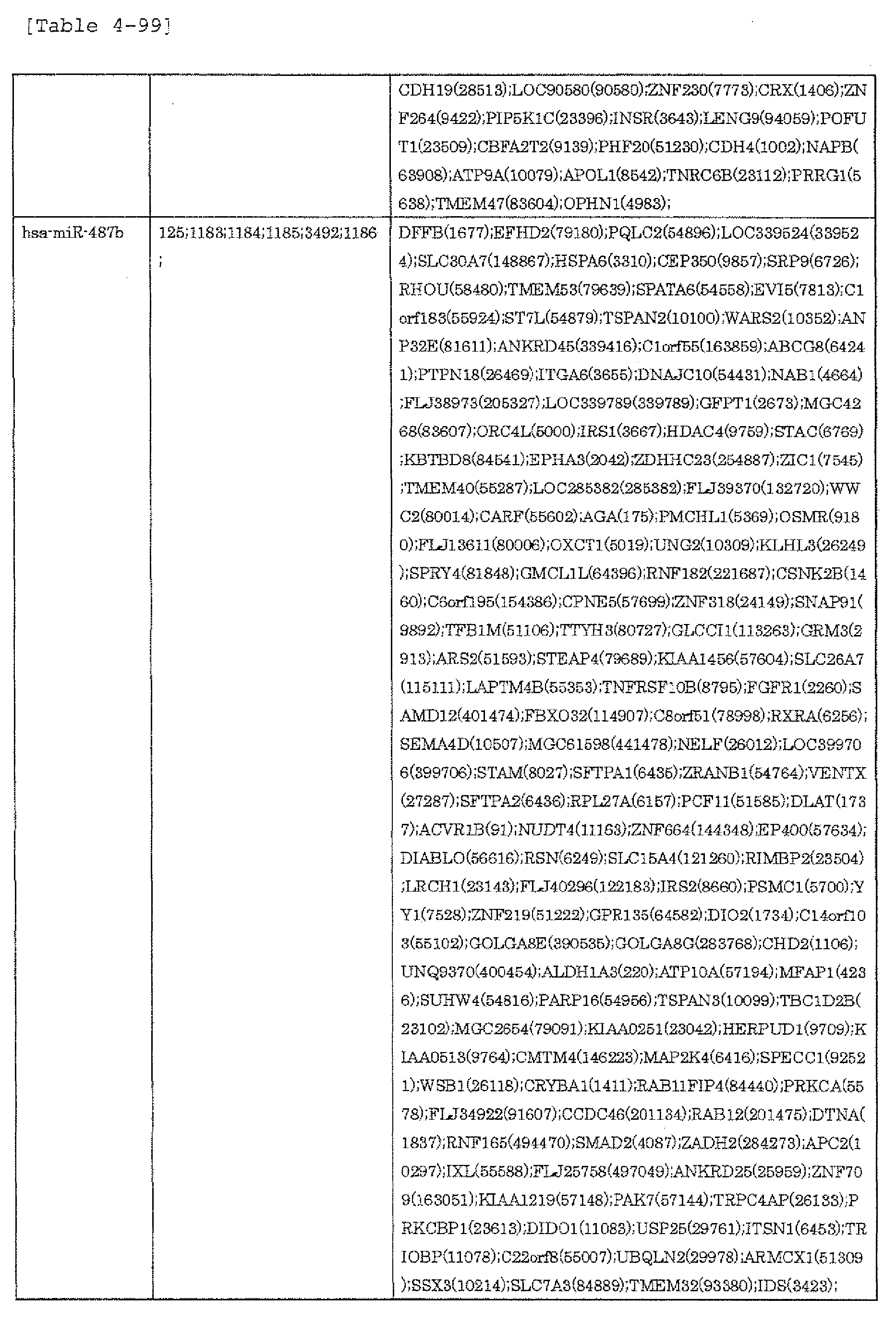

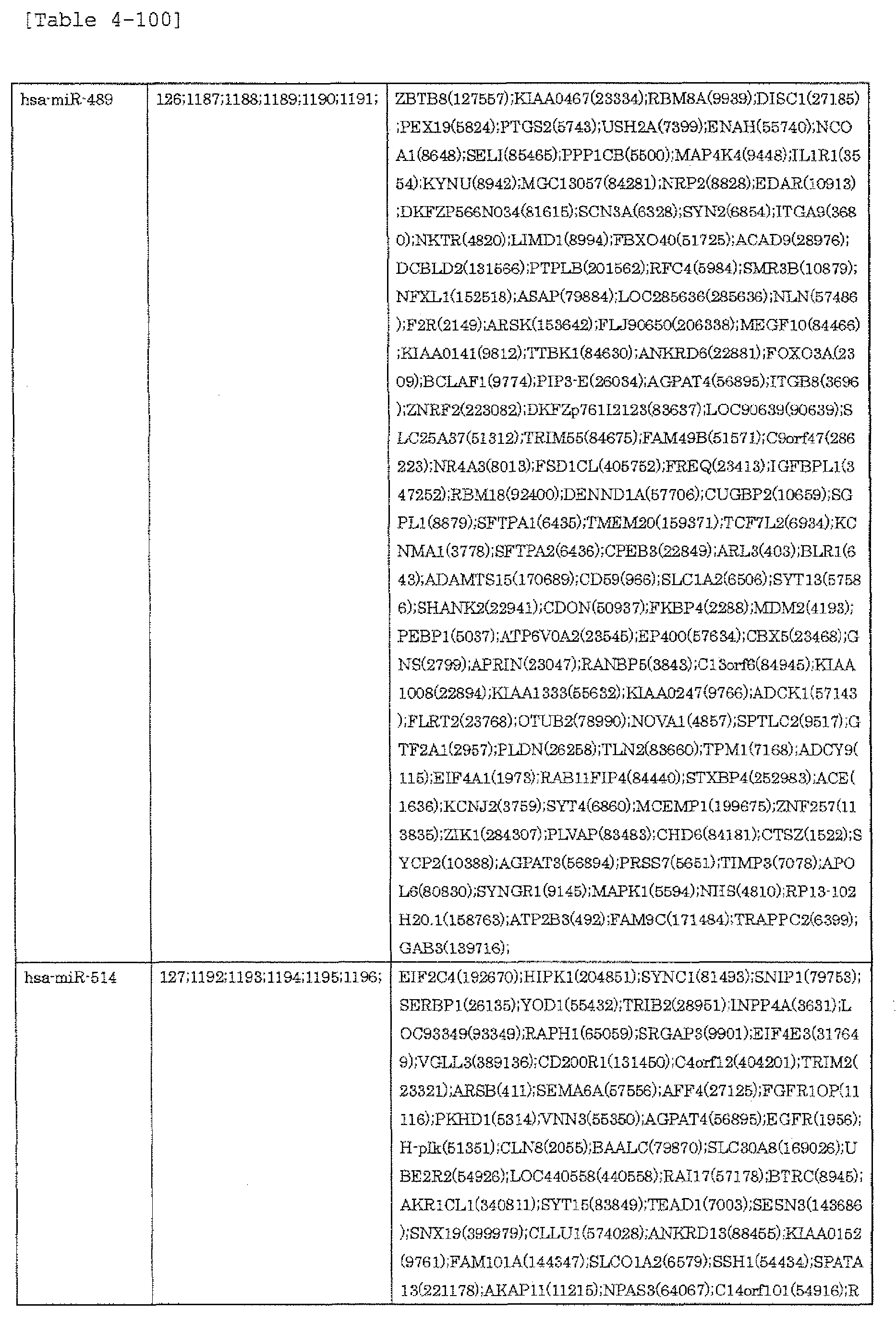

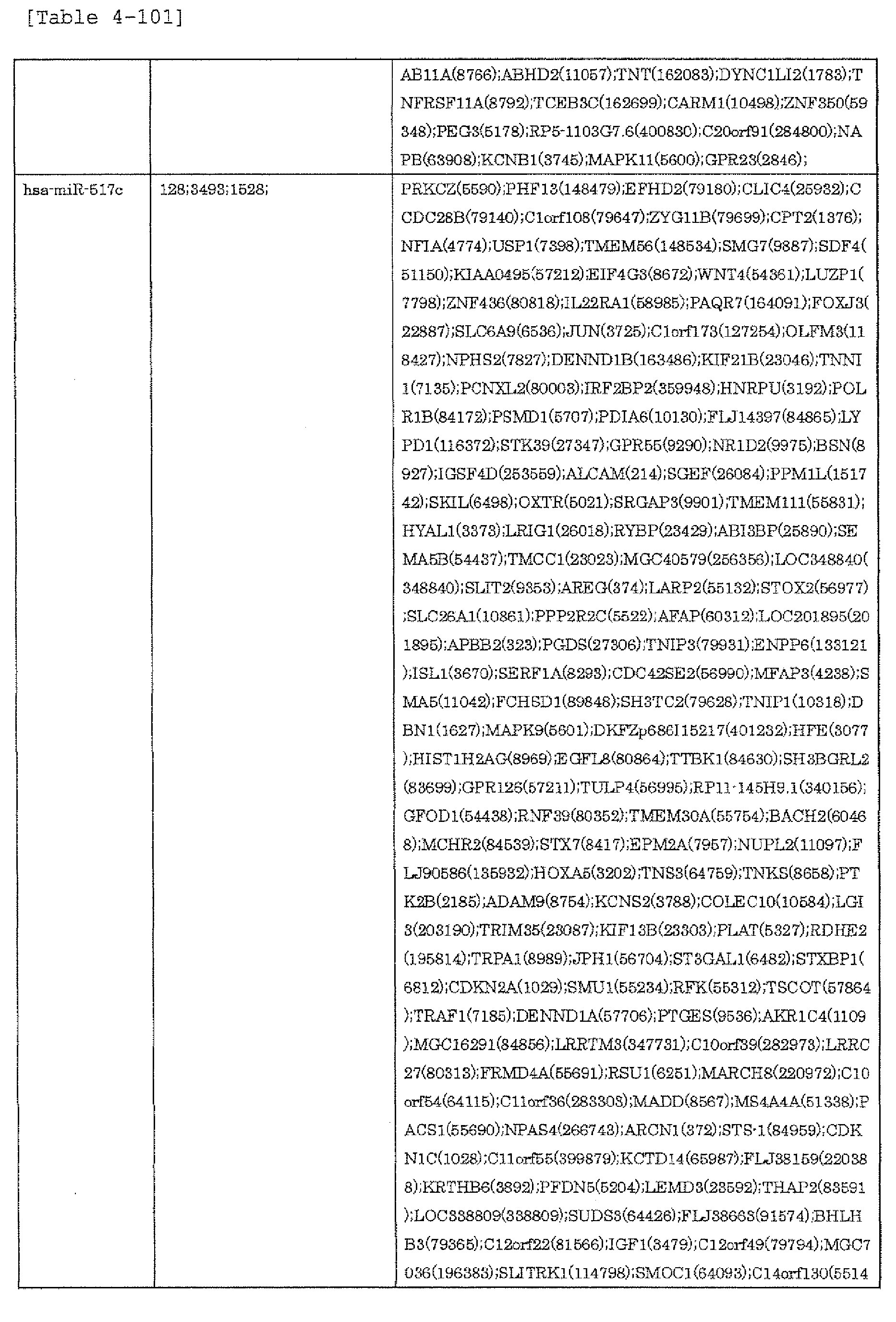

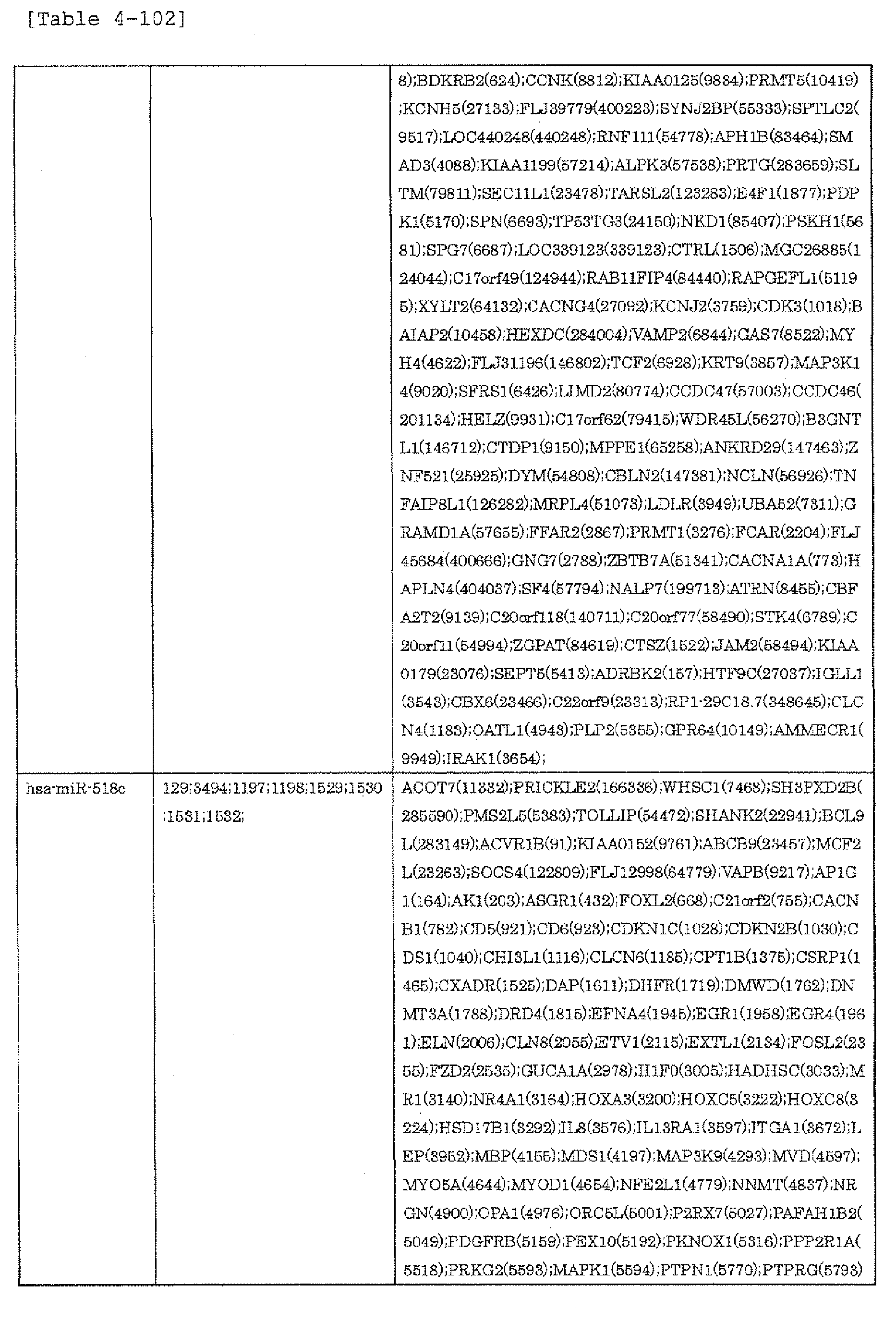

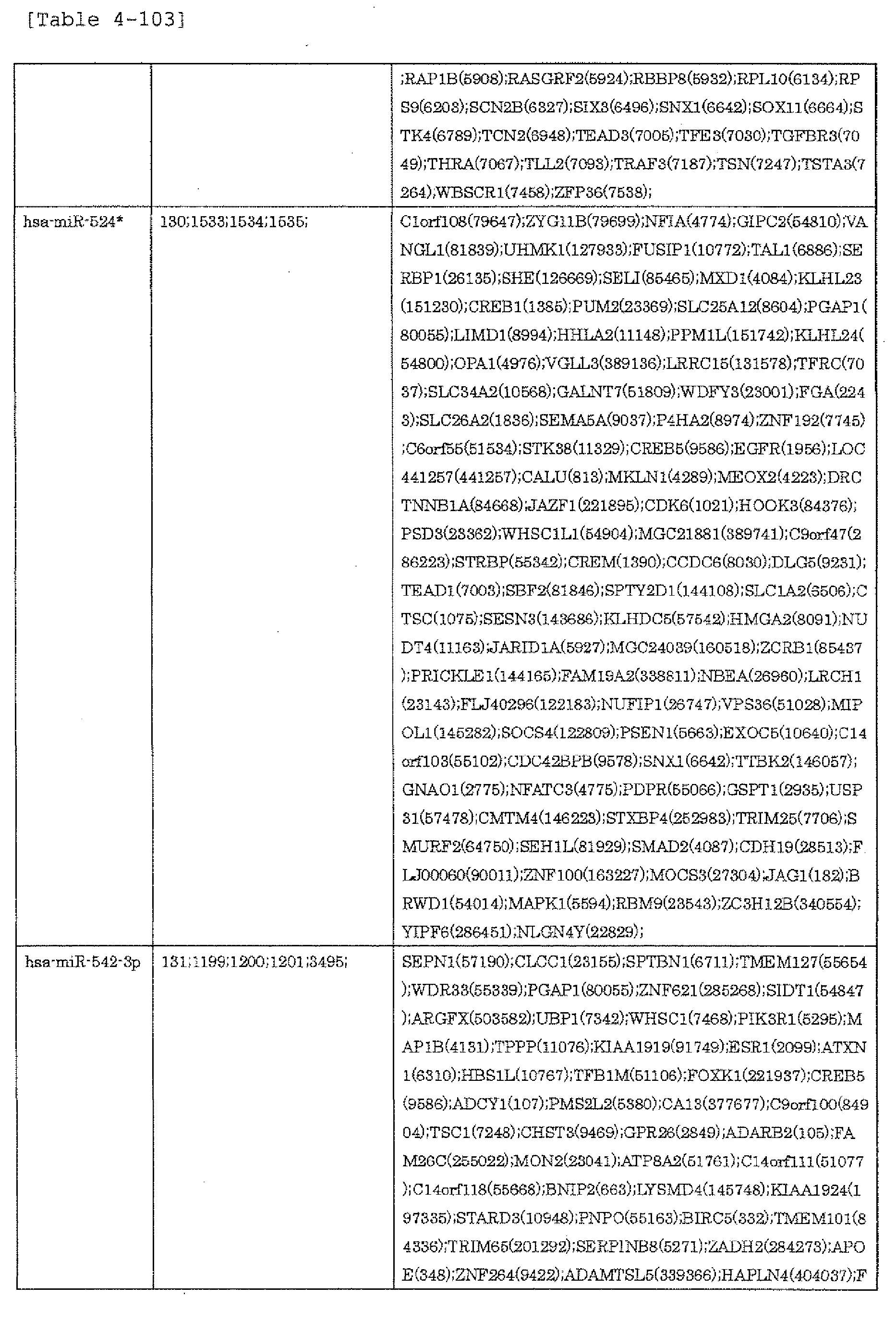

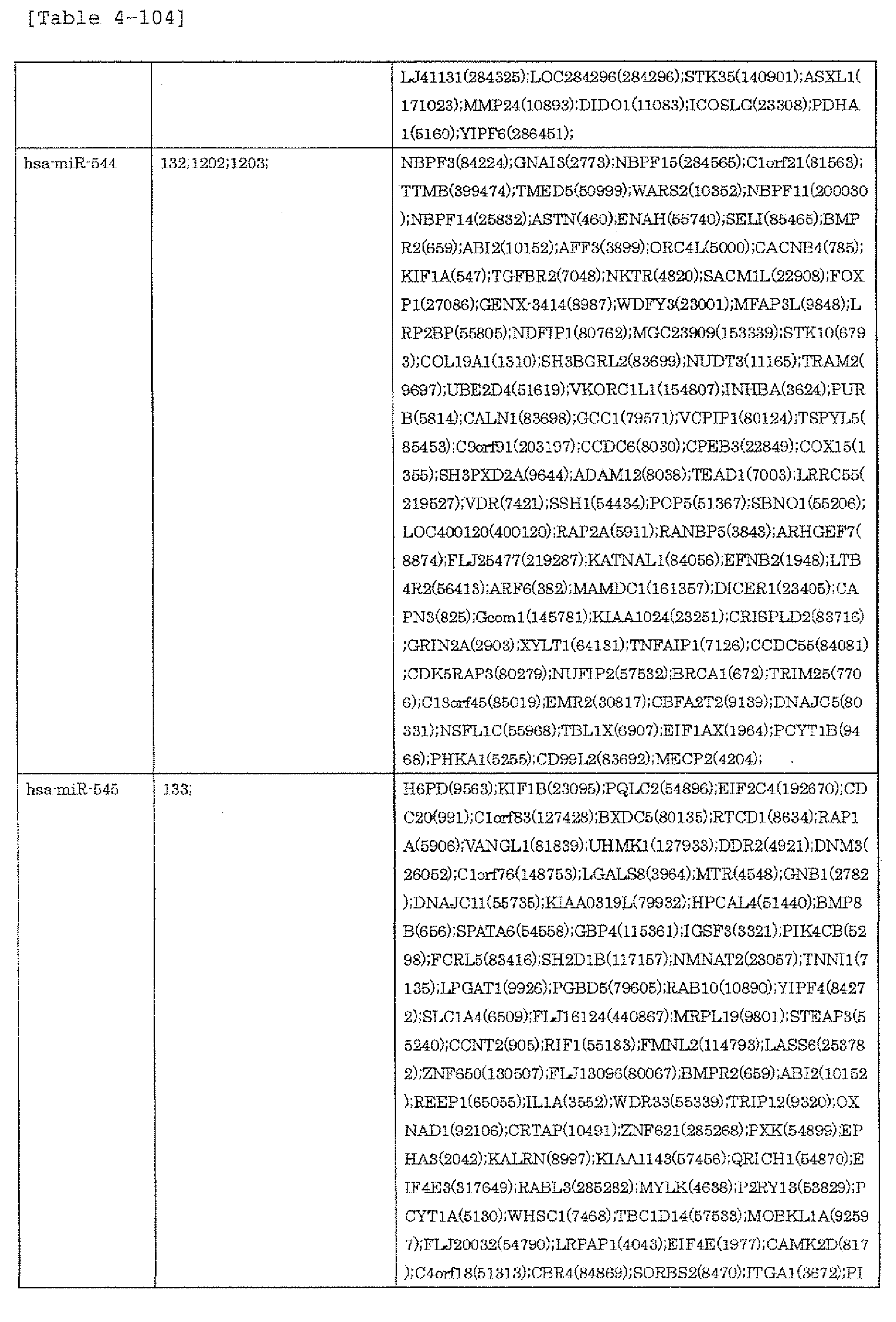

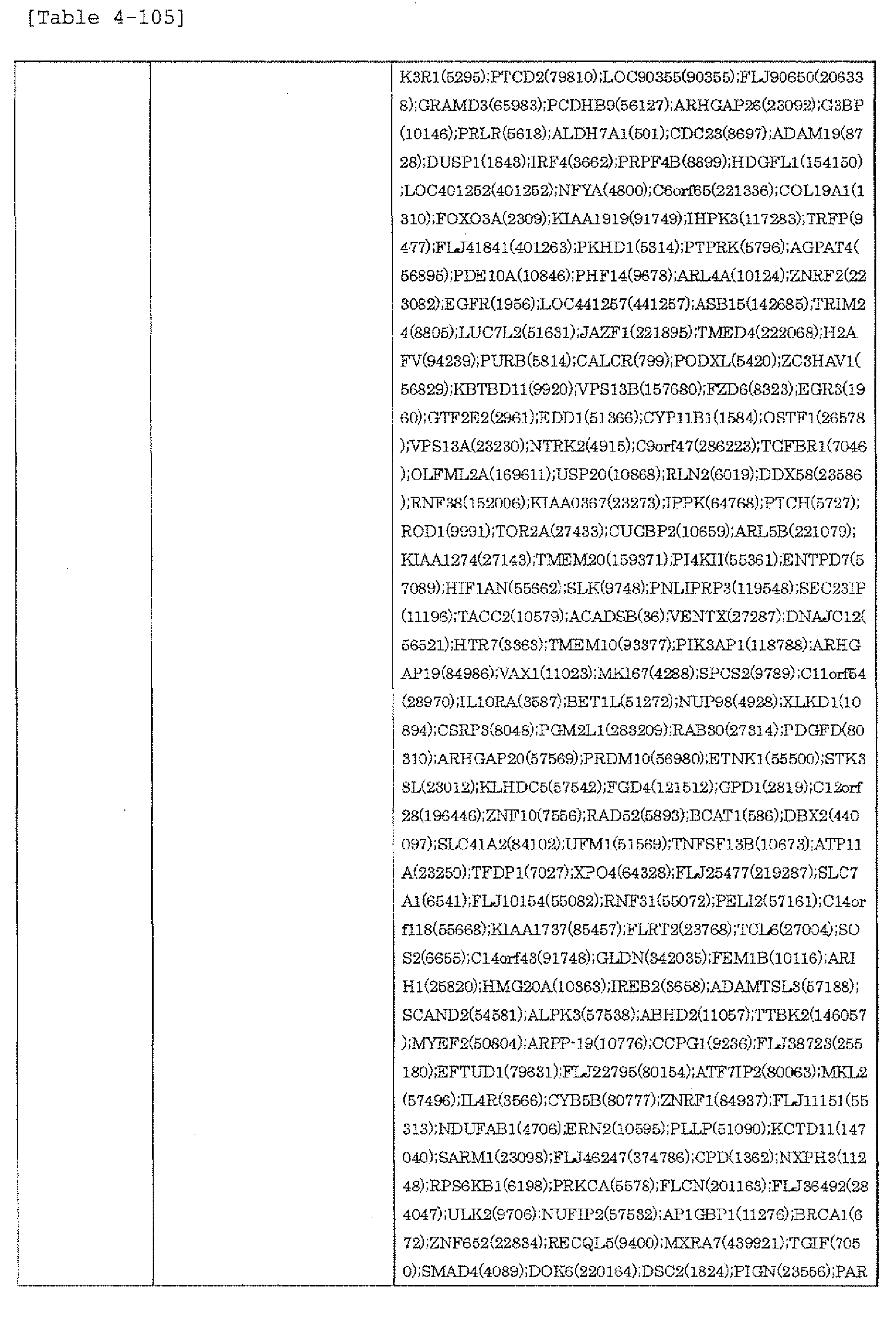

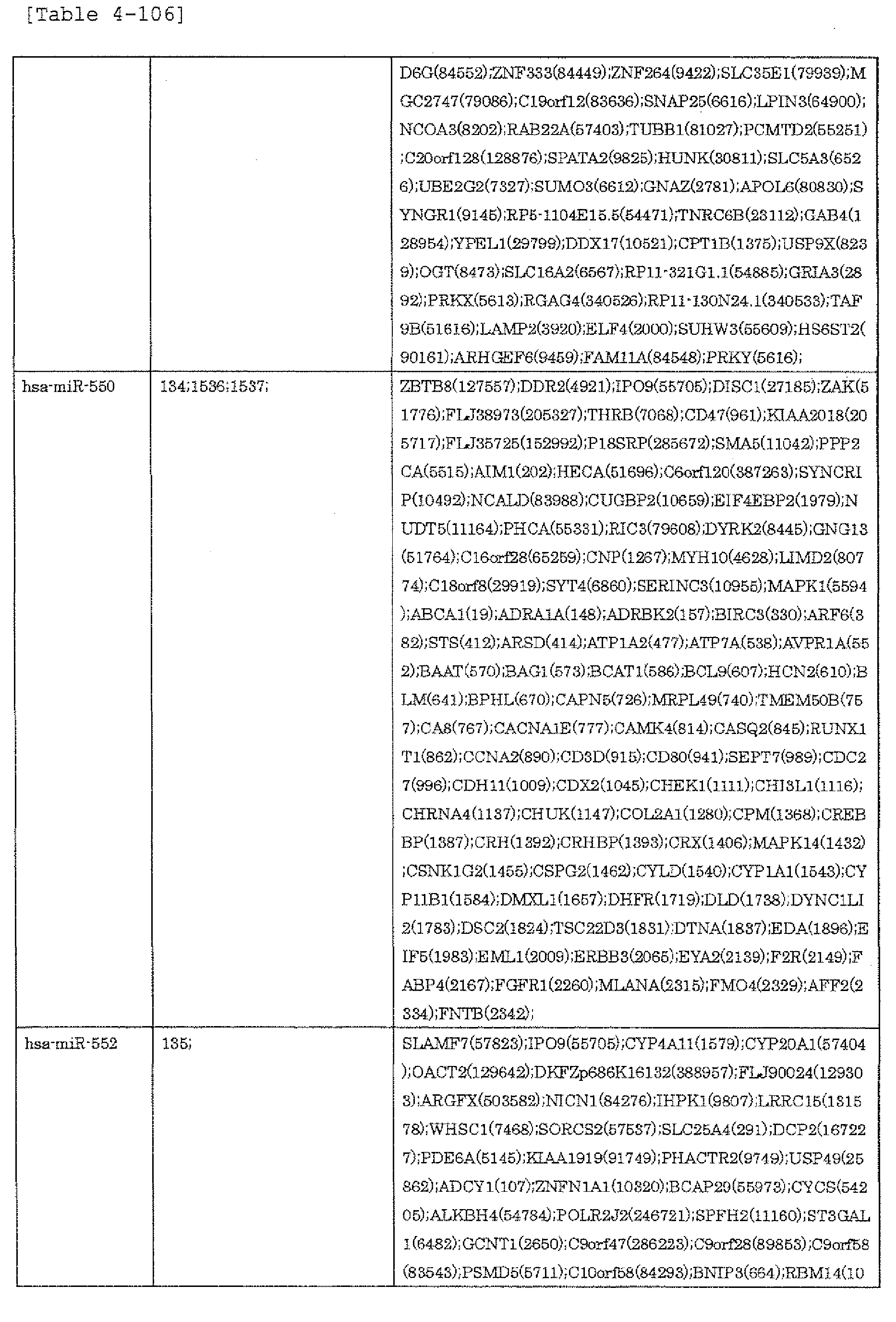

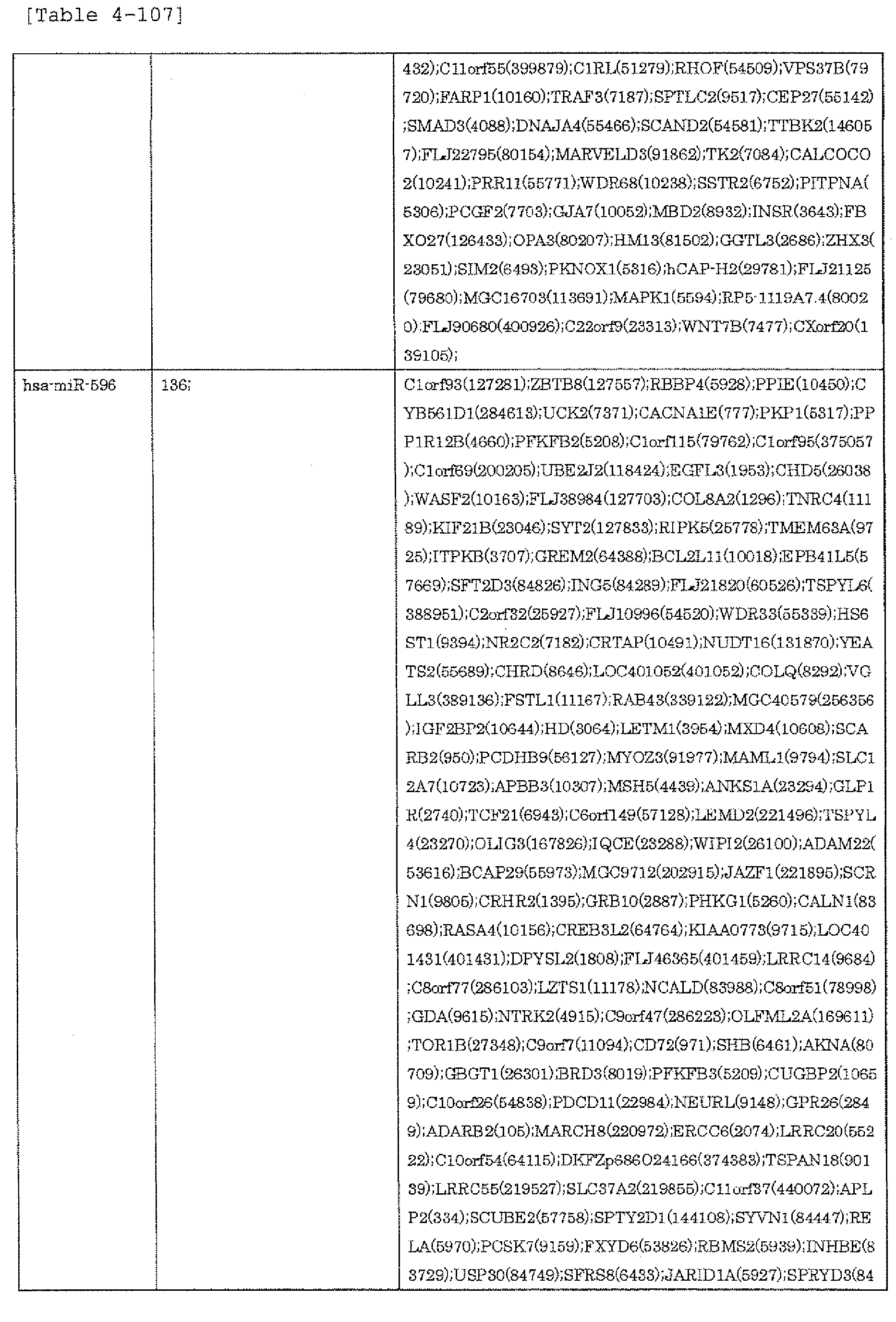

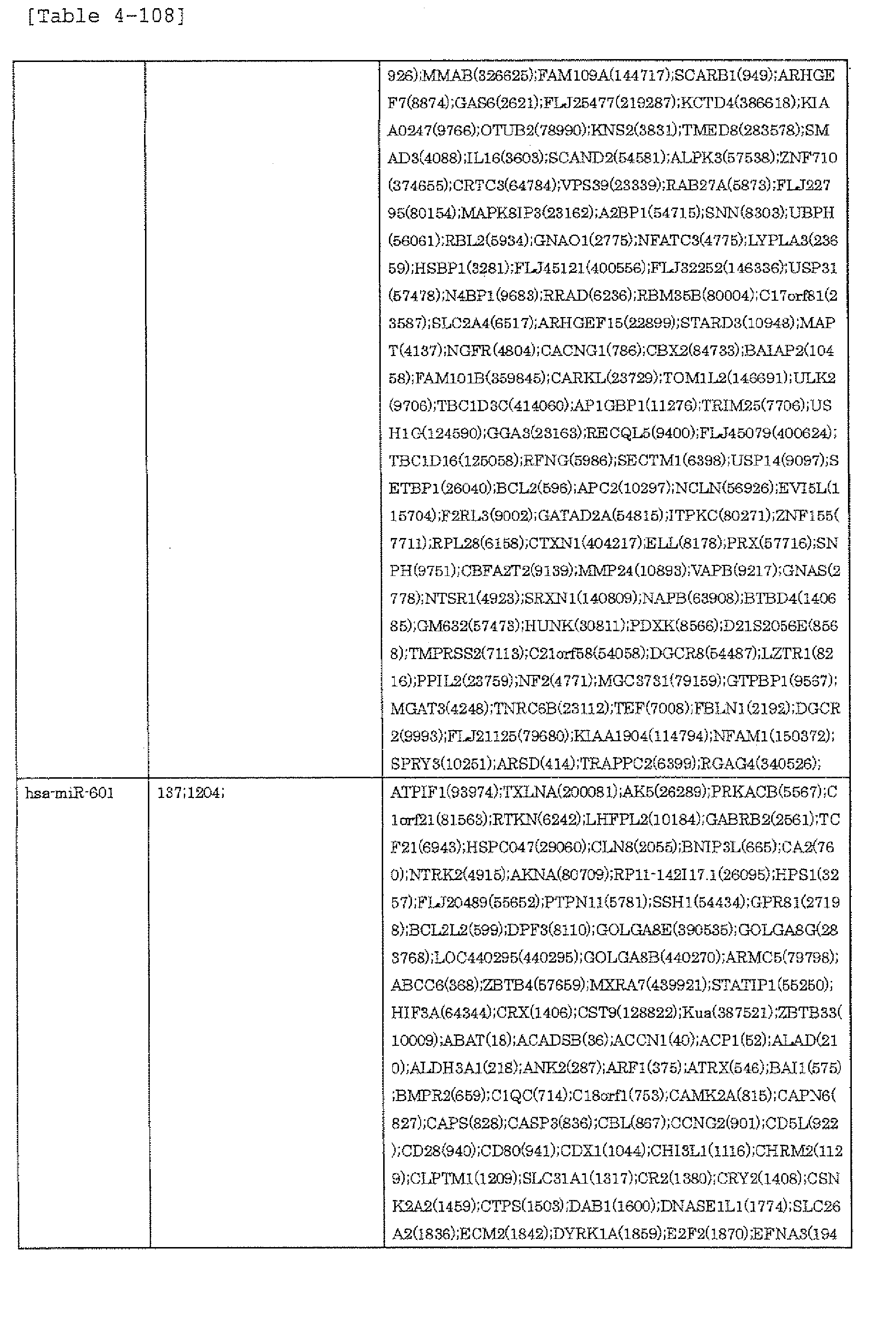

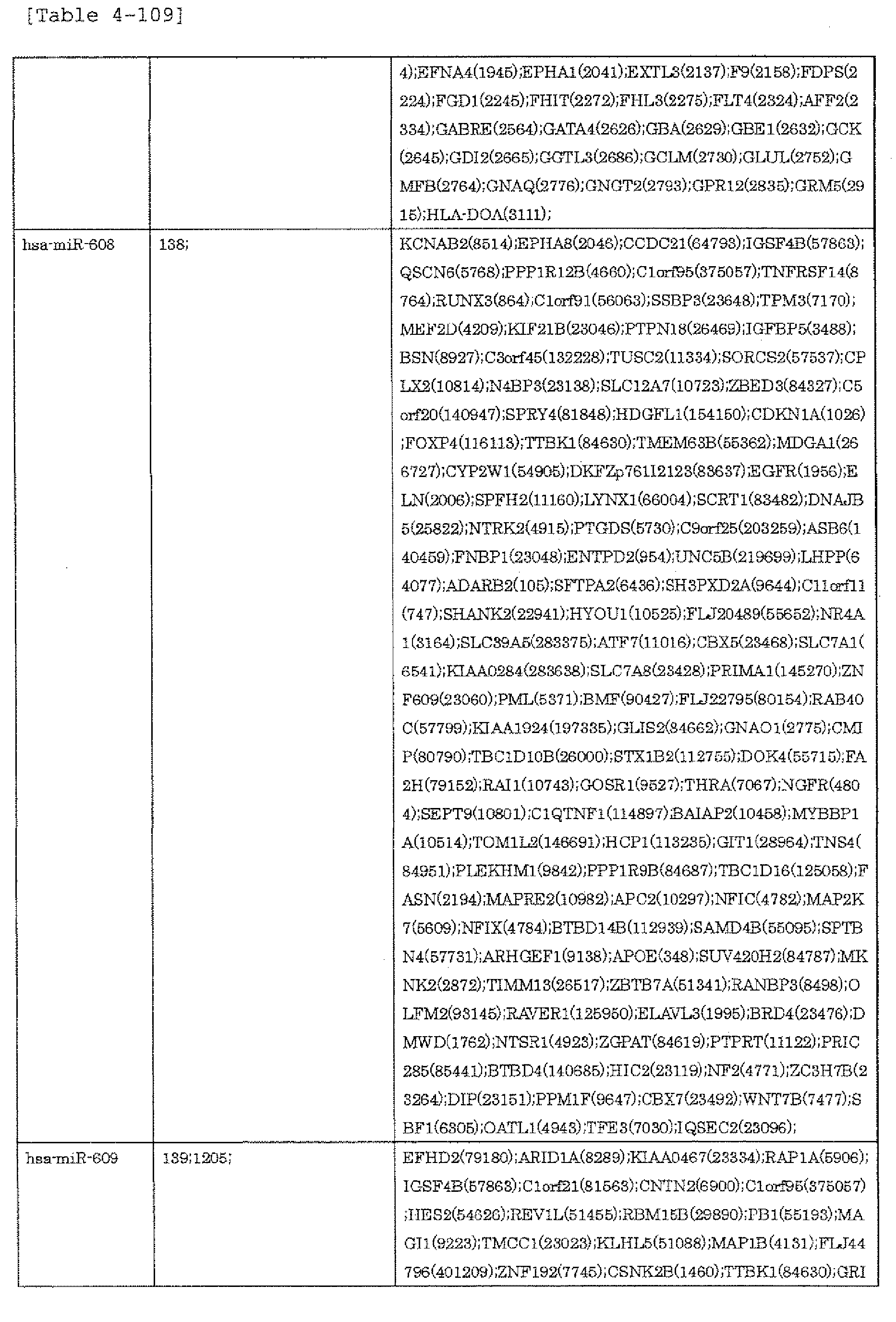

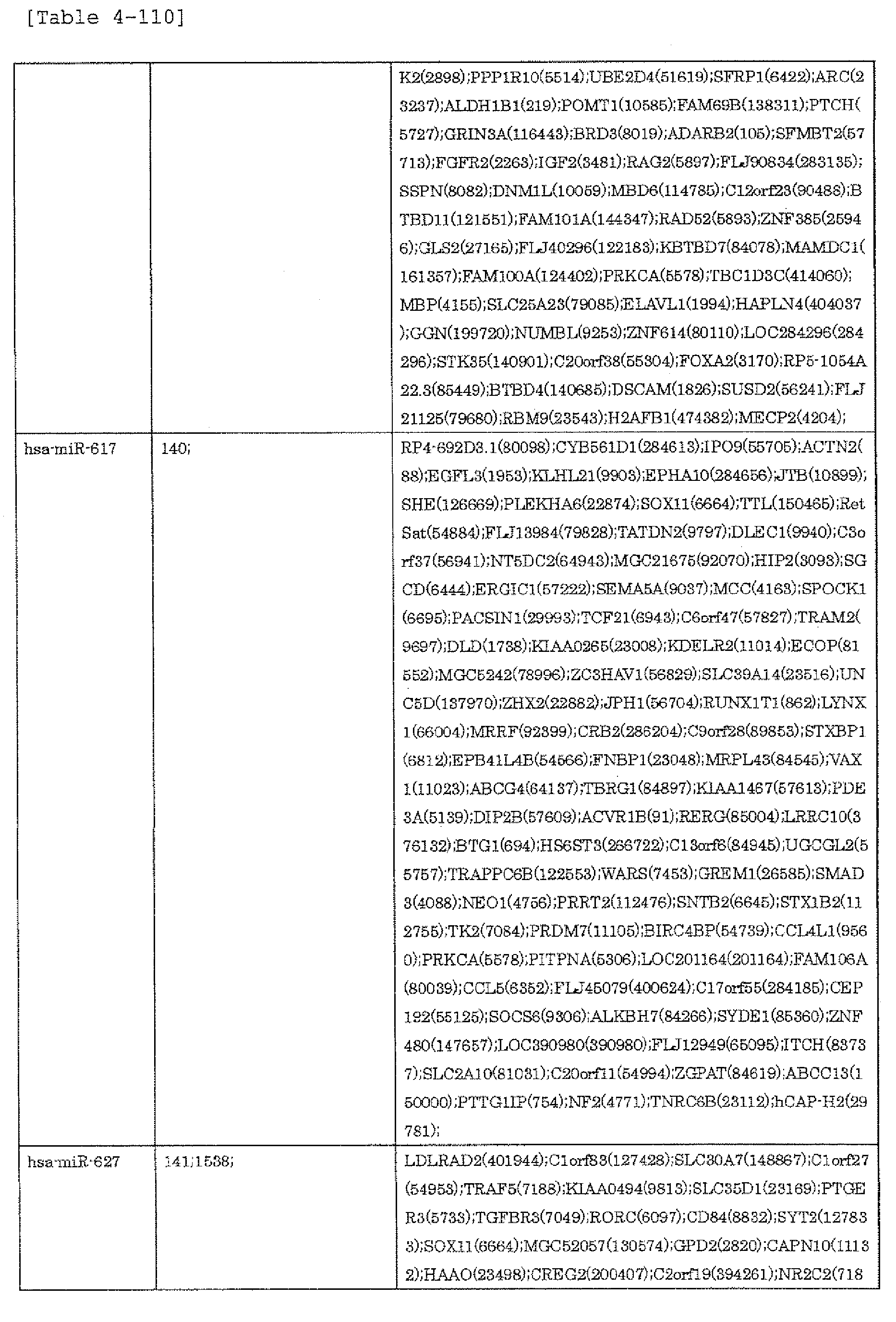

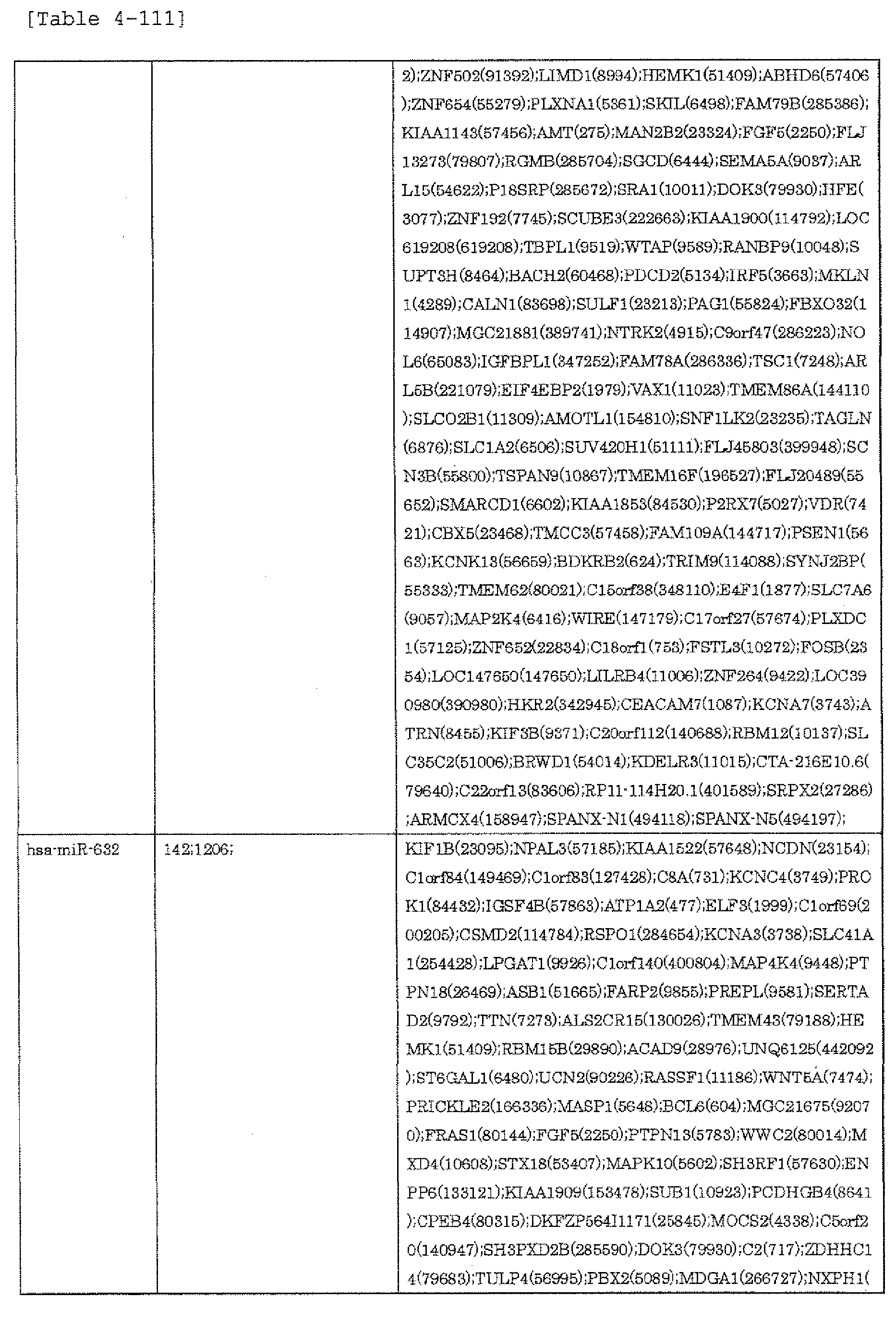

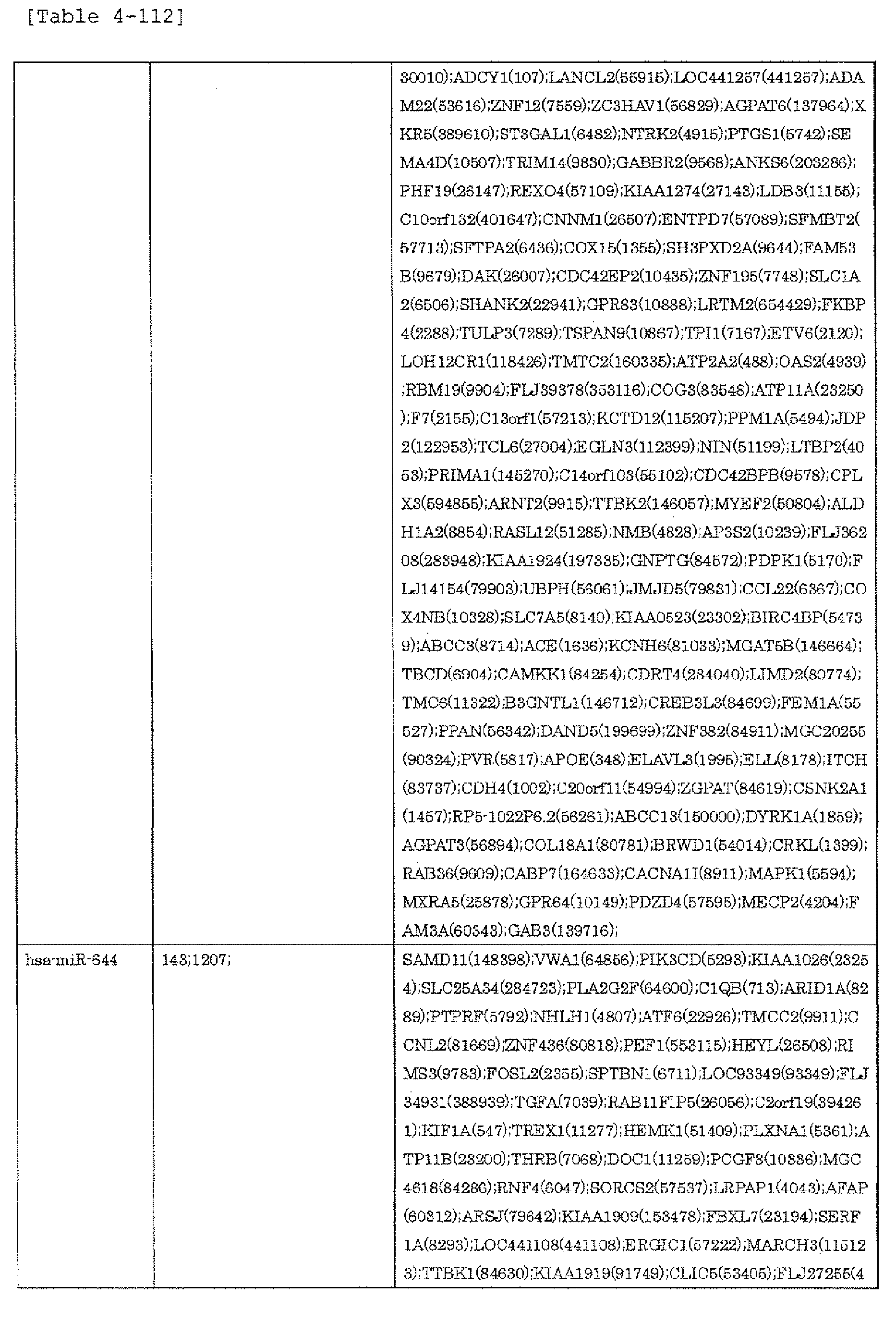

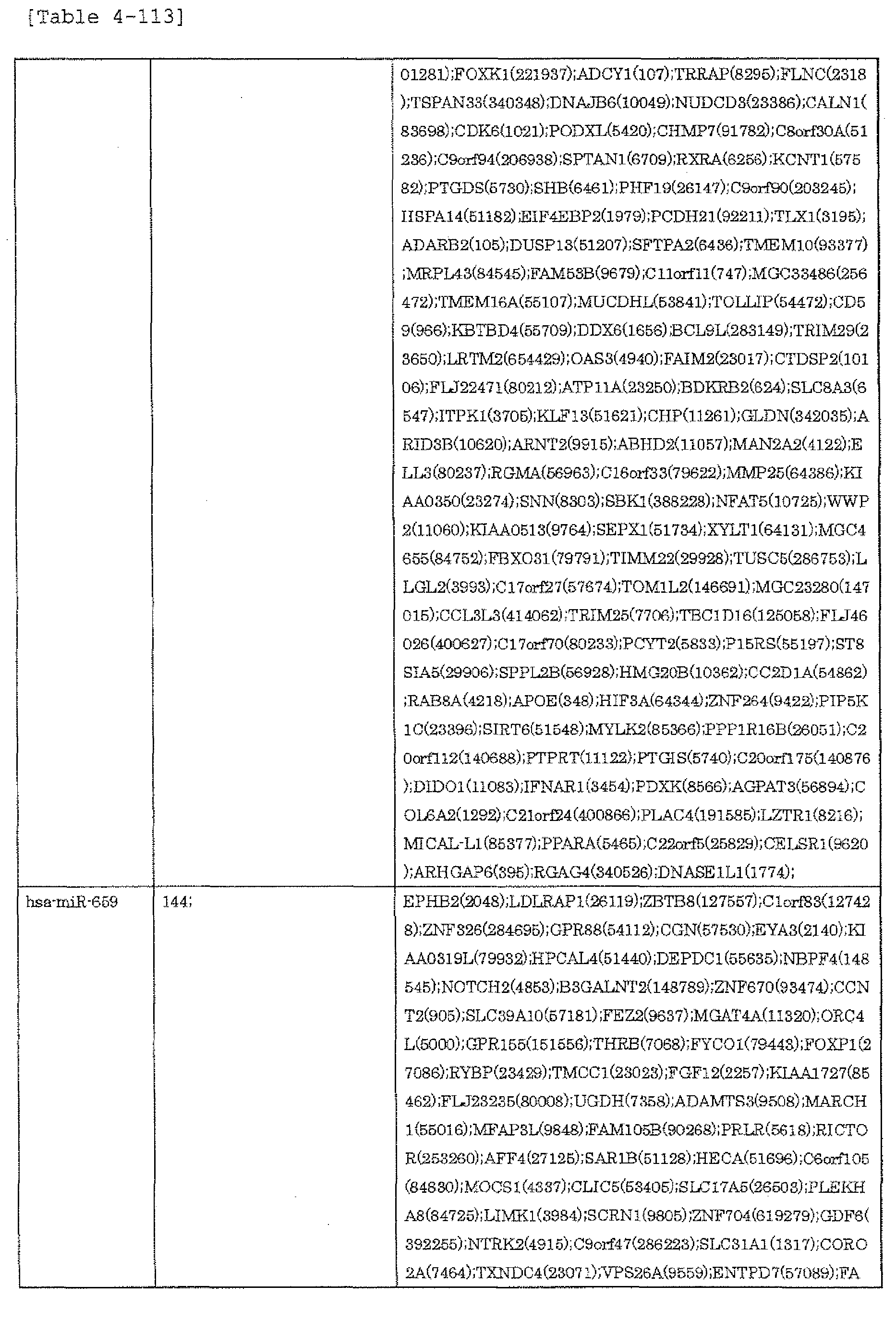

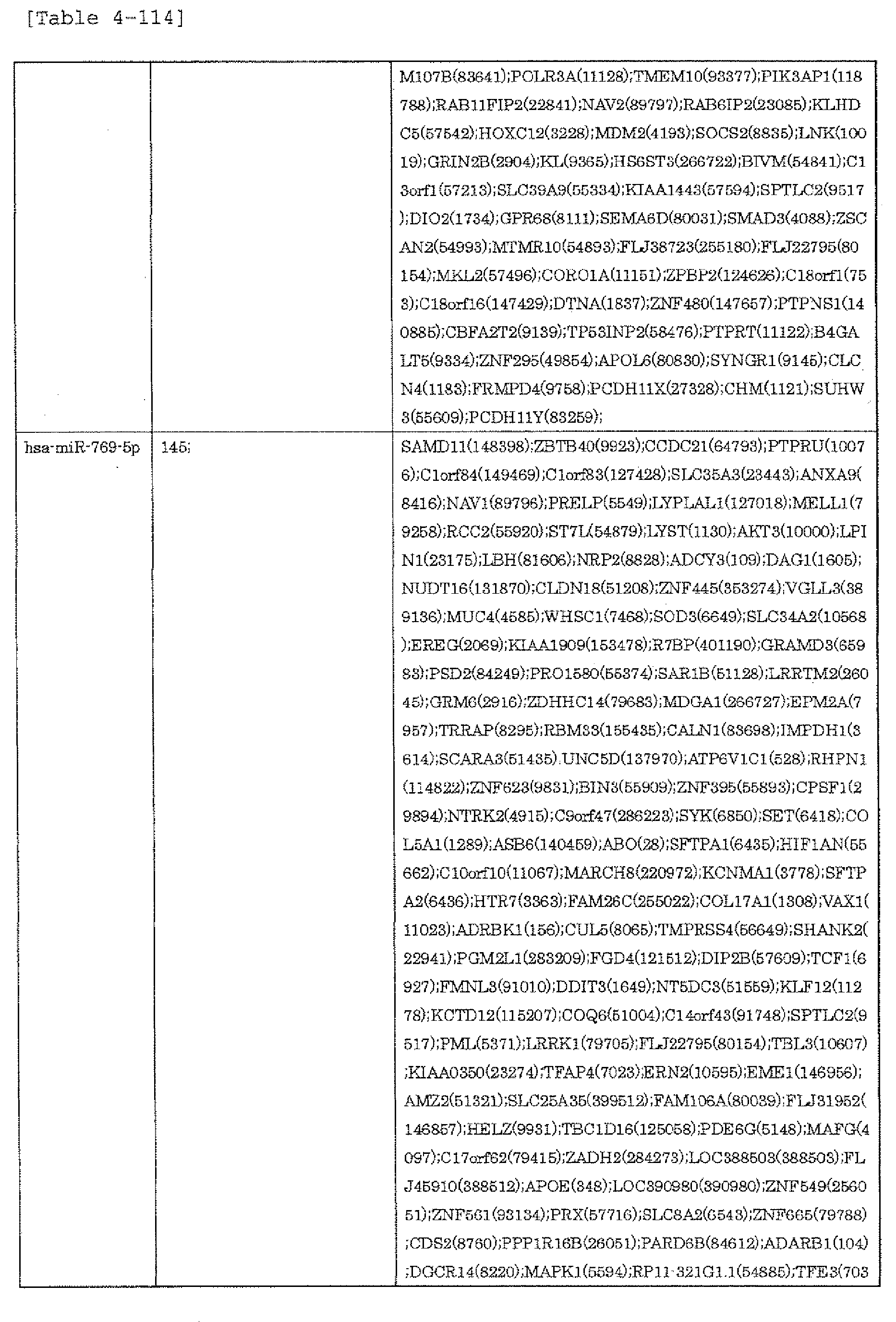

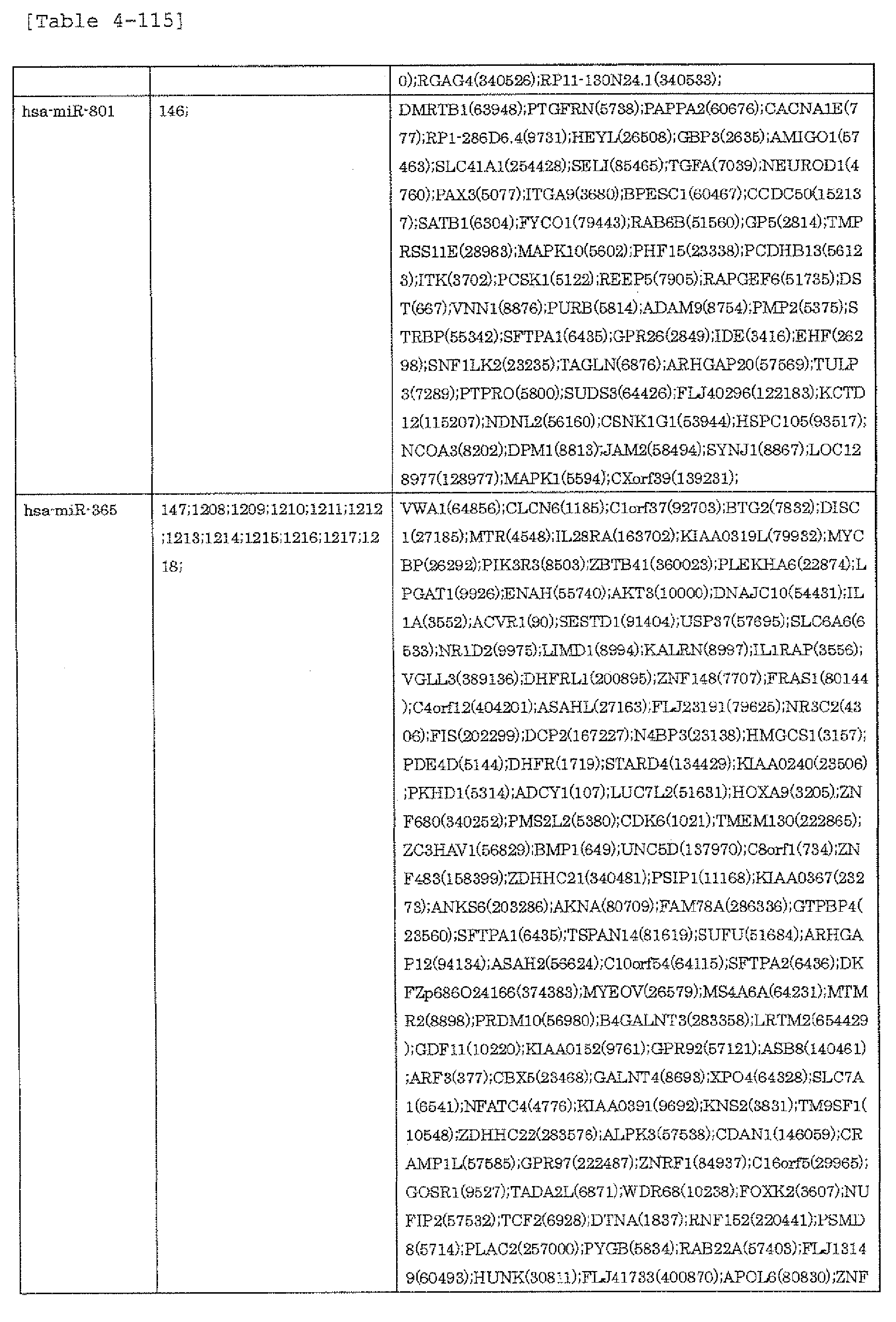

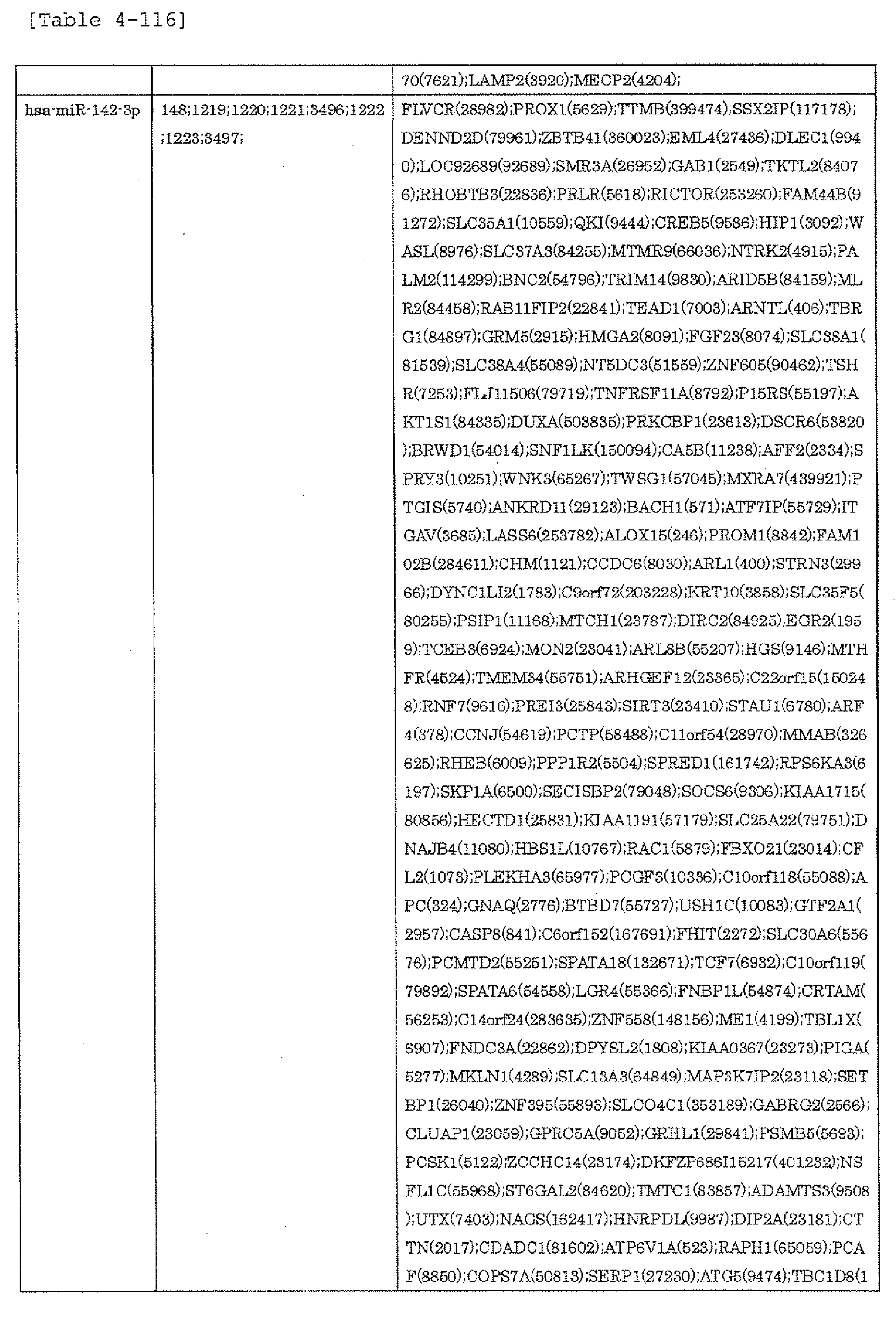

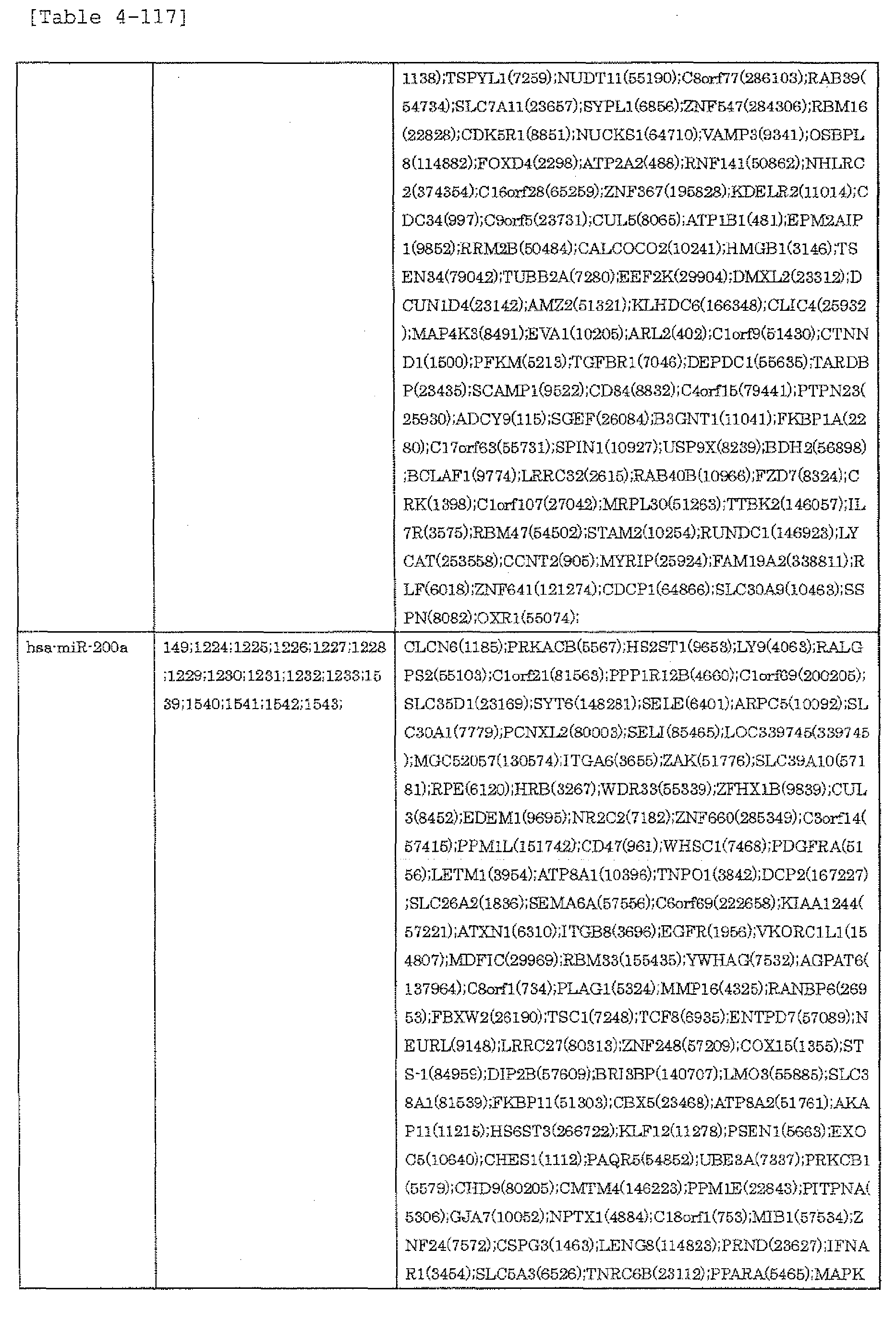

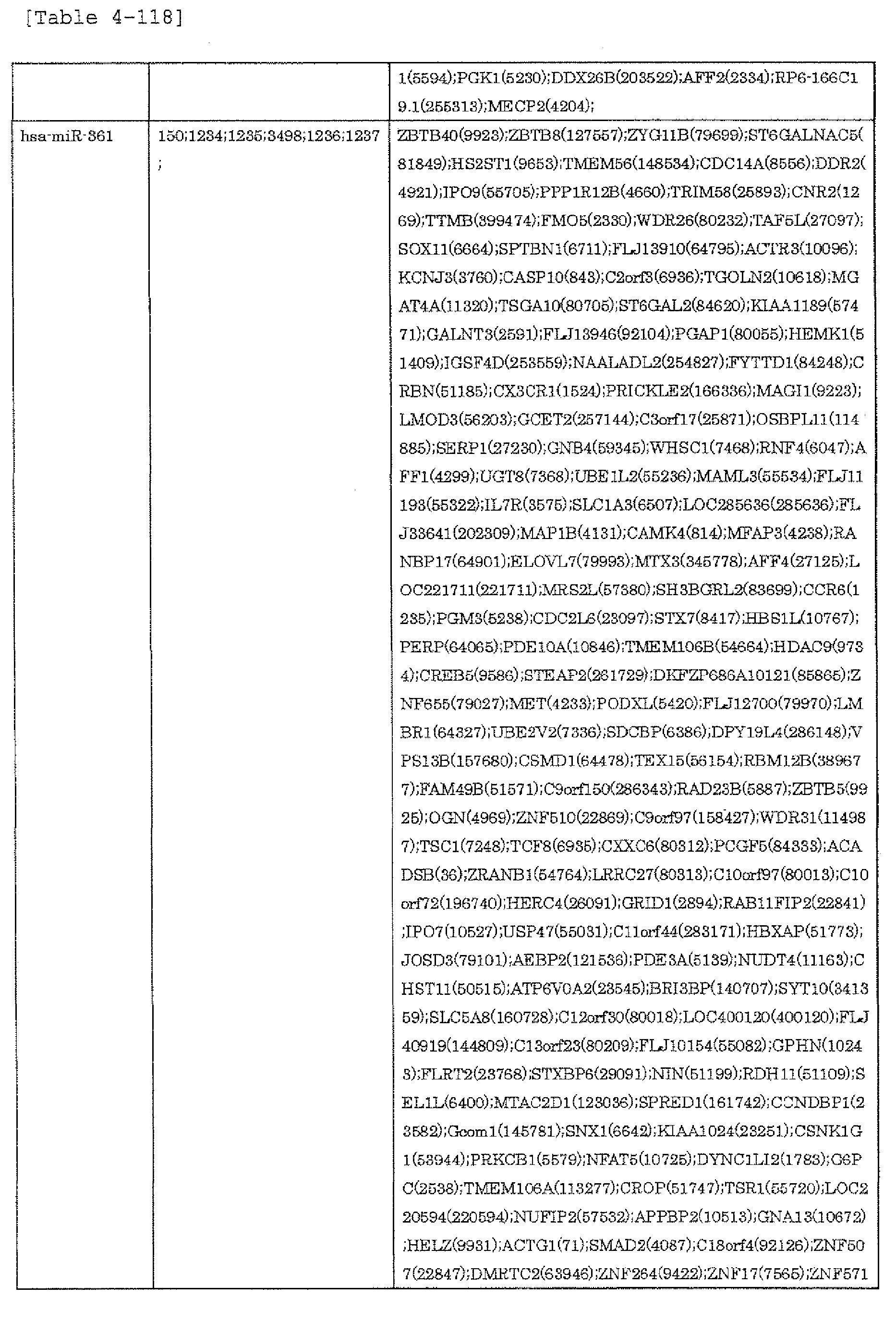

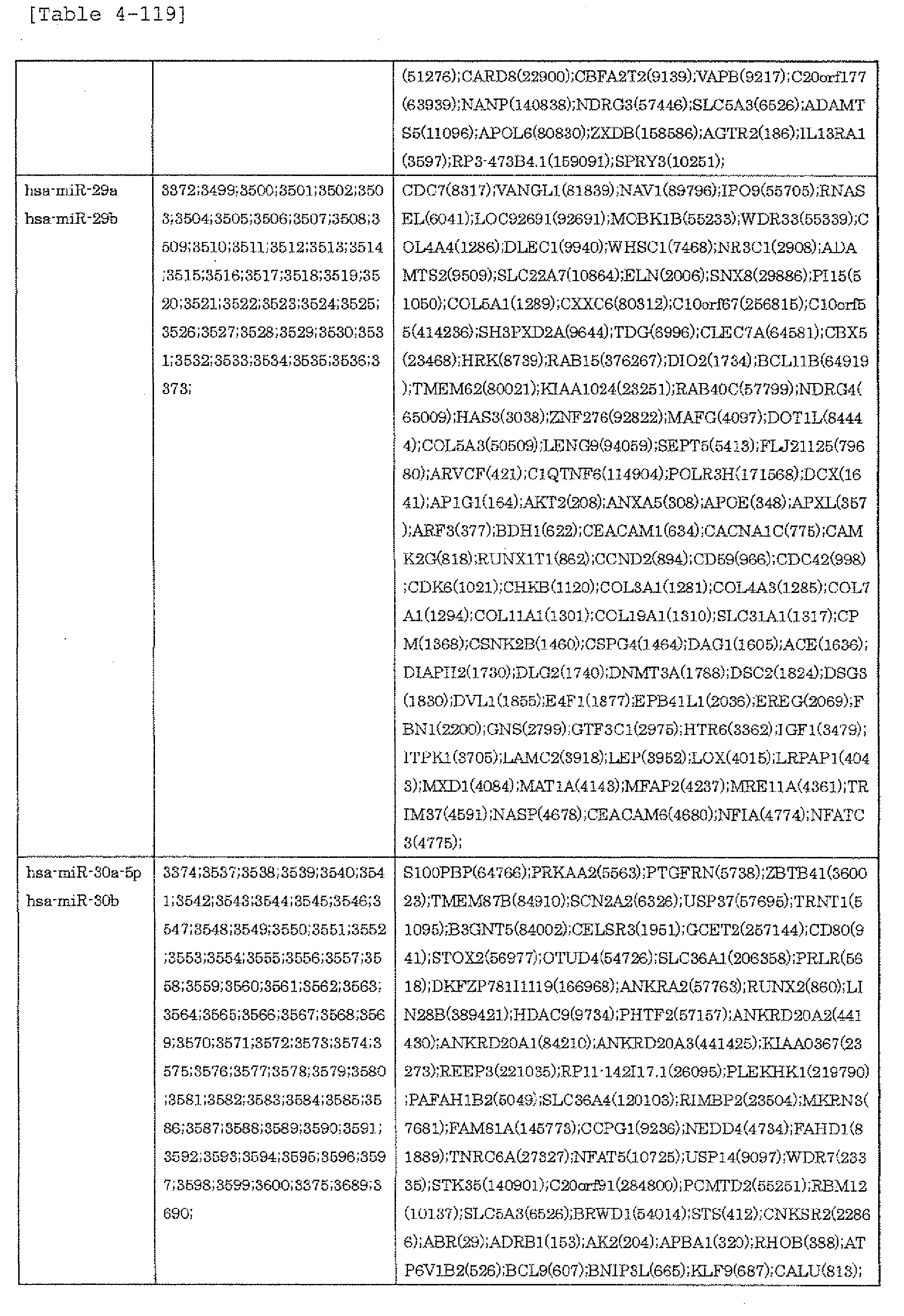

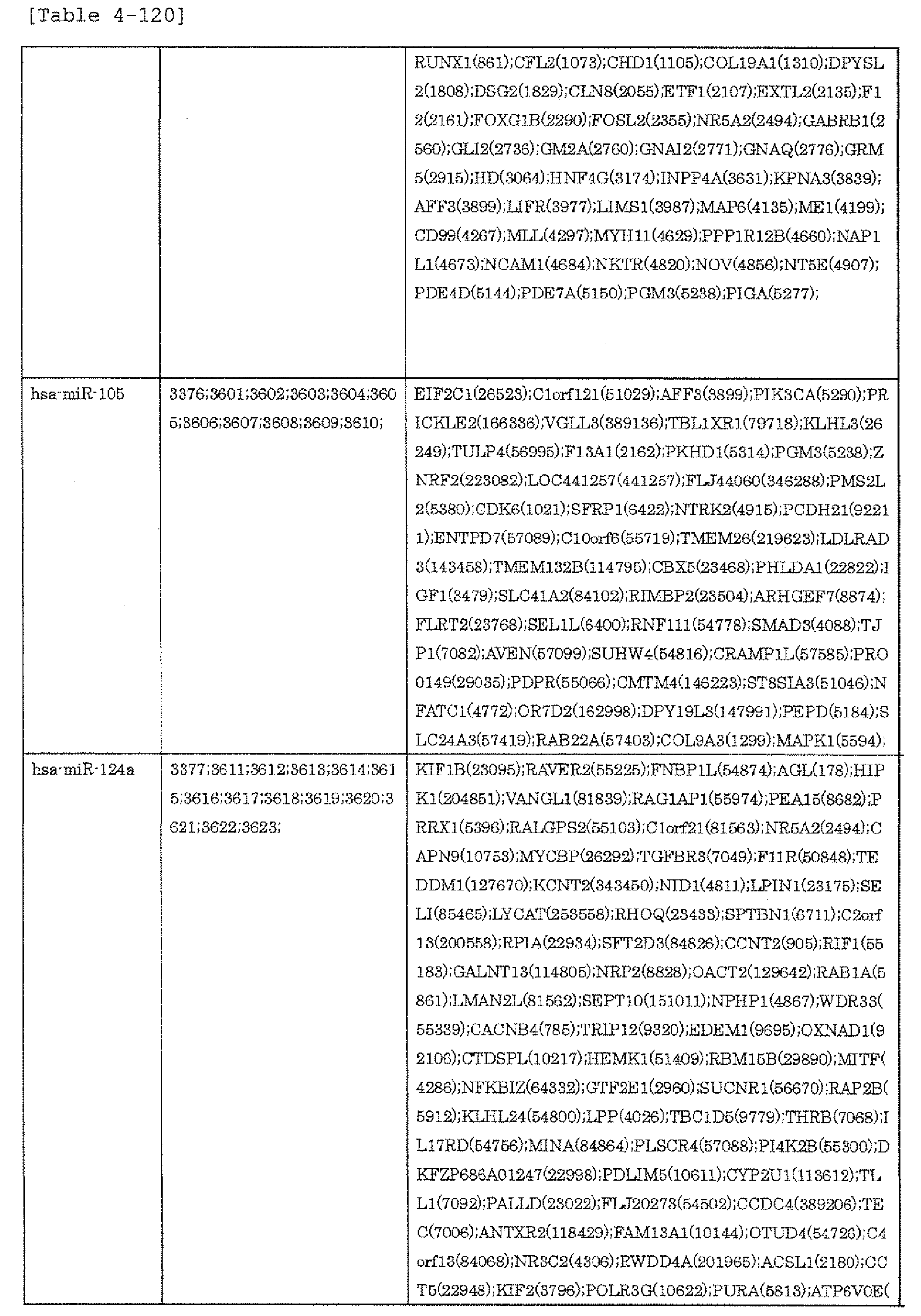

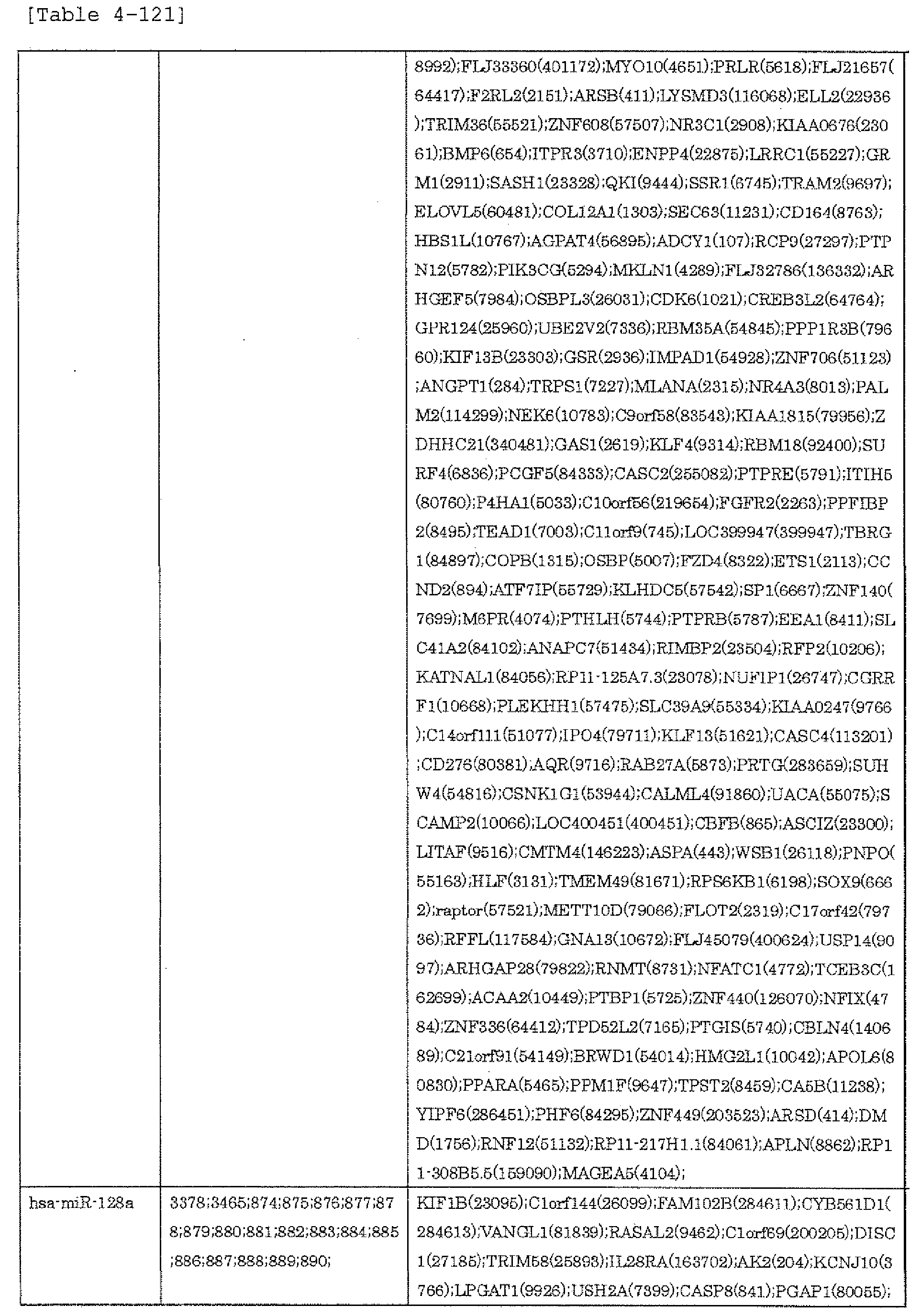

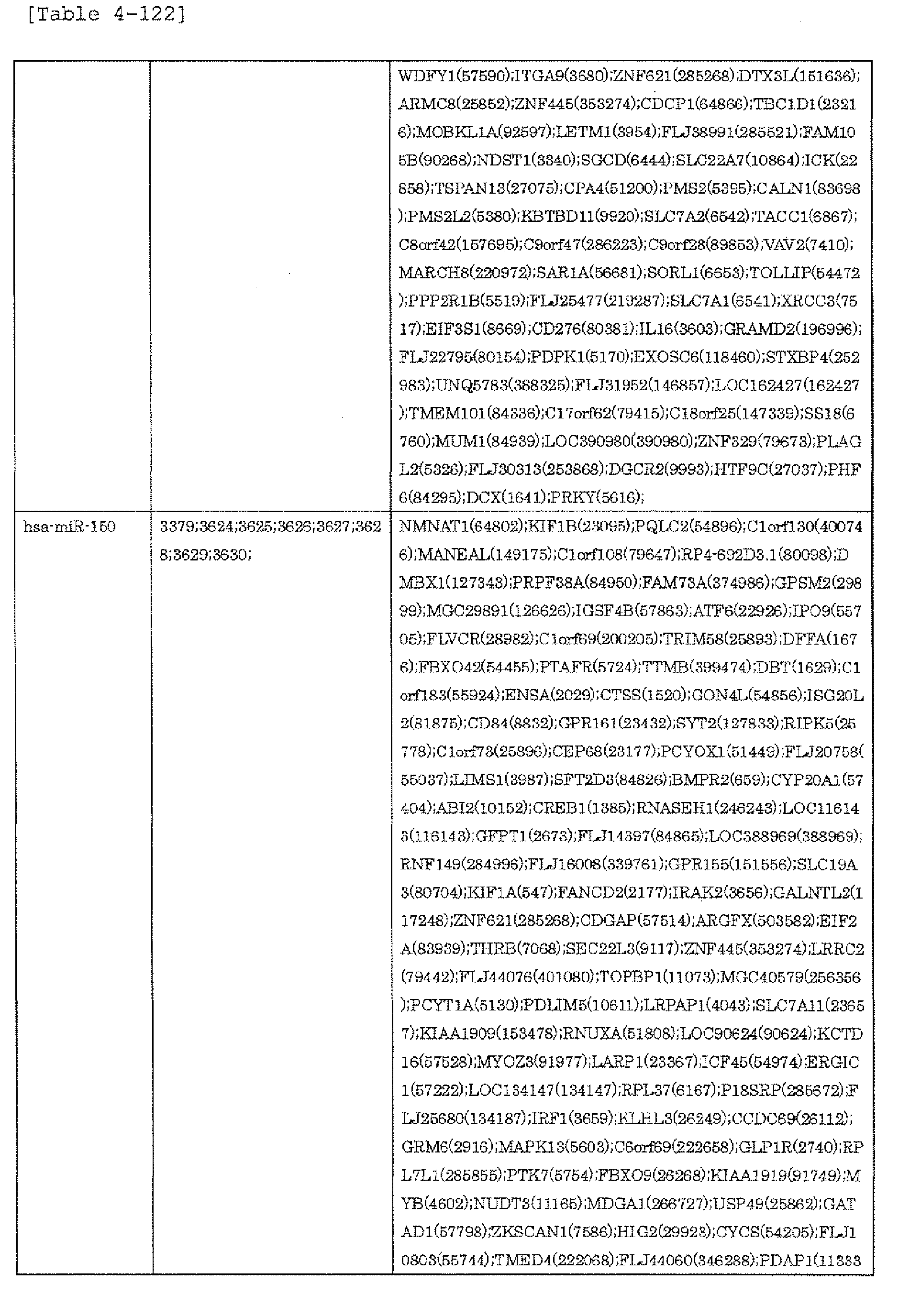

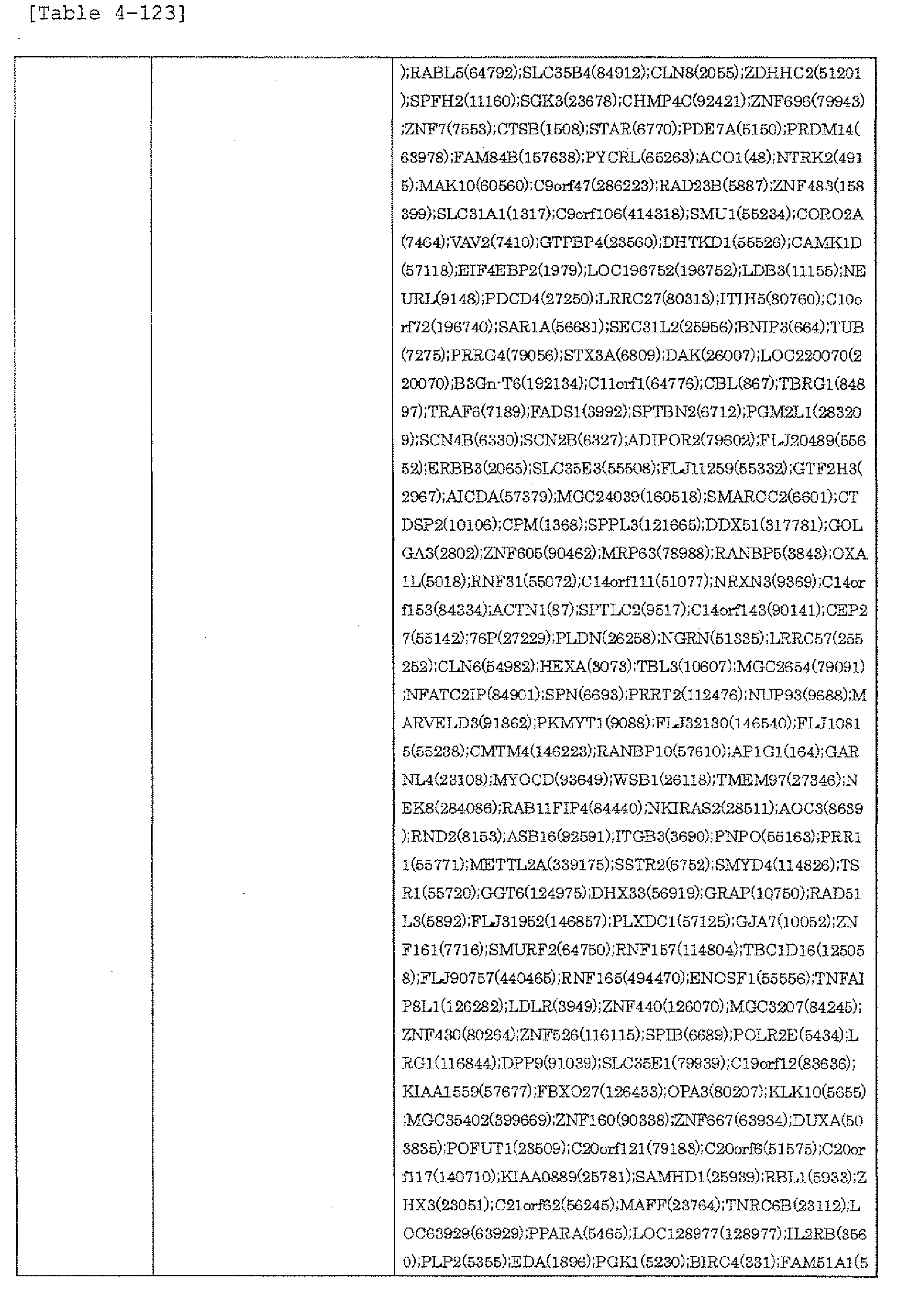

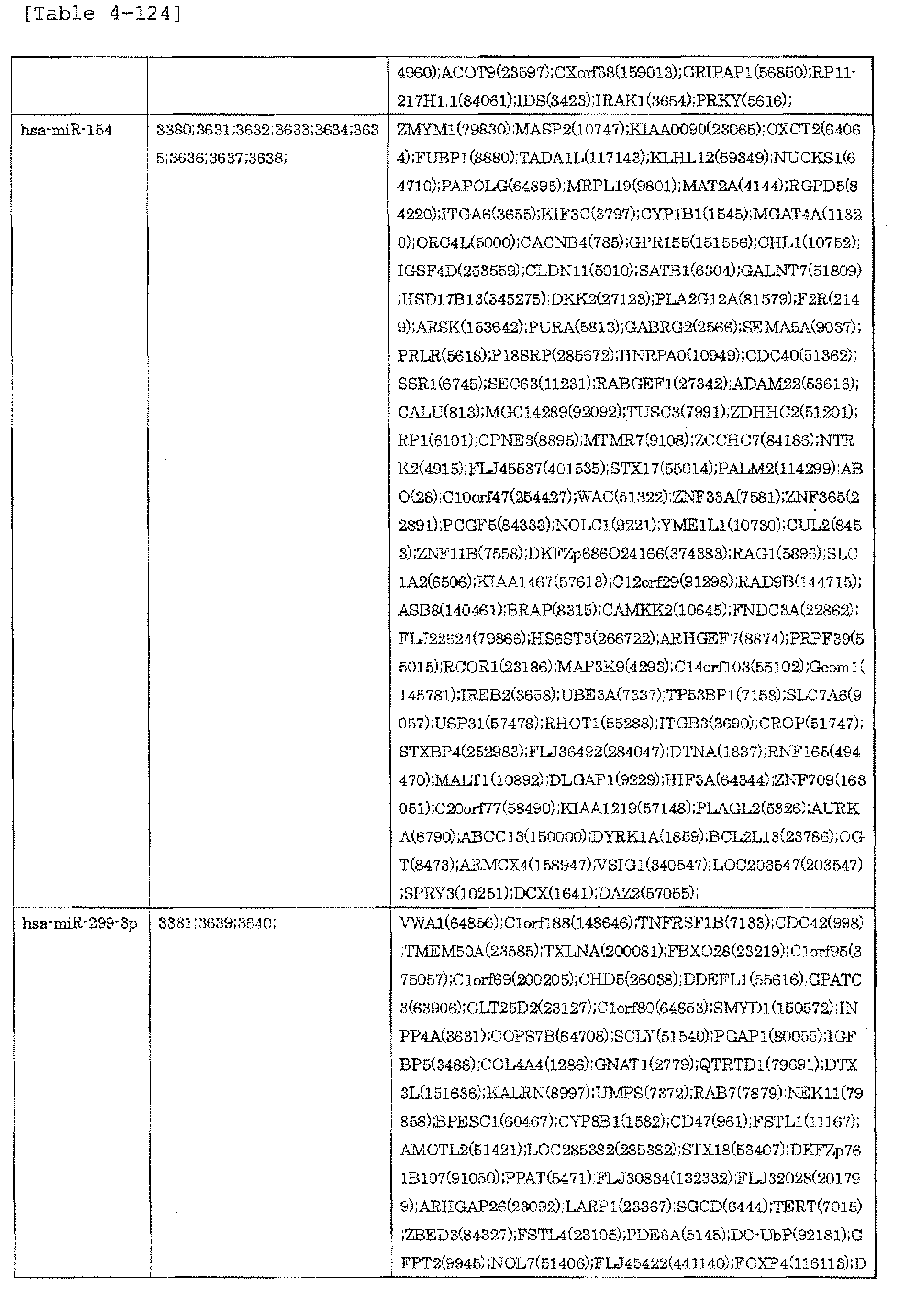

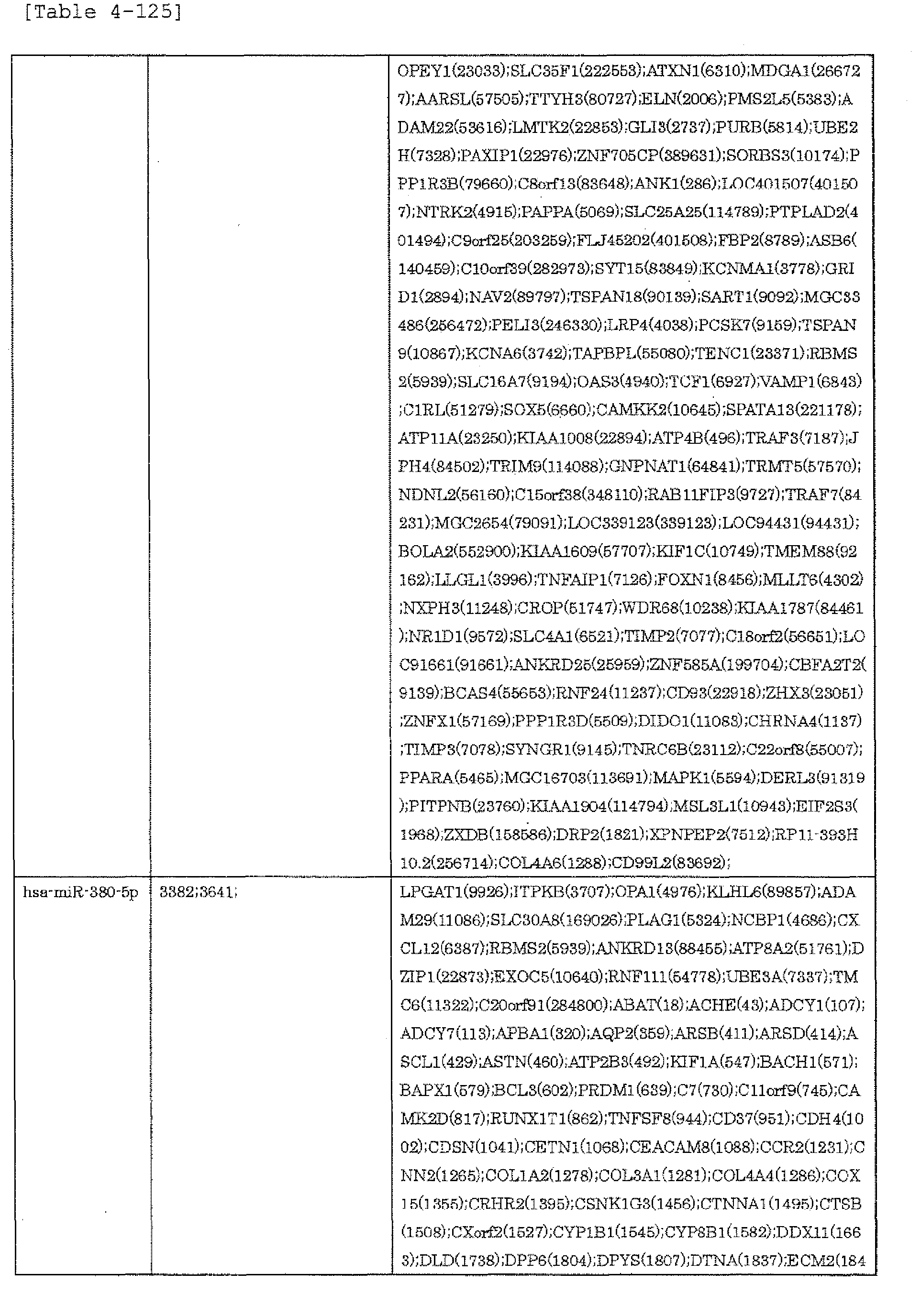

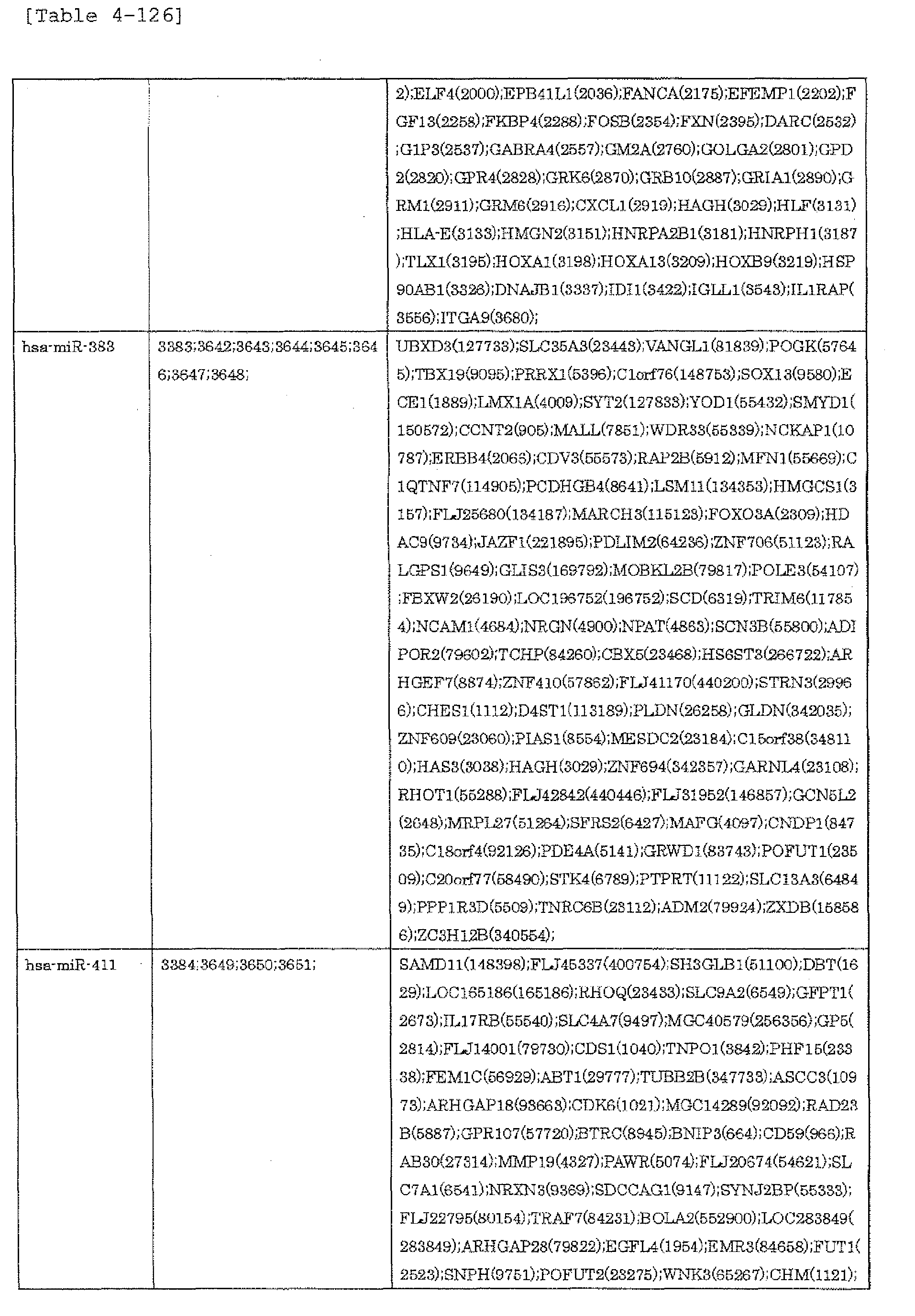

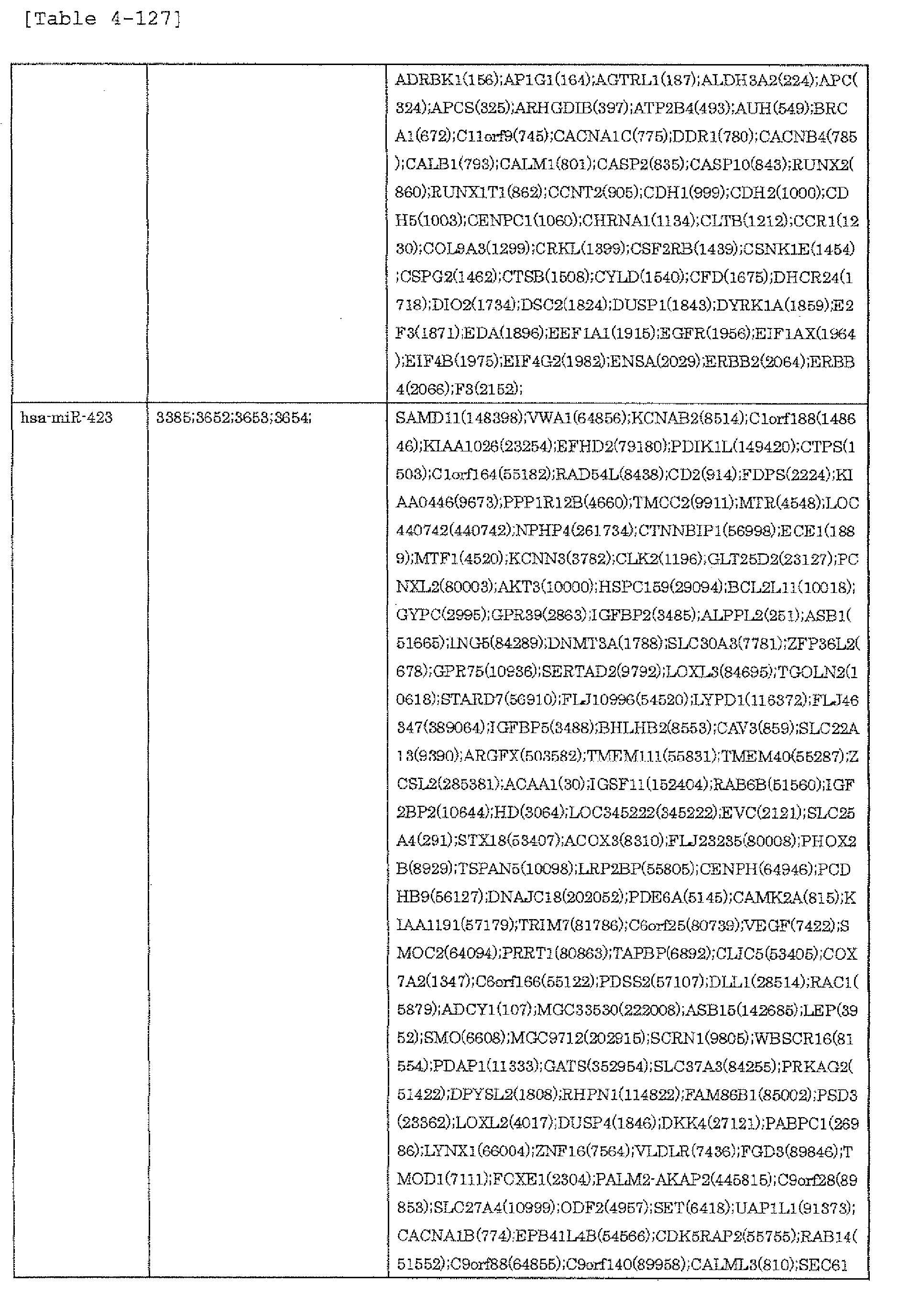

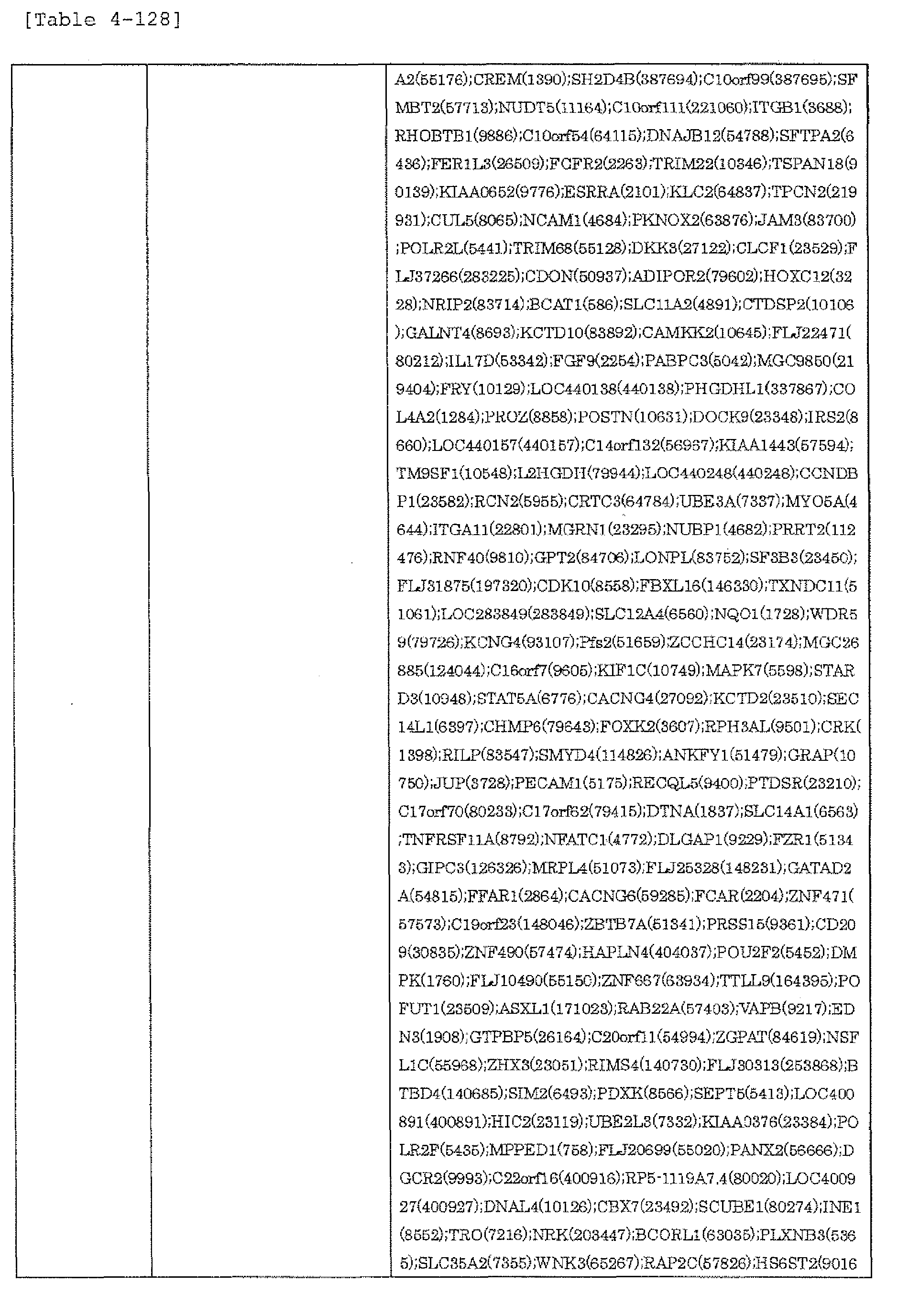

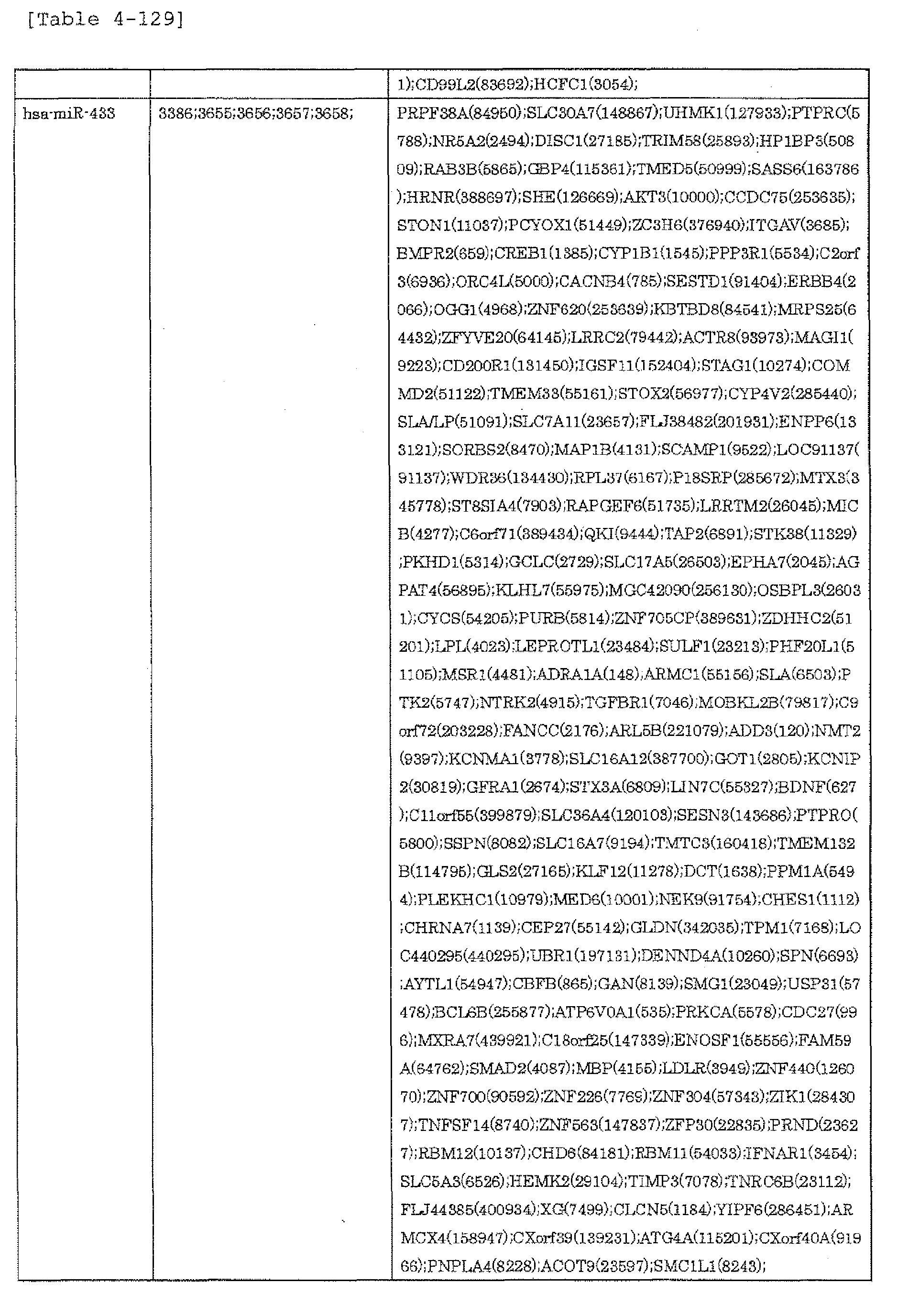

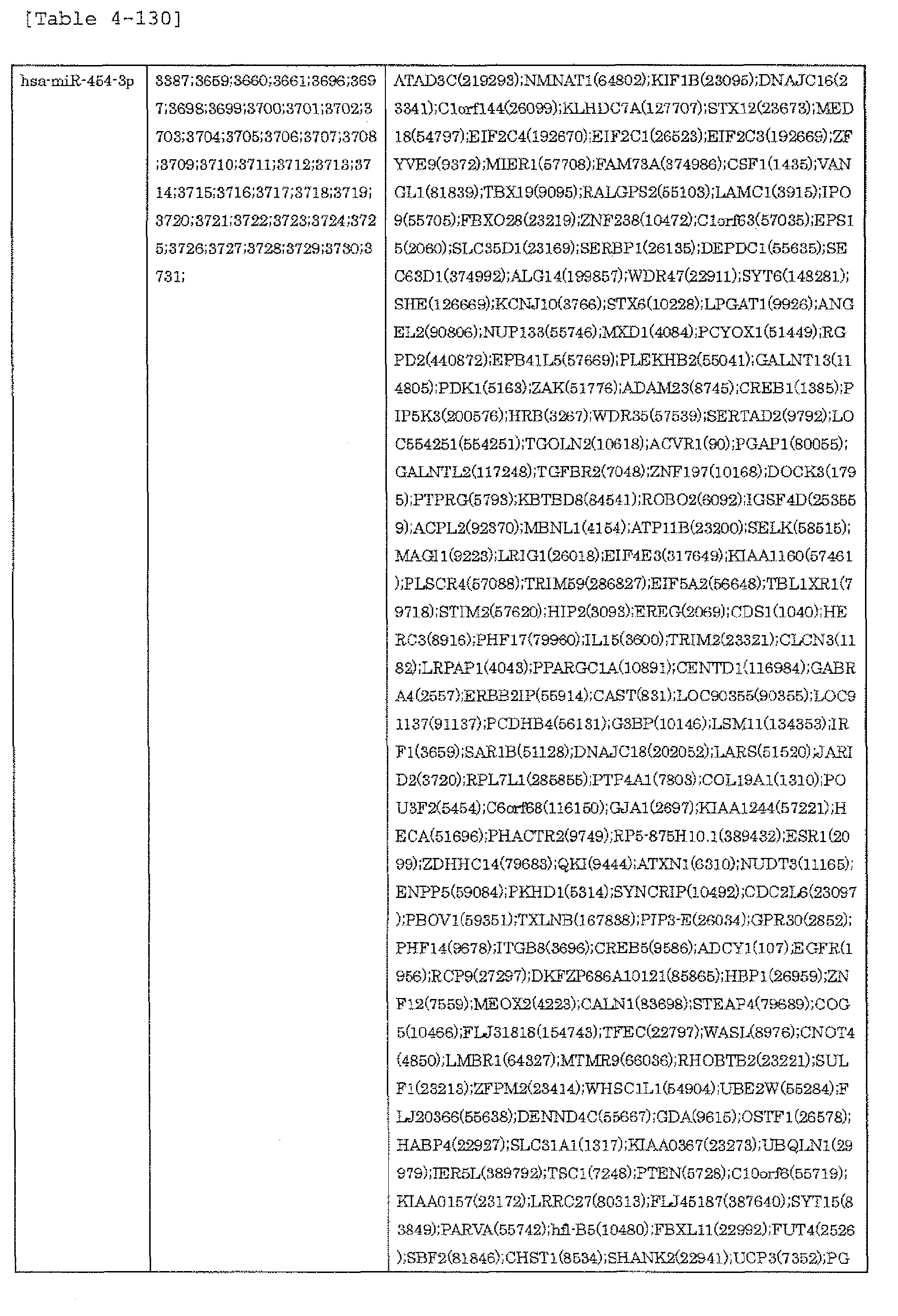

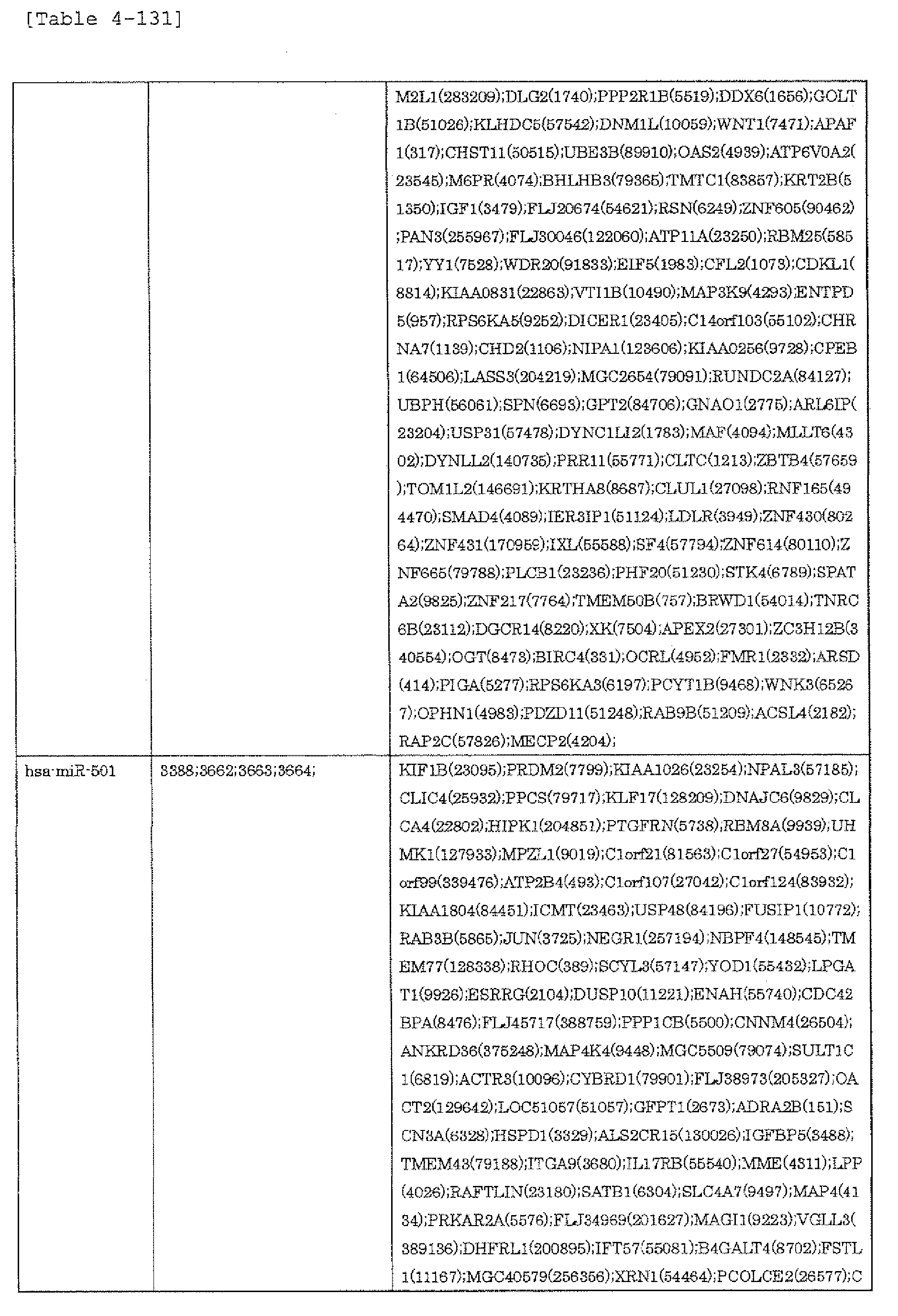

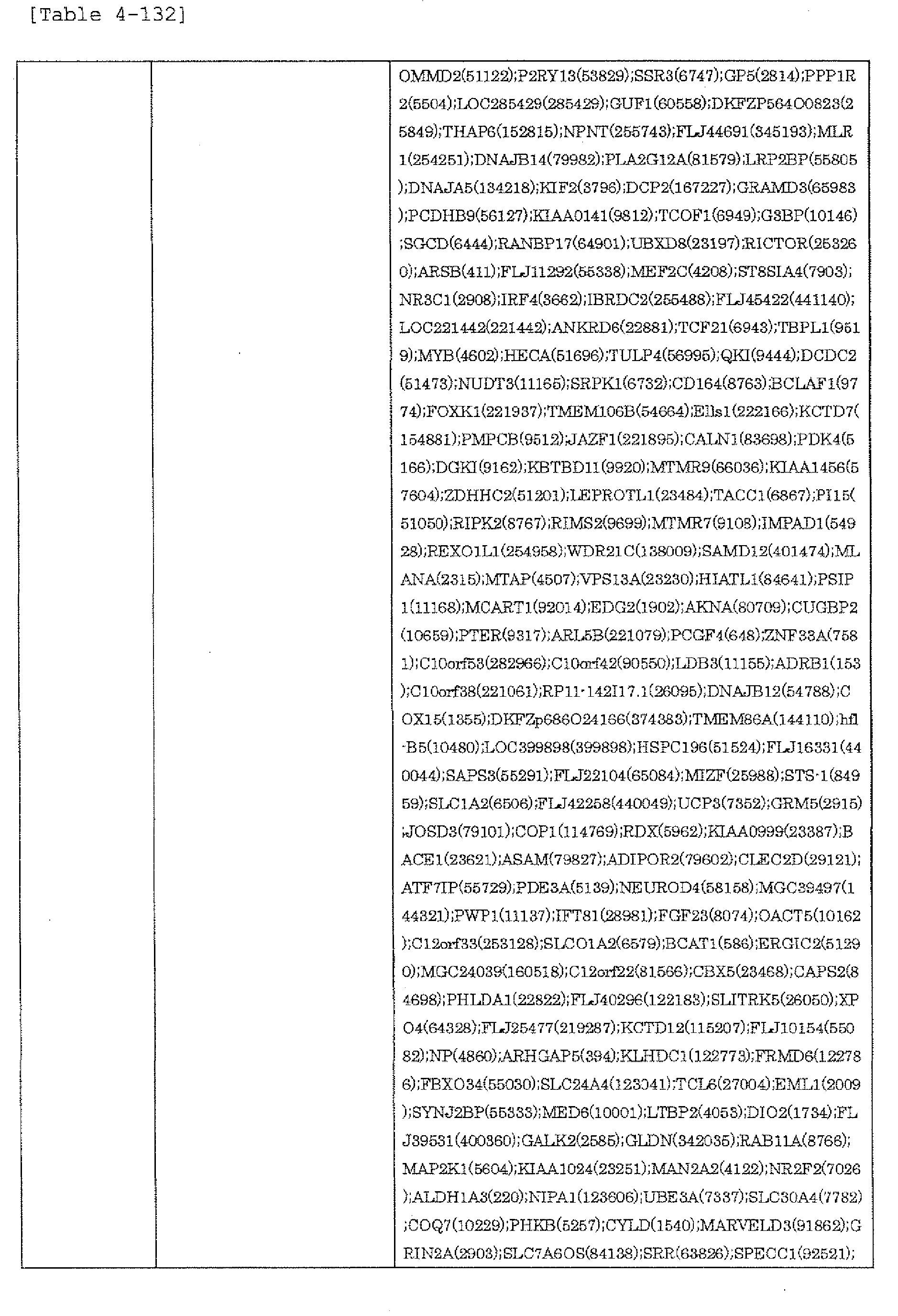

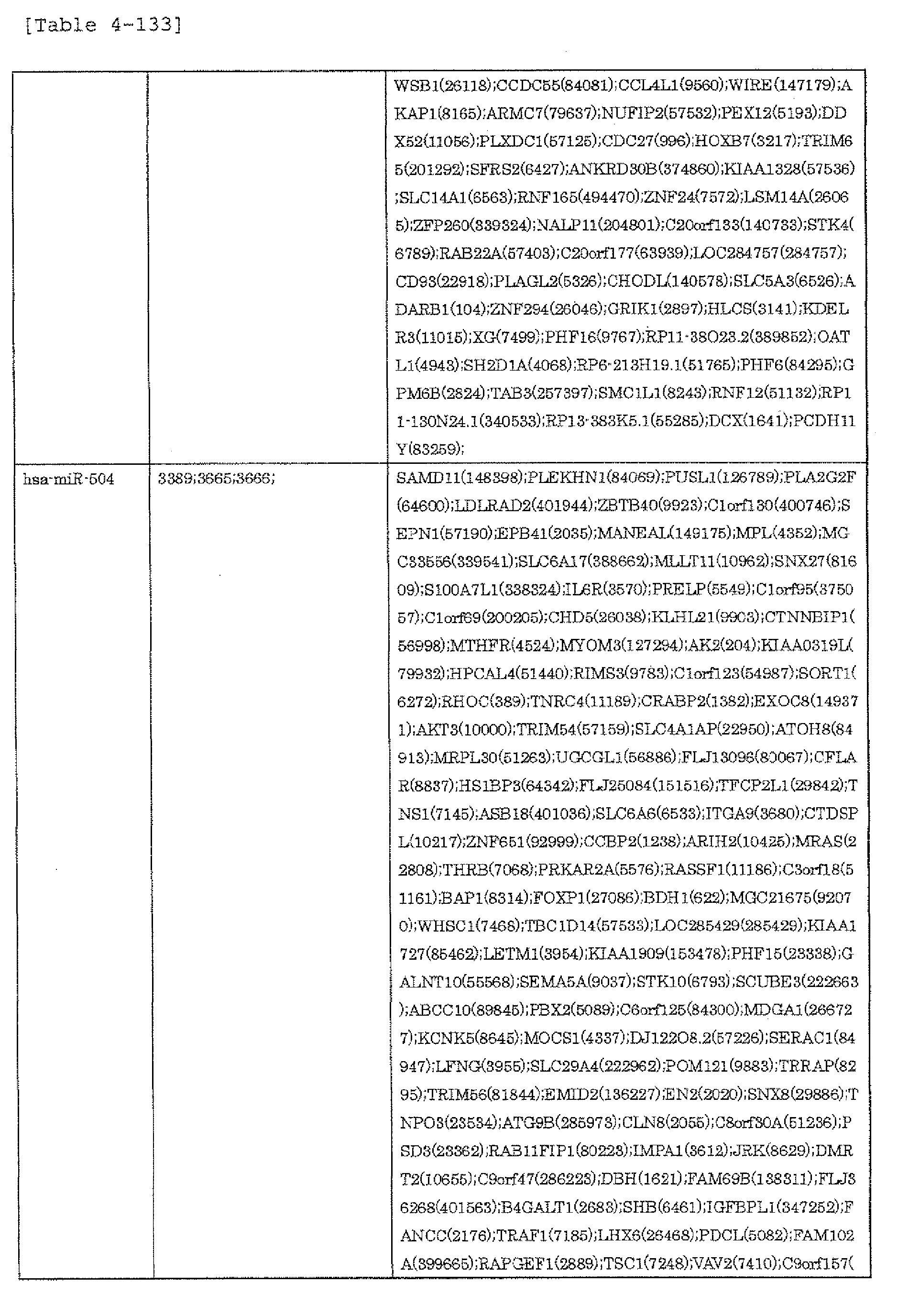

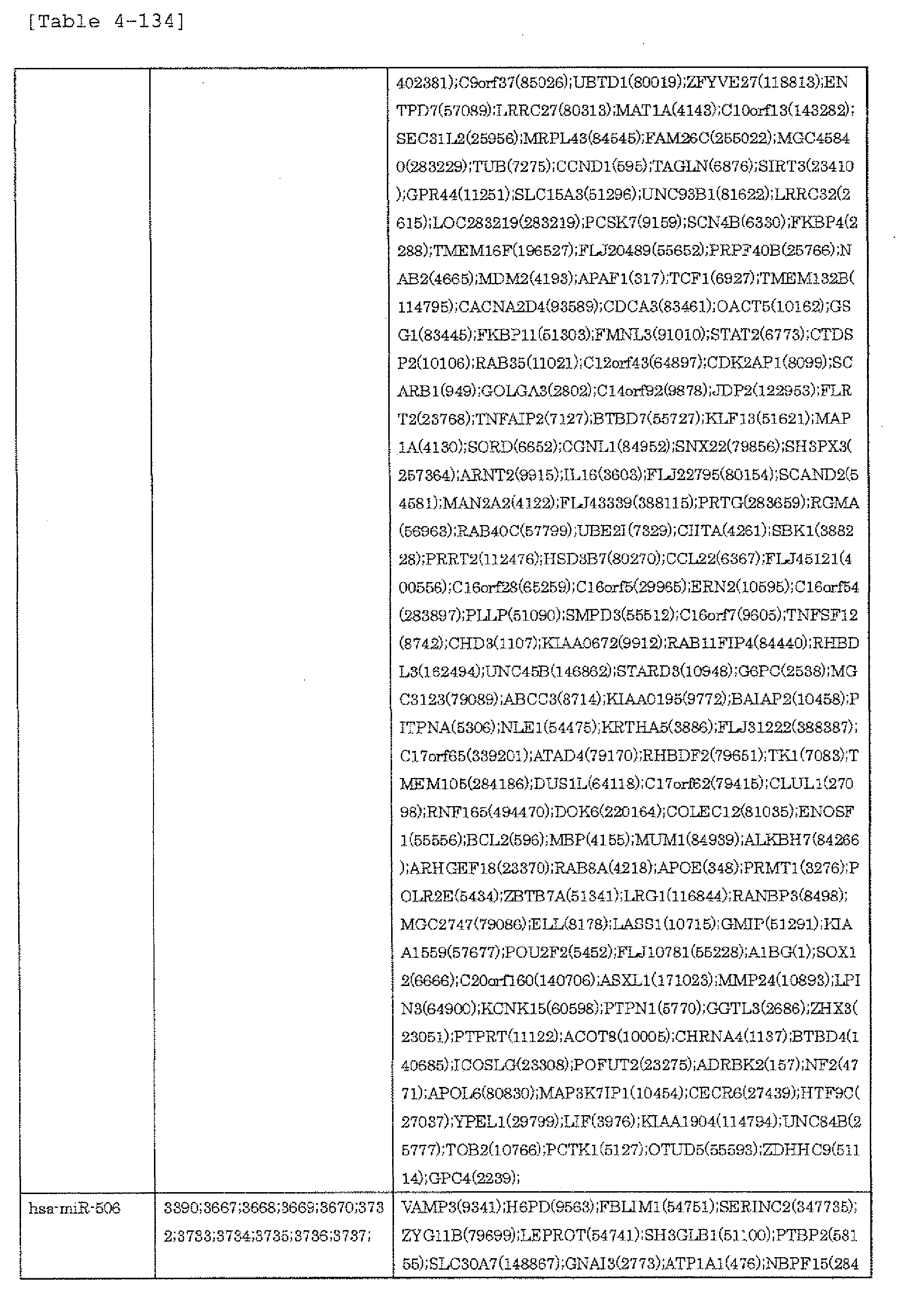

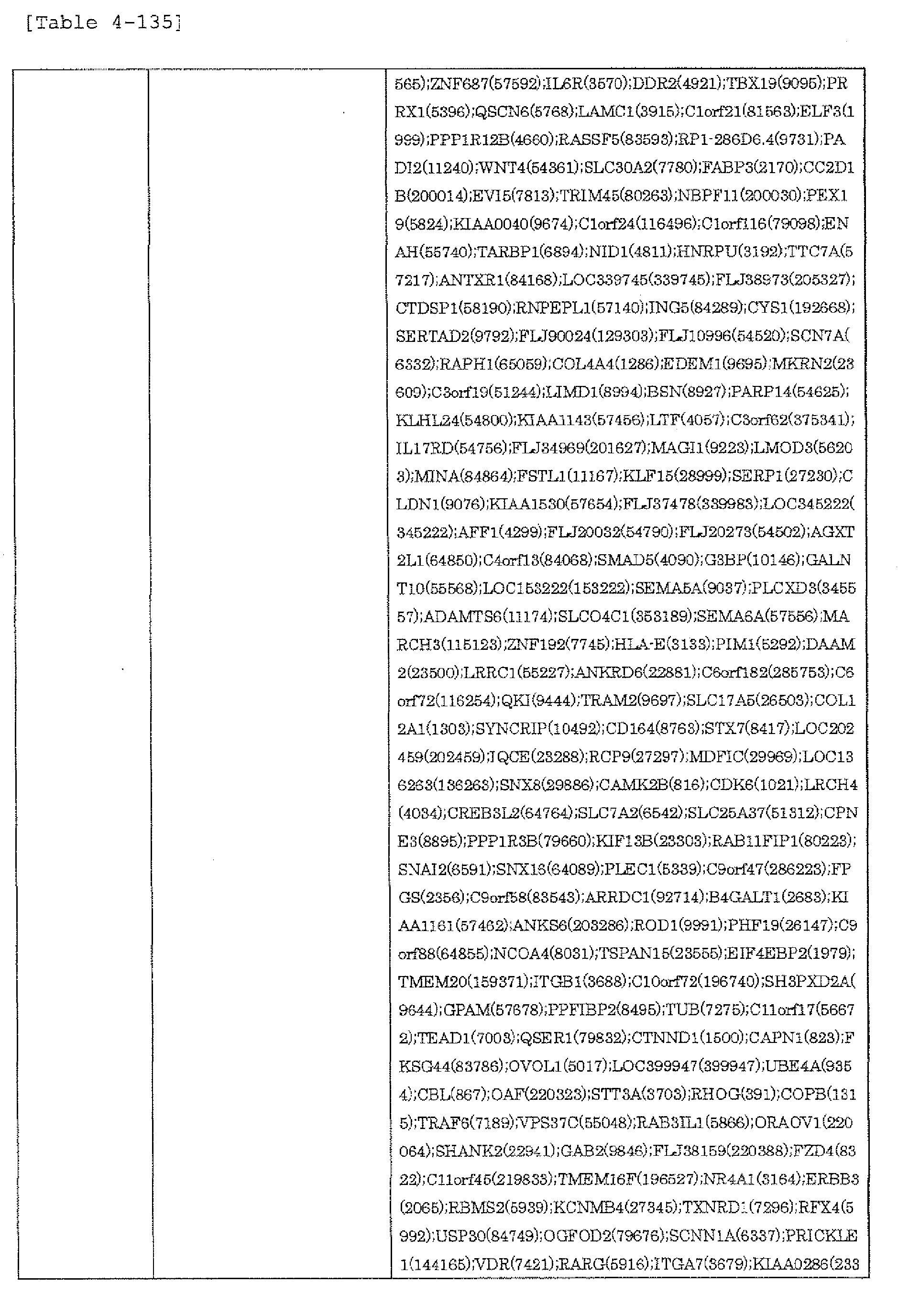

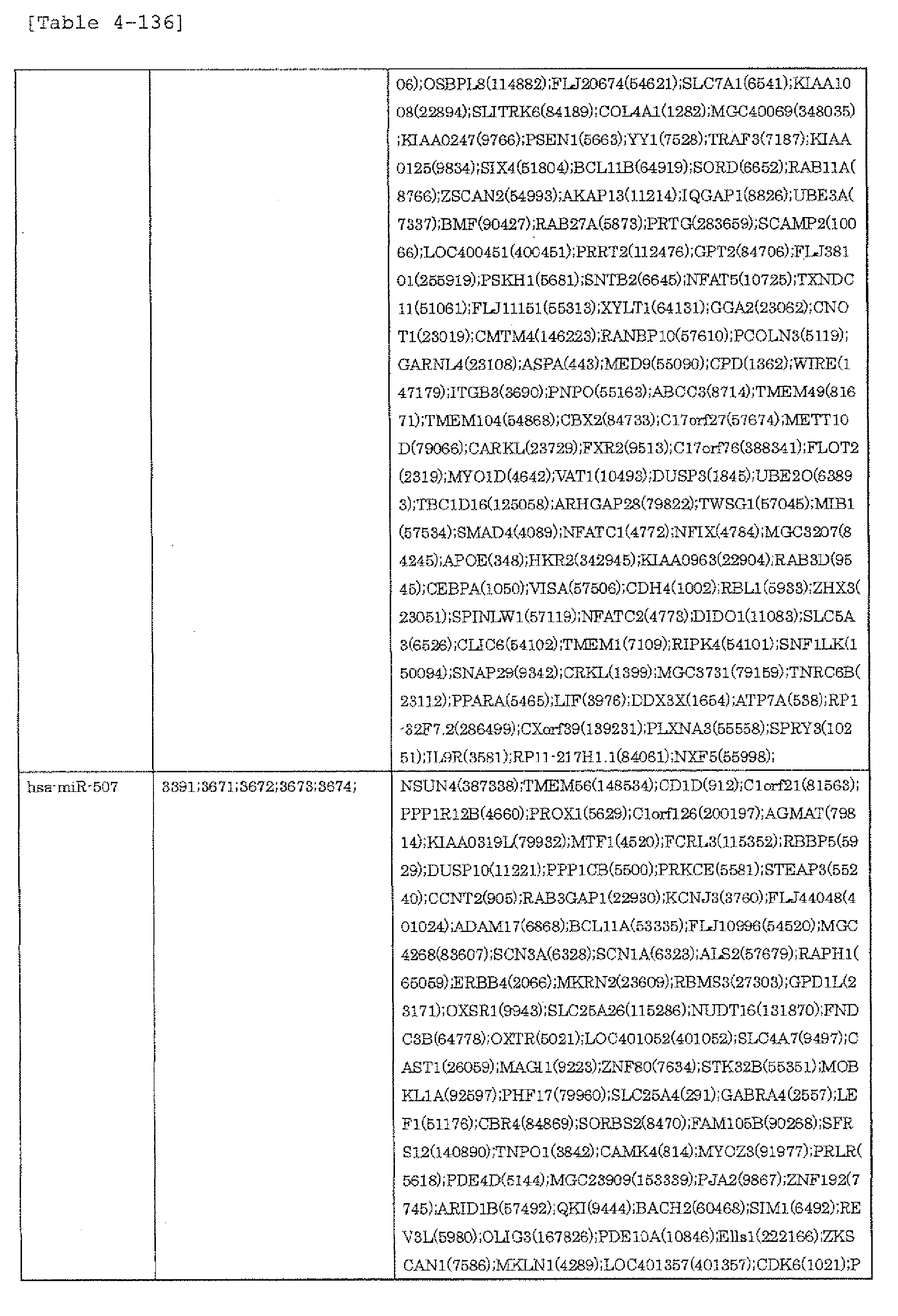

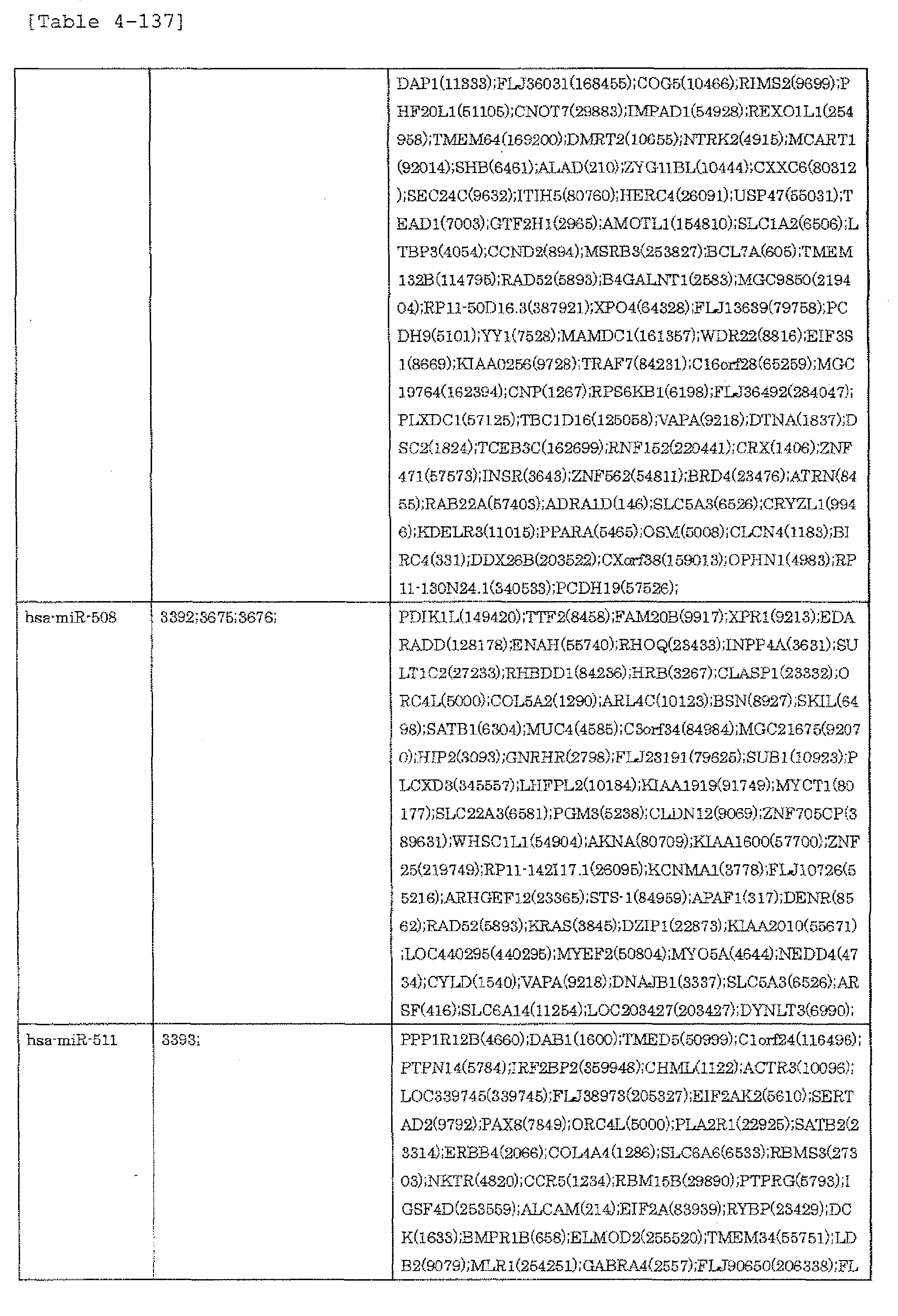

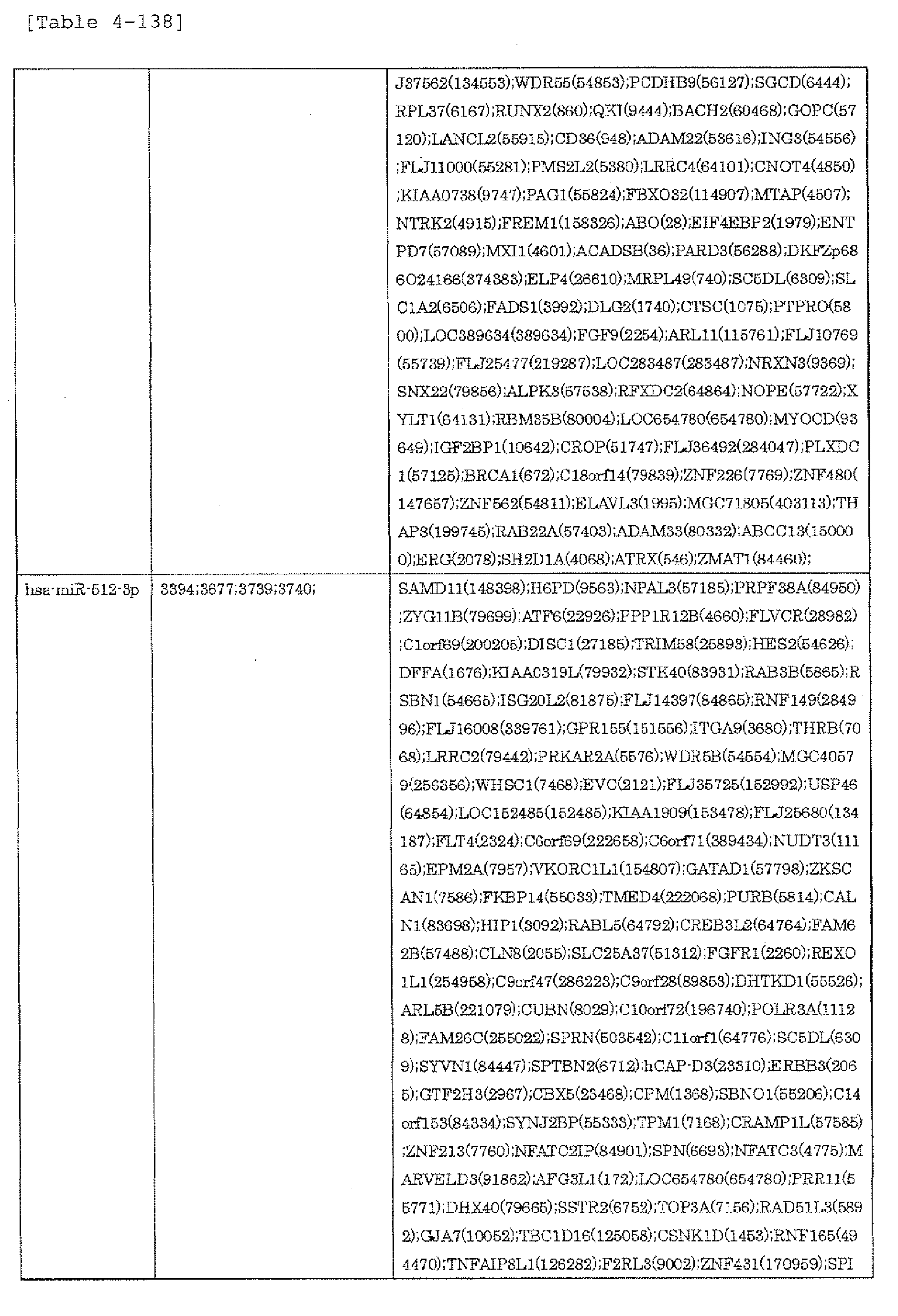

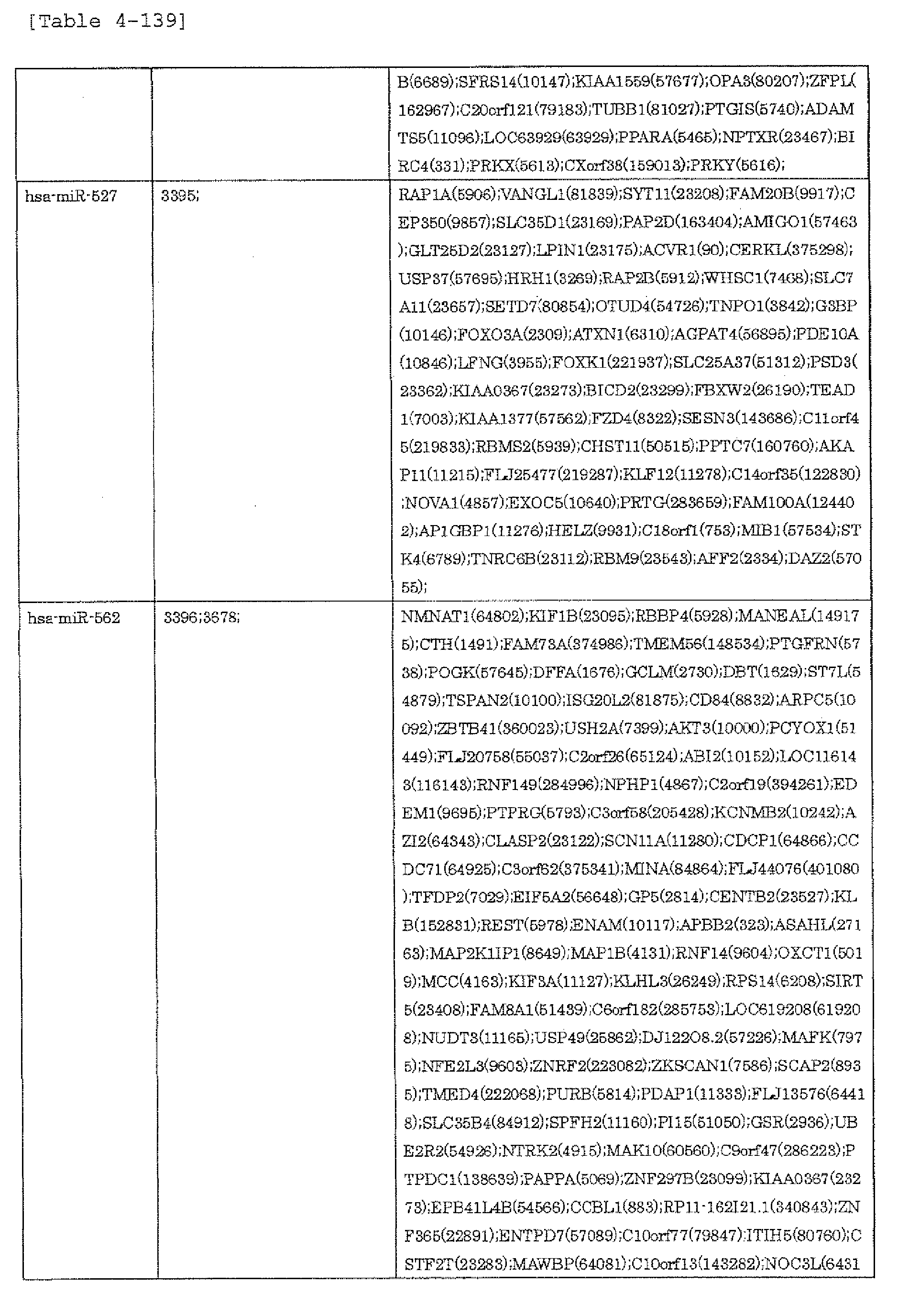

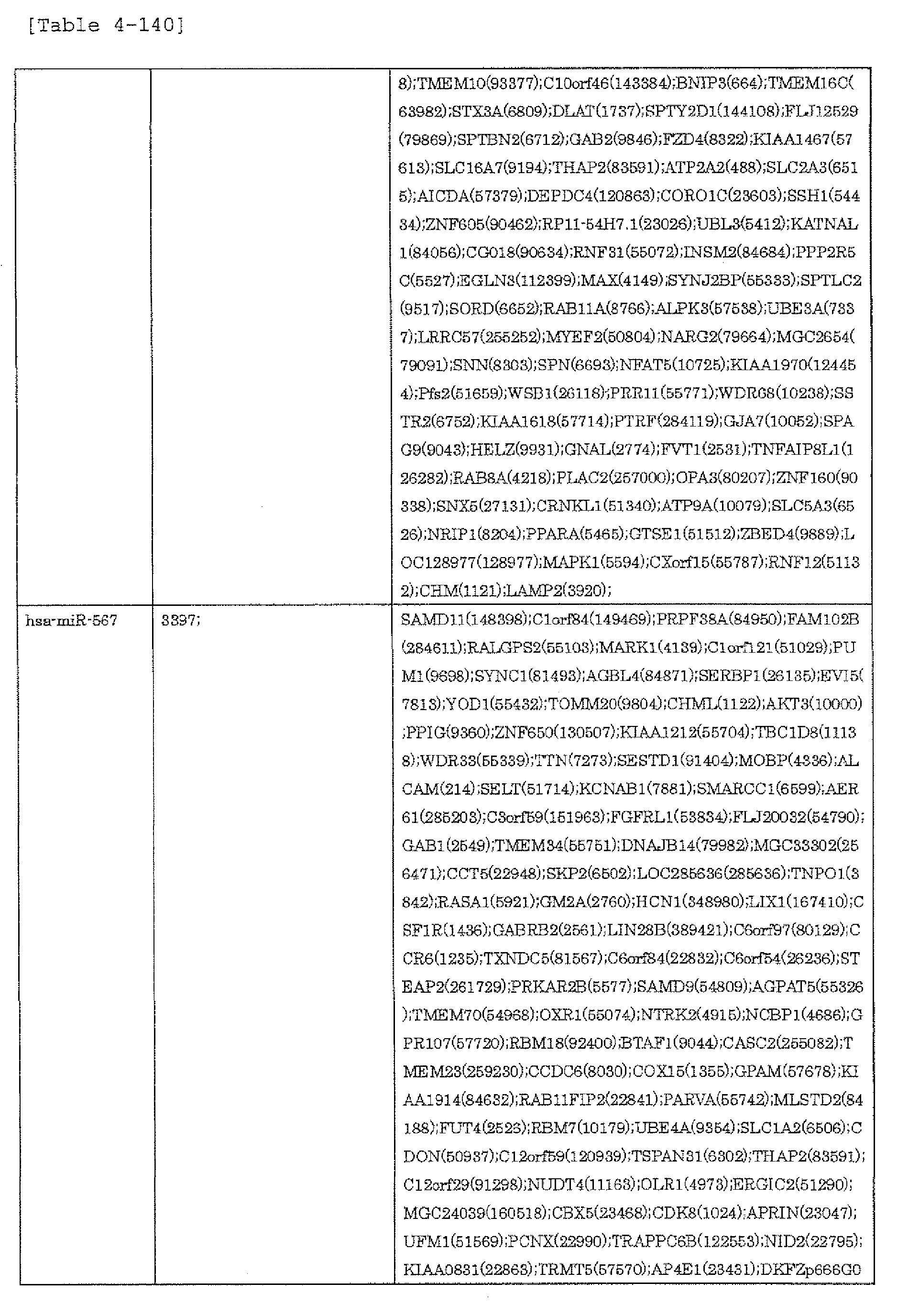

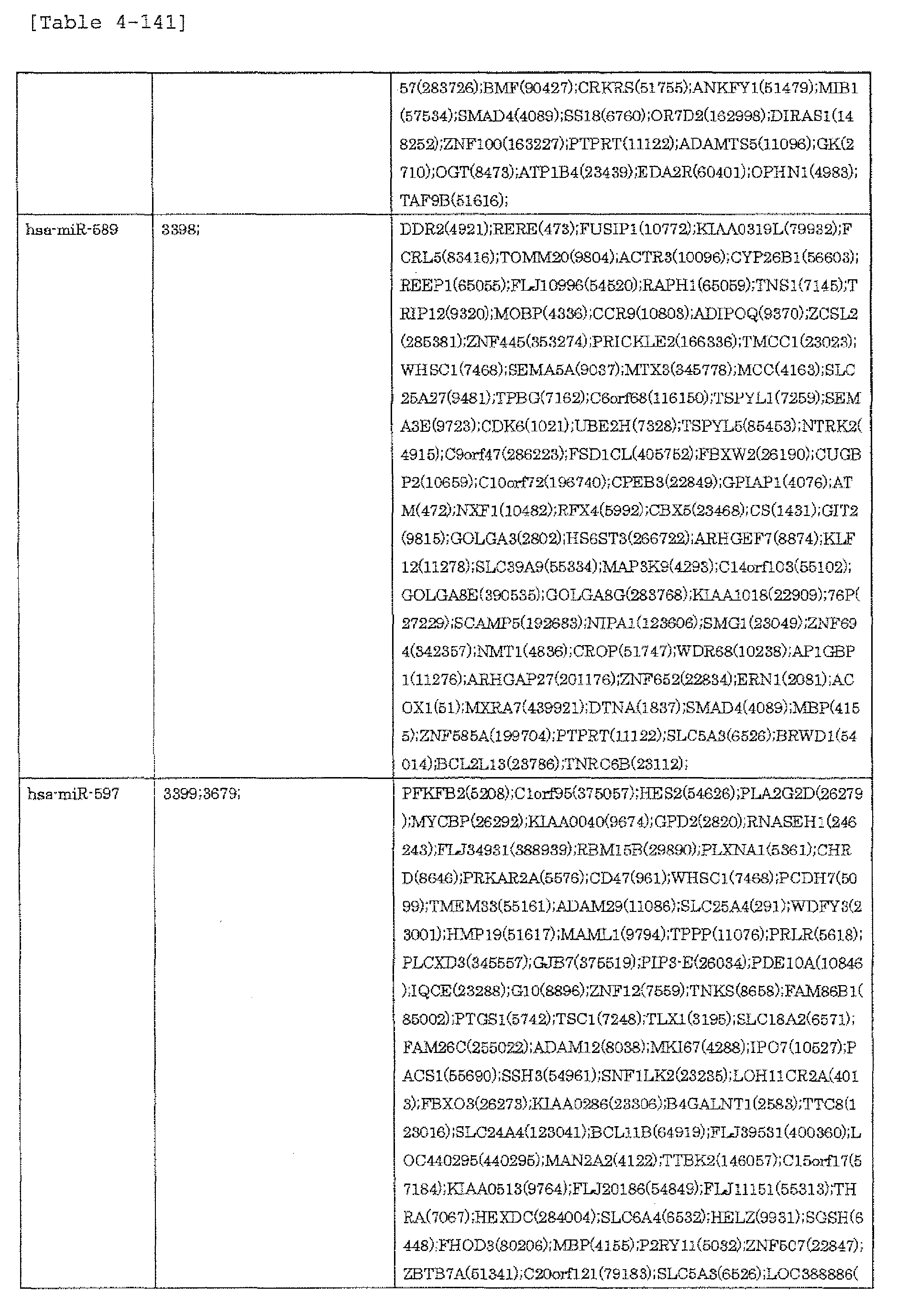

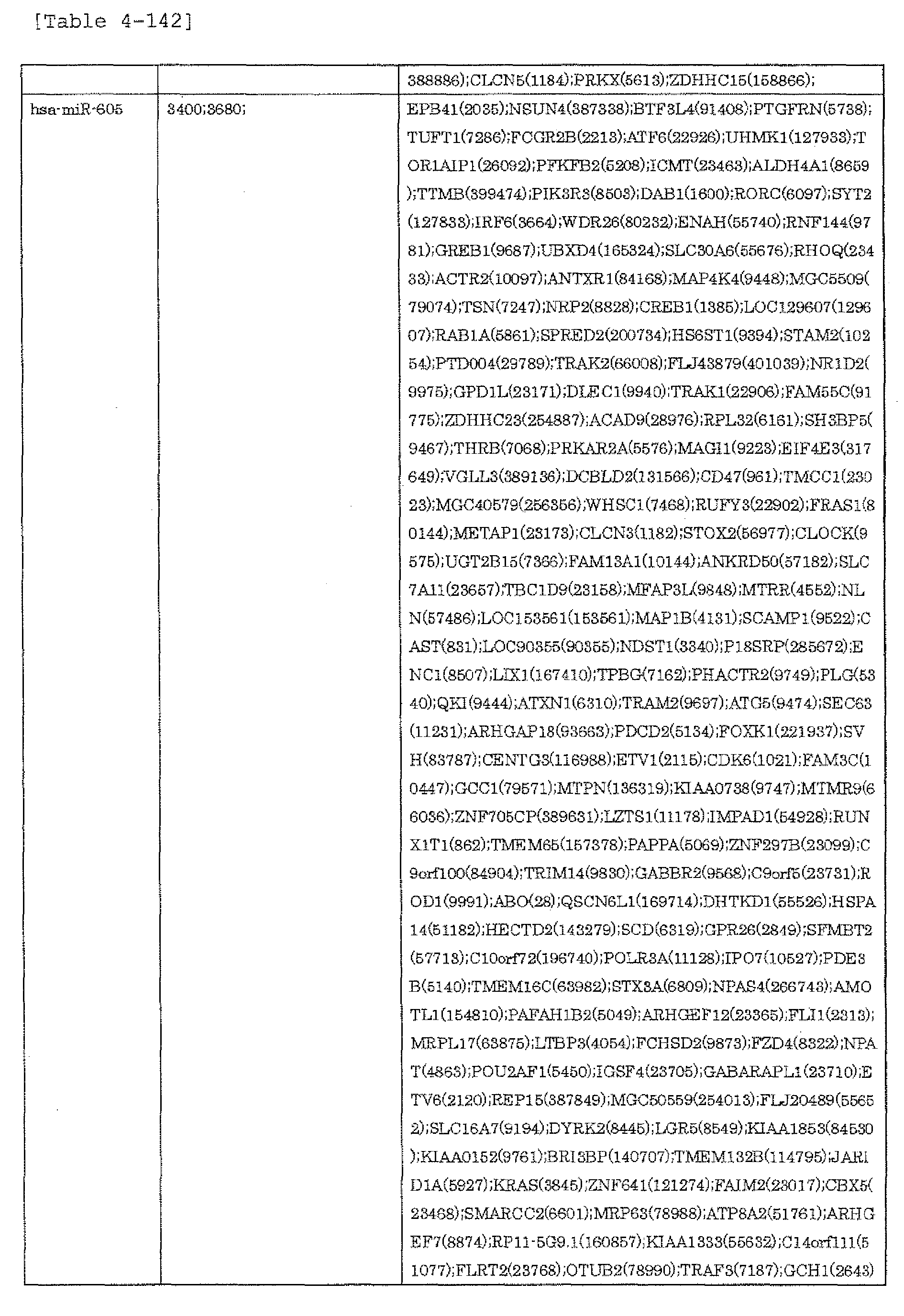

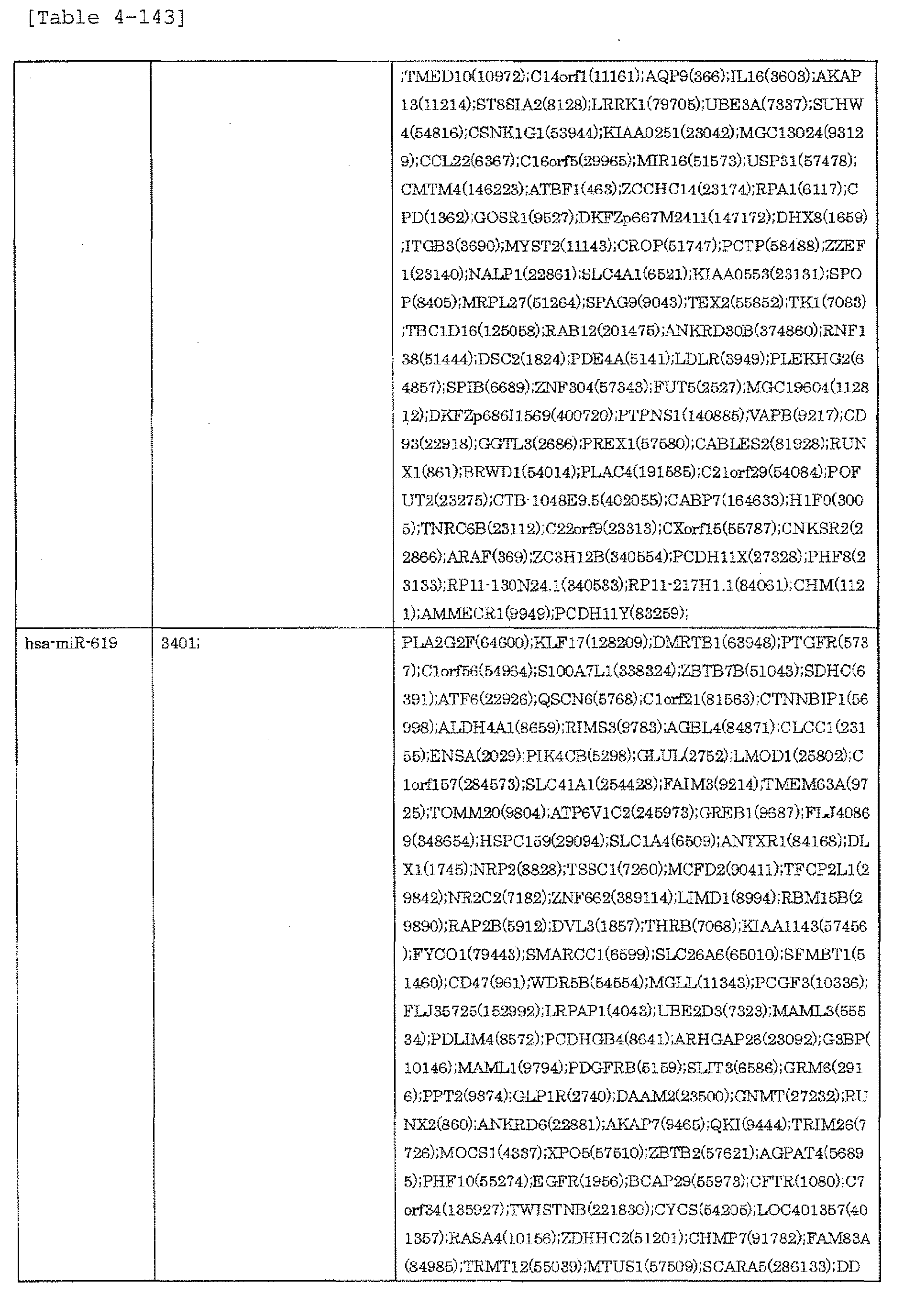

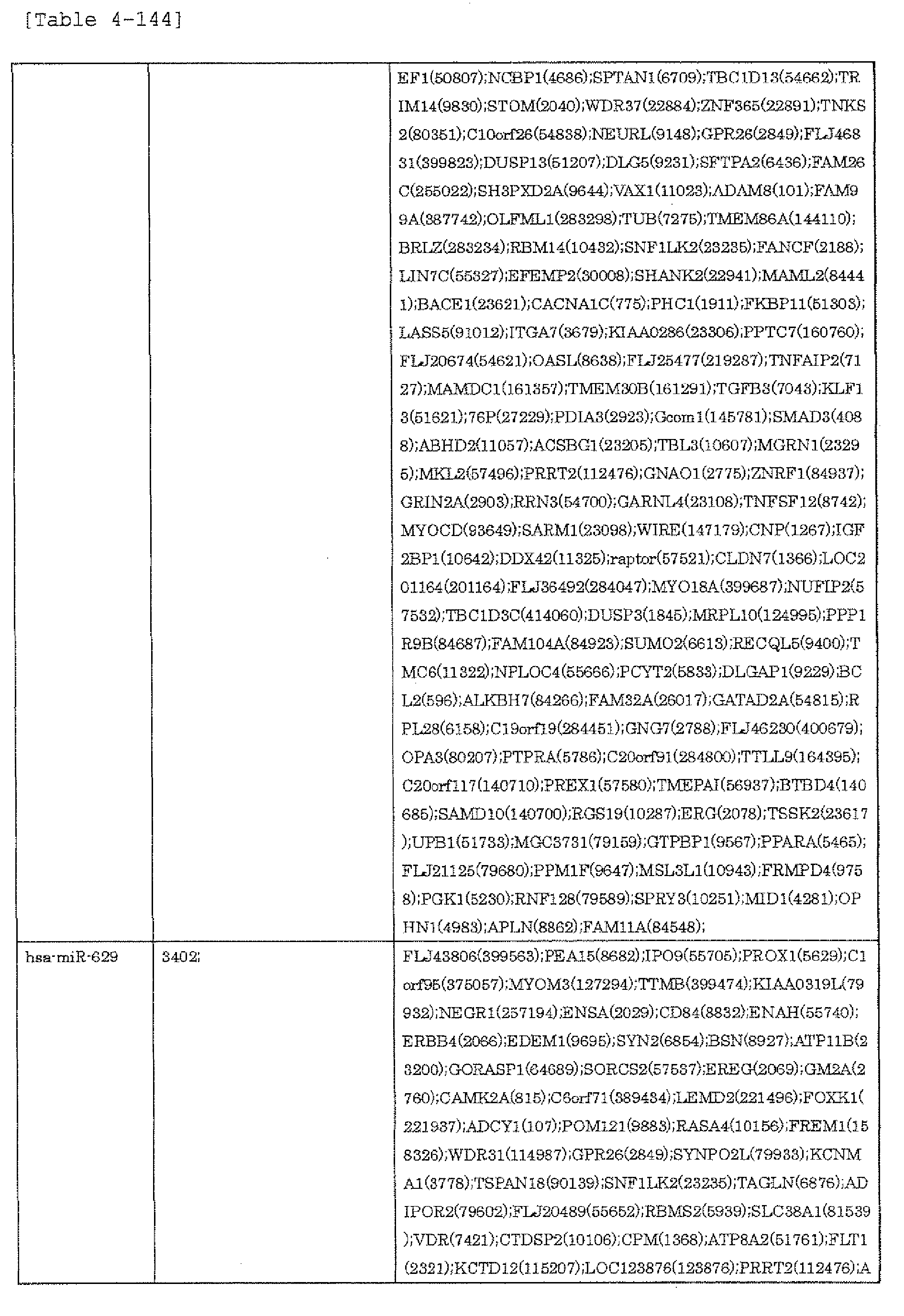

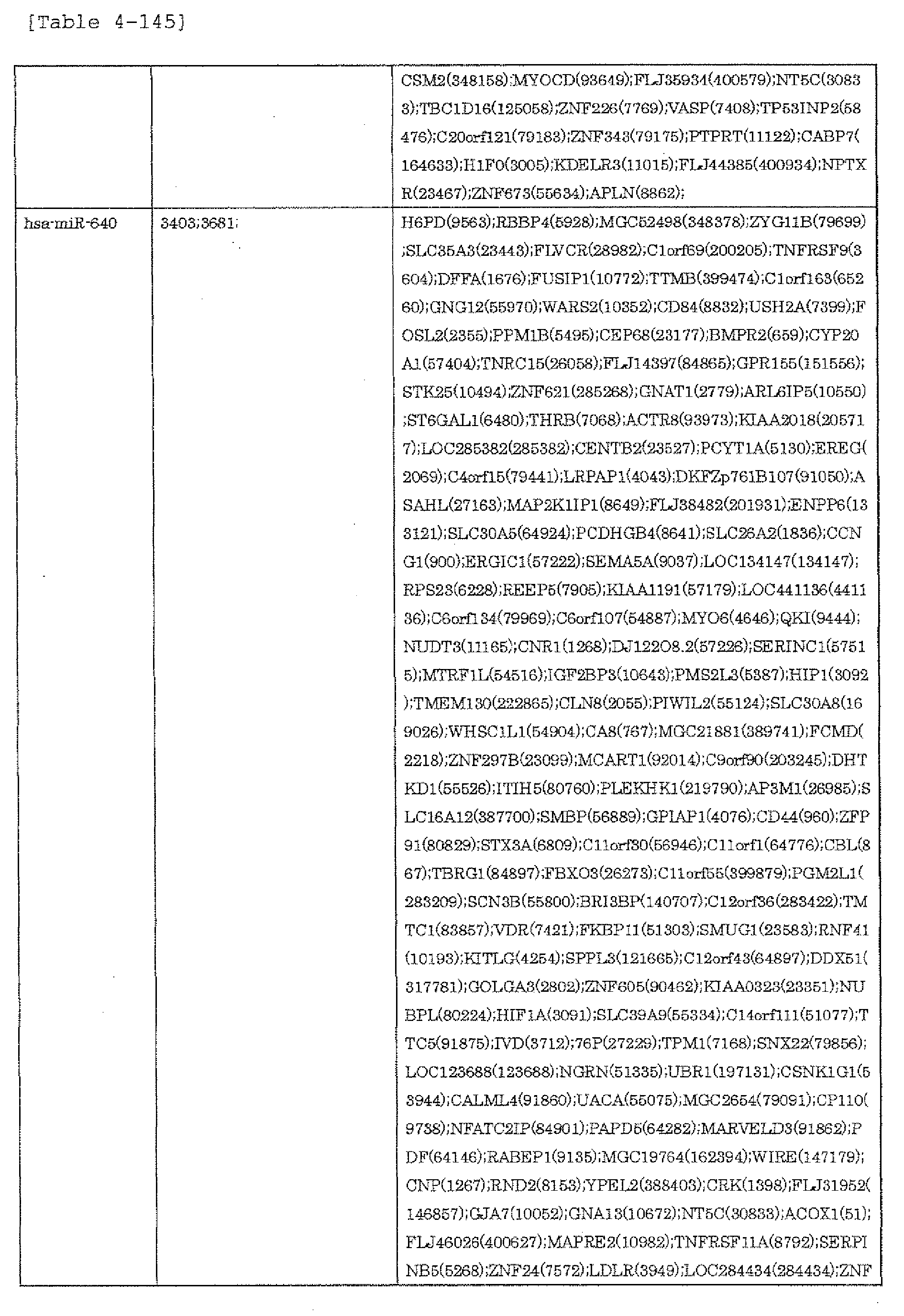

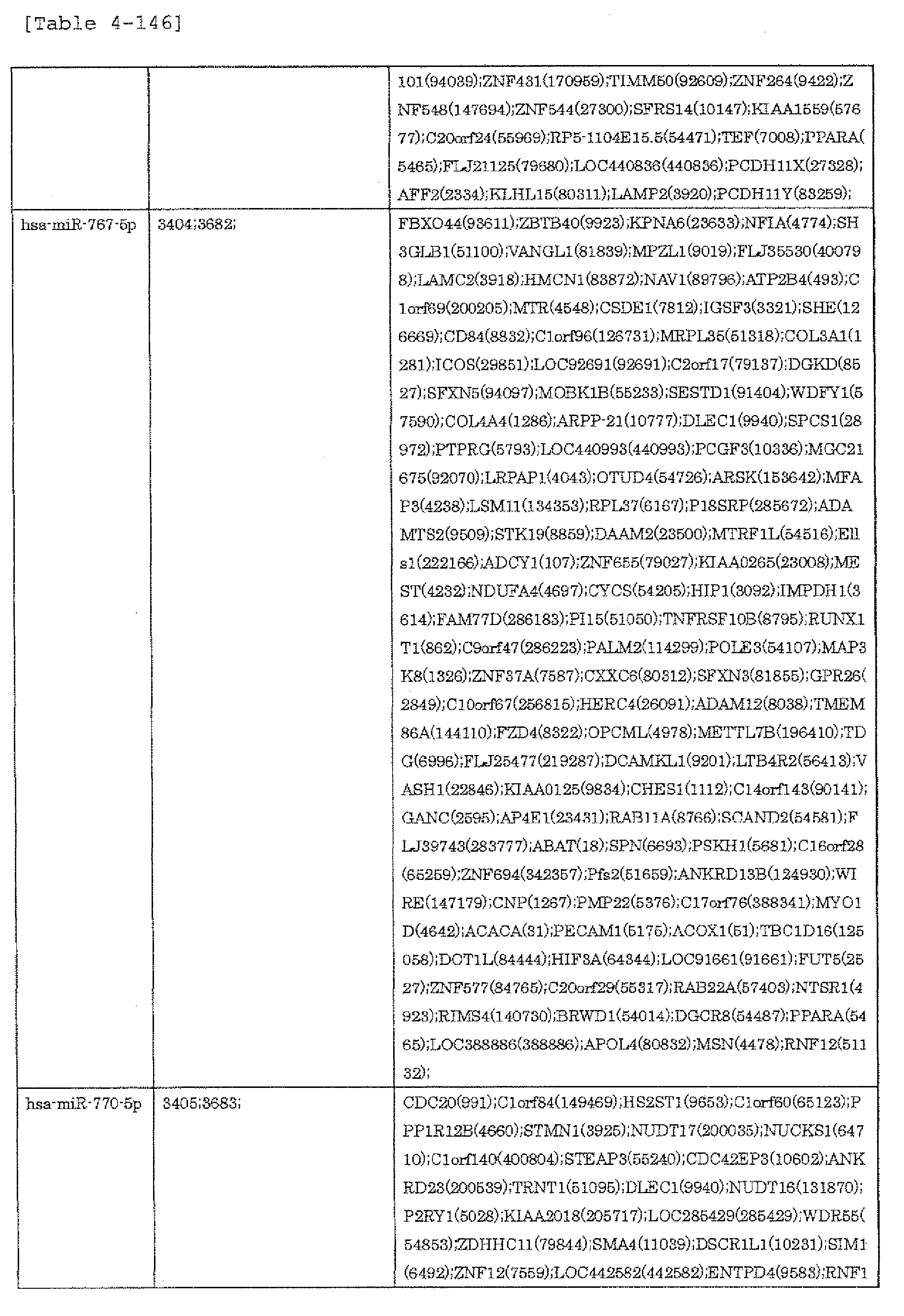

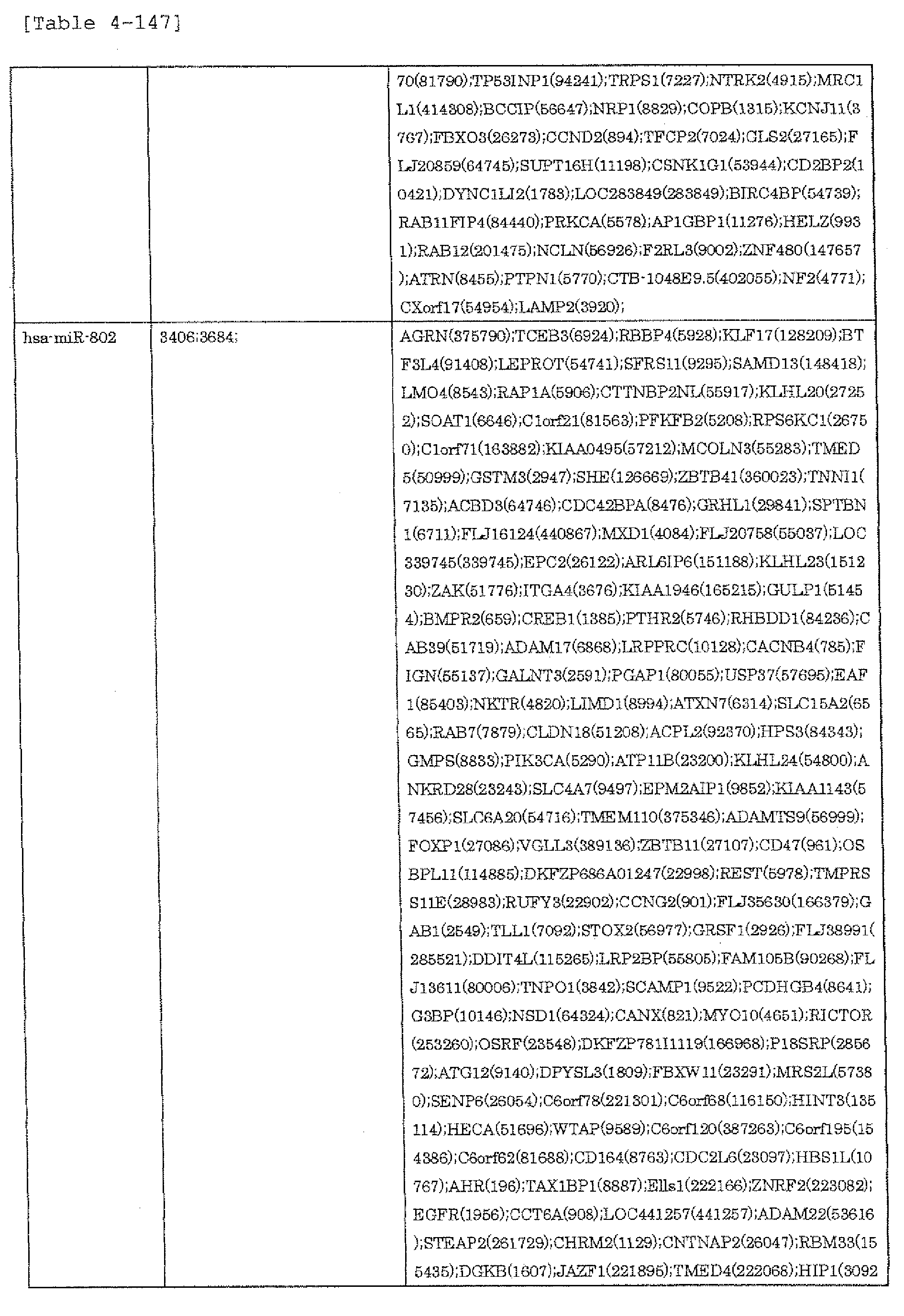

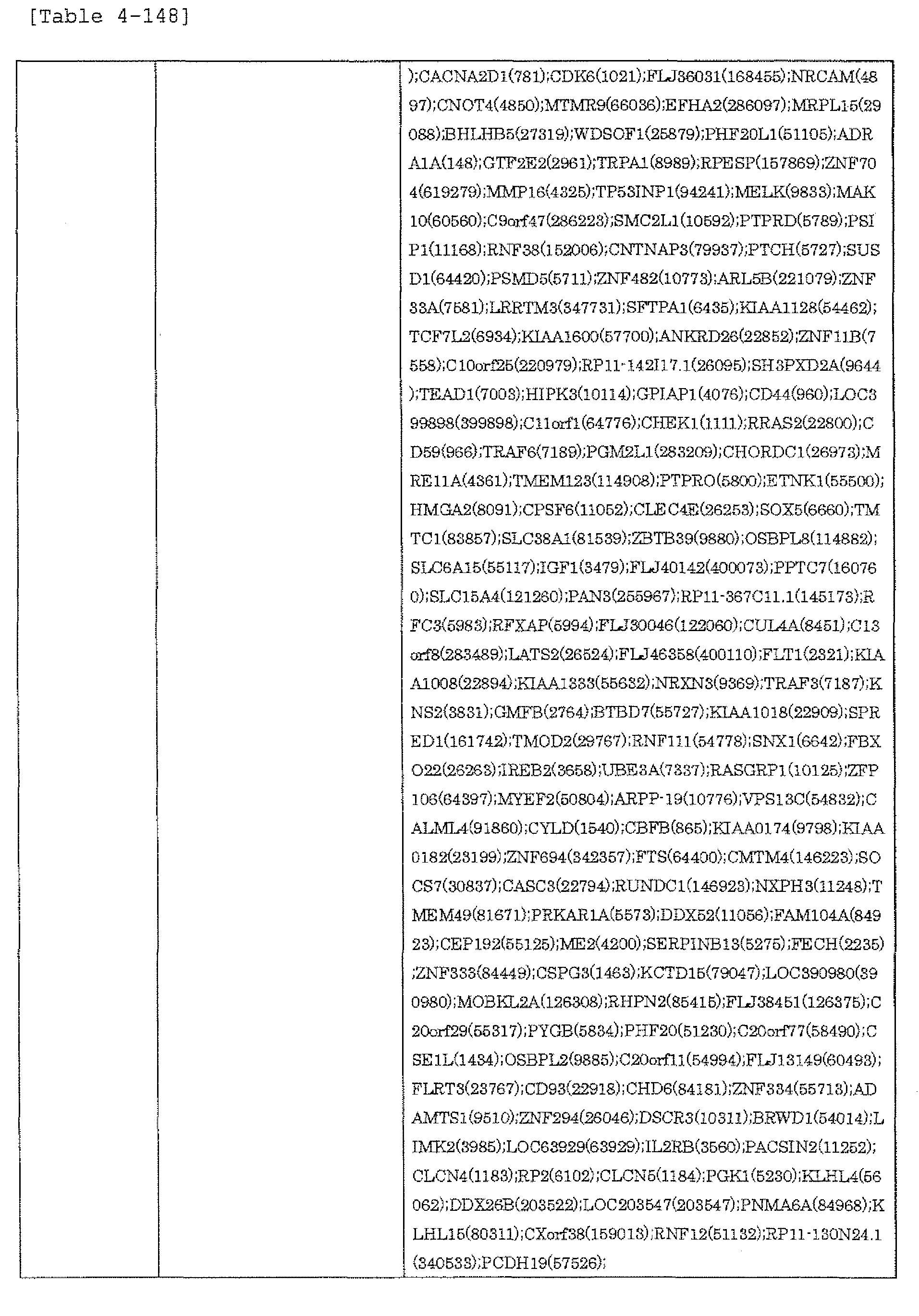

The substance that controls the expression or a function of a nucleic acid, such as a micro-RNA, used in the present invention, may be any substance such as a low-molecular compound, antibody, or siRNA, as far as it inhibits the expression or a function of the nucleic acid, and an siRNA is particularly preferable. As substances that suppress the expression of a nucleic acid, such as a micro-RNA, used in the present invention, substances that inhibit the function of a factor that is essential to the biosynthesis of the micro-RNA can also be used. Factors that are essential to the biosynthesis of a micro-RNA include, for example, Dicer, TRBP, Exportin5, Drosha, DGCR8 and the like.

As a method of expressing a nucleic acid such as a micro-RNA in cells in the present invention, a method using, for example, besides a gene encoding mRNA, a nucleic acid that causes the expression of the micro-RNA and the like when introduced into the cells can be mentioned. As the nucleic acid, a DNA, an RNA, or a nucleotide analogue, as well as a chimeric molecule thereof, or a derivative of the nucleic acid can also be used. Specifically, the nucleic acid can be designed in the same way as with Pre-miR^{™} miRNA Precursor Molecules (manufactured by Ambion) or miRIDIAN microRNA Mimics (manufactured by GE Healthcare), and the nucleic acid such as the micro-RNA of the present invention can be expressed in cells. Any method can be used to express a micro-RNA, as far as it allows the micro-RNA to be finally formed in the cells; for example, (1) a method wherein a single-stranded RNA that is a precursor of the micro-RNA is introduced, as well as (2) a method wherein an RNA consisting of double-strand consisting of the micro-RNA as it is and a complementary strand for the micro-RNA, which completely complement each other, is introduced, and (3) a method wherein a double-stranded RNA assumed to have resulted from cleavage of a micro-RNA with a Dicer is introduced, can be mentioned. As examples of commercial products based on these methods, miCENTURY OX Precursor (manufactured by B-Bridge), miCENTURY OX siMature (manufactured by B-Bridge), and miCENTURY OX miNatural (manufactured by B-Bridge), respectively, can be mentioned.

The method of producing a nucleic acid used in the present invention is not particularly limited; the same can be produced by a method using a publicly known chemical synthesis, or an enzymatic transcription method and the like. As methods using a publicly known chemical synthesis, the phosphoroamidite method, the phosphorothioate method, the phosphotriester method and the like can be mentioned; for example, the same can be synthesized using the ABI3900 high throughput nucleic acid synthesizer (manufactured by Applied Biosystems). As an enzymatic transcription method, transcription with a plasmid or DNA having a desired nucleotide sequence as the template using a typical phage RNA polymerase, for example, T7, T3, or SP6RNA polymerase, can be mentioned.

As an example of a method of screening a substance that suppresses the expression or a function of a nucleic acid by using the nucleic acid used in the present invention, a method can be mentioned wherein a vector that expresses the nucleic acid is introduced into cells, and a substance that promotes or suppresses the expression or a function of an RNA having a target nucleotide sequence therefor is screened for.
A pharmaceutical with a nucleic acid used in the present invention as an active ingredient can be used to diagnose or treat a disease caused by an abnormality of mast cell.

As diseases caused by a mast cell abnormality, specifically, atopic dermatitis, asthma, chronic obstructive lung disease, and allergic disease and the like can be mentioned.
Because abnormalities of mast cells include abnormalities in degranulation, a nucleic acid used in the present invention can be used as a mast cell degranulation control agent, that is, a degranulation promoter or a degranulation suppressant.

Mast cell degranulation suppressants are suitably used as prophylactic agents or therapeutic agents for atopic dermatitis, asthma, chronic obstructive pulmonary disease, allergic diseases and the like.
Mast cell degranulation promoters are suitably used as immunopotentiators.
The present invention is hereinafter described in detail.

### 1. Method of detecting the expression of nucleic acids such as micro-RNAs and micro-RNA precursors expressed in mast cells

### (1-1) Acquisition and cultivation of mast cells

The method of acquiring human mast cells is not particularly limited, as far as the human mast cells are acquired safely and efficiently; human mast cells can, for example, be prepared from a human lung, skin, fetal liver and the like by a commonly known method (J. Immunol. Methods, 169, 153 (1994); J. Immunol., 138, 861 (1987); J. Allergy Clin. Immunol., 107, 322 (2001); J. Immunol. Methods., 240, 101 (2000)). Human mast cells can also be prepared by culturing mononuclear cells prepared from human umbilical blood, peripheral blood, bone marrow, lung or skin in the presence of a stem cell factor (hereinafter abbreviated SCF) according to a commonly known method (J. Immunol., 157, 343, (1996); Blood, 91, 187 (1998); J. Allergy Clin. Immunol., 106, 141 (2000); Blood, 97, 1016 (2001); Blood, 98, 1127 (2001); Blood, 100, 3861 (2002); Blood, 97, 2045 (2001)) to allow the mononuclear cells to differentiate into mast cells.

A cell line established from a human mast cell can also be used. As human mast cell lines, LAD2 (Leuk. Res., 27, 671 (2003); Leuk. Res., 27, 677 (2003)), which is known to well retain the properties of human mast cells, and the like can be mentioned.

### (1-2) Acquisition of RNA

The method of extracting total RNA from the mast cells acquired by the various methods described above is not particularly limited, as far as a low-molecular RNA such as a micro-RNA is contained; for example, this extraction can be performed by a method described in Molecular Cloning, 3rd edition. Alternatively, the total RNA can also be extracted using Trizol (manufactured by Invitrogen Company), ISOGEN (manufactured by Nippon Gene Company), mirVana^{™} miRNA Isolation Kit (manufactured by Ambion Company) , miRNeasy Mini Kit (manufactured by QIAGEN Company) and the like.

A low-molecular RNA can also be cloned from a total RNA containing the low-molecular RNA. As a method of cloning a low-molecular RNA, specifically, a method wherein separation and cutting out of a low-molecular RNA by 15% polyacrylamide gel electrophoresis as described in Genes & Development 15, 188-200 (2000), can be mentioned. Then, 5' terminal dephosphorylation, 3'-adapter ligation, phosphorylation, 5'-adapter ligation, reverse transcription, PCR amplification, concatemerization, and ligation to a vector are performed sequentially, thereafter the low-molecular RNA is cloned, and the nucleotide sequence of the clone is determined. Alternatively, for example, a method wherein 5'-adenylation 3'-adapter ligation, 5'-adapter ligation, reverse transcription, PCR amplification, concatemerization, and ligation to a vector are performed sequentially, thereafter the low-molecular RNA is cloned, and the nucleotide sequence of the clone is also determined, as described in Science 294, 858-862 (2001).

Alternatively, separation and cleavage of low-molecular RNA by 15% polyacrylamide gel electrophoresis, 5' terminal dephosphorylation, 3'-adapter ligation, phosphorylation, 5'-adapter ligation, reverse transcription, PCR amplification, and ligation to a microbead vector are performed sequentially, thereafter the low-molecular RNA is cloned, and the nucleotide sequence of the microbeads is read to determine the nucleotide sequence, whereby nucleotide sequence information of the low-molecular RNA can also be acquired, as described in Nucleic Acids Research 34, 1765-1771

As another method of acquiring a low-molecular RNA, a method involving the use of the low-molecular RNA Cloning Kit (manufactured by Takara Bio Company) can be mentioned.

### (1-3) Identification of micro-RNA

Whether or not the low-molecular RNA sequence is a micro-RNA can be determined on the basis of whether or not the criteria described in RNA, 9, 277-279 (2003) are met. For example, in cases where the low-molecular RNA was newly acquired and the nucleotide sequence thereof was determined, this can be performed as described below.

A surrounding genome sequence wherein a DNA sequence corresponding to the nucleotide sequence of the low-molecular RNA acquired is extended by about 50 nucleotides toward the 5' terminal side and the 3' terminal side, respectively, is acquired, and the secondary structure of the RNA expected to be transcribed from the genome sequence is predicted. If the result shows that a hairpin structure is present and the nucleotide sequence of the low-molecular RNA is located in one chain of the hairpin, the low-molecular RNA can be judged to be a micro-RNA. Genome sequences are open to the general public, and are available from, for example, UCSC Genome Bioinformatics (http://genome.ucsc.edu/). For prediction of secondary structures, various programs are open; for example, RNAfold [Nucleic Acids Research 31, 3429-3431 (2003)], Mfold [Nucleic Acids Research 31, 3406-3415 (2003)] and the like can be used. Existing micro-RNA sequences are registered in a database called miRBase (http://microrna.sanger.ac.uk/); whether or not a micro-RNA is identical to an existing micro-RNA can be determined on the basis of whether or not the sequence thereof is identical to one of the sequences listed therein.

### (1-4) Method of detecting the expression levels of nucleic acid such as micro-RNA

As examples of methods of detecting the expression levels of nucleic acid such as micro-RNA, a precursor thereof and the like, methods by (1) Northern hybridization, (2) dot blot hybridization, (3) in situ hybridization, (4) quantitative PCR, (5) differential hybridization, (6) microarray, (7) ribonuclease protection assay and the like can be mentioned.

The Northern hybridization is a method wherein a sample-derived RNA is separated by gel electrophoresis, then transferred to a solid support such as a nylon filter, and an appropriately-labeled probe is prepared on the basis of the nucleotide sequence of the nucleic acid of the present invention, and hybridization and washing are performed, whereby a band specifically bound to the nucleic acid is detected; specifically, for example, this method can be performed as described in Science 294, 853-858 (2001) and the like.

A labeled probe can be prepared by incorporating a radioisotope, biotin, digoxigenin, a fluorescent group, a chemiluminescent group and the like in a DNA, RNA, or LNA having a sequence complementary to the nucleotide sequence of the nucleic acid to be used by a method, for example, nick translation, random priming or 5'-terminal phosphorylation. Because the amount of labeled probe bound reflects the expression level of the nucleic acid, the expression level of the nucleic acid can be quantified by quantifying the amount of labeled probe bound. Electrophoresis, membrane transfer, probe preparation, hybridization, and nucleic acid detection can be achieved by a method described in Molecular Cloning, 3rd edition.

Dot blot hybridization is a method wherein an RNA extracted from a tissue or a cell is spotted in dot forms and immobilized on a membrane, and hybridized with a labeled polynucleotide to be a probe, and an RNA that specifically hybridizes with the probe is detected. The probe used may be the same as that used for Northern hybridization. RNA preparation, RNA spotting, hybridization, and RNA detection can be achieved by a method described in Molecular Cloning, 3rd edition.

In situ hybridization is a method wherein a paraffin-embedded or cryostat-treated section of a tissue acquired from a living organism, or a cell fixed, is used as a sample and subjected to steps for hybridization with a labeled probe and washing, and the distribution and localization of a nucleic acid in the tissue or cell are examined by microscopic examination [Methods in Enzymology, 254, 419 (1995)]. The probe used may be the same as that used for Northern hybridization. Specifically, a nucleic acid such as micro-RNA etc. can be detected in accordance with a method described in Nature Method 3, 27 (2006).

In quantitative PCR, a cDNA synthesized from a sample-derived RNA using a primer for reverse transcription and a reverse transcriptase (hereunder, this cDNA is also referred to as a sample-derived cDNA) is used for the measurement. As a primer for reverse transcription to be supplied for cDNA synthesis, a random primer or a specific RT primer and the like can be used. A specific RT primer refers to a primer having a sequence complementary to a nucleotide sequence corresponding to a nucleic acid and a genomic sequence therearound.

For example, a sample-derived cDNA is synthesized, after which a PCR is performed with this cDNA as the template, using a template-specific primer designed from a nucleotide sequence corresponding to a nucleic acid such as micro-RNA or micro-RNA precursor and a genomic sequence therearound, or from a nucleotide sequence corresponding to a primer for reverse transcription, to amplify a cDNA fragment containing the nucleic acid used in the present invention, and the amount of the nucleic acid of the present invention contained in the sample-derived RNA is detected from the number of cycles for reach to a given amount of the fragment. As the template-specific primer, an appropriate region corresponding to a nucleic acid and a genomic sequence therearound is selected, and a pair of a DNA or LNA consisting of a sequence of 20 to 40 nucleotides at the 5' terminus of the nucleotide sequence of the region, and a DNA or LNA consisting of a sequence complementary to a sequence of 20 to 40 nucleotides at the 3' terminus, can be used. Specifically, this can be performed in accordance with a method described in Nucleic Acids Research, 32, e43 (2004) and the like.

Alternatively, as the primer for reverse transcription to be supplied for cDNA synthesis, a specific RT primer having a stem-loop structure can also be used. Specifically, this can be performed using a method described in Nucleic Acid Research, 33, e179 (2005), or TaqMan MicroRNA Assays (manufactured by Applied Biosystems).
Furthermore, by hybridizing a sample-derived cDNA to a DNA, or LNA corresponding to a nucleotide sequence comprising at least one or more of nucleic acids such as micro-RNA, micro-RNA precursor and the like to be used in the present invention immobilized on a substrate such as a filter, glass slide, or silicone, and performing washing, a change in the amount of the nucleic acid can be detected.
As such methods based on hybridization, methods using differential hybridization [Trends Genet., 7, 314 (1991)] or a microarray (Genome Res., 6, 639 (1996)) can be mentioned. Both methods enable accurate detection of a difference in the amount of a nucleic acid between a control sample and a target sample by immobilizing an internal control, such as a nucleotide sequence corresponding to U6RNA, on a filter or a substrate. Also, by synthesizing labeled cDNAs using differently labeled dNTPs (mixtures of dATP, dGTP, dCTP, and dTTP) on the basis of RNAs derived from a control sample and a target sample, and simultaneously hybridizing the two labeled cDNAs to a single filter or a single substrate, accurate quantitation of a nucleic acid can be performed. For example, nucleic acids such as micro-RNA and the like can be detected using a microarray described in Proc. Natl. Acad. Sci. USA, 101, 9740-9744 (2004), Nucleic Acid Research, 32, e188 (2004), RNA, 13, 151-159 (2007) and the like. Specifically, a micro-RNA can be detected or quantified in the same manner as mirVana miRNA Bioarray (manufactured by Ambion).

In ribonuclease protection assay, first, a promoter sequence such as the T7 promoter or the SP6 promoter is bound to the 3' terminus of a nucleotide sequence corresponding to the nucleic acid such as micro-RNA or micro-RNA precursor to be used in the present invention or a genomic sequence therearound, and a labeled antisense RNA is synthesized with an in vitro transcription system using a labeled NTP (a mixture of ATP, GTP, CTP, and UTP) and an RNA polymerase. The labeled antisense RNA is bound to a sample-derived RNA to form an RNA-RNA hybrid, after which the hybrid is digested with ribonuclease A, which degrades single-stranded RNAs only. The digest is subjected to gel electrophoresis to detect or quantify an RNA fragment protected against the digestion by forming the RNA-RNA hybrid, as a nucleic acid. Specifically, the fragment can be detected or quantified using the mirVana miRNA Detection Kit (manufactured by Ambion).

### 2. Synthesis of nucleic acid

The nucleic acid to be used in the present invention such as a micro-RNA or micro-RNA precursor can synthesize not only an RNA, which is a polymer of a ribonucleotide, but also a DNA, which is a polymer of a deoxyribonucleotide, on the basis of the nucleotide sequences. For example, on the basis of the nucleotide sequence of the micro-RNA identified in 1 above, the nucleotide sequence of a DNA can be determined. The nucleotide sequence of a DNA corresponding to the nucleotide sequence of an RNA can be determined, without exception, by reading the U (uracil) contained in the sequence of the RNA as T (thymine). A polymer being a mixture of a ribonucleotide and a deoxyribonucleotide and a polymer comprising a nucleotide analogue and a derivative of a nucleic acid can also be synthesized in the same manner.

The method of synthesizing a nucleic acid used in the present invention is not particularly limited; a method using a publicly known chemical synthesis, or an enzymatic transcription method and the like can be mentioned. As methods using a publicly known chemical synthesis, the phosphoroamidite method, the phosphorothioate method, the phosphotriester method and the like can be mentioned; for example, a nucleic acid can be synthesized using the ABI3900 high throughput nucleic acid synthesizer (manufactured by Applied Biosystems). As an enzymatic transcription method, a method by transcription with a plasmid or DNA having a desired nucleotide sequence as the template using a typical phage RNA polymerase, for example, T7, T3, or SP6RNA polymerase, can be mentioned.

### 3. Method of detecting a function of a nucleic acid such as micro-RNA or micro-RNA precursor

As a method of detecting a function of a nucleic acid such as a micro-RNA, a method can be mentioned wherein the function is determined on the basis of whether or not the translation of the RNA having a target nucleotide sequence is suppressed.

Micro-RNAs are known to suppress the translation of an RNA comprising a target nucleotide sequence thereof on the 3' side (3'UTR) [Current Biology, 15, R458-R460 (2005)]. Hence, a DNA wherein a target nucleotide sequence for the single-stranded DNA to be measured is inserted into the 3'UTR of an appropriate reporter gene expression vector is prepared and introduced into a host cell suitable for the expression vector, and the expression of the reporter gene is measured when the cell is allowed to express the single-stranded RNA, whereby whether or not a function of a micro-RNA is possessed can be determined.

The reporter gene expression vector may be any one, as far as it has a promoter upstream of a reporter gene, and is capable of expressing the reporter gene in the host cell. Any reporter gene can be used; for example, the firefly luciferase gene, the Renilla luciferase gene, the chloramphenicol acetyltransferase gene, the β-glucuronidase gene, the β-galactosidase gene, the β-lactamase gene, the aequorin gene, the green fluorescent protein gene, the DsRed fluorescence gene and the like can be utilized. As examples of reporter gene expression vectors having these properties, psiCHECK-1 (manufactured by Promega), psiCHECK-2 (manufactured by Promega), pGL3-Control (manufactured by Promega), pGL4 (manufactured by Promega), pRNAi-GL (manufactured by Takara Bio Inc.), pCMV-DsRed-Express (manufactured by CLONTECH) and the like can be mentioned. A single-stranded RNA can be expressed by the method described in 5 below.

As the function of a micro-RNA of a single-stranded RNA, detection can be achieved as described below. First, host cells are cultured on a multi-well plate and the like, and a reporter gene expression vector having a target sequence and a single-stranded RNA are expressed. Thereafter, reporter activity is measured both when the single-stranded RNA is and is not expressed, based on which the function of micro-RNA can be detected. 4. Method of detecting a mutation of a nucleic acid such as micro-RNA or micro-RNA precursor
As a method of detecting a mutation of a nucleic acid such as a micro-RNA or micro-RNA precursor, a method can be used wherein a heteroduplex formed by hybridization of a normal type nucleic acid and a mutated type nucleic acid are detected.

As methods of detecting a heteroduplex, (1) detection of a heteroduplex by polyacrylamide gel electrophoresis (Trends genet., 7, 5 (1991)), (2) single-strand conformation polymorphism analysis (Genomics, 16, 325-332 (1993)), (3) chemical cleavage of mismatches (CCM) (Human Genetics (1996), Tom Strachan and Andrew P. Read, BIOS Scientific Publishers Limited), (4) enzymatic cleavage of mismatches (Nature Genetics, 9, 103-104 (1996)), (5) denatured gel electrophoresis (Mutat. Res., 288, 103-112 (1993)) and the like can be mentioned.

Detection of a heteroduplex by polyacrylamide gel electrophoresis is, for example, performed as described below. First, with a sample-derived DNA or a sample-derived cDNA as a template,
and using a primer designed on the basis of a genomic nucleotide sequence comprising the nucleotide sequence of a nucleic acid used in the present invention, a fragment smaller than 200 nucleotide pairs is amplified. Heteroduplexs, if formed, are slower in mobility than mutation-free homoduplexs, and can be detected as extra bands. In the case of search for a fragment smaller than 200 nucleotide pairs, almost any insertions, deletions, and substitutions of one or more nucleotides can be detected. It is desirable that heteroduplex analysis be performed using a single gel in combination with the single strand conformation analysis described below.

In single strand conformation polymorphism analysis (SSCP analysis), a DNA amplified as a fragment smaller than 200 bp with a sample-derived DNA or sample-derived cDNA as the template, using a primer designed on the basis of a genomic nucleotide sequence comprising the nucleotide sequence of a nucleic acid of the present invention, is denatured, after which it is electrophoresed in non-denatured polyacrylamide gel. By labeling the primer with an isotope or a fluorescent dye at the time of DNA amplification, or by silver-staining the non-labeled amplification product, the amplified DNA can be detected as a band. To clarify a difference from the wild type pattern, a control sample may be electrophoresed simultaneously, whereby a fragment with a mutation can be detected on the basis of a difference in mobility.

In chemical cleavage of mismatches (CCM method), a DNA fragment amplified with a sample-derived DNA or sample-derived cDNA as the template, using a primer designed on the basis of a genomic nucleotide sequence comprising the nucleotide sequence of a nucleic acid of the present invention is hybridized to a labeled nucleic acid prepared by allowing a nucleic acid to incorporate an isotope or a fluorescent target, and treated with osmium tetraoxide to cleave one strand of the DNA at the mismatched portion, whereby a mutation can be detected. CCM is one of the most sensitive methods of detection, and can be applied to samples of kilobase length.

In place of osmium tetraoxide used above, T4 phage resolvase and an enzyme involved in mismatch repair in cells, such as endonuclease VII, and RNaseA may be used in combination to enzymatically cleave a mismatch. In denaturing gradient gel electrophoresis (DGGE method), a DNA amplified with a sample-derived DNA or sample-derived cDNA as the template, using a primer designed on the basis of a genomic nucleotide sequence comprising the nucleotide sequence of a nucleic acid of the present invention is electrophoresed using a gel having a chemical denaturant density gradient or a temperature gradient. The DNA fragment amplified migrates in the gel to a position where it denatures to a single strand, and no longer migrates after the denaturation. Because the migration of the amplified DNA in the gel differs between the presence and absence of a mutation, the presence of the mutation can be detected. To increase the detection sensitivity, the addition of a poly (G:C) end to each primer is effective.

By directly determining and analyzing the nucleotide sequence of a sample-derived DNA or sample-derived cDNA, a mutation of a nucleic acid used in the present invention can also be detected.

### 5. Methods of expressing a nucleic acid such as micro-RNA or micro-RNA precursor

A nucleic acid used in the present invention can be expressed by using a vector such that a nucleic acid is transcribed and hence biosynthesized when the vector is introduced into a cell. Specifically, by preparing a DNA fragment comprising a hairpin portion on the basis of the aforementioned nucleotide sequence of a nucleic acid or a genomic nucleotide sequence comprising the foregoing nucleotide sequence, and inserting the fragment downstream of a promoter in the expression vector to construct an expression plasmids, and then introducing the expression plasmid into a host cell suitable for the expression vector, whereby the aforementioned nucleic acid can be expressed.

As an expression vector, one capable of self-replication in the host cell, and capable of being incorporated in the chromosome, which comprises a promoter at a position enabling the transcription of a gene comprising the nucleotide sequence of a nucleic acid of the present invention is used. The promoter may be any one, as far as it is capable of expressing in the host cell; for example, a RNA, polymerase II (pol II) system promoter, a RNA polymerase III (pol III) system promoter being a U6RNA and H1RNA transcription system and the like can be mentioned. As examples of pol II system promoters, the promoter of the cytomegalovirus (human CMV) IE (immediate early) gene, the early promoter of SV40 and the like can be mentioned. As examples of expression vectors using them, pCDNA6.2-GW/miR (manufactured by Invitrogen) , pSilencer 4.1-CMV (manufactured by Ambion) and the like can be mentioned. As pol III system promoters, U6RNA, H1RNA or tRNA gene promoters can be mentioned. As examples of expression vectors using them, pSINsi-hH1 DNA (manufactured by Takara Bio Inc.), pSINsi-hU6 DNA (manufactured by Takara Bio Inc.), pENTR/U6 (manufactured by Invitrogen) and the like can be mentioned.

Alternatively, by inserting a gene comprising the nucleotide sequence of a nucleic acid used in the present invention into the downstream of the promoter in a viral vector to construct a recombinant viral vector, and introducing the vector into a packaging cell to produce a recombinant virus, the gene comprising the nucleic acid can be expressed.
The packaging cell may be any cell, as far as it is capable of supplementing a recombinant viral vector deficient in any one of the genes that encode the proteins necessary for the packaging of the virus with the lacked protein; for example, human kidney-derived HEK293 cells, mouse fibroblasts NIH3T3 and the like can be used. As the protein supplemented by the packaging cell, in the case of a retrovirus vector, proteins derived from a mouse retrovirus, such as gag, pol, and env, can be used; in the case of a lentivirus vector, proteins derived from a HIV virus, such as gag, pol, env, vpr, vpu, vif, tat, rev, and nef, can be used; in the case of an adenovirus vector, proteins derived from an adenovirus, such as E1A and E1B, can be used; in the case of an adeno-associated viral vector, proteins such as Rep (p5, p19, p40) and Vp(Cap), can be used.

Aside from using an expression vector, it is also possible to introduce a nucleic acid used in the present invention directly to the cell without using a vector. Such nucleic acids useful in this method include not only DNA, RNA or nucleotide analogues, but also chimera molecules thereof, or derivatives of the nucleic acids. Specifically, in the same manner as with Pre-miR^{™} mRNA Precursor Molecules (manufactured by Ambion Company) and miRIDIAN microRNA Mimics (manufactured by GE Healthcare Company), a nucleic acid used in the present invention can be expressed. When a micro-RNA is expressed, any method can be used, as far as it allows the micro-RNA to be eventually produced in the cell; examples include methods wherein (1) a single-stranded RNA which is a micro-RNA precursor, (2) an RNA, consisting of a double strand consisting of a micro-RNA and a strand complementary to the micro-RNA which are completely complementary to each other, (3) or a double-stranded RNA assuming a state after the micro-RNA is cut by a Dicer, is introduced. Available products involving the use of these methods are miCENTURY OX Precursor (manufactured by B-Bridge Company), miCENTURY OX siMature (manufactured by B-Bridge Company), and miCENTURY OX miNatural (manufactured by B-Bridge Company).

### 6. Methods of suppressing the activity of a nucleic acid such as micro-RNA or micro-RNA precursor

The activation of a nucleic acid such as micro-RNA or micro-RNA precursor to be used in the present invention can be suppressed using an antisense technology (Bioscience and Industry, 50, 322 (1992); Kagaku, 46, 681 (1991), Biotechnology, 9, 358 (1992), Trends in Biotechnology, 10, 87 (1992), Trends in Biotechnology, 10, 152 (1992); Cell Technology, 16, 1463 (1997)], triple helix technology (Trends in Biotechnology, 10, 132 (1992)), ribozyme technology (Current Opinion in Chemical Biology, 3, 274 (1999), FEMS Microbiology Reviews, 23, 257 (1999), Frontiers in Bioscience, 4, D497 (1999), Chemistry & Biology, 6, R33 (1999), Nucleic Acids Research, 26, 5237 (1998), Trends In Biotechnology, 16, 438 (1998)), decoy DNA method (Nippon Rinsho - Japanese Journal of Clinical Medicine, 56, 563 (1998), Circulation Research, 82, 1023 (1998), Experimental Nephrology, 5, 429 (1997), Nippon Rinsho - Japanese Journal of Clinical Medicine, 54, 2583 (1996)), or a siRNA (short interfering RNA).

An antisense refers to one that allows nucleotide sequence-specific hybridization of a nucleic acid having a nucleotide sequence complementary to a certain target nucleic acid to enable the suppression of the expression of the target nucleic acid. As the nucleic acid used as the antisense, a DNA, an RNA or a nucleotide analogue, as well as a chimeric molecule thereof, or a derivative of the nucleic acid can also be used. Specifically, by preparing an antisense in accordance with a method described in Nature 432, 226 (2004) and the like, the expression can be suppressed. In addition, in the same way as with Anti-miR^{™} miRNA Inhibitors (manufactured by Ambion) and miRIDIAN miRNA Inhibitors (manufactured by GE Healthcare), the expression of a nucleic acid used in the present invention can be suppressed.

An siRNA refers to a short double-stranded RNA comprising the nucleotide sequence of a certain target nucleic acid, that is capable of suppressing the expression of the target nucleic acid by RNA interference (RNAi). The sequence of an siRNA can be designed as appropriate from the target nucleotide sequence on the basis of conditions shown in the literature (Genes Dev., 13, 3191 (1999)). By synthesizing two RNAs having a 19-nucleotide sequence selected and a complementary sequence, with TT added to the 3' terminus of each thereof using a nucleic acid synthesizer, and performing annealing, an siRNA can be prepared. By inserting a DNA corresponding to the above-described 19-nucleotide sequence selected into an siRNA expression vector such as pSilencer 1.0-U6 (manufactured by Ambion) or pSUPER (manufactured by OligoEngine), a vector that expresses an siRNA capable of suppressing the expression of the gene can be prepared. Although any siRNA can be used to suppress a function of a nucleic acid, such as a micro-RNA, used in the present invention, as far as it is capable of suppressing the function of the nucleic acid, preference is given to an siRNA designed on the basis of sequence information on SEQ ID NO:1 to 3371. The number of nucleotide residues constituting one strand of the siRNA is preferably 17 to 30 residues, more preferably 18 to 25 residues, still more preferably 19 to 23 residues.

Using an antisense or siRNA specific for a nucleic acid such as micro-RNA expressed in mast cells, or a precursor of the micro-RNA, the expression of a micro-RNA expressed in mast cells, a precursor of the micro-RNA and the like can be suppressed. For example, by administering an antisense oligonucleotide or siRNA specific for the micro-RNA or the micro-RNA precursor, the activation of the micro-RNA can be suppressed, and the action of the micro-RNA or micro-RNA precursor in mast cells can be controlled.

In addition, in the case of a patient affected by an abnormality of the expression of a micro-RNA expressed in mast cells or a precursor thereof, by administering an antisense oligonucleotide or siRNA specific for the micro-RNA or precursor thereof to the patient, it is possible to control a function of mast cells to treat a disease that develops as a result of the above-described expression abnormality. Hence, an antisense oligonucleotide or siRNA that is specific for the micro-RNA or precursor thereof is useful as a therapeutic agent for a disease caused by a cell abnormality of mast cells.

When an antisense oligonucleotide or siRNA specific for a nucleic acid such as a micro-RNA or a precursor thereof is used as the above-described therapeutic agent, the antisense oligonucleotide or siRNA alone, or a nucleic acid that encodes the same after being inserted into an appropriate vector such as a retrovirus vector, adenovirus vector, or adeno-associated virus vector, may be prepared as a pharmaceutical preparation according to the conventional method described in 9 below, and administered.

### 7. Methods of suppressing functions or expression of target genes using a nucleic acid such as micro-RNA or micro-RNA precursor

Any method can be used to suppress a function or the expression of the target gene for a nucleic acid used in the present invention, as far as it is based on an activity of a nucleic acid such as a micro-RNA to suppress the expression of the mRNA having the target nucleotide sequence. For example, a method can be mentioned wherein a nucleic acid used in the present invention such as a micro-RNA is expressed to increase the amount of the nucleic acid such as the micro-RNA in cells, whereby the translation of the mRNA having the target sequence is suppressed to suppress the expression of the gene. The expression of a nucleic acid used in the present invention can be achieved by the method described in 5 above. As examples of mRNAs having a target nucleotide sequence for a nucleic acid consisting of a nucleotide sequence of any one of SEQ ID NOs:1-1543, and 3372-3741, the respective sets of genes shown in the foregoing Table 4 can be mentioned.

Using an siRNA against a target gene shown in Table 4, it is possible to suppress a function of the target gene.

### 8. Methods of screening for substances that controls the expression or a function of a nucleic acid such as a micro-RNA or micro-RNA precursor using the nucleic acid

A substance that controls, that is, promotes or suppresses the expression or a function of a nucleic acid such as a micro-RNA or a precursor thereof can be screened for using a nucleic acid used in the present invention. For example, a nucleotide sequence to be targeted for screening is chosen from the nucleotide sequence of a micro-RNA or a precursor thereof to be used in the present invention and by means of cells that express a nucleic acid having the nucleotide sequence, a substance that promotes or suppresses the expression or a function of the chosen micro-RNA or precursor thereof can be screened for.

As cells that express a nucleic acid having the nucleotide sequence of a micro-RNA or micro-RNA precursor, used for screening, mast cells, as well as transformant cells obtained by introducing a vector that expresses a nucleic acid having the nucleotide sequence into a host cell such as an animal cell or yeast, cells incorporating a nucleic acid having the nucleotide sequence introduced directly without using a vector and the like as described in 5 above can also be used.

As specific screening methods, a method comprising using a change in the expression level of a nucleic acid such as an micro-RNA or precursor thereof to be targeted in the screening as an index, as well as a method comprising using a change in the expression level of an mRNA having a target sequence for a nucleic acid such as a micro-RNA or a gene product encoded thereby as an index can be mentioned.

### (a) Screening method comprising using a change in the expression level of a micro-RNA or precursor thereof to be targeted in the screening as an index

A test substance is brought into contact with a cell that expresses the nucleic acid, and with a change in the expression level of the nucleic acid selected as an index, a substance that promotes or suppresses the nucleic acid such as a micro-RNA and precursor thereof is obtained. The expression level of a nucleic acid can be detected by the method described in 3 above.

### (b) Screening method comprising using a change in the expression level of an mRNA having a target sequence for a nucleic acid such as a micro-RNA to be targeted in the screening or a gene product encoded thereby as an index

A test substance is brought into contact with a cell that expresses the mRNA, and with a change in the expression level of an mRNA having a target sequence of the nucleic acid selected or a gene product encoded thereby as an index, a substance that promotes or suppresses the expression or a function of a nucleic acid such as a micro-RNA and a precursor thereof can be obtained. Alternatively, a DNA having a target sequence of a nucleic acid of the present invention such as micro-RNA inserted into the 3' UTR of an appropriate reporter gene expression vector is prepared and introduced into a host cell suitable for the expression vector, a test substance is brought into contact with the cell, and with a change in the expression level of the reporter gene as an index, a substance that promotes or suppresses the expression or a function of a nucleic acid such as a micro-RNA and a precursor thereof can be obtained.

Choice of a target sequence can be achieved by the method described in 7 above; as examples of an mRNA having a target sequence of a nucleic acid such as a micro-RNA consisting of the nucleotide sequence of any one of SEQ ID NOs:1 to 1543 and 3372 to 3741, the above-described genes shown in Table 4 above can be exemplified.

### 9. Mast cell degranulation control agents comprising a nucleic acid such as a micro-RNA or a micro-RNA precursor

Nucleic acids such as micro-RNAs and micro-RNA precursors and nucleic acids having nucleotide sequences complementary to the nucleotide sequences thereof, used in the present invention, can be used as mast cell degranulation control agents, that is, degranulation suppressants or degranulation promoters, because they control the expression of genes having a target sequence.

As active ingredients of mast cell degranulation suppressants, the nucleic acids (a) to (h) below can be mentioned.
(a) A nucleic acid consisting of a nucleotide sequence of any one of SEQ ID NOs:1 to 70, 151 to 581, 1238 to 1461, and 3407 to 3452.
(b) A nucleic acid of 17 to 28 nucleotides comprising a nucleic acid consisting of a nucleotide sequence of any one of SEQ ID NOs:1 to 70, 151 to 581, 1238 to 1461, and 3407 to 3452.
(c) A nucleic acid consisting of a nucleotide sequence having an identity of 90% or more to a nucleotide sequence of any one of SEQ ID NOs:1 to 70, 151 to 581, 1238 to 1461, and 3407 to 3452.
(d) A nucleic acid that hybridizes under stringent conditions with a complementary strand for a nucleic acid consisting of a nucleotide sequence of any one of SEQ ID NOs:1 to 70, 151 to 581, 1238 to 1461, and 3407 to 3452.
(e) A nucleic acid comprising the 2nd to 8th nucleotides of a nucleotide sequence of any one of SEQ ID NOs:1 to 70, 151 to 581, 1238 to 1461, and 3407 to 3452.
(f) A nucleic acid consisting of a nucleotide sequence of any one of SEQ ID NOs:1544 to 1621, 1634, 1665 to 1668, 1688, 1709 to 2196, 2862 to 2864, 2978 to 3287, 3365, and 3780 to 3828.
(g) A nucleic acid consisting of a nucleotide sequence having an identity of 90% or more to a nucleotide sequence of any one of SEQ ID NOs:1544 to 1621, 1634, 1665 to 1668, 1688, 1709 to 2196, 2862 to 2864, 2978 to 3287, 3365, and 3780 to 3828.
(h) A nucleic acid that hybridizes under stringent conditions with a complementary strand for a nucleic acid consisting of a nucleotide sequence of any one of SEQ ID NOs:1544 to 1621, 1634, 1665 to 1668, 1688, 1709 to 2196, 2862 to 2864, 2978 to 3287, 3365, and 3780 to 3828.
As the above-described nucleic acids, micro-RNAs and micro-RNA precursors are suitably used.

Substances that promote a function or the expression of the nucleic acids (a) to (h) above can also be used as mast cell degranulation suppressants.
Substances that suppress a function or the expression of a target gene for the nucleic acids (a) to (h) above can also be used as mast cell degranulation suppressants. As substances that suppress the expression of a target gene, an siRNA against the mRNA of the target gene, an antisense against the target gene, and the like can be mentioned.

Meanwhile, substances that suppress a function or the expression of the nucleic acids (i) to (p) below can also be used as mast cell degranulation suppressants. As substances that suppress a function or the expression of the nucleic acids, siRNAs and antisenses against the nucleic acids, and the like can be mentioned.
As active ingredients of mast cell degranulation promoters, the nucleic acids (i) to (p) below can be mentioned.
(i) A nucleic acid consisting of a nucleotide sequence of any one of SEQ ID NOs:71 to 150, 582 to 1237, 1462 to 1543, 3372 to 3406, and 3453 to 3741.
(j) A nucleic acid of 17 to 28 nucleotides comprising a nucleic acid consisting of a nucleotide sequence of any one of SEQ ID NOs:71 to 150, 582 to 1237, 1462 to 1543, 3372 to 3406, and 3453 to 3741.
(k) A nucleic acid consisting of a nucleotide sequence having an identity of 90% or more to a nucleotide sequence of any one of SEQ ID NOs:71 to 150, 582 to 1237, 1462 to 1543, 3372 to 3406, and 3453 to 3741.
(l) A nucleic acid that hybridizes under stringent conditions with a complementary strand for a nucleic acid consisting of a nucleotide sequence of any one of SEQ ID NOs:71 to 150, 582 to 1237, 1462 to 1543, 3372 to 3406, and 3453 to 3741.
(m) A nucleic acid comprising the 2nd to 8th nucleotides of a nucleotide sequence of any one of SEQ ID NOs:71 to 150, 582 to 1237, 1462 to 1543, 3372 to 3406, and 3453 to 3741.
(n) A nucleic acid consisting of a nucleotide sequence of any one of SEQ ID NOs:1557, 1575, 1590, 1592 to 1593, 1622 to 1708, 1919 to 1925, 1927 to 1929, 2112, 2197 to 2977, 3092 to 3093, 3162 to 3163, 3169, 3177, 3281, 3288 to 3371, 3742 to 3779, 3790 to 3792, 3807, and 3829 to 4147.
(o) A nucleic acid consisting of a nucleotide sequence having an identity of 90% or more to a nucleotide sequence of any one of SEQ ID NOs:1557, 1575, 1590, 1592 to 1593, 1622 to 1708, 1919 to 1925, 1927 to 1929, 2112, 2197 to 2977, 3092 to 3093, 3162 to 3163, 3169, 3177, 3281, 3288 to 3371, 3742 to 3779, 3790 to 3792, 3807, and 3829 to 4147.
(p) A nucleic acid that hybridizes under stringent conditions with a complementary strand for a nucleic acid consisting of a nucleotide sequence of any one of SEQ ID NOs:1557, 1575, 1590, 1592 to 1593, 1622 to 1708, 1919 to 1925, 1927 to 1929, 2112, 2197 to 2977, 3092 to 3093, 3162 to 3163, 3169, 3177, 3281, 3288 to 3371, 3742 to 3779, 3790 to 3792, 3807, and 3829 to 4147.
As the above-described nucleic acids, micro-RNAs and micro-RNA precursors are suitably used.

Substances that promote a function or the expression of the nucleic acids (i) to (p) above can also be used as mast cell degranulation promoters.
Substances that suppress a function or the expression of a target gene for the nucleic acids (i) to (p) above can also be used as mast cell degranulation promoters. As substances that suppress the expression of a target gene, an siRNA against the mRNA of the target gene, an antisense against the target gene and the like can be mentioned.

Meanwhile, substances that suppress a function or the expression of the nucleic acids (a) to (h) above can also be used as mast cell degranulation promoters. As substances that suppress a function or the expression of the nucleic acids, an siRNA and antisense against the nucleic acids and the like can be mentioned.
As a mast cell degranulation control agent of the present invention, a vector that expresses one of the nucleic acids (a) to (h) and (i) to (p) above can also be used.

Regarding the preparation forms, methods of administration and the like for the mast cell degranulation control agents of the present invention, the same applies as with the diagnostic agents and therapeutic agents containing a nucleic acid such as a micro-RNA, or a micro-RNA precursor, described in 10 below.

### 10. Diagnostic agents and therapeutic agents containing a nucleic acid such as a micro-RNA or a micro-RNA precursor

Nucleic acids, such as micro-RNAs and micro-RNA precursors, used in the present invention can be used as therapeutic agents for diseases resulting from a mast cell abnormality and the like, because they control the expression of genes having a target sequence, or control the expression of nucleic acids, such as micro-RNAs, used in the present invention. Also, siRNAs against a target gene for the nucleic acids can be used as therapeutic agents for diseases resulting from a mast cell abnormality and the like because they control the expression of the gene. As mast cell abnormalities, abnormalities of mast cell differentiation and degranulation, inflammatory mediator production, cytokine production, chemokine production and the like can be mentioned; as diseases caused thereby, atopic dermatitis, asthma, chronic obstructive lung disease, allergic disease and the like can be mentioned.

By quantifying a nucleic acid of the present invention or detecting a mutation therein, a disease resulting from a mast cell abnormality and the like can be diagnosed.
A diagnostic agent comprising a nucleic acid of the present invention, according to the desired diagnostic method, may comprise reagents necessary for quantitation or detection of mutation of_a nucleic acid used in the present invention, for example, buffering agents, salts, reaction enzymes, labeled proteins that bind to_a nucleic acid of the present invention, and a color developer for detection and the like.

Although a therapeutic agent containing as an active ingredient a nucleic acid used in the present invention can be administered alone, the same is normally desirably administered as a pharmaceutical preparation produced by an optionally chosen method known well in the technical field of pharmaceutical making with one or more pharmacologically acceptable carriers blended therein.
The route of administration used is desirably the most effective one in treatment; oral administration, or parenteral administration such as intraoral administration, airway administration, intrarectal administration, subcutaneous administration, intramuscular administration and intravenous administration can be mentioned, and desirably intravenous administration can be mentioned.

As dosage forms, sprays, capsules, tablets, granules, syrups, emulsions, suppositories, injection formulations, ointments, tapes and the like can be mentioned.
As preparations appropriate for oral administration, emulsions, syrups, capsules, tablets, powders, granules and the like can be mentioned.
Liquid preparations like emulsions and syrups can be produced using water, saccharides such as sucrose, sorbitol, and fructose, glycols such as polyethylene glycol and propylene glycol, oils such as sesame oil, olive oil, and soybean oil, antiseptics such as p-hydroxybenzoic acid esters, flavors such as strawberry flavor and peppermint and the like as additives.

Capsules, tablets, powders, granules and the like can be produced using excipients such as lactose, glucose, sucrose, and mannitol, disintegrants such as starch and sodium alginate, lubricants such as magnesium stearate and talc, binders such as polyvinyl alcohol, hydroxypropylcellulose, and gelatin, surfactants such as fatty acid esters, plasticizers such as glycerin and the like as additives.

As appropriate preparations for parenteral administration, injection formulations, suppositories, sprays and the like can be mentioned.
An injection formulation is prepared using a carrier consisting of a salt solution, a glucose solution or a mixture of both and the like. A suppository is prepared using a carrier such as cacao butter, hydrogenated fat or carboxylic acid. A spray is prepared using a carrier that does not stimulate the recipient's oral cavity and airway mucosa, and that disperses the active ingredients as fine particles to facilitate the absorption thereof, and the like.

As examples of the carrier, specifically, lactose, glycerin and the like can be exemplified. Depending on the nature of the nucleic acid used in the present invention, and of the carrier used, preparations such as aerosols and dry powders are possible. In these parenteral preparations, components exemplified as additives for oral preparations can also be added.
The dose or frequency of administration varies depending on desired therapeutic effect, method of administration, duration of treatment, age, body weight and the like, and is normally 10 µg/kg to 20 mg/kg per day for an adult.

A therapeutic agent containing as an active ingredient a nucleic acid used in the present invention can also be produced by blending a vector that expresses the nucleic acid used in the present invention and a base for nucleic acid-based therapeutic agents (Nature Genet., 8, 42(1994)).
The base used in the therapeutic agent of the present invention may be any base for ordinary use in injection formulations; distilled water, solutions of salts such as sodium chloride or a mixture of sodium chloride and an inorganic salt, solutions of mannitol, lactose, dextran, glucose and the like, solutions of amino acids such as glycine and arginine, mixed solutions of organic acid solutions or salt solutions and glucose solution and the like can be mentioned. In accordance with a conventional method, using auxiliary agents such as an osmoregulator, a pH adjuster, a vegetable oil such as sesame oil or soybean oil, lecithin, and a surfactant such as a non-ionic surfactant in these bases, an injection formulation may be prepared as a solution, suspension, or dispersion. These injection formulations can also be prepared as preparations for dissolution before use, by procedures such as powdering and lyophilization. A therapeutic agent of the present invention can be used for treatment as is in the case of a liquid, or after being dissolved in a base described above, optionally sterilized, in the case of a solid, just before treatment.

As a vector that expresses a nucleic acid used in the present invention, the recombinant viral vector prepared by the method described in 5 above can be mentioned, more specifically, a retrovirus vector and a lentivirus vector and the like can be mentioned.
In addition, by combining a nucleic acid used in the present invention with a polylysine-conjugated antibody that is specific for adenovirus hexon protein to prepare a complex, and binding the complex obtained to an adenovirus vector, a viral vector can be prepared. The viral vector is capable of stably reaching the desired cell, being incorporated into cells by endosome, being decomposed in the cells, and efficiently expressing the nucleic acid.

A viral vector based on Sendai virus, which is a (-) strand RNA virus, has been developed (WO97/16538, WO97/16539); using the Sendai virus, a Sendai virus vector that expresses a nucleic acid used in the present invention can be prepared.
A nucleic acid used in the present invention can also be introduced by a non-viral nucleic acid introduction method. The same can be introduced by, for example, calcium phosphate coprecipitation (Virology, 52, 456-467 (1973); Science, 209, 1414-1422 (1980)], microinjection method (Proc. Natl. Acad. Sci. USA, 77, 5399-5403 (1980); Proc. Natl. Acad. Sci. USA, 77, 7380-7384 (1980); Cell, 27, 223-231 (1981); Nature, 294, 92-94 (1981)), membrane fusion-mediated introduction mediated by liposome (Proc. Natl. Acad. Sci. USA, 84, 7413-7417 (1987); Biochemistry, 28, 9508-9514 (1989); J. Biol. Chem., 264, 12126-12129 (1989); Hum. Gene Ther., 3, 267-275, (1992); Science, 249, 1285-1288 (1990); Circulation, 83, 2007-2011 (1992)) or direct DNA uptake and receptor-mediated DNA introduction method (Science, 247, 1465-1468 (1990); J. Biol. Chem., 266, 14338-14342 (1991); Proc. Natl. Acad. Sci. USA, 87, 3655-3659 (1991); J. Biol. Chem., 264, 16985-16987 (1989); BioTechniques, 11, 474-485 (1991); Proc. Natl. Acad. Sci. USA, 87, 3410-3414 (1990); Proc. Natl. Acad. Sci. USA, 88, 4255-4259 (1991); Proc. Natl. Acad. Sci. USA, 87, 4033-4037 (1990); Proc. Natl. Acad. Sci. USA, 88, 8850-8854 (1991); Hum. Gene Ther., 3, 147-154 (1991)) and the like.

Membrane fusion-mediated introduction mediated by liposome allows a nucleic acid used in the present invention to be incorporated locally in the tissue, and to be expressed, by administering a liposome preparation directly to the target tissue (Hum. Gene Ther., 3, 399 (1992)). For direct targeting of a nucleic acid to a focus, a technology including direct uptake of nucleic acid is preferable.
For receptor-mediated nucleic acid introduction, for example, a method performed by binding a nucleic acid to a protein ligand via polylysine can be mentioned. A ligand is chosen on the basis of the presence of a corresponding ligand receptor on the cell surface of the desired cell or tissue. The ligand-nucleic acid conjugate can be injected directly into a blood vessel as desired, and can be directed to a target tissue wherein receptor binding and nucleic acid-protein complex internalization occur. To prevent the destruction of the nucleic acid in a cell, an adenovirus may be infected simultaneously to destroy the endosome function.

### 11. Method of measuring the degree of mast cell activation

The fact that a nucleic acid such as a micro-RNA or a micro-RNA precursor exhibits at least one of the actions of activation suppression, degranulation suppression, inflammatory mediator production suppression, cytokine production suppression, and chemokine production suppression on mast cells can be confirmed by, for example, introducing a nucleic acid used in the present invention or an siRNA against a target gene for the nucleic acid, into a mast cell, thereafter stimulating the mast cell, measuring a released substance such as (i) histamine or p-hexosaminidase, which can be an index of degranulation, (ii) an inflammatory mediator such as LTC4, LTD4, LTE4, or PGD2, (iii) a cytokine such as TNF-α or GM-CSF, or (iv) a chemokine such as IL-8, I-209, or MIP-1α, and comparing the result with that obtained when the nucleic acid used in the present invention, or the siRNA against the target gene for the nucleic acid, is not introduced.

Also, by introducing a nucleic acid, micro RNA or micro-RNA precursor used in the present invention, or an siRNA for a target gene of the micro-RNA, into a mast cell, and detecting the induction of apoptosis by a measurement of the fragmentation of chromatin DNA, the TUNEL method and the like, the fact that the siRNA possesses apoptosis inducing action can be confirmed.
The fact can also be confirmed by introducing a nucleic acid used in the present invention or an siRNA against a target gene for the nucleic acid into a mast cell in the presence of a substance that suppresses the activation of mast cells, thereafter stimulating the mast cell, and comparing the result with that obtained when the nucleic acid used in the present invention or the siRNA against a target gene for the nucleic acid is not introduced.

As example of methods of stimulating a mast cell, a method wherein an anti-IgE antibody is added after the cell is cultured with the addition of IgE, a method wherein Compound 48/80 is added, a method wherein polymyxin B is added, a method wherein dextran is added, a method wherein a calcium ionophore is added, a method wherein acetylcholine is added, a method wherein carbachol is added, a method wherein thrombin is added, a method wherein concanavalin A is added, a method wherein a calcium ionophore is added, a method wherein ATP is added, a method wherein doxorubicin is added, and the like can be mentioned. As examples of substances that suppress the activation of mast cells, substances that inhibit the process wherein a micro-RNA is biosynthesized from a micro-RNA precursor can be mentioned.

Mast cell activation can also be examined by measuring, in place of degranulation, production of cytokines such as TNF-α and GM-CSF, production of chemokines such as IL-8, 1-309, and MIP-1α, production of inflammatory mediators such as LTC4, LTD4, LTE4, and PGD2 and the like [Blood, 100, 3861(2002)].
The present invention is hereinafter described specifically by means of the following Examples. However, the present invention is not limited to these Examples.

### [Example 1]

### Action of forcible expression of micro-RNA precursor on degranulation of human mast cells

A human micro-RNA precursor was introduced into the human mast cell line LAD2, and the influence of the micro-RNA precursor on the degranulation was examined.
LAD2, a recently established human mast cell line, is known to well retain the nature of human mast cells (Leuk. Res., 27, 671 (2003); Leuk. Res., 27, 677 (2003)). LAD2 was obtained from the National Institute of Allergy and Infectious Diseases, National Institutes of Health (Bethesda, MD 20892-1881, USA), and cultured using Stem Pro-34 medium (manufactured by Invitrogen Company) containing 100 ng/mLSCF.

LAD2 was seeded to a 96-well plate at 4×10³ cells per well, a micro-RNA precursor was introduced using a lipofection method, specifically TransIT-TKO (manufactured by Mirus Company), to obtain a final concentration of 25 nM. The micro-RNA precursors used were human micro-RNA library Ver.1 and Ver.2 synthesized at Ambion Company. Lipofection was performed according to the method described in the instruction manual attached to the product.

Two days after the introduction of the micro-RNA precursor by the lipofection method, human myeloma IgE (manufactured by Cosmo Bio Company) was added to obtain a final concentration of 0.3 µg/mL, and the cells were cultured overnight in an incubator at 37°C and a 5% CO₂ concentration. The following day, a rabbit antihuman IgE antibody (manufactured by DAKO Company) was added to obtain a final concentration of 10 µg/mL, and the cells were incubated for 30 minutes in an incubator at 37°C and a 5% CO₂ concentration to induce degranulation. Degrees of degranulation were evaluated by measuring the β-hexosaminidase activity of the enzyme in the granules released in the medium. A measurement of the β-hexosaminidase activity was performed by adding to the medium 40 µL of 20 mmol/L 4-methylumbelliferyl-N-acetyl-β-D-glucosaminide (manufactured by Sigma Company) dissolved in the Tyrode buffer solution (126.1 mmol/L NaCl, 4.0 mmol/L KCl, 1.0 mmol/L CaCl₂, 0.6 mmol/L MgCl₂, 0.6 mmol/L KH₂PO₄, 10 mM HERPES, 5.6 mmol/L D-glucose, 0.1% bovine serum albumin, pH 7.4), incubating the cells at 37°C for 3 hours, thereafter determining the absorbance at 450 nm using the plate reader EnVision (manufactured by Perkin-Elmer Company). After the measurement, Triton X-100 was added to the medium to obtain a final concentration of 1%, and the same experiment was performed, whereby the total β-hexosaminidase activity contained in LAD2 was measured. The ratio of degranulation induced by the anti-IgE antibody was calculated as the ratio (%) to the total β-hexosaminidase activity released with 1% Triton X-100. The relative activity of degranulation was evaluated by the deviation from the median by a calculation of subtracting the median from the degranulation efficiency, and dividing the difference by the mean deviation. Degranulation promoting activity was expressed with plus symbols, and degranulation suppressing activity with minus symbols.
Two independent experiments were performed; the results are shown in Table 5-1 to Table 5-2.

**[Table 5-1]**

| introduced micro-RNA | relative activity of degranulalion | |
|---|---|---|
| | first time | second time |
| hsa-miR-593 | -4.42 | -4.65 |
| hsa-miR-34a | -3.51 | -3.04 |
| hsa-miR-634 | -3.47 | -3.29 |
| hsa-miR-449b | -3.17 | -2.23 |
| hsa-miR-654 | -3.03 | -0.73 |
| hsa-miR-140 | -2.92 | -0.81 |
| hsa-miR-25 | -2.78 | -3.02 |
| hsa-miR-338 | -2.70 | -0.86 |
| hsa-miR-199a | -2.61 | -2.78 |
| hsa-miR-658 | -2.59 | -1.49 |
| hsa-miR-328 | -2.57 | -1.18 |
| hsa-miR-587 | -2.57 | -1.62 |
| hsa-miR-208 | -2.51 | -0.61 |
| hsa-miR-214 | -2.46 | -1.51 |
| hsa-miR-199b | -2.40 | -1.95 |
| hsa-miR-18a | -2.40 | -3.94 |
| hsa-miR-660 | -2.36 | -1.60 |
| hsa-miR-595 | -2.33 | -1.55 |
| hsa-miR-525* | -2.32 | -1.67 |
| hsa-miR-647 | -2.11 | -1.29 |
| hsa-miR-625 | -2,10 | -2.65 |
| hsa-miR-520d | -2.09 | -0.10 |
| hsa-miR-18b | -2.06 | -3.26 |
| hsa-miR-21 | -2.06 | -1.07 |
| hsa-miR-210 | -2.02 | -0.48 |
| hsa-miR-345 | -1.91 | -1.70 |
| hsa-miR-765 | -1.88 | -1.85 |
| hsa-miR-329 | -1.85 | -1.53 |
| hsa-miR-218 | -1.77 | -2.81 |
| hsa-miR-604 | -1.76 | -1.53 |
| hsa-miR-635 | -1.75 | -2.26 |
| hsa-miR-668 | -1.62 | -1.78 |
| hsa-miR-573 | -1.58 | -2.16 |
| hsa-miR-517* | -1.41 | -2.38 |
| hsa-miR-637 | -1.13 | -2.38 |
| hsa-miR-211 | -1.10 | -1.39 |
| hsa-miR-17-3p | -0.76 | -2.05 |
| hsa-miR-515-3p | -0.74 | -2.34 |
| hsa-miR-486 | -0.69 | -2.72 |
| hsa-miR-16 | -0.65 | -3.31 |
| hsa-miR-485-5p | -0.14 | -2.48 |
| hsa-miR-510 | -0.09 | -2.68 |
| hsa-miR-200a* | 0.31 | 2.32 |
| hsa-miR-378 | 0.48 | 4.12 |
| hsa-miR-223 | 0.66 | 2.50 |
| hsa-miR-596 | 0.66 | 2.05 |
| hsa-miR-409-3p | 0.68 | 2.65 |
| hsa-miR-27b | 0.70 | 2.85 |
| hsa-miR-449 | 0.70 | -2.11 |
| hsa-miR-613 | 0.79 | 2.12 |
| hsa-miR-146a | 0.93 | 2.09 |
| hsa-miR-429 | 0.97 | 2.08 |
| hsa-miR-544 | 1.09 | 2.43 |

**[Table 5-2]**

| introduced micro-RNA | relative activity of degranulation | |
|---|---|---|
| | first time | second time |
| hsa-miR-617 | 1.12 | 2.57 |
| hsa-miR-106a | 1.17 | 2.07 |
| hsa-miR-632 | 1.26 | 2.74 |
| hsa-miR-374 | 1.57 | 1.55 |
| hsa-miR-135b | 1.72 | 1.56 |
| hsa-miR-181a | 1.73 | 1.91 |
| hsa-miR-296 | 1.77 | 1.52 |
| hsa-miR-550 | 1.80 | 2.37 |
| hsa-miR-133b | 1.85 | 2.06 |
| hsa-miR-376a | 1.94 | 2.80 |
| hsa-miR-206 | 2.06 | 3.09 |
| hsa-miR-28 | 2.08 | 2.01 |
| hsa-miR-127 | 2.09 | 1.28 |
| hsa-miR-518c | 2.09 | 0.68 |
| hsa-miR-450 | 2.11 | 0.08 |
| hsa-miR-487b | 2.12 | 2.67 |
| hsa-miR-362 | 2.12 | -0.08 |
| hsa-let-7e | 2.45 | 0.46 |
| hsa-miR-33b | 2.47 | 2.39 |
| hsa-miR-216 | 2.62 | 1.84 |
| hsa-miR-373* | 2.80 | 0.32 |
| hsa-miR-182* | 2.82 | 1.18 |
| hsa-miR-146b | 2.87 | 1.63 |
| hsa-miR-1 | 3.23 | 3.69 |
| hsa-miR-659 | 3.71 | 3.41 |
| hsa-miR-126* | 3.73 | 4.52 |

Seven days after the introduction of the micro-RNA precursor as well, human myeloma IgE (manufactured by Cosmo Bio Company) was added to obtain a final concentration of 0.3 µg/mL, and the cells were cultured overnight in an incubator at 37°C and a 5% CO₂ concentration; the following day, degranulation was induced by the addition of a rabbit antihuman IgE antibody, and the ratio of degranulation was measured. The results are shown in Table 6-1 to Table 6-2.

**[Table 6-1]**

| introduced micro-RNA | relative activity of degranulation | |
|---|---|---|
| | first time | second time |
| hsa-miR-593 | -6.23 | -4.03 |
| hsa-miR-634 | -3.91 | -3.29 |
| hsa-miR-647 | -3.14 | -1.75 |
| hsa-miR-595 | -2.70 | -1.60 |
| hsa-miR-190 | -2.50 | -2.22 |
| hsa-miR-587 | -2.48 | -1.87 |
| hsa-miR-658 | -2.43 | -2.46 |
| hsa-miR-25 | -2.33 | -3.04 |
| hsa-miR-329 | -2.32 | -1.69 |
| hsa-miR-520h | -2.25 | -0.68 |
| hsa-miR-195 | -2.23 | -1.44 |
| hsa-miR-325 | -2.22 | -1.55 |
| hsa-miR-449b | -2.19 | -1.66 |
| hsa-miR-199a | -2.16 | -3.53 |
| hsa-miR-425-5p | -2.15 | -1.82 |
| hsa-miR-299-5p | -2.07 | -1.53 |
| hsa-miR-202* | -2.05 | -1.08 |
| hsa-miR-211 | -2.04 | -1.47 |
| hsa-miR-520f | -1.96 | -2.00 |
| hsa-miR-26b | -1.94 | -2.07 |
| hsa-miR-32 | -1.93 | -1.92 |
| hsa-miR-484 | -1.72 | -2.47 |
| hsa-miR-612 | -1.64 | -2.45 |
| hsa-miR-214 | -1.59 | -1.69 |
| hsa-miR-30a-3p | -1.59 | -1.98 |
| hsa-miR-522 | -1.58 | -1.53 |
| hsa-miR-515-5p | -1.50 | -2.18 |
| hsa-miR-199b | -1.49 | -2.49 |
| hsa-miR-107 | -1.45 | -2.09 |
| hsa-miR-24 | -1.33 | -2.63 |
| hsa-miR-20b | -1.26 | -2.12 |
| hsa-miR-488 | -1.18 | -2.37 |
| hsa-miR-675 | -1.13 | -2.71 |
| hsa-miR-766 | -0.86 | -4.41 |
| hsa-miR-486 | -0.57 | -2.18 |
| hsa-miR-148b | -0.15 | -2.08 |
| hsa-miR-514 | -0.04 | 2.18 |
| hsa-miR-650 | 0.14 | -2.34 |
| hsa-miR-524* | 0.64 | 2.92 |
| hsa-miR-133a | 0.74 | 2.55 |
| hsa-miR-432* | 0.82 | 2.09 |
| hsa-miR-448 | 0.85 | 3.29 |
| hsa-miR-489 | 0.93 | 2.03 |
| hsa-miR-192 | 1.02 | 2.37 |
| hsa-miR-550 | 1.18 | 2.54 |
| hsa-miR-128b | 1.31 | 2.78 |
| hsa-miR-31 | 1.42 | 2.10 |
| hsa-miR-542-3p | 1.51 | 2.68 |
| hsa-miR-27b | 1.52 | 2.58 |
| hsa-miR-22D | 1.53 | 1.82 |
| hsa-miR-409-3p | 1.53 | 2.36 |
| hsa-miR-33b | 1.54 | 2.07 |
| hsa-miR-187 | 1.54 | 1.71 |

**[Table 6-2]**

| introduced micro-RNA | relative activity of degranulation | |
|---|---|---|
| | first time | second time |
| hsa-miR-452 | 1.69 | 2.11 |
| hsa-miR-450 | 1.72 | 1.79 |
| hsa-miR-517c | 1.79 | 2.62 |
| hsa-miR-451 | 1.85 | 2.15 |
| hsa-miR-609 | 1.88 | 1.81 |
| hsa-miR-215 | 1.92 | 3.26 |
| hsa-miR-552 | 1.92 | 1.64 |
| hsa-miR-100 | 1.94 | 1.75 |
| hsa-miR-409-5p | 2.04 | 0.84 |
| hsa-miR-608 | 2.04 | 0.37 |
| hsa-miR-142-5p | 2.07 | 2.50 |
| hsa-miR-224 | 2.09 | 2.21 |
| hsa-miR-601 | 2.10 | 1.63 |
| hsa-miR-545 | 2.11 | 1.97 |
| hsa-miR-28 | 2.12 | 1.26 |
| hsa-miR-96 | 2.16 | 1.90 |
| hsa-miR-129 | 2.16 | 3.08 |
| hsa-miR-487b | 2.20 | 2.61 |
| hsa-let-7e | 2.25 | -0.14 |
| hsa-miR-223 | 2.26 | 1.23 |
| hsa-miR-181a | 2.26 | 1.38 |
| hsa-miR-801 | 2.27 | 1.90 |
| hsa-miR-136 | 2.31 | 0.22 |
| hsa-miR-183 | 2.34 | 1.66 |
| hsa-miR-9 | 2.35 | 1.94 |
| hsa-miR-302c* | 2.37 | 0.92 |
| hsa-miR-133b | 2.38 | 3.02 |
| hsa-miR-1 | 2.39 | 2.59 |
| hsa-miR-769-5p | 2.40 | 1.18 |
| hsa-miR-627 | 2.46 | 1.55 |
| hsa-miR-302b* | 2.47 | -0.25 |
| hsa-miR-222 | 2.54 | 2.14 |
| hsa-miR-217 | 2.65 | 0.86 |
| hsa-miR-378 | 2.66 | 2.93 |
| hsa-miR-23b | 2.71 | -1.04 |
| hsa-miR-644 | 2.88 | 1.45 |
| hsa-miR-182* | 3.11 | 4.32 |
| hsa-miR-216 | 3.34 | 0.70 |
| hsa-miR-659 | 3.77 | 2.95 |

These results revealed that the ratio of degranulation decreased with the introduction of micro-RNA precursors corresponding to hsa-miR-16, 195, 17-3p, 18a, 18b, 20b, 21, 24, 25, 32, 26b, 30a-3p, 34a, 449, 449b, 107, 140, 148b, 190, 199a, 199b, 202*, 208, 210, 211, 214, 218, 299-5p, 325, 328, 329, 338, 345, 425-5p, 484, 485-5p, 486, 488, 510, 515-3p, 515-5p, 517*, 520d, 520f, 520h, 522, 525*, 573, 587, 593, 595, 604, 612, 625, 634, 635, 637, 647, 650, 654, 658, 660, 668, 675, 765, and 766 (these are nucleic acids consisting of the nucleotide sequences of SEQ ID NOs:1 to 66, respectively), and conversely the ratio of degranulation increased with the introduction of micro-RNA precursors corresponding to hsa-let-7e, hsa-miR-1, 206, 613, 9, 23b, 27b, 28, 31, 33b, 96, 100, 106a, 126*, 127, 128b, 129, 133a, 133b, 135b, 136, 142-5p, 146a, 181a, 182*, 183, 187, 192, 215, 200a*, 216, 217, 220, 222, 223, 224, 296, 302b*, 302c*, 362, 373*, 374, 376a, 378, 409-3p, 409-5p, 429, 432*, 448, 450, 451, 452, 487b, 489, 514, 517c, 518c, 524*, 542-3p, 544, 545, 550, 552, 596, 601, 608, 609, 617, 627, 632, 644, 659, 769-5p, and 801 (these are nucleic acids consisting of the nucleotide sequences of SEQ ID NOs:71 to 118 and 120 to 146).

A measurement of degranulation activity using a 6-well plate was also performed. LAD2 was seeded to a 6-well plate at 5×10⁵ cells per well, a micro-RNA precursor was introduced using a lipofection method, specifically Gene Silencer (manufactured by Genlantis Company), to obtain a final concentration of 25 nM. The micro-RNA, precursors used were Pre-miR™miRNA Precursor Molecules synthesized at Ambion Company. Lipofection was performed according to the method described in the instruction manual attached to the product.

Two days after the introduction of the micro-RNA precursor by the lipofection method, 1.0 µg/mL human myeloma IgE (manufactured by Cosmo Bio Company) was added, and the cells were cultured overnight in an incubator at 37°C and a 5% CO₂ concentration. The following day, the medium was removed via centrifugation, and the plate was washed with the Tyrode buffer solution (126.1 mmol/L NaCl, 4.0 mmol/L KCl, 1.0 mmol/L CaCl₂, 0.6 mmol/L MgCl₂, 0.6 mmol/L KH₂PO₄, 10 mM HEPES, 5.6 mmol/L D-glucose, 0.1% bovine serum albumin, pH 7.4), after which the cells were suspended by the addition of 1.5 mL of the Tyrode buffer solution, and the suspension was dispensed to a 96-well plate at 100 µL per well. Subsequently, a rabbit antihuman IgE antibody (manufactured by DAKO Company) was added to obtain a final concentration of 10 µg/mL, and the cells were incubated for 20 minutes in an incubator at 37°C and a 5% CO₂ concentration to induce degranulation. The supernatant was recovered via centrifugation, and the β-hexosaminidase activity in the supernatant was measured to determine the degree of degranulation.

The β-hexosaminidase activity was evaluated by adding to 50 µL of the recovered supernatant 50 µL of 4 mmol/L p-nitrophenyl-N-acetyl-β-glucosaminide (manufactured by Sigma Company) dissolved in 40 mmol/L citrate solution (pH 4.5), and incubating the supernatant at 37°C for 1 hour, thereafter measuring the absorbance of a sample supplemented with 100 µL of 0.2 mol/L glycine (pH 10.7) at 405 nm using the plate reader 1420 ARVOsx (manufactured by Perkin-Elmer Company). Separately, Triton X-100 was added at a final concentration of 1% in place of the rabbit antihuman IgE antibody, and the same experiment was performed, whereby the total β-hexosaminidase activity in LAD2 was measured. The ratio of degranulation was calculated as the ratio (%) of the β-hexosaminidase activity in the supernatant to the total β-hexosaminidase activity, and the relative activity of degranulation was calculated for each precursor with the ratio of degranulation in a negative control plot (Gene Silencer only) taken as 1.0.

Seven days after the introduction of the micro-RNA precursor, human myeloma IgE (manufactured by Cosmo Bio Company) was added to obtain a final concentration of 1.0 µg/mL, and the cells were cultured overnight in an incubator at 37°C and a 5% CO₂ concentration; the following day, a rabbit antihuman IgE antibody was added to induce degranulation, the ratio of degranulation was measured, and the relative activity of degranulation was calculated for each precursor with the ratio of degranulation in a negative control plot (Gene Silencer only) taken as 1.0.
The results for the relative activity of degranulation at 3 days and 8 days after the introduction of each micro-RNA precursor are shown in Table 7.

**[Table 7]**

| introduced mism-RNA/siRNA | relative activity of degranulation | |
|---|---|---|
| | 3 days later | 5 days later |
| hsa-miR-197 | 0.52 | 0.62 |
| hsa-miR-221 | 0.60 | 0.63 |
| hsa-miR-200a | 1.17 | 2.16 |
| hsa-miR-200c | 1.64 | 2.73 |
| hsa-miR-142-3p | 1.71 | 2.94 |
| hsa-miR-361 | 1.82 | 1.13 |

As shown in Table 7, it was found that the ratio of degranulation increased with the introduction of micro-RNA precursors corresponding to hsa-miR-200c, 142-3p, 200a, and 361 (these are nucleic acids consisting of the nucleotide sequences of SEQ ID NOs:119 and 148 to 150, respectively), and that conversely the ratio of degranulation decreased with the introduction of hsa-miR-197 and 221 (these are nucleic acids consisting of the nucleotide sequences of SEQ ID NOs:69 and 70, respectively).

### [Example 2]

### Degranulation activity in human mast cells with micro-RNA antisense introduced thereto

Each of antisense oligonucleotides against hsa-miR-194, hsa-miR-500 and hsa-miR-365 (these are nucleic acids consisting of the nucleotide sequences of SEQ ID NOs: 67, 68 and 147, respectively) was introduced into LAD2 using a lipofection method to obtain a final concentration of 25 nM. The micro-RNA antisense oligonucleotides used were Anti-miR™miRNA Inhibitors (manufactured by Ambion Company). Lipofection was performed according to the method described in the instruction manual attached to the product.

Two days after the introduction of the micro-RNA antisense oligonucleotide by the lipofection method, human myeloma IgE (manufactured by Cosmo Bio Company) was added to obtain a final concentration of 0.3 µg/mL, and the cells were cultured overnight in an incubator at 37°C and a 5% CO₂ concentration. The following day, a rabbit antihuman IgE antibody (manufactured by DAKO Company) was added to obtain a final concentration of of 10 µg/mL, and the cells were incubated for 30 minutes in an incubator at 37°C and a 5% CO₂ concentration to induce degranulation. Degrees of degranulation were evaluated by measuring the β-hexosaminidase activity of the enzyme in the granules released into the medium. The measurement of the β-hexosaminidase activity was performed by adding to the medium 40 µL of 20 mmol/L 4-methylumbelliferyl-N-acetyl-β-D-glucosaminide (manufactured by Sigma Company) dissolved in the Tyrode buffer solution (126.1 mmol/L NaCl, 4.0 mmol/L KCl, 1.0 mmol/L CaCl, 0.6 mmol/L MgCl₂, 0.6 mmol/L KH₂PO₄, 10 mM HEPES, 5.6 mmol/L D-glucose, 0.1% bovine serum albumin, pH 7.4), and incubating the cells at 37°C for 3 hours, thereafter determining the absorbance at 450 nm using the plate reader Envision (manufactured by Perkin-Elmer Company).

After the measurement, Triton X-100 was added to the medium to obtain a final concentration of 1%, and the same experiment was performed, whereby the total β-hexosaminidase activity contained in LAD2 was measured. The mean for the wells with no cells seeded thereto was calculated, and this was subtracted as the background from each measured value. The ratio of degranulation induced by the anti-IgE antibody was calculated as the ratio (%) of the β-hexosaminidase activity to the total β-hexosaminidase activity released with 1% Triton X-100. The relative activity of degranulation was evaluated by the deviation from the median by a calculation of subtracting the median from the degranulation efficiency, and dividing the difference by the mean deviation. Degranulation promoting activity was expressed with plus symbols, and degranulation suppressing activity with minus symbols. Two independent experiments were performed; the results are shown in Table 8.

**[Table 8]**

| introduced antisense | relative active of degranulation, | |
|---|---|---|
| | first time | second time |
| hsa-miR-194 | 3.69 | 2.67 |
| hsa-miR-500 | 4.42 | 2.36 |
| hsa-miR-365 | -2.35 | -2.16 |

Seven days after the introduction of the antisense oligonucleotide against the micro-RNA, human myeloma IgE (manufactured by Cosmo Bio Company) was added to obtain a final concentration of 0.3 µg/mL, and the cells were cultured overnight in an incubator at 37°C and a 5% CO₂ concentration; the following day, a rabbit antihuman IgE antibody was added to induce degranulation, and the ratio of degranulation was measured. The results are shown in Table 9.

**[Table 9]**

| introduced antisense | relative activity of degranulation | |
|---|---|---|
| | first time | second time |
| hsa-miR-194 | 2.63 | 2.05 |
| hsa-miR-500 | 3.75 | 2.82 |
| hsa-miR-365 | -0.94 | -0.77 |

As shown in Tables 8 and 9, it was found that the ratio of degranulation increased with the introduction of the antisense oligonucleotide against hsa-miR-194 or hsa-miR-500, and that conversely the ratio of degranulation decreased with the introduction of the antisense oligonucleotide against hsa-miR-365.

### [Example 3]

### Action of forcible expression of micro-RNA precursor on degranulation in mast cells in degranulation-suppressed state

An siRNA of the Dicerl gene (hereinafter, the Dicerl-siRNA) and a human micro-RNA precursor were co-transfected to LAD2, and the influence of the micro-RNA precursor on the degranulation stimulated by Compound 48/80 was examined.

LAD2 was seeded to a 96-well plate at 2×10⁴ cells per well, and the Dicerl-siRNA and the micro-RNA precursor were co-transfected using a lipofection method, specifically GeneSilencer (manufactured by Genlantis Company), to obtain a final concentration of 25 nM of each. The sequence of the siRNA against the human Dicerl gene used was the Dicerl-siRNA that targets SEQ ID NO:4148 (manufactured by QIAGEN Company). The micro-RNA precursor used was a human micro-RNA library synthesized at Ambion Company. As negative controls for the siRNA and the micro-RNA precursor, All Stars Negative Control siRNA (hereinafter, siRNA allstars) (manufactured by QIAGEN Company) and Pre-miR™miRNA Precursor Negative Control #1 (hereinafter, miR-negacon#1) (manufactured by Ambion Company), respectively, were provided, and were introduced into LAD2 in the same manner. Lipofection was performed according to the method described in the instruction manual attached to the product.

Three days after the introduction of the micro-RNA precursor by the lipofection method, the culture medium was replaced with the Tyrode buffer solution (126.1 mmol/L NaCl, 4.0 mmol/L KCl, 1.0 mmol/L CaCl₂, 0.6 mmol/L MgCl₂, 0.6 mmol/L KH₂PO₄, 10 mM HEPES, 5.6 mmol/L D-glucose, 0.1% bovine serum albumin, pH 7.4), and the cells were suspended. Subsequently, equal volumes of the cell suspension were seeded to two different sites in a 96-well plate, and Compound 48/80 (manufactured by Sigma-Aldrich Company) at a final concentration of 1.0 µg/mL and Triton X-100 at a final concentration of 1%, respectively, were added. After incubation at 37°C for 20 minutes to induce degranulation, centrifugation was performed, and the culture supernatant was recovered.

Degrees of degranulation were evaluated by measuring the β-hexosaminidase activity of the enzyme in the granules released into the culture supernatant. The β-hexosaminidase activity was evaluated by adding to the recovered culture supernatant 50 mL of 4 mmol/L 4-nitrophenyl-N-acetyl-β-D-glucosaminide (manufactured by Sigma-Aldrich Company) dissolved in 40 mmol/L citric acid buffer solution (pH 4.5), and incubating the supernatant at 37°C for 1 hour, thereafter determining the absorbance of a sample supplemented with 100 mL of 0.2 mol/L glycine (pH 10.7) at 405 nm using the plate reader 1420 ARVOsx (manufactured by Perkin-Elmer Company).

The ratio of degranulation induced by Compound 48/80 was calculated as the ratio (%) to the β-hexosaminidase activity in the plot with the addition of 1% Triton X-100. The activity of the micro-RNA for cancelling the degranulation suppression due to introduction of the Dicerl-siRNA was calculated as a relative activity with the ratio of degranulation in the plot with co-transfection of the Dicerl-siRNA and miR-negacon#1 taken as 0%, and with the ratio of degranulation in the plot with co-transfection of the siRNA allstars and micro-RNA-negacon#1 taken as 100%. The results are shown in Table 10.

**[Table 10]**

| introduced micro-RNA | relative activity of degranulation | |
|---|---|---|
| | first time | second time |
| hsa-miR-29b | 153.1 | 115.1 |
| hsa-miR-589 | 152.2 | ND |
| hsa-miR-299-3p | 131.2 | 94.1 |
| hsa-miR-29a | 120.6 | 88.1 |
| hsa-miR-30b | 118.2 | 58.3 |
| hsa-miR-506 | 117.3 | 97.2 |
| hsa-miR-105 | 117.3 | 110.1 |
| hsa-miR-127 | 113.8 | 167.3 |
| hsa-miR-769-5p | 104.7 | ND |
| hsa-miR-597 | 104.4 | ND |
| hsa-miR-504 | 102.1 | 63.9 |
| hsa-miR-770-5p | 101.4 | ND |
| hsa-miR-150 | 101.2 | 90.4 |
| hsa-miR-605 | 101.0 | ND |
| hsa-miR-640 | 100.0 | ND |
| hsa-miR-154 | 99.7 | 151.0 |
| hsa-miR-380-5p | 97.7 | 13.6 |
| hsa-miR-629 | 92.6 | ND |
| hsa-miR-660 | 92.4 | ND |
| hsa-miR-128b | 90.0 | 112.9 |
| hsa-miR-518c | 90.0 | -40.0 |
| hsa-miR-767-5p | 88.5 | ND |
| hsa-miR-423 | 87.1 | 11.1 |
| hsa-miR-567 | 87.1 | ND |
| hsa-miR-33a-5p | 86.2 | 60.9 |
| hsa-miR-383 | 84.2 | -16.9 |
| hsa-miR-497 | 82.8 | 33.3 |
| hsa-miR-124a | 82.3 | 83.4 |
| hsa-miR-512-3p | 81.8 | 117.5 |
| hsa-miR-133a | 81.0 | 63.5 |
| hsa-miR-501 | 75.9 | 65.6 |
| hsa-miR-133b | 74.1 | 103.8 |
| hsa-miR-514 | 69.7 | 50.5 |
| hsa-miR-619 | 64.8 | 151.1 |
| hsa-miR-527 | 62.9 | 55.0 |
| hsa-miR-454-3p | 61.3 | 54.9 |
| hsa-miR-148a | 60.5 | 82.0 |
| hsa-miR-411 | 58.4 | 71.9 |
| hsa-miR-128a | 58.4 | 96.1 |
| hsa-miR-511 | 58.1 | 71.2 |
| hsa-miR-508 | 55.3 | 91.1 |
| hsa-miR-562 | 53.8 | 81.3 |
| hsa-miR-802 | 52.2 | 58.2 |
| hsa-miR-146a | 52.1 | 56.1 |
| hsa-miR-373* | 31.5 | 82.1 |
| hsa-miR-433 | 22.6 | 84.7 |
| hsa-miR-507 | -6.2 | 107.4 |

### [Example 4]

### mRNA expression analysis of LAD2 forcibly overexpressing miR-142-3p precursor and miR-24 precursor

Each of the miR-142-3p and miR-24 precursors of human micro-RNA was introduced into LAD2, and gene clusters the amounts of whose expression fluctuate due to introduction of each of the micro-RNA, precursors were comprehensively searched for using an RNA array.
LAD2 was seeded to a 6-well plate at 5×10⁵ cells per well, and each micro-RNA precursor was introduced using a lipofection method, specifically GeneSilencer (manufactured by Genlantis Company), to obtain a final concentration of 30 nM. The micro-RNA precursors used were Pre-miR™ miRNA Precursor Molecules synthesized at Ambion Company. For negative control, Pre-miR™RNA Precursor Negative Control#2 manufactured by Ambion Company (hereinafter, miR-negacon#2) was used. Lipofection was performed according to the method described in the instruction manual attached to the product.

Two days after the introduction of each of the micro-RNA precursors by the lipofection method, the cells were recovered, and total RNA was purified from the cells using miRNeasy (manufactured by QIAGEN Company).
The purified total RNA was analyzed for mRNA expression using the Whole Human Genome Oligo Microarray manufactured by Agilent Technologies Company. The analytical procedure was in accordance with the protocol of Agilent Technologies Company. Specifically, a cRNA obtained by labeling the total RNA with Cy3 was hybridized to the microarray at 65°C for about 17 hours, and the microarray was washed, after which the microarray was scanned at a resolution of 5 µm using the Agilent technologies Microarray Scanner to acquire signal values.

To examine the gene clusters exhibiting an expressional fluctuation in the plots with the introduction of the miR-142-3p or miR-24 precursor, respectively, the relative activity of the amount expressed was calculated with the signal value for each gene detected in the plot with the introduction of miR-negacon#2 taken as 1.0. In the plots with the introduction of the miR-142-3p precursor or the miR-24 precursor, gene clusters whose expression was suppressed to the extent of a relative activity of 0.8 or less are shown in Table 11 and Table 12-1 to Table 12-3, respectively.

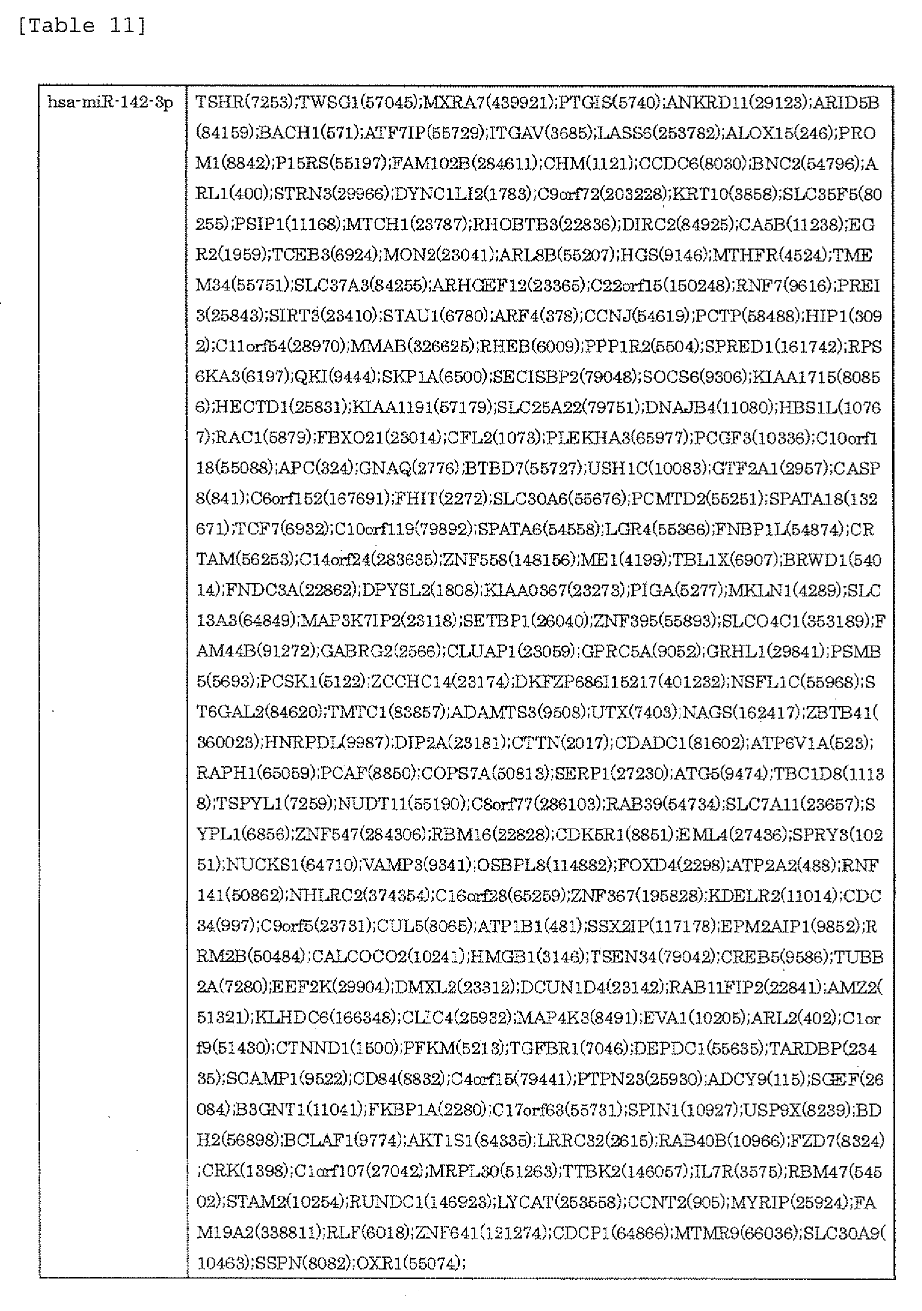

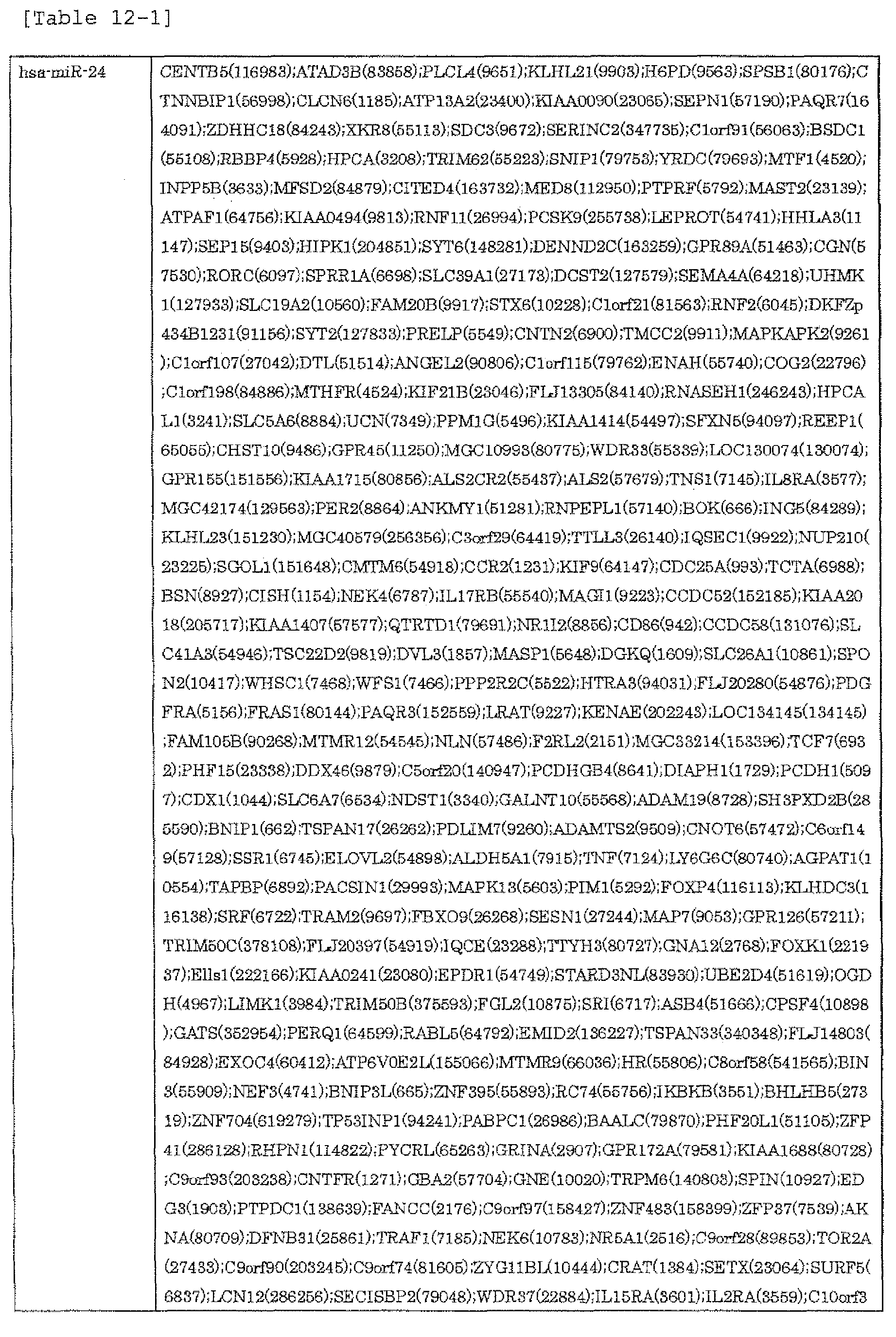

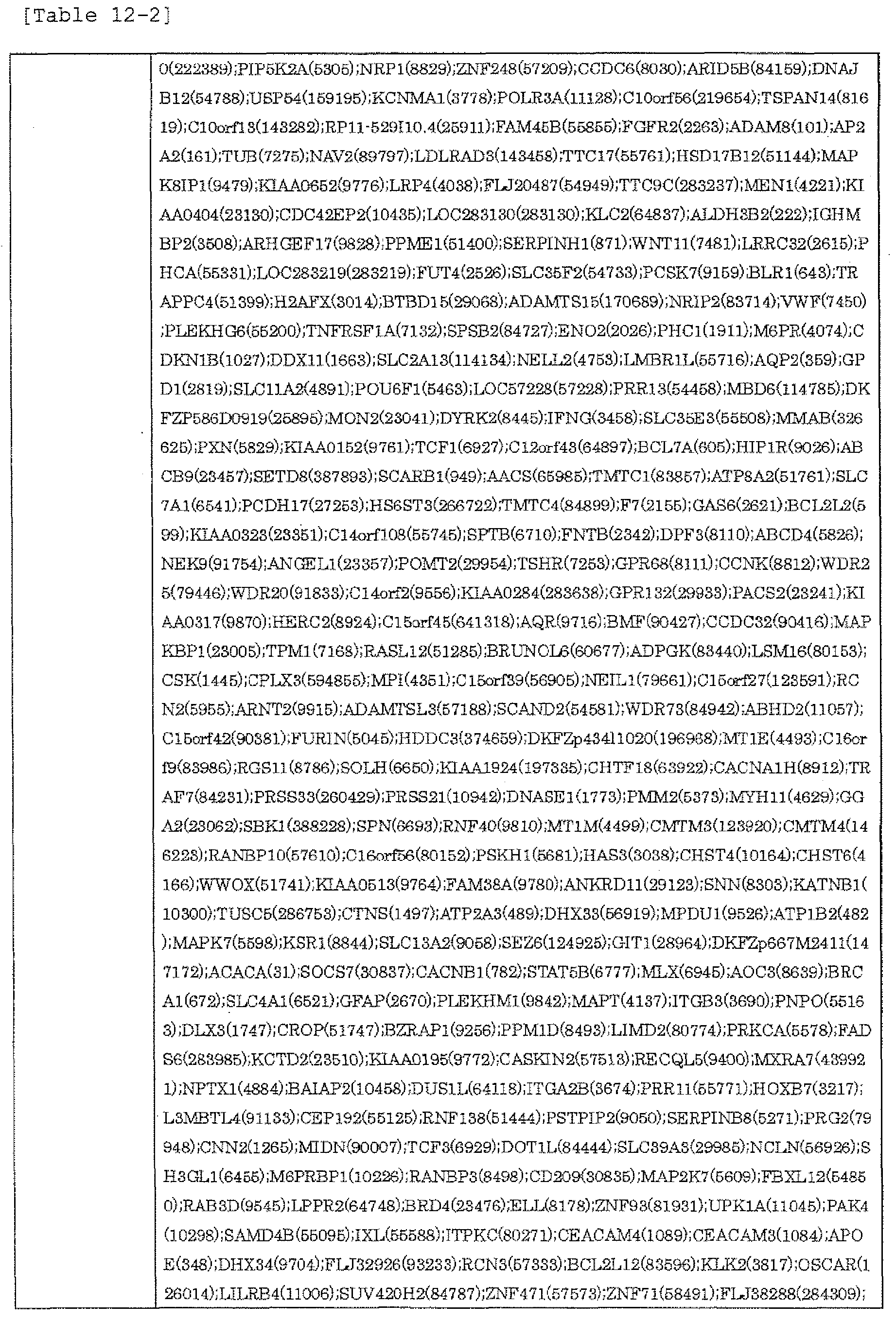

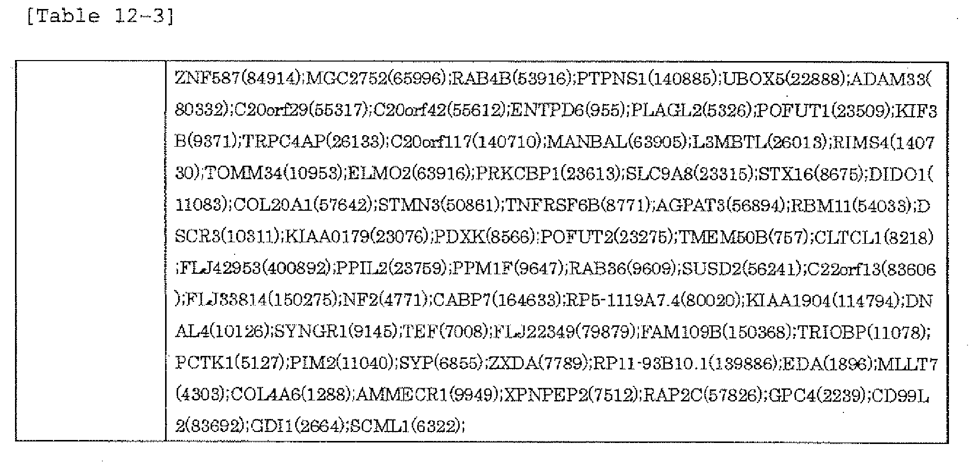

### Industrial Applicability

According to the present invention, a mast cell degranulation control agent, a diagnostic agent or therapeutic agent for a disease resulting from mast cell degranulation control, a method of controlling mast cell degranulation, and a screening method for a mast cell degranulation control agent can be provided. These are useful in the diagnosis or treatment of a disease resulting from mast cell degranulation control.

### [Free text of sequence listing]

SEQ ID NO:3916 - n represents a, c, g or u
SEQ ID NO:4148 - explanation of artificial sequences: synthetic DNAs

## Claims

1. A mast cell degranulation control agent comprising as an active ingredient any one of the nucleic acids (a) to (h) below:
(a) a nucleic acid consisting of a nucleotide sequence of any one of SEQ ID NOs:1 to 1543 and 3372 to 3741,
(b) a nucleic acid of 17 to 28 nucleotides comprising a nucleic acid consisting of a nucleotide sequence of any one of SEQ ID NOs:1 to 1543 and 3372 to 3741,
(c) a nucleic acid consisting of a nucleotide sequence having an identity of 90% or more to a nucleotide sequence of any one of SEQ ID NOs:1 to 1543 and 3372 to 3741,
(d) a nucleic acid that hybridizes under stringent conditions with a complementary strand for a nucleic acid consisting of a nucleotide sequence of any one of SEQ ID NOs:1 to 1543 and 3372 to 3741,
(e) a nucleic acid comprising the 2nd to 8th nucleotides of a nucleotide sequence of any one of SEQ ID Nos:1 to 1543 and 3372 to 3741,
(f) a nucleic acid consisting of a nucleotide sequence of any one of SEQ ID NOs:1544 to 3371 and 3742 to 4147,
(g) a nucleic acid consisting of a nucleotide sequence having an identity of 90% or more to a nucleotide sequence of any one of SEQ ID NOs:1544 to 3371 and 3742 to 4147, and
(h) a nucleic acid that hybridizes under stringent conditions with a complementary strand for a nucleic acid consisting of a nucleotide sequence of any one of SEQ ID NOs:1544 to 3371 and 3742 to 4147.

2. The mast cell degranulation control agent according to claim 1, wherein the nucleic acid is a micro-RNA or a micro-RNA precursor.

3. A mast cell degranulation control agent comprising as an active ingredient a nucleic acid consisting of a nucleotide sequence complementary to the nucleotide sequence of the nucleic acid of claim 1.

4. A mast cell degranulation control agent comprising as an active ingredient a double-stranded nucleic acid consisting of the nucleic acid of claim 1 and a nucleic acid consisting of a nucleotide sequence complementary to the nucleotide sequence of the former nucleic acid.

5. A mast cell degranulation control agent comprising as an active ingredient a vector that expresses the nucleic acid of any one of claims 1 to 3 or the double-stranded nucleic acid of claim 4.

6. A mast cell degranulation control agent comprising as an active ingredient a substance that controls the expression or a function of the nucleic acid of claim 1.

7. The mast cell degranulation control agent according to claim 6, wherein the substance that controls the expression or a function of the nucleic acid is an siRNA or an antisense oligonucleotide.

8. A mast cell degranulation control agent comprising as an active ingredient a substance that suppresses the expression of a target gene for the nucleic acid of claim 1.

9. The mast cell degranulation control agent according to claim 8, wherein the substance that suppresses the expression of a target sequence for a nucleic acid is an siRNA or an antisense oligonucleotide.

10. A diagnostic agent or therapeutic agent for a disease resulting from an abnormality of mast cells, comprising as an active ingredient the nucleic acid of any one of claims 1 to 3, the double-stranded nucleic acid of claim 4, the vector of claim 5, or the substance of any one of claims 6 to 9.

11. A diagnostic agent for a disease resulting from an abnormality of mast cells, comprising as an active ingredient a reagent for detecting the amount expressed of the nucleic acid of claim 1, a mutation of the nucleic acid or a mutation of the genome that encodes the nucleic acid.

12. The diagnostic agent or therapeutic agent according to claim 10 or 11, wherein the disease resulting from an abnormality of mast cells is a disease selected from the group consisting of atopic dermatitis, asthma, chronic obstructive pulmonary disease and allergic diseases.

13. A method for treating a disease resulting from an abnormality of mast cells, comprising administering an effective amount of the degranulation suppressant of any one of claims 1 to 9 to a subject in need thereof.

14. The method according to claim 13, wherein the disease resulting from an abnormality of mast cells is a disease selected from the group consisting of atopic dermatitis, asthma, chronic obstructive pulmonary disease and allergic diseases.

15. A use of the degranulation suppressant of any one of claims 1 to 9 for producing a therapeutic agent for a disease resulting from an abnormality of mast cells.

16. The use according to claim 15, wherein the disease resulting from an abnormality of mast cells is a disease selected from the group consisting of atopic dermatitis, asthma, chronic obstructive pulmonary disease and allergic diseases.

17. A method of controlling mast cell degranulation, comprising using the nucleic acid of any one of claims 1 to 3, the double-stranded nucleic acid of claim 4, the vector of claim 5, or the substance of any one of claims 6 to 9.

18. A screening method for a mast cell degranulation control agent, wherein the promotion or suppression of the expression or a function of the nucleic acid of claim 1 serves as an index.
